(19) Europäisches Patentamt
European Patent Office
Office européen des brevets

(11) EP 3 494 982 A1

(12) **EUROPEAN PATENT APPLICATION**

(43) Date of publication:
**12.06.2019 Bulletin 2019/24**

(21) Application number: **19152916.3**

(22) Date of filing: **29.12.2015**

(51) Int Cl.:
***A61K 38/00*** *(2006.01)* ***C12N 15/67*** *(2006.01)*
***C12N 15/85*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2014 PCT/EP2014/003479**

(62) Document number(s) of the earlier application(s) in accordance with Art. 76 EPC:
**15820544.3 / 3 240 558**

(71) Applicant: **CureVac AG**
**72076 Tübingen (DE)**

(72) Inventors:
- **GRUND, Stefanie**
  **72076 Tübingen (DE)**
- **SCHLAKE, Thomas**
  **72076 Tübingen (DE)**

(74) Representative: **Graf von Stosch Patentanwaltsgesellschaft mbH**
**Prinzregentenstraße 22**
**80538 München (DE)**

Remarks:
This application was filed on 21-01-2019 as a divisional application to the application mentioned under INID code 62.

# (54) ARTIFICIAL NUCLEIC ACID MOLECULES

(57) The invention relates to an artificial nucleic acid molecule comprising at least one open reading frame and at least one 3'-untranslated region element (3'-UTR element) and/or at least one 5'-untranslated region element (5'-UTR element), wherein the at least one 3'-UTR element and/or the at least one 5'-UTR element prolongs and/or increases protein production from said artificial nucleic acid molecule and wherein the at least one 3'-UTR element and/or the at least one 5'-UTR element is derived from a stable mRNA. The invention further relates to the use of such an artificial nucleic acid molecule in gene therapy and/or genetic vaccination. Furthermore, methods for identifying a 3'-UTR element and/or a 5'-UTR derived from a stable mRNA element are disclosed.

EP 3 494 982 A1

SEQUENCE LISTING

<110> CureVac AG

<120> ARTIFICIAL NUCLEIC ACID MOLECULES

<130> CU01P167WO1EPT1

<150> PCT/EP2014/003479
<151> 2014-12-30

<160> 388

<170> PatentIn version 3.5

<210> 1
<211> 351
<212> DNA
<213> Artificial Sequence

<220>
<223> Mus musculus GNAS 3'-UTR

<400> 1
gaagggaaca cccaaattta attcagcctt aagcacaatt aattaagagt gaaacgtaat 60

gtacaagcag ttggtcaccc accatagggc atgatcaaca ccgcaacctt tcctttttcc 120

cccagtgatt ctgaaaaacc cctcttccct tcagcttgct tagatgttcc aaatttagta 180

agcttaaggc ggcctacaga agaaaaagaa aaaaaaggcc acaaaagttc cctctcactt 240

tcagtaaata aaataaaagc agcaacagaa ataaagaaat aaatgaaatt caaaatgaaa 300

taaatattgt ttgtgcagca ttaaaaaatc aataaaaatt aaaaatgagc a 351

<210> 2
<211> 353
<212> DNA
<213> Artificial Sequence

<220>
<223> Mus musculus GNAS 3'-UTR

<400> 2
gaagggaaca cccaaattta attcagcctt aagcacaatt aattaagagt gaaacgtaat 60

tgtacaagca gttggtcacc caccataggg catgatcaac accgcaacct ttcctttttc 120

ccccagtgat tctgaaaaac ccctcttccc ttcagcttgc ttagatgttc caaatttagt 180

aagcttaagg cggcctacag aagaaaaaga aaaaaaaggc cacaaaagtt ccctctcact 240

ttcagtaaat aaaataaaag cagcaacaga aataaagaaa taaatgaaat tcaaaatgaa 300

ataaatattg tgttgtgcag cattaaaaaa tcaataaaaa ttaaaaatga gca 353

<210> 3
<211> 385
<212> DNA
<213> Artificial Sequence

<220>
<223> Homo sapiens GNAS 3'-UTR

<400> 3
gaagggaacc cccaaattta attaaagcct taagcacaat taattaaaag tgaaacgtaa 60

ttgtacaagc agttaatcac ccaccatagg gcatgattaa caaagcaacc tttcccttcc 120

cccgagtgat tttgcgaaac cccctttttcc cttcagcttg cttagatgtt ccaaatttag 180

aaagcttaag gcggcctaca gaaaaaggaa aaaaggccac aaaagttccc tctcactttc 240

agtaaaaata aataaaacag cagcagcaaa caaataaaat gaaataaaag aaacaaatga 300

aataaatatt gtgttgtgca gcattaaaaa aaatcaaaat aaaaattaaa tgtgagcaaa 360

gaatgaaaaa aaaaaaaaaa aaaaa 385

<210> 4
<211> 1476
<212> DNA
<213> Artificial Sequence

<220>
<223> Homo sapiens GNAS 3'-UTR

<400> 4
tggaggacgc cgtccagatt ctccttgttt tcatggattc aggtgctgga gaatctggta 60

aaagcaccat tgtgaagcag atgaggatcc tgcatgttaa tgggtttaat ggagagggcg 120

gcgaagagga cccgcaggct gcaaggagca acagcgatgg cagtgagaag gcaaccaaag 180

tgcaggacat caaaaacaac ctgaaagagg cgattgaaac cattgtggcc gccatgagca 240

acctggtgcc ccccgtggag ctggccaacc ccgagaacca gttcagagtg gactacatcc 300

tgagtgtgat gaacgtgcct gactttgact tccctcccga attctatgag catgccaagg 360

ctctgtggga ggatgaagga gtgcgtgcct gctacgaacg ctccaacgag taccagctga 420

ttgactgtgc ccagtacttc ctggacaaga tcgacgtgat caagcaggct gactatgtgc 480

cgagcgatca ggacctgctt cgctgccgtg tcctgacttc tggaatcttt gagaccaagt 540

tccaggtgga caaagtcaac ttccacatgt ttgacgtggg tggccagcgc gatgaacgcc 600

gcaagtggat ccagtgcttc aacgatgtga ctgccatcat cttcgtggtg gccagcagca 660

gctacaacat ggtcatccgg gaggacaacc agaccaaccg cctgcaggag gctctgaacc 720

tcttcaagag catctggaac aacagatggc tgcgcaccat ctctgtgatc ctgttcctca 780

acaagcaaga tctgctcgct gagaaagtcc ttgctgggaa atcgaagatt gaggactact 840

ttccagaatt tgctcgctac actactcctg aggatgctac tcccgagccc ggagaggacc 900

cacgcgtgac ccgggccaag tacttcattc gagatgagtt tctgaggatc agcactgcca 960

gtggagatgg gcgtcactac tgctaccctc atttcacctg cgctgtggac actgagaaca 1020

tccgccgtgt gttcaacgac tgccgtgaca tcattcagcg catgcacctt cgtcagtacg 1080

agctgctcta agaagggaac ccccaaattt aattaaagcc ttaagcacaa ttaattaaaa 1140

gtgaaacgta attgtacaag cagttaatca cccaccatag ggcatgatta acaaagcaac 1200

ctttcccttc ccccgagtga ttttgcgaaa ccccctttc ccttcagctt gcttagatgt 1260

tccaaattta gaaagcttaa ggcggcctac agaaaaagga aaaaaggcca caaaagttcc 1320

ctctcacttt cagtaaaaat aaataaaaca gcagcagcaa acaaataaaa tgaaataaaa 1380

gaaacaaatg aaataaatat tgtgttgtgc agcattaaaa aaaatcaaaa taaaaattaa 1440

atgtgagcaa agaatgaaaa aaaaaaaaaa aaaaaa 1476

<210> 5
<211> 117
<212> DNA
<213> Artificial Sequence

<220>
<223> Mus musculus MORN2 3'-UTR

<400> 5
acctgctgcc ttaacgctga gatgtggcct ctgcaacccc ccttaggcaa agcaactgaa 60

ccttctgcta aagtgacctg ccctcttccg taagtccaat aaagttgtca tgcaccc 117

<210> 6
<211> 135
<212> DNA
<213> Artificial Sequence

<220>
<223> Mus musculus MORN2 3'-UTR

<400> 6
acctgctgcc ttaacgctga gatgtggcct ctgcaacccc ccttaggcaa agcaactgaa 60

ccttctgcta aagtgacctg ccctcttccg taagtccaat aaagttgtca tgcacccaca 120

aaaaaaaaaa aaaaa 135

<210> 7
<211> 210
<212> DNA
<213> Artificial Sequence

<220>
<223> Homo sapiens MORN2 3'-UTR

<400> 7
catgtagatg tgatgttaaa ttaaagttga aatgtagtaa ttgaagcttt tagttgtaag 60

gaaagcaact taatctgtta tttgaaatga cttcatacac taccccctata agtttgccaa 120

taaaaccatc acctgcttac acctttttga actttatatt cattgtctta caattagttt 180

aaaataaatg acatgattca aaaaaaaaaa 210

<210> 8
<211> 438
<212> DNA
<213> Artificial Sequence

<220>
<223> Mus musculus GSTM1 3'-UTR

<400> 8
gcccttgcta cacgggcact cactaggagg acctgtccac actggggatc ctgcaggccc 60

tgggtgggga cagcaccctg gccttctgca ctgtggctcc tggttctctc tccttcccgc 120

tcccttctgc agcttggtca gccccatctc ctcaccctct tcccagtcaa gtccacacag 180

ccttcattct ccccagtttc tttcacatgg ccccttcttc attggctccc tgacccaacc 240

tcacagcccg tttctgcgaa ctgaggtctg tcctgaactc acgcttccta gaattacccc 300

gatggtcaac actatcttag tgctagccct ccctagagtt accccgaagg tcaatacttg 360

agtgccagcc tgttcctggt ggagtagcct ccccaggtct gtctcgtcta caataaagtc 420

tgaaacacac ttgccatg 438

<210> 9
<211> 455
<212> DNA
<213> Artificial Sequence

<220>
<223> Mus musculus GSTM1 3'-UTR

<400> 9
gcccttgcta cacgggcact cactaggagg acctgtccac actggggatc ctgcaggccc 60

tgggtgggga cagcaccctg gccttctgca ctgtggctcc tggttctctc tccttcccgc 120

tcccttctgc agcttggtca gccccatctc ctcaccctct tcccagtcaa gtccacacag 180

ccttcattct ccccagtttc tttcacatgg ccccttcttc attggctccc tgacccaacc 240

tcacagcccg tttctgcgaa ctgaggtctg tcctgaactc acgcttccta gaattacccc 300

gatggtcaac actatcttag tgctagccct ccctagagtt accccgaagg tcaatacttg 360

agtgccagcc tgttcctggt ggagtagcct ccccaggtct gtctcgtcta caataaagtc 420

tgaaacacac ttgccatgaa aaaaaaaaaa aaaaa 455

<210> 10
<211> 531
<212> DNA
<213> Artificial Sequence

<220>
<223> Homo sapiens GSTM1 3'-UTR

<400> 10

ggccttgaag gccaggaggt gggagtgagg agcccatact cagcctgctg cccaggctgt 60

gcagcgcagc tggactctgc atcccagcac ctgcctcctc gttcctttct cctgtttatt 120

cccatcttta ctcccaagac ttcattgtcc ctcttcactc cccctaaacc cctgtcccat 180

gcaggcccctt tgaagcctca gctacccact atccttcgtg aacatcccct cccatcatta 240

cccttccctg cactaaagcc agcctgacct tccttcctgt tagtggttgt gtctgcttta 300

aagggcctgc ctggcccctc gcctgtggag ctcagccccg agctgtcccc gtgttgcatg 360

aaggagcagc attgactggt ttacaggccc tgctcctgca gcatggtccc tgccttaggc 420

ctacctgatg gaagtaaagc ctcaaccaca aaaaaaaaaa aaaaaaaaaa aaaaaaaaaa 480

aaaaaaaaaa aaaaaaaaaa aaaaaaaaaa aaaaaaaaaa aaaaaaaaaa a 531

<210> 11
<211> 133
<212> DNA
<213> Artificial Sequence

<220>
<223> Mus musculus NDUFA1 3'-UTR

<400> 11
ggaagcattt tcctggctga ttaaaagaaa ttactcagct atggtcatct gttcctgtta 60

gaaggctatg cagcatatta tatactatgc gcatgttatg aaatgcataa taaaaaattt 120

taaaaaatct aaa 133

<210> 12
<211> 148
<212> DNA
<213> Artificial Sequence

<220>
<223> Homo sapiens NDUFA1 3'-UTR

<400> 12
ggaagcattt tcctgattga tgaaaaaaat aactcagtta tggccatcta cccctgctag 60

aaggttacag tgtattatgt agcatgcaat gtgttatgta gtgcttaata aaaataaaat 120

gaaaaaaatg caaaaaaaaa aaaaaaaa 148

<210> 13
<211> 237
<212> DNA
<213> Artificial Sequence

<220>
<223> Mus musculus CBR2 3'-UTR

<400> 13
tctgctcagt tgccgcggac atctgagtgg ccttcttagc cccaccctca gccaaagcat 60

ttactgatct cgtgactccg ccctcatgct acagccacgc ccaccacgca gctcacagtt 120

ccaccccat gttactgtcg atcccacaac cactccaggc gcagaccttg ttctctttgt 180

ccactttgtt gggctcattt gcctaaataa acgggccacc gcgttacctt taactat 237

<210> 14
<211> 416
<212> DNA
<213> Artificial Sequence

<220>
<223> Mus musculus YBX1 3'-UTR

<400> 14
atgccggctt accatctcta ccatcatccg gtttggtcat ccaacaagaa gaaatgaata 60

tgaaattcca gcaataagaa atgaacaaag attggagctg aagaccttaa gtgcttgctt 120

tttgcccgct gaccagataa cattagaact atctgcatta tctatgcagc atggggtttt 180

tattattttt acctaaagat gtctcttttt ggtaatgaca aacgtgtttt ttaagaaaaa 240

aaaaaaaggc ctggtttttc tcaatacacc tttaacggtt tttaaattgt ttcatatctg 300

gtcaagttga gatttttaag aacttcattt ttaatttgta ataaagttta caacttgatt 360

ttttcaaaaa agtcaacaaa ctgcaagcac ctgttaataa aggtcttaaa taataa 416

<210> 15
<211> 418
<212> DNA
<213> Artificial Sequence

<220>
<223> Mus musculus YBX1 3'-UTR

<400> 15
atgccggctt accatctcta ccatcatccg gtttggtcat ccaacaagaa gaaatgaata 60

tgaaattcca gcaataagaa atgaacaaag attggagctg aagaccttaa gtgcttgctt 120

tttgccctct gaccagataa cattagaact atctgcatta tctatgcagc atggggtttt 180

tattattttt acctaaagat gtctcttttt ggtaatgaca aacgtgtttt ttaagaaaaa 240

aaaaaaaaag gcctggtttt tctcaataca cctttaacgg tttttaaatt gtttcatatc 300

tggtcaagtt gagattttta agaacttcat ttttaatttg taataaagtt tacaacttga 360

ttttttcaaa aaagtcaaca aactgcaagc acctgttaat aaaggtctta aataataa 418

<210> 16
<211> 415
<212> DNA
<213> Artificial Sequence

<220>
<223> Homo sapiens YBX1 3'-UTR

<400> 16

atgccggctt accatctcta ccatcatccg gtttagtcat ccaacaagaa gaaatatgaa 60

attccagcaa taagaaatga acaaaagatt ggagctgaag acctaaagtg cttgcttttt 120

gcccgttgac cagataaata gaactatctg cattatctat gcagcatggg gtttttatta 180

tttttaccta aagacgtctc tttttggtaa taacaaacgt gttttttaaa aaagcctggt 240

ttttctcaat acgcctttaa aggtttttaa attgtttcat atctggtcaa gttgagattt 300

ttaagaactt catttttaat ttgtaataaa agtttacaac ttgatttttt caaaaaagtc 360

aacaaactgc aagcacctgt taataaaggt cttaaataat aaaaaaaaaa aaaaa 415

<210> 17
<211> 66
<212> DNA
<213> Artificial Sequence

<220>
<223> Mus musculus Ndufb8 3'-UTR

<400> 17
ggaggcttga tgggcttttt gccctcgttc ctagaggctt aaccataata aaatccctaa 60

taaagc 66

<210> 18
<211> 125
<212> DNA
<213> Artificial Sequence

<220>
<223> Homo sapiens Ndufb8 3'-UTR

<400> 18
ggaggcttcg tgggcttttg ggtcctctaa ctaggactcc ctcattccta gaaatttaac 60

cttaatgaaa tccctaataa aactcagtgc tgtgttattt gtgcctcaaa aaaaaaaaaa 120

aaaaa 125

<210> 19
<211> 377
<212> DNA
<213> Artificial Sequence

<220>
<223> Homo sapiens Ndufb8 3'-UTR

<400> 19
gtgaggaaga ggagtgctgt tcctgccttc ctagcccagc tgggtctgac cagaggctac 60

tgtgtaccca tttaccatgc gtgattgtta actcagagtg gggtgtagcc aggtattgac 120

tgaatgtatg ttcttgctga cctgtgtttt tttctgtagg gaccaaagca gtatccttac 180

aataatctgt acctggaacg aggcggtgat ccctccaaag aaccagagcg ggtggttcac 240

tatgagatct gaggaggctt cgtgggcttt tgggtcctct aactaggact ccctcattcc 300

tagaaattta accttaatga aatccctaat aaaactcagt gctgtgttat ttgtgcctca 360

aaaaaaaaaa aaaaaaa 377

<210> 20
<211> 922
<212> DNA
<213> Artificial Sequence

<220>
<223> Homo sapiens CNTN1-004 3'-UTR

<400> 20
tcgttgacac tcaccatttc tgtgaaagac tttttttttt tttaacatat tatactagat 60

ttgactaact caatcttgta gcttctgcag ttctccccac cccaacccta gttcttagag 120

tatgtttccc cttttgaaac atgtaaacat actttgggca taaatatttt ttaaaatata 180

actataatgc ttcactaata ccttaaaaat gcctagtgaa ctaactcagt acattatata 240

atggccaagt gaaagttttg tgttttcatg tcctgttttt ctttgaaatt atatagccca 300

gaaattagct cattatctga aaaacgtata agaactgatg aattgtataa tacaggagta 360

ttgccattga atgtactgtt tgatttattc aagcaggtaa tgaacaatgt tgtcaaactc 420

tctaatgaga catcataatt aggacataag ctaaaagggg cattactccg gcagtctttt 480

tttcttaatc ctagtaccat acatattctt tggcatgaaa gaatgaaaag cattagtaaa 540

caactgaagt cctaccatgg ctctgtaggg tttttggaac aattcctgga attggaaagt 600

gaaaatggat agcatgtggg ggaaaccctc atctgagtag caagatttta gtaaagatga 660

ctaagccatt aacagcatgc attcatattt aattttattg actcctgcca tcagcttttg 720

tagatctttt gggtggaagg ttgtgatttt tactgggagg acttgagtag aagtggatga 780

ttaaaattga ggagtatata attctttctg ggactgctta aatgttattg tttgaaaatg 840

ccttcacttt ccccctttgg tcaaagagat gtgcttaaaa ttcttattcc ttcacaataa 900

ataattttga ttttcttaga ca 922

<210> 21
<211> 922
<212> DNA
<213> Artificial Sequence

<220>
<223> Homo sapiens CNTN1-004 3'-UTR

<400> 21
tcgttgacac tcaccatttc tgtgaaagac tttttttttt ttaacatatt atactagatt 60

tgactaactc aatcttgtag cttctgcagt tctccccacc cccaacctag ttcttagagt 120

atgtttcccc ttttgaaaca tgtaaacata ctttgggcat aaatattttt taaaatataa 180

ctataatgct tcactaatac cttaaaaatg cctagtgaac taactcagta cattatataa 240

tggccaagtg aaagttttgt gttttcatgt cctgtttttc tttgaaatta tatagcccag 300

aaattagctc attatctgaa aaacgtatga agaactgatg aattgtataa tacaggagta 360

ttgccattga atgtactgtt tgatttattc aagcaggtaa tgaacaatgt tgtcaaactc 420

tctaatgaga catcataatt aggacataag ctaaaagggg cattactccg gcagtctttt 480

tttcttaatc ctagtaccat acatattctt tggcatgaaa gaatgaaaag cattagtaaa 540

caactgaagt cctaccatgg ctctgtaggg tttttggaac aattcctgga attggaaagt 600

gaaaatggat agcatgtggg ggaaaccctc atctgagtag caagatttta gtaaagatga 660

ctaagccatt aacagcatgc attcatattt aattttattg actcctgcca tcagcttttg 720

tagatcgttt gggtggaagg ttgtgatttt tactgggagg acttgagtag aagtggatga 780

ttaaaattga ggagtatata attctttctg ggactgctta aatgttattg tttgaaaata 840

ccttcacttt ccccctttgg tcaaagagat gtgcttaaaa ttcttattcc ttcacaataa 900

ataattttga ttttcttaga ca 922

<210> 22
<211> 928
<212> DNA
<213> Artificial Sequence

<220>
<223> Homo sapiens CNTN1-004 3'-UTR

<400> 22
tttttcgtt gacactcacc atttctgtga aagacttttt tttttttaa catattatac 60

tagatttgac taactcaatc ttgtagcttc tgcagttctc cccaccccca acctagttct 120

tagagtatgt ttcccctttt gaaacatgta aacatacttt gggcataaat attttttaaa 180

atataactat aatgcttcac taatacctta aaaatgccta gtgaactaac tcagtacatt 240

atataatggc caagtgaaag ttttgtgttt tcatgtcctg tttttctttg aaattatata 300

gcccagaaat tagctcatta tctgaaaaac gtatgaagaa ctgatgaatt gtataataca 360

ggagtattgc cattgaatgt actgtttgat ttattcaagc aggtaatgaa caatgttgtc 420

aaactctcta atgagacatc ataattagga cataagctaa aaggggcatt actccggcag 480

tcttttttc ttaatcctag taccatacat attctttggc atgaaagaat gaaaagcatt 540

agtaaacaac tgaagtccta ccatggctct gtagggtttt tggaacaatt cctggaattg 600

gaaagtgaaa atggatagca tgtgggggaa accctcatct gagtagcaag attttagtaa 660

agatgactaa gccattaaca gcatgcattc atatttaatt ttattgactc ctgccatcag 720

cttttgtaga tcttttgggt ggaaggttgt gatttttact gggaggactt gagtagaagt 780

ggatgattaa aattgaggag tatataattc tttctgggac tgcttaaatg ttattgtttg 840

aaaatgcctt cactttcccc ctttggtcaa agagatgtgc ttaaaattct tattccttca 900

caataaataa ttttgatttt cttagaca 928

<210> 23
<211> 2380
<212> DNA
<213> Artificial Sequence

<220>
<223> Homo sapiens CNTN1-004 3′-UTR

<400> 23
atgtgttgtg acagctgctg ttcccatccc agctcagaag acacccttca accctgggat 60

gaccacaatt ccttccaatt tctgcggctc catcctaagc caaataaatt atactttaac 120

aaactattca actgatttac aacacacatg atgactgagg cattcgggaa ccccttcatc 180

caaaagaata aacttttaaa tggatataaa tgatttttaa ctcgttccaa tatgccttat 240

aaaccactta acctgattct gtgacagttg catgatttaa cccaatggga caagttacag 300

tgttcaattc aatactatag gctgtagagt gaaagtcaaa tcaccatata caggtgcttt 360

aaatttaata acaagttgtg aaatataata gagattgaaa tgttggttgt atgtggtaaa 420

tgtaagagta atacagtctc ttgtactttc ctcactgttt tgggtactgc atattattga 480

atggccccta tcattcatga catcttgagt tttcttgaaa agacaataga gtgtaacaaa 540

tattttgtca gaaatcccat tatcaaatca tgagttgaaa gattttgact attgaaaacc 600

aaattctaga acttactatc agtattctta ttttcaaagg aaataatttt ctaaatattt 660

gattttcaga atcagttttt taatagtaaa gttaacatac catatagatt tttttttact 720

tttatattct actctgaagt tattttatgc ttttcttatc aatttcaaat ctcaaaaatc 780

acagctctta tctagagtat cataatattg ctatatttgt tcatatgtgg agtgacaaat 840

tttgaaaagt agagtgcttc ctttttttatt gagatgtgac agtctttaca tggttaggaa 900

taagtgacag ttaagtgaat atcacaatta ctagtatgtt ggtttttctg cttcattcct 960

aagtattacg tttctttatt gcagatgtca gatcaaaaag tcacctgtag gttgaaaaag 1020

ctaccgtatt ccattttgta aaaataacaa taataataat aataataatt agttttaagc 1080

tcatttccca cttcaatgca atactgaaaa ctggctaaaa ataccaaatc aatatactgc 1140

taatggtact ttgaagagta tgcaaaactg gaaggccagg aggaggcaaa taatatgtct 1200

ttccgatggt gtctcccaag tgttggtgct ttgggttttt ataagttgtg aaaaggaaga 1260

tgcacatttc ttcattctcc atggtgtgca tggaaatgtg tttgagtgtg gatgtaaaag 1320

aaatcgagta ataaagaatt agctggcttg tgaaatagtg cagtgttgga tgcttcaaga 1380

ggtataatcc tattttatta gcacaaactt gctagctaat tagagtttat ctttttagaa 1440

aggacaccgt ataggttcgt aaaaaatatt tacaggaagc aaaatagatc tattactact 1500

ttaccgactt tacccccttt ctttaatttg tataattttt gtactatata tcgatgtgta 1560

aatgtttaga gtcttcatta tgaaaatatc aataaatatt tcattagttt acatttaact 1620

ctggtataaa atgaaacttt taaaaataag tgaaatggat gatttcccag tggaagtatg 1680

tcaacagtct taagatcatt gccagatttc ataaaatatt taagtatttg aaaaagaaac 1740

aaaatgtctt catactttag ggaaacgaat accctgtata ccttctgtac aaatgtttgt 1800

gttttcattg ttacactttg gggttttact tttgcaatgt gacccatgtt gggcattttt 1860

atataatcaa caactaaatc ttttgccaaa tgcatgcttg ccttttattt tctaatatat 1920

gataataacg agcaaaactg gttagatttt gcatgaaatg gttctgaaag gtaagaggaa 1980

aacagacttt ggaggttgtt tagttttgaa tttctgacag agataaagta gtttaaaatc 2040

tctcgtacac tgataactca agcttttcat tttctcatac agttgtacag atttaactgg 2100

gaccatcagt tttaaactgt tgtcaagcta actaataatc atctgcttta agacgcaaga 2160

ttctgaatta aactttatat aggtatagat acatctgttg tttctttgta tttcaggaaa 2220

ggtgatagta gttttatttg atactgataa atattgaatt gatttttttag ttattttttta 2280

tcatttttttc aatggagtag tataggactg tgctttgtcc tttttatgaa tgaaaaaatt 2340

agtataaagt aataaatgtc ttatgttacc caagaaaaaa 2380

<210> 24
<211> 1204
<212> DNA
<213> Artificial Sequence

<220>
<223> Homo sapiens CNTN1-004 3'-UTR

<400> 24
tcgttgacac tcaccatttc tgtgaaagac tttttttttt ttaacatatt atactagatt 60

tgactaactc aatcttgtag cttctgcagt tctccccacc cccaacctag ttcttagagt 120

atgtttcccc ttttgaaaca tgtaaacata ctttgggcat aaatattttt taaaatataa 180

ctataatgct tcactaatac cttaaaaatg cctagtgaac taactcagta cattatataa 240

tggccaagtg aaagttttgt gttttcatgt cctgtttttc tttgaaatta tatagcccag 300

aaattagctc attatctgaa aaacgtatga agaactgatg aattgtataa tacaggagta 360

ttgccattga atgtactgtt tgatttattc aagcaggtaa tgaacaatgt tgtcaaactc 420

tctaatgaga catcataatt aggacataag ctaaaagggg cattactccg gcagtctttt 480

tttcttaatc ctagtaccat acatattctt tggcatgaaa gaatgaaaag cattagtaaa 540

caactgaagt cctaccatgg ctctgtaggg tttttggaac aattcctgga attggaaagt 600

gaaaatggat agcatgtggg ggaaaccctc atctgagtag caagatttta gtaaagatga 660

ctaagccatt aacagcatgc attcatattt aattttattg actcctgcca tcagcttttg 720

tagatcgttt gggtggaagg ttgtgatttt tactgggagg acttgagtag aagtggatga 780

ttaaaattga ggagtatata attctttctg ggactgctta aatgttattg tttgaaaata 840

ccttcacttt ccccctttgg tcaaagagat gtgcttaaaa ttcttattcc ttcacaataa 900

ataattttga ttttcttaga caggtttgtg tttaggtatg agtttctctt ttacttcatc 960

tagcaattct ctctgtggtc agaagaactc tgaagaaagc tttgagggaa atgaatataa 1020

ctcttaaatt attatatgtg tgtgtatata tatagtttaa ctttaaaaat aatttattag 1080

tcatcataaa gaaataaatg tctctggctc aagatgttac ttatttcctt cttttatatt 1140

ttctagtctc aattactgtt ccaaaaggag ctatcttaga acttagacta gagatccaga 1200

ttaa 1204

<210> 25
<211> 54
<212> DNA
<213> Artificial Sequence

<220>
<223> Mus musculus MP68 5'-UTR

<400> 25
ctttcccatt ctgtagcaga atttggtgtt gcctgtggtc ttggtcccgc ggag 54

<210> 26
<211> 97
<212> DNA
<213> Artificial Sequence

<220>
<223> Homo sapiens MP68 5'-UTR

<400> 26
cttcccggca tcccctgcgc gcgcctgcgc gctcggtgac ctttccgagt tggctgcaga 60

tttgtggtgc gttctgagcc gtctgtcctg cgccaag 97

<210> 27
<211> 315
<212> DNA
<213> Artificial Sequence

<220>
<223> Homo sapiens MP68 5'-UTR

<400> 27
cttcccggca tcccctgcgc gcgcctgcgc gctcggtgac ctttccgagt tggctgcaga 60

tttgtggtgc gttctgagcc gtctgtcctg cgccaaggga gcgtaccttg gccttgagag 120

gttcagctgc ctaacccaga ggctacgcag agttagagaa gccagagtcc aagccaagaa 180

ctctgactcc acatccagtc ccttctctcc tttataactc aagtttcctt gcgccacact 240

gccctccacg ttatgctgta catgacaact tgggtgaggc aacagggaag ctgaaaagag 300

atcatacggt gctga 315

<210> 28
<211> 81
<212> DNA
<213> Artificial Sequence

<220>
<223> Mus musculus NDUFA4 5'-UTR

<400> 28
gtccgctcag ccaggttgca gaagcggctt agcgtgtgtc ctaatcttct ctctgcgtgt 60

aggtaggcct gtgccgcaaa c 81

<210> 29
<211> 81
<212> DNA
<213> Artificial Sequence

<220>
<223> Homo sapiens NDUFA4 5'-UTR

<400> 29
guccgcucag ccagguugca gaagcggcuu agcgugugucc cuaaucuucu cucugcgugu 60

agguaggccu gugccgcaaa c 81

<210> 30
<211> 129
<212> DNA
<213> Artificial Sequence

<220>
<223> Homo sapiens NDUFA4 5'-UTR

<400> 30
gggtccttca ggtaggaggt cctgggtgac tttggaagtc cgtagtgtct cattgcagat 60

aatttttagc ttagggcctg gtggctaggt cggttctctc ctttccagtc ggagacctct 120

gccgcaaac 129

<210> 31
<211> 186
<212> DNA
<213> Artificial Sequence

<220>
<223> Human albumin 3'-UTR

<400> 31
catcacattt aaaagcatct cagcctacca tgagaataag agaaagaaaa tgaagatcaa 60

aagcttattc atctgttttt ctttttcgtt ggtgtaaagc caacaccctg tctaaaaaac 120

ataaatttct ttaatcattt tgcctctttt ctctgtgctt caattaataa aaaatggaaa 180

gaatct 186

<210> 32
<211> 186
<212> DNA
<213> Artificial Sequence

<220>
<223> albumin7 3'-UTR

<400> 32
catcacattt aaaagcatct cagcctacca tgagaataag agaaagaaaa tgaagatcaa 60

tagcttattc atctcttttt ctttttcgtt ggtgtaaagc caacaccctg tctaaaaaac 120

ataaatttct ttaatcattt tgcctctttt ctctgtgctt caattaataa aaaatggaaa 180

gaacct 186

<210> 33
<211> 42
<212> DNA
<213> Artificial Sequence

<220>
<223> 5'-UTR of human ribosomal protein Large 32 lacking the 5' terminal oligopyrimidine tract

<400> 33
ggcgctgcct acggaggtgg cagccatctc cttctcggca tc 42

<210> 34
<211> 24
<212> DNA
<213> Artificial Sequence

<220>
<223> histone stem-loop

<400> 34
caaaggctct tttcagagcc acca 24

<210> 35
<211> 1848
<212> RNA
<213> Artificial Sequence

<220>
<223> 32L4 - PpLuc(GC) - A64 - C30 - hSL

<400> 35
ggggcgcugc cuacggaggu ggcagccauc uccuucucgg caucaagcuu gaggauggag 60

gacgccaaga acaucaagaa gggcccggcg cccuucuacc cgcuggagga cgggaccgcc 120

ggcgagcagc uccacaaggc caugaagcgg uacgcccugg ugccgggcac gaucgccuuc 180

accgacgccc acaucgaggu cgacaucacc uacgcggagu acuucgagau gagcgugcgc 240

cuggccgagg ccaugaagcg guacggccug aacaccaacc accggaucgu ggugugcucg 300

gagaacagcc ugcaguucuu caugccggug cugggcgccc ucuucaucgg cguggccguc 360

gccccggcga acgacaucua caacgagcgg gagcugcuga acagcauggg gaucagccag 420

ccgaccgugg uguucgugag caagaagggc cugcagaaga uccugaacgu gcagaagaag 480

cugcccauca uccagaagau caucaucaug gacagcaaga ccgacuacca gggcuuccag 540

ucgauguaca cguucgugac cagccaccuc ccgccgggcu ucaacgagua cgacuucguc 600

ccggagagcu ucgaccggga caagaccauc gcccugauca ugaacagcag cggcagcacc 660

ggccugccga agggggugc ccugccgcac cggaccgccu gcgugcgcuu cucgcacgcc 720

cgggacccca ucuucggcaa ccagaucauc ccggacaccg ccauccugag cguggugccg 780

uuccaccacg gcuucggcau guucacgacc cugggcuacc ucaucugcgg cuuccgggug 840

guccugaugu accgguucga ggaggagcug uuccugcgga gccugcagga cuacaagauc 900

cagagcgcgc ugcucgugcc gacccuguuc agcuucuucg ccaagagcac ccugaucgac 960

aaguacgacc ugucgaaccu gcacgagauc gccagcgggg gcgccccgcu gagcaaggag 1020

gugggcgagg ccguggccaa gcgguuccac cucccgggca uccgccaggg cuacggccug 1080

accgagacca cgagcgcgau ccugaucacc cccgaggggg acgacaagcc gggcgccgug 1140

ggcaaggugg ucccguucuu cgaggccaag gugguggacc uggacaccgg caagacccug 1200

ggcgugaacc agcggggcga gcugugcgug cgggggccga ugaucaugag cggcuacgug 1260

aacaacccgg aggccaccaa cgcccucauc gacaaggacg gcuggcugca cagcggcgac 1320

aucgccuacu gggacgagga cgagcacuuc uucaucgucg accggcugaa gucgcugauc 1380

aaguacaagg gcuaccaggu ggcgccggcc gagcuggaga gcauccugcu ccagcacccc 1440

aacaucuucg acgccggcgu ggccgggcug ccggacgacg acgccggcga gcugccggcc 1500

gcgguggugg ugcuggagca cggcaagacc augacggaga aggagaucgu cgacuacgug 1560

gccagccagg ugaccaccgc caagaagcug cggggcggcg uggguguucgu ggacgagguc 1620

ccgaagggcc ugaccgggaa gcucgacgcc cggaagaucc gcgagauccu gaucaaggcc 1680

aagaagggcg gcaagaucgc cguguaagac uaguagaucu aaaaaaaaaa aaaaaaaaaa 1740

aaaaaaaaaa aaaaaaaaaa aaaaaaaaaa aaaaaaaaaa aaaaugcauc cccccccccc 1800

cccccccccc cccccccccc aaaggcucuu uucagagcca ccagaauu 1848

<210> 36
<211> 2201
<212> RNA
<213> Artificial Sequence

<220>
<223> 32L4 - PpLuc(GC) - gnas-A64-C30-hSL

<400> 36
ggggcgcugc cuacggaggu ggcagccauc uccuucucgg caucaagcuu gaggauggag 60
gacgccaaga acaucaagaa gggcccggcg cccuucuacc cgcuggagga cgggaccgcc 120
ggcgagcagc uccacaaggc caugaagcgg uacgcccugg ugccgggcac gaucgccuuc 180
accgacgccc acaucgaggu cgacaucacc uacgcggagu acuucgagau gagcgugcgc 240
cuggccgagg ccaugaagcg guacggccug aacaccaacc accggaucgu ggugugcucg 300
gagaacagcc ugcaguucuu caugccggug cugggcgccc ucuucaucgg cguggccguc 360
gccccggcga acgacaucua caacgagcgg gagcugcuga acagcauggg gaucagccag 420
ccgaccgugg uguucgugag caagaagggc cugcagaaga uccugaacgu gcagaagaag 480
cugcccauca uccagaagau caucaucaug gacagcaaga ccgacuacca gggcuuccag 540
ucgauguaca cguucgugac cagccaccuc ccgccgggcu ucaacgagua cgacuucguc 600
ccggagagcu ucgaccggga caagaccauc gcccugauca ugaacagcag cggcagcacc 660
ggccugccga agggggugc ccugccgcac cggaccgccu gcgugcgcuu cucgcacgcc 720
cgggacccca ucuucggcaa ccagaucauc ccggacaccg ccauccugag cgugguggccg 780
uuccaccacg gcuucggcau guucacgacc cugggcuacc ucaucugcgg cuuccgggug 840
guccugaugu accgguucga ggaggagcug uuccugcgga gccugcagga cuacaagauc 900
cagagcgcgc ugcucgugcc gacccuguuc agcuucuucg ccaagagcac ccugaucgac 960
aaguacgacc ugucgaaccu gcacgagauc gccagcgggg gcgccccgcu gagcaaggag 1020
gugggcgagg ccguggccaa gcgguuccac cucccgggca uccgccaggg cuacggccug 1080
accgagacca cgagcgcgau ccugaucacc cccgaggggg acgacaagcc gggcgccgug 1140
ggcaaggugg ucccguucuu cgaggccaag guggugggacc uggacaccgg caagacccug 1200
ggcgugaacc agcggggcga gcugugcgug cgggggccga ugaucaugag cggcuacgug 1260
aacaacccgg aggccaccaa cgcccucauc gacaaggacg gcuggcugca cagcggcgac 1320
aucgccuacu gggacgagga cgagcacuuc uucaucgucg accggcugaa gucgcugauc 1380
aaguacaagg gcuaccaggu ggcgccggcc gagcuggaga gcauccugcu ccagcacccc 1440
aacaucuucg acgccggcgu ggccgggcug ccggacgacg acgccggcga gcugccggcc 1500
gcgguggugg ugcuggagca cggcaagacc augacggaga aggagaucgu cgacuacgug 1560
gccagccagg ugaccaccgc caagaagcug cggggcggcg ugguguucgu ggacgagguc 1620
ccgaagggcc ugaccgggaa gcucgacgcc cggaagaucc gcgagauccu gaucaaggcc 1680
aagaagggcg gcaagaucgc cguguaagac uagugaaggg aacacccaaa uuuaauucag 1740
ccuuaagcac aauuaauuaa gagugaaacg uaauuguaca agcaguuggu cacccaccau 1800

agggcaugau caacaccgca accuuuccuu uuuccccag ugauucugaa aaaccccucu 1860

ucccuucagc uugcuuagau guuccaaauu uaguaagcuu aaggcggccu acagaagaaa 1920

aagaaaaaa aggccacaaa aguucccucu cacuuucagu aaauaaaaua aaagcagcaa 1980

cagaaauaaa gaaauaaaug aaauucaaaa ugaaauaaau auuguguugu gcagcauuaa 2040

aaaaucaaua aaaauuaaaa augagcaaga ucuaaaaaaa aaaaaaaaaa aaaaaaaaaa 2100

aaaaaaaaaa aaaaaaaaaa aaaaaaaaaa aaaaaaaugc aucccccccc cccccccccc 2160

cccccccccc cccaaaggcu cuuuucagag ccaccagaau u 2201

<210> 37
<211> 1965
<212> RNA
<213> Artificial Sequence

<220>
<223> 32L4 - PpLuc(GC) - morn2- A64 - C30 - hSL

<400> 37
ggggcgcugc cuacggaggu ggcagccauc uccuucucgg caucaagcuu gaggauggag 60

gacgccaaga acaucaagaa gggcccggcg cccuucuacc cgcuggagga cgggaccgcc 120

ggcgagcagc uccacaaggc caugaagcgg uacgcccugg ugccgggcac gaucgccuuc 180

accgacgccc acaucgaggu cgacaucacc uacgcggagu acuucgagau gagcgugcgc 240

cuggccgagg ccaugaagcg guacggccug aacaccaacc accggaucgu ggugugcucg 300

gagaacagcc ugcaguucuu caugccggug cugggcgccc ucuucaucgg cguggccguc 360

gccccggcga acgacaucua caacgagcgg gagcugcuga acagcauggg gaucagccag 420

ccgaccgugg uguucgugag caagaagggc cugcagaaga uccugaacgu gcagaagaag 480

cugcccauca uccagaagau caucaucaug gacagcaaga ccgacuacca gggcuuccag 540

ucgauguaca cguucgugac cagccaccuc ccgccgggcu ucaacgagua cgacuucguc 600

ccggagagcu ucgaccggga caagaccauc gcccugauca ugaacagcag cggcagcacc 660

ggccugccga agggggugc ccugccgcac cggaccgccu gcgugcgcuu cucgcacgcc 720

cgggacccca ucuucggcaa ccagaucauc ccggacaccg ccauccugag cgguggugccg 780

uuccaccacg gcuucggcau guucacgacc cugggcuacc ucaucugcgg cuuccgggug 840

guccugaugu accgguucga ggaggagcug uuccugcgga gccugcagga cuacaagauc 900

cagagcgcgc ugcucgugcc gacccuguuc agcuucuucg ccaagagcac ccugaucgac 960

aaguacgacc ugucgaaccu gcacgagauc gccagcgggg gcgccccgcu gagcaaggag 1020

gugggcgagg ccguggccaa gcgguuccac cucccgggca uccgccaggg cuacggccug 1080

accgagacca cgagcgcgau ccugaucacc cccgaggggg acgacaagcc gggcgccgug 1140

ggcaaggugg ucccguucuu cgaggccaag gugguggacc uggacaccgg caagacccug 1200

ggcgugaacc agcggggcga gcugugcgug cgggggccga ugaucaugag cggcuacgug 1260

aacaacccgg aggccaccaa cgcccucauc gacaaggacg gcuggcugca cagcggcgac 1320

aucgccuacu gggacgagga cgagcacuuc uucaucgucg accggcugaa gucgcugauc 1380

aaguacaagg gcuaccaggu ggcgccggcc gagcuggaga gcauccugcu ccagcacccc 1440

aacaucuucg acgccggcgu ggccgggcug ccggacgacg acgccggcga gcugccggcc 1500

gcgguggugg ugcuggagca cggcaagacc augacggaga aggagaucgu cgacuacgug 1560

gccagccagg ugaccaccgc caagaagcug cggggcggcg ugguguucgu ggacgagguc 1620

ccgaagggcc ugaccgggaa gcucgacgcc cggaagaucc gcgagauccu gaucaaggcc 1680

aagaagggcg gcaagaucgc cguguaagac uaguaccugc ugccuuaacg cugagaugug 1740

gccucugcaa ccccccuuag gcaaagcaac ugaaccuucu gcuaaaguga ccugcccucu 1800

uccguaaguc caauaaaguu gucaugcacc cagaucuaaa aaaaaaaaaa aaaaaaaaaa 1860

aaaaaaaaaa aaaaaaaaaa aaaaaaaaaa aaaaaaaaaa augcaucccc cccccccccc 1920

cccccccccc cccccccaaa ggcucuuuuc agagccacca gaauu 1965

<210> 38
<211> 2286
<212> RNA
<213> Artificial Sequence

<220>
<223> 32L4 - PpLuc(GC) - gstm1- A64 - C30 - hSL

<400> 38
ggggcgcugc cuacggaggu ggcagccauc uccuucucgg caucaagcuu gaggauggag 60

gacgccaaga acaucaagaa gggcccggcg cccuucuacc cgcuggagga cgggaccgcc 120

ggcgagcagc uccacaaggc caugaagcgg uacgcccugg ugccgggcac gaucgccuuc 180

accgacgccc acaucgaggu cgacaucacc uacgcggagu acuucgagau gagcgugcgc 240

cuggccgagg ccaugaagcg guacggccug aacaccaacc accggaucgu ggugugcucg 300

gagaacagcc ugcaguucuu caugccggug cugggcgccc ucuucaucgg cguggccguc 360

gccccggcga acgacaucua caacgagcgg gagcugcuga acagcauggg gaucagccag 420

ccgaccgugg uguucgugag caagaagggc cugcagaaga uccugaacgu gcagaagaag 480

cugcccauca uccagaagau caucaucaug gacagcaaga ccgacuacca gggcuuccag 540

ucgauguaca cguucgugac cagccaccuc ccgccgggcu ucaacgagua cgacuucguc 600

ccggagagcu ucgaccggga caagaccauc gcccugauca ugaacagcag cggcagcacc 660

ggccugccga agggggugg ccugccgcac cggaccgccu gcgugcgcuu cucgcacgcc 720

cgggacccca ucuucggcaa ccagaucauc ccggacaccg ccauccugag cgguggugccg 780

uuccaccacg gcuucggcau guucacgacc cugggcuacc ucaucugcgg cuuccgggug 840

guccugaugu accgguucga ggaggagcug uuccugcgga gccugcagga cuacaagauc 900

cagagcgcgc ugcucgugcc gacccuguuc agcuucuucg ccaagagcac ccugaucgac 960

aaguacgacc ugucgaaccu gcacgagauc gccagcgggg gcgccccgcu gagcaaggag 1020

gugggcgagg ccguggccaa gcgguuccac cucccgggca uccgccaggg cuacggccug 1080

accgagacca cgagcgcgau ccugaucacc cccgaggggg acgacaagcc gggcgccgug 1140

ggcaaggugg ucccguucuu cgaggccaag gugguggacc uggacaccgg caagacccug 1200

ggcgugaacc agcggggcga gcugugcgug cgggggccga ugaucaugag cggcuacgug 1260

aacaacccgg aggccaccaa cgcccucauc gacaaggacg gcuggcugca cagcggcgac 1320

aucgccuacu gggacgagga cgagcacuuc uucaucgucg accggcugaa gucgcugauc 1380

aaguacaagg gcuaccaggu ggcgccggcc gagcuggaga gcauccugcu ccagcacccc 1440

aacaucuucg acgccggcgu ggccgggcug ccggacgacg acgccggcga gcugccggcc 1500

gcgguggugg ugcuggagca cggcaagacc augacggaga aggagaucgu cgacuacgug 1560

gccagccagg ugaccaccgc caagaagcug cggggcggcg ugguguucgu ggacgagguc 1620

ccgaagggcc ugaccgggaa gcucgacgcc cggaagaucc gcgagauccu gaucaaggcc 1680

aagaagggcg gcaagaucgc cguguaagac uagugcccuu gcuacacggg cacucacuag 1740

gaggaccugu ccacacuggg gauccugcag gcccugggug gggacagcac ccuggccuuc 1800

ugcacugugg cuccugguuc ucucuccuuc ccgcucccuu cugcagcuug gucagcccca 1860

ucuccucacc cucuucccag ucaaguccac acagccuuca uucuccccag uuucuuucac 1920

auggccccuu cuucauuggc ucccugaccc aaccucacag cccguuucug cgaacugagg 1980

ucuguccuga acucacgcuu ccuagaauua ccccgauggu caacacuauc uuagugcuag 2040

cccucccuag aguuaccccg aaggucaaua cuugagugcc agccuguucc ugguggagua 2100

gccuccccag gucugucucg ucuacaauaa agucugaaac acacuugcca ugagaucuaa 2160

aaaaaaaaaa aaaaaaaaaa aaaaaaaaaa aaaaaaaaaa aaaaaaaaaa aaaaaaaaaa 2220

aaugcauccc cccccccccc cccccccccc cccccccaa aggcucuuuu cagagccacc 2280

agaauu 2286

<210> 39
<211> 1981
<212> RNA
<213> Artificial Sequence

<220>
<223> 32L4 - PpLuc(GC) - ndufa1 - A64 - C30 - hSL

<400> 39

```
ggggcgcugc cuacggaggu ggcagccauc uccuucucgg caucaagcuu gaggauggag      60
gacgccaaga acaucaagaa gggcccggcg cccuucuacc cgcuggagga cgggaccgcc     120
ggcgagcagc uccacaaggc caugaagcgg uacgcccugg ugccgggcac gaucgccuuc     180
accgacgccc acaucgaggu cgacaucacc uacgcggagu acuucgagau gagcgugcgc     240
cuggccgagg ccaugaagcg guacggccug aacaccaacc accggaucgu ggugugcucg     300
gagaacagcc ugcaguucuu caugccggug cugggcgccc ucuucaucgg cguggccguc     360
gccccggcga acgacaucua caacgagcgg gagcugcuga acagcauggg gaucagccag     420
ccgaccgugg uguucgugag caagaagggc cugcagaaga uccugaacgu gcagaagaag     480
cugcccauca uccagaagau caucaucaug gacagcaaga ccgacuacca gggcuuccag     540
ucgauguaca cguucgugac cagccaccuc ccgccgggcu ucaacgagua cgacuucguc     600
ccggagagcu ucgaccggga caagaccauc gcccugauca ugaacagcag cggcagcacc     660
ggccugccga agggggugc ccugccgcac cggaccgccu gcgugcgcuu cucgcacgcc      720
cgggacccca ucuucggcaa ccagaucauc ccggacaccg ccauccugag cgugguggcg     780
uuccaccacg gcuucggcau guucacgacc cugggcuacc ucaucugcgg cuuccgggug     840
guccugaugu accgguucga ggaggagcug uuccugcgga gccugcagga cuacaagauc     900
cagagcgcgc ugcucgugcc gacccuguuc agcuucuucg ccaagagcac ccugaucgac     960
aaguacgacc ugucgaaccu gcacgagauc gccagcgggg gcgccccgcu gagcaaggag    1020
gugggcgagg ccguggccaa gcgguuccac cucccgggca uccgccaggg cuacggccug    1080
accgagacca cgagcgcgau ccugaucacc cccgaggggg acgacaagcc gggcgccgug    1140
ggcaaggugg ucccguucuu cgaggccaag guggugggacc uggacaccgg caagacccug   1200
ggcgugaacc agcggggcga gcugugcgug cgggggccga ugaucaugag cggcuacgug    1260
aacaacccgg aggccaccaa cgcccucauc gacaaggacg gcuggcugca cagcggcgac    1320
aucgccuacu gggacgagga cgagcacuuc uucaucgucg accggcugaa gucgcugauc    1380
aaguacaagg gcuaccaggu ggcgccggcc gagcuggaga gcauccugcu ccagcacccc    1440
aacaucuucg acgccggcgu ggccgggcug ccggacgacg acgccggcga gcugccggcc    1500
gcgguggugg ugcuggagca cggcaagacc augacggaga aggagaucgu cgacuacgug    1560
gccagccagg ugaccaccgc caagaagcug cggggcggcg ugguguucgu ggacgagguc    1620
ccgaagggcc ugaccgggaa gcucgacgcc cggaagaucc gcgagauccu gaucaaggcc    1680
aagaagggcg gcaagaucgc cguguaagac uaguggaagc auuuuccugg cugauuaaaa    1740
gaaauuacuc agcuaugguc aucuguuccu guuagaaggc uaugcagcau auuauauacu    1800
augcgcaugu uaugaaaugc auaauaaaaa auuuuaaaaa aucuaaaaga ucuaaaaaaa    1860
aaaaaaaaaa aaaaaaaaaa aaaaaaaaaa aaaaaaaaaa aaaaaaaaaa aaaaaaaugc    1920
```

aucccccccc cccccccccc cccccccccc cccaaaggcu cuuuucagag ccaccagaau 1980

u 1981

<210> 40
<211> 2085
<212> RNA
<213> Artificial Sequence

<220>
<223> 32L4 - PpLuc(GC) - cbr2 - A64 - C30 - hSL

<400> 40
ggggcgcugc cuacggaggu ggcagccauc uccuucucgg caucaagcuu gaggauggag 60

gacgccaaga acaucaagaa gggcccggcg cccuucuacc cgcuggagga cgggaccgcc 120

ggcgagcagc uccacaaggc caugaagcgg uacgcccugg ugccgggcac gaucgccuuc 180

accgacgccc acaucgaggu cgacaucacc uacgcggagu acuucgagau gagcgugcgc 240

cuggccgagg ccaugaagcg guacggccug aacaccaacc accggaucgu ggugugcucg 300

gagaacagcc ugcaguucuu caugccggug cugggcgccc ucuucaucgg cguggccguc 360

gccccggcga acgacaucua caacgagcgg gagcugcuga acagcauggg gaucagccag 420

ccgaccgugg uguucgugag caagaagggc cugcagaaga uccugaacgu gcagaagaag 480

cugcccauca uccagaagau caucaucaug gacagcaaga ccgacuacca gggcuuccag 540

ucgauguaca cguucgugac cagccaccuc ccgccgggcu ucaacgagua cgacuucguc 600

ccggagagcu ucgaccggga caagaccauc gcccugauca ugaacagcag cggcagcacc 660

ggccugccga agggggugge ccugccgcac cggaccgccu gcgugcgcuu cucgcacgcc 720

cgggacccca ucuucggcaa ccagaucauc ccggacaccg ccauccugag cguggugccg 780

uuccaccacg gcuucggcau guucacgacc cugggcuacc ucaucugcgg cuuccgggug 840

guccugaugu accgguucga ggaggagcug uuccugcgga gccugcagga cuacaagauc 900

cagagcgcgc ugcucgugcc gacccuguuc agcuucuucg ccaagagcac ccugaucgac 960

aaguacgacc ugucgaaccu gcacgagauc gccagcgggg gcgccccgcu gagcaaggag 1020

gugggcgagg ccguggccaa gcgguuccac cucccgggca uccgccaggg cuacggccug 1080

accgagacca cgagcgcgau ccugaucacc cccgaggggg acgacaagcc gggcgccgug 1140

ggcaaggugg ucccguucuu cgaggccaag gugguggacc uggacaccgg caagacccug 1200

ggcgugaacc agcgggggcga gcugugcgug cgggggccga ugaucaugag cggcuacgug 1260

aacaacccgg aggccaccaa cgcccucauc gacaaggacg gcuggcugca cagcggcgac 1320

aucgccuacu gggacgagga cgagcacuuc uucaucgucg accggcugaa gucgcugauc 1380

aaguacaagg gcuaccaggu ggcgccggcc gagcuggaga gcauccugcu ccagcacccc 1440

aacaucuucg acgccggcgu ggccgggcug ccggacgacg acgccggcga gcugccggcc 1500

gcgguggugg ugcuggagca cggcaagacc augacggaga aggagaucgu cgacuacgug 1560

gccagccagg ugaccaccgc caagaagcug cggggcggcg ugguguucgu ggacgagguc 1620

ccgaagggcc ugaccgggaa gcucgacgcc cggaagaucc gcgagauccu gaucaaggcc 1680

aagaagggcg gcaagaucgc cguguaagac uaguucugcu caguugccgc ggacaucuga 1740

guggccuucu uagccccacc cucagccaaa gcauuuacug aucucgugac uccgcccuca 1800

ugcuacagcc acgcccacca cgcagcucac aguuccaccc ccauguuacu gucgauccca 1860

caaccacucc aggcgcagac cuuguucucu uuguccacuu uguugggcuc auuugccuaa 1920

auaaacgggc caccgcguua ccuuuaacua uagaucuaaa aaaaaaaaaa aaaaaaaaaa 1980

aaaaaaaaaa aaaaaaaaaa aaaaaaaaaa aaaaaaaaaa augcaucccc cccccccccc 2040

cccccccccc cccccccaaa ggcucuuuuc agagccacca gaauu 2085

<210> 41
<211> 1997
<212> RNA
<213> Artificial Sequence

<220>
<223> PpLuc(GC) - albumin7- A64 - C30 - hSL

<400> 41
gggagaaagc uugaggaugg aggacgccaa gaacaucaag aagggcccgg cgcccuucua 60

cccgcuggag gacgggaccg ccggcgagca gcuccacaag gccaugaagc gguacgcccu 120

ggugccgggc acgaucgccu ucaccgacgc ccacaucgag gucgacauca ccuacgcgga 180

guacuucgag augagcgugc gccuggccga ggccaugaag cgguacggcc ugaacaccaa 240

ccaccggauc gugguguccu cggagaacag ccugcaguuc uucaugccgg ugcugggcgc 300

ccucuucauc ggcguggccg ucgccccggc gaacgacauc uacaacgagc gggagcugcu 360

gaacagcaug ggaucagcc agccgaccgu gguguucgug agcaagaagg gccugcagaa 420

gauccugaac gugcagaaga agcugcccau cauccagaag aucaucauca uggacagcaa 480

gaccgacuac cagggcuucc agucgaugua cacguucgug accagccacc ucccgccggg 540

cuucaacgag uacgacuucg ucccggagag cuucgaccgg gacaagacca ucgcccugau 600

caugaacagc agcggcagca ccggccugcc gaagggggug gcccugccgc accggaccgc 660

cugcgugcgc uucucgcacg cccgggaccc caucuucggc aaccagauca ucccggacac 720

cgccauccug agcguggugc cguuccacca cggcuucggc auguucacga cccugggcua 780

ccucaucugc ggcuuccggg ugguccugau guaccgguuc gaggaggagc uguuccugcg 840

gagccugcag gacuacaaga uccagagcgc gcugcucgug ccgacccugu ucagcuucuu 900

cgccaagagc acccugaucg acaaguacga ccugucgaac cugcacgaga ucgccagcgg 960

gggcgccccg cugagcaagg aggugggcga ggccguggcc aagcgguucc accucccggg 1020

cauccgccag ggcuacggcc ugaccgagac cacgagcgcg auccugauca cccccgaggg 1080

ggacgacaag ccgggcgccg ugggcaaggu ggucccguuc uucgaggcca agguggugga 1140

ccuggacacc ggcaagaccc ugggcgugaa ccagcggggc gagcugugcg ugcgggggcc 1200

gaugaucaug agcggcuacg ugaacaaccc ggaggccacc aacgcccuca ucgacaagga 1260

cggcuggcug cacagcggcg acaucgccua cugggacgag gacgagcacu ucuucaucgu 1320

cgaccggcug aagucgcuga ucaaguacaa gggcuaccag guggcgccgg ccgagcugga 1380

gagcauccug cuccagcacc ccaacaucuu cgacgccggc guggccgggc ugccggacga 1440

cgacgccggc gagcugccgg ccgcgguggu ggugcuggag cacggcaaga ccaugacgga 1500

gaaggagauc gucgacuacg uggccagcca ggugaccacc gccaagaagc ugcggggcgg 1560

cgugguguuc guggacgagg ucccgaaggg ccugaccggg aagcucgacg cccggaagau 1620

ccgcgagauc cugaucaagg ccaagaaggg cggcaagauc gccguguaag acuagugcau 1680

cacauuuaaa agcaucucag ccuaccauga gaauaagaga aagaaaauga agaucaauag 1740

cuuauucauc ucuuuuucuu uuucguuggu guaaagccaa cacccugucu aaaaaacaua 1800

aauuucuuua aucauuuugc cucuuuucuc ugugcuucaa uuaauaaaaa auggaaagaa 1860

ccuagaucua aaaaaaaaaa aaaaaaaaaa aaaaaaaaaa aaaaaaaaaa aaaaaaaaaa 1920

aaaaaaaaaa aaaugcaucc cccccccccc cccccccccc ccccccccca aaggcucuuu 1980

ucagagccac cagaauu 1997

<210> 42
<211> 2048
<212> RNA
<213> Artificial Sequence

<220>
<223> Mp68 - PpLuc(GC) - albumin7- A64 - C30 - hSL

<400> 42
gggcuuuccc auucuguagc agaauuuggu guugccugug gucuuggucc cgcggagaag 60

cuugaggaug gaggacgcca agaacaucaa gaagggcccg gcgcccuucu acccgcugga 120

ggacgggacc gccggcgagc agcuccacaa ggccaugaag cgguacgccc uggugccggg 180

cacgaucgcc uucaccgacg cccacaucga ggucgacauc accuacgcgg aguacuucga 240

gaugagcgug cgccuggccg aggccaugaa gcgguacggc cugaacacca accaccggau 300

cgugguguge ucggagaaca gccugcaguu cuucaugccg gugcugggcg cccucuucau 360

cggcguggcc gucgccccgg cgaacgacau cuacaacgag cgggagcugc ugaacagcau 420

ggggaucagc cagccgaccg ugguguucgu gagcaagaag ggccugcaga agauccugaa 480

cgugcagaag aagcugccca ucauccagaa gaucaucauc auggacagca agaccgacua 540

ccagggcuuc cagucgaugu acacguucgu gaccagccac cucccgccgg gcuucaacga 600

guacgacuuc gucccggaga gcuucgaccg ggacaagacc aucgcccuga ucaugaacag 660

cagcggcagc accggccugc cgaagggggu ggcccugccg caccggaccg ccugcgugcg 720

cuucucgcac gcccgggacc ccaucuucgg caaccagauc aucccggaca ccgccauccu 780

gagcguggug ccguuccacc acggcuucgg cauguucacg acccugggcu accucaucug 840

cggcuuccgg gugguccuga uguaccgguu cgaggaggag cuguuccugc ggagccugca 900

ggacuacaag auccagagcg cgcugcucgu gccgacccug uucagcuucu ucgccaagag 960

cacccugauc gacaaguacg accugucgaa ccugcacgag aucgccagcg ggggcgcccc 1020

gcugagcaag gaggugggcg aggccguggc caagcgguuc caccucccgg gcauccgcca 1080

gggcuacggc cugaccgaga ccacgagcgc gauccugauc acccccgagg gggacgacaa 1140

gccgggcgcc gugggcaagg ugguccguu cuucgaggcc aagguggugg accuggacac 1200

cggcaagacc cugggcguga accagcgggg cgagcugugc gugcgggggc cgaugaucau 1260

gagcggcuac gugaacaacc cggaggccac caacgcccuc aucgacaagg acggcuggcu 1320

gcacagcggc gacaucgccu acugggacga ggacgagcac uucuucaucg ucgaccggcu 1380

gaagucgcug aucaaguaca agggcuacca gguggcgccg gccgagcugg agagcauccu 1440

gcuccagcac cccaacaucu ucgacgccgg cguggccggg cugccggacg acgacgccgg 1500

cgagcugccg gccgcggugg uggugcugga gcacggcaag accaugacgg agaaggagau 1560

cgucgacuac guggccagcc aggugaccac cgccaagaag cugcggggcg gcgugguguu 1620

cguggacgag gucccgaagg gccugaccgg gaagcucgac gcccggaaga uccgcgagau 1680

ccugaucaag gccaagaagg gcggcaagau cgccguguaa gacuagugca ucacauuuaa 1740

aagcaucuca gccuaccaug agaauaagag aaagaaaaug aagaucaaua gcuuauucau 1800

cucuuuuucu uuuucguugg uguaaagcca acacccuguc uaaaaaacau aaauuucuuu 1860

aaucauuuug ccucuuuucu cugugcuuca auuaauaaaa aauggaaaga accuagaucu 1920

aaaaaaaaaa aaaaaaaaaa aaaaaaaaaa aaaaaaaaaa aaaaaaaaaa aaaaaaaaaa 1980

aaaaugcauc cccccccccc cccccccccc cccccccccc aaaggcucuu uucagagcca 2040

ccagaauu 2048

<210> 43
<211> 2075
<212> RNA
<213> Artificial Sequence

<220>
<223> Ndufa4 - PpLuc(GC) - albumin7- A64 - C30 - hSL

<400> 43
ggggguccgcu cagccagguu gcagaagcgg cuuagcgugu guccuaaucu ucucucugcg 60

uguagguagg ccugugccgc aaacaagcuu gaggauggag gacgccaaga acaucaagaa 120

gggcccggcg cccuucuacc cgcuggagga cgggaccgcc ggcgagcagc uccacaaggc 180

caugaagcgg uacgcccugg ugccgggcac gaucgccuuc accgacgccc acaucgaggu 240

cgacaucacc uacgcggagu acuucgagau gagcgugcgc cuggccgagg ccaugaagcg 300

guacggccug aacaccaacc accggaucgu ggugugcucg gagaacagcc ugcaguucuu 360

caugccggug cugggcgccc ucuucaucgg cguggccguc gccccggcga acgacaucua 420

caacgagcgg gagcugcuga acagcauggg gaucagccag ccgaccgugg uguucgugag 480

caagaagggc cugcagaaga uccugaacgu gcagaagaag cugcccauca uccagaagau 540

caucaucaug gacagcaaga ccgacuacca gggcuuccag ucgauguaca cguucgugac 600

cagccaccuc ccgccgggcu ucaacgagua cgacuucguc ccggagagcu ucgaccggga 660

caagaccauc gcccugauca ugaacagcag cggcagcacc ggccugccga agggggugc 720

ccugccgcac cggaccgccu gcgugcgcuu cucgcacgcc cgggacccca ucuucggcaa 780

ccagaucauc ccggacaccg ccauccugag cgguggugccg uuccaccacg gcuucggcau 840

guucacgacc cugggcuacc ucaucugcgg cuuccgggug guccugaugu accgguucga 900

ggaggagcug uuccugcgga gccugcagga cuacaagauc cagagcgcgc ugcucgugcc 960

gacccuguuc agcuucuucg ccaagagcac ccugaucgac aaguacgacc ugucgaaccu 1020

gcacgagauc gccagcgggg gcgccccgcu gagcaaggag gugggcgagg ccguggccaa 1080

gcgguuccac cucccgggca uccgccaggg cuacggccug accgagacca cgagcgcgau 1140

ccugaucacc cccgaggggg acgacaagcc gggcgccgug ggcaaggugg ucccguucuu 1200

cgaggccaag guggugggacc uggacaccgg caagacccug ggcgugaacc agcggggcga 1260

gcugugcgug cgggggccga ugaucaugag cggcuacgug aacaacccgg aggccaccaa 1320

cgcccucauc gacaaggacg gcuggcugca cagcggcgac aucgccuacu gggacgagga 1380

cgagcacuuc uucaucgucg accggcugaa gucgcugauc aaguacaagg gcuaccaggu 1440

ggcgccggcc gagcuggaga gcauccugcu ccagcacccc aacaucuucg acgccggcgu 1500

ggccgggcug ccggacgacg acgccggcga gcugccggcc gcggugugg ugcuggagca 1560

cggcaagacc augacggaga aggagaucgu cgacuacgug gccagccagg ugaccaccgc 1620

caagaagcug cggggcggcg ugguguucgu ggacgagguc ccgaagggcc ugaccgggaa 1680

gcucgacgcc cggaagaucc gcgagauccu gaucaaggcc aagaagggcg gcaagaucgc 1740

cguguaagac uagugcauca cauuuaaaag caucucagcc uaccaugaga auaagagaaa 1800

gaaaaugaag aucaauagcu uauucaucuc uuuuucuuuu ucguuggugu aaagccaaca 1860

cccugucuaa aaaacauaaa uuucuuuaau cauuuugccu cuuuucucug ugcuucaauu 1920

aauaaaaaau ggaaagaacc uagaucuaaa aaaaaaaaaa aaaaaaaaaa aaaaaaaaaa 1980

aaaaaaaaaa aaaaaaaaaa aaaaaaaaaa augcaucccc cccccccccc cccccccccc 2040

cccccccaaa ggcucuuuuc agagccacca gaauu 2075

<210> 44
<211> 1810
<212> DNA
<213> Artificial Sequence

<220>
<223> PpLuc(GC) - A64 - C30 - hSL

<400> 44
gggagaaagc ttgaggatgg aggacgccaa gaacatcaag aagggcccgg cgcccttcta 60

cccgctggag gacgggaccg ccggcgagca gctccacaag gccatgaagc ggtacgccct 120

ggtgccgggc acgatcgcct tcaccgacgc ccacatcgag gtcgacatca cctacgcgga 180

gtacttcgag atgagcgtgc gcctggccga ggccatgaag cggtacggcc tgaacaccaa 240

ccaccggatc gtggtgtgct cggagaacag cctgcagttc ttcatgccgg tgctgggcgc 300

cctcttcatc ggcgtggccg tcgccccggc gaacgacatc tacaacgagc gggagctgct 360

gaacagcatg gggatcagcc agccgaccgt ggtgttcgtg agcaagaagg gcctgcagaa 420

gatcctgaac gtgcagaaga agctgcccat catccagaag atcatcatca tggacagcaa 480

gaccgactac cagggcttcc agtcgatgta cacgttcgtg accagccacc tcccgccggg 540

cttcaacgag tacgacttcg tcccggagag cttcgaccgg gacaagacca tcgccctgat 600

catgaacagc agcggcagca ccggcctgcc gaagggggtg gccctgccgc accggaccgc 660

ctgcgtgcgc ttctcgcacg cccgggaccc catcttcggc aaccagatca tcccggacac 720

cgccatcctg agcgtggtgc cgttccacca cggcttcggc atgttcacga ccctgggcta 780

cctcatctgc ggcttccggg tggtcctgat gtaccggttc gaggaggagc tgttcctgcg 840

gagcctgcag gactacaaga tccagagcgc gctgctcgtg ccgaccctgt tcagcttctt 900

cgccaagagc accctgatcg acaagtacga cctgtcgaac ctgcacgaga tcgccagcgg 960

gggcgccccg ctgagcaagg aggtgggcga ggccgtggcc aagcggttcc acctcccggg 1020

catccgccag ggctacggcc tgaccgagac cacgagcgcg atcctgatca cccccgaggg 1080

ggacgacaag ccgggcgccg tgggcaaggt ggtcccgttc ttcgaggcca aggtggtgga 1140

cctggacacc ggcaagaccc tgggcgtgaa ccagcggggc gagctgtgcg tgcgggggcc 1200

gatgatcatg agcggctacg tgaacaaccc ggaggccacc aacgccctca tcgacaagga 1260

cggctggctg cacagcggcg acatcgccta ctgggacgag gacgagcact tcttcatcgt 1320

cgaccggctg aagtcgctga tcaagtacaa gggctaccag gtggcgccgg ccgagctgga 1380

gagcatcctg ctccagcacc ccaacatctt cgacgccggc gtggccgggc tgccggacga 1440

cgacgccggc gagctgccgg ccgcggtggt ggtgctggag cacggcaaga ccatgacgga 1500

gaaggagatc gtcgactacg tggccagcca ggtgaccacc gccaagaagc tgcggggcgg 1560

cgtggtgttc gtggacgagg tcccgaaggg cctgaccggg aagctcgacg cccggaagat 1620

ccgcgagatc ctgatcaagg ccaagaaggg cggcaagatc gccgtgtaag actagtagat 1680

ctaaaaaaaa aaaaaaaaaa aaaaaaaaaa aaaaaaaaaa aaaaaaaaaa aaaaaaaaaa 1740

aaaaaatgca tccccccccc cccccccccc cccccccccc ccaaaggctc ttttcagagc 1800

caccagaatt 1810

<210> 45
<211> 2163
<212> RNA
<213> Artificial Sequence

<220>
<223> PpLuc(GC) - gnas- A64 - C30 - hSL

<400> 45
gggagaaagc uugaggaugg aggacgccaa gaacaucaag aagggcccgg cgcccuucua 60

cccgcuggag gacgggaccg ccggcgagca gcuccacaag gccaugaagc gguacgcccu 120

ggugccgggc acgaucgccu ucaccgacgc ccacaucgag gucgacauca ccuacgcgga 180

guacuucgag augagcgugc gccuggccga ggccaugaag cgguacggcc ugaacaccaa 240

ccaccggauc gugguguguu cggagaacag ccugcaguuc uucaugccgg ugcugggcgc 300

ccucuucauc ggcguggccg ucgccccggc gaacgacauc uacaacgagc gggagcugcu 360

gaacagcaug ggaucagcc agccgaccgu ggguguucgug agcaagaagg gccugcagaa 420

gauccugaac gugcagaaga agcugcccau cauccagaag aucaucauca uggacagcaa 480

gaccgacuac cagggcuucc agucgaugua cacguucgug accagccacc ucccgccggg 540

cuucaacgag uacgacuucg ucccggagag cuucgaccgg gacaagacca ucgcccugau 600

caugaacagc agcggcagca ccggccugcc gaaggggggug gcccugccgc accggaccgc 660

cugcgugcgc uucucgcacg cccgggaccc caucuucggc aaccagauca ucccggacac 720

cgccauccug agcguggugc cguuccacca cggcuucggc auguucacga cccugggcua 780

ccucaucugc ggcuuccggg ugguccugau guaccgguuc gaggaggagc uguuccugcg 840

gagccugcag gacuacaaga uccagagcgc gcugcucgug ccgacccugu ucagcuucuu 900

cgccaagagc acccugaucg acaaguacga ccugucgaac cugcacgaga ucgccagcgg 960

gggcgccccg cugagcaagg aggugggcga ggccguggcc aagcgguucc accucccggg 1020

cauccgccag ggcuacggcc ugaccgagac cacgagcgcg auccugauca cccccgaggg 1080

ggacgacaag ccgggcgccg ugggcaaggu ggucccguuc uucgaggcca agguggugga 1140

ccuggacacc ggcaagaccc ugggcgugaa ccagcggggc gagcugugcg ugcgggggcc 1200

gaugaucaug agcggcuacg ugaacaaccc ggaggccacc aacgcccuca ucgacaagga 1260

cggcuggcug cacagcggcg acaucgccua cugggacgag gacgagcacu ucuucaucgu 1320

cgaccggcug aagucgcuga ucaaguacaa gggcuaccag guggcgccgg ccgagcugga 1380

gagcauccug cuccagcacc ccaacaucuu cgacgccggc guggccgggc ugccggacga 1440

cgacgccggc gagcugccgg ccgcggugu ggugcuggag cacggcaaga ccaugacgga 1500

gaaggagauc gucgacuacg uggccagcca ggugaccacc gccaagaagc ugcggggcgg 1560

cgugguguuc guggacgagg ucccgaaggg ccugaccggg aagcucgacg cccggaagau 1620

ccgcgagauc cugaucaagg ccaagaaggg cggcaagauc gccguguaag acuagugaag 1680

ggaacaccca aauuuaauuc agccuuaagc acaauuaauu aagagugaaa cguaauugua 1740

caagcaguug gucacccacc auagggcaug aucaacaccg caaccuuucc uuuuuccccc 1800

agugauucug aaaaaccccu cuucccuuca gcuugcuuag auguuccaaa uuuaguaagc 1860

uuaaggcggc cuacagaaga aaaagaaaaa aaaggccaca aaaguucccu cucacuuuca 1920

guaaauaaaa uaaaagcagc aacagaaaua aagaaauaaa ugaaauucaa aaugaaauaa 1980

auauuguguu gugcagcauu aaaaaaucaa uaaaaauuaa aaaugagcaa gaucuaaaaa 2040

aaaaaaaaaa aaaaaaaaaa aaaaaaaaaa aaaaaaaaaa aaaaaaaaaa aaaaaaaaau 2100

gcaucccccc cccccccccc cccccccccc cccccaaagg cucuuuucag agccaccaga 2160

auu 2163

<210> 46
<211> 2264
<212> RNA
<213> Artificial Sequence

<220>
<223> 32L4 - PpLuc(GC) - Ybx1(V2)-A64-C30-hSL

<400> 46
ggggcgcugc cuacggaggu ggcagccauc uccuucucgg caucaagcuu gaggauggag 60

gacgccaaga acaucaagaa gggcccggcg cccuucuacc cgcuggagga cgggaccgcc 120

ggcgagcagc uccacaaggc caugaagcgg uacgcccugg ugccgggcac gaucgccuuc 180

accgacgccc acaucgaggu cgacaucacc uacgcggagu acuucgagau gagcgugcgc 240

cuggccgagg ccaugaagcg guacggccug aacaccaacc accggaucgu ggugugcucg 300

gagaacagcc ugcaguucuu caugccggug cugggcgccc ucuucaucgg cguggccguc 360

gccccggcga acgacaucua caacgagcgg gagcugcuga acagcauggg gaucagccag 420

ccgaccgugg uguucgugag caagaagggc cugcagaaga uccugaacgu gcagaagaag 480

cugcccauca uccagaagau caucaucaug gacagcaaga ccgacuacca gggcuuccag 540
ucgauguaca cguucgugac cagccaccuc ccgccgggcu ucaacgagua cgacuucguc 600
ccggagagcu ucgaccggga caagaccauc gcccugauca ugaacagcag cggcagcacc 660
ggccugccga agggggugge ccugccgcac cggaccgccu gcgugcgcuu cucgcacgcc 720
cgggacccca ucuucggcaa ccagaucauc ccggacaccg ccauccugag cguggugccg 780
uuccaccacg gcuucggcau guucacgacc cugggcuacc ucaucugcgg cuuccgggug 840
guccugaugu accgguucga ggaggagcug uuccugcgga gccugcagga cuacaagauc 900
cagagcgcgc ugcucgugcc gacccuguuc agcuucuucg ccaagagcac ccugaucgac 960
aaguacgacc ugucgaaccu gcacgagauc gccagcgggg gcgccccgcu gagcaaggag 1020
gugggcgagg ccguggccaa gcgguuccac cucccgggca uccgccaggg cuacggccug 1080
accgagacca cgagcgcgau ccugaucacc cccgaggggg acgacaagcc gggcgccgug 1140
ggcaaggugg ucccguucuu cgaggccaag guggugggacc uggacaccgg caagacccug 1200
ggcgugaacc agcggggcga gcugugcgug cgggggccga ugaucaugag cggcuacgug 1260
aacaacccgg aggccaccaa cgcccucauc gacaaggacg gcuggcugca cagcggcgac 1320
aucgccuacu gggacgagga cgagcacuuc uucaucgucg accggcugaa gucgcugauc 1380
aaguacaagg gcuaccaggu ggcgccggcc gagcuggaga gcauccugcu ccagcacccc 1440
aacaucuucg acgccggcgu ggccgggcug ccggacgacg acgccggcga gcugccggcc 1500
gcggugguggg ugcuggagca cggcaagacc augacggaga aggagaucgu cgacuacgug 1560
gccagccagg ugaccaccgc caagaagcug cggggcggcg uggguguucgu ggacgagguc 1620
ccgaagggcc ugaccgggaa gcucgacgcc cggaagaucc gcgagauccu gaucaaggcc 1680
aagaagggcg gcaagaucgc cguguaagac uaguaugccg gcuuaccauc ucuaccauca 1740
uccgguuugg ucauccaaca agaagaaaug aauaugaaau uccagcaaua agaaaugaac 1800
aaagauugga gcugaagacc uuaagugcuu gcuuuuugcc cgcugaccag auaacauuag 1860
aacuaucugc auuaucuaug cagcaugggg uuuuuauuau uuuuaccuaa agaugucucu 1920
uuuugguaau gacaaacgug uuuuuuaaga aaaaaaaaa aggccugguu uuucucaaua 1980
caccuuuaac gguuuuuaaa uuguuucaua ucuggucaag uugagauuuu uaagaacuuc 2040
auuuuuaauu uguaauaaag uuuacaacuu gauuuuuuca aaaaagucaa caaacugcaa 2100
gcaccuguua auaaaggucu uaaauaauaa agaucuaaaa aaaaaaaaaa aaaaaaaaaa 2160
aaaaaaaaaa aaaaaaaaaa aaaaaaaaaa aaaaaaaaaa ugcauccccc cccccccccc 2220
cccccccccc ccccccaaag gcucuuuuca gagccaccag aauu 2264

<210> 47

<211> 1914
<212> RNA
<213> Artificial Sequence

<220>
<223> 32L4 - PpLuc(GC) - Ndufb8-A64-C30-hSL

<400> 47
gggcgcugc cuacggaggu ggcagccauc uccuucucgg caucaagcuu gaggauggag 60

gacgccaaga acaucaagaa gggcccggcg cccuucuacc cgcuggagga cgggaccgcc 120

ggcgagcagc uccacaaggc caugaagcgg uacgcccugg ugccgggcac gaucgccuuc 180

accgacgccc acaucgaggu cgacaucacc uacgcggagu acuucgagau gagcgugcgc 240

cuggccgagg ccaugaagcg guacggccug aacaccaacc accggaucgu ggugugcucg 300

gagaacagcc ugcaguucuu caugccggug cugggcgccc ucuucaucgg cguggccguc 360

gccccggcga acgacaucua caacgagcgg gagcugcuga acagcauggg gaucagccag 420

ccgaccgugg uguucgugag caagaagggc cugcagaaga uccugaacgu gcagaagaag 480

cugcccauca uccagaagau caucaucaug gacagcaaga ccgacuacca gggcuuccag 540

ucgauguaca cguucgugac cagccaccuc ccgccgggcu ucaacgagua cgacuucguc 600

ccggagagcu ucgaccggga caagaccauc gcccugauca ugaacagcag cggcagcacc 660

ggccugccga agggggugc ccugccgcac cggaccgccu gcgugcgcuu cucgcacgcc 720

cgggacccca ucuucggcaa ccagaucauc ccggacaccg ccauccugag cgguggugccg 780

uuccaccacg gcuucggcau guucacgacc cugggcuacc ucaucugcgg cuuccgggug 840

guccugaugu accgguucga ggaggagcug uuccugcgga gccugcagga cuacaagauc 900

cagagcgcgc ugcucgugcc gacccuguuc agcuucuucg ccaagagcac ccugaucgac 960

aaguacgacc ugucgaaccu gcacgagauc gccagcgggg gcgccccgcu gagcaaggag 1020

gugggcgagg ccguggccaa gcgguuccac cucccgggca uccgccaggg cuacggccug 1080

accgagacca cgagcgcgau ccugaucacc cccgaggggg acgacaagcc gggcgccgug 1140

ggcaaggugg ucccguucuu cgaggccaag gugguggacc uggacaccgg caagacccug 1200

ggcgugaacc agcggggcga gcugugcgug cgggggccga ugaucaugag cggcuacgug 1260

aacaacccgg aggccaccaa cgcccucauc gacaaggacg gcuggcugca cagcggcgac 1320

aucgccuacu gggacgagga cgagcacuuc uucaucgucg accggcugaa gucgcugauc 1380

aaguacaagg gcuaccaggu ggcgccggcc gagcuggaga gcauccugcu ccagcacccc 1440

aacaucuucg acgccggcgu ggccgggcug ccggacgacg acgccggcga gcugccggcc 1500

gcgguggugg ugcuggagca cggcaagacc augacggaga aggagaucgu cgacuacgug 1560

gccagccagg ugaccaccgc caagaagcug cggggcggcg ugguguucgu ggacgagguc 1620

ccgaagggcc ugaccgggaa gcucgacgcc cggaagaucc gcgagauccu gaucaaggcc 1680

aagaagggcg gcaagaucgc cguguaagac uaguggaggc uugaugggcu uuuugcccuc 1740

guuccuagag gcuuaaccau aauaaaaucc cuaauaaagc agaucuaaaa aaaaaaaaaa 1800

aaaaaaaaaa aaaaaaaaaa aaaaaaaaaa aaaaaaaaaa aaaaaaaaaa ugcauccccc 1860

cccccccccc cccccccccc cccccaaag gcucuuuuca gagccaccag aauu 1914

<210> 48
<211> 2771
<212> RNA
<213> Artificial Sequence

<220>
<223> 32L4 - PpLuc(GC) - Cntn1-004(V2)-A64-C30-hSL

<400> 48
ggggcgcugc cuacggaggu ggcagccauc uccuucucgg caucaagcuu gaggauggag 60

gacgccaaga acaucaagaa gggcccggcg cccuucuacc cgcuggagga cgggaccgcc 120

ggcgagcagc uccacaaggc caugaagcgg uacgcccugg ugccgggcac gaucgccuuc 180

accgacgccc acaucgaggu cgacaucacc uacgcggagu acuucgagau gagcgugcgc 240

cuggccgagg ccaugaagcg guacggccug aacaccaacc accggaucgu ggugugcucg 300

gagaacagcc ugcaguucuu caugccggug cugggcgccc ucuucaucgg cguggccguc 360

gccccggcga acgacaucua caacgagcgg gagcugcuga acagcauggg gaucagccag 420

ccgaccgugg uguucgugag caagaagggc cugcagaaga uccugaacgu gcagaagaag 480

cugcccauca uccagaagau caucaucaug gacagcaaga ccgacuacca gggcuuccag 540

ucgauguaca cguucgugac cagccaccuc ccgccgggcu ucaacgagua cgacuucguc 600

ccggagagcu ucgaccggga caagaccauc gcccugauca ugaacagcag cggcagcacc 660

ggccugccga agggggguggc ccugccgcac cggaccgccu gcgugcgcuu cucgcacgcc 720

cgggacccca ucuucggcaa ccagaucauc ccggacaccg ccauccugag cguggugccg 780

uuccaccacg gcuucggcau guucacgacc cugggcuacc ucaucugcgg cuuccgggug 840

guccugaugu accgguucga ggaggagcug uuccugcgga gccugcagga cuacaagauc 900

cagagcgcgc ugcucgugcc gacccuguuc agcuucuucg ccaagagcac ccugaucgac 960

aaguacgacc ugucgaaccu gcacgagauc gccagcgggg gcgccccgcu gagcaaggag 1020

gugggcgagg ccguggccaa gcgguuccac cucccgggca uccgccaggg cuacggccug 1080

accgagacca cgagcgcgau ccugaucacc cccgaggggg acgacaagcc gggcgccgug 1140

ggcaaggugg ucccguucuu cgaggccaag guggugggacc uggacaccgg caagacccug 1200

ggcgugaacc agcggggcga gcugugcgug cgggggccga ugaucaugag cggcuacgug 1260

aacaacccgg aggccaccaa cgcccucauc gacaaggacg gcuggcugca cagcggcgac 1320

aucgccuacu gggacgagga cgagcacuuc uucaucgucg accggcugaa gucgcugauc 1380

aaguacaagg gcuaccaggu ggcgccggcc gagcuggaga gcauccugcu ccagcacccc 1440

aacaucuucg acgccggcgu ggccgggcug ccggacgacg acgccggcga gcugccggcc 1500

gcgguggugg ugcuggagca cggcaagacc augacggaga aggagaucgu cgacuacgug 1560

gccagccagg ugaccaccgc caagaagcug cggggcggcg ugguguucgu ggacgagguc 1620

ccgaagggcc ugaccgggaa gcucgacgcc cggaagaucc gcgagauccu gaucaaggcc 1680

aagaagggcg gcaagaucgc cguguaagac uaguucguug acacucacca uuucugugaa 1740

agacuuuuuu uuuuuuuaac auauuauacu agauuugacu aacucaaucu uguagcuucu 1800

gcaguucucc ccaccccaa ccuaguucuu agaguauguu uccccuuuug aaacauguaa 1860

acauacuuug ggcauaaaua uuuuuuaaaa uauaacuaua augcuucacu aauaccuuaa 1920

aaaugccuag ugaacuaacu caguacauua uauaauggcc aagugaaagu uuuguguuuu 1980

cauguccugu uuuucuuuga aauuauauag cccagaaauu agcucauuau cugaaaaacg 2040

uaugaagaac ugaugaauug uauaauacag gaguauugcc auugaaugua cuguuugauu 2100

uauucaagca gguaaugaac aauguuguca aacucucuaa ugagacauca uaauuaggac 2160

auaagcuaaa aggggcauua cuccggcagu cuuuuuuucu uaauccuagu accauacaua 2220

uucuuuggca ugaaagaaug aaaagcauua guaaacaacu gaaguccuac cauggcucug 2280

uaggguuuuu ggaacaauuc cuggaauugg aaagugaaaa uggauagcau gugggggaaa 2340

cccucaucug aguagcaaga uuuuaguaaa gaugacuaag ccauuaacag caugcauuca 2400

uauuuaauuu uauugacucc ugccaucagc uuuuguagau cuuuugggug gaagguugug 2460

auuuuuacug ggaggacuug aguagaagug gaugauuaaa auugaggagu auauaauucu 2520

uucugggacu gcuuaaaugu uauuguuuga aaaugccuuc acuuuccccc uuuggucaaa 2580

gagaugugcu uaaaauucuu auuccuucac aauaaauaau uuugauuuuc uuagacaaga 2640

ucuaaaaaaa aaaaaaaaaa aaaaaaaaaa aaaaaaaaaa aaaaaaaaaa aaaaaaaaaa 2700

aaaaaaaugc auccccccccc cccccccccc cccccccccc cccaaaggcu cuuuucagag 2760

ccaccagaau u 2771

<210> 49
<211> 201
<212> DNA
<213> Artificial Sequence

<220>
<223> Homo sapiens SLC38A6 3′-UTR
SLC38A6-001 ENST00000267488

<400> 49
aagaaatatt ttcctacttc ttacaagaat aatatacccc tagttgcaag aatgaattat 60

tccggaagac accctggatg aaaaataaca ttttaataaa aattattaac agaaaagcag 120

aacaaaatgg cagtgggtat ggggaagtaa gagtgtggca gttttaatca aaaaaagaaa 180

caaactcgaa atgctcttaa a 201

<210> 50
<211> 102
<212> DNA
<213> Artificial Sequence

<220>
<223> Homo sapiens DECR1 3'-UTR
NM_001359.1

<400> 50
gaccactttg gccttcatct tggttacaga aaagggaata gaaatgaaac aaattatctc 60

tcatcttttg actatttcaa gtctaataaa ttcttaatta ac 102

<210> 51
<211> 369
<212> DNA
<213> Artificial Sequence

<220>
<223> Homo sapiens PIGK 3'-UTR

<400> 51
acttgatgat gaatgaagaa tgcatggagg actgcaaact tggataataa tttatgtcat 60

tatatatttt taaaaatgtg tttctcttgt atgaattgga aataagtata aggaaactaa 120

atttgaatca actattaatt ttataactta aagaaaaata attgttaatg caactgctta 180

atggcactaa atatattcca gttttgtatt ttgtgtatta taaaagcgaa tgagacagag 240

atcagaatac attgactgtt tttgaaaata gtaatttccc cttatcccct tttcatttgg 300

aaaagaaaca attgtgaaga cattaaattc tcactaacag aagtaacttt ggttaattat 360

tttttgtat 369

<210> 52
<211> 460
<212> DNA
<213> Artificial Sequence

<220>
<223> Homo sapiens FAM175A 3'-UTR
FAM175A-009 ENST00000506553

<400> 52
tccttttaac cttacaagga gattttttta tttggctgat gggtaaagcc aaacatttct 60

attgttttta ctatgttgag ctacttgcag taagttcatt tgtttttact atgttcacct 120

gtttgcagta atacacagat aactcttagt gcatttactt cacaaagtac tttttcaaac 180

atcagatgct tttatttcca aaccttttt tcacctttca ctaagttgtt gaggggaagg 240

cttacacaga cacattcttt agaattggaa aagtgagacc aggcacagtg gctcacacct 300

gtaatcccag cacttaggga agacaagtca ggaggattga ttgaagttag gagttagaga 360

ccagcctggg caacgtattg agaccatgtc tattaaaaaa taaaatggaa aagcaagaat 420

agccttattt tcaaaatatg gaaagaaatt tatatgaaaa 460

<210> 53
<211> 505
<212> DNA
<213> Artificial Sequence

<220>
<223> Homo sapiens PHYH 3′-UTR
PHYH-002 ENST00000396913

<400> 53
aatagccatc tgctataact ctttcaacag aaaaccaaaa ccaaacgaaa tgtctaagga 60

aaatgttttc ttaatgagat gatgtaacct tttctatcac ttgttaaaag cagaaaacat 120

gtatcaggta cttaattgca tagagttagt tttgcagcac aatggtgttg ctttaatgga 180

aaaaaaaaac agtaaaagtg aaatattact gttttaagga aaactaattt agggtggcag 240

ccaataaagg tggttggtgt ctaatttaag tgttaaatca atttctttca ttcagttagc 300

tctttaccca agaagaagtg aatgatttgg agcttagggt atgttttgta tcccctttct 360

gataaaccca ttccctacca attttatgtc ataagagatt tttttccccc aaatctagaa 420

caatgtataa tacattcaca tctagtcaag ggcataggaa cggtgtcatg gagtccaaat 480

aaagtggata ttcctgctcg gacaa 505

<210> 54
<211> 404
<212> DNA
<213> Artificial Sequence

<220>
<223> Homo sapiens TBC1D19 3′-UTR
TBC1D19-001 ENST00000264866

<400> 54
tcttcttcac agtcactggc aacacatcta gtttttcatt agaaacaaat catgaactat 60

gcaaactctg cataaaacca aaatgaaact ttgcatataa gccaataaag atcatgttcc 120

ctcttcagtt aaacctaagt agtttctcac tttttgaaac aataactctg caccaaatat 180

tgcatcgcat gctgctgatt ttcaagagag aagcaataaa cacaacttct gctaaattga 240

gcattatata tataatatta taatatatat ataatcctga cttgtcaatg gcatgtaata 300

atatatgcaa taagaactaa agatactgta ataaacttca agaggtaatg tagcttcttg 360

gataattctt ttatgtcagt ttataaattt atctctagat aatg 404

<210> 55
<211> 353
<212> DNA
<213> Artificial Sequence

<220>
<223> Homo sapiens TBC1D19 NM_018317.2 3'-UTR

<400> 55
tcttcttcac agtcactggc aacacatcta gtttttcatt agaaacaaat catgaactat 60

gcaaactctg cataaaacca aaatgaaact ttgcatataa gccaataaag atcatgttcc 120

ctcttcagtt aaacctaagt agtttctcac tttttgaaac aataactctg caccaaatat 180

tgcatcgcat gctgctgatt ttcaagagag aagcaataaa cacaacttct gctaaattga 240

gcattatata tataatatta taatatatat ataatcctga cttgtcaatg gcatgtaata 300

atatatgcaa taagaactaa agatactgta ataaacttca agaggtaaaa aaa 353

<210> 56
<211> 242
<212> DNA
<213> Artificial Sequence

<220>
<223> Homo sapiens PIGB 3'-UTR
PIGB-201 ENST00000539642

<400> 56
aaattcaaca tgaagatgaa attctgaact ttcctagata aattaacatt gctgggtgga 60

aatattcaga tgctgcttaa atacttcggt aaacactggg taagattcat ggaacttaga 120

aaaaagctgt atgaactgct ttaccaaata tcactactga ggaaatgtat aaaataccac 180

atagtataaa attacatgtt aatacaatgc cagattttaa ataaagacct ttagttttcc 240

tc 242

<210> 57
<211> 157
<212> DNA
<213> Artificial Sequence

<220>
<223> Homo sapiens ALG6 3'-UTR
ALG6-006 ENST00000263440

<400> 57
ctgtattcct aaacaaattg tttcctaaac aaatgtgaaa atgtgaacag tgctgaaagg 60

ttttgtgaac tttttgctat gtataaatga aattaccatt ttgagaacca tggaaccaca 120

ggaaaggaaa tggtgaaaag tcattgttgt ctacaca 157

<210> 58
<211> 324

<212> DNA
<213> Artificial Sequence

<220>
<223> Homo sapiens CRYZ 3'-UTR
CRYZ-005 ENST00000370871

<400> 58
tgattaattc tttcatggat ttcctatgta attagaggta ctgtctttcc cccagttgta 60

cttaccctat cttttcttta attaacattc gattccatga gcttcttatg tgaaaaaata 120

agatttttct ttagagagca gaagcagaag agtaaaattt attgtatagc tagcaatatt 180

tttttatgcc atctgtctca aatcaaagag tcatcatagt aggaaataac atgttagttg 240

tcatttggca tgagtgtgca ttccagtaat tcttaattga tatttgatta attccatacc 300

tttgattaaa acatgctagt tcaa 324

<210> 59
<211> 510
<212> DNA
<213> Artificial Sequence

<220>
<223> Homo sapiens BRP44L 3'-UTR
BRP44L-001 ENST00000360961

<400> 59
caatggaaaa ggaagaacaa ggtcttgaag ggacagcatt gccagctgct gctgagtcac 60

agatttcatt ataaatagcc tccctaagga aaatacactg aatgctattt ttactaacca 120

ttctattttt atagaaatag ctgagagttt ctaaaccaac tctctgctgc cttacaagta 180

ttaaatattt tacttctttc cataaagagt agctcaaaat atgcaattaa tttaataatt 240

tctgatgatg gttttatctg cagtaatatg tatatcatct attagaattt acttaatgaa 300

aaactgaaga gaacaaaatt tgtaaccact agcacttaag tactcctgat tcttaacatt 360

gtctttaatg accacaagac aaccaacagc tggccacgta cttaaaattt tgtccccact 420

gtttaaaaat gttacctgtg tatttccatg cagtgtatat attgagatgc tgtaacttaa 480

tggcaataaa tgatttaaat atttgttaaa 510

<210> 60
<211> 354
<212> DNA
<213> Artificial Sequence

<220>
<223> Homo sapiens ACADSB 3'-UTR
ACADSB-004

<400> 60
cgtctatagg agtgggaccc ctccctggtg tcactgctgt aaaattttaa acggttgtgt 60

cttgttggga gtaagtgcct tgcgtgggaa taaacttcca cagcattcga atattttaat 120

gaagccctta gtcagggtcc tggtgttggc ctttttggtt ttctcttttc aggctgttta 180

acttaggcac aggagatcca cttttaaact tgggaaataa gcacctgtat ttttttccaa 240

aactgttttt aaagctgtat acgcatacat atatatattt ttactctgtc ttactctgtc 300

acccaggcta gagtgcagtg gcgcgatctc agctcactgc agccttgacc tcct 354

<210> 61
<211> 539
<212> DNA
<213> Artificial Sequence

<220>
<223> Homo sapiens TMEM14A 3'-UTR
NM_014051.3

<400> 61
gcatctggag gaacagaaaa ctaagttcat gtcatcctgc tgtaatgggc agagcatatt 60

tttttgtat ttaaaagata aacttcaata tggaatgcta gaaacacaaa tagcactgtc 120

acctctaata tgaacattag tttgaggtag ttttttcta aagcaaaaat tttaactgtt 180

ttctaattgt caagcactat tttcattaaa agtgtctaat gaatcatgat atactcttcc 240

atttgttgtg tctatttttt atatatttgg tattttttga aaattccaaa tactcatgtc 300

tcaagtaagc ttaaactaca acttgtcaca taaaggaagt cttaagtgga gttcacagaa 360

tgataatgta tctatttgtc atttgtgtta tatttgaaat tattagaaat tatgcttttt 420

ccattttaat tgtattgctg ccagtgctat tttttcttt aaaaaatttt attcttagca 480

cactgttatg tcctaactga atgtattcag tattcaaata aaagacattt tggttcaaa 539

<210> 62
<211> 292
<212> DNA
<213> Artificial Sequence

<220>
<223> Homo sapiens GRAMD1C 3'-UTR
GRAMD1C-005 ENST00000472026

<400> 62
tgatctgaag gactaaaacc gcagagatac ttggaactta aagaaaatac ctggaagaaa 60

accagacgaa tgaaggattt tggcatagaa catttctatg ttttttcatt attgagattt 120

ctaatatgaa catttctttc agtaacattt atttgataat tagtttctgc tggccttaat 180

aatccatcct ttcacttctt atagatattt ttaagctgtg aatttcttca gtgaaccatg 240

aaatatatta tagaactgaa tttctctgat acaaaaagaa aatgacacac cc 292

<210> 63
<211> 94
<212> DNA

<213> Artificial Sequence

<220>
<223> Homo sapiens C11orf80 3'-UTR
C11orf80-201 ENST00000360962

<400> 63
gccgggtccc cttccgcaag cgcccaccga tccggaggct gcgggcagcc gttatcccgt 60

ggtttaataa agctgccgcg cgctcaccaa gtcc 94

<210> 64
<211> 266
<212> DNA
<213> Artificial Sequence

<220>
<223> Homo sapiens ANXA4 3'-UTR
ANXA4-002 ENST00000409920

<400> 64
aataaaaatc ccagaaggac aggaggattc tcaacacttt gaattttttt aacttcattt 60

ttctacactg ctattatcat tatctcagaa tgcttatttc caattaaaac gcctacagct 120

gcctcctaga atatagactg tctgtattat tattcaccta taattagtca ttatgatgct 180

ttaaagctgt acttgcattt caaagcttat aagatataaa tggagatttt aaagtagaaa 240

taaatatgta ttccatgttt ttaaaa 266

<210> 65
<211> 490
<212> DNA
<213> Artificial Sequence

<220>
<223> Homo sapiens TBCK 3'-UTR
TBCK-002 ENST00000361687

<400> 65
agaaccaaga gtgtgactgc caaaacttag tgtggcatca gcaccaacag cacagttctt 60

catatccacg ccactctcag acaaaactag atgtccagat tgttgcattt ccgtaaagtt 120

tgtcacgaga catttttttaa aatctcataa cccacatgtt cagttatcca tgcaagaaac 180

ttgactctac atgtattgct gaaagaattt tcttaacagt gaaatctgat catatatttt 240

taccacactg ccacataaag cccaagaaat tcagctgaca agacagattt agcattatca 300

agaaatccca tttgccctga aaaagctgtc ctccattgta ctgaacagac agtcctgtcg 360

attgtgttat ttagaaacat acactgaatg tgggctgaaa tcatcatctt tccataatga 420

aaactgagaa actattcaca atgcattcct tataaataaa tgctacattt agtaactcat 480

ttcacccaaa 490

<210> 66

<211> 320
<212> DNA
<213> Artificial Sequence

<220>
<223> Homo sapiens IFI6 3'-UTR
IFI6-001 ENST00000361157

<400> 66
ccagcagctc ccagaacctc ttcttccttc ttggcctaac tcttccagtt aggatctaga 60

actttgcctt tttttttttt tttttttttt tgagatgggt tctcactata ttgtccaggc 120

tagagtgcag tggctattca cagatgcgaa catagtacac tgcagcctcc aactcctagc 180

ctcaagtgat cctcctgtct caacctccca agtaggatta caagcatgcg ccgacgatgc 240

ccagaatcca gaactttgtc tatcactctc cccaacaacc tagatgtgaa aacagaataa 300

acttcaccca gaaaacactt 320

<210> 67
<211> 479
<212> DNA
<213> Artificial Sequence

<220>
<223> Homo sapiens CAMKMT 3'-UTR
(synonym C2orf34) ENST00000378494

<400> 67
aagattaagc ttctcaaaga cgaagaaacg tatcaagtgc atagggaata tttttacaaa 60

aacggaaatc tgtaaggggt ataatcgcct gcctgcgccc tttgcagcat ttcacgtgtg 120

ggctatggac tccacctgtc ctcacccacg ttattcccca gctgccctct ccagctccct 180

ccccgcctct ttttacactc tgcttgttgc tcgtcctgcc ctaaaccttt gtttgtcttt 240

aaatgtgtat aagctgcctg tctgtgactt gaatttgact ggtgaacaaa ctaaatattt 300

ttccctgtaa ttgagacaga atttcttttg atgataccca tccctccttc attttttttt 360

ttttttggt ctttgttctg ttttggtggt ggtagttttt aatcagtaaa cccagcaaat 420

atcatgattc tttcctggtt agaaaaataa ataaagtgta tctttttatc tccctccaa 479

<210> 68
<211> 476
<212> DNA
<213> Artificial Sequence

<220>
<223> Homo sapiens ALDH6A1 3'-UTR
NM_005589.2

<400> 68
aaacaagttt gtttaagact gactccatcc tgagtaatct ccctttattt ttgaccagct 60

tcatttgtca gctttgctca gatcagatcg atgggattgg aatacattgt aactaaaatc 120

ttcctcagga ctattaaccc ccgcaaagtt tctataggga actgcctagt gtaacaatga 180

aaccagattt ctcacttgct cttcatactt ctattttgag gtaactgttg taactatgaa 240

atgcttatct gaaagtagtg cttaaacctg atttctaaaa attatcccat tttctgatga 300

tttgaagggg agaaaagcca gtgtatgtaa agaaaatgtt ccagccaggc gcggtggctc 360

acgcctgtaa ttccatcatt ttgggaggcc acagtgggca gattgcttga gcccaggagt 420

tgaagaacgt ggcgaaaccc cgtatctatt atttaaaaaa attgaaaaag taaaaa 476

<210> 69
<211> 567
<212> DNA
<213> Artificial Sequence

<220>
<223> Homo sapiens AGTPBP1 3′-UTR
AGTPBP1-004 ENST00000357081

<400> 69
gcccgctgcc atctcttgtt aactgcaaag aataaatgaa atatcttggt ttttatttcc 60

caggaagctt gagagaaatg agtttataca gagctgactc aaaaagacaa aaagtaactt 120

gggccagttt ggtttcaaga taataaatgt gttattaatt aatgataaaa ttggcgcttg 180

ttttattttc gatattcaat gcactttatg tagcattgaa tgatcaaata ttggatttac 240

ctttaaaaaa aaaacctgag tatcattgca tgaattttta tctccctatg gttatatcct 300

gcatcaagtg gataattttg aagtgtgttc agaatataaa attgaaattt tagagttgtt 360

gaaaatcctg acttgttgaa aactaatata tatgtacatg gatttctata gatgtgtttg 420

tttagaagtg ggtagatatt gcagataaga ctgttcttca gaatcatgtt aactattggg 480

ttgtgactga agtagtccag ggtttgcctt gaaaccatta cattctacat ttaccaaatt 540

aaacaaataa aaactgtatt aaatgtt 567

<210> 70
<211> 169
<212> DNA
<213> Artificial Sequence

<220>
<223> Homo sapiens CCDC53 3′-UTR
CCDC53-001 ENST00000240079

<400> 70
gcttaatttt gataagaatt acatatgcat gcataggggt acatttacat tctgtaagag 60

attgagcctg aactctctta gtcataaaaa catcaaatgg ccacatgtcc actaccaagc 120

ttcttctatg ttaaaaaaat aataataaag cagttttaac ctgccagta 169

<210> 71
<211> 194

<212> DNA
<213> Artificial Sequence

<220>
<223> Homo sapiens LRRC28 3′-UTR
LRRC28-002 ENST00000331450

<400> 71
taaacactca agaacctcag gagcgctgcc agcttgacac tggggaatcc agccagtcca 60

gcacactctt ccatcctgtc ctgtccaatg cgggggcact gcagaactct ctagaaatgt 120

catgattgag cttcagagct aaaatgcctt cacccttccc ccaagttgga atatatcctc 180

ccccaaatta agga 194

<210> 72
<211> 120
<212> DNA
<213> Artificial Sequence

<220>
<223> Homo sapiens CCDC109B 3′-UTR
NM_017918.4

<400> 72
tcttacagtt ttaaatgtcg tcagattttc cattatgtat tgattttgca acttaggatg 60

tttttgagtc ccatggttca ttttgattgt ttaatctttg ttattaaatt cttgtaaaac 120

<210> 73
<211> 426
<212> DNA
<213> Artificial Sequence

<220>
<223> Homo sapiens PUS10 3′-UTR
PUS10-001 ENST00000316752

<400> 73
ctttcaaatt tggagacaaa gagtatggtt ttcctggcat gatgtggaca tccatggagc 60

acatgccgta aaatggctgt ttacccacca taacggtgtc ttgaaaacta tttggatcat 120

gttgatctat ataattgtta atttgttgta acatctcagg atctatatat gtgtatattt 180

tgtgttaaat tgttccaagg atgtcttagg atttttctca ttccctcttt cacccccaca 240

aaccaaacta tgaataatga aataattctc cttaattctt tcatttagag aggtgcacaa 300

acaggacaca ttctctgtta acctaagaag ctgtaatttc agcaagattt ccctccacaa 360

gagatatacc acctttaaaa tcatgttcta atttttgtaa attatctgaa taaaagttat 420

atctag 426

<210> 74
<211> 71
<212> DNA
<213> Artificial Sequence

<220>
<223> Homo sapiens CCDC104 3'-UTR
CCDC104-002 ENST00000339012

<400> 74
taattaagaa caatttaaca aaatggaagt tcaaattgtc ttaaaaataa attatttagt 60

ccttacactg a 71

<210> 75
<211> 125
<212> DNA
<213> Artificial Sequence

<220>
<223> Homo sapiens CASP1 3'-UTR
CASP1-007 ENST00000527979

<400> 75
aataaggaaa ctgtatgaat gtctgtgggc aggaagtgaa gagatccttc tgtaaaggtt 60

tttggaatta tgtctgctga ataataaact tttttgaaat aataaatctg gtagaaaaat 120

gaaaa 125

<210> 76
<211> 174
<212> DNA
<213> Artificial Sequence

<220>
<223> Homo sapiens SNX14 3'-UTR
SNX14-007 ENST00000513865

<400> 76
acacttggat ttggtataga ataacccatt gaaatttctg ctgtgcgagg gtggtagaaa 60

tttacttttt tgggtatatt cttatatata ttatgtacat cgctgtctga aattttagtt 120

attttttgtt tttaataaag actaacacaa acttaatgat taaaagtgat tgag 174

<210> 77
<211> 237
<212> DNA
<213> Artificial Sequence

<220>
<223> Homo sapiens SKAP2 3'-UTR
SKAP2-201 (part of SKAP2.001 ENST00000345317)

<400> 77
gagtcctgga aaaggaaaat tcttctgctt gtctgcaaat gctttggatt tagaagcgtc 60

atgaaagcac gagtgacagc tcctaacctc tccttgtttt attaaacatt acttatcttt 120

gactgttatt ttatgcagtc gctcattaaa atattcctct gatgtgaaat taaatgaagg 180

atattaatgt aaattagatg caaccagtta agttatacct gttgctattt tgcaaag 237

<210> 78
<211> 362
<212> DNA
<213> Artificial Sequence

<220>
<223> Homo sapiens NDUFB6 3'-UTR
NM_182739.2

<400> 78
agattatgta aaaagttaaa aggcttatga gcctaagttt gttcctatat taccatattt 60

actgaatttt ctggaaaagt aactttaata aagtttaatc tcagaaattg tcatatctgt 120

tttcaagcat tgtacaattt gagactgagt aatttaacaa taagtaaaaa gtggacatgc 180

taaacaaata tgagagacta cctacttttt ctggtcattc ttgacttgga aaacggtatg 240

gaaaagtatt tagttacatg tttgtttgtt tttttcttac acagtactta cactaatttg 300

gtatcagggt atgcaacagt gaaatatcac aataaacaaa tgtaagaaca aaaaaaaaaa 360

aa 362

<210> 79
<211> 549
<212> DNA
<213> Artificial Sequence

<220>
<223> Homo sapiens EFHA1 3'-UTR
EFHA1-001 ENST00000382374

<400> 79
taaaagatat aatagtatgg caattatatt gttccaaatg tcaaaatttg tgatttttta 60

gaagtacttg ctatttatct tcttaagtct tcattgatat tctgtgtgaa ataagcatgt 120

cttgtacttg ctttctgatt cataatttta ttaaagaact tagtagaaag aaaagtaagt 180

ataaaaatag atattggatt ctgtcagaag gcctagattt gaaataatgt tttgtacttc 240

ggtaagatgg aaaacttagt gattcactga tttcttagac actctaatat gatatgcttt 300

ctggaaggat aaaacaaata catatgggaa aaagtacttg agaccaaggc cagcatcaat 360

tccagacatc ttcatgttcc taataggcta aatgaagtta aaaacttatt tcagattttt 420

ctcatctgta ccttatatct cataaattta ttgcatattt tatgtcagta gcttagctgt 480

ttattgtctt taaaataaca tgtaaacttc aatgttctat ctggaagcag aataaaatat 540

ttacataga 549

<210> 80
<211> 288
<212> DNA
<213> Artificial Sequence

<220>

<223> Homo sapiens BCKDHB 3'-UTR
BCKDHB-005 ENST00000356489

<400> 80
ccatatagaa aagctggaag attatgacta gatatggaaa tattttttct gaattttttt 60

ttatatttcc tccgacttac ctctttttga aaagagagtt tttattaagt gaaccatcac 120

gatattggct gaaaagttct acattctatt attgtattgt aacacacatg tattgatgat 180

tttcattaag agtttcagat taactttgaa aaatattcca catggtaatc ttataaattc 240

tgtttaatta catctgtaaa tattatgtgt gtgatagtat tcaataaa 288

<210> 81
<211> 414
<212> DNA
<213> Artificial Sequence

<220>
<223> Homo sapiens BCKDHB 3'-UTR
NM_001164783.1

<400> 81
gacctgctca gcccaccccc acccatcctc agctaccccg agaggtagcc ccactctaag 60

gggagcaggg ggacctgaca gcacaccact gtcttcccca gtcagctccc tctaaaatac 120

tcagcggcca gggcggctgc cactcttcac ccctgctcct cccggctgtt acattgtcag 180

gggacagcat ctgcagcagt tgctgaggct ccgtcagccc cctcttcacc tgttgttaca 240

gtgccttctc ccaggggctg ggtgagggca cattcaggac tagaagcccc tctgggcatg 300

gggtggacat ggcaggtcag cctgtggaac ttgcgcaggt gcgagtggcc agcagaggtc 360

acgaataaac tgcatctctg cgcctggctc tctaccaaaa aaaaaaaaaa aaaa 414

<210> 82
<211> 414
<212> DNA
<213> Artificial Sequence

<220>
<223> Homo sapiens BBS2 3'-UTR
NM_031885.3

<400> 82
gtgaggaaaa tacaggtcat gaagttcctg gcaaagattt tctgttaaaa acctatgctg 60

gtttgctttg gatcacaccc tggtgaaccc cgggtgctaa gaatgaaaat aaccttggtg 120

agttgtacaa attaaagaca aagaactaca tgtgaagata gacttgcttt ctatttttaa 180

atcagtagta gtactgttgc tgaataatac taggttttta tggaatagga tgaatgcttt 240

tgaagtatta gggcttcaga gtccaatttt gcttatttat ggtatataaa tacatatttt 300

tttcttgaaa ttgcaattga gtttgtactt ttcaaataga ttatctactt tttcattaaa 360

atgtaaagat gttaaacttt gtgttgattg attataaaat caccaccaaa tcag 414

<210> 83
<211> 409
<212> DNA
<213> Artificial Sequence

<220>
<223> Homo sapiens LMBRD1 3'UTR
NM_018368.3

<400> 83
cagccttctg tcttaaaggt tttataatgc tgactgaata tctgttatgc atttttaaag 60

tattaaacta acattaggat ttgctaacta gctttcatca aaaatgggag catggctata 120

agacaactat attttattat atgttttctg aagtaacatt gtatcataga ttaacatttt 180

aaattaccat aatcatgcta tgtaaatata agactactgg ctttgtgagg gaatgtttgt 240

gcaaaatttt ttcctctaat gtataatagt gttaaattga ttaaaaatct tccagaatta 300

atattccctt ttgtcacttt ttgaaaacat aataaatcat ctgtatctgt gccttaggtt 360

ctccagagtg atgtggaatt ttaaagtgtc tctctctgat tgcctccaa 409

<210> 84
<211> 466
<212> DNA
<213> Artificial Sequence

<220>
<223> Homo sapiens ITGA6 3'-UTR
ITGA6-003 ENST00000409532

<400> 84
tattgatcta cttctgtaat tgtgtggatt ctttaaacgc tctaggtacg atgacagtgt 60

tccccgatac catgctgtaa ggatccggaa agaagagcga gagatcaaag atgaaaagta 120

tattgataac cttgaaaaaa aacagtggat cacaaagtgg aacgaaaatg aaagctactc 180

atagcggggg cctaaaaaaa aaaagcttca cagtacccaa actgcttttt ccaactcaga 240

aattcaattt ggatttaaaa gcctgctcaa tccctgagga ctgatttcag agtgactaca 300

cacagtacga acctacagtt ttaactgtgg atattgttac gtagcctaag gctcctgttt 360

tgcacagcca aatttaaaac tgttggaatg gatttttctt taactgccgt aatttaactt 420

tctgggttgc ctttattttt ggcgtggctg acttacatca tgtgtt 466

<210> 85
<211> 285
<212> DNA
<213> Artificial Sequence

<220>
<223> Homo sapiens HERC5 3'-UTR
HERC5-001 ENST00000264350

<400> 85
ccagcttgct tgtccaacag ccttattttg ttgttgttat cgttgttgtt gttgttgttg 60

ttgttgtttc tctactttgt tttgttttag gcttttagca gcctgaagcc atggtttttc 120

atttctgtct ctagtgataa gcaggaaaga gggatgaaga agagggttta ctggccggtt 180

agaacccgtg actgtattct ctcccttgga tacccctatg cctacatcat attccttacc 240

tcttttggga aatatttttc aaaaataaaa taaccgaaaa attaa 285

<210> 86
<211> 515
<212> DNA
<213> Artificial Sequence

<220>
<223> Homo sapiens HADHB 3'-UTR
HADHB-001 ENST00000317799

<400> 86
tagatccaga agaagtgacc tgaagtttct gtgcaacact cacactaggc aatgccattt 60

caatgcatta ctaaatgaca tttgtagttc ctagctcctc ttaggaaaac agttcttgtg 120

gccttctatt aaatagtttg cacttaagcc ttgccagtgt tctgagcttt tcaataatca 180

gtttactgct ctttcaggga tttctaagcc accagaatct cacatgagat gtgtgggtgg 240

ttgtttttgg tctctgttgt cactaaagac taaatgaggg tttgcagttg ggaaagaggt 300

caactgagat ttggaaatca tctttgtaat atttgcaaat tatacttgtt cttatctgtg 360

tcctaaagat gtgttctcta taaaatacaa accaacgtgc ctaattaatt atggaaaaat 420

aattcagaat ctaaacacca ctgaaaactt ataaaaaatg tttagataca taaatatggt 480

ggtcagcgtt aataaagtgg agaaatattg gagaa 515

<210> 87
<211> 116
<212> DNA
<213> Artificial Sequence

<220>
<223> Homo sapiens ANAPC4 3'-UTR
ANAPC4-001 ENST00000315368

<400> 87
tctagcttgc cattattgtg tgtgtaatta tggccaaaag gacataggag atggactaag 60

atgtcttgga ccacctttgt gtaacaaaga aataaacagt aaattttatt ttttca 116

<210> 88
<211> 154
<212> DNA
<213> Artificial Sequence

<220>
<223> Homo sapiens PCCB 3'-UTR

NM_000532.4

<400> 88
acaaatcaaa ggaaaagaaa ccaagaactg aattactgtc tgcccattca catcccattc 60

ctgccttttg caatcatgaa acctgggaat ccaaatagtt ggataactta gaataactaa 120

gtttattaaa ttctagaaag atctcaaaaa aaaa 154

<210> 89
<211> 90
<212> DNA
<213> Artificial Sequence

<220>
<223> Homo sapiens ABCB7 3'-UTR
ABCB7-001 ENST00000253577

<400> 89
gtcacataag acattttctt tttttgttgt tttggactac atatttgcac tgaagcagaa 60

ttgttttatt aaaaaaatca tacattccca 90

<210> 90
<211> 318
<212> DNA
<213> Artificial Sequence

<220>
<223> Homo sapiens PGCP 3'-UTR
CPQ-001 ENST00000220763

<400> 90
aaacagtaag aaagaaacgt tttcatgctt ctggccagga atcctgggtc tgcaactttg 60

gaaaactcct cttcacataa caatttcatc caattcatct tcaaagcaca actctatttc 120

atgctttctg ttattatctt tcttgatact ttccaaattc tctgattcta gaaaaaggaa 180

tcattctccc ctccctccca ccacatagaa tcaacatatg gtagggatta cagtgggggc 240

atttctttat atcacctctt aaaaacattg tttccacttt aaaagtaaac acttaataaa 300

tttttggaag atctctga 318

<210> 91
<211> 133
<212> DNA
<213> Artificial Sequence

<220>
<223> Homo sapiens NFU1 3'-UTR
NM_001002755.2

<400> 91
aataatctgg attttctttg ggcataacag tcagacttgt tgataatata tatcaagttt 60

ttattattaa tatgctgagg aacttgaaga ttaataaaat atgctcttca gagaatgata 120

tataaatatt gca 133

<210> 92
<211> 246
<212> DNA
<213> Artificial Sequence

<220>
<223> Homo sapiens OMA1 3'-UTR
OMA1-001 ENST00000371226

<400> 92
attaaaattt atgagacaca agatatatga agaatgttgc agtccttatc attttatgtt 60

actttttaaa aaatgatgtt tgaagtgaaa aaaaaaagga tattcagggt caaatcatgt 120

acattacaga tattatctaa attcttctag aatttatttt tcatgaaata ttgatgtatt 180

ttaatctatg ttaaaatatc ttcaatgagg aaaatgtcac agaataaatt tatattacac 240

atttta 246

<210> 93
<211> 423
<212> DNA
<213> Artificial Sequence

<220>
<223> Homo sapiens HHLA3 3'-UTR
NM_001036646.1

<400> 93
ggcgaatcca tagagtaagc ttagtgatgt gtgtcagacc tctgagccca agcaaagcca 60

tcatatcccc tgtgacctgc atgtatacat ccagatggcc tgaagcaagt gaagaatcac 120

aaaagaagtg aaaagggccg gttcctgcct taactgatga cattccacca ttgtgatttg 180

ttcctgcccc accttaactg agcgattaac ctgtgaactt ccttctcctg gctcagaagc 240

ttccccactg agcaccttgt gacccccgcc cctgcctgcc atagaacaac cccctttgat 300

tgtaattttc ctttacctac ccaaatccta taaaacggcc ccacccctat ctcccttcgc 360

tgacactctc tttggactca gcctgcctgc acctaggtga ttaaaaagct ttattgctca 420

cgc 423

<210> 94
<211> 292
<212> DNA
<213> Artificial Sequence

<220>
<223> Homo sapiens HHLA3 3'-UTR
NM_001031693.2

<400> 94
aaagggccgg ttcctgcctt aactgatgac attccaccat tgtgatttgt tcctgcccca 60

ccttaactga gcgattaacc tgtgaacttc cttctcctgg ctcagaagct tccccactga 120

gcaccttgtg acccccgccc ctgcctgcca tagaacaacc ccctttgatt gtaattttcc 180

tttacctacc caaatcctat aaaacggccc cacccctatc tcccttcgct gacactctct 240

ttggactcag cctgcctgca cctaggtgat taaaaagctt tattgctcac gc 292

<210> 95
<211> 342
<212> DNA
<213> Artificial Sequence

<220>
<223> Homo sapiens ACAA2 3′-UTR
NM_006111.2

<400> 95
agagaccagt gagctcactg tgacccatcc ttactctact tggccaggcc acagtaaaac 60

aagtgacctt cagagcagct gccacaactg gccatgccct gccattgaaa cagtgattaa 120

gtttgatcaa gccatggtga cacaaaaatg cattgatcat gaataggagc ccatgctaga 180

agtacattct ctcagatttg aaccagtgaa atatgatgta tttctgagct aaaactcaac 240

tatagaagac attaaaagaa atcgtattct tgccaagtaa ccaccacttc tgccttagat 300

aatatgatta taaggaaatc aaataaatgt tgccttaact tc 342

<210> 96
<211> 446
<212> DNA
<213> Artificial Sequence

<220>
<223> Homo sapiens GSTM4 3′-UTR
GSTM4-001 ENST00000369836

<400> 96
tgccttgaag gccaggaggt gggagtgagg agcccatact cagcctgctg cccaggctgt 60

gcagcgcagc tggactctgc atcccagcac ctgcctcctc gttcctttct cctgtttatt 120

cccatcttta cccccaagac tttattgggc ctcttcactt ccccctaaacc cctgtcccat 180

gcaggcccctt tgaagcctca gctacccact ttccttcatg aacatccccc tcccaacact 240

acccttccct gcactaaagc cagcctgacc ttccttcctg ttagtggttg tatctgcttt 300

gaagggccta cctggcccct cgcctgtgga gctcagccct gagctgtccc cgtgttgcat 360

gacagcattg actggtttac aggccctgct cctgcagcat ggcccctgcc ttaggcctac 420

ctgatcaaaa taaagcctca gccaca 446

<210> 97
<211> 465
<212> DNA
<213> Artificial Sequence

<220>
<223> Homo sapiens GSTM4 3'-UTR
GSTM4-003 ENST00000326729

<400> 97
tggtcaattt tctgcatcaa cttgactggg ctaagggatg ctcagatggc aggtaaaatc 60

attgtgcttg tgagggtgtt tccagaagag atttgccttt gaatcagaag acagcaaaga 120

tttccttcag caatgaagga ggcatccacc aaactgtcag ggcccagaga gaagaaaaag 180

acaggaaggg tgaatttgac ctctctgact gggacatcca tctctgccta tcctgggacc 240

tccacactcc tggttctctg gccttcagac ttgatcaggg actaacacca tcgcctccca 300

cccccacctt tgttctgagg cctttagcct ctgaatgata ccactggctt tcctgcttct 360

ctatcctgca gtcggcagat catgggactt cttcactcca aaattgtgtg agccaattcc 420

cataacagat agataaattt ataaataaac acacaaattt cctac 465

<210> 98
<211> 274
<212> DNA
<213> Artificial Sequence

<220>
<223> Homo sapiens ALG8 3'-UTR
NM_001007027.2

<400> 98
ctgaaacctc cgcctcccag aaaagaaaaa cctcttttta attggatgga aactttctac 60

ctgcttggcc tggggcctct ggaagtctgc tgtgaatttg tattcccttt cacctcctgg 120

aaggtgaagt accccttcat ccctttgtta ctaacctcag tgtattgtgc agtaggcatc 180

acatatgctt ggttcaaact gtatgtttca gtattgattg actctgctat tggcaagaca 240

aagaaacaat gaataaagga actgcttaga tatg 274

<210> 99
<211> 122
<212> DNA
<213> Artificial Sequence

<220>
<223> Homo sapiens C11orf74 3'UTR

<400> 99
ttcacagagg cattttgtgt gtgtgtgctt attttaattt tgttcttatt ctagcaacat 60

tagaataaaa gataaaccta ctataattcc ctttgtggaa atttaaaaaa aaaaaaaaaa 120

aa 122

<210> 100
<211> 133
<212> DNA
<213> Artificial Sequence

<220>
<223> Mus musculus Ndufa1 3′-UTR
Ndufa1-001 ENSMUST00000016571

<400> 100
ggaagcattt tcctggctga ttaaaagaaa ttactcagct atggtcatct gttcctgtta 60

gaaggctatg cagcatatta tatactatgc gcatgttatg aaatgcataa taaaaaattt 120

taaaaaatct aaa 133

<210> 101
<211> 155
<212> DNA
<213> Artificial Sequence

<220>
<223> Mus musculus Atp5e 3′-UTR
NM_025983

<400> 101
ctgaatctga agcctgaagt gctgagtctt gaaggtgaag catgtgggcc cctgttctgg 60

cagatggaaa tcaacctcac ctcctggggg acaggctgcc catctcgttg ataaattgac 120

tatgccaata aattaacatg gttcactttc aaaaa 155

<210> 102
<211> 136
<212> DNA
<213> Artificial Sequence

<220>
<223> Mus musculus Gstm5 3′-UTR
NM_010360

<400> 102
gccagagctc gctgctgctg agccatcttg ccctgagggg cccacactct tagctcactg 60

tcagtcttgt tccatcctgt cctgagggcc cccactctgt ctcctctgct ctttctaata 120

aacagcagtt gcatta 136

<210> 103
<211> 189
<212> DNA
<213> Artificial Sequence

<220>
<223> Mus musculus Uqcr11 3′-UTR
NM_025650

<400> 103
gcagcccctc ccccaccaca ggcctcgatg gtaccatgtg ccgaggcctc agacacagcg 60

tagtcctgtg gaagacactg aggaagctgg acactggaga ggtctgcacc gctcagggag 120

cttccatgtt gacagacact agggctgcct tgatgggtgc agcattaaac cttattctta 180

tgccttgga 189

<210> 104
<211> 143
<212> DNA
<213> Artificial Sequence

<220>
<223> Mus musculus IFi27I2a 3'-UTR
IFi27I2a-001 ENSMUST00000055071; NM_029803

<400> 104
gcttaggaga tgacacttct atcagctcaa ctcaaagcct gtacagacta cgcaggagat 60

gaagttccaa aaggcacctt cagaaccctc actgatgtca aagaatgatg aaaacaacaa 120

agtatatggg ctggtgttcc taa 143

<210> 105
<211> 237
<212> DNA
<213> Artificial Sequence

<220>
<223> Mus musculus Cbr2 3'-UTR
NM_007621

<400> 105
tctgctcagt tgccgcggac atctgagtgg ccttcttagc cccaccctca gccaaagcat 60

ttactgatct cgtgactccg ccctcatgct acagccacgc ccaccacgca gctcacagtt 120

ccaccccat gttactgtcg atcccacaac cactccaggc gcagaccttg ttctctttgt 180

ccactttgtt gggctcattt gcctaaataa acgggccacc gcgttacctt taactat 237

<210> 106
<211> 118
<212> DNA
<213> Artificial Sequence

<220>
<223> Mus musculus Atp5l 3'-UTR
Atp5l-201 ENSMUST00000043675

<400> 106
agaccaatct ttaacttctg atttgagttc ttatttgaat gttcttggac catgtgtaac 60

aggactgcta tctgaataaa atactaggtg ttgaaaacac tgctgtgttt tctctgtc 118

<210> 107
<211> 271
<212> DNA
<213> Artificial Sequence

<220>
<223> Mus musculus Tmsb10 3'-UTR
NM_025284

<400> 107
aagcctagga agatttcccc accccacccc accccgcccc atcatctcca agaccccctc 60

gtgatgtgga ggaagagcca cctgcaagat ggacgcgagc cacaagctgc actgtgaaac 120

ccgggcactc cgagccgatg ccaccggccc gcgggtctct gaaggggacc cctccactaa 180

tcggactgcc aaatttcacc ggtttgccct gggatattat agaaaattat ttgtatgatt 240

gatgaaaata aaaacacctc gtggcatggt t 271

<210> 108
<211> 116
<212> DNA
<213> Artificial Sequence

<220>
<223> Mus musculus Nenf 3'-UTR
NM_025424

<400> 108
tgtctagctg agaagcagcc ggttctaggg agaagtgagg ggacaggagt taagtgtccc 60

tcggaacaag cggaggaagc ctccgagtgc cctgcagctg aataaagcga atgttt 116

<210> 109
<211> 123
<212> DNA
<213> Artificial Sequence

<220>
<223> Mus musculus Atp5k 3'-UTR
NM_007507

<400> 109
ggcgtcagcg agcttgcttt tctctagtcg ttgagaacga ataaagcttc attgtgtgaa 60

aaaaaaaaaa aaaaaaaaaa aaaaaaaaaa aaaaaaaaaa aaaaaaaaaa aaaaaaaaaa 120

aaa 123

<210> 110
<211> 260
<212> DNA
<213> Artificial Sequence

<220>
<223> Mus musculus 1110008P14Rik 3'-UTR
1110008P14Rik-001 ENSMUST00000048792

<400> 110
gtgccgggag cccccatcca ggccctaccc tcacctctct aggccatgtt ctggcctggg 60

tagatactac ttggcttaga caccatctcg ggtactggcc tccagatcct agtgggtcta 120

ccagcctgga ccagtcccca ttcactgccc atcacccttc ctggagtcag gtgcaatcct 180

acagttctcc cacttgtctg tcttctttcc cctccatcca gactgagagt ccgaattaaa 240

gatgtctccc acaccactgc 260

<210>  111
<211>  102
<212>  DNA
<213>  Artificial Sequence

<220>
<223>  Mus musculus Cox4i1 3'-UTR
NM_009941

<400>  111
gagcccgctg cctgccggct ccctgcctcc ctcactccct cggcatgctg gaagctgccg    60

tatccaatgg tccatgctaa taaaagacca gtttacgtgg tg    102

<210>  112
<211>  189
<212>  DNA
<213>  Artificial Sequence

<220>
<223>  Mus musculus Cox6a1 3'-UTR
NM_007748

<400>  112
agagaacctg gcctccccca ggcaacaaag ggaccacagc actggttttg gacccttact    60

ctgtgtggac cacgaaaacc ctttggatgc taagctcgtg tctcctttcc tcagatggcg    120

accattactc tgatcttcca tcccttctgc ttgtaagagg agatgcctta aataaataac    180

ttaaactca    189

<210>  113
<211>  139
<212>  DNA
<213>  Artificial Sequence

<220>
<223>  Mus musculus Ndufs6 3'-UTR
NM_010888

<400>  113
tgtgggctgt gtcctggtcc tctgactcct atggaacatc tccacgctgg gtgttctgtg    60

tgaggccact gctctgtgaa tggtgtccct tgttttgaat aaaggatgct cccaccatga    120

aaaaaaaaaa aaaaaaaaa    139

<210>  114
<211>  171
<212>  DNA
<213>  Artificial Sequence

<220>
<223>  Mus musculus Sec61b 3'-UTR
NM_024171

<400>  114

attgggctac atccatctgt catctgaaga agaagaagaa ggaaaaaaac ccaacatatc 60

ttggaccaaa agtgtagtga ttttctgttc acgtgtatta ttttacagag aataagaatt 120

gactttgaga aatcagtttt ttctatggct aataaacttt ggaattgctt t 171

<210> 115
<211> 101
<212> DNA
<213> Artificial Sequence

<220>
<223> Mus musculus Romo1 3'-UTR
NM_025946

<400> 115
ttagggctag gatgccctgc aatacctaaa cttccccatc catttcgacc cttgtacaat 60

aataaagttg ttttcttctc gttaaaaaaa aaaaaaaaaa a 101

<210> 116
<211> 370
<212> DNA
<213> Artificial Sequence

<220>
<223> Mus musculus Gnas 3'-UTR
NM_010309

<400> 116
gaagggaaca cccaaattta attcagcctt aagcacaatt aattaagagt gaaacgtaat 60

tgtacaagca gttggtcacc caccataggg catgatcaac accgcaacct ttcctttttc 120

ccccagtgat tctgaaaaac ccctcttccc ttcagcttgc ttagatgttc caaatttagt 180

aagcttaagg cggcctacag aagaaaaaga aaaaaaaggc cacaaaagtt ccctctcact 240

ttcagtaaat aaaataaaag cagcaacaga aataaagaaa taaatgaaat tcaaaatgaa 300

ataaatattg tgttgtgcag cattaaaaaa tcaataaaaa ttaaaaatga gcaaaaaaaa 360

aaaaaaaaaa 370

<210> 117
<211> 96
<212> DNA
<213> Artificial Sequence

<220>
<223> Mus musculus Snrpd2 3'-UTR
NM_026943

<400> 117
agcctgctcc ctgccctgcg aaggcctgca gaaccctgcc cagtgggcga gaaataaaac 60

cctgtgcttt ttggttaaaa aaaaaaaaaa aaaaaa 96

<210> 118

<211> 119
<212> DNA
<213> Artificial Sequence

<220>
<223> Mus musculus Mgst3 3'-UTR
NM_025569

<400> 118
ggtgtggagg gccttccgac tctcactcac ctccagcgac tcaccctgat ttccagttgc 60

actggttttt tttttttttt taatataata aaaacttatc tggcatcagc ctcatacct 119

<210> 119
<211> 304
<212> DNA
<213> Artificial Sequence

<220>
<223> Mus musculus Aldh2 3'-UTR
NM_009656

<400> 119
agcggcatgc ctgcttcctc agcccgcacc cgaaaaccca acaagatata ctgagaaaaa 60

ccgccacaca cactgcgcct ccaaagagaa accccttcac caaagtgtct tgggtcaaga 120

aagaatttta taaacagggc ggggctggtg ggggggaaag ctcctgataa actgggtagg 180

ggatgaagct caatgcagac cgatcacgcg tccagatgtg caggatgctg ccttcaacct 240

gcagtcccta agcagcaaat gagcaataaa aatcagcaga tcaaagccac ggggtcagtt 300

ctct 304

<210> 120
<211> 134
<212> DNA
<213> Artificial Sequence

<220>
<223> Mus musculus Mp68 (2010107E04Rik) 3'-UTR
NM_027360

<400> 120
ctgctccgaa tccacaagat gaagacgtcg gctaaacttg agcaagcttt gttagatggg 60

aacatggaac atcactgtac acttatctaa gtaccattta taatgtggca ttaataaatg 120

tatctgtgaa tacc 134

<210> 121
<211> 87
<212> DNA
<213> Artificial Sequence

<220>
<223> Mus musculus Ssr4 3'-UTR
NM_001166480

<400> 121
gggcagcaac ttcagccgtc cattgcttct ttcaataaac agtcactatt tgacatgagt 60

acattcaaga aaaaaaaaaa aaaaaaa 87

<210> 122
<211> 186
<212> DNA
<213> Artificial Sequence

<220>
<223> Mus musculus Myl6 3'-UTR
NM_010860

<400> 122
ggacattctg tatcccgagt ctgttccttg cccagtgtga tttctgtgtg gctccagagg 60

ctcccctgtc acagcacctt gcccatttgg tttcttttgg atgatgtttg ccttccccaa 120

ataaaatttg ctctctttgc cctccaaaaa aaaaaaaaaa aaaaaaaaaa aaaaaaaaaa 180

aaaaaa 186

<210> 123
<211> 58
<212> DNA
<213> Artificial Sequence

<220>
<223> Mus musculus Prdx4 3'-UTR
Prdx4-001, NM_016764

<400> 123
aaagtacttc agttatgatg tttggacctt ctcaataaag gtcattgtgt tattacca 58

<210> 124
<211> 130
<212> DNA
<213> Artificial Sequence

<220>
<223> Mus musculus Ubl5 3'-UTR
NM_025401

<400> 124
agggggattc cttctcctcc tcgccctgct ctgccctgcc ctcctctccc atcctcatct 60

gacactggtg tagatggtca tttttaacag ttcacatgaa taaaaacttg gctgctgctt 120

tgctgctgtc 130

<210> 125
<211> 87
<212> DNA
<213> Artificial Sequence

<220>
<223> Mus musculus 1110001J03Rik 3'-UTR
NM_025363

<400>  125
tgcagagagt cctcagatgt tccttcattc aagagtttaa ccatttctaa caatatgtag    60

ttatcattaa atctttttta aagtgtg    87

<210>  126
<211>  33
<212>  DNA
<213>  Artificial Sequence

<220>
<223>  Mus musculus Ndufa13 3'-UTR
Ndufa13-201 ENSMUST00000110167

<400>  126
ggcctgagcc aacgcacata ataaagagtg gtc    33

<210>  127
<211>  69
<212>  DNA
<213>  Artificial Sequence

<220>
<223>  Mus musculus Ndufa3 3'-UTR
NM_025348

<400>  127
atgcctctgc tgatggaaga ggccccttcc ctgttgctct ccaataaaaa tgtgaaaact    60

aataacccc    69

<210>  128
<211>  96
<212>  DNA
<213>  Artificial Sequence

<220>
<223>  Mus musculus Gstp2 3'-UTR
NM_181796

<400>  128
tggactgaag agacaagagc ttcttgtccc cgttttccca gcactaataa agtttgtaag    60

acaaaaaaaa aaaaaaaaaa aaaaaaaaaa aaaaaa    96

<210>  129
<211>  89
<212>  DNA
<213>  Artificial Sequence

<220>
<223>  Mus musculus Tmem160 3'-UTR
NM_026938

<400>  129
acaacagggc tgtggggact ggctgggcct gacgactggg acattaaaac ctgacccttc    60

cgcaaaaaaa aaaaaaaaaa aaaaaaaaa    89

<210> 130
<211> 129
<212> DNA
<213> Artificial Sequence

<220>
<223> Mus musculus Ergic3 3'-UTR
NM_025516

<400> 130
ctctctccct tccccacagc ttgtcctgcc ctctcttccc ctgtgggttt accctccagc 60

ctgtcaacta cccatatcct ctcctcagcc agcccagccc agggcaataa atatgaattg 120

tgataggaa 129

<210> 131
<211> 295
<212> DNA
<213> Artificial Sequence

<220>
<223> Mus musculus Pgcp 3'-UTR
NM_018755

<400> 131
ggagaacaag aagagaggac cttgttctct gtagttggga atcccaactc tgaatcttta 60

caacatccat cgtcacaaaa gagtgttata catttaatcc acagggcata gttttcttta 120

taccttctgt taatcatctt tccttaatac tttcttatct gtttctagaa taaatcatga 180

tccctactgc accaccttga aaatgttgtt tccagtttta aaataagcaa taaatatttg 240

aaatgcttct gatttttcat tttcatttaa aaacattaaa ttaaatgtaa tgaga 295

<210> 132
<211> 263
<212> DNA
<213> Artificial Sequence

<220>
<223> Mus musculus Slpi 3'-UTR
NM_011414

<400> 132
gcctgatccc tgacattggc gccggctctg gactcgtgct cggtgtgctc tggaaactac 60

ttccctgctc ccaggcgtcc ctgctccggg ttccatggct cccggctccc tgtatcccag 120

gcttggatcc tgtggaccag ggttactgtt ttaccactaa catctccttt tggctcagca 180

ttcaccgatc tttagggaaa tgctgttgga gagcaaataa ataaacgcat tcatttctct 240

atgcaaaaaa aaaaaaaaaa aaa 263

<210> 133
<211> 241

<212> DNA
<213> Artificial Sequence

<220>
<223> Mus musculus Myeov2 3'-UTR
NM_001163425

<400> 133
ggccgcccgg tcctatgtgc tccatgtctg tgatgtgtct ggagtctctc gggacacgac 60

cagctgattg tagacaccgt gttgatatca ctagaaatga agaccttgtc aaccaataga 120

ggaactgtct gaaccaactg ggtactgatg tctctgggaa tgccagcccg tgtccttgtt 180

taagttaata aagaacactg taacacgcag ggtgatttta aaaaaaaaaa aaaaaaaaaa 240

a 241

<210> 134
<211> 162
<212> DNA
<213> Artificial Sequence

<220>
<223> Mus musculus Ndufa4 3'-UTR
NM_010886

<400> 134
actatgaagt tcactgtaaa gctgctgata atgaaggtct ttcagaagcc atccgcacaa 60

ttttccactt aagcaggaaa tatgtctctg aatgcatgaa atcatgttga tttttttttt 120

ttttggagtt tattacactg atgaataaat ctctgaaact tg 162

<210> 135
<211> 143
<212> DNA
<213> Artificial Sequence

<220>
<223> Mus musculus Ndufs5 3'-UTR
NM_001030274

<400> 135
gcggggcagc tggaggccgc tgtcatgctc tgttttcccc tggagagaat atttaaggaa 60

agctccttca ttaagtatta agtatgtgga aataaagaat tactcagtct taaaaaaaaa 120

aaaaaaaaaa aaaaaaaaaa aaa 143

<210> 136
<211> 455
<212> DNA
<213> Artificial Sequence

<220>
<223> Mus musculus Gstm1 3'-UTR
NM_010358

<400> 136

gcccttgcta cacgggcact cactaggagg acctgtccac actggggatc ctgcaggccc 60

tgggtgggga cagcaccctg gccttctgca ctgtggctcc tggttctctc tccttcccgc 120

tcccttctgc agcttggtca gccccatctc ctcaccctct tcccagtcaa gtccacacag 180

ccttcattct ccccagtttc tttcacatgg ccccttcttc attggctccc tgacccaacc 240

tcacagcccg tttctgcgaa ctgaggtctg tcctgaactc acgcttccta gaattacccc 300

gatggtcaac actatcttag tgctagccct ccctagagtt accccgaagg tcaatacttg 360

agtgccagcc tgttcctggt ggagtagcct ccccaggtct gtctcgtcta caataaagtc 420

tgaaacacac ttgccatgaa aaaaaaaaaa aaaaa 455

<210> 137
<211> 109
<212> DNA
<213> Artificial Sequence

<220>
<223> Mus musculus 1810027O10Rik 3'-UTR
1810027O10Rik-001 ENSMUST00000094065

<400> 137
agtctcttgt ttaagcgccc agtcctggcc tttctgggta attgggcgca gagggaagga 60

gccaatgttg aagcagaaaa gaaattaaaa gaaaaaggca tataaagaa 109

<210> 138
<211> 49
<212> DNA
<213> Artificial Sequence

<220>
<223> Mus musculus 1810027O10Rik 3'-UTR
BC117077

<400> 138
agtctcttgt ttaagcgccc agtcctggcc tttctgggta attgggcgc 49

<210> 139
<211> 55
<212> DNA
<213> Artificial Sequence

<220>
<223> Mus musculus Atp5o 3'-UTR
NM_138597

<400> 139
gagactgtca cctgtgtgag ctcttgtcct tggagcaaca ataaaatgct tcctg 55

<210> 140
<211> 174
<212> DNA
<213> Artificial Sequence

<220>
<223> Mus musculus Shfm1 3'-UTR
NM_009169

<400> 140
catctgggaa tgtcccagga acctcaatca tggactctac cacagtctag gacagagaaa 60

gcaggacggg atactttaaa gaacatgttt atttcattat ctgcttcaat ttatttttgt 120

tttataacaa aaaaaataag taaataaatg ttttgattta atctttttgg ttca 174


<210> 141
<211> 260
<212> DNA
<213> Artificial Sequence

<220>
<223> Mus musculus Tspo 3'-UTR
NM_009775

<400> 141
aggcacccag ccatcaggaa tgcagccctg ccagccaggc accatgggtg gcagccatca 60

tgcttttatg accattgggc ctgctggtct acctggtctt agcccaggaa gccaccaggt 120

aggttagggt ggtcagtgcc gagtctcctg cagacacagt tatacctgcc tttctgcact 180

gctccaggca tgcccttaga gcatggtgtt ttaaagctaa ataaagtctc taacttcatg 240

tgtaaaaaaa aaaaaaaaaa 260


<210> 142
<211> 92
<212> DNA
<213> Artificial Sequence

<220>
<223> Mus musculus S100a6 3'-UTR
NM_011313

<400> 142
aatgggaccg ttgagatgac ttccgggggc ctctctcggt caaatccagt ggtgggtagt 60

tatacaataa atatttcgtt tttgttatgc ct 92


<210> 143
<211> 200
<212> DNA
<213> Artificial Sequence

<220>
<223> Mus musculus Taldo1 3'-UTR
NM_011528

<400> 143
tgcaacaccc gaggccccag tcctgcaccg aggctgaccc cagacctgca ctgcctttga 60

gctgggtcct aattgcacat ggcttgtgac gaatgaatct tgcatttttt agtgatcgga 120

gaagggatgg atcataggat tctgatttta tgtgaaattt tgtctaattc attaaagcag 180

ttgcttttcc tatgctgttt 200

<210> 144
<211> 83
<212> DNA
<213> Artificial Sequence

<220>
<223> Mus musculus Bloc1s1 3'-UTR
NM_015740

<400> 144
actaaaaccc accctctta cttcaccctc ctggacagga gggaaactgg tgagccacga 60

ataaaaacac aagcttccat tct 83

<210> 145
<211> 93
<212> DNA
<213> Artificial Sequence

<220>
<223> Mus musculus Ndufb11 3'-UTR
NM_019435

<400> 145
tggcttaccg agcagggcct aagaagcatt actcatccgc tgcttgttat ttacctggtt 60

cctcagaaca ccttattaaa ggaattgaaa gta 93

<210> 146
<211> 454
<212> DNA
<213> Artificial Sequence

<220>
<223> Mus musculus Map1lc3a 3'-UTR
NM_025735

<400> 146
gtcaagagga ggggaggggg gtggctggga gttctggtca ggttctcccc agggaggtcc 60

tggctcctaa actaagctat ttcagtcccc agtggattag gcagagatgt gacacccact 120

ccccccccca ggtaggggcc accagccagc ctaccacatc ctgggtaggt cctgggccag 180

tcatgttcgg gttgctcttt tgggtgctgg ctgggttggg agtgggtggg gagcagcatc 240

cctgctctgt ggggtttgtc attttgttag gcccttgcct gtctgcccat cttgcccctc 300

atccacctga ggctttgcct cctgccagga cctgccccac ccctgaaagg ctggctcccc 360

ttgtcctgac tcggtgtatg gatctgtggt catttcctct gcagaaagaa taaagactgc 420

tcaggcctgc ctggccaaaa aaaaaaaaaa aaaa 454

<210> 147
<211> 135

<212> DNA
<213> Artificial Sequence

<220>
<223> Mus musculus Morn2 3'-UTR
NM_194269

<400> 147
acctgctgcc ttaacgctga gatgtggcct ctgcaacccc ccttaggcaa agcaactgaa 60

ccttctgcta aagtgacctg ccctcttccg taagtccaat aaagttgtca tgcacccaca 120

aaaaaaaaaa aaaaa 135

<210> 148
<211> 238
<212> DNA
<213> Artificial Sequence

<220>
<223> Mus musculus Gpx4 3'-UTR
NM_008162.2

<400> 148
ctagccctac aagtgtgtgc ccctacaccg agcccccctg ccctgtgacc cctggagcct 60

tccaccccgg cactcatgaa ggtctgcctg aaaaccagcc tgctggtggg gcagtcctga 120

ggacctggcg tgcatccctg ccggaggaag gtccagaggc ctgtggccct gggctcgagc 180

ttcaccctgg ctgccttgtg ggaataaaat gtagaaatgt gaaaaaaaaa aaaaaaaa 238

<210> 149
<211> 124
<212> DNA
<213> Artificial Sequence

<220>
<223> Mus musculus Mif 3'-UTR
NM_010798.2

<400> 149
gtcctggccc cacttacctg caccgctgtt ctttgagcct cgctccacgt agtgttctgt 60

gtttatccac cggtagcgat gcccaccttc cagccgggag aaataaatgg tttataagag 120

acca 124

<210> 150
<211> 135
<212> DNA
<213> Artificial Sequence

<220>
<223> Mus musculus Cox6b1 3'-UTR
NM_025628

<400> 150
cctggctccg cccacctctc ctctgttctt tgtctttctc cccggataga aaagggggac 60

ctcagcatat gatggtcctt accctgggac cctgaatcat gatgcaacta ctaataaaaa 120

ctcactggaa aagtt 135

<210> 151
<211> 267
<212> DNA
<213> Artificial Sequence

<220>
<223> Mus musculus RIKEN cDNA2900010J23 (Swi5) 3′-UTR
NM_175190

<400> 151
gcagcttctt ggagattttc atctacagcc cacagggaca ggaggatggg ggcataaaag 60

gcagagtcta gacagtatgt tcatatggtt ttcagatttt aaaagatgct agaagccctc 120

caaagtttgg ggtgggttct agagaagagg agtattggga ggggtgggta ttgtcaatgt 180

taaggttcct aaacatactt gtgagtaggt gtgtgtggtt gtcccttttg ttaataaaca 240

tatgagcagt caaaaaaaaa aaaaaaa 267

<210> 152
<211> 160
<212> DNA
<213> Artificial Sequence

<220>
<223> Mus musculus Sec61g 3′-UTR
NM_011343.3

<400> 152
gtccttctca tcatgggacg agtgagccag agcgggggaa agggcatgaa gtaaagcgtt 60

gcctgaatgc tgtgtggtgt tttgtttctt cctccttcct atgaggtttt ctacttctca 120

attaaaataa tttcaaaata aacacttttt ccataacaga 160

<210> 153
<211> 62
<212> DNA
<213> Artificial Sequence

<220>
<223> Mus musculus 2900010M23Rik 3′-UTR
BC_030629

<400> 153
ccgtggggtc tgatactcat caataaaact gcctggtttc tcccacaaaa aaaaaaaaaa 60

aa 62

<210> 154
<211> 338
<212> DNA
<213> Artificial Sequence

<220>
<223> Mus musculus Anapc5 3'-UTR
Anapc5-201 ENSMUST00000086216

<400> 154
ccaggactcc ctgcttgatg gtgtgcattt aggggtgggt cattacatgc tatcttgtca 60

ataaactgtt ctgatcagtt tgtctgaagt gggtttttttt ttatttttct gggttgaatt 120

gtcagtatct ttgttaagaa ctgtgtatct aggggctgga gagatggctt agcagttaag 180

agcactaact gttcttctaa aggacctggg ttcaattcct agcaccctca tgacagctca 240

cagctgtctg taactcctgt tccagggact ctgacaccct caggcagaca taaaagcagt 300

caaaacaccg atgtacataa aattaaaata aattattt 338

<210> 155
<211> 71
<212> DNA
<213> Artificial Sequence

<220>
<223> Mus musculus Mars2 3'-UTR
BC132343.1

<400> 155
gaactcagct cttactgact ggtagtaaaa gatcaaatgt attcttttg cgtttttaag 60

taaagtcatg c 71

<210> 156
<211> 176
<212> DNA
<213> Artificial Sequence

<220>
<223> Mus musculus Phpt1 3'-UTR
NM_029293

<400> 156
agctctgccc caccccccac cccccggact aagtcaggtc tctgctcttg ctgtgttctg 60

ttttgagggg ctggccctgt gctttccttt tgtaccttag gcagcatagc acctgccagg 120

ccttagaggc cagaccaatc tggtccatag gaattaaaag cattgatatg cctact 176

<210> 157
<211> 66
<212> DNA
<213> Artificial Sequence

<220>
<223> Mus musculus Ndufb8 3'-UTR
NM_026061

<400> 157
ggaggcttga tgggcttttt gccctcgttc ctagaggctt aaccataata aaatccctaa 60

taaagc 66

<210> 158
<211> 170
<212> DNA
<213> Artificial Sequence

<220>
<223> Mus musculus Pfdn5 3'-UTR
NM_027044

<400> 158
gagtgcactg cagaaatgaa gcagagtgag ggacccttct tcaaggggcc tgggactttt 60

tccggcaatg gcctcctggg aaagtggcct gggaagagag tgttttgtgt ttaatgttaa 120

taaatgtgac cgctgcgcaa aaaaaaaaaa aaaaaaaaaa aaaaaaaaaa 170

<210> 159
<211> 278
<212> DNA
<213> Artificial Sequence

<220>
<223> Mus musculus Arpc3 3'-UTR
NM_019824

<400> 159
gaggagcctg ggcagcacca tcacgtggag acacatcata ggacacacag gccaatgtgt 60

ctgttcatac ctaccgtatc aaggagagaa gagagcctgt ctttgctgga aaagctcttg 120

gtcaagaatt gggagggtgg gtgttgggcg atttcgattt ttggcagttt taagctggta 180

cttaatatat aataaatgtc actgcttatg ttagacattg aattaaaaca tttttgagaa 240

aaagctttaa aaaaaaaaaa aaaaaaaaaa aaaaaaaa 278

<210> 160
<211> 58
<212> DNA
<213> Artificial Sequence

<220>
<223> Mus musculus Ndufb7 3'-UTR
NM_025843

<400> 160
ggattacccg ccagcctgtg gacctatcag tgaaataaaa gctttgggtc acctgcct 58

<210> 161
<211> 51
<212> DNA
<213> Artificial Sequence

<220>
<223> Mus musculus Atp5h 3'-UTR
NM_027862

<400> 161

agcagcctgg gacggagccc cggccgacat gaaataaaac atttaaatag t 51

<210> 162
<211> 80
<212> DNA
<213> Artificial Sequence

<220>
<223> Mus musculus Mrpl23 3'-UTR
NM_011288

<400> 162
cctatgacag caggatttgg accacagacc ctagtgagca cagtggttct gacaagccca 60

aataaaaatt ctttgtggag 80

<210> 163
<211> 387
<212> DNA
<213> Artificial Sequence

<220>
<223> Mus musculus Tomm6 3'-UTR
NM_025365.3

<400> 163
ccagagaatg gaactcctgt gtattcagac tttccaaaga cagcctactg tctgtgacca 60

caagatccta cctgagtggc agctgaagtt gactccctct ccttgcctga accccccccc 120

actgcccccc catcccccag tgtcggctga gatgttgcct ctgcacggtt ctgtgtgcag 180

ttcccaactt tctgcagaag atggtccttg cccttgtcct gaagagtagt aatggttctt 240

gaaaaagatt tcaaataaag cctgcacata aaagacaggt attttattct tttaataaga 300

aacttattac aaaaacaagg tgtaaaaagt ccgcttacaa aaatcaaata aacatgactt 360

gtatttcaaa aaaaaaaaaa aaaaaaa 387

<210> 164
<211> 327
<212> DNA
<213> Artificial Sequence

<220>
<223> Mus musculus Tomm6 3'-UTR
Tomm6-002 ENSMUST00000113301

<400> 164
ccaggtgaga gcagttctcc tgtgtttccc cgtttctgat gctgttatct gcttacagag 60

aatggaactc ctgtgtattc agactttcca aagacagcct actgtctgtg accacaagat 120

cctacctgag tggcagctga agttgactcc ctctccttgc ctgaaccccc ccccactgcc 180

cccccatccc ccagtgtcgg ctgagatgtt gcctctgcac ggttctgtgt gcagttccca 240

actttctgca gaagatggtc cttgcccttg tcctgaagag tagtaatggt tcttgaaaaa 300

gatttcaaat aaagcctgca cataaaa 327

<210> 165
<211> 273
<212> DNA
<213> Artificial Sequence

<220>
<223> Mus musculus Tomm6 3'-UTR

<400> 165
ccagagaatg gaactcctgt gtattcagac tttccaaaga cagcctactg tctgtgacca 60

caagatccta cctgagtggc agctgaagtt gactccctct ccttgcctga accccccccc 120

actgcccccc catcccccag tgtcggctga gatgttgcct ctgcacggtt ctgtgtgcag 180

ttcccaactt tctgcagaag atggtccttg cccttgtcct gaagagtagt aatggttctt 240

gaaaaagatt tcaaataaag cctgcacata aaa 273

<210> 166
<211> 631
<212> DNA
<213> Artificial Sequence

<220>
<223> Mus musculus Mtch1 3'-UTR
NM_019880

<400> 166
cctaagctgc ccgaccaaac atttatgggg tcttagccta cccctggtga ggacccatca 60

tctcagatgc ccaagggtga ctccagccca gcctggcttc atgtccatat ttgccatgtg 120

tctgtccaga tgtgggctgg tggaggtggg tcacctggga cctggggaag cctgggggag 180

cagtgttggg gtggcatccc cttcctgcct agaggtactg gagtccatct tgtactcagg 240

cagaggcagg ctgcagaggc aaacgtcact cagtggcaag gcttccctgc acctctagcc 300

cagctcatcc tgccagtcag ccagaagcac ccccgccccc cacttcctgc tttgtaaatt 360

gggcgccatc acacctgggc catgggaggc tggagctatg ttcccaacac taattttctt 420

atacaagggt ggtgccttct cctgaatagg aaatcatgtt ctcctcagac catcccctca 480

tctgcttgtc tgtgctggtg acgccaggtg tgagggttca gtcactgtgc tgggtgcgaa 540

tacgcacagg ttacataggc cgacatctag tcctcccctc gtggtaagat agacccatct 600

cctcgaataa atgtattggt ggtgatttgg a 631

<210> 167
<211> 158
<212> DNA
<213> Artificial Sequence

<220>
<223> Mus musculus Pcbd2 3'-UTR

NM_028281

<400> 167
tctgcgcctg ccttgtctgc agcgttgttt gcaagccact tatgttaata aattgtcata 60

aagtagttca tagttacatg tatacattgt tgtatgattg atgctcaaat acagaatgat 120

ttgaagccaa aaaaaaaaaa aaaaaaaaaa aaaaaaaa 158

<210> 168
<211> 93
<212> DNA
<213> Artificial Sequence

<220>
<223> Mus musculus Ecm1 3'-UTR
NM_007899

<400> 168
gtcaccctga gcctcagagg attagatggg ggaactccgc cctactccac cctcctcgaa 60

cactcattac aataaatgcc tcttggattt ggc 93

<210> 169
<211> 458
<212> DNA
<213> Artificial Sequence

<220>
<223> Mus musculus Hrsp12 3'-UTR
Hrsp12-001 ENSMUST00000022946

<400> 169
ctataagtag ccatgctgat gttgactccg gaggttttag aatgtctttc acactttaat 60

ttttacaaat gatgctggga agtataaaaa tgaccagagt ggttgaagtt attgtggaag 120

tgatcaaata tgtggagatt tgacattaat tggagattat tcagtatagt gactgatgtt 180

ctaatttcac ttatgttgct gggtgtgaga gaagaggtgc acagctactg agatgggaag 240

cagaaggaaa gatgggctgt tgtacatgag aaatagtaag gagcacatct acttaaatca 300

tattaatttg ctcatgtgaa atacttagtt cttatgttag atataagaaa ctaaattgaa 360

atattcaaac ttgaatagta ccaggagaac aagtggacca aaatcttata cagataatat 420

tactttaatt gaaataaaaa atagatgtgt aactttcc 458

<210> 170
<211> 183
<212> DNA
<213> Artificial Sequence

<220>
<223> Mus musculus Mecr 3'-UTR
NM_025297

<400> 170
ttgctccaga ggaccaggag gaaagcagga gaggcaagac tggctgtctg ctggcccctc 60

catgagaacc ccagccttcc cagactgcct cacccatatt gtctcttcct accaggaggg 120

tgggggacca actctaggct ccctaataaa cccttaactt cccgagtgga ggatgaagag 180

tac 183

<210> 171
<211> 120
<212> DNA
<213> Artificial Sequence

<220>
<223> Mus musculus Uqcrq 3'-UTR
NM_025352

<400> 171
acggcctgca cctgggtgac agtcccctgc ctctgaaaga cccttctctg ggagaggaat 60

ccacactgta gtcttgaaga caataaacta cttatggact tccctttgaa aaaaaaaaaa 120

<210> 172
<211> 511
<212> DNA
<213> Artificial Sequence

<220>
<223> Mus musculus Gstm3 3'-UTR
NM_010359

<400> 172
gcccctgcca tgctgtcact cagagtgggg gacctgtcca tactgcggat cctgcaggct 60

ctgggtgggg acagcaccct ggccttctgc actgtggctc ccggttctct ctccttcccg 120

ctcccttctg cagcttggtc agccccatct cctcatcctc accccagtca agcccatgca 180

gcctttattc tccccatttt tttttcacat ggccccttct tcattggtgc ccagacccaa 240

cctcacagcc cttttctgca atctgaggtc tgtcctgaac tcaggctccc tagagttacc 300

ccaatggtca acactatctt agtgccagcc ctccctagag ataccctgat ggtcaatact 360

atcttagtga cggccctccc tagagttacc ctgaaggtca atactcgagt gccagcctgt 420

tcctgtttaa ggagctgccc caggcctgtc tcatgtacaa taaagcctga aacacacttg 480

aaacacaata aacactgaac acttgctgtg a 511

<210> 173
<211> 312
<212> DNA
<213> Artificial Sequence

<220>
<223> Mus musculus Lsm4 3'-UTR
NM_015816

<400> 173
tcactccctg cctgagccga gcccagaacg gtgggtgagg cctcagggca cctttgtgtg 60

aagccccact tggcgtctgg tccagtgaag tccctcgctg gccactgact cagtttctgg 120

aaggttccga gtctgaggtg cctgtggagc cttagatgcc ctttgaaggg ctgacttctt 180

ccaggcatgt ttgagtttca gttggagctg caggctcagc ccatggcggc tcacctgtcc 240

tttaccagcc ataccctgta catcttctgt ttgaaaaata aaagcaaaca ccatagaaag 300

aaaaaaaaaa aa 312

<210> 174
<211> 195
<212> DNA
<213> Artificial Sequence

<220>
<223> Mus musculus Park7 3'-UTR
NM_020569

<400> 174
agcccaagcc ctgggcccca cgcttgagca ggcattggaa gcccactggt gtgtccagag 60

cccagggaac ctcagcagta gtatgtgaag cagccgccac acggggctct catcccgggt 120

ctgtatgttt ctgaaccttg ctagtagaat aaacagttta ccaagctcct gccagctaaa 180

aaaaaaaaaa aaaaa 195

<210> 175
<211> 131
<212> DNA
<213> Artificial Sequence

<220>
<223> Mus musculus Usmg5 3'-UTR
NM_023211

<400> 175
atggattttg aaatgtctga cctcacctgt taagtcccat gcctgaagaa gctgatgtga 60

actcatcatg taatactcaa tttgtacaat aaattatgaa cccaaaaaaa aaaaaaaaaa 120

aaaaaaaaaa a 131

<210> 176
<211> 231
<212> DNA
<213> Artificial Sequence

<220>
<223> Mus musculus Cox8a 3'-UTR
NM_007750

<400> 176
agggagcagt cttccctcat cctttgacta gaccactttt gccagcccac cttgatcatg 60

ttgcctgcat tcctggctgg ccttccccgg gatcatgtta ttcaattcca gtcacctctt 120

ctgcaatcat gacctctcga tgtctccatg gtgacaactg ggaccacatg tattggctct 180

gcttggtggg gtcccccttt gtaacaataa agtctattta aaccttgctc c 231

<210> 177
<211> 403
<212> DNA
<213> Artificial Sequence

<220>
<223> Mus musculus Ly6c1 3'-UTR
NM_010741

<400> 177
tggtccttcc aatgaccccc acccttttcc ttttatcttc atgtgcaacc actctttcct 60

ggagtcctct agtgacaaat tatatgttat agaaggtcca atgtggggat agtgtgtgga 120

acaccctgtt tcacctttat agcccctgct gggtaagtgc ccgactcctc tctagggctt 180

tcaaatctgt acttcttgca atgccattta gttgtggatt tctattcttg gccctggagg 240

catgtggcca gcacatgcaa caggcagtat tccaaggtat tatagtatca ccatccacac 300

ataagtatct ggggtcctgc agggttccca tgtatgcctg tcaatgaccc ctgttgagtc 360

caataaaagc tttgttctcc cagccaaaaa aaaaaaaaaa aaa 403

<210> 178
<211> 309
<212> DNA
<213> Artificial Sequence

<220>
<223> Mus musculus Ly6c1 3'-UTR
NM_001252058.1

<400> 178
actcataaaa atgctcctgc ctcggtcttc caagttctag gattgcaagt ctgacttcaa 60

catgccttac agacaactct gggacatcca ggcctagtgg catgttgccc agatatgggg 120

atgctctgtg gcccctgcat aagaagtgag tcactccctg atttcttgca gactctcaaa 180

gaaggaaact aaagacccgt cagtgccttt ctttctgccc tgctggtgtg ccaatcaggg 240

atcctaacat cagggagagg acttcctgtt gcagcgaaga cctctgcaat gcagcagttc 300

ccactgcag 309

<210> 179
<211> 802
<212> DNA
<213> Artificial Sequence

<220>
<223> Mus musculus Cox7b 3'-UTR
NM_025379

<400> 179
tcgtgccagc tggtacaata atcaaggaat tgtttaaaac caacttataa gtgaatgcca 60

agtcaaagaa tcatgtactc attatactat ggcagattga agaacaaata aagaaataaa 120

gtaccttaac cttcattcta ggctttgttt ttttcctttg taaatgaagc ccaagcatgg 180

tgacttctca tttatttaag ctgtattgtc tcttaaaatg gctttttacc ctatgaggtg 240

gtatgaggga aatctatgat caggagggca cctttatagt aagctgaaat tacagagaat 300

gaagaaataa gcacagagct gttttaggag cccactgggt cattggccat ataggttatg 360

cttactgccc tctacctcgt ggttatattt ggaattgcca ttagctccct tctgcttaga 420

gactggactg tcaccaaacc caaggggata gtgatcctgt aatgatcctg tgtgaactag 480

gtttgctaaa gactaccacc tccttacact gtatggcata ttcatctgaa ataggtgcta 540

atttttcagc ataatcctta atctttagga tctgtcatac ttcctagtaa tttaactgtt 600

gctgaagaaa taaaggctat ctgttaaaaa aaaaaaaaaa aaaaaaaaaa aaaaaaaaaa 660

aaaaaaaaaa aaaaaaaaaa aaaaaaaaaa aaaaaaaaaa aaaaaaaaaa aaaaaaaaaa 720

aaaaaaaaaa aaaaaaaaaa aaaaaaaaaa aaaaaaaaaa aaaaaaaaaa aaaaaaaaaa 780

aaaaaaaaaa aaaaaaaaaa aa 802

<210> 180
<211> 231
<212> DNA
<213> Artificial Sequence

<220>
<223> Mus musculus Ppib 3'-UTR
NM_011149

<400> 180
agagcctggg ggacctcatc cctctaagca gctgtctgtg tgggtcctgt caatccccac 60

acagacgaag gtagccagtc acaaggttct gtgccaccct ggccctagtg cttccatctg 120

atggggtgac cacacccctc acattccaca ggcctgattt ttataaaaaa ctaccaatgc 180

tgatcaataa agtgggtttt ttttatagct tgaaaaaaaa aaaaaaaaaa a 231

<210> 181
<211> 205
<212> DNA
<213> Artificial Sequence

<220>
<223> Mus musculus Bag1 3'-UTR
NM_009736

<400> 181
agtgcagtgg agagtggctg tactggcctg aagagcagct ttacagccct gccctctctg 60

gaacagaagt cgcctgtttc tccatggctg ccaggggcaa ctagccaaat gtcaatttcc 120

ctgctcctcc gtcggttctc aatgaaaaag tcctgtcttt gcaacctgaa ttagacttgt 180

gttttctcaa aaaaaaaaaa aaaaa 205

<210> 182
<211> 140
<212> DNA
<213> Artificial Sequence

<220>
<223> Mus musculus S100a4 3'-UTR
S100a4-201 ENSMUST00000001046

<400> 182
agactcctca gatgaagtgt tggggtgtag tttgccagtg ggggatcttc cctgttggct 60

gtgagcatag tgccttactc tggcttcttc gcacatgtgc acagtgctga gcaaattcaa 120

taaaaggttt tgaaactatt 140

<210> 183
<211> 374
<212> DNA
<213> Artificial Sequence

<220>
<223> Mus musculus Bcap31 3'-UTR
NM_012060

<400> 183
aggcttggtg tttccctgcc tgccgctggc ttctacctga cccatgctta ctgcttcctt 60

ggagcccaga ctatccctct ggtacttggg tttattccct acttccccaa ttttcttcca 120

tggcttatag atcattattt tggcaccatt acacatactg ctcttatacc aaaagggacc 180

tgattgttgt ttattcagag tacttttgcc actgttctgc ctggctaggg cactttccac 240

tcctggaagt gtagaaaagc actggtgacc tggcctgcag tttgaacccc tttttatttt 300

gcaatgtacc ctaaaggagg ctgctgtgaa gcaggtcaac tgttttatcc tgaggggaat 360

aaatgttgtt atgt 374

<210> 184
<211> 126
<212> DNA
<213> Artificial Sequence

<220>
<223> Mus musculus Tecr 3'-UTR
NM_134118

<400> 184
gcagctcctc acggctctgc ccagtaatac tctccacccc tcactgcccc tgtcctgatg 60

tgtggctggc catggctctc cagcagcaac aataaaacct gcttacccaa aaaaaaaaaa 120

aaaaaa 126

<210> 185

<211> 171
<212> DNA
<213> Artificial Sequence

<220>
<223> Mus musculus Rabac1 3'-UTR
NM_010261

<400> 185
agtgtcctcc aggacctgcc ggcctctcct gccggccggc tgtcccatct ctgtctgttc 60

tcgtcctacc tggccttgct gctcagctcc gagccttcca cctgaggcct caaacccagg 120

gaggggcttt tgtctttgga aataaagctg ttacaattgc tatttggcca a 171

<210> 186
<211> 144
<212> DNA
<213> Artificial Sequence

<220>
<223> Mus musculus Robld3 3'-UTR
NM_031248 (Lamtor2)

<400> 186
cagcgtgatg gaggctggag tagaaaaggg atgatgatct ggagggaggg gcggggccct 60

agaaacgcca tatcgggcga ggtacaggaa ggggggggttg ctttttctg aataaatttt 120

caactcttaa aaaaaaaaaa aaaa 144

<210> 187
<211> 80
<212> DNA
<213> Artificial Sequence

<220>
<223> Mus musculus Sod1 3'-UTR
NM_011434

<400> 187
acattccctg tgtggtctga gtctcagact catctgctac cctcaaacca ttaaactgta 60

atctgaaaaa aaaaaaaaaa 80

<210> 188
<211> 262
<212> DNA
<213> Artificial Sequence

<220>
<223> Mus musculus Nedd8 3'-UTR
NM_008683

<400> 188
agaaacttgg ttccgtttac ctccttgccc tgccaatcat aatgtggcat cacatatcct 60

ctcactctct gggacaccag agccactgcc ccctctcttg gatgcccaat cttgtgtgtc 120

tactggtggg agaatgtgag gaccccaggg tgcagtgttc ctggcccaga tggcccctgc 180

tggctattgg gttttagttt gcagtcatgt gtgcttccct gtcttatggc tgtatccttg 240

gttatcaata aaatatttcc tg 262

<210> 189
<211> 257
<212> DNA
<213> Artificial Sequence

<220>
<223> Mus musculus Higd2a 3'-UTR
NM_025933

<400> 189
gtatagccgg gtcttaaagc gccatggaaa ccattacaaa acccaggaac aacagacatc 60

cctgtcagac ttgctccctc cgtttcagac cggaccttat tgtcatttgg gtgaggaagt 120

ggccgatttt gtaactgatt tgcgcttcca ccgctgcccc ctcccgctcc caaaatccca 180

ggttcatttc agttgggttg catgcttcta tttgtgatgc gtccccttaa ttacttaata 240

aaagcttatt acacttg 257

<210> 190
<211> 268
<212> DNA
<213> Artificial Sequence

<220>
<223> Mus musculus Trappc6a 3'-UTR
Trappc6a-001 ENSMUST00000002112

<400> 190
ggaccccaga ccccaggctt gcccttccct aagcttagcc tcggaatgtg gcacctgacc 60

ctgcctcact gctcaccttt gcaggtcgcc ttgaagctgg agctcacagg ctctggggag 120

gtcacatgtg cttcagacaa gggaatgaaa gggccgggag ggtcccggga ggtgggacca 180

tcccctgagt tccaagtcag catggaggga cattagggca tcacccagat gacagatgtt 240

cagtaaaggt tctttatgtg caaacaga 268

<210> 191
<211> 188
<212> DNA
<213> Artificial Sequence

<220>
<223> Mus musculus Ldhb 3'-UTR
Ldhb-001 ENSMUST00000032373

<400> 191
ctgccagtct ctaggctgta gaacacaaac ctccaatgtg accatgaacc tttagtcttc 60

agccatgtat gtaggtcaca gtttgcttct tccctgacat gtgatatgag ctcacagatc 120

aaagcccagg cttgtttgat gtttgcacta ggagctcctg atcaaataaa gttagcaatt 180

gcagcata 188

<210> 192
<211> 112
<212> DNA
<213> Artificial Sequence

<220>
<223> Mus musculus Nme2 3'-UTR
Nme2-001 ENSMUST00000021217

<400> 192
acatgaagaa accagaatcc ttttcagcac tactgatggg tttctggaca gagctcttca 60

tcccactgac aggatggatc atcttttcta aaacaataaa gactttggaa ct 112

<210> 193
<211> 119
<212> DNA
<213> Artificial Sequence

<220>
<223> Mus musculus Snrpg 3'-UTR
NM_026506

<400> 193
cctgtgctca gcaagcagtg tccacatccc tccccaaagg cctgtttgat tgtgatgtag 60

aattaggtca tgtacatttt catatggaac tttttactaa ataaactttt gtgatactc 119

<210> 194
<211> 235
<212> DNA
<213> Artificial Sequence

<220>
<223> Mus musculus Ndufa2 3'-UTR
NM_010885

<400> 194
aggtctccac tgaggactgt gagcgagagc agctgaacct gctggactga agacagtgtg 60

gggaaatgtg tgctttgggt ccttataaag cttacgctgt acagtgtccc ttcagaatgt 120

cctcttcatt accttctccc tcttactgcg caacactgag gcaaagtagt tttatataaa 180

aatactcctt tatttctcct caaaaaaaaa aaaaaaaaaa acccaccagg tgcca 235

<210> 195
<211> 196
<212> DNA
<213> Artificial Sequence

<220>
<223> Mus musculus Serf1 3'-UTR
Serf1-003 ENSMUST00000142155

<400> 195

tgactggctt tttggaaaac ctgggtgcta ttgccagtgg gtgcatcata cgctctaaga 60

ttaaaatttc acagtgacta atcattatat gtgttataac ttgtccttat aaaactattt 120

taaactttac tcttcagcct atcttaatgt gatgttttaa gaccatcaaa aaataaagta 180

ctgaccttgc atgtaa 196

<210> 196
<211> 286
<212> DNA
<213> Artificial Sequence

<220>
<223> Mus musculus Oaz1 3'-UTR
Oaz1-001 ENSMUST00000180036

<400> 196
gtgccagccc tgcccagtgt ccctgtgccc tctcctgggt tagtccacat gtcgtgattg 60

tgcagaataa acgctcactc cattagcggg gtgcttcttc gagctgaatg ctgtgtttgt 120

cacactcaag tgttggcttt aattctaaat aaaggtttct attttacttt tttattgctg 180

tttaagatgg tcaggtgacc tatgctatag cagtctcctt tgaagtctgg aaaaatagtg 240

tcacctcccc tggctcaaat ccaataaagt gatctcgttc attggc 286

<210> 197
<211> 418
<212> DNA
<213> Artificial Sequence

<220>
<223> Mus musculus Ybx1 3'-UTR
Ybx1-001 ENSMUST00000079644

<400> 197
atgccggctt accatctcta ccatcatccg gtttggtcat ccaacaagaa gaaatgaata 60

tgaaattcca gcaataagaa atgaacaaag attggagctg aagaccttaa gtgcttgctt 120

tttgccctct gaccagataa cattagaact atctgcatta tctatgcagc atggggtttt 180

tattattttt acctaaagat gtctcttttt ggtaatgaca aacgtgtttt ttaagaaaaa 240

aaaaaaaaag gcctggtttt tctcaataca cctttaacgg tttttaaatt gtttcatatc 300

tggtcaagtt gagattttta agaacttcat ttttaatttg taataaagtt tacaacttga 360

ttttttcaaa aaagtcaaca aactgcaagc acctgttaat aaaggtctta aataataa 418

<210> 198
<211> 421
<212> DNA
<213> Artificial Sequence

<220>
<223> Mus musculus Ybx1(v2) 3'-UTR
with mutation T128bpG and deletion del236-237bp

<400> 198
tttttatgcc ggcttaccat ctctaccatc atccggtttg gtcatccaac aagaagaaat 60

gaatatgaaa ttccagcaat aagaaatgaa caaagattgg agctgaagac cttaagtgct 120

tgctttttgc ccgctgacca gataacatta gaactatctg cattatctat gcagcatggg 180

gtttttatta tttttaccta aagatgtctc tttttggtaa tgacaaacgt gtttttttaag 240

aaaaaaaaaa aaggcctggt ttttctcaat acacctttaa cggtttttaa attgtttcat 300

atctggtcaa gttgagattt ttaagaactt catttttaat ttgtaataaa gtttacaact 360

tgattttttc aaaaaagtca acaaactgca agcacctgtt aataaaggtc ttaaataata 420

a 421

<210> 199
<211> 798
<212> DNA
<213> Artificial Sequence

<220>
<223> Mus musculus Sepp1 3′-UTR
NM_009155

<400> 199
attatttaaa acaaggcata cctctcccca actcagtcta aagacacaat ttcattttga 60

gaatgtttac agcccattta attaatcagt gaactaaaag tcatagaaat tggatttgtg 120

caaatgtaga gaaatctacc atattggctt ccaaaattta aaaattttat gccacagaac 180

atttcatcca aatcagattt gtacaatagg gcacctgaaa agtgactgca gcctttggtt 240

aatatgtctt tctttttcct ttttccagtg ttctagttac attaatgaga acagaaacat 300

aaactatgac ctaggggttt ctgttggata gcttgtaatt aagaacggag aaagaacaac 360

aaagacatat tttccagttt tttttttctt tacttaaact ctgaaaacaa cagaaacttt 420

gtcttcctac tcttacattc taaaccgatg aaatctttaa cagattacac tttaaatatc 480

tactcatcat tttctctctc agagtcctag cttgagttgc actgcatgta tctgtgcatc 540

ttgttctctt catttaatgc tgtactgttc tgctgagctc tgagggacta tcttgagaga 600

tgtaatggaa ggaaagcgtg gtgttaatct gcgtactgct taagacagta tttccataat 660

caatgatggt ttcatagaga aactaagtcc tatgaacctg acctctttta tggctaatac 720

gactaagcaa gaatggagta cagaattaag tggctacagt acacacttat caaaataaat 780

gcaattttaa aacctttc 798

<210> 200
<211> 390
<212> DNA
<213> Artificial Sequence

<220>
<223> Mus musculus Gaa 3'-UTR
Gaa-001 ENSMUST00000106259

<400> 200
gagagtccgt cgtttacaga ggcctccagg gaggcagagg gagcttgagc tggctctggc 60

tggtggctcc tgtaaggacc tgcgtcctgc tctcctgaca catctttgag cttttcccac 120

cgtgttactg catgcgcccc tgaagctctg tgttcttagg agagtgaggc tcgcctcacc 180

tgccccaccc cagctgtctg tccctcacct ggcactagag aatgtggagc tcggcgtggg 240

gacatcgtgt ctgcaccaac atcaggctgt gcagccactg cagccgcaac cctgcagaga 300

cagagctggt gccttcacca ggttcccaag actcgagaaa cttactgtga agtgtactta 360

cttttaataa aaaggatatt gtttggaagc 390

<210> 201
<211> 481
<212> DNA
<213> Artificial Sequence

<220>
<223> Homo sapiens ACTR10 3'-UTR
ACTR10-002 ENST00000254286

<400> 201
aagtttgatt aaaaatcaac cttgcttcat atcaaatatt taaccaatta taagcaaatt 60

gtacaaagta tgtaggatgt tttgttatag aggactatag tggaagtgaa agcattctgt 120

gtttactctt tgcattaata tataattctt ttgactttgt ttctcttgtg tagtggtaaa 180

atggtagctg gtgcttattg agatttgctg tatttatatc aataaagtat agtaaagcag 240

tttgattttg gaagtttgtt atgtggcttt tttttttttt tttttttgga gacggagtct 300

cgctctgtca cttaggctgg agtgcagtgg cacaatctct actcattgca agctccgcct 360

cccgggttta cgccattctg tctcagcctc ctgagtagct gggactatag gcatacgcca 420

ccccgcccgg ctaatttttt gtatatttag tagagacggg gtttcaccat gttagccagg 480

a 481

<210> 202
<211> 347
<212> DNA
<213> Artificial Sequence

<220>
<223> Homo sapiens PIGF 3'-UTR
NM_173074

<400> 202
gtaacttaat cctgacaacc gtagtgcaag gtatggccca tctcctgtac gcttggagcg 60

acctttggct acgtggctgg ccttgttatt tcaccactct ggatatactg gaatagaaag 120

caacttacat acaagaacaa ttaactggag caaagggaga tatttctttg tgcagattct 180

gtaagggctg ggcagaaatg tgtatggtca aagccaagca gttccattta cagctctgtt 240

ttttacgtag ttacaacatg atgtgattgt agctttttaa actatgaaac ccctgagaga 300

ttgtaccttc tagttgaaat aaagtattta taatagattg tggcttc 347

<210> 203
<211> 233
<212> DNA
<213> Artificial Sequence

<220>
<223> Homo sapiens PIGF 3'-UTR
NM_002643.3

<400> 203
ctggagcaaa gggagatatt tctttgtgca gattctgtaa gggctgggca gaaatgtgta 60

tggtcaaagc caagcagttc catttacagc tctgtttttt acgtagttac aacatgatgt 120

gattgtagct ttttaaacta tgaaacccct gagagattgt accttctagt tgaaataaag 180

tatttataat agattgtggc ttcaaaaaaa aaaaaaaaaa aaaaaaaaaa aaa 233

<210> 204
<211> 116
<212> DNA
<213> Artificial Sequence

<220>
<223> Homo sapiens MGST3 3'-UTR
MGST3-001 ENST00000367889

<400> 204
agaattatag gggtttaaaa actctcattc attttaaatg acttaccttt atttccagtt 60

acattttttt tctaaatata ataaaaactt acctggcatc agcctcatac ctaaaa 116

<210> 205
<211> 913
<212> DNA
<213> Artificial Sequence

<220>
<223> Homo sapiens SCP2 3'-UTR
NM_001193599

<400> 205
agaactccct ttggctactt ttgaaaatca agatgagata tatagatata tatccataca 60

ttttattgtc agaatttaga ctgaaactac acattggcaa atagcgtggg atagatttgt 120

ttcttaatgg gtgtgaccaa tcctgttttt cctatgctct gggtgaatag agcctgatgg 180

tatactactg ctttgcggaa ttgcatacaa ctgtgcatta caaagttaat atggtaatta 240

tggtctgggg taaaattgag tttcagaata aaattaggaa cagtaaaatc caaagaacta 300

tgtaaacaaa aaagcttttg ttttgcttac aaagtatatt taaggattat tctgctgaag 360

attcagttta agagttttcc ttgggagaac taagtaagaa acacaatgcc aacagctggc 420

cagtaattag tgttgtgcac ttcatgtcat taatcaattt ctcaatagtt cttaaaatta 480

gtgagattaa aaatctaaaa attttgcatt tcatgctatc agaaacagta ttttcttccc 540

aaatcaaaat aaaagaaata tgatcagagc ttgaacacag gcttattttt aaaataaaaa 600

tatttttaac atgggtttcc ttattgaaaa atcagtgtat tagtcataaa acaccatcat 660

taagaataat tgaacaataa agtttgcttt cagatgcagt tttcaaatta taatctcatt 720

tcaatttata acgttctcag tcctttgtta taattttcct ttttcatgta agtttaatta 780

tctgcattta tctttttttcc tagtttttct aatactaatg ttatttctta aaattcagtg 840

agatatagga taaaataatg ctttgagaag aatgtttaat agaaaattaa aataactttt 900

tctggcctct ctt 913

<210> 206
<211> 409
<212> DNA
<213> Artificial Sequence

<220>
<223> Homo sapiens SCP2 3'-UTR
SCP2-015 ENST00000435345

<400> 206
agaactccct ttggctactt ttgaaaatca agatgagata tatagatata tatccataca 60

ttttattgtc agaatttaga ctgaaactac acattggcaa atagcgtggg atagatttgt 120

ttcttaatgg gtgtgaccaa tcctgttttt cctatgctct gggtgaatag agcctgatgg 180

tatactactg ctttgcggaa ttgcatacaa ctgtgcatta caaagttaat atggtaatta 240

tggtctgggg taaaattgag tttcagaata aaattaggaa cagtaaaatc caaagaacta 300

tgtaaacaaa aaagcttttg ttttgcttac aaagtatatt taaggattat tctgctgaag 360

attcagttta agagttttcc ttgggagaac taagtaagaa acacaatgc 409

<210> 207
<211> 591
<212> DNA
<213> Artificial Sequence

<220>
<223> Homo sapiens HPRT1 3'-UTR
HPRT1-001 ENST00000298556

<400> 207
gatgagagtt caagttgagt ttggaaacat ctggagtcct attgacatcg ccagtaaaat 60

tatcaatgtt ctagttctgt ggccatctgc ttagtagagc tttttgcatg tatcttctaa 120

gaattttatc tgttttgtac tttagaaatg tcagttgctg cattcctaaa ctgtttattt 180

gcactatgag cctatagact atcagttccc tttgggcgga ttgttgttta acttgtaaat 240

gaaaaaattc tcttaaacca cagcactatt gagtgaaaca ttgaactcat atctgtaaga 300

aataaagaga agatatatta gtttttaat tggtatttta atttttatat atgcaggaaa 360

gaatagaagt gattgaatat tgttaattat accaccgtgt gttagaaaag taagaagcag 420

tcaattttca catcaaagac agcatctaag aagttttgtt ctgtcctgga attattttag 480

tagtgtttca gtaatgttga ctgtattttc caacttgttc aaattattac cagtgaatct 540

ttgtcagcag ttccctttta aatgcaaatc aataaattcc caaaaattta a 591

<210> 208
<211> 283
<212> DNA
<213> Artificial Sequence

<220>
<223> ACSF2
Homo sapiens

<400> 208
ataaagcagc aggcctgtcc tggccggttg gcttgactct ctcctgtcag aatgcaacct 60

ggctttatgc acctagatgt ccccagcacc cagttctgag ccaggcacat caaatgtcaa 120

ggaattgact gaacgaacta agagctcctg gatgggtccg ggaactcgcc tgggcacaag 180

gtgccaaaag gcaggcagcc tgcccaggcc ctccctcctg tccatccccc acattcccct 240

gtctgtcctt gtgatttggc ataaagagct tctgttttct ttg 283

<210> 209
<211> 555
<212> DNA
<213> Artificial Sequence

<220>
<223> Homo sapiens VPS13A 3'-UTR
NM_033305

<400> 209
aattcatatg ttctttattt tacttggaat gtttcattaa catgttttgt atgacttata 60

ccataatgcc catatgtcca tttataggga ggtaaaacac attttctttt aaaatgtttt 120

cctacacatt ttcataaagc aaaataattg tattatttaa gcacagaaaa aaatgtatct 180

tacatccaaa gtagggaggg catccaacat attatagatt tgcttttata tattttatag 240

ctttgtattg catagtttgt ctttaagagt tcaagttaga cttaaatata attttgatgt 300

tcactggttt tattttaaat tgccttctta tttgttagca aaatgccttt ttttaatggt 360

ctctgtaaat tttctgggct ttaatgtaat gccactgtgt aaaaaaaaag gaagaaaata 420

gtaatagcca tttaatgttt tatatttatc attttaaaga tatttttgtc aaatttcttt 480

taataataat aaacatatgt aatctaaaaa aaaaaaaaaa aaaaaaaaaa aaaaaaaaaa 540

aaaaaaaaaa aaaaa 555

<210> 210
<211> 724
<212> DNA
<213> Artificial Sequence

<220>
<223> Homo sapiens CTH 3'-UTR
NM_001190463.1

<400> 210
tattccagag ctgctattag aagctgcttc ctgtgaagat caaatcttcc tgagtaatta 60

aatggaccaa caatgagcct ttgcaaaatt ttcaagcgga aattttaagg cacctcatta 120

tctttcataa ctgtaatttt cttagggatc atctctgtta aaaagttttc tgtatgtcat 180

gttataatta caggtcaatt ctgttaatat ctttttgtta attttgctct atgtttgcct 240

ctgaaggagg tgagatttgt gctactttgg gagattatgt tctttttca tgtctaagat 300

ttattttgat catgtttata atataatggt aattcatttt tgatgttttg tgaagaattt 360

aaatttaaac gaatgttctt aaatcaagtg tgattttttt gcatatcatt gaaaagaaca 420

ttaaaagcaa tggtttacac ttagttacca taagccgaaa atcaaatact tgaaaagttt 480

actgtgaaat tctactgatt taagactata cttaatattt ttaaaaaaat aaatcagctg 540

ggcgcggtgg ctcacgcatg taatgccagc acttttggag gataaggcgg gcggatcacg 600

aggtcaggag attgagacca tcctggctag cgcagtgaaa cccccatctc tactaaaaat 660

gcaaaaaaaa ttagacggac gtggtggcgg gtgcctgtag tcccagctac ttgggaggct 720

gagg 724

<210> 211
<211> 443
<212> DNA
<213> Artificial Sequence

<220>
<223> Homo sapiens CTH 3'-UTR
CTH-001 ENST00000370938

<400> 211
tattccagag ctgctattag aagctgcttc ctgtgaagat caaatcttcc tgagtaatta 60

aatggaccaa caatgagcct ttgcaaaatt ttcaagcgga aattttaagg cacctcatta 120

tctttcataa ctgtaatttt cttagggatc atctctgtta aaaagttttc tgtatgtcat 180

gttataatta caggtcaatt ctgttaatat ctttttgtta attttgctct atgtttgcct 240

ctgaaggagg tgagatttgt gctactttgg gagattatgt tctttttca tgtctaagat 300

ttattttgat catgtttata atataatggt aattcatttt tgatgttttg tgaagaattt 360

aaatttaaac gaatgttctt aaatcaagtg tgattttttt gcatatcatt gaaaagaaca 420

ttaaaagcaa tggtttacac tta 443

<210> 212
<211> 77
<212> DNA
<213> Artificial Sequence

<220>
<223> Homo sapiens CTH 3'-UTR
CTH-002 ENST00000346806

<400> 212
tattccagag ctgctattag aagctgcttc ctgtgaagat caaatcttcc tgagtaatta 60

aatggaccaa caatgag 77

<210> 213
<211> 286
<212> DNA
<213> Artificial Sequence

<220>
<223> Homo sapiens NXT2 3'-UTR
NXT2-004 ENST00000372107

<400> 213
aggggcaaaa gtccattctc atttggtcca ttagttccag caattgaaat ttatgtgaat 60

tattttgatt gtagaagcac tataatatgt gctgaaacta aatttcttta atattttcta 120

ttcctgtcag caccttttct agcagctgcc agtttggagc attgccctct aagagcttta 180

aaactatttt tttacatgcc ttatatacat tccactaatg acattcttat aataatatta 240

aacacatgat cttggtacta acatactcac tgtgaaccca gcctat 286

<210> 214
<211> 121
<212> DNA
<213> Artificial Sequence

<220>
<223> Homo sapiens MGST2 3'-UTR
NM_002413

<400> 214
ctttttctct tccctttaat gcttgcagaa gctgttccca ccatgaaggt aatatggtat 60

catttgttaa ataaaaataa agtctttatt ctgtttttct tgaaaaaaaa aaaaaaaaaa 120

a 121

<210> 215
<211> 582
<212> DNA
<213> Artificial Sequence

<220>
<223> Homo sapiens MGST2 3'-UTR NM_001204366.1

<400> 215
cttttctct tccctttaat gcttgcagaa gctgttccca ccatgaaggc ttgaagccac 60

agtgcatggc cagaaccagc cagacctttg gagttcaaga actcgagagg tgggtgaaaa 120

ctgccattgc ctccacagac tgtcttctcc gtggaaagaa gacctgagtc accagggctg 180

ggaaacctgc accactgaga cgagcacagc ctctgccggc atgcaagtgg ccgctgtcag 240

gacacatgga ctgaaagtgg tttgtcagct gctccattag gtttttttta cccatatgtt 300

tgctaccttt ctttccttga tttaaaaata gggaggggga gcagtctcag ctgtcttcag 360

ctgctaggga gatttttttc cccctcctga gctactgttt cccccaaccc gagcctttct 420

ctcttattgt acccaccctt tctgatgaag tcatcaaagc aaagattgca taactgatgc 480

ataggcctat cttgtgttat actgggagac aggccaatgt ttccattaat agacaagagc 540

accaccacgc tgccaaatgg agctctctgc tgcaaccact ac 582

<210> 216
<211> 37
<212> DNA
<213> Artificial Sequence

<220>
<223> Homo sapiens C11orf67 3'-UTR AAMDC-005 ENST00000526415

<400> 216
tggagcctta agaggagaat aaatcactaa gtgccta 37

<210> 217
<211> 266
<212> DNA
<213> Artificial Sequence

<220>
<223> Homo sapiens PCCA 3'-UTR NM_000282

<400> 217
aggatttata acctttcagt catcacccaa tttaattagc catttgcatg atgctttcac 60

acacaattga ttcaagcatt atacaggaac acccctgtgc agctacgttt acgtcgtcat 120

ttattccaca gagtcaagac caatattctg ccaaaaaatc accaatggaa attttcattg 180

atataaatac ttgtacatat gatttgtact tctgctgtga gattccctag tgtcaaaatt 240

aaatcaataa aactgagcat ttgtct 266

<210> 218
<211> 142

<212>  DNA
<213>  Artificial Sequence

<220>
<223>  Homo sapiens GLMN 3'-UTR
NM_053274

<400>  218
aagttccatt tcctaaataa aaactaataa aatatagtac tttccattat gattcattta      60

atacctttat aaaaaatttt tctgtaaaaa tttactgctt gaaaaataaa tgtagctttt     120

ctcatttatc aaaaaaaaaa aa                                              142


<210>  219
<211>  276
<212>  DNA
<213>  Artificial Sequence

<220>
<223>  Homo sapiens DHRS1 3'-UTR
NM_001136050

<400>  219
ccctcctggt ctgacactac gtctctgctt gtcttctcat ttggacttgg tggttcgtcc      60

tgtctcagtg aaacagcagc ctttcttgtt tacccatacc cttgatatga agagaagccc     120

tctgctgtgt gtccgtggtg agttctgggg tgcgcctagg tcccttcttt gtgccttggt     180

tttccttgtc cttcttttta ctttttgcct tagtattgaa aaatgctctt ggagctaata     240

aaagtctcat ttctctttca aaaaaaaaaa aaaaaa                               276


<210>  220
<211>  450
<212>  DNA
<213>  Artificial Sequence

<220>
<223>  Homo sapiens PON2 3'-UTR
PON2-001 ENST00000433091

<400>  220
attgtacttt tggcatgaaa gtgcgataac ttaacaatta attttctatg aattgctaat      60

tctgagggaa tttaaccagc aacattgacc cagaaatgta tggcatgtgt agttaatttt     120

attccagtaa ggaacggccc ttttagttct tagagcactt ttaacaaaaa aggaaaatga     180

acaggttctt taaaatgcca agcaagggac agaaaagaaa gctgctttcg aataaagtga     240

atacattttg cacaaagtaa gcctcacctt tgccttccaa ctgccagaac atggattcca     300

ctgaaataga gtgaattata tttccttaaa atgtgagtga cctcacttct ggcactgtga     360

ctactatggc tgtttagaac tactgataac gtattttgat gttttgtact tacatctttg     420

tttaccatta aaaagttgga gttatattaa                                      450

<210> 221
<211> 277
<212> DNA
<213> Artificial Sequence

<220>
<223> Homo sapiens NME7 3'-UTR
NM_013330

<400> 221
tggtgtggaa agtaaagaag tcacaggttg ggacatttag acaagagtga atcacacacg 60

aggaatgtgt tcattctttt attgtccgtt gttttaacct gactgaatac aagatcaaca 120

agagcactgt actcctggca attattacat atgttagaac atggattttg cactgtagac 180

aacatttaac accagtctat ggggtactgc attgcttttt ataaagttca aaataaagat 240

ttattttcaa acaaaaaaaa aaaaaaaaaa aaaaaaa 277

<210> 222
<211> 163
<212> DNA
<213> Artificial Sequence

<220>
<223> Homo sapiens ETFDH 3'-UTR
NM_004453

<400> 222
actgcagcta gccagtttct ttcaagtatg gcaagctaac gttaaaatgt ttagagatta 60

acagatttca gaatgtcttt ctgcatatta ctgaacagaa tagtcacaaa atgattatca 120

aataaaaatt ttatactata tgtaagattg tcccataaag aaa 163

<210> 223
<211> 275
<212> DNA
<213> Artificial Sequence

<220>
<223> Homo sapiens ALG13 3'-UTR
BC117377

<400> 223
gatccagcag tatgaagtat tcttgcactg ccattttctt gctgtttttg tttttaaaaa 60

gtattttatg ttagtggtta aatgatttag gtgattagtg tttactattg tatttgtctt 120

taaaattatt ttatcttttg atttaaaata gtactttaaa attaaggggt attattttgg 180

gctgtgacta aggaaattga gatggatgta caactagccc catattgagc atacttcatt 240

gtattcagct gttttcctgt cagccatttg tcagc 275

<210> 224
<211> 664
<212> DNA
<213> Artificial Sequence

<220>
<223> Homo sapiens ALG13 3'-UTR
NM_001099922.2

<400> 224
gatccagcag tatgaagtat tcttgcactg ccattttctt gctgtttttg tttttaaaaa 60

gtattttatg ttagtggtta aatgatttag gtgattagtg tttactattg tatttgtctt 120

taaaattatt ttatcttttg atttaaaata gtactttaaa attaaggggt attattttgg 180

gctgtgacta aggaaattga gatggatgta caactagccc catattgagc atacttcatt 240

gtattcagct gttttcctgt cagccatttg tcagctttat attagctgat ggtaccaatt 300

gataaaatga atataaagta tttcattggt tcaaaaatca cacatcatat taaaccatgc 360

agaattggag taacttccac ttttttctag aaagtaaaac caagagcctt tgcttctgga 420

taactcactt aatattaaat taaagagctc ttcacgtttc ttgagaatta tctgaagcca 480

gttgcattct gtgatatcag ttttgaaggc acatggttct ctgctttaga tttatcccat 540

atgctattgt ttaatactgg atgtatgtaa gtgttttact gcactgtatt gaattggtgt 600

cttttgcaca gttagcagta aataaaaatt agcatttaaa attgccaaaa aaaaaaaaaa 660

aaaa 664

<210> 225
<211> 640
<212> DNA
<213> Artificial Sequence

<220>
<223> Homo sapiens DDX60 3'-UTR
DDX60-001 ENST00000393743

<400> 225
aaacaaagtc tatgcaaacc acttaaaaat aattccatag tagtttttca ggtcacgttt 60

ttgattctta tgcttcttgc cagaaataca ttatgataaa gtggaaatac attacgatga 120

agtggaaaga gcaaacactt tggaatcaaa cagagttgca atcaaacctg ccatgttctg 180

tcatgaatac tcacaaatta tttagtatac ctgaatcttg gtttcttttt ataactgagt 240

aataatggtt acatctcagg tagtttgagg attgactaaa aaaatgcgag aatgttgtat 300

gtgactgaat aacaattttt actctgcgaa gccaaagtaa atataatatt atcagtaact 360

ttatccccag tgtcagtatt tataaaatgt ttattaaggc tagaaaaaat gaatacaata 420

tcctgaaggt gaaatatatt ctcttcaatt agcataaata tgatttacat aagttagcta 480

tacagctatt gagatagtac tttctagtaa acttaaacta ctttttaaac atacattttg 540

tgatgattta acaaaaatat agagaatgat ttgctttatt gtaattgtat ataagtgact 600

ggaaaagcac aaagaaataa agtgggttcg atctgtttac 640

<210> 226
<211> 451
<212> DNA
<213> Artificial Sequence

<220>
<223> Homo sapiens DYNC2LI1 3'-UTR
NM_015522.3

<400> 226
aattcatttg atgtagatga acctgttcac tggaaaatta cagcaattta ttaaaacctc 60

agtaagagca aaacaaggaa gaagattcct tatatcttct tgttagacat cttctgtgat 120

tgttatggca tattacacca atcagagaaa tagagtttta aagtagtggt ttgatattga 180

ttttataatc tctgtaaaaa tgaagataaa aagccagatt gtacaaaagt cacctgacaa 240

agactagatg aagctacaac tttaagcaag gggtagagtt gtaatagcct tcaccatcac 300

tctgtatttt acattcattt cgtttctgtc acttattcag tatcttttta tcatctgaca 360

gctaattaaa ttataaagtt gctatgatgg taacacaagt tcttcaaata caataataaa 420

tatcatcatc tggaaaaaaa aaaaaaaaaa a 451

<210> 227
<211> 606
<212> DNA
<213> Artificial Sequence

<220>
<223> Homo sapiens VPS8 3'-UTR
NM_001009921, NM_015303

<400> 227
tgactccatg gagcctggcc caggagaacc agagatgatc ccgaggcagc tggggagagg 60

ccccgcctct ggtgggcttg gcctccacca cctcccacgc ttctgagaag aggttccaaa 120

ttgggcttct gtgcccagag cgtccacagc accattccca gtgtagactc ccagtcttct 180

ccacattgct gtcatggcgt cagttcacca gactcattga ttttgttttg cttgttaagc 240

aaaggaatgt cacatacctc tgtccagctt tttaggaaat acatttcgcc tattgcgact 300

ttttccattt accctgaagc ctagaaagta ggtggaactc acacaaatgg cattccagag 360

tctgccatac tccgtctcct ccagctgctg gataatacag aggaacttca acttctacag 420

ggaacagtgg ttggccaggc tgcagtataa ctgaagcatg ccttggagag agcagacact 480

gtgggggcca gggccatctc cctttaatgt gttcatgtta aaacctattt gagtgtaaga 540

cttgcccttt ctaacaataa atgctccgtg tttaagttct gcaggtctca aaaaaaaaaa 600

aaaaaa 606

<210> 228
<211> 360

<212> DNA
<213> Artificial Sequence

<220>
<223> Homo sapiens ITFG1 3'-UTR
NM_030790

<400> 228
cttgccttta atattacata atggaatggc tgttcacttg attagttgaa acacaaattc 60

tggcttgaaa aaatagggga gattaaatat tatttataaa tgatgtatcc catggtaatt 120

attggaaagt attcaaataa atatggtttg aatatgtcac aaggtctttt tttttaaagc 180

actttgtata taaaaatttg ggttctctat tctgtagtgc tgtacatttt tgttcctttg 240

tggaatgtgt tgcatgtact ccagtgtttg tgtatttata atcttatttg catcatgatg 300

atggaaaaag ttgtgtaaat aaaaataatt aaaaaaaaaa aaaaaaaaaa aaaaaaaaaa 360

<210> 229
<211> 215
<212> DNA
<213> Artificial Sequence

<220>
<223> Homo sapiens CDK5 3'-UTR
NM_004935

<400> 229
gccccgggac ccccggcctc caggctgggg cctggcctat ttaagccccc tcttgagagg 60

ggtgagacag tgggggtgcc tggtgcgctg tgctccagca gtgctgggcc cagccggggt 120

ggggtgcctg agcccgaatt tctcactccc tttgtggact ttatttaatt tcataaattg 180

gctcctttcc cacagtcaaa aaaaaaaaaa aaaaa 215

<210> 230
<211> 146
<212> DNA
<213> Artificial Sequence

<220>
<223> Homo sapiens C1orf112 3'-UTR
BC091516

<400> 230
aacttatcac taggcagaac tgggtttgat gctttgtcaa ctgaaaatac ttatgtctgt 60

acattttcta acagatataa aacaaatttt gtaaagttga aaaaaaaaaa aaaaaaaaaa 120

aaaaaaaaaa aaaaaaaaaa aaaaaa 146

<210> 231
<211> 239
<212> DNA
<213> Artificial Sequence

<220>

<223> Homo sapiens IFT52 3'-UTR
NM_016004

<400> 231
agaccatgcc tcttgaagct ttttctgcct cctgattctc tctttgtaaa ctattttcaa 60

attgtttttc aactccttat caaaattgtt tatacactct ttcctccatg agctctggaa 120

ggtatatgca tcttctgtaa tactcagata ggtataagat ttttcacaaa atccttatgt 180

aagatacatt ccatttttaa aaattaaatg tatggttgca tctgtctttt tatacccta 239

<210> 232
<211> 146
<212> DNA
<213> Artificial Sequence

<220>
<223> Homo sapiens CLYBL 3'-UTR
CLYBL-003 ENST00000339105

<400> 232
tctgttaaat gaagctgtca tcaggctaaa gggtattgaa gctgcagagg gatcaacttg 60

tgcttgccag aggacgccaa tgaagtttga aacaccaaca atcagagatt ttgtttctgt 120

tcctcattaa atcatgagct tttgtg 146

<210> 233
<211> 477
<212> DNA
<213> Artificial Sequence

<220>
<223> Homo sapiens FAM114A2 3'-UTR
FAM114A2-006 ENST00000520667

<400> 233
agaatggaga cgttttgacc tgggacttgt gacggccaag gaatgccacc ttattctggc 60

tactcctgca gaaatgaagg agtggggtta ttttagtata taaaaattca ggcaggagag 120

atggtttaaa gaggaagatt gttgccttca gtgtttgatt gaagtattca ggttctcaca 180

gtattctttc cagttgttgt aattcataaa ttatttgaaa agaaactttt gtagaaagtc 240

caagaataat aactctagat aaagattagt gggacactca ggcaaaaatg ttggtctttc 300

tttgacatgt tgcaaaatgt tatcaatttt gtcatggata taatttgcag cccatggata 360

taactggttg ataagccaga gaaaaataat ttagtgttct aaaattcatg gcatgtgtgg 420

tttattaatg ccatgtactt tctcctttct ggaataaaat ctatggcttt aagaaaa 477

<210> 234
<211> 310
<212> DNA
<213> Artificial Sequence

<220>

<223>  Homo sapiens NUDT7 3'-UTR
NM_001243661

<400>  234
tttactagag caagagacaa agaactattc acgaggattc tgtgtgtgct tattcgtaga 60

acaacaacaa tgccagctgt tggaatttga caggtgtgaa tatttttttct gcagtatgta 120

gttagaatcc ttgcctcttt tccagttgcc ttctattgtc tgaaaaagta aaagccattc 180

aaaaatgaaa actatgttca tagtgttgca tattttcacc cacaatatgt taataatatt 240

tttcttacac atataataaa gaatatctgg cacatactag gcccttaata aagatttttt 300

gaatatataa 310

<210>  235
<211>  513
<212>  DNA
<213>  Artificial Sequence

<220>
<223>  Homo sapiens AKD1 3'-UTR
NM_001145128

<400>  235
tttacttagg tgatagcagc ctgaatctca agagttatct gaaagtgata gagggaaact 60

gagagaagta gattgaaaat ctgggcctct tggaagtact tttgcctcct gagcaaggta 120

ccatggctgc cagacttcag gtgaactcaa aggtctgcca gccaggaagg agcactctta 180

tggaaacaag ttttaataca attttaaaat gtattgctct ttgcctgaac tttgatgctt 240

taacaaaata aacattctat ttataattcc atatagaaaa gttaagtgac ttatttaata 300

aatgtattat tttccttttt aacattttca gtagaaaagt cagtctctgt taaaattact 360

cattaaatgt tagaaagctt taagacattt aacattgtta taaatgaaac caaaatatgg 420

gttatacatt ttacatacaa aactgtttgt gaactttgtg aacataagat actatcattt 480

tcccaataaa ataaatggat tttgcaacaa ctt 513

<210>  236
<211>  160
<212>  DNA
<213>  Artificial Sequence

<220>
<223>  Homo sapiens MAGED2 3'-UTR
NM_014599

<400>  236
gattttagat attgttaatc ctgccagtct ttctcttcaa gccagggtgc atcctcagaa 60

acctactcaa cacagcactc taggcagcca ctatcaatca attgaagttg acactctgca 120

ttaaatctat ttgccatttc aaaaaaaaaa aaaaaaaaaa 160

<210> 237
<211> 498
<212> DNA
<213> Artificial Sequence

<220>
<223> Homo sapiens HRSP12 3'-UTR
HRSP12-001 ENST00000254878

<400> 237
gtgggcccag tgctgtgtag tctggaattg ttaacatttt aatttttaca attgatgtaa 60

catcttaatt aacctttttaa ttttcacaat tgatgacagt gtgagtttga tgaaaatatc 120

tgaagctatt atggaaatac catgtaatag ggagagttga acatgaatat tagagaagga 180

atccagttac ttttttaaat tacacctgtg tgcacctgta ttactgaata taggaaagag 240

atacccatta catagttact cagtaaacaa aagagaaata ccaggtagga aagaagagtt 300

actattcctg agaaataatc aagaacatat ttaatttaaa ctaatgatgt gaactattta 360

gttttgatgt ccgttatgtg attctgcttt tacttgagta aaattaaagt gtttaaattt 420

gagatcaagg agaagatagt ggaacaaaat gttatataga taatattttt ctaatggaaa 480

taaaataggc agatttcc 498

<210> 238
<211> 127
<212> DNA
<213> Artificial Sequence

<220>
<223> Homo sapiens STX8 NM_004853 3'-UTR

<400> 238
tggcagtaaa gagaccacca gcagtgacac ctgccaatga cagatgcaag cccaacaccc 60

ttttggtacg caaaacctgc tctcaataaa ttcccccaaa gctctgaaaa aaaaaaaaaa 120

aaaaaaa 127

<210> 239
<211> 386
<212> DNA
<213> Artificial Sequence

<220>
<223> Homo sapiens ACAT1 3'-UTR
ACAT1-001 ENST00000265838

<400> 239
acaacctctg ctatttaagg agacaaccct atgtgaccag aaggcctgct gtaatcagtg 60

tgactactgt gggtcagctt atattcagat aagctgtttc atttttttatt attttctatg 120

ttaactttta aaaatcaaaa tgatgaaatc ccaaaacatt ttgaaattaa aaataaattt 180

cttcttctgc ttttttcttg gtaaccttga aaagtttgat acatttttgc attctgagtc 240

tatacttatc gaaatatggt agaaatacca atgtgtaata ttagtgactt acataagtag 300

ctagaagttt ccatttgtga gaacacattt atatttttga ggattgttaa aggtcaagtg 360

aatgctcttt ataggtaatt tacatt 386

<210> 240
<211> 189
<212> DNA
<213> Artificial Sequence

<220>
<223> Homo sapiens IFT74 3'-UTR
IFT74-201 ENST00000433700

<400> 240
gtttaagtcc actgaaagtc tctaaggaag tatcctcttg ctgctaaact tggtacaagt 60

tgactaccaa aaaaaaaaaa agcttacttt tggagtttac ctaaaatttc tgaatgttat 120

aatttttgtg gcctctttta agaatgatat tttaaaatag taaatagttc aataaatggt 180

ttgcatatt 189

<210> 241
<211> 361
<212> DNA
<213> Artificial Sequence

<220>
<223> Homo sapiens KIFAP3 3'-UTR
NM_014970

<400> 241
taaagtatct gtttccatgt gtaatctcag cttagaagaa atctgtgtgg gttgggttaa 60

ttttggatct ttgcctaata atgcatgttg atgttattgt gggtctgtgt ttgtttttat 120

ttttatatgt tgttagctgc agattaaccc cagcccctct gtcttctgtt aagtacagtt 180

gatactgaca ttgttcactc atcaaaccac atcttgatgc taagtaacat ttcccatgag 240

ccacaaaact gaatgctgaa aagctactag actggaaaac aaacactgca ttatgtatgt 300

taagtgacta atttaatttc aattaaaaag cgtaaagtga aaatgaaaaa aaaaaaaaaa 360

a 361

<210> 242
<211> 783
<212> DNA
<213> Artificial Sequence

<220>
<223> Homo sapiens CAPN1 3'-UTR
NM_005186

<400> 242
ggcagggact cggtccccct tgccgtgctc cctccctcc tcgtctgcca agcctcgcct 60

cctaccacac cacaccaggc caccccagct gcaagtgcct tccttggagc agagaggcag 120

cctcgtcctc ctgtcccctc tcctcccagc caccatcgtt catctgctcc gggcagaact 180

gtgtggcccc tgcctgtgcc agccatgggc tcgggatgga ctccctgggc cccacccatt 240

gccaagccag gaaggcagct ttcgcttgtt cctgcctcgg gacagccccg ggtttcccca 300

gcatcctgat gtgtcccctc tccccacttc agaggccacc cactcagcac caccggcctg 360

gccttgcctg cagactataa actataacca ctagctcgac acagtctgca gtccaggcgt 420

gtggagccgc ctcccggctc ggggaggccc cggggctggg aacgcctgtg ccttcctgcg 480

ccgaagccaa cgccccctct gtccttccct ggccctgctg ccgaccagga gctgcccagc 540

ctgtgggcgg tcggccttcc ctccttcgct cctttttat attagtgatt ttaaagggga 600

ctcttcaggg acttgtgtac tggttatggg ggtgccagag gcactaggct tggggtgggg 660

aggtcccgtg ttccatatag aggaacccca aataataaaa ggccccacat ctgtctgtga 720

aaaaaaaaaa aaaaaaaaaa aaaaaaaaaa aaaaaaaaaa aaaaaaaaaa aaaaaaaaaa 780

aaa 783

<210> 243
<211> 88
<212> DNA
<213> Artificial Sequence

<220>
<223> Homo sapiens COX11 3'-UTR
NM_001162861

<400> 243
agagttggca cctttgatgt ggtagtgagc tgatcatcca ctttcttcta aaataaagag 60

aagaaaatgg ccagtaaaaa aaaaaaaa 88

<210> 244
<211> 77
<212> DNA
<213> Artificial Sequence

<220>
<223> Homo sapiens GLT8D4 3'-UTR
BC127733

<400> 244
atattttgtc ttgttgcaag tcaattaggt gtcttgtgaa caaggaaata ctaatctcta 60

agctgcctgg gtctttt 77

<210> 245
<211> 215
<212> DNA
<213> Artificial Sequence

<220>

<223> Homo sapiens GLT8D4 3'-UTR
NM_001080393

<400> 245
atattttgtc ttgttgcaag tcaattaggt gtcttgtgac caaggaaata ctaatctcta 60

agctgcctgg gtctttttgt gtgaatattt aatggtgctc catgactgtt gagttttaaa 120

aacctcgtta aattttgcca aatcagttgc ccccaaaagg gaatatgctt ttccttattt 180

ttttttctaa aatgctattt atctctaagg aaaaa 215

<210> 246
<211> 140
<212> DNA
<213> Artificial Sequence

<220>
<223> Homo sapiens HACL1 3'-UTR
NM_012260

<400> 246
ataaagacgc cagttggtgg tcttgagttt tctctttctt gcaagatgaa attttatttt 60

ccacagcaaa attactctac tgttaaaatt gtgcaaaata aaataaacat ttaaaatgac 120

attttacagt aaaaaaaaaa 140

<210> 247
<211> 273
<212> DNA
<213> Artificial Sequence

<220>
<223> Homo sapiens IFT88 3'-UTR
NM_175605

<400> 247
tattcacttt aatatttatt aaaggaaaga aattgcctta tgagatcatc ctcatgttaa 60

accttggatt aaatatctaa cctgtaatta ttttttttca ctgtcaaaac ttaagtaagt 120

gtattctatt ctgtatgtat gcatttaagt tgttttttttc ttttaaggaa taaaaacagg 180

taaaactaat actttaggcc agtgacttcc ttagcttttt gaaaacattg acacacagga 240

agaaataaat ttcataacac aaaaaaaaaa aaa 273

<210> 248
<211> 187
<212> DNA
<213> Artificial Sequence

<220>
<223> Homo sapiens IFT88 3'-UTR
IFT88-001 ENST00000351808

<400> 248
tattcacttt aatatttatt aaaggaaaga aattgcctta tgagatcatc ctcatgttaa 60

acccttggatt aaatatctaa cctgtaatta ttttttttca ctgtcaaaac ttaagtaagt 120

gtattctatt ctgtatgtat gcatttaagt tgttttttttc ttttaaggaa taaaaacagg 180

taaaact 187

<210> 249
<211> 150
<212> DNA
<213> Artificial Sequence

<220>
<223> Homo sapiens NDUFB3 3'-UTR
NM_002491

<400> 249
agataatacc tggaagcatc atagtggttt cttaactctc caaaataaga tttcttctct 60

gtagcctact tgtctggttt atcccttaca gaatattagt aagatttaat caattaaaat 120

atatatatat gccaaaaaaa aaaaaaaaaa 150

<210> 250
<211> 589
<212> DNA
<213> Artificial Sequence

<220>
<223> Homo sapiens ANO10 3'-UTR
NM_018075

<400> 250
gtgcccagcg tgcccagctg ccctgttggc agaggcctgt gtctgtgcca cacctgccac 60

ggtggcaggg ggggtacccg gggcagcatc gtggctcctg aacccagacc caatgcttag 120

ccaaacgaag tggctcccat gtggcaagca cccttctcag tttcgcagtg gcttggctcg 180

ggatccttgg cagttccccc agccccaccc tgtctgctcc ttcccagttc cttcccgggc 240

cccacacgct gctccagctg ccaactttgc tgcagagcca ctgccgccct tgagcctctc 300

accatgagtg agccaccagc tctccacgtt cccctcatag cagtgtcact cccaacccca 360

ccatggccca gggacccgtg gacaggttgg ggatggggtg tgtgcccact gtgctcatca 420

caggagcctc agttgagagt gagcggggta cagtaaggca gtgcttccca cactggacct 480

ctttcctggt tctcttttgc aatacattaa cagacccttt atcaacataa acaatagtaa 540

ctgagctatt aaaggcaacc tctctgactc cttctgccta aaaaaaaaa 589

<210> 251
<211> 263
<212> DNA
<213> Artificial Sequence

<220>
<223> Homo sapiens ANO10 3'-UTR
ANO10-005 ENST00000451430

<400> 251
gtgcccagcg tgcccagctg ccctgttggc agaggcctgt gtctgtgcca cacctgccac 60

ggtggcaggg ggggtacccg gggcagcatc gtggctcctg aacccagacc caatgcttag 120

ccaaacgaag tggctcccat gtggcaagca cccttctcag tttcgcagtg gcttggctcg 180

ggatccttgg cagttccccc agccccaccc tgtctgctcc ttcccagttc cttcccgggc 240

cccacacgct gctccagctg cca 263

<210> 252
<211> 486
<212> DNA
<213> Artificial Sequence

<220>
<223> Homo sapiens ARL6 3'-UTR
NM_032146

<400> 252
aaagataata gttggaaacc tcagcaattt tcaattcaag gaatctatct aagacaaata 60

gaatacattt tgtaaaagat gtttatgcat caaaaaatat aattttctgc ttgcatttat 120

ggactctgac ctttttaaga acataggact tcaggtatgc taatttggcc attaattatt 180

taaaaactaa atattccctc aaaagggctc cctagaatta tcaagttctt agtgaaggtc 240

tacatttgat tgtacgtaga atgtttaaaa gtcagttata agccatctca tcccatcata 300

atttatgata tgtttaatat attttatttt ttaattgtct ttttaaaaaa tttagtttat 360

gactttgcag tatgaattgt gcttgtgaaa aagaacttta aatatttata agggaccatg 420

ggtaattaat atatattcaa tttttactat gtgtcactgt caataaaatg taaaatataa 480

tgtgcc 486

<210> 253
<211> 719
<212> DNA
<213> Artificial Sequence

<220>
<223> Homo sapiens LPCAT3 3'-UTR
NM_005768

<400> 253
tccatttccc tggtggcctg tgcgggactg gtgcagaaac tactcgtctc ccttttcaca 60

gcactccttt gccccagagc agagaatgga aaagccaggg aggtggaaga tcgatgcttc 120

cagctgtgcc tctgctgcca gccaagtctt catttggggc caaaggggaa actttttttt 180

ggagaaggcg tcttgctttg tcacccacgc tggaatgcag tggcgggatc tcagctcacc 240

gcaacctcca cctcctgggt tcaagtgatt ttcctgcctc agcctcccaa gtagctggga 300

atacaggcac gccaccatgc ccagctaatt tttgtatttt cagtagaaac gggatttcac 360

cacgttggcc aggctggtct cgaactcctg accgcaagtg atccacccgc ctccgcctcc 420

caaagtgctg ggattacagg cgtgagccac cgtgcccggc ccaaagggga aactcttgtg 480

ggaggagcag aggggctcac atctcccctc tgattccccc atgcacattg ccttatctct 540

ccccatctag ccaggaatct attgtgtttt tcttctgcca atttactatg attgtgtatg 600

tgccgctacc accacccccc ccatgggggg gtggagaggg gtgcaaggcc ctgcctgctc 660

cacttttttct accttggaac tgtattagat aaaatcactt ctgtttgttc agtttttca 719

<210> 254
<211> 154
<212> DNA
<213> Artificial Sequence

<220>
<223> Homo sapiens ABCD3 3'-UTR
NM_001122674

<400> 254
aaaccagaca aatgtattgg ccaggcgtgg tggctcatgc ctgtaatccc agcactttgg 60

gaggctgaga tgggaggatc gcttgaatcc aggagttcga gacaagcctg gacaaaaagc 120

gagacccgct tctttaaaaa ataataataa aaca 154

<210> 255
<211> 448
<212> DNA
<213> Artificial Sequence

<220>
<223> Homo sapiens COPG2 3'-UTR
NM_012133

<400> 255
atgcttactg gacaagagga aactgatgca cactacatgg tcagtgggct tttaggctag 60

tggcatcagt ttcccagaat cagacttttg aagatgaatg actttggaga agcaaattaa 120

acatttggcc ctgagccagc agatcaagca aatgtctatc tttgcgcatg ggttgttttt 180

tttttttttc tttttattct acttggtcag ctttgggacg atagtgcagc tttgggtgat 240

cttgaaaatc aaatactatc ctatactcca gctgcttaac ttcattttat tctttaatgt 300

gtacctgaaa gctcctggca atgctggaaa atttttatcc cagaggggtg gggggaggg 360

gggaggggaa gccagagtcc acttttgtca caattcattt ttattaatag aaaataaaca 420

cttattccag tttcaaaaaa aaaaaaaa 448

<210> 256
<211> 176
<212> DNA
<213> Artificial Sequence

<220>
<223> Homo sapiens MIPEP 3'-UTR
NM_005932

<400> 256
aagaaacact ctacacctct taaatcaagg tcatgtagat aatgactttg ttataaatgc 60

tacagctgtg agagcttgtt tctgatttca ttgttcgctt ctgtaattct gaaaaacttt 120

aaactggtag aacttggaat aaataatttg ttttaattaa aaaaaaaaaa aaaaaa 176

<210> 257
<211> 478
<212> DNA
<213> Artificial Sequence

<220>
<223> Homo sapiens LEPR 3'-UTR
NM_002303

<400> 257
tttcactgaa gaaaccttca gatttgtgtt ataatgggta atataaagtg taatagatta 60

tagttgtggg tgggagagag aaaagaaacc agagtcaaat ttgaaaataa ttgttccaaa 120

tgaatgttgt ctgtttgttc tctcttagta acatagacaa aaaatttgag aaagccttca 180

taagcctacc aatgtagaca cgctcttcta ttttattccc aagctctagt gggaaggtcc 240

cttgtttcca gctagaaata agcccaacag acaccatctt ttgtgagatg taattgtttt 300

ttcagagggc gtgttgtttt acctcaagtt tttgttttgt accaacacac acacacacac 360

acattcttaa cacatgtcct tgtgtgtttt gagagtatat tatgtattta tattttgtgc 420

tatcagactg taggatttga agtaggactt tcctaaatgt ttaagataaa cagaattc 478

<210> 258
<211> 64
<212> DNA
<213> Artificial Sequence

<220>
<223> Homo sapiens LEPR 3'-UTR
NM_001198688

<400> 258
gaaatgcttg tagactacgt cctacctcgc tgccgcacct gctctccctg aggtgtgcac 60

aatg 64

<210> 259
<211> 28
<212> DNA
<213> Artificial Sequence

<220>
<223> Homo sapiens C2orf76 3'-UTR
NM_001017927

<400> 259
aaacatctcg agggcttcct ttttgcat 28

<210> 260
<211> 244
<212> DNA
<213> Artificial Sequence

<220>
<223> Homo sapiens C2orf76 3'-UTR
C2orf76-001 ENST00000409466

<400> 260
aaacatctcg agggcttcct ttttgcatac ctgtattaag ctctttattc cactgctgaa 60

tttttgaaat tgacaaacaa atcttaaaaa attaatccca ggctatactc tttgagctaa 120

aatctggtta tttctttctc ttcaggtctt tccttctctc tttctttttc tttgttgttg 180

taaaataata tattatgaga aaaacatttg atctttttaa agggaaataa attgttatta 240

aaaa 244

<210> 261
<211> 267
<212> DNA
<213> Artificial Sequence

<220>
<223> Homo sapiens ABCA6 3'-UTR
NM_080284.2

<400> 261
aacctcaaac ctagtaattt tttgttgatc tcctataaac tcatgtttta tgtaataatt 60

aatagtatgt ttaattttaa agatcattta aaattaacat caggtatatt ttgtaaattt 120

agttaacaaa tacataaatt ttaaaattat tcttcctctc aaacataggg gtgatagcaa 180

acctgtgata aaggcaatac aaaatattag taaagtcacc caaagagtca ggcactgggt 240

attgtggaaa taaaactata taaactt 267

<210> 262
<211> 45
<212> DNA
<213> Artificial Sequence

<220>
<223> Homo sapiens LY96 3'-UTR
NM_015364.4

<400> 262
aataaattga gtatttaaaa aaaaaaaaaa aaaaaaaaaa aaaaa 45

<210> 263
<211> 755
<212> DNA
<213> Artificial Sequence

<220>
<223> Homo sapiens CROT 3'-UTR
NM_ 001243745.1

<400> 263
tgatgatgtt taaagaatga taaataaaaa gtgcatagtt tttattttta aattattgct 60

gtaaaaattt ttacagttat tattgttatt ttcataatcc aaaagaagga atgaatcact 120

taactttggg agttttcagt gggtggattc gggaacttgt taaaatgcag atttgctggg 180

ataagtgatt ctgattcaca tggctggaat gaggcccaga gattcttatt ttaacaatca 240

cttcatgtgg tttggctgca ggtaatctgt agaccatgct gaaggaaaac attttgtcca 300

ggtgactagc ttgaaaaatc agaaacacta aaatagacat gtcacatagg tggcatagaa 360

atattttcgt agtacaatgg agaaagggaa tcattaaaaa tcagagtgga gaatggttat 420

gtatattgta tatttcagtt agataaattg aggaagctag tataataatt attgaaggtc 480

tcaataattt tccacaaaat tctttaactt cttcagctca accatttctg tacttctcta 540

ctatgaatca gaggatgagg ttgtataatt caaaagcatt gccttagtct agaaataatt 600

attgtaccta tcatttagtt ttagaaataa aaagcaagct gatttttttt gatgaaccat 660

ttatatctgt gatggaataa taaaatttca cacttccgga ttcctttgtt ctcaattttg 720

agccttgagt tgttttaatt aaagaggggt aaagg 755

<210> 264
<211> 911
<212> DNA
<213> Artificial Sequence

<220>
<223> Homo sapiens ENPP5 3'-UTR
ENPP5-002 ENST00000230565

<400> 264
tgttactttg aagtggattt gcatattgaa gtggagattc cataattatg tcagtgttta 60

aaggtttcaa attctgggaa accagttcca aacatttgca gaaaccatta agcagttaca 120

tatttaggta tacacacaca cacacacaca catacacaca cacggaccaa aatacttaca 180

cctgcaaagg aataaagatg tgagagtatg tctccattgt tcactgtagc atagggatag 240

ataagatcct gctttatttg gacttggcgc agataatgta tatatttagc aactttgcac 300

tatgtaaagt accttatgta ttgcacttta aatttctctc ctgatgggta ctttaatttg 360

aaatgcactt tatgcacagt tatgtcttat aacttgattg aaaatgacaa ctttttgcac 420

ccatgtcaca gaatacttgt tacgcattgt tcaaactgaa ggaaatttct aataatcccg 480

aataatgaac gtagaaatct atctccataa attgagagaa gaagaaggtg ataagtgttg 540

aaaattaaat gtgataacct ttgaaccttg aattttggag atgtattccc aacagcagaa 600

tgcaactgtg ggcatttctt gtcttatttc tttccagaga acgtggtttt catttatttt 660

tccctcaaaa gagagtcaaa tactgacaga ttcgttctaa atatattgtt tctgtcataa 720

aattattgtg atttcctgat gagtcatatt actgtgattt tcataataat gaagacacca 780

tgaatatact tttttctat atagttcagc aatggcctga atagaagcaa ccaggcacca 840

tctcagcaat gttttctctt gtttgtaatt atttgctcct ttgaaaatta aatcactatt 900

aattacatta a 911

<210> 265
<211> 300
<212> DNA
<213> Artificial Sequence

<220>
<223> Homo sapiens SERPINB7 3'-UTR
SERPINB7-203 ENST00000546027

<400> 265
aaatccaatt ggtttctgtt atagcagtcc ccacaacatc aaagaaccac cacaagtcaa 60

tagatttgag tttaattgga aaaatgtggt gtttcctttg agtttatttc ttcctaacat 120

tggtcagcag atgacactgg tgacttgacc cttcctagac acctggttga ttgtcctgat 180

ccctgctctt agcattctac caccatgtgt ctcacccatt tctaatttca ttgtctttct 240

tcccacgctc atttctatca ttctccccca tgacccgtct ggaaattatg gagagtgctc 300

<210> 266
<211> 509
<212> DNA
<213> Artificial Sequence

<220>
<223> Homo sapiens TCP11L2 3'-UTR
NM_152772

<400> 266
agaagaactg acattggacg agagattgga aatccagtac tttggtatcc agtccacttc 60

cattgatggc attagagatc cagcacattc tcagtactgt ggtgcagtat tagcccaaat 120

ctgtgtaatg ggtaatatta gcattacaga agacacacac atcacataga ccctcagaag 180

acgtaaacat cacatagacc ctatttgtgc atcattttca agtttaaaac agatatttgt 240

aatgaacaga aaacaatttg taattaatta tattacctat ataatacttg taaatgtttt 300

cttaaccatt tatatttggc ttatgacatt taacccctaa ggagttgttt ttctcacttg 360

ttattatcaa acctaatggt ttttaatttt ggtacaactc cttaaagggt tgaaggttgt 420

gacaataact gagggaactg atgttctgaa taaatgatgt gaagtaaaca caattgtatt 480

tgaaaaaaaa aaaaaaaaaa aaaaaaaaa 509

<210> 267
<211> 84
<212> DNA
<213> Artificial Sequence

<220>
<223> Homo sapiens IRAK1BP1 3'-UTR
NM_001010844

<400> 267
aattccaaac aaattatatt gtacttgtat ctttttacct atttttatac tttttataat 60

gtttacgttt gtcctgaata tata 84


<210> 268
<211> 338
<212> DNA
<213> Artificial Sequence

<220>
<223> Homo sapiens CDKL2 3'-UTR
CDKL2-002 ENST00000307465

<400> 268
gaaccatttt ggttctgaac tggatgatgc tcttgcactt gagatgacat cttcttgcag 60

caagagtgct gatatcccaa gaggagagat tcatggtttt gatcatttcc ttctgaactg 120

cctgcatttt ctgaggaagg ccttctagaa gaaggaaaga caaagacttc caaatgtttc 180

aaaggaagat tgaacaaatg gccctcccca actgttatcc cattaccttt cacgtccacc 240

gatgctattt caagacatat ccagtggaat aacagtgata tggttcttgt tacatgaatg 300

tgtatttact gttaggagat tgtatatttt aagttacc 338


<210> 269
<211> 367
<212> DNA
<213> Artificial Sequence

<220>
<223> Homo sapiens GHR 3'-UTR
GHR-202 ENST00000537449

<400> 269
cctttctttg gtttcccaag agctacgtat ttaatagcaa agaattgact ggggcaataa 60

cgtttaagcc aaaacaatgt ttaaaccttt tttgggggag tgacaggatg gggtatggat 120

tctaaaatgc cttttcccaa aatgttgaaa tatgatgtta aaaaaataag aagaatgctt 180

aatcagatag atattcctat tgtgcaatgt aaatatttta aagaattgtg tcagactgtt 240

tagtagcagt gattgtctta atattgtggg tgttaatttt tgatactaag cattgaatgg 300

ctatgttttt aatgtatagt aaatcacgct ttttgaaaaa gcgaaaaaat caggtggctt 360

ttgcggt 367

<210> 270
<211> 406
<212> DNA
<213> Artificial Sequence

<220>
<223> Homo sapiens KIAA1107 3'-UTR
NM_015237

<400> 270
gtgttaacat tttggaaaaa tttatgccac tcctttattt tttgatgcct atattatatc 60

caaatgataa ttgcattagc cggatataaa ctttctttaa tattgagtct ttccaattta 120

atgaggtaaa catagtttat ttattaatat atcacatata gaaaaatgtt tttctaaagt 180

ttttgagcat gttttctcta attattagag aaattagaag acttataagg aaaccctagc 240

ttcagttttc ctttcctagc tgatgatttg ttcacttaat cattattcaa gaatttaaaa 300

tgtgaatgca gaagtagatc agtcccttta ctttttgctc tgcatagggt aacatagtaa 360

tttaacaata aaaacttacc gtgcttgtgt ccaaaaaaaa aaaaaa 406

<210> 271
<211> 301
<212> DNA
<213> Artificial Sequence

<220>
<223> Homo sapiens RPS6KA6 3'-UTR
RPS6KA6-001 ENST00000262752

<400> 271
gatttgtggt gttcctaggc caaactggat gaagatgaaa ttaaatgtgt ggcttttttc 60

ctattcttat caaaggcatc gttgtctgct aaattacttg aatattaagt aatattaaat 120

ccccattttt aggggaagtg agatttaaaa aaccattcac aggtccacaa tattcatact 180

atgtgtttgc agtagtgttc aagtgtttat ttaagcatat aattggtgtc caccaggtcc 240

tcacaacttc tctgcacaca agcttctaaa attcctttca aataaagtta ctttaatatt 300

t 301

<210> 272
<211> 777
<212> DNA
<213> Artificial Sequence

<220>
<223> Homo sapiens CLGN 3'-UTR
NM_004362, NM_001130675

<400> 272
actagattga aatattttta attcccgaga gggatgtttg gcattgtaaa aatcagcatg 60

ccagacctga actttaatca gtctgcacat cctgtttcta atatctagca acattatatt 120

ctttcagaca tttattttag tccttcattt cagaggaaaa agaagcaact ttgaagttac 180

ctcatctttg aatttagaat aaaagtggca cattacatat cggatctaag agattaatac 240

cattagaagt tacacagttt tagttgtttg gagatagttt tggtttgtac agaacaaaat 300

aatatgtagc agcttcattg ctattggaaa aatcagttat tggaatttcc acttaaatgg 360

ctatacaaca atataactgg tagttctata ataaaaatga gcatatgttc tgttgtgaag 420

agctaaatgc aataaagttt ctgtatggtt gtttgattct atcaacaatt gaaagtgttg 480

tatatgaccc acatttacct agtttgtgtc aaattatagt tacagtgagt tgtttgctta 540

aattatagat tcctttaagg acatgccttg ttcataaaat cactggatta tattgcagca 600

tattttacat ttgaatacaa ggataatggg ttttatcaaa acaaaatgat gtacagattt 660

tttttcaagt ttttatagtt gctttatgcc agagtggttt accccattca caaaatttct 720

tatgcataca ttgctattga aaataaaatt taaatatttt ttcatcctga aaaaaaa 777

<210> 273
<211> 466
<212> DNA
<213> Artificial Sequence

<220>
<223> Homo sapiens CLGN-202 3'-UTR
NM_004362, NM_001130675
ENST00000325617

<400> 273
actagattga aatattttta attcccgaga gggatgtttg gcattgtaaa aatcagcatg 60

ccagacctga actttaatca gtctgcacat cctgtttcta atatctagca acattatatt 120

ctttcagaca tttattttag tccttcattt cagaggaaaa agaagcaact ttgaagttac 180

ctcatctttg aatttagaat aaaagtggca cattacatat cggatctaag agattaatac 240

cattagaagt tacacagttt tagttgtttg gagatagttt tggtttgtac agaacaaaat 300

aatatgtagc agcttcattg ctattggaaa aatcagttat tggaatttcc acttaaatgg 360

ctatacaaca atataactgg tagttctata ataaaaatga gcatatgttc tgttgtgaag 420

agctaaatgc aataaagttt ctgtatggtt gtttgattct atcaac 466

<210> 274
<211> 423
<212> DNA
<213> Artificial Sequence

<220>
<223> Homo sapiens TMEM45A 3'-UTR
NM_018004

<400> 274
ctttgatgag cttccagttt ttctagataa accttttctt ttttacattg ttcttggttt 60

tgtttctcga tcttttgttt ggagaacagc tggctaagga tgactctaag tgtactgttt 120

gcatttccaa tttggttaaa gtatttgaat ttaaatattt tctttttagc tttgaaaata 180

ttttgggtga tactttcatt ttgcacatca tgcacatcat ggtattcagg ggctagagtg 240

attttttttcc agattatcta aagttggatg cccacactat gaaagaaata tttgttttat 300

ttgccttata gatatgctca aggttactgg gcttgctact atttgtaact ccttgaccat 360

ggaattatac ttgtttatct tgttgctgca atgagaaata aatgaatgta tgtattttgg 420

tgc 423

<210> 275
<211> 152
<212> DNA
<213> Artificial Sequence

<220>
<223> Homo sapiens TBC1D8B 3'-UTR
TBC1D8B-007 ENST00000276175

<400> 275
atccctagga attgcctatc atagacaagt ttactaacat tcctgtagct gtcagtttga 60

ttcctgtgag tagggctcag ggatttatct tgttaccaat gtgtctgaag gccaaaatat 120

atatccagaa gcacaatgca tcattccttt gt 152

<210> 276
<211> 81
<212> DNA
<213> Artificial Sequence

<220>
<223> Homo sapiens ACP6 3'-UTR
NM_016361

<400> 276
ctgatttata aaagcaggat gtgttgattt taaaataaag tgcctttata caatgccaaa 60

aaaaaaaaaa aaaaaaaaaa a 81

<210> 277
<211> 111
<212> DNA
<213> Artificial Sequence

<220>
<223> Homo sapiens RP6-213H19.1 3'-UTR
MST4-003 (RBM4B-003 ENST00000496850)

<400> 277
gaaacttatt attggcttct gtttcatatg gacccagaga gccccaccaa acctacgtca 60

agattaacaa tgcttaaccc atgagctcca tgtgcctttt ggatctttgc a 111

<210> 278
<211> 138

<212> DNA
<213> Artificial Sequence

<220>
<223> Homo sapiens SNRPN 3'-UTR
NM_022807

<400> 278
catactgttg atccatctca gtcacttttt cccctgcaat gcgtcttgtg aaattgtgta 60

gagtgtttgt gagctttttg ttccctcatt ctgcattaat aatagctaat aataaatgca 120

tagagcaatt aaactgtg 138

<210> 279
<211> 425
<212> DNA
<213> Artificial Sequence

<220>
<223> Homo sapiens GLRB 3'-UTR
GLRB-005 ENST00000512619

<400> 279
gatctaatga cttcagcatt gttggaagct taccaagaga ttttgaacta tccaattatg 60

actgctatgg aaaacccatt gaagttaaca acggacttgg gaaatctcag gctaagaaca 120

acaagaagcc tccccctgcg aaacctgtta ttccaacagc agcaaagcga attgatcttt 180

atgcaagagc attgtttcct ttctgcttct tgttcttcaa tgttatatat tggtctatat 240

atttatgata aatcttttcc atttgtacaa aataaaattc catttcattg tgacctactc 300

ctttcataaa tgccaatctg tgagaacttt tgaattttca tagcaacatt gcattttgga 360

tgccatttga ttgtaataaa actgtggcac cttaattttg aatggcagca tgatcatgta 420

atatc 425

<210> 280
<211> 651
<212> DNA
<213> Artificial Sequence

<220>
<223> Homo sapiens HERC6 3'-UTR
NM_017912

<400> 280
tcacctctga gagactcagg gtgggctttc tcacacttgg atccttctgt tcttccttac 60

acctaaataa tacaagagat taatgaatag tggttagaag tagttgaggg agagattggg 120

ggaatgggga gatgatgatg atggtcaaag ggtgcaaaat ctcacacaag actgaggcag 180

gagaataggg tacagagata gggatctaag gatgacttgg acacactccc tggcactgaa 240

gagtctgaac actggcctgt gattggtcca ttccaggacc ttcatttgca taaggtatca 300

aaccacatca gcctctgatt ggccatgggc cagacctgca ctctggccaa tgattggttc 360

attccaggac attcatttgc ataaggagtc aaaccacacc agtcttggat tggctgtgag 420

ccaattcacc tcagtctcta attggctgtg agtcagtctt tcatttacat agggtgtaac 480

catcaagaaa cctctacagg gtacttaagc cccagaagat tttgctacca gggctcttga 540

gccacttgct ctagcccact cccaccctgt ggaatgtact ttcacttttg ctgcttcact 600

gccttgtgct ccaataaatc cactccttca ccacccaaaa aaaaaaaaaa a 651

<210> 281
<211> 264
<212> DNA
<213> Artificial Sequence

<220>
<223> Homo sapiens CFH 3'-UTR
NM_000186

<400> 281
aatcaatcat aaagtgcaca cctttattca gaactttagt attaaatcag ttctcaattt 60

catttttat gtattgtttt actccttttt attcatacgt aaaattttgg attaatttgt 120

gaaaatgtaa ttataagctg agaccggtgg ctctcttctt aaaagcacca tattaaatcc 180

tggaaaacta aaaaaaaaaa aaaaaaaaaa aaaaaaaaaa aaaaaaaaaa aaaaaaaaaa 240

aaaaaaaaaa aaaaaaaaaa aaaa 264

<210> 282
<211> 401
<212> DNA
<213> Artificial Sequence

<220>
<223> Homo sapiens GALC 3'-UTR
GALC-002 ENST00000393569

<400> 282
tacttaacag ggcatcatag aatactctgg attttcttcc cttctttttg gttttggttc 60

agagccaatt cttgtttcat tggaacagta tatgaggctt ttgagactaa aaataatgaa 120

gagtaaaagg ggagagaaat ttatttttaa tttaccctgt ggaagatttt attagaatta 180

attccaaggg gaaaactggt gaatctttaa cattacctgg tgtgttccct aacattcaaa 240

ctgtgcattg gccataccct taggagtggt ttgagtagta cagacctcga agccttgctg 300

ctaacactga ggtagctctc ttcatcttat ttgcaagcgg tcctgtagat ggcagtaact 360

tgatcatcac tgagatgtat ttatgcatgc tgaccgtgtg t 401

<210> 283
<211> 51
<212> DNA
<213> Artificial Sequence

<220>
<223> Homo sapiens GALC 3'-UTR
GALC-005 ENST00000393568

<400> 283
tacttaacag ggcatcatag aatactctgg attttcttcc cttcttttttg g 51

<210> 284
<211> 360
<212> DNA
<213> Artificial Sequence

<220>
<223> Homo sapiens PDE1A 3'-UTR
NM_001003683.2

<400> 284
acacctttaa gtaaaacctc gtgcatggtg gcagctctaa tttgaccaaa agacttggag 60

attttgatta tgcttgctgg aaatctaccc tgtcctgtgt gagacaggaa atctattttt 120

gcagattgct caataagcat catgagccac ataaataaca gctgtaaact ccttaattca 180

ccgggctcaa ctgctaccga acagattcat ctagtggcta catcagcacc ttgtgctttc 240

agatatctgt ttcaatggca ttttgtggca tttgtcttta ccgagtgcca ataaattttc 300

tttgagcagc taattgctaa ttttgtcatt tctacaataa agcttggtcc acctgttttc 360

<210> 285
<211> 308
<212> DNA
<213> Artificial Sequence

<220>
<223> Homo sapiens PDE1A 3'-UTR
PDE1A-003 ENST00000410103

<400> 285
acacctttaa gtaaaacctc gtgcatggtg gcagctctaa tttgaccaaa agacttggag 60

attttgatta tgcttgctgg aaatctaccc tgtcctgtgt gagacaggaa atctattttt 120

gcagattgct caataagcat catgagccac ataaataaca gctgtaaact ccttaattca 180

ccgggctcaa ctgctaccga acagattcat ctagtggcta catcagcacc ttgtgctttc 240

agatatctgt ttcaatggca ttttgtggca tttgtcttta ccgagtgcca ataaattttc 300

tttgagca 308

<210> 286
<211> 855
<212> DNA
<213> Artificial Sequence

<220>
<223> Homo sapiens GSTM5 3'-UTR
NM_000851

<400> 286
ggcccagtga tgccagaaga tgggagggag gagccaacct tgctgcctgc gaccctggag 60

gacagcctga ctccctggac ctgccttctt cctttttcct tctttctact ctcttctctt 120

ccccaaggcc tcattggctt cctttcttct aacatcatcc ctccccgcat cgaggctctt 180

taaagcttca gctccccact gtcctccatc aaagtccccc tcctaacgtc ttcctttccc 240

tgcactaacg ccaacctgac tgcttttcct gtcagtgctt ttctcttctt tgagaagcca 300

gactgatctc tgagctccct agcactgtcc tcaaagacca tctgtatgcc ctgctccctt 360

tgctgggtcc ctaccccagc tccgtgtgat gcccagtaaa gcctgaacca tgcctgccat 420

gtcttgtctt attccctgag gctcccttga ctcaggactg tgctcgaatt gtgggtggtt 480

ttttgtcttc tgttgtccac agccagagct tagtggatgg gtgtgtgtgt gtgtgtgttg 540

ggggtggtga tcaggcaggt tcataaattt ccttggtcat ttctgccctc tagccacatc 600

cctctgttcc tcactgtggg gattactaca gaaaggtgct ctgtgccaag ttcctcactc 660

attcgcgctc ctgtaggccg tctagaactg gcatggttca aagaggggct aggctgatgg 720

ggaagggggc tgagcagctc ccaggcagac tgccttcttt caccctgtcc tgatagactt 780

ccctgatcta gatatccttc gtcatgacac ttctcaataa aacgtatccc accgtattgt 840

aaaaaaaaaa aaaaa 855

<210> 287
<211> 419
<212> DNA
<213> Artificial Sequence

<220>
<223> Homo sapiens CADPS2 3′-UTR
CADPS2-002 ENST00000412584

<400> 287
tatcacacag ctttgcagaa ggaaggaaga ccttgatcga cattgttttt tattttttta 60

accttgtcct tgtaattaca ttcattgttt gttttggcca aataaaaatg cttgtatttc 120

tttaaaaagt aagcctgaat gtagagtaaa aggggaaatg ccaagatttt ggggtttttt 180

tgtttccttt ttttgtttgt ttgtttgttt gtttttttgg agaagagcat cctcttttgt 240

gtagtttgac ctaaaaatga accttggctc tgcttgtgat cagaacatga actttttttt 300

ttaaagaaga tttgagcatt tttctgtaat cacatcaaaa tgatgttttc tgtgtaaagc 360

gagatacata tttctcataa tgcagcattg tgagaagtca gttcggacca ctgcaccaa 419

<210> 288
<211> 162
<212> DNA
<213> Artificial Sequence

<220>

<223> Homo sapiens CADPS2 3′-UTR
CADPS2-001 ENST00000449022

<400> 288
tatcacacag ctttgcagaa ggaaggaaga ccttgatcga cattgttttt tattttttta 60

accttgtcct tgtaattaca ttcattgttt gttttggcca aataaaaatg cttgtatttc 120

tttaaaaagt aagcctgaat gtagagtaaa aggggaaatg cc 162

<210> 289
<211> 247
<212> DNA
<213> Artificial Sequence

<220>
<223> Homo sapiens AASS 3′-UTR
AASS-001 ENST00000417368

<400> 289
ttgggaatta tattttgttt ttttcttccc aggcaataca cctctgaaca tgtgtgtgat 60

aaatgggttt gctaatgtgc tgttttaaag tataaagcat aatatgtttt ggttaacaca 120

atgtactttt tgaactataa atctttattt taatatggaa atgtttggaa caggagatgc 180

aagccactaa cagagaactt taataattct accctgtatt ttataaatac gtatgtgaaa 240

gtgatga 247

<210> 290
<211> 695
<212> DNA
<213> Artificial Sequence

<220>
<223> Homo sapiens TRIM6-TRIM34 3′-UTR
NM_001003819

<400> 290
attttctcat ttcttcacct acaacccttt gtcttgactt atctcctgca actgactcat 60

ctgcaacatt cacaccattg cttccttgtg gtttcccttc tttagaactt ttactcatcc 120

ttgagatgta tggtgtattt ggcttgagtt atgagagatg cttatttatt catttactct 180

ttttcatatt ttcagagaaa gttacctaat ccctcctaaa gacacagcag tatgggtata 240

acatccttgc cttcccattt atccatgttt cactttatca ctgatatgaa gaggcccaaa 300

gcctgttagc caccatccat gctacctagg tagtccatag gaaccacccc catgaccacc 360

accaacatca actaaaggtt cttggagggt atgtcagtgt gttgctcagg ataccccagg 420

tacatcaagg aatcaaggag aggaaaatat gagcaatatg tgtattcaga gtgaagattt 480

tatgtccaga gtatttgagc tcaaaccttg cctgttgttt tctaatcatg atgaatactt 540

tctcagtttc tttttcctga aatataaatt gggatttaag actgtaccta actattaaga 600

tcactgtgta aaactaagtg tctctaaatg taatgcatcg atttagtgtc tggaacataa 660

taaatatttg ctctcatgat tgctaaaaaa aaaaa 695

<210> 291
<211> 918
<212> DNA
<213> Artificial Sequence

<220>
<223> Homo sapiens SEPP1 3'-UTR
NM_005410

<400> 291
atatttaaaa taggacatac tccccaattt agtctagaca caatttcatt tccagcattt 60

ttataaacta ccaaattagt gaaccaaaaa tagaaattag atttgtgcaa acatggagaa 120

atctactgaa ttggcttcca gattttaaat tttatgtcat agaaatattg actcaaacca 180

tattttttat gatggagcaa ctgaaaggtg attgcagctt ttggttaata tgtctttttt 240

tttctttttc cagtgttcta tttgctttaa tgagaataga aacgtaaact atgacctagg 300

ggtttctgtt ggataattag cagtttagaa tggaggaaga acaacaaaga catgctttcc 360

atttttttct ttacttatct ctcaaaacaa tattactttg tcttttcaat cttctacttt 420

taactaataa aataagtgga ttttgtattt taagatccag aaatacttaa cacgtgaata 480

ttttgctaaa aaagcatata taactatttt aaatatccat ttatcttttg tatatctaag 540

actcatcctg atttttacta tcacacatga ataaagcctt tgtatctttc tttctctaat 600

gttgtatcat actcttctaa aacttgagtg gctgtcttaa aagatataag ggaaaagata 660

atattgtctg tctctatatt gcttagtaag tatttccata gtcaatgatg gtttaatagg 720

taaaccaaac cctataaacc tgacctcctt tatggttaat actattaagc aagaatgcag 780

tacagaattg gatacagtac ggatttgtcc aaataaattc aataaaaacc ttaaagctga 840

aaaaaaaaaa aaaaaaaaaa aaaaaaaaaa aaaaaaaaaa aaaaaaaaaa aaaaaaaaaa 900

aaaaaaaaaa aaaaaaaa 918

<210> 292
<211> 589
<212> DNA
<213> Artificial Sequence

<220>
<223> Homo sapiens SEPP1 3'-UTR
SEPP1-004 ENST00000506577

<400> 292
atatttaaaa taggacatac tccccaattt agtctagaca caatttcatt tccagcattt 60

ttataaacta ccaaattagt gaaccaaaaa tagaaattag atttgtgcaa acatggagaa 120

atctactgaa ttggcttcca gattttaaat tttatgtcat agaaatattg actcaaacca 180

tattttttat gatggagcaa ctgaaaggtg attgcagctt ttggttaata tgtctttttt 240

tttctttttc cagtgttcta tttgctttaa tgagaataga aacgtaaact atgacctagg 300

ggtttctgtt ggataattag cagtttagaa tggaggaaga acaacaaaga catgctttcc 360

atttttttct ttacttatct ctcaaaacaa tattactttg tcttttcaat cttctacttt 420

taactaataa aataagtgga ttttgtattt taagatccag aaatacttaa cacgtgaata 480

ttttgctaaa aaagcatata taactatttt aaatatccat ttatcttttg tatatctaag 540

actcatcctg atttttacta tcacacatga ataaagcctt tgtatcttt 589

<210> 293
<211> 60
<212> DNA
<213> Artificial Sequence

<220>
<223> Homo sapiens PDE5A 3'-UTR
PDE5A-002 ENST00000264805

<400> 293
gtggcctatt tcatgcagag ttgaagttta cagagatggt gtgttctgca atatgcctag 60

<210> 294
<211> 422
<212> DNA
<213> Artificial Sequence

<220>
<223> Homo sapiens SATB1 3'-UTR
SATB1-004 ENST00000417717

<400> 294
gataaaagta tttgtttcgt tcaacagtgc cactggtatt tactaacaaa atgaaaagtc 60

caccttgtct tctctcagaa aacctttgtt gttcattgtt tggccaatga atcttcaaaa 120

acttgcacaa acagaaaagt tggaaaagga taatacagac tgcactaaat gttttcctct 180

gttttacaaa ctgcttggca gccccaggtg aagcatcaag gattgtttgg tattaaaatt 240

tgtgttcacg ggatgcacca aagtgtgtac cccgtaagca tgaaaccagt gtttttttgtt 300

tttttttag ttcttattcc ggagcctcaa acaagcatta taccttctgt gattatgatt 360

tcctctccta taattatttc tgtagcactc cacactgatc tttggaaact tgccccttat 420

tt 422

<210> 295
<211> 981
<212> DNA
<213> Artificial Sequence

<220>
<223> Homo sapiens CCPG1 3'-UTR
CCPG1-002 ENST00000442196

<400> 295
ttcacaattg agttaaatta gacaactgta agagaaaaat ttatgctttg tataatgttt 60

ggtattgaaa ctaatgaaat taccaagatg acaatgtctt ttcttttgtt tctaagtatc 120

agtttgataa ctttatatta ttcctcagaa gcattagtta aaagtctact aacctgcatt 180

ttcctgtagt ttagcttcgt tgaatttttt ttgacactgg aaatgttcaa ctgtagtttt 240

attaaggaag ccaggcatgc aacagatttt gtgcatgaaa tgagacttcc tttcagtgta 300

agagcttaaa gcaagctcag tcatacatga caaagtgtaa ttaacactga tgtttgtgtt 360

aaatttgcag cagagcttga gaaaagtaca ttgttctgga atttcatcat taacatttta 420

taatcttaca ctcacttctt gtctttttgt gggttcaaga gccctctgac ttgtgaagaa 480

tttgctgccc tcttaagagc ttgctgactt gttttcttgt gaaatttttt gcacatctga 540

atatcgtgga agaaacaata aaactacacc atgaggaaaa ctaaaggtct ttatttaaaa 600

tctggcattg tattaacatg taattttata ctatgtggta ttttatacat ttcctcagta 660

gtgatatttg gtaaagcagt tcatacagct tttttctaag ttccatgaat cttacccagt 720

gtttaccgaa gtatttaagc agcatctgaa tatttccacc cagcaatgtt aatttatcta 780

ggaaagttca gaatttcatc ttcatgttga atttcccttt taacttccgt tcatagacat 840

atatgtgact tccaattcga ccctctggca agtgagtgtg gaagaaaaca gcagttcttt 900

tataattgct tgaaattagg aaagcgctta tttcctagaa gcaaataaat gtttaagtaa 960

ataaaggcta cattttgctg a 981

<210> 296
<211> 575
<212> DNA
<213> Artificial Sequence

<220>
<223> Homo sapiens CCPG1 3′-UTR
CCPG1-004 ENST00000425574

<400> 296
ttcacaattg agttaaatta gacaactgta agagaaaaat ttatgctttg tataatgttt 60

ggtattgaaa ctaatgaaat taccaagatg acaatgtctt ttcttttgtt tctaagtatc 120

agtttgataa ctttatatta ttcctcagaa gcattagtta aaagtctact aacctgcatt 180

ttcctgtagt ttagcttcgt tgaatttttt ttgacactgg aaatgttcaa ctgtagtttt 240

attaaggaag ccaggcatgc aacagatttt gtgcatgaaa tgagacttcc tttcagtgta 300

agagcttaaa gcaagctcag tcatacatga caaagtgtaa ttaacactga tgtttgtgtt 360

aaatttgcag cagagcttga gaaaagtaca ttgttctgga atttcatcat taacatttta 420

taatcttaca ctcacttctt gtctttttgt gggttcaaga gccctctgac ttgtgaagaa 480

tttgctgccc tcttaagagc ttgctgactt gttttcttgt gaaatttttt gcacatctga 540

atatcgtgga agaaacaata aaactacacc atgag 575

<210> 297
<211> 230
<212> DNA
<213> Artificial Sequence

<220>
<223> Homo sapiens CNTN1 3'-UTR
CNTN1-002 ENST00000348761

<400> 297
atgtgttgtg acagctgctg ttcccatccc agctcagaag acacccttca accctgggat 60

gaccacaatt ccttccaatt tctgcggctc catcctaagc caaataaatt atactttaac 120

aaactattca actgatttac aacacacatg atgactgagg cattcgggaa ccccttcatc 180

caaaagaata aacttttaaa tggatataaa tgatttttaa ctcgttccaa 230

<210> 298
<211> 922
<212> DNA
<213> Artificial Sequence

<220>
<223> Homo sapiens CNTN1 3'-UTR
CNTN1-004 ENST00000547849

<400> 298
tcgttgacac tcaccatttc tgtgaaagac tttttttttt ttaacatatt atactagatt 60

tgactaactc aatcttgtag cttctgcagt tctccccacc cccaacctag ttcttagagt 120

atgtttcccc ttttgaaaca tgtaaacata ctttgggcat aaatattttt taaaatataa 180

ctataatgct tcactaatac cttaaaaatg cctagtgaac taactcagta cattatataa 240

tggccaagtg aaagttttgt gttttcatgt cctgtttttc tttgaaatta tatagcccag 300

aaattagctc attatctgaa aaacgtatga agaactgatg aattgtataa tacaggagta 360

ttgccattga atgtactgtt tgatttattc aagcaggtaa tgaacaatgt tgtcaaactc 420

tctaatgaga catcataatt aggacataag ctaaaagggg cattactccg gcagtctttt 480

tttcttaatc ctagtaccat acatattctt tggcatgaaa gaatgaaaag cattagtaaa 540

caactgaagt cctaccatgg ctctgtaggg tttttggaac aattcctgga attggaaagt 600

gaaaatggat agcatgtggg ggaaaccctc atctgagtag caagatttta gtaaagatga 660

ctaagccatt aacagcatgc attcatattt aattttattg actcctgcca tcagcttttg 720

tagatcgttt gggtggaagg ttgtgatttt tactgggagg acttgagtag aagtggatga 780

ttaaaattga ggagtatata attctttctg ggactgctta aatgttattg tttgaaaata 840

ccttcacttt ccccctttgg tcaaagagat gtgcttaaaa ttcttattcc ttcacaataa 900

ataattttga ttttcttaga ca 922

<210> 299
<211> 928
<212> DNA
<213> Artificial Sequence

<220>
<223> Homo sapiens CNTN1 3'-UTR
CNTN1-004 ENST00000547849
+T at pos. 30bp, mutations G727bpT, A840bpG

<400> 299
ttttttcgtt gacactcacc atttctgtga aagacttttt ttttttttaa catattatac 60

tagatttgac taactcaatc ttgtagcttc tgcagttctc cccaccccca acctagttct 120

tagagtatgt ttcccctttt gaaacatgta aacatacttt gggcataaat attttttaaa 180

atataactat aatgcttcac taatacctta aaaatgccta gtgaactaac tcagtacatt 240

atataatggc caagtgaaag ttttgtgttt tcatgtcctg tttttctttg aaattatata 300

gcccagaaat tagctcatta tctgaaaaac gtatgaagaa ctgatgaatt gtataataca 360

ggagtattgc cattgaatgt actgtttgat ttattcaagc aggtaatgaa caatgttgtc 420

aaactctcta atgagacatc ataattagga cataagctaa aaggggcatt actccggcag 480

tctttttttc ttaatcctag taccatacat attctttggc atgaaagaat gaaaagcatt 540

agtaaacaac tgaagtccta ccatggctct gtagggtttt tggaacaatt cctggaattg 600

gaaagtgaaa atggatagca tgtgggggaa accctcatct gagtagcaag attttagtaa 660

agatgactaa gccattaaca gcatgcattc atatttaatt ttattgactc ctgccatcag 720

cttttgtaga tcttttgggt ggaaggttgt gatttttact gggaggactt gagtagaagt 780

ggatgattaa aattgaggag tatataattc tttctgggac tgcttaaatg ttattgtttg 840

aaaatgcctt cactttcccc ctttggtcaa agagatgtgc ttaaaattct tattccttca 900

caataaataa ttttgatttt cttagaca 928

<210> 300
<211> 734
<212> DNA
<213> Artificial Sequence

<220>
<223> Homo sapiens LMBRD2 3'-UTR

<400> 300
agtctgaaaa agtttgtggg accactaacc aaggtcaaca catcagttca gtcttgatga 60

acatctgtgt accctagaat ttcctctata cacagtgaaa agtgtcaaga taacaaaaaa 120

ggcactgaga attaattata tcttaggaat aatagtttaa tgtgcattga atagagtatc 180

acctttttca acaagattta ttacatatca tttcctaagc atctgcctta gaaatacagt 240

tacagtggaa ggactttaag aaagatcaac atatgttaag aacatgcagt tcagtttgtt 300

tcagattaat ttttttcaa gagagttatt ttaaagattc aaggaagcca taagtcatac 360

taaataatat tatatacagt tttgttattg tgacttacat ttttgttact tctaaaaagt 420

atattcaacc tgtatttccc aaagaaatgt aagtgaatgg agacctcaaa taataactgt 480

attcataaaa ctcgtgtctt aaaacaaggc ttacttacta gacataactg aatgtaaaaa 540

gtgctttttc aaatctgttt gcaaactcgt gggggatttt tgcatgtata agattaagat 600

tatacttcaa gtgatgcgtg tctgtgtatt tagcatgtgt actataatca ggtgatatag 660

tattccttca gtctttgtag taactggatt tttttatgct tctggtattg ctttataaaa 720

gattttcatt tcag 734

<210> 301
<211> 241
<212> DNA
<213> Artificial Sequence

<220>
<223> Homo sapiens TLR3 3'-UTR
NM_003265

<400> 301
atttatttaa atattcaatt agcaaaggag aaactttctc aatttaaaaa gttctatggc 60

aaatttaagt tttccataaa ggtgttataa tttgtttatt catatttgta aatgattata 120

ttctatcaca attacatctc ttctaggaaa atgtgtctcc ttatttcagg cctatttttg 180

acaattgact taattttacc caaaataaaa catataagca cgtaaaaaaa aaaaaaaaaa 240

a 241

<210> 302
<211> 527
<212> DNA
<213> Artificial Sequence

<220>
<223> Homo sapiens BCAT1 3'-UTR
BCAT1-002 ENST00000342945

<400> 302
atggaaaata gaggatacaa tggaaaatag aggataccaa ctgtatgcta ctgggacaga 60

ctgttgcatt tgaattgtga tagatttctt tggctacctg tgcataatgt agtttgtagt 120

atcaatgtgt tacaagagtg attgtttctt catgccagag aaaatgaatt gcaatcatca 180

aatggtgttt cataacttgg tagtagtaac ttaccttacc ttacctagaa aaacattaat 240

gtaagccata taacatggga ttttcctcaa tgattttagt gcctcctttt gtacttcact 300

cagatactaa atagtagttt attctttaat ataagttaca ttctgctcct caaacaaatg 360

caattttttg tgtgtgtttg aaagctaatt tgagaaaatt tcataggtta catttcctgc 420

agcctatctt tatccacaga aagtgttttc ttttttttaa atcaagactt ttaaaactgg 480

atttcctccc atcactgttt tttgaaggtc ctccaagtcc gtgttaa 527

<210> 303
<211> 199
<212> DNA
<213> Artificial Sequence

<220>
<223> Homo sapiens BCAT1 3'-UTR

<400> 303
atggaaaata gaggatacaa tggaaaatag aggataccaa ctgtatgcta ctgggacaga 60

ctgttgcatt tgaattgtga tagatttctt tggctacctg tgcataatgt agtttgtagt 120

atcaatgtgt tacaagagtg attgtttctt catgccagag aaaatgaatt gcaatcatca 180

aatggtgttt cataacttg 199

<210> 304
<211> 716
<212> DNA
<213> Artificial Sequence

<220>
<223> Homo sapiens TOM1L1 3'-UTR
TOM1L1-001 ENST00000575882

<400> 304
gaagaaagtg gatgatcagc tcactaccac atcaaaggtg ccaactctct aaaacgtaga 60

ctctgtgcag ctttgaagcc tggaagacaa tacctaccaa catgtcaaag ccatggtggc 120

acatttctgc tataatgaag attaaataga ataacagttc caggataaca ctgattcctg 180

acaacagcgt gagatttcaa cagaacttgt ttggaacaaa tactcactta aaacttcagc 240

agaagaaaaa ttacttagtc cttaggccaa ccaatttaac tgcagtgtca tgtttcacag 300

gccttcctac atttagaaat cgtcacacag ctgtgataag agtagattat tttactatga 360

aataattctg aatagatgaa agcataaaat gtgagaaact gaatgtatta ttcaggaaga 420

atactgagtg ccttcattta actaaagttg aatgtaaaag tcaatttgca cttctttata 480

atcctctggt ttagaattat aaattgttaa aaccttgata attgtcattt aattatattt 540

caggtgtcct gaacaggtca ctagactcta cattgggcag cctttaaata tgattctttg 600

taatgctaaa tagccttttt ttctcttttt actgcaactt aatatttcta tttagaacac 660

agaaaatgaa aatatttaga ataagttgta catttgatga caaataaatc actatt 716

<210> 305
<211> 804

<212> DNA
<213> Artificial Sequence

<220>
<223> Homo sapiens SLC35A1 3'-UTR
SLC35A1-201 ENST00000369556

<400> 305
ttttagcctc acgtgagact ccttttaaga ctaaaccatt tgcattaaac tagagcctta 60

agtcaatctc agaaggtagc ataaacaaat aaaaattaac tgtatggcat gatcagtgcg 120

gttatgtgga aacaacaaca aacaaacgaa gctatctgag tgaactgcta atacagaaac 180

ttaatgtaga cctgtttggg gtctactatt gttttagaat gaaggaattg tattattgtg 240

tgtatatata atttgtaaat aaaaagtatg gagatgatac ggtgttaaaa aaaatcatgg 300

taaggctaca atactcaagt aacaaggttt gggacaatgt ctaagggtta aagtgccaaa 360

gccatttctg tactaactgt tctcttgttc cggtaccggg gagaaggatg acccctcctt 420

attctccaat tcatgtacag tattttgtcc tagcagcata aagacctagc tcttttctta 480

caagaggcag aaacaagaca ggctagttca taaacaaact gtgtaacttc tcaaaatgaa 540

tctatttcat aactcggaca atttctgggt ggtgactgag taccccttta gtgagtaccc 600

ctttagtgct atatttgtgc cattcattat ctggttcata tttcttttct gttagatgat 660

acacatttct tcaaaaaaat ttctaatgtc acttttgtac ttttttaaat aaagtatgtt 720

taactgttgg gctctcaata atttgtgaaa tttcagtgtt ttctataatg ttaatgggga 780

aattcagcaa taaactttat ttgt 804

<210> 306
<211> 332
<212> DNA
<213> Artificial Sequence

<220>
<223> Homo sapiens GLYATL2 3'-UTR
GLYATL2-003 ENST00000532258

<400> 306
ttgattccac tgtccatttc aaatctttct tatcagtaaa aaaacattaa ttcaaacaca 60

agcattgtga tctacattag cacaaaatgc aactgattat ctaggatctg tgtattactt 120

aagctcaccc ttaacagttt taccttcctt ctcctctgta ttcttacaga aaattagaag 180

ctcaatttta tggtctcata atttccttta tgacagacat ctcagaatta aaatcaccca 240

aagccaatca ttagtgccaa gataaccctt taacggcaac actttcttaa atgaagacta 300

tttctttcat gaaaaaattc acttttatga ct 332

<210> 307
<211> 260
<212> DNA

<213> Artificial Sequence

<220>
<223> Homo sapiens STAT4 3'-UTR
STAT4-002 ENST00000392320

<400> 307
caggataaac tctgacgcac caagaaagga agcaaatgaa aaagtttaaa gactgttctt 60

tgcccaataa ccacatttta tttcttcagc tttgtaaata ccaggttcta ggaaatgttt 120

gacatctgaa gctctcttca cactcccgtg gcactcctca attgggagtg ttgtgactga 180

aatgcttgaa accaaagctt cagataaact tgcaagataa gacaacttta agaaaccagt 240

gttaataaca atattaacag 260

<210> 308
<211> 270
<212> DNA
<213> Artificial Sequence

<220>
<223> Homo sapiens GULP1 3'-UTR
GULP1-002 ENST00000409609

<400> 308
catcaagaac aagaaatcct gattcatgtt aaatgtgttt gtatacacat gtcatttatt 60

attattactt taagataggt attattcatg tgtcaatgtt tttgaatatt ttaatatttt 120

gaaaattttc tcagttaaat ttcctcacct tcactattga tctgtaattt ttattttaaa 180

aacagcttac tgtaaagtag atcatacttt tatgttcctt tctgtttcta ctgtagatga 240

atttgtaatt gaaagacata ttatacaaat 270

<210> 309
<211> 79
<212> DNA
<213> Artificial Sequence

<220>
<223> Homo sapiens GULP1 3'-UTR
GULP1-010 ENST00000409805

<400> 309
catcaagaac aagaaatcct gattcatgtt aaatgtgttt gtatacacat gtcatttatt 60

attattactt taagatagg 79

<210> 310
<211> 256
<212> DNA
<213> Artificial Sequence

<220>
<223> Homo sapiens EHHADH 3'-UTR
EHHADH-002 ENST00000456310

<400> 310
ttcagtcttc cagattatgc ctcacatgct agcatcaggt aatgctgact gaatttcagt 60

gaaattaaat caaaaatcca aagtaagatt gttctgaaat acaaagcaaa ataaataatc 120

attagaatct tctgtgtaac gactctaatg gtcaaatctt taggaatgtg cttcctatgc 180

ctctgaatct gtccttatca gataaattca atgcatgaac ttgtgtgaat ataataccat 240

aatagctaat gaaaga 256

<210> 311
<211> 640
<212> DNA
<213> Artificial Sequence

<220>
<223> Homo sapiens NBEAL1 3'-UTR
NM_001114132.1

<400> 311
ttgttatttc cattttctgt tatgattact gaaacctgat ttattgcttt gtcactttaa 60

ccacatctct caactctctg caatgttgca aggcttttat ccctgaaaat catttacaga 120

taaccacaat ttgctgtggt atataaacta attcttggtc tatactaaga tgtatttgag 180

aaaatacatt tgatttgatt ttgtggccca ttcctaaagg tcattgtatc catttttaaa 240

acaaactaaa atgagaacat taggttcaat tttcttatta ttccaaatga taaaatttaa 300

gatttttcta ataaaagagt acagataatg ggacagttga gagagatggc tttaaataca 360

ttcttaagta atcattttcc tatttactga ccactgtaat gaaaatatat caatttattt 420

atggaactcc tgattgggga taatatttta aaggtatctg ttgcacactt ggattttcaa 480

aactcggtga aagttacaag tttgcatggt aagaataaaa taagaatatt gaaactggta 540

cattagctaa ttctattact acttagcgtg tttctaatga gaagttactg aaatctatta 600

ctgtccttaa taaaaattga gtagaaaaaa gtggaactag 640

<210> 312
<211> 225
<212> DNA
<213> Artificial Sequence

<220>
<223> Homo sapiens KIAA1598 3'-UTR
NM_001258299.1

<400> 312
tctgaatcag aaaatactgc aactccttcc tccttttgtc tgccttttgt tctccaaaag 60

taagtggaaa ttacatttcc aagaaaggaa atgaaataat tgcaggccca aggtctgcaa 120

aatatgtgtt gaattgacag tgaaaaggat ccatgtgttg acagacacag ttgttagatg 180

ccataaaggc agatgtgaag ctcaatttat ttctcatctt gcttg 225

<210> 313
<211> 991
<212> DNA
<213> Artificial Sequence

<220>
<223> Homo sapiens HFE 3'-UTR
HFE-006 ENST00000317896

<400> 313
cacgcagcct gcagactcac tgtgggaagg agacaaaact agagactcaa agagggagtg 60

catttatgag ctcttcatgt ttcaggagag agttgaacct aaacatagaa attgcctgac 120

gaactccttg attttagcct tctctgttca tttcctcaaa aagatttccc catttaggtt 180

tctgagttcc tgcatgccgg tgatccctag ctgtgacctc tcccctggaa ctgtctctca 240

tgaacctcaa gctgcatcta gaggcttcct tcatttcctc cgtcacctca gagacataca 300

cctatgtcat ttcatttcct atttttggaa gaggactcct taaatttggg ggacttacat 360

gattcatttt aacatctgag aaaagctttg aaccctggga cgtggctagt cataacctta 420

ccagattttt acacatgtat ctatgcattt tctggacccg ttcaactttt cctttgaatc 480

ctctctctgt gttacccagt aactcatctg tcaccaagcc ttggggattc ttccatctga 540

ttgtgatgtg agttgcacag ctatgaaggc tgtacactgc acgaatggaa gaggcacctg 600

tcccagaaaa agcatcatgg ctatctgtgg gtagtatgat gggtgttttt agcaggtagg 660

aggcaaatat cttgaaaggg gttgtgaaga ggtgtttttt ctaattggca tgaaggtgtc 720

atacagattt gcaaagttta atggtgcctt catttgggat gctactctag tattccagac 780

ctgaagaatc acaataattt tctacctggt ctctccttgt tctgataatg aaaattatga 840

taaggatgat aaaagcactt acttcgtgtc cgactcttct gagcacctac ttacatgcat 900

tactgcatgc acttcttaca ataattctat gagataggta ctattatccc catttctttt 960

ttaaatgaag aaagtgaagt aggccgggca c 991

<210> 314
<211> 761
<212> DNA
<213> Artificial Sequence

<220>
<223> Homo sapiens HFE 3'-UTR
HFE-004 ENST00000349999

<400> 314
cacgcagcct gcagactcac tgtgggaagg agacaaaact agagactcaa agagggagtg 60

catttatgag ctcttcatgt ttcaggagag agttgaacct aaacatagaa attgcctgac 120

gaactccttg attttagcct tctctgttca tttcctcaaa aagatttccc catttaggtt 180

tctgagttcc tgcatgccgg tgatccctag ctgtgacctc tcccctggaa ctgtctctca 240

tgaacctcaa gctgcatcta gaggcttcct tcatttcctc cgtcacctca gagacataca 300

cctatgtcat ttcatttcct atttttggaa gaggactcct taaatttggg ggacttacat 360

gattcatttt aacatctgag aaaagctttg aaccctggga cgtggctagt cataacctta 420

ccagattttt acacatgtat ctatgcattt tctggacccg ttcaactttt cctttgaatc 480

ctctctctgt gttacccagt aactcatctg tcaccaagcc ttggggattc ttccatctga 540

ttgtgatgtg agttgcacag ctatgaaggc tgtacactgc acgaatggaa gaggcacctg 600

tcccagaaaa agcatcatgg ctatctgtgg gtagtatgat gggtgttttt agcaggtagg 660

aggcaaatat cttgaaaggg gttgtgaaga ggtgtttttt ctaattggca tgaaggtgtc 720

atacagattt gcaaagttta atggtgcctt catttgggat g 761

<210> 315
<211> 142
<212> DNA
<213> Artificial Sequence

<220>
<223> Homo sapiens HFE 3'-UTR
HFE-005 ENST00000397022

<400> 315
cacgcagcct gcagactcac tgtgggaagg agacaaaact agagactcaa agagggagtg 60

catttatgag ctcttcatgt ttcaggagag agttgaacct aaacatagaa attgcctgac 120

gaactccttg attttagcct tc 142

<210> 316
<211> 31
<212> DNA
<213> Artificial Sequence

<220>
<223> Homo sapiens HFE 3'-UTR
HFE-012 ENST00000336625

<400> 316
cacgcagcct gcagactcac tgtgggaagg a 31

<210> 317
<211> 394
<212> DNA
<213> Artificial Sequence

<220>
<223> Homo sapiens KIAA1324L 3'-UTR
KIAA1324L-005 ENST00000416314

<400> 317
agagacagtg ctgtagcctt gagactaatg aacaaagaaa cctgctctag ttttacagga 60

ccatatttta gggtctgtcc tcatacctgt cacattggtg atctcacaga ggagggccat 120

gccgctgaaa agggaaggag attgaaacat ttgattgcct tatcacatgg tcaagtacct 180

tgccaaataa aggaaagcaa atgatttggg tctcaactga agatgaagct caactcagga 240

agagatttat ctgtatatac acataactga aaaccaagtt taagcccacc aatgcactgc 300

tgatgcatgc catataatta atgggtaact tttattcttt atgatgtcta cataacaagt 360

gtgatttgga aggcacatgt gagcatatgc atta 394

<210> 318
<211> 743
<212> DNA
<213> Artificial Sequence

<220>
<223> Homo sapiens MANSC1 NM_018050 3'-UTR

<400> 318
ggatggaact cggtgtctct taattcattt agtaaccaga agcccaaatg caatgagttt 60

ctgctgactt gctagtctta gcaggaggtt gtattttgaa gacaggaaaa tgccccccttc 120

tgctttcctt tttttttttt ggagacagag tcttgctttg ttgcccaggc tggagtgcag 180

tagcacgatc tcggctctca ccgcaacctc cgtctcctgg gttcaagcga ttctcctgcc 240

tcagcctcct aagtatctgg gattacaggc atgtgccacc acacctgggt gatttttgta 300

tttttagtag agacggggtt tcaccatgtt ggtcaggctg gtctcaaact cctgacctag 360

tgatccaccc tcctcggcct cccaaagtgc tgggattaca ggcatgagcc accacagctg 420

gccccccttct gttttatgtt tggtttttga gaaggaatga agtgggaacc aaattaggta 480

attttgggta atctgtctct aaaatattag ctaaaaacaa agctctatgt aaagtaataa 540

agtataattg ccatataaat ttcaaaattc aactggcttt tatgcaaaga aacaggttag 600

gacatctagg ttccaattca ttcacattct tggttccaga taaaatcaac tgtttatatc 660

aatttctaat ggatttgctt ttctttttat atggattcct ttaaaactta ttccagatgt 720

agttccttcc aattaaatat ttg 743

<210> 319
<211> 142
<212> DNA
<213> Artificial Sequence

<220>
<223> Homo sapiens LTA4H 5'-UTR
LTA4H-001 ENST00000228740

<400> 319
aagaaacttc ctttcccggc gtgcaccgcg aatccctcct cctcttcttt acctctctcc 60

ctcctcctca ggttctctat cgacgagtct ggtagctgag cgttgggctg taggtcgctg 120

tgctgtgtga tcccccagag cc 142

<210> 320
<211> 88
<212> DNA
<213> Artificial Sequence

<220>
<223> Homo sapiens DECR1 5'-UTR
DECR1-001 ENST00000220764

<400> 320
tccagccccg agaactttgt tctttttgtc ccgccccctg cgcccaaccg cctgcgccgc 60

cttccggccc gagttctgga gactcaac 88

<210> 321
<211> 45
<212> DNA
<213> Artificial Sequence

<220>
<223> Homo sapiens PIGK 5'-UTR

<400> 321
actgcctccg ccccttcagg tgcgggaagt ctgaagccgg taaac 45

<210> 322
<211> 122
<212> DNA
<213> Artificial Sequence

<220>
<223> Homo sapiens BRP44L 5'-UTR
BRP44L-001

<400> 322
gtcgtgaggc gggccttcgg gctggctcgc cgtcggctgc cggggggttg gccggggtgt 60

cattggctct gggaagcggc agcagaggca gggaccactc ggggtctggt gtcggcacag 120

cc 122

<210> 323
<211> 117
<212> DNA
<213> Artificial Sequence

<220>
<223> Homo sapiens ACADSB 5'-UTR
ACADSB-004 NM_001609.3 ENST00000368869

<400> 323
agggattaag ggggggtgtg tgcggggcgg gtactgagtg ggcggggcct tgctcgggta 60

actcccaggg gctggctaga gacccagagg cgcagagcgg agaggcctgc ggcgagg 117

<210> 324
<211> 166

<212> DNA
<213> Artificial Sequence

<220>
<223> Homo sapiens SUPT3H 5'-UTR
SUPT3H-006 ENST00000371459

<400> 324
cacagccgag tcaccttttc cctttctaca ctccacactc tcagtccccc accccgcccc 60

tttccaagcg tgtcccgggc cgcagcagca gaaaccgcac catctccacc cccacattct 120

cctcgcggga agcgcagcag tgcctccaag ggttcttaaa gcagag 166

<210> 325
<211> 176
<212> DNA
<213> Artificial Sequence

<220>
<223> Homo sapiens TMEM14A 5'-UTR
NM_014051.3

<400> 325
gtttccagga gggagcggcc tttgctcagc gcgagacggc tgggcgccga gtgggacagc 60

gctggtgcgg agactgcttc cggactccag gtaccgcgct tggcggcagc tggccccaga 120

cttctgtctt ttcagctgca gtgaaggctc ggggctgcag aattgcaacc ttgcca 176

<210> 326
<211> 222
<212> DNA
<213> Artificial Sequence

<220>
<223> Homo sapiens C9orf46 5'-UTR
AF225420.1

<400> 326
gagcgaggcc cggtccctgc agcgggcgaa aggagcccgg gcctggaggt ttgcgtaccg 60

gtcgcctggt cccggcacca gcgccgccca gtgtggtttc ccataaggaa gctcttcttc 120

ctgcttggct tccaccttta acccttccac ctgggagcgt cctctaacac attcagacta 180

caagtccaga cccaggagag caaggcccag aaagaggtca aa 222

<210> 327
<211> 227
<212> DNA
<213> Artificial Sequence

<220>
<223> Homo sapiens ANXA4 5'-UTR
NM_001153.3

<400> 327
gccccaggtg cgcttcccct agagagggat tttccggtct cgtgggcaga ggaacaacca 60

ggaacttggg ctcagtctcc accccacagt ggggcggatc cgtcccggat aagacccgct 120

gtctggccct gagtagggtg tgacctccgc agccgcagag gaggagcgca gcccggcctc 180

gaagaacttc tgcttgggtg gctgaactct gatcttgacc tagagtc 227

<210> 328
<211> 123
<212> DNA
<213> Artificial Sequence

<220>
<223> Homo sapiens IFI6 5'-UTR
NM_022873.2

<400> 328
ccagccttca gccggagaac cgtttactcg ctgctgtgcc catctatcag caggctccgg 60

gctgaagatt gcttctcttc tctcctccaa ggtctagtga cggagcccgc gcgcggcgcc 120

acc 123

<210> 329
<211> 58
<212> DNA
<213> Artificial Sequence

<220>
<223> Homo sapiens C2orf34 5'-UTR
CAMKMT -008 ENST00000402247

<400> 329
tcctggcagg ggacgagctg cggcggtggc acctccgggt gtggaaggct ccagtgag 58

<210> 330
<211> 104
<212> DNA
<213> Artificial Sequence

<220>
<223> Homo sapiens C2orf34 5'-UTR
NM_024766.3

<400> 330
gagggtgccg ggcgtcacag gtcctgacag ggaagaagtt ggcaggtcct ggcaggggac 60

gagctgcggc ggtggcacct ccgggtgtgg aaggctccag tgag 104

<210> 331
<211> 53
<212> DNA
<213> Artificial Sequence

<220>
<223> Homo sapiens ALDH6A1 5'-UTR
ALDH6A1-002 ENST00000350259

<400> 331
agtgcttctg ggcagtagag gcgcggggtg cggagctagg gcggccgaga gcc 53

<210> 332
<211> 117
<212> DNA
<213> Artificial Sequence

<220>
<223> Homo sapiens CCDC53 5'-UTR
CCDC53-002 ENST00000545679

<400> 332
ggaagggccc cggaggcggg cacttggggg gaaagttgag acgtgattac cgggttgggc 60

gggccccatc tgggaggggt ttgtgggtga actcggggtc caccgcccgc tgaggag 117


<210> 333
<211> 44
<212> DNA
<213> Artificial Sequence

<220>
<223> Homo sapiens CASP1 5'-UTR
NM_001257119.1

<400> 333
atactttcag tttcagtcac acaagaaggg aggagagaaa agcc 44


<210> 334
<211> 124
<212> DNA
<213> Artificial Sequence

<220>
<223> Homo sapiens NDUFB6 5'-UTR
NM_182739.2

<400> 334
gtaataaccg cgcgcggcgc tcggcgttcc cgcaaggtcg ctttgcagag cgggagcgcg 60

cttaagtaac tagtccgtag ttcgagggtg cgccgtgtcc ttttgcgttg gtaccagcgg 120

cgac 124


<210> 335
<211> 47
<212> DNA
<213> Artificial Sequence

<220>
<223> Homo sapiens BCKDHB 5'-UTR
BCKDHB-002 ENST00000369760

<400> 335
aggcggcgtg cggctgcata gcctgagaat cccggtggtg agcgggg 47


<210> 336
<211> 39
<212> DNA

<213>  Artificial Sequence

<220>
<223>  Homo sapiens BCKDHB 5′-UTR
NM_001164783.1

<400>  336
ctacgtgagt gccggaccgc tgagtggttg ttagccaag                              39

<210>  337
<211>  234
<212>  DNA
<213>  Artificial Sequence

<220>
<223>  Homo sapiens BBS2 5′-UTR
NM_031885.3

<400>  337
cacagaaggc gccgaggctc caccgcgcag ccgcaaaaag agcggacggg tctgcgccgc        60

cgcaggagga gcaggcggta cctggacggg ttcgtcccgg gctgtttcgc gtccggcctg       120

aggcggctgg ggccgcgcag gtagtgtccc tgcacttctt gcccgggcgc gtgaggccag       180

ctccgctgcg cttgtctcca gcttccagcc ctcctcccct aagccgccgc catc             234

<210>  338
<211>  153
<212>  DNA
<213>  Artificial Sequence

<220>
<223>  Homo sapiens HERC5 5′-UTR
HERC5-001 ENST00000264350

<400>  338
tcagtagctg aggctgcggt tccccgacgc cacgcagctg cgcgcagctg gttcccgctc        60

tgcagcgcaa cgcctgaggc agtgggcgcg ctcagtcccg ggaccaggcg ttctctcctc       120

tcgcctctgg gcctgggacc ccgcaaagcg gcg                                    153

<210>  339
<211>  65
<212>  DNA
<213>  Artificial Sequence

<220>
<223>  Homo sapiens FAM175A 5′-UTR
NM_139076.2

<400>  339
accacagggt cttgcctccg cgcgccccgc cctcgtcctc ttgtgtagcc tgaggcggcg        60

gtagc                                                                    65

<210>  340
<211>  82

<212> DNA
<213> Artificial Sequence

<220>
<223> Homo sapiens NT5DC1 5'-UTR
NT5DC1-002 ENST00000319550

<400> 340
cggtcctgtc ccgcagcgtc ccgccagcca gctccttgca cccttcgcgg ccgaggcgct 60

ccctggtgct ccccgcgcag cc 82

<210> 341
<211> 246
<212> DNA
<213> Artificial Sequence

<220>
<223> Homo sapiens RAB7A 5'-UTR
RAB7A-001 ENST00000265062

<400> 341
gtctcgtgac aggtacttcc gctcggggcg gcggcggtgg cggaagtggg agcgggcctg 60

gagtcttggc cataaagcct gaggcggcgg cagcggcgga gttggcggct tggagagctc 120

gggagagttc cctggaacca gaacttggac cttctcgctt ctgtcctccg tttagtctcc 180

tcctcggcgg gagccctcgc gacgcgcccg gcccggagcc cccagcgcag cggccgcgtt 240

tgaagg 246

<210> 342
<211> 128
<212> DNA
<213> Artificial Sequence

<220>
<223> Homo sapiens AGA 5'-UTR
AGA-001 ENST00000264595

<400> 342
agggacgcct gagcgaaccc ccgagagagc gggcgtgggc gccaggcggg cggggcactg 60

gggattaatt gttcggcgat cgctggctgc cgggactttt ctcgcgctgg tctcttcggt 120

ggtcaggg 128

<210> 343
<211> 103
<212> DNA
<213> Artificial Sequence

<220>
<223> Homo sapiens TPK1 5'-UTR
TPK1-001 ENST00000360057

<400> 343
aaggctcctc agccgagcgc cgagcggtcg atcgccgtag ctcccgcagc ctgcgatctc 60

cagtctgtgg ctcctaccag ccattgtagg ccaataatcc gtt 103

<210> 344
<211> 79
<212> DNA
<213> Artificial Sequence

<220>
<223> Homo sapiens MBNL3 5'-UTR
MBNL3-001 ENST00000370839

<400> 344
aattcatttt taatccttta atagtccaca gtaatattgt cctaaagagg gtacattgga 60

ttttaatttt gctttcaat 79

<210> 345
<211> 129
<212> DNA
<213> Artificial Sequence

<220>
<223> Homo sapiens MCCC2 5'-UTR
MCCC2-001 ENST00000340941

<400> 345
agaatcagag aaaccttctc tggggctgca aggacctgag ctcagcttcc gccccagcca 60

gggaagcggc aggggaaagc accggctcca ggccagcgtg ggccgctctc tcgctcggtg 120

cccgccgcc 129

<210> 346
<211> 89
<212> DNA
<213> Artificial Sequence

<220>
<223> Homo sapiens CAT 5'-UTR
CAT-001 ENST00000241052

<400> 346
actcggggca acaggcagat ttgcctgctg agggtggaga cccacgagcc gaggcctcct 60

gcagtgttct gcacagcaaa ccgcacgct 89

<210> 347
<211> 142
<212> DNA
<213> Artificial Sequence

<220>
<223> Homo sapiens ANAPC4 5'-UTR
ANAPC4-001 ENST00000315368

<400> 347
cccgacgccg gaagtgcctg gagcgcgcga cagcggcggg gcggggcggc ctggaggctg 60

tggcgcgcgg ccggcagagg gaggggagag gccactgggg ccgtgttagt ctgccggtgg 120

ggactcttgc agggccgtcc cc 142

<210> 348
<211> 24
<212> DNA
<213> Artificial Sequence

<220>
<223> Homo sapiens PHKB 5'-UTR
PHKB-002 ENST00000323584

<400> 348
ggccaaggcg gcgaccggag cgcg 24

<210> 349
<211> 25
<212> DNA
<213> Artificial Sequence

<220>
<223> Homo sapiens ABCB7 5'-UTR
ABCB7-001 ENST00000253577

<400> 349
ctcggttcct ctttcctcgc tcaag 25

<210> 350
<211> 133
<212> DNA
<213> Artificial Sequence

<220>
<223> Homo sapiens GPD2 5'-UTR
GPD2-002 ENST00000438166

<400> 350
cccgcgcgcc tcgctgggag cacccgggcc gaggctctga ttctgggggg aggccgactc 60

caccctggct ggaggaactg ggtgctcctg cccgctggcc cctcgcgcgt gaggatctat 120

ctcaggctaa gaa 133

<210> 351
<211> 117
<212> DNA
<213> Artificial Sequence

<220>
<223> Homo sapiens TMEM38B 5'-UTR
TMEM38B-001 ENST00000374692

<400> 351
gctggagccg gcgcggagga gcgggcggcc gcggctgtgc cctctcctac tcctcaccgc 60

gcgagcgcgg ggaaccagta gccgcggctg cttcggttgc cgcggtcggt ggtcgtt 117

<210> 352

<211> 206
<212> DNA
<213> Artificial Sequence

<220>
<223> Homo sapiens NFU1 5'-UTR
NM_001002755.2

<400> 352
gggaaaggtt ccccggcctc tcttggtcag ggtgacgcag tagcctgcaa acctcggcgc 60

gtaggccacc gcacttatcc gcagcaggac cgcccgcagc cggtagggtg ggctcttccc 120

agtgcccgcc cagctaccgg ccagcctgcg gctgcgcaga tctttcgtgg ttctgtcagg 180

gagaccctta ggcactccgg actaag 206

<210> 353
<211> 99
<212> DNA
<213> Artificial Sequence

<220>
<223> Homo sapiens LOC128322/NUTF2 5'-UTR
NM_005796.1

<400> 353
ggaagggaca gtcggccgca gaccgcgctg ggttgccgct gccgctgccg ccatcgtgcc 60

agcccctcgg gtctccgtga ggccgggtga cgctccaga 99

<210> 354
<211> 55
<212> DNA
<213> Artificial Sequence

<220>
<223> Homo sapiens NUBPL 5'-UTR
NM_025152.2

<400> 354
actccgcgcc acccgcgaca gtttcccagc agggctcaca gcagcgttcc gcgtc 55

<210> 355
<211> 75
<212> DNA
<213> Artificial Sequence

<220>
<223> Homo sapiens LANCL1 5'-UTR
LANCL1-004 ENST00000233714

<400> 355
gagaagggct tcaggacgcg ggaggcgcac ttgcttcaag tcgcgggcgt gggaacgggg 60

cttgcttccg gcgtc 75

<210> 356
<211> 204

<212> DNA
<213> Artificial Sequence

<220>
<223> Homo sapiens PIR 5'-UTR
PIR-002 ENST00000380420

<400> 356
cctcccgcct cctctaggcc gccggccgcg aagcgctgag tcacggtgag gctactggac 60

ccacactctc ttaacctgcc ctccctgcac tcgctcccgg cggctcttcg cgtcaccccc 120

gccgctaagg ctccaggtgc cgctaccgca gcccctccat cctctacagc tcagcatcag 180

aacactctct ttttagactc cgat 204

<210> 357
<211> 65
<212> DNA
<213> Artificial Sequence

<220>
<223> Homo sapiens CTBS 5'-UTR
NM_004388.2

<400> 357
gacgcgcagc aggccccgcc cacccaggcg gtaggaaccc actccggccc gctagacctg 60

ctgct 65

<210> 358
<211> 314
<212> DNA
<213> Artificial Sequence

<220>
<223> Homo sapiens GSTM4 5'-UTR
NM_000850.4

<400> 358
aagctggcga ggccgagccc ctcctagtgc ttccggacct tgctccctga acactcggag 60

gtggcggtgg atcttactcc ttccagccag tgaggatcca gcaacctgct ccgtgcctcc 120

cgcgcctgtt ggttggaagt gacgaccttg aagatcggcc ggttggaagt gacgaccttg 180

aagatcggcg ggcgcagcgg ggccgagggg gcgggtctgg cgctaggtcc agcccctgcg 240

tgccgggaac cccagaggag gtcgcagttc agcccagctg aggcctgtct gcagaatcga 300

caccaaccag catc 314

<210> 359
<211> 73
<212> DNA
<213> Artificial Sequence

<220>
<223> Mus musculus Ndufa1 5'-UTR
Ndufa1-001 ENSMUST00000016571

<400> 359
gccggaagag aggtaaagcc gggtcacctc tgaggagccg gtgacgggtt ggcgtgcgag 60

taacggtgcg gag 73

<210> 360
<211> 105
<212> DNA
<213> Artificial Sequence

<220>
<223> Mus musculus Atp5e 5'-UTR
NM_025983

<400> 360
cccacccctt ccgctactca ggcctgacct tcctgctgcc gggccggttt gaggctactc 60

tgaagcgacc cagcggttct gcccgacgcg cccgctcgag acacc 105

<210> 361
<211> 100
<212> DNA
<213> Artificial Sequence

<220>
<223> Mus musculus Gstm5 5'-UTR
NM_010360

<400> 361
gagacagttc ggtcgcgtca gcccggccca cagcgtccag tataaagtta gccgcccaca 60

gtccatcgct gtatccccga aggggctaag atcgcccaaa 100

<210> 362
<211> 49
<212> DNA
<213> Artificial Sequence

<220>
<223> Mus musculus Cbr2 5'-UTR
NM_007621

<400> 362
ataaaagctg agcccatctc ttgcttcgga agaagctggt gtcagcagc 49

<210> 363
<211> 78
<212> DNA
<213> Artificial Sequence

<220>
<223> Mus musculus Anapc13 5'-UTR
NM_181394

<400> 363
gtgacccaga agaagggcgg ggccgggagg aagccgacgc gcgcgcagtg ggcctgacaa 60

gatcaaagct gcaggagg 78

<210> 364
<211> 16
<212> DNA
<213> Artificial Sequence

<220>
<223> Mus musculus Ndufa7 5'-UTR
NM_023202

<400> 364
tcggagcgga aggaat 16

<210> 365
<211> 17
<212> DNA
<213> Artificial Sequence

<220>
<223> Mus musculus Atp5k 5'-UTR
NM_007507

<400> 365
cgaaggtcac ggacaaa 17

<210> 366
<211> 67
<212> DNA
<213> Artificial Sequence

<220>
<223> Mus musculus Cox4i1 5'-UTR
NM_009941

<400> 366
cttccggtcg cgagcacccc agggtgtaga gggcggtcgc ggcggtcgcc tgggcagcgg 60

tggcaga 67

<210> 367
<211> 36
<212> DNA
<213> Artificial Sequence

<220>
<223> Mus musculus Ndufs6 5'-UTR
NM_010888

<400> 367
ttggtacgac gcgtggggtc aagggtcacc ggcaag 36

<210> 368
<211> 90
<212> DNA
<213> Artificial Sequence

<220>
<223> Mus musculus Sec61b 5'-UTR

NM_024171

<400> 368
agagcctgta tctacgagag ttctgagtgc tcggcaactt cacgacttcc ctcttcctgc 60

ctcctgtgcc caccgttctt aggcatcagc 90

<210> 369
<211> 55
<212> DNA
<213> Artificial Sequence

<220>
<223> Mus musculus Snrpd2 5'-UTR
NM_026943

<400> 369
aaggctggag caacgcgctt ggaggcggga gtgatctgcg agcgaaacct acacc 55

<210> 370
<211> 54
<212> DNA
<213> Artificial Sequence

<220>
<223> Mus musculus Mgst3 5'-UTR
NM_025569

<400> 370
actgctgtgc ttctcaggtc tgtaccaggc gcacgaaggt gagccagagc caag 54

<210> 371
<211> 54
<212> DNA
<213> Artificial Sequence

<220>
<223> Mus musculus Mp68 (2010107E04Rik) 5'-UTR
NM_027360

<400> 371
ctttcccatt ctgtagcaga atttggtgtt gcctgtggtc ttggtcccgc ggag 54

<210> 372
<211> 92
<212> DNA
<213> Artificial Sequence

<220>
<223> Mus musculus Prdx4-001, 5'-UTR
NM_016764

<400> 372
gcgcggtctc cagcgcgccg ttttagctgg ctgcctggcg gcaggggact ctgtgcttta 60

gcagagggac gtgttttcgc gcttgcttgg tc 92

<210> 373

<211> 215
<212> DNA
<213> Artificial Sequence

<220>
<223> Mus musculus Pgcp 5'-UTR
NM_176073

<400> 373
gctgtcctgg cacacaaaga agccaggcct gcagactact ggggctccgg gctgttcctg 60

aggcctctgg aggcccgccc tgtggctcca gtgcgctctg aggaccttcc tggtcccgcc 120

cccgaacgtg cctgtggtct gcaggcctca ccgggtgttg tggccgctgc tgctccgcag 180

agcctcgtga tcaggaagaa aagcaactag gaaca 215

<210> 374
<211> 43
<212> DNA
<213> Artificial Sequence

<220>
<223> Mus musculus Myeov2 5'-UTR
NM_001163425

<400> 374
agaaggggct ggccggaagt gagcgcaacg ccgccttgtc gag 43

<210> 375
<211> 81
<212> DNA
<213> Artificial Sequence

<220>
<223> Mus musculus Ndufa4 5'-UTR
NM_010886

<400> 375
gtccgctcag ccaggttgca gaagcggctt agcgtgtgtc ctaatcttct ctctgcgtgt 60

aggtaggcct gtgccgcaaa c 81

<210> 376
<211> 76
<212> DNA
<213> Artificial Sequence

<220>
<223> Mus musculus Ndufs5 5'-UTR
NM_001030274

<400> 376
acggcaggcg tctgcgtcct cccgcagccg gcggtcggga attgcaccag ggacctgaca 60

agggcactgc agagcc 76

<210> 377
<211> 198

<212> DNA
<213> Artificial Sequence

<220>
<223> Mus musculus Gstm1 5'-UTR
NM_010358

<400> 377
ctgccttccg ctttagggtc tgctgctctg gttacagacc taggaagggg agtgcctaat 60

tgggattggt gcagggttgg gagggacccg ctgttttgtc ctgcccacgt ttctctagta 120

gtctgtataa agtcacaact ccaaacacac aggtcagtcc tgctgaagcc agtttgagaa 180

gaccacagca ccagcacc 198

<210> 378
<211> 75
<212> DNA
<213> Artificial Sequence

<220>
<223> Mus musculus Atp5o 5'-UTR
NM_138597

<400> 378
ctggcgcgcg cgcgtgcgct ctggcgccag tagtctcttt tcatttgggt ttgacctaca 60

gccgcccggg aaaag 75

<210> 379
<211> 101
<212> DNA
<213> Artificial Sequence

<220>
<223> Mus musculus Tspo 5'-UTR
NM_009775

<400> 379
gtcagcggct accaacctct gtgcgcagtg tccttcacgg aacaaccagc gactgcgtga 60

gcggggctgt ggatctttcc agaacatcag ttgcaatcac c 101

<210> 380
<211> 62
<212> DNA
<213> Artificial Sequence

<220>
<223> Mus musculus Taldo1 5'-UTR
NM_011528

<400> 380
gacgcgcggg gcattgtggg ttagcacgca ccggctaccg cctcagctgt tcgcgtttcg 60

cc 62

<210> 381

<211>  89
<212>  DNA
<213>  Artificial Sequence

<220>
<223>  Mus musculus Bloc1s1 5'-UTR
NM_015740

<400>  381
gtgacgcctt ccgggtgagc caaggcatag tccagttcct gcagccttag ggaggggtcc      60

gccgtgccca cacccagcca gactcgacc                                        89


<210>  382
<211>  57
<212>  DNA
<213>  Artificial Sequence

<220>
<223>  Mus musculus Hexa 5'-UTR
NM_010421

<400>  382
agctgaccgg ggctcacgtg ggctcagcct gctggaaggg gagctggccg gtgggcc         57


<210>  383
<211>  1810
<212>  RNA
<213>  Artificial Sequence

<220>
<223>  32L4 - PpLuc(GC) - A64-C30-hSL

<400>  383
gggagaaagc uugaggaugg aggacgccaa gaacaucaag aagggcccgg cgcccuucua      60

cccgcuggag gacgggaccg ccggcgagca gcuccacaag gccaugaagc gguacgcccu     120

ggugccgggc acgaucgccu ucaccgacgc ccacaucgag gucgacauca ccuacgcgga     180

guacuucgag augagcgugc gccuggccga ggccaugaag cgguacggcc ugaacaccaa     240

ccaccggauc guggugugcu cggagaacag ccugcaguuc uucaugccgg ugcugggcgc     300

ccucuucauc ggcguggccg ucgccccggc gaacgacauc uacaacgagc gggagcugcu     360

gaacagcaug gggaucagcc agccgaccgu gguguucgug agcaagaagg gccugcagaa     420

gauccugaac gugcagaaga agcugcccau cauccagaag aucaucauca uggacagcaa     480

gaccgacuac cagggcuucc agucgaugua cacguucgug accagccacc ucccgccggg     540

cuucaacgag uacgacuucg ucccggagag cuucgaccgg gacaagacca ucgcccugau     600

caugaacagc agcggcagca ccggccugcc gaagggggug gcccugccgc accggaccgc     660

cugcgugcgc uucucgcacg cccgggaccc caucuucggc aaccagauca ucccggacac     720

cgccauccug agcguggugc cguuccacca cggcuucggc auguucacga cccugggcua     780

ccucaucugc ggcuuccggg ugguccugau guaccgguuc gaggaggagc uguuccugcg     840

gagccugcag gacuacaaga uccagagcgc gcugcucgug ccgacccugu ucagcuucuu 900

cgccaagagc acccugaucg acaaguacga ccugucgaac cugcacgaga ucgccagcgg 960

gggcgccccg cugagcaagg aggugggcga ggccguggcc aagcgguucc accucccggg 1020

cauccgccag ggcuacggcc ugaccgagac cacgagcgcg auccugauca cccccgaggg 1080

ggacgacaag ccgggcgccg ugggcaaggu ggucccguuc uucgaggcca agguggugga 1140

ccuggacacc ggcaagaccc ugggcgugaa ccagcggggc gagcugugcg ugcgggggcc 1200

gaugaucaug agcggcuacg ugaacaaccc ggaggccacc aacgcccuca ucgacaagga 1260

cggcuggcug cacagcggcg acaucgccua cugggacgag gacgagcacu ucuucaucgu 1320

cgaccggcug aagucgcuga ucaaguacaa gggcuaccag guggcgccgg ccgagcugga 1380

gagcauccug cuccagcacc ccaacaucuu cgacgccggc guggccgggc ugccggacga 1440

cgacgccggc gagcugccgg ccgcggug gu ggugcuggag cacggcaaga ccaugacgga 1500

gaaggagauc gucgacuacg uggccagcca ggugaccacc gccaagaagc ugcggggcgg 1560

cgugguguuc guggacgagg ucccgaaggg ccugaccggg aagcucgacg cccggaagau 1620

ccgcgagauc cugaucaagg ccaagaaggg cggcaagauc gccguguaag acuaguagau 1680

cuaaaaaaaa aaaaaaaaaa aaaaaaaaaa aaaaaaaaaa aaaaaaaaaa aaaaaaaaaa 1740

aaaaaaugca uccccccccc cccccccccc cccccccccc ccaaaggcuc uuuucagagc 1800

caccagaauu 1810

<210> 384
<211> 1927
<212> RNA
<213> Artificial Sequence

<220>
<223> PpLuc(GC) - morn2- A64 - C30 - hSL

<400> 384
gggagaaagc uugaggaugg aggacgccaa gaacaucaag aagggcccgg cgcccuucua 60

cccgcuggag gacgggaccg ccggcgagca gcuccacaag gccaugaagc gguacgcccu 120

ggugccgggc acgaucgccu ucaccgacgc ccacaucgag gucgacauca ccuacgcgga 180

guacuucgag augagcgugc gccuggccga ggccaugaag cgguacggcc ugaacaccaa 240

ccaccggauc guggugugcu cggagaacag ccugcaguuc uucaugccgg ugcugggcgc 300

ccucuucauc ggcguggccg ucgccccggc gaacgacauc uacaacgagc gggagcugcu 360

gaacagcaug ggaucagcc agccgaccgu gguguucgug agcaagaagg gccugcagaa 420

gauccugaac gugcagaaga agcugcccau cauccagaag aucaucauca uggacagcaa 480

gaccgacuac cagggcuucc agucgaugua cacguucgug accagccacc ucccgccggg 540

cuucaacgag uacgacuucg ucccggagag cuucgaccgg gacaagacca ucgcccugau 600

caugaacagc agcggcagca ccggccugcc gaaggggggug gcccugccgc accggaccgc 660

cugcgugcgc uucucgcacg cccgggaccc caucuucggc aaccagauca ucccggacac 720

cgccauccug agcguggugc cguuccacca cggcuucggc auguucacga cccugggcua 780

ccucaucugc ggcuuccggg ugguccugau guaccgguuc gaggaggagc uguuccugcg 840

gagccugcag gacuacaaga uccagagcgc gcugcucgug ccgacccugu ucagcuucuu 900

cgccaagagc acccugaucg acaaguacga ccugucgaac cugcacgaga ucgccagcgg 960

gggcgccccg cugagcaagg aggugggcga ggccguggcc aagcgguucc accucccggg 1020

cauccgccag ggcuacggcc ugaccgagac cacgagcgcg auccugauca cccccgaggg 1080

ggacgacaag ccgggcgccg ugggcaaggu ggucccguuc uucgaggcca agguggugga 1140

ccuggacacc ggcaagaccc ugggcgugaa ccagcggggc gagcugugcg ugcgggggcc 1200

gaugaucaug agcggcuacg ugaacaaccc ggaggccacc aacgcccuca ucgacaagga 1260

cggcuggcug cacagcggcg acaucgccua cugggacgag gacgagcacu ucuucaucgu 1320

cgaccggcug aagucgcuga ucaaguacaa gggcuaccag guggcgccgg ccgagcugga 1380

gagcauccug cuccagcacc ccaacaucuu cgacgccggc guggccgggc ugccggacga 1440

cgacgccggc gagcugccgg ccgcgguggu ggugcuggag cacggcaaga ccaugacgga 1500

gaaggagauc gucgacuacg uggccagcca ggugaccacc gccaagaagc ugcggggcgg 1560

cgugguguuc guggacgagg ucccgaaggg ccugaccggg aagcucgacg cccggaagau 1620

ccgcgagauc cugaucaagg ccaagaaggg cggcaagauc gccguguaag acuaguaccu 1680

gcugccuuaa cgcugagaug uggccucugc aaccccccuu aggcaaagca acugaaccuu 1740

cugcuaaagu gaccugcccu cuuccguaag uccaauaaag uugucaugca cccagaucua 1800

aaaaaaaaaa aaaaaaaaaa aaaaaaaaaa aaaaaaaaaa aaaaaaaaaa aaaaaaaaaa 1860

aaaugcaucc cccccccccc cccccccccc cccccccccca aaggcucuuu ucagagccac 1920

cagaauu 1927

<210> 385
<211> 1943
<212> RNA
<213> Artificial Sequence

<220>
<223> PpLuc(GC) - ndufa1- A64 - C30 - hSL

<400> 385
gggagaaagc uugaggaugg aggacgccaa gaacaucaag aagggcccgg cgcccuucua 60

cccgcuggag gacgggaccg ccggcgagca gcuccacaag gccaugaagc gguacgcccu 120

ggugccgggc acgaucgccu ucaccgacgc ccacaucgag gucgacauca ccuacgcgga 180

guacuucgag augagcgugc gccuggccga ggccaugaag cgguacggcc ugaacaccaa 240

ccaccggauc guggugugcu cggagaacag ccugcaguuc uucaugccgg ugcugggcgc 300

ccucuucauc ggcguggccg ucgccccggc gaacgacauc uacaacgagc gggagcugcu 360

gaacagcaug ggaucagcc agccgaccgu gguguucgug agcaagaagg gccugcagaa 420

gauccugaac gugcagaaga agcugcccau cauccagaag aucaucauca uggacagcaa 480

gaccgacuac cagggcuucc agucgaugua cacguucgug accagccacc ucccgccggg 540

cuucaacgag uacgacuucg ucccggagag cuucgaccgg gacaagacca ucgcccugau 600

caugaacagc agcggcagca ccggccugcc gaaggggggug gcccugccgc accggaccgc 660

cugcgugcgc uucucgcacg cccgggaccc caucuucggc aaccagauca ucccggacac 720

cgccauccug agcguggugc cguuccacca cggcuucggc auguucacga cccugggcua 780

ccucaucugc ggcuuccggg ugguccugau guaccgguuc gaggaggagc uguuccugcg 840

gagccugcag gacuacaaga uccagagcgc gcugcucgug ccgacccugu ucagcuucuu 900

cgccaagagc acccugaucg acaaguacga ccugucgaac cugcacgaga ucgccagcgg 960

gggcgccccg cugagcaagg aggugggcga ggccguggcc aagcgguucc accucccggg 1020

cauccgccag ggcuacggcc ugaccgagac cacgagcgcg auccugauca cccccgaggg 1080

ggacgacaag ccgggcgccg ugggcaaggu ggucccguuc uucgaggcca agguggugga 1140

ccuggacacc ggcaagaccc ugggcgugaa ccagcggggc gagcugugcg ugcgggggcc 1200

gaugaucaug agcggcuacg ugaacaaccc ggaggccacc aacgcccuca ucgacaagga 1260

cggcuggcug cacagcggcg acaucgccua cugggacgag gacgagcacu ucuucaucgu 1320

cgaccggcug aagucgcuga ucaaguacaa gggcuaccag guggcgccgg ccgagcugga 1380

gagcauccug cuccagcacc ccaacaucuu cgacgccggc guggccgggc ugccggacga 1440

cgacgccggc gagcugccgg ccgcggugggu ggugcuggag cacggcaaga ccaugacgga 1500

gaaggagauc gucgacuacg uggccagcca ggugaccacc gccaagaagc ugcggggcgg 1560

cgugguguuc guggacgagg ucccgaaggg ccugaccggg aagcucgacg cccggaagau 1620

ccgcgagauc cugaucaagg ccaagaaggg cggcaagauc gccguguaag acuaguggaa 1680

gcauuuuccu ggcugauuaa aagaaauuac ucagcuaugg ucaucuguuc cuguuagaag 1740

gcuaugcagc auauuauaua cuaugcgcau guuaugaaau gcauaauaaa aaauuuuaaa 1800

aaaucuaaaa gaucuaaaaa aaaaaaaaaa aaaaaaaaaa aaaaaaaaaa aaaaaaaaaa 1860

aaaaaaaaaa aaaaaaaaau gcaucccccc cccccccccc cccccccccc cccccaaagg 1920

cucuuuucag agccaccaga auu 1943

<210> 386

<211> 1941
<212> RNA
<213> Artificial Sequence

<220>
<223> PpLuc(GC) - NDUFA1- A64 - C30 - hSL

<400> 386
gggagaaagc uugaggaugg aggacgccaa gaacaucaag aagggcccgg cgcccuucua 60

cccgcuggag gacgggaccg ccggcgagca gcuccacaag gccaugaagc gguacgcccu 120

ggugccgggc acgaucgccu ucaccgacgc ccacaucgag gucgacauca ccuacgcgga 180

guacuucgag augagcgugc gccuggccga ggccaugaag cgguacggcc ugaacaccaa 240

ccaccggauc guggugugcu cggagaacag ccugcaguuc uucaugccgg ugcugggcgc 300

ccucuucauc ggcguggccg ucgccccggc gaacgacauc uacaacgagc gggagcugcu 360

gaacagcaug ggaucagcc agccgaccgu gguguucgug agcaagaagg gccugcagaa 420

gauccugaac gugcagaaga agcugcccau cauccagaag aucaucauca uggacagcaa 480

gaccgacuac cagggcuucc agucgaugua cacguucgug accagccacc ucccgccggg 540

cuucaacgag uacgacuucg ucccggagag cuucgaccgg gacaagacca ucgcccugau 600

caugaacagc agcggcagca ccggccugcc gaagggggug gcccugccgc accggaccgc 660

cugcgugcgc uucucgcacg cccgggaccc caucuucggc aaccagauca ucccggacac 720

cgccauccug agcguggugc cguuccacca cggcuucggc auguucacga cccugggcua 780

ccucaucugc ggcuuccggg ugguccugau guaccgguuc gaggaggagc uguuccugcg 840

gagccugcag gacuacaaga uccagagcgc gcugcucgug ccgacccugu ucagcuucuu 900

cgccaagagc acccugaucg acaaguacga ccugucgaac cugcacgaga ucgccagcgg 960

gggcgccccg cugagcaagg aggugggcga ggccguggcc aagcgguucc accucccggg 1020

cauccgccag ggcuacggcc ugaccgagac cacgagcgcg auccugauca cccccgaggg 1080

ggacgacaag ccgggcgccg ugggcaaggu ggucccguuc uucgaggcca agguggugga 1140

ccuggacacc ggcaagaccc ugggcgugaa ccagcggggc gagcugugcg ugcgggggcc 1200

gaugaucaug agcggcuacg ugaacaaccc ggaggccacc aacgcccuca ucgacaagga 1260

cggcuggcug cacagcggcg acaucgccua cugggacgag gacgagcacu ucuucaucgu 1320

cgaccggcug aagucgcuga ucaaguacaa gggcuaccag guggcgccgg ccgagcugga 1380

gagcauccug cuccagcacc ccaacaucuu cgacgccggc guggccgggc ugccggacga 1440

cgacgccggc gagcugccgg ccgcggugu ggugcuggag cacggcaaga ccaugacgga 1500

gaaggagauc gucgacuacg uggccagcca ggugaccacc gccaagaagc ugcggggcgg 1560

cgugguguuc guggacgagg ucccgaaggg ccugaccggg aagcucgacg cccggaagau 1620

ccgcgagauc cugaucaagg ccaagaaggg cggcaagauc gccguguaag acuaguggaa 1680

gcauuuuccu gauugaugaa aaaaauaacu caguuauggc caucuacccc ugcuagaagg 1740

uuacagugua uuauguagca ugcaaugugu uauguagugc uuaauaaaaa uaaaaugaaa 1800

aaaaugcaga ucuaaaaaaa aaaaaaaaaa aaaaaaaaaa aaaaaaaaaa aaaaaaaaaa 1860

aaaaaaaaaa aaaaaaaugc aucccccccc cccccccccc cccccccccc cccaaaggcu 1920

cuuuucagag ccaccagaau u 1941

<210> 387
<211> 1861
<212> RNA
<213> Artificial Sequence

<220>
<223> Mp68 - PpLuc(GC) - A64 - C30 - hSL

<400> 387
gggcuuuccc auucuguagc agaauuuggu guugccugug gucuugguccc cgcggagaag 60

cuugaggaug gaggacgcca agaacaucaa gaagggcccg gcgcccuucu acccgcugga 120

ggacgggacc gccggcgagc agcuccacaa ggccaugaag cgguacgccc uggugccggg 180

cacgaucgcc uucaccgacg cccacaucga ggucgacauc accuacgcgg aguacuucga 240

gaugagcgug cgccuggccg aggccaugaa gcgguacggc cugaacacca accaccggau 300

cgugguguge ucggagaaca gccugcaguu cuucaugccg gugcugggcg cccucuucau 360

cggcguggcc gucgccccgg cgaacgacau cuacaacgag cgggagcugc ugaacagcau 420

ggggaucagc cagccgaccg ugguguucgu gagcaagaag ggccugcaga agauccugaa 480

cgugcagaag aagcugccca ucauccagaa gaucaucauc auggacagca agaccgacua 540

ccagggcuuc cagucgaugu acacguucgu gaccagccac cucccgccgg gcuucaacga 600

guacgacuuc gucccggaga gcuucgaccg ggacaagacc aucgcccuga ucaugaacag 660

cagcggcagc accggccugc cgaagggggu ggcccugccg caccggaccg ccugcgugcg 720

cuucucgcac gcccgggacc ccaucuucgg caaccagauc aucccggaca ccgccauccu 780

gagcguggug ccguuccacc acggcuucgg cauguucacg acccugggcu accucaucug 840

cggcuuccgg gugguccuga uguaccgguu cgaggaggag cuguuccugc ggagccugca 900

ggacuacaag auccagagcg cgcugcucgu gccgacccug uucagcuucu ucgccaagag 960

cacccugauc gacaaguacg accugucgaa ccugcacgag aucgccagcg gggcgccccc 1020

gcugagcaag gaggugggcg aggccguggc caagcgguuc caccucccgg gcauccgcca 1080

gggcuacggc cugaccgaga ccacgagcgc gauccugauc acccccgagg gggacgacaa 1140

gccgggcgcc gugggcaagg ugguccccguu cuucgaggcc aagguggugg accuggacac 1200

cggcaagacc cugggcguga accagcgggg cgagcugugc gugcgggggc cgaugaucau 1260

gagcggcuac gugaacaacc cggaggccac caacgcccuc aucgacaagg acggcuggcu 1320

gcacagcggc gacaucgccu acugggacga ggacgagcac uucuucaucg ucgaccggcu 1380

gaagucgcug aucaaguaca agggcuacca gguggcgccg gccgagcugg agagcauccu 1440

gcuccagcac cccaacaucu ucgacgccgg cguggccggg cugccggacg acgacgccgg 1500

cgagcugccg gccgcggugg uggugcugga gcacggcaag accaugacgg agaaggagau 1560

cgucgacuac guggccagcc aggugaccac cgccaagaag cugcggggcg gcgugguguu 1620

cguggacgag gucccgaagg gccugaccgg gaagcucgac gcccggaaga uccgcgagau 1680

ccugaucaag gccaagaagg gcggcaagau cgccguguaa gacuaguaga ucuaaaaaaa 1740

aaaaaaaaaa aaaaaaaaaa aaaaaaaaaa aaaaaaaaaa aaaaaaaaaa aaaaaaaugc 1800

aucccccccc cccccccccc cccccccccc cccaaaggcu cuuuucagag ccaccagaau 1860

u 1861

<210> 388
<211> 1888
<212> RNA
<213> Artificial Sequence

<220>
<223> Ndufa4 - PpLuc(GC) - A64 - C30 - hSL

<400> 388
gggguccgcu cagccagguu gcagaagcgg cuuagcgugu guccuaaucu ucucucugcg 60

uguagguagg ccugugccgc aaacaagcuu gaggauggag gacgccaaga acaucaagaa 120

gggcccggcg cccuucuacc cgcuggagga cgggaccgcc ggcgagcagc uccacaaggc 180

caugaagcgg uacgcccugg ugccgggcac gaucgccuuc accgacgccc acaucgaggu 240

cgacaucacc uacgcggagu acuucgagau gagcgugcgc cuggccgagg ccaugaagcg 300

guacggccug aacaccaacc accggaucgu ggugugcucg gagaacagcc ugcaguucuu 360

caugccggug cugggcgccc ucuucaucgg cguggccguc gccccggcga acgacaucua 420

caacgagcgg gagcugcuga acagcauggg gaucagccag ccgaccgugg uguucgugag 480

caagaagggc cugcagaaga uccugaacgu gcagaagaag cugcccauca uccagaagau 540

caucaucaug gacagcaaga ccgacuacca gggcuuccag ucgauguaca cguucgugac 600

cagccaccuc ccgccgggcu ucaacgagua cgacuucguc ccggagagcu ucgaccggga 660

caagaccauc gcccugauca ugaacagcag cggcagcacc ggccugccga agggggugc 720

ccugccgcac cggaccgccu gcgugcgcuu cucgcacgcc cgggacccca ucuucggcaa 780

ccagaucauc ccggacaccg ccauccugag cgugguugccg uuccaccacg gcuucggcau 840

guucacgacc cugggcuacc ucaucugcgg cuuccgggug guccugaugu accgguucga 900

ggaggagcug uuccugcgga gccugcagga cuacaagauc cagagcgcgc ugcucgugcc 960

```
gacccuguuc agcuucuucg ccaagagcac ccugaucgac aaguacgacc ugucgaaccu    1020
gcacgagauc gccagcgggg gcgccccgcu gagcaaggag gugggcgagg ccguggccaa    1080
gcgguuccac cucccgggca uccgccaggg cuacggccug accgagacca cgagcgcgau    1140
ccugaucacc cccgaggggg acgacaagcc gggcgccgug ggcaaggugg ucccguucuu    1200
cgaggccaag gugguggacc uggacaccgg caagacccug ggcgugaacc agcggggcga    1260
gcugugcgug cgggggccga ugaucaugag cggcuacgug aacaacccgg aggccaccaa    1320
cgcccucauc gacaaggacg gcuggcugca cagcggcgac aucgccuacu gggacgagga    1380
cgagcacuuc uucaucgucg accggcugaa gucgcugauc aaguacaagg gcuaccaggu    1440
ggcgccggcc gagcuggaga gcauccugcu ccagcacccc aacaucuucg acgccggcgu    1500
ggccgggcug ccggacgacg acgccggcga gcugccggcc gcgguggugg ugcuggagca    1560
cggcaagacc augacggaga aggagaucgu cgacuacgug gccagccagg ugaccaccgc    1620
caagaagcug cggggcggcg ugguguucgu ggacgagguc ccgaagggcc ugaccgggaa    1680
gcucgacgcc cggaagaucc gcgagauccu gaucaaggcc aagaagggcg gcaagaucgc    1740
cguguaagac uaguagaucu aaaaaaaaaa aaaaaaaaaa aaaaaaaaaa aaaaaaaaaa    1800
aaaaaaaaaa aaaaaaaaaa aaaaugcauc cccccccccc cccccccccc cccccccccc    1860
aaaggcucuu uucagagcca ccagaauu                                       1888
```

## Description

The present invention was made with support from the Government under Agreement No. HR0011-11-3-0001 awarded by DARPA. The Government has certain rights in the invention.This application claims the priority of international patent application PCT/EP2014/003479 filed on December 30, 2014, which is incorporated herein by reference.

The invention relates to artificial nucleic acid molecules comprising an open reading frame, a 3'-untranslated region element (3'-UTR element) and/or a 5'-untranslated region element (5'-UTR element) and optionally a poly(A) sequence and/or a polyadenylation-signal. The invention relates further to a vector comprising a 3'-UTR element and/or a 5'-UTR element, to a cell comprising the artificial nucleic acid molecule or the vector, to a pharmaceutical composition comprising the artificial nucleic acid molecule or the vector and to a kit comprising the artificial nucleic acid molecule, the vector and/or the pharmaceutical composition, preferably for use in the field of gene therapy and/or genetic vaccination. Gene therapy and genetic vaccination belong to the most promising and quickly developing methods of modern medicine. They may provide highly specific and individual options for therapy of a large variety of diseases. Particularly, inherited genetic diseases but also autoimmune diseases, cancerous or tumour-related diseases as well as inflammatory diseases may be the subject of such treatment approaches. Also, it is envisaged to prevent early onset of such diseases by these approaches.

The main conceptual rational behind gene therapy is appropriate modulation of impaired gene expression associated with pathological conditions of specific diseases. Pathologically altered gene expression may result in lack or overproduction of essential gene products, for example, signalling factors such as hormones, housekeeping factors, metabolic enzymes, structural proteins or the like. Altered gene expression may not only be due to mis-regulation of transcription and/or translation, but also due to mutations within the ORF coding for a particular protein. Pathological mutations may be caused by e.g. chromosomal aberration, or by more specific mutations, such as point or frame-shift-mutations, all of them resulting in limited functionality and, potentially, total loss of function of the gene product. However, misregulation of transcription or translation may also occur, if mutations affect genes encoding proteins which are involved in the transcriptional or translational machinery of the cell. Such mutations may lead to pathological up- or down-regulation of genes which are - as such - functional. Genes encoding gene products which exert such regulating functions, may be, e.g., transcription factors, signal receptors, messenger proteins or the like. However, loss of function of such genes encoding regulatory proteins may, under certain circumstances, be reversed by artificial introduction of other factors acting further downstream of the impaired gene product. Such gene defects may also be compensated by gene therapy via substitution of the affected gene itself.

Genetic vaccination allows evoking a desired immune response to selected antigens, such as characteristic components of bacterial surfaces, viral particles, tumour antigens or the like. Generally, vaccination is one of the pivotal achievements of modern medicine. However, effective vaccines are currently available only for a limited number of diseases. Accordingly, infections that are not preventable by vaccination still affect millions of people every year.

Commonly, vaccines may be subdivided into "first", "second" and "third" generation vaccines. "First generation" vaccines are, typically, whole-organism vaccines. They are based on either live and attenuated or killed pathogens, e.g. viruses, bacteria or the like. The major drawback of live and attenuated vaccines is the risk for a reversion to life-threatening variants. Thus, although attenuated, such pathogens may still intrinsically bear unpredictable risks. Killed pathogens may not be as effective as desired for generating a specific immune response. In order to minimize these risks, "second generation" vaccines were developed. These are, typically, subunit vaccines, consisting of defined antigens or recombinant protein components which are derived from pathogens.

Genetic vaccines, i.e. vaccines for genetic vaccination, are usually understood as "third generation" vaccines. They are typically composed of genetically engineered nucleic acid molecules which allow expression of peptide or protein (antigen) fragments characteristic for a pathogen or a tumor antigen *in vivo.* Genetic vaccines are expressed upon administration to a patient after uptake by target cells. Expression of the administered nucleic acids results in production of the encoded proteins. In the event these proteins are recognized as foreign by the patient's immune system, an immune response is triggered.

As can be seen from the above, both methods, gene therapy and genetic vaccination, are essentially based on the administration of nucleic acid molecules to a patient and subsequent transcription and/or translation of the encoded genetic information. Alternatively, genetic vaccination or gene therapy may also comprise methods which include isolation of specific body cells from a patient to be treated, subsequent *in ex vivo* transfection of such cells, and re-administration of the treated cells to the patient.

DNA as well as RNA may be used as nucleic acid molecules for administration in the context of gene therapy or genetic vaccination. DNA is known to be relatively stable and easy to handle. However, the use of DNA bears the risk of undesired insertion of the administered DNA-fragments into the patient's genome potentially resulting mutagenic events such as in loss of function of the impaired genes. As a further risk, the undesired generation of anti-DNA antibodies has emerged. Another drawback is the limited expression level of the encoded peptide or protein that is achievable upon DNA administration because the DNA must enter the nucleus in order to be transcribed before the resulting mRNA can

be translated. Among other reasons, the expression level of the administered DNA will be dependent on the presence of specific transcription factors which regulate DNA transcription. In the absence of such factors, DNA transcription will not yield satisfying amounts of RNA. As a result, the level of translated peptide or protein obtained is limited.

By using RNA instead of DNA for gene therapy or genetic vaccination, the risk of undesired genomic integration and generation of anti-DNA antibodies is minimized or avoided. However, RNA is considered to be a rather unstable molecular species which may readily be degraded by ubiquitous RNAses.

Typically, RNA degradation contributes to the regulation of the RNA half-life time. That effect was considered and proven to fine tune the regulation of eukaryotic gene expression (Friedel et al., 2009. Conserved principles of mammalian transcriptional regulation revealed by RNA half-life, Nucleic Acid Research 37(17): 1-12). Accordingly, each naturally occurring mRNA has its individual half-life depending on the gene from which the mRNA is derived and in which cell type it is expressed. It contributes to the regulation of the expression level of this gene. Unstable RNAs are important to realize transient gene expression at distinct points in time. However, long-lived RNAs may be associated with accumulation of distinct proteins or continuous expression of genes. *In vivo,* the half-life of mRNAs may also be dependent on environmental factors, such as hormonal treatment, as has been shown, e.g., for insulin-like growth factor I, actin, and albumin mRNA (Johnson et al., Newly synthesized RNA: Simultaneous measurement in intact cells of transcription rates and RNA stability of insulin-like growth factor I, actin, and albumin in growth hormone-stimulated hepatocytes, Proc. Natl. Acad. Sci., Vol. 88, pp. 5287-5291, 1991).

For gene therapy and genetic vaccination, usually stable RNA is desired. This is, on the one hand, due to the fact that it is usually desired that the product encoded by the RNA sequence accumulates *in vivo.* On the other hand, the RNA has to maintain its structural and functional integrity when prepared for a suitable dosage form, in the course of its storage, and when administered. Thus, efforts were made to provide stable RNA molecules for gene therapy or genetic vaccination in order to prevent them from being subject to early degradation or decay.

It has been reported that the G/C-content of nucleic acid molecules may influence their stability. Thus, nucleic acids comprising an increased amount of guanine (G) and/or cytosine (C) residues may be functionally more stable than nucleic acids containing a large amount of adenine (A) and thymine (T) or uracil (U) nucleotides. In this context, WO02/098443 provides a pharmaceutical composition containing an mRNA that is stabilised by sequence modifications in the coding region. Such a sequence modification takes advantage of the degeneracy of the genetic code. Accordingly, codons which contain a less favourable combination of nucleotides (less favourable in terms of RNA stability) may be substituted by alternative codons without altering the encoded amino acid sequence. This method of RNA stabilization is limited by the provisions of the specific nucleotide sequence of each single RNA molecule which is not allowed to leave the space of the desired amino acid sequence. Also, that approach is restricted to coding regions of the RNA.

As an alternative option for mRNA stabilisation, it has been found that naturally occurring eukaryotic mRNA molecules contain characteristic stabilising elements. For example, they may comprise so-called untranslated regions (UTR) at their 5'-end (5'-UTR) and/or at their 3'-end (3'-UTR) as well as other structural features, such as a 5'-cap structure or a 3'-poly(A) tail. Both, 5'-UTR and 3'-UTR are typically transcribed from the genomic DNA and are, thus, an element of the premature mRNA. Characteristic structural features of mature mRNA, such as the 5'-cap and the 3'-poly(A) tail (also called poly(A) tail or poly(A) sequence) are usually added to the transcribed (premature) mRNA during mRNA processing.

A 3'-poly(A) tail is typically a monotonous sequence stretch of adenosine nucleotides added to the 3'-end of the transcribed mRNA. It may comprise up to about 400 adenosine nucleotides. It was found that the length of such a 3'-poly(A) tail is a potentially critical element for the stability of the individual mRNA.

Also, it was shown that the 3'-UTR of $\alpha$-globin mRNA may be an important factor for the well-known stability of $\alpha$-globin mRNA (Rodgers et al., Regulated $\alpha$-globin mRNA decay is a cytoplasmic event proceeding through 3'-to-5' exosome-dependent decapping, RNA, 8, pp. 1526-1537, 2002). The 3'-UTR of $\alpha$-globin mRNA is apparently involved in the formation of a specific ribonucleoprotein-complex, the $\alpha$-complex, whose presence correlates with mRNA stability *in vitro* (Wang et al., An mRNA stability complex functions with poly(A)-binding protein to stabilize mRNA in vitro, Molecular and Cellular biology, Vol 19, No. 7, July 1999, p. 4552-4560).

An interesting regulatory function has further been demonstrated for the UTRs in ribosomal protein mRNAs: while the 5'-UTR of ribosomal protein mRNAs controls the growth-associated translation of the mRNA, the stringency of that regulation is conferred by the respective 3'-UTR in ribosomal protein mRNAs (Ledda et al., Effect of the 3'-UTR length on the translational regulation of 5'-terminal oligopyrimidine mRNAs, Gene, Vol. 344, 2005, p. 213-220). This mechanism contributes to the specific expression pattern of ribosomal proteins, which are typically transcribed in a constant manner so that some ribosomal protein mRNAs such as ribosomal protein S9 or ribosomal protein L32 are referred to as housekeeping genes (Janovick-Guretzky et al., Housekeeping Gene Expression in Bovine Liver is Affected by Physiological State, Feed Intake, and Dietary Treatment, J. Dairy Sci., Vol. 90, 2007, p. 2246-2252). The growth-associated expression pattern of ribosomal proteins is thus mainly due to regulation on the level of translation.

Irrespective of factors influencing mRNA stability, effective translation of the administered nucleic acid molecules by the target cells or tissue is crucial for any approach using nucleic acid molecules for gene therapy or genetic vaccination.

As can be seen from the examples cited above, along with the regulation of stability, also translation of the majority of mRNAs is regulated by structural features like UTRs, 5'-cap and 3'-poly(A) tail. In this context, it has been reported that the length of the poly(A) tail may play an important role for translational efficiency as well. Stabilizing 3'-elements, however, may also have an attenuating effect on translation.

It is the object of the invention to provide nucleic acid molecules which may be suitable for application in gene therapy and/or genetic vaccination. Particularly, it is the object of the invention to provide an mRNA species which is stabilized against preterm degradation or decay without exhibiting significant functional loss in translational efficiency. It is also an object of the invention to provide an artificial nucleic acid molecule, preferably an mRNA, which is characterized by enhanced expression of the respective protein encoded by said nucleic acid molecule. One particular object of the invention is the provision of an mRNA, wherein the efficiency of translation of the respective encoded protein is enhanced. Another object of the present invention is to provide nucleic acid molecules coding for such a superior mRNA species which may be amenable for use in gene therapy and/or genetic vaccination. It is a further object of the present invention to provide a pharmaceutical composition for use in gene therapy and/or genetic vaccination. In summary, it is the object of the present invention to provide improved nucleic acid species which overcome the above discussed disadvantages of the prior art by a cost-effective and straight-forward approach.

The object underlying the present invention is solved by the claimed subject matter.

For the sake of clarity and readability the following definitions are provided. Any technical feature mentioned for these definitions may be read on each and every embodiment of the invention. Additional definitions and explanations may be specifically provided in the context of these embodiments.

Adaptive immune response: The adaptive immune response is typically understood to be an antigen-specific response of the immune system. Antigen specificity allows for the generation of responses that are tailored to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naive T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are, typically, responsible for presentation of an antigen to T-cells. Therein, presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Adaptive immune system: The adaptive immune system is essentially dedicated to eliminate or prevent pathogenic growth. It typically regulates the adaptive immune response by providing the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of such a cell will then inherit genes encoding the same receptor specificity, including the Memory B cells and Memory T cells that are the keys to long-lived specific immunity.

Adjuvant/adjuvant component: An adjuvant or an adjuvant component in the broadest sense is typically a pharmacological and/or immunological agent that may modify, e.g. enhance, the effect of other agents, such as a drug or vaccine. It is to be interpreted in a broad sense and refers to a broad spectrum of substances. Typically, these substances are able to increase the immunogenicity of antigens. For example, adjuvants may be recognized by the innate immune systems and, e.g., may elicit an innate immune response. "Adjuvants" typically do not elicit an adaptive immune response. Insofar, "adjuvants" do not qualify as antigens. Their mode of action is distinct from the effects triggered by antigens resulting in an adaptive immune response.

Antigen: In the context of the present invention "antigen" refers typically to a substance which may be recognized by the immune system, preferably by the adaptive immune system, and is capable of triggering an antigen-specific

immune response, e.g. by formation of antibodies and/or antigen-specific T cells as part of an adaptive immune response. Typically, an antigen may be or may comprise a peptide or protein which may be presented by the MHC to T-cells. In the sense of the present invention an antigen may be the product of translation of a provided nucleic acid molecule, preferably an mRNA as defined herein. In this context, also fragments, variants and derivatives of peptides and proteins comprising at least one epitope are understood as antigens. In the context of the present invention, tumour antigens and pathogenic antigens as defined herein are particularly preferred.

Artificial nucleic acid molecule: An artificial nucleic acid molecule may typically be understood to be a nucleic acid molecule, e.g. a DNA or an RNA, that does not occur naturally. In other words, an artificial nucleic acid molecule may be understood as a non-natural nucleic acid molecule. Such nucleic acid molecule may be non-natural due to its individual sequence (which does not occur naturally) and/or due to other modifications, e.g. structural modifications of nucleotides which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term "wild type" may be understood as a sequence occurring in nature. Further, the term "artificial nucleic acid molecule" is not restricted to mean "one single molecule" but is, typically, understood to comprise an ensemble of identical molecules. Accordingly, it may relate to a plurality of identical molecules contained in an aliquot. Bicistronic RNA, multicistronic RNA: A bicistronic or multicistronic RNA is typically an RNA, preferably an mRNA, that typically may have two (bicistronic) or more (multicistronic) open reading frames (ORF). An open reading frame in this context is a sequence of codons that is translatable into a peptide or protein.

Carrier / polymeric carrier: A carrier in the context of the invention may typically be a compound that facilitates transport and/or complexation of another compound (cargo). A polymeric carrier is typically a carrier that is formed of a polymer. A carrier may be associated to its cargo by covalent or non-covalent interaction. A carrier may transport nucleic acids, e.g. RNA or DNA, to the target cells. The carrier may -for some embodiments - be a cationic component.

Cationic component: The term "cationic component" typically refers to a charged molecule, which is positively charged (cation) at a pH value typically from 1 to 9, preferably at a pH value of or below 9 (e.g. from 5 to 9), of or below 8 (e.g. from 5 to 8), of or below 7 (e.g. from 5 to 7), most preferably at a physiological pH, e.g. from 7.3 to 7.4. Accordingly, a cationic component may be any positively charged compound or polymer, preferably a cationic peptide or protein which is positively charged under physiological conditions, particularly under physiological conditions *in vivo.* A "cationic peptide or protein" may contain at least one positively charged amino acid, or more than one positively charged amino acid, e.g. selected from Arg, His, Lys or Orn. Accordingly, "polycationic" components are also within the scope exhibiting more than one positive charge under the conditions given.

5'-cap: A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety.

Cellular immunity/cellular immune response: Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

The term "derived from" as used throughout the present specification in the context of a nucleic acid, i.e. for a nucleic acid "derived from" (another) nucleic acid, means that the nucleic acid, which is derived from (another) nucleic acid, shares at least 50%, preferably at least 60%, preferably at least 70%, more preferably at least 75%, more preferably at least 80%, more preferably at least 85%, even more preferably at least 90%, even more preferably at least 95%, and particularly preferably at least 98% sequence identity with the nucleic acid from which it is derived. The skilled person is aware that sequence identity is typically calculated for the same types of nucleic acids, i.e. for DNA sequences or for

RNA sequences. Thus, it is understood, if a DNA is "derived from" an RNA or if an RNA is "derived from" a DNA, in a first step the RNA sequence is converted into the corresponding DNA sequence (in particular by replacing the uracils (U) by thymidines (T) throughout the sequence) or, vice versa, the DNA sequence is converted into the corresponding RNA sequence (in particular by replacing the thymidines (T) by uracils (U) throughout the sequence). Thereafter, the sequence identity of the DNA sequences or the sequence identity of the RNA sequences is determined. Preferably, a nucleic acid "derived from" a nucleic acid also refers to nucleic acid, which is modified in comparison to the nucleic acid from which it is derived, e.g. in order to increase RNA stability even further and/or to prolong and/or increase protein production. It goes without saying that such modifications are preferred, which do not impair RNA stability, e.g. in comparison to the nucleic acid from which it is derived.

DNA: DNA is the usual abbreviation for deoxy-ribonucleic acid. It is a nucleic acid molecule, i.e. a polymer consisting of nucleotides. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerise by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA sequence. DNA may be single stranded or double stranded. In the double stranded form, the nucleotides of the first strand typically hybridize with the nucleotides of the second strand, e.g. by A/T-base-pairing and G/C-base-pairing.

Epitope: (also called "antigen determinant") can be distinguished in T cell epitopes and B cell epitopes. T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Fragment of a sequence: A fragment of a sequence may typically be a shorter portion of a full-length sequence of e.g. a nucleic acid molecule or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 5%, 10%, 20%, preferably at least 30%, more preferably at least 40%, more preferably at least 50%, even more preferably at least 60%, even more preferably at least 70%, and most preferably at least 80% of the total (i.e. full-length) molecule from which the fragment is derived.

G/C modified: A G/C-modified nucleic acid may typically be a nucleic acid, preferably an artificial nucleic acid molecule as defined herein, based on a modified wild-type sequence comprising a preferably increased number of guanosine and/or cytosine nucleotides as compared to the wild-type sequence. Such an increased number may be generated by substitution of codons containing adenosine or thymidine nucleotides by codons containing guanosine or cytosine nucleotides. If the enriched G/C content occurs in a coding region of DNA or RNA, it makes use of the degeneracy of the genetic code. Accordingly, the codon substitutions preferably do not alter the encoded amino acid residues, but exclusively increase the G/C content of the nucleic acid molecule.

Gene therapy: Gene therapy may typically be understood to mean a treatment of a patient's body or isolated elements of a patient's body, for example isolated tissues/cells, by nucleic acids encoding a peptide or protein. It typically may comprise at least one of the steps of a) administration of a nucleic acid, preferably an artificial nucleic acid molecule as defined herein, directly to the patient - by whatever administration route - or *in vitro* to isolated cells/tissues of the patient, which results in transfection of the patient's cells either *in vivo*/*ex vivo or in vitro;* b) transcription and/or translation of the introduced nucleic acid molecule; and optionally c) re-administration of isolated, transfected cells to the patient, if the nucleic acid has not been administered directly to the patient.

Genetic vaccination: Genetic vaccination may typically be understood to be vaccination by administration of a nucleic acid molecule encoding an antigen or an immunogen or fragments thereof. The nucleic acid molecule may be

administered to a subject's body or to isolated cells of a subject. Upon transfection of certain cells of the body or upon transfection of the isolated cells, the antigen or immunogen may be expressed by those cells and subsequently presented to the immune system, eliciting an adaptive, i.e. antigen-specific immune response. Accordingly, genetic vaccination typically comprises at least one of the steps of a) administration of a nucleic acid, preferably an artificial nucleic acid molecule as defined herein, to a subject, preferably a patient, or to isolated cells of a subject, preferably a patient, which usually results in transfection of the subject's cells either *in vivo* or *in vitro;* b) transcription and/or translation of the introduced nucleic acid molecule; and optionally c) re-administration of isolated, transfected cells to the subject, preferably the patient, if the nucleic acid has not been administered directly to the patient.

Heterologous sequence: Two sequences are typically understood to be 'heterologous' if they are not derivable from the same gene. I.e., although heterologous sequences may be derivable from the same organism, they naturally (in nature) do not occur in the same nucleic acid molecule, such as in the same mRNA.

Humoral immunity/humoral immune response: Humoral immunity refers typically to antibody production and optionally to accessory processes accompanying antibody production. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Immunogen: In the context of the present invention an immunogen may be typically understood to be a compound that is able to stimulate an immune response. Preferably, an immunogen is a peptide, polypeptide, or protein. In a particularly preferred embodiment, an immunogen in the sense of the present invention is the product of translation of a provided nucleic acid molecule, preferably an artificial nucleic acid molecule as defined herein. Typically, an immunogen elicits at least an adaptive immune response.

Immunostimulatory composition: In the context of the invention, an immunostimulatory composition may be typically understood to be a composition containing at least one component which is able to induce an immune response or from which a component which is able to induce an immune response is derivable. Such immune response may be preferably an innate immune response or a combination of an adaptive and an innate immune response. Preferably, an immunostimulatory composition in the context of the invention contains at least one artificial nucleic acid molecule, more preferably an RNA, for example an mRNA molecule. The immunostimulatory component, such as the mRNA may be complexed with a suitable carrier. Thus, the immunostimulatory composition may comprise an mRNA/carrier-complex. Furthermore, the immunostimulatory composition may comprise an adjuvant and/or a suitable vehicle for the immunostimulatory component, such as the mRNA.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

Immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Immunostimulatory RNA: An immunostimulatory RNA (isRNA) in the context of the invention may typically be an RNA that is able to induce an innate immune response. It usually does not have an open reading frame and thus does not provide a peptide-antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

Innate immune system: The innate immune system, also known as non-specific (or unspecific) immune system, typically comprises the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be, e.g., activated by ligands of Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like

receptor, an immunostimulatory nucleic acid, an immunostimulatory RNA (isRNA), a CpG-DNA, an antibacterial agent, or an anti-viral agent. The pharmaceutical composition according to the present invention may comprise one or more such substances. Typically, a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system; and/or acting as a physical and chemical barrier to infectious agents.

Cloning site: A cloning site is typically understood to be a segment of a nucleic acid molecule, which is suitable for insertion of a nucleic acid sequence, e.g., a nucleic acid sequence comprising an open reading frame. Insertion may be performed by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Nucleic acid molecule: A nucleic acid molecule is a molecule comprising, preferably consisting of nucleic acid components. The term nucleic acid molecule preferably refers to DNA or RNA molecules. It is preferably used synonymous with the term "polynucleotide". Preferably, a nucleic acid molecule is a polymer comprising or consisting of nucleotide monomers which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

Open reading frame: An open reading frame (ORF) in the context of the invention may typically be a sequence of several nucleotide triplets which may be translated into a peptide or protein. An open reading frame preferably contains a start codon, i.e. a combination of three subsequent nucleotides coding usually for the amino acid methionine (ATG), at its 5'-end and a subsequent region which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "protein coding region".

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Pharmaceutically effective amount: A pharmaceutically effective amount in the context of the invention is typically understood to be an amount that is sufficient to induce a pharmaceutical effect, such as an immune response, altering a pathological level of an expressed peptide or protein, or substituting a lacking gene product, e.g., in case of a pathological situation. Protein A protein typically comprises one or more peptides or polypeptides. A protein is typically folded into 3-dimensional form, which may be required for to protein to exert its biological function.

Poly(A) sequence: A poly(A) sequence, also called poly(A) tail or 3'-poly(A) tail, is typically understood to be a sequence of adenosine nucleotides, e.g., of up to about 400 adenosine nucleotides, e.g. from about 20 to about 400, preferably from about 50 to about 400, more preferably from about 50 to about 300, even more preferably from about 50 to about 250, most preferably from about 60 to about 250 adenosine nucleotides. A poly(A) sequence is typically located at the 3'end of an mRNA. In the context of the present invention, a poly(A) sequence may be located within an mRNA or any other nucleic acid molecule, such as, e.g., in a vector, for example, in a vector serving as template for the generation of an RNA, preferably an mRNA, e.g., by transcription of the vector.

Polyadenylation: Polyadenylation is typically understood to be the addition of a poly(A) sequence to a nucleic acid molecule, such as an RNA molecule, e.g. to a premature mRNA. Polyadenylation may be induced by a so called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

Restriction site: A restriction site, also termed restriction enzyme recognition site, is a nucleotide sequence recognized by a restriction enzyme. A restriction site is typically a short, preferably palindromic nucleotide sequence, e.g. a sequence comprising 4 to 8 nucleotides. A restriction site is preferably specifically recognized by a restriction enzyme. The restriction enzyme typically cleaves a nucleotide sequence comprising a restriction site at this site. In a double-stranded nucleotide sequence, such as a double-stranded DNA sequence, the restriction enzyme typically cuts

both strands of the nucleotide sequence.

RNA, mRNA: RNA is the usual abbreviation for ribonucleic-acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. Typically, transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino-acid sequence of a particular peptide or protein. Typically, a mature mRNA comprises a 5'-cap, a 5'-UTR, an open reading frame, a 3'-UTR and a poly(A) sequence. Aside from messenger RNA, several non-coding types of RNA exist which may be involved in regulation of transcription and/or translation.

Sequence of a nucleic acid molecule: The sequence of a nucleic acid molecule is typically understood to be the particular and individual order, i.e. the succession of its nucleotides. The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an alignment.

Stabilized nucleic acid molecule: A stabilized nucleic acid molecule is a nucleic acid molecule, preferably a DNA or RNA molecule that is modified such, that it is more stable to disintegration or degradation, e.g., by environmental factors or enzymatic digest, such as by an exo- or endonuclease degradation, than the nucleic acid molecule without the modification. Preferably, a stabilized nucleic acid molecule in the context of the present invention is stabilized in a cell, such as a prokaryotic or eukaryotic cell, preferably in a mammalian cell, such as a human cell. The stabilization effect may also be exerted outside of cells, e.g. in a buffer solution etc., for example, in a manufacturing process for a pharmaceutical composition comprising the stabilized nucleic acid molecule.

Transfection: The term "transfection" refers to the introduction of nucleic acid molecules, such as DNA or RNA (e.g. mRNA) molecules, into cells, preferably into eukaryotic cells. In the context of the present invention, the term "transfection" encompasses any method known to the skilled person for introducing nucleic acid molecules into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. Preferably, the introduction is non-viral.

Vaccine: A vaccine is typically understood to be a prophylactic or therapeutic material providing at least one antigen, preferably an immunogen. The antigen or immunogen may be derived from any material that is suitable for vaccination. For example, the antigen or immunogen may be derived from a pathogen, such as from bacteria or virus particles etc., or from a tumor or cancerous tissue. The antigen or immunogen stimulates the body's adaptive immune system to provide an adaptive immune response.

Vector: The term "vector" refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector which allows the convenient storage of a nucleic acid molecule, for example, of an mRNA molecule. Thus, the vector may comprise a sequence corresponding, e.g., to a desired mRNA sequence or a part thereof, such as a sequence corresponding to the open reading frame and the 3'-UTR and/or the 5'-UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA polymerase promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector which is suitable for transferring nucleic acid molecules into cells or organisms,

for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

Vehicle: A vehicle is typically understood to be a material that is suitable for storing, transporting, and/or administering a compound, such as a pharmaceutically active compound. For example, it may be a physiologically acceptable liquid which is suitable for storing, transporting, and/or administering a pharmaceutically active compound.

3'-untransiated region (3'-UTR): Generally, the term "3'-UTR" refers to a part of the artificial nucleic acid molecule, which is located 3' (i.e. "downstream") of an open reading frame and which is not translated into protein. Typically, a 3'-UTR is the part of an mRNA which is located between the protein coding region (open reading frame (ORF) or coding sequence (CDS)) and the poly(A) sequence of the mRNA. In the context of the invention, the term 3'-UTR may also comprise elements, which are not encoded in the template, from which an RNA is transcribed, but which are added after transcription during maturation, e.g. a poly(A) sequence. A 3'-UTR of the mRNA is not translated into an amino acid sequence. The 3'-UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excize optional introns and modifications of the 3'-end, such as polyadenylation of the 3'-end of the pre-mature mRNA and optional endo-/ or exonuclease cleavages etc.. In the context of the present invention, a 3'-UTR corresponds to the sequence of a mature mRNA which is located between the the stop codon of the protein coding region, preferably immediately 3' to the stop codon of the protein coding region, and the poly(A) sequence of the mRNA. The term "corresponds to" means that the 3'-UTR sequence may be an RNA sequence, such as in the mRNA sequence used for defining the 3'-UTR sequence, or a DNA sequence which corresponds to such RNA sequence. In the context of the present invention, the term "a 3'-UTR of a gene", is the sequence which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence (both sense and antisense strand and both mature and immature) of the 3'-UTR. Preferably, the 3'UTRs have a length of more than 20, 30, 40 or 50 nucleotides.

5'-untranslated region (5'-UTR): Generally, the term "5'-UTR" refers to a part of the artificial nucleic acid molecule, which is located 5' (i.e. "upstream") of an open reading frame and which is not translated into protein. A 5'-UTR is typically understood to be a particular section of messenger RNA (mRNA), which is located 5' of the open reading frame of the mRNA. Typically, the 5'-UTR starts with the transcriptional start site and ends one nucleotide before the start codon of the open reading frame. Preferably, the 5'UTRs have a length of more than 20, 30, 40 or 50 nucleotides. The 5'-UTR may comprise elements for controlling gene expression, also called regulatory elements. Such regulatory elements may be, for example, ribosomal binding sites. The 5'-UTR may be posttranscriptionally modified, for example by addition of a 5'-CAP. A 5'-UTR of the mRNA is not translated into an amino acid sequence. The 5'-UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excize optional introns and modifications of the 3'-end, such as polyadenylation of the 3'-end of the pre-mature mRNA and optional endo-/ or exonuclease cleavages etc.. In the context of the present invention, a 5'-UTR corresponds to the sequence of a mature mRNA which is located between the start codon and, for example, the 5'-CAP. Preferably, the 5'-UTR corresponds to the sequence which extends from a nucleotide located 3' to the 5'-CAP, more preferably from the nucleotide located immediately 3' to the 5'-CAP, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence which corresponds to such RNA sequence. In the context of the present invention, the term "a 5'-UTR of a gene" is the sequence which corresponds to the 5'-UTR of the mature mRNA derived from this gene, i.e. the mRNA obtained by transcription of the gene and maturation of the pre-mature mRNA. The term "5'-UTR of a gene" encompasses the DNA sequence and the RNA sequence (both sense and antisense strand and both mature and immature) of the 5'-UTR.

5'Terminal Oligopyrimidine Tract (TOP): The 5'terminal oligopyrimidine tract (TOP) is typically a stretch of pyrimidine nucleotides located in the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. Messenger

RNA that contains a 5'terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purin residue in said gene or mRNA. A TOP motif in the sense of the present invention is preferably located at the 5'end of a sequence which represents a 5'-UTR or at the 5'end of a sequence which codes for a 5'-UTR. Thus, preferably, a stretch of 3 or more pyrimidine nucleotides is called "TOP motif" in the sense of the present invention if this stretch is located at the 5'end of a respective sequence, such as the artificial nucleic acid molecule, the 5'-UTR element of the artificial nucleic acid molecule, or the nucleic acid sequence which is derived from the 5'-UTR of a TOP gene as described herein. In other words, a stretch of 3 or more pyrimidine nucleotides, which is not located at the 5'-end of a 5'-UTR or a 5'-UTR element but anywhere within a 5'-UTR or a 5'-UTR element, is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes with a tissue specific translational regulation are known. As defined above, the 5'-UTR of a TOP gene corresponds to the sequence of a 5'-UTR of a mature mRNA derived from a TOP gene, which preferably extends from the nucleotide located 3' to the 5'-CAP to the nucleotide located 5' to the start codon. A 5'-UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'-UTRs of TOP genes are generally rather short. The lengths of 5'-UTRs of TOP genes may vary between 20 nucleotides up to 500 nucleotides, and are typically less than about 200 nucleotides, preferably less than about 150 nucleotides, more preferably less than about 100 nucleotides. Exemplary 5'-UTRs of TOP genes in the sense of the present invention are the nucleic acid sequences extending from the nucleotide at position 5 to the nucleotide located immediately 5' to the start codon (e.g. the ATG) in the sequences according to SEQ ID Nos. 1-1363 of the patent application WO2013/143700, whose disclosure is incorporated herewith by reference. In this context a particularly preferred fragment of a 5'-UTR of a TOP gene is a 5'-UTR of a TOP gene lacking the 5'TOP motif. The terms "5'-UTR of a TOP gene" or "5'-TOP UTR" preferably refer to the 5'-UTR of a naturally occurring TOP gene.

In a first aspect, the present invention relates to an artificial nucleic acid molecule comprising

a. at least one open reading frame (ORF); and

b. at least one 3'-untranslated region element (3'-UTR element) and/or at least one 5'-untranslated region element (5'-UTR element), wherein the at least one 3'-UTR element and/or the at least one 5'-UTR element prolongs and/or increases protein production from said artificial nucleic acid molecule and wherein the at least one 3'-UTR element and/or the at least one 5'-UTR element is derived from a stable mRNA.

Preferably, the artificial nucleic acid molecule according to the present invention does not comprise a 3'-UTR (element) and/or a 5'-UTR (element) of ribosomal protein S6, of RPL36AL, of rps16 or of ribosomal protein L9. More preferably, the artificial nucleic acid molecule according to the present invention does not comprise a 3'-UTR (element) and/or a 5'-UTR (element) of ribosomal protein S6, of RPL36AL, of rps16 or of ribosomal protein L9 and the open reading frame of the artificial nucleic acid molecule according to the present invention does not code for a GFP protein. Even more preferably, the artificial nucleic acid molecule according to the present invention does not comprise a 3'-UTR (element) and/or a 5'-UTR (element) of ribosomal protein S6, of RPL36AL, of rps16 or of ribosomal protein L9 and the open reading frame of the artificial nucleic acid molecule according to the present invention does not code for a reporter protein, e.g., selected from the group consisting of globin proteins (particularly beta-globin), luciferase protein, GFP proteins, glucurinodase proteins (particularly beta- glucurinodase) or variants thereof, for example, variants exhibiting at least 70% sequence identity to a globin protein, a luciferase protein, a GFP protein, or a glucurinodase protein.

The term "3'-UTR element" refers to a nucleic acid sequence which comprises or consists of a nucleic acid sequence that is derived from a 3'-UTR or from a variant or a fragment of a 3'-UTR. A "3'-UTR element" preferably refers to a nucleic acid sequence which is comprised by a 3'-UTR of an artificial nucleic acid sequence, such as an artificial mRNA. Accordingly, in the sense of the present invention, preferably, a 3'-UTR element may be comprised by the 3'-UTR of an mRNA, preferably of an artificial mRNA, or a 3'-UTR element may be comprised by the 3'-UTR of the respective transcription template. Preferably, a 3'-UTR element is a nucleic acid sequence which corresponds to the 3'-UTR of an

mRNA, preferably to the 3'-UTR of an artificial mRNA, such as an mRNA obtained by transcription of a genetically engineered vector construct. Preferably, a 3'-UTR element in the sense of the present invention functions as a 3'-UTR or codes for a nucleotide sequence that fulfils the function of a 3'-UTR.

Accordingly, the term "5'-UTR element" refers to a nucleic acid sequence which comprises or consists of a nucleic acid sequence that is derived from a 5'-UTR or from a variant or a fragment of a 5'-UTR. A "5'-UTR element" preferably refers to a nucleic acid sequence which is comprised by a 5'-UTR of an artificial nucleic acid sequence, such as an artificial mRNA. Accordingly, in the sense of the present invention, preferably, a 5'-UTR element may be comprised by the 5'-UTR of an mRNA, preferably of an artificial mRNA, or a 5'-UTR element may be comprised by the 5'-UTR of the respective transcription template. Preferably, a 5'-UTR element is a nucleic acid sequence which corresponds to the 5'-UTR of an mRNA, preferably to the 5'-UTR of an artificial mRNA, such as an mRNA obtained by transcription of a genetically engineered vector construct. Preferably, a 5'-UTR element in the sense of the present invention functions as a 5'-UTR or codes for a nucleotide sequence that fulfils the function of a 5'-UTR.

The 3'-UTR element and/or the 5'-UTR element in the artificial nucleic acid molecule according to the present invention prolongs and/or increases protein production from said artificial nucleic acid molecule. Thus, the artificial nucleic acid molecule according to the present invention may in particular comprise:

- a 3'-UTR element which increases protein production from said artificial nucleic acid molecule,
- a 3'-UTR element which prolongs protein production from said artificial nucleic acid molecule,
- a 3'-UTR element which increases and prolongs protein production from said artificial nucleic acid molecule,
- a 5'-UTR element which increases protein production from said artificial nucleic acid molecule,
- a 5'-UTR element which prolongs protein production from said artificial nucleic acid molecule,
- a 5'-UTR element which increases and prolongs protein production from said artificial nucleic acid molecule,
- a 3'-UTR element which increases protein production from said artificial nucleic acid molecule and a 5'-UTR element which increases protein production from said artificial nucleic acid molecule,
- a 3'-UTR element which increases protein production from said artificial nucleic acid molecule and a 5'-UTR element which prolongs protein production from said artificial nucleic acid molecule,
- a 3'-UTR element which increases protein production from said artificial nucleic acid molecule and a 5'-UTR element which increases and prolongs protein production from said artificial nucleic acid molecule,
- a 3'-UTR element which prolongs protein production from said artificial nucleic acid molecule and a 5'-UTR element which increases protein production from said artificial nucleic acid molecule,
- a 3'-UTR element which prolongs protein production from said artificial nucleic acid molecule and a 5'-UTR element which prolongs protein production from said artificial nucleic acid molecule,
- a 3'-UTR element which prolongs protein production from said artificial nucleic acid molecule and a 5'-UTR element which increases and prolongs protein production from said artificial nucleic acid molecule,
- a 3'-UTR element which increases and prolongs protein production from said artificial nucleic acid molecule and a 5'-UTR element which increases protein production from said artificial nucleic acid molecule,
- a 3'-UTR element which increases and prolongs protein production from said artificial nucleic acid molecule and a 5'-UTR element which prolongs protein production from said artificial nucleic acid molecule, or
- a 3'-UTR element which increases and prolongs protein production from said artificial nucleic acid molecule and a 5'-UTR element which increases and prolongs protein production from said artificial nucleic acid molecule.

Preferably, the artificial nucleic acid molecule according to the present invention comprises a 3'-UTR element which prolongs protein production from said artificial nucleic acid molecule and/or a 5'-UTR element which increases protein production from said artificial nucleic acid molecule.

Preferably, the artificial nucleic acid molecule according to the present invention comprises at least one 3'-UTR element and at least one 5'-UTR element, i.e. at least one 3'-UTR element which prolongs and/or increases protein production from said artificial nucleic acid molecule and which is derived from a stable mRNA and at least one 5'-UTR element which prolongs and/or increases protein production from said artificial nucleic acid molecule and which is derived from a stable mRNA.

"Prolonging and/or increasing protein production from said artificial nucleic acid molecule" in general refers to the amount of protein produced from the artificial nucleic acid molecule according to the present invention with the respective 3'-UTR element and/or the 5'-UTR element in comparison to the amount of protein produced from a respective reference nucleic acid lacking a 3'-UTR and/or a 5'-UTR or comprising a reference 3'-UTR and/or a reference 5'-UTR, such as a 3'-UTR and/or a 5'-UTR naturally occurring in combination with the ORF.

In particular, the at least one 3'-UTR element and/or the 5'-UTR element of the artificial nucleic acid molecule according to the present invention prolongs protein production from the artificial nucleic acid molecule according to the present invention, e.g. from an mRNA according to the present invention, compared to a respective nucleic acid lacking a 3'-UTR and/or 5'-UTR or comprising a reference 3'-UTR and/or 5'-UTR, such as a 3'- and/or 5'-UTR naturally occurring

in combination with the ORF.

In particular, the at least one 3'-UTR element and/or 5'-UTR element of the artificial nucleic acid molecule according to the present invention increases protein production, in particular the protein expression and/or total protein production, from the artificial nucleic acid molecule according to the present invention, e.g. from an mRNA according to the present invention, compared to a respective nucleic acid lacking a 3'- and/or 5'-UTR or comprising a reference 3'- and/or 5'-UTR, such as a 3'- and/or 5'-UTR naturally occurring in combination with the ORF.

Preferably, the at least one 3'-UTR element and/or the at least one 5'-UTR element of the artificial nucleic acid molecule according to the present invention do not negatively influence translational efficiency of a nucleic acid compared to the translational efficiency of a respective nucleic acid lacking a 3'-UTR and/or a 5'-UTR or comprising a reference 3'-UTR and/or a reference 5'-UTR, such as a 3'-UTR and/or a 5'-UTR naturally occurring in combination with the ORF. Even more preferably, the translation efficiency is enhanced by the 3'-UTR and/or a 5'-UTR in comparison to the translation efficiency of the protein encoded by the respective ORF in its natural context.

The term "respective nucleic acid molecule" or "reference nucleic acid molecule" as used herein means that - apart from the different 3'-UTRs and/or 5'-UTRs - the reference nucleic acid molecule is comparable, preferably identical, to the inventive artificial nucleic acid molecule comprising the 3'-UTR element and/or the 5'-UTR element.

In order to assess the protein production in vivo or in vitro as defined herein (i.e. in vitro referring to ("living") cells and/or tissue, including tissue of a living subject; cells include in particular cell lines, primary cells, cells in tissue or subjects, preferred are mammalian cells, e.g. human cells and mouse cells and particularly preferred are the human cell lines HeLa, and U-937 and the mouse cell lines NIH3T3, JAWSII and L929, furthermore primary cells are particularly preferred, in particular preferred embodiments human dermal fibroblasts (HDF)) by the inventive artificial nucleic acid molecule, the expression of the encoded protein is determined following injection/transfection of the inventive artificial nucleic acid molecule into target cells/tissue and compared to the protein expression induced by the reference nucleic acid. Quantitative methods for determining protein expression are known in the art (e.g. Western-Blot, FACS, ELISA, mass spectrometry). Particularly useful in this context is the determination of the expression of reporter proteins like luciferase, Green fluorescent protein (GFP), or secreted alkaline phosphatase (SEAP). Thus, an artificial nucleic acid according to the invention or a reference nucleic acid is introduced into the target tissue or cell, e.g. via transfection or injection, preferably in a mammalian expression system, such as in mammalian cells, e.g. in HeLa or HDF cells. Several hours or several days (e.g. 6, 12, 24, 48 or 72 hours) post initiation of expression or post introduction of the nucleic acid molecule, a target cell sample is collected and measured via FACS and/or lysed. Afterwards the lysates can be used to detect the expressed protein (and thus determine the efficiency of protein expression) using several methods, e.g. Western-Blot, FACS, ELISA, mass spectrometry or by fluorescence or luminescence measurement.

Therefore, if the protein expression from an artificial nucleic acid molecule according to the invention is compared to the protein expression from a reference nucleic acid molecule at a specific point in time (e.g. 6, 12, 24, 48 or 72 hours post initiation of expression or post introduction of the nucleic acid molecule), both nucleic acid molecules are introduced separately into target tissue/cells, a sample from the tissue/cells is collected after a specific point in time, protein lysates are prepared according to the particular protocol adjusted to the particular detection method (e.g. Western Blot, ELISA, fluorescence or luminescence measurement, etc. as known in the art) and the protein is detected by the chosen detection method. As an alternative to the measurement of expressed protein amounts in cell lysates - or, in addition to the measurement of protein amounts in cell lysates prior to lysis of the collected cells or using an aliquot in parallel - protein amounts may also be determined by using FACS analysis.

The term "prolonging protein production" from an artificial nucleic acid molecule such as an artificial mRNA preferably means that the protein production from the artificial nucleic acid molecule such as the artificial mRNA is prolonged compared to the protein production from a reference nucleic acid molecule such as a reference mRNA, e.g. comprising a reference 3'- and/or 5'-UTR or lacking a 3'- and/or 5'-UTR, preferably in a mammalian expression system, such as in HeLa or HDF cells. Thus, protein produced from the artificial nucleic acid molecule such as the artificial mRNA is observable for a longer period of time than what may be seen for a protein produced from a reference nucleic acid molecule. In other words, the amount of protein produced from the artificial nucleic acid molecule such as the artificial mRNA measured at a later point in time, e.g. 48 hours or 72 hours after transfection, is larger than the amount of protein produced from a reference nucleic acid molecule such as a reference mRNA at a corresponding later point in time. Such a "later point in time" may be, for example, any time beyond 24 hours post initiation of expression, such as post transfection of the nucleic acid molecule, e.g. 36, 48, 60, 72, 96 hours post initiation of expression, i.e. after transfection. Moreover, for the same nucleic acid, the amount of protein produced at a later point in time may be normalized to the amount produced an earlier (reference) point in time, for example the amount of protein at a later point in time may be expressed as percentage of the amount of protein at 24 h after transfection.

Preferably, this effect of prolonging protein production is determined by (i) measuring protein amounts, e.g. obtained by expression of an encoded reporter protein such as luciferase, preferably in a mammalian expression system such as in HeLa or HDF cells, over time, (ii) determining the amount of protein observed at a "reference" point in time $t_1$, for example $t_1$ = 24h after transfection, and setting this protein amount to 100%, (iii) determining the amount of protein

observed at one or more later points in time $t_2$, $t_3$, etc., for example $t_2$ = 48h and $t_3$ = 72h after transfection, and calculating the relative amount of protein observed at a later point in time as a percentage of the protein amount at a point in time $t_1$. For example, a protein which is expressed at $t_1$ in an amount of "80", at $t_2$ in an amount of "20", and at $t_3$ in an amount of "10", the relative amount of protein at $t_2$ would be 25%, and at $t_3$ 12,5%. These relative amounts at a later point in time may then be compared in a step (iv) to relative protein amounts for the corresponding points in time for a nucleic acid molecule lacking a 3'- and/or 5'-UTR, respectively, or comprising a reference 3'- and/or 5'-UTR, respectively. By comparing the relative protein amount produced from the artificial nucleic acid molecule according to the present invention to the relative protein amount produced from the reference nucleic acid molecule, i.e. the nucleic acid molecule lacking a 3'- and/or 5'-UTR, respectively, or comprising a reference 3'- and/or 5'-UTR, respectively, a factor may be determined by which the protein production from the artificial nucleic acid molecule according to the present invention is prolonged compared to the protein production from the reference nucleic acid molecule.

Preferably, the at least one 3'- and/or 5'-UTR element in the artificial nucleic acid molecule according to the invention prolongs protein production from said artificial nucleic acid molecule at least 1.2 fold, preferably at least 1.5 fold, more preferably at least 2 fold, even more preferably at least 2.5 fold, compared to the protein production from a reference nucleic acid molecule lacking 3'- and/or 5'-UTR, respectively, or comprising a reference 3'- and/or 5'-UTR, respectively. In other words, the (relative) amount of protein produced from in the artificial nucleic acid molecule according to the invention at a certain later point in time as described above is increased by a factor of at least 1.2, preferably at least 1.5, more preferably at least 2, even more preferably at least 2.5, compared to the (relative) amount of protein produced from a reference nucleic acid molecule, which is e.g. lacking a 3'- and/or 5'-UTR, respectively, or comprising a reference 3'- and/or 5'-UTR, respectively, for the same later point in time.

Alternatively, the effect of prolonging protein production may also be determined by (i) measuring protein amounts, e.g. obtained by expression of an encoded reporter protein such as luciferase, preferably in a mammalian expression system such as in HeLa or HDF cells, over time, (ii) determining the point in time at which the protein amount undercuts the amount of protein observed, e.g., at 1, 2, 3, 4, 5, or 6 hours post initiation of expression, e.g. 1, 2, 3, 4, 5, or 6 hours post transfection of the artificial nucleic acid molecule, and (iii) comparing the point in time at which the protein amount undercuts the protein amount observed at 1, 2, 3, 4, 5, or 6 hours post initiation of expression to said point in time determined for a nucleic acid molecule lacking a 3'- and/or 5'-UTR, respectively, or comprising a reference 3'- and/or 5'-UTR, respectively.

For example, the protein production from the artificial nucleic acid molecule such as the artificial mRNA - in an amount which is at least the amount observed in the initial phase of expression, such as 1, 2, 3, 4, 5, or 6 hours post initiation of expression, such as post transfection of the nucleic acid molecule - is prolonged by at least about 5 hours, preferably by at least about 10 hours, more preferably by at least about 24 hours compared to the protein production from a reference nucleic acid molecule, such as a reference mRNA, in a mammalian expression system, such as in mammalian cells, e.g. in HeLa or HDF cells. Thus, the artificial nucleic acid molecule according to the present invention preferably allows for prolonged protein production in an amount which is at least the amount observed in the initial phase of expression, such as 1, 2, 3, 4, 5, or 6 hours post initiation of expression, such as post transfection, by at least about 5 hours, preferably by at least about 10 hours, more preferably by at least about 24 hours compared to a reference nucleic acid molecule lacking a 3'- and/or 5'-UTR, respectively, or comprising a reference 3'- and/or 5'-UTR, respectively.

In preferred embodiments, the period of protein production from the artificial nucleic acid molecule according to the present invention is extended at least 1.2 fold, preferably at least 1.5 fold, more preferably at least 2 fold, even more preferably at least 2.5 fold, compared to the protein production from a reference nucleic acid molecule lacking a 3'- and/or 5'-UTR, respectively, or comprising a reference 3'- and/or 5'-UTR, respectively.

Preferably, this prolonging effect on protein production is achieved, while the total amount of protein produced from the artificial nucleic acid molecule according to the present invention, e.g. within a time span of 48 or 72 hours, corresponds at least to the amount of protein produced from a reference nucleic acid molecule lacking a 3'- and/or 5'-UTR, respectively, or comprising a reference 3'- and/or 5'-UTR, respectively, such as a 3'-UTR and/or 5'-UTR naturally occurring with the ORF of the artificial nucleic acid molecule. Thus, the present invention provides an artificial nucleic acid molecule which allows for prolonged protein production in a mammalian expression system, such as in mammalian cells, e.g. in HeLa or HDF cells, as specified above, wherein the total amount of protein produced from said artificial nucleic acid molecule, e.g. within a time span of 48 or 72 hours, is at least the total amount of protein produced, e.g. within said time span, from a reference nucleic acid molecule lacking a 3'- and/or 5'-UTR, respectively, or comprising a reference 3'- and/or 5'-UTR, respectively, such as a 3'- and/or 5'-UTR naturally occurring with the ORF of the artificial nucleic acid molecule.

Moreover, the term "prolonged protein expression" also includes "stabilized protein expression", whereby "stabilized protein expression" preferably means that there is more uniform protein production from the artificial nucleic acid molecule according to the present invention over a predetermined period of time, such as over 24 hours, more preferably over 48 hours, even more preferably over 72 hours, when compared to a reference nucleic acid molecule, for example, an mRNA comprising a reference 3'- and/or 5'-UTR, respectively, or lacking a 3'- and/or 5'-UTR, respectively.

Accordingly, the level of protein production, e.g. in a mammalian system, from the artificial nucleic acid molecule comprising a 3'- and/or 5'-UTR element according to the present invention, e.g. from an mRNA according to the present invention, preferably does not drop to the extent observed for a reference nucleic acid molecule, such as a reference mRNA as described above. To assess to which extent the protein production from a specific nucleic acid molecule drops, for example, the amount of a protein (encoded by the respective ORF) observed 24 hours after initiation of expression, e.g. 24 hours post transfection of the artificial nucleic acid molecule according to the present invention into a cell, such as a mammalian cell, may be compared to the amount of protein observed 48 hours after initiation of expression, e.g. 48 hours post transfection. Thus, the ratio of the amount of protein encoded by the ORF of the artificial nucleic acid molecule according to the present invention, such as the amount of a reporter protein, e.g., luciferase, observed at a later point in time, e.g. 48 hours, post initiation of expression, e.g. post transfection, to the amount of protein observed at an earlier point in time, e.g. 24 hours, post initiation of expression, e.g. post transfection, is preferably higher than the corresponding ratio (including the same points in time) for a reference nucleic acid molecule comprising a reference 3'- and/or 5'-UTR, respectively, or lacking a 3'- and/or 5'-UTR, respectively.

Preferably, the ratio of the amount of protein encoded by the ORF of the artificial nucleic acid molecule according to the present invention, such as the amount of a reporter protein, e.g., luciferase, observed at a later point in time, e.g. 48 hours, post initiation of expression, e.g. post transfection, to the amount of protein observed at an earlier point in time, e.g. 24 hours, post initiation of expression, e.g. post transfection, is preferably at least 0.2, more preferably at least about 0.3, even more preferably at least about 0.4, even more preferably at least about 0.5, and particularly preferably at least about 0.7. For a respective reference nucleic acid molecule, e.g. an mRNA comprising a reference 3'- and/or 5'-UTR, respectively, or lacking a 3'- and/or 5'-UTR, respectively, said ratio may be, for example between about 0.05 and about 0.35.

Thus, the present invention provides an artificial nucleic acid molecule comprising an ORF and a 3'- and/or 5'-UTR element as described above, wherein the ratio of the protein amount, e.g. the amount of luciferase, observed 48 hours after initiation of expression to the protein amount observed 24 hours after initiation of expression, preferably in a mammalian expression system, such as in mammalian cells, e.g. in HDF cells or in HeLa cells, is preferably at least 0.2, more preferably at least about 0.3, more preferably at least about 0.4, even more preferably at least about 0.5, even more preferably at least about 0.6, and particularly preferably at least about 0.7. Thereby, preferably the total amount of protein produced from said artificial nucleic acid molecule, e.g. within a time span of 48 hours, corresponds at least to the total amount of protein produced, e.g. within said time span, from a reference nucleic acid molecule lacking a 3'- and/or 5'-UTR, respectively, or comprising a reference 3'- and/or 5'-UTR, respectively, such as a 3'-UTR and/or 5'-UTR naturally occurring with the ORF of the artificial nucleic acid molecule.

Preferably, the present invention provides an artificial nucleic acid molecule comprising an ORF and a 3'-UTR element and/or a 5'-UTR element as described above, wherein the ratio of the protein amount, e.g. the amount of luciferase, observed 72 hours after initiation of expression to the protein amount observed 24 hours after initiation of expression, preferably in a mammalian expression system, such as in mammalian cells, e.g. in HeLa cells or HDF cells, is preferably above about 0.05, more preferably above about 0.1, more preferably above about 0.2, even more preferably above about 0.3, wherein preferably the total amount of protein produced from said artificial nucleic acid molecule, e.g. within a time span of 72 hours, is at least the total amount of protein produced, e.g. within said time span, from a reference nucleic acid molecule lacking a 3'- and/or 5'-UTR, respectively, or comprising a reference 3'- and/or 5'-UTR, respectively, such as a 3'- and/or 5'-UTR naturally occurring with the ORF of the artificial nucleic acid molecule.

"Increased protein expression" or "enhanced protein expression" in the context of the present invention preferably means an increased/enhanced protein expression at one point in time after initiation of expression or an increased/enhanced total amount of expressed protein compared to the expression induced by a reference nucleic acid molecule. Thus, the protein level observed at a certain point in time after initiation of expression, e.g. after transfection, of the artificial nucleic acid molecule according to the present invention, e.g. after transfection of an mRNA according to the present invention, for example, 6, 12, 24, 48 or 72 hours post transfection, is preferably higher than the protein level observed at the same point in time after initiation of expression, e.g. after transfection, of a reference nucleic acid molecule, such as a reference mRNA comprising a reference 3'- and/or 5'-UTR, respectively, or lacking a 3'- and/or 5'-UTR, respectively. In a preferred embodiment, the maximum amount of protein (as determined e.g. by protein activity or mass) expressed from the artificial nucleic acid molecule is increased with respect to the protein amount expressed from a reference nucleic acid comprising a reference 3'- and/or 5'-UTR, respectively, or lacking a 3'- and/or 5'-UTR, respectively. Peak expression levels are preferably reached within 48 hours, more preferably within 24 hours and even more preferably within 12 hours after, for instance, transfection.

Preferably, the term "increased total protein production" or "enhanced total protein production" from an artificial nucleic acid molecule according to the invention refers to an increased/enhanced protein production over a time span, in which protein is produced from an artificial nucleic acid molecule, e.g. 48 hours or 72 hours, preferably in a mammalian expression system, such as in mammalian cells, e.g. in HeLa or HDF cells in comparison to a reference nucleic acid molecule lacking a 3'- and/or 5'-UTR, respectively, or comprising a reference 3'- and/or 5'-UTR, respectively. According

to a preferred embodiment, the cumulative amount of protein expressed over time is increased when using the artificial nucleic acid molecule according to the invention.

The total amount of protein for a specific time period may be determined by (i) collecting tissue or cells at several points in time after introduction of the artificial nucleic acid molecule (e.g. 6, 12, 24, 48 and 72 hours post initiation of expression or post introduction of the nucleic acid molecule), and the protein amount per point in time can be determined as explained above. In order to calculate the cumulative protein amount, a mathematical method of determining the total amount of protein can be used, e.g. the area under the curve (AUC) can be determined according to the following formula:

$$\mathrm{AUC} = \int_a^b f(x)\, d(x)$$

In order to calculate the area under the curve for total amount of protein, the integral of the equation of the expression curve from each end point (a and b) is calculated.

Thus, "total protein production" preferably refers to the area under the curve (AUC) representing protein production over time.

Preferably, the at least one 3'- or 5'-UTR element according to the present invention increases protein production from said artificial nucleic acid molecule at least 1.5 fold, preferably at least 2 fold, more preferably at least 2.5 fold, compared to the protein production from a reference nucleic acid molecule lacking a 3'- and/or 5'-UTR, respectively. In other words, the total amount of protein produced from in the artificial nucleic acid molecule according to the invention at a certain point in time, e.g. 48 hours or 72 hours post initiation of expression, e.g. post transfection, is increased by a factor of at least 1.5, preferably at least 2, more preferably at least 2.5, compared to the (relative) amount of protein produced from a reference nucleic acid molecule, which is e.g. lacking a 3'- and/or 5'-UTR, respectively, or comprising a reference 3'- and/or 5'-UTR, respectively, for the corresponding later point in time.

The mRNA and/or protein production prolonging effect and efficiency and/or the protein production increasing effect and efficiency of the variants, fragments and/or variant fragments of the 3'-UTR and/or the 5'-UTR as well as the mRNA and/or protein production prolonging effect and efficiency and/or the protein production increasing effect and efficiency of the at least one 3'-UTR element and/or the at least one 5'-UTR element of the artificial nucleic acid molecule according to the present invention may be determined by any method suitable for this purpose known to skilled person.

For example, artificial mRNA molecules may be generated comprising a coding sequence/open reading frame (ORF) for a reporter protein, such as luciferase, and a 3'-UTR element according to the present invention, i.e. which prolongs and/or increases protein production from said artificial mRNA molecule. In addition such an inventive mRNA molecule may further comprise a a 5'-UTR element according to the present invention, i.e. which prolongs and/or increases protein production from said artificial mRNA molecule, no 5'-UTR element or a 5'-UTR element which is not according to the present invention, e.g. a reference 5'-UTR. Accordingly, artificial mRNA molecules may be generated comprising a coding sequence/open reading frame (ORF) for a reporter protein, such as luciferase, and a 5'-UTR element according to the present invention, i.e. which prolongs and/or increases protein production from said artificial mRNA molecule. In addition such an inventive mRNA molecule may further comprise a a 3'-UTR element according to the present invention, i.e. which prolongs and/or increases protein production from said artificial mRNA molecule, no 3'-UTR element or a 3'-UTR element which is not according to the present invention, e.g. a reference 3'-UTR.

According to the present invention mRNAs may be generated, for example, by *in vitro* transcription of respective vectors such as plasmid vectors, e.g. comprising a T7 promoter and a sequence encoding the respective mRNA sequences. The generated mRNA molecules may be transfected into cells by any transfection method suitable for transfecting mRNA, for example they may be lipofected into mammalian cells, such as HeLa cells or HDF cells, and samples may be analyzed certain points in time after transfection, for example, 6 hours, 24 hours, 48 hours, and 72 hours post transfection. Said samples may be analyzed for mRNA quantities and/or protein quantities by methods well known to the skilled person. For example, the quantities of reporter mRNA present in the cells at the sample points in time may be determined by quantitative PCR methods. The quantities of reporter protein encoded by the respective mRNAs may be determined, e.g., by Western Blot, ELISA assays, FACS analysisor reporter assays such as luciferase assays depending on the reporter protein used. The effect of stabilizing protein expression and/or prolonging protein expression may be, for example, analyzed by determining the ratio of the protein level observed 48 hours post transfection and the protein level observed 24 hours post transfection. The closer said value is to 1, the more stable the protein expression is within this time period. Such measurements may of course also be performed at 72 or more hours and the ratio of the protein level observed 72 hours post transfection and the protein level observed 24 hours post transfection may be determined to determine stability of protein expression.

Moreover, the at least one 3'-UTR element and/or the at least one 5'-UTR element in the artificial nucleic acid molecule according to the present invention, is derived from a stable mRNA. Thereby, "derived" from a stable mRNA means that the at least one 3'-UTR element and/or the at least one 5'-UTR element shares at least 50%, preferably at least 60%, preferably at least 70%, more preferably at least 75%, more preferably at least 80%, more preferably at least 85%, even more preferably at least 90%, even more preferably at least 95%, and particularly preferably at least 98% sequence identity with a 3'-UTR element and/or a 5'-UTR element of a stable mRNA. Preferably, the stable mRNA is a naturally occurring mRNA and, thus, a 3'-UTR element and/or a 5'-UTR element of a stable mRNA refers to a 3'-UTR and/or a 5'-UTR, or fragments or variants thereof, of naturally occurring mRNA. Moreover, a 3'-UTR element and/or a 5'-UTR element derived from a stable mRNA preferably also refers to a 3'-UTR element and/or a 5'-UTR element, which is modified in comparison to a naturally occurring 3'-UTR element and/or 5'-UTR element, e.g. in order to increase RNA stability even further and/or to prolong and/or increase protein production. It goes without saying that such modifications are preferred, which do not impair RNA stability, e.g. in comparison to a naturally occurring (non-modifed) 3'-UTR element and/or 5'-UTR element. In particular, the term mRNA as used herein refers to an mRNA molecule, however, it may also refer to an mRNA species as defined herein.

Preferably, the stability of mRNA, i.e. mRNA decay and/or half-life, is assessed under standard conditions, for example standard conditions (standard medium, incubation, etc.) for a certain cell line used.

The term "stable mRNA" as used herein refers in general to an mRNA having a slow mRNA decay. Thus, a "stable mRNA" has typically a long half-life. The half-life of an mRNA is the the time required for degrading 50% of the *in vivo or in vitro* existing mRNA molecules. Accordingly, stability of mRNA is usually assessed *in vivo or in vitro.* Thereby, *in vitro* refers in particular to ("living") cells and/or tissue, including tissue of a living subject. Cells include in particular cell lines, primary cells, cells in tissue or subjects. In specific embodiments cell types allowing cell culture may be suitable for the present invention. Particularly preferred are mammalian cells, e.g. human cells and mouse cells. In particularly preferred embodiments the human cell lines HeLa, and U-937 and the mouse cell lines NIH3T3, JAWSII and L929 are used. Furthermore primary cells are particularly preferred, in particular preferred embodiments human dermal fibroblasts (HDF) may be used. Alternatively also a tissue of a subject may be used.

Preferably, the half-life of a "stable mRNA" is at least 5 h, at least 6 h, at least 7 h, at least 8 h, at least 9 h, at least 10 h, at least 11 h, at least 12 h, at least 13 h, at least 14 h, and/or at least 15 h. The half-life of an mRNA of interest may be determined by different methods known to the person skilled in the art. Typically, the half-life of an mRNA of interest is determined by determining the decay constant, whereby usually an ideal *in vivo* (or *in vitro* as defined above) situation is assumed, in which transcription of the mRNA of interest can be "turned off" completely (or at least to an undetectable level). In such an ideal situation it is usually assumed that mRNA decay follows first-order kinetics. Accordingly, the decay of an mRNA may usually be described by the following equation:

$$A(t) = A_0 * e^{-\lambda t}$$

with $A_0$ being the amount (or concentration) of the mRNA of interest at time 0, i.e. before the decay starts, A(t) being the amount (or concentration) of the mRNA of interest at a time t during decay and $\lambda$ being the decay constant. Thus, if the amount (or concentration) of the mRNA of interest at time 0 ($A_0$) and the amount (or concentration) of the mRNA of interest at a certain time t during the decay process (A(t) and t) are known, the decay constant $\lambda$ may be calculated. Based on the decay constant $\lambda$, the half-life $t_{1/2}$ can be calculated by the following equation:

$$t_{1/2} = \ln 2 / \lambda.$$

since per definition A(t)/A0 = 1/2 at $t_{1/2}$. Thus, to assess the half-life of an mRNA of interest, usually the amount or concentration of the mRNA is determined during the RNA decay process *in vivo* (or *in vitro* as defined above).

To determine the amount or concentration of mRNA during the RNA decay process *in vivo* (or *in vitro* as defined above), various methods may be used, which are known to the skilled person. Non-limiting examples of such methods include general inhibition of transcription, e.g. with a transcription inhibitor such as actinomycin D, use of inducible promotors to specifically promote transient transcription, e.g. c-fos serum-inducible promotor system and Tet-off regulatory promotor system, and kinetic labelling techniques, e.g. pulse labelling, for example by 4-Thiouridine (4sU), 5-Ethynyluridine (EU) or 5'-Bromo-Uridine (BrU). Further details and preferred embodiments regarding how to determine the amount or concentration of mRNA during the RNA decay are outlined below, in the context of a method for identifying a 3'-UTR element and/or the at least one 5'-UTR element, which is derived from a stable mRNA, according to the present invention. The respective description and preferred embodiments of how to determine the amount or concentration of mRNA during the RNA decay apply here as well.

Preferably, a "stable mRNA" in the sense of the present invention has a slower mRNA decay compared to

average mRNA, preferably assessed *in vivo* (or *in vitro* as defined above). For example, "average mRNA decay" may be assessed by investigating mRNA decay of a plurality of mRNA species, preferably 100, at least 300, at least 500, at least 1000, at least 2000, at least 3000, at least 4000, at least 5000, at least 6000, at least 7000, at least 8000, at least 9000, at least 10000, at least 11000, at least 12000, at least 13000, at least 14000, at least 15000, at least 16000, at least 17000, at least 18000, at least 19000, at least 20000, at least 21000, at least 22000, at least 23000, at least 24000, at least 25000, at least 26000, at least 27000, at least 28000, at least 29000, at least 30000 mRNA species. It is particularly preferred that the whole transcriptome is assessed, or as many mRNA species of the transcriptome as possible. This may be achieved, for example, by using a micro array providing whole transcript coverage.

An "mRNA species", as used herein, corresponds to a genomic transcription unit, i.e. usually to a gene. Thus, within one "mRNA species" different transcripts may occur, for example, due to mRNA processing. For example, an mRNA species may be represented by a spot on a microarray. Accordingly, a microarray provides an advantageous tool to determine the amount of a plurality of mRNA species, e.g. at a certain point in time during mRNA decay. However, also other techniques known to the skilled person, e.g. RNA-seq, quantitative PCR etc. may be used.

In the present invention it is particularly preferred that a stable mRNA is characterized by an mRNA decay wherein the ratio of the amount of said mRNA at a second point in time to the amount of said mRNA at a first point in time is at least 0.5 (50%), at least 0.6 (60%), at least 0.7 (70%), at least 0.75 (75%), at least 0.8 (80%), at least 0.85 (85%), at least 0.9 (90%), or at least 0.95 (95%). Thereby, the second point in time is later in the decay process than the first point in time.

Preferably, the first point in time is selected such that only mRNA undergoing a decay process is considered, i.e. emerging mRNA - e.g. in ongoing transcription - is avoided. For example, if kinetic labelling techniques, e.g. pulse labelling, are used, the first point in time is preferably selected such that the incorporation of the label into mRNA is completed, i.e. no ongoing incorporation of the label into mRNA occurs. Thus, if kinetic labelling is used, the first point in time may be at least 10 min, at least 20 min, at least 30 min, at least 40 min, at least 50 min, at least 60 min, at least 70 min, at least 80 min, or at least 90 min after the end of the experimental labelling procedure, e.g. after the end of the incubation of cells with the label. For example, the first point in time may be preferably from 0 to 6 h after the stop of transcription (e.g. by a transcriptional inhibitor), stop of promotor induction in case of inducible promotors or after stop of pulse or label supply, e.g. after end of labelling. More preferably, the first point in time may be 30 min to 5 h, even more preferably 1 h to 4 h and particularly preferably about 3 h after the stop of transcription (e.g. by a transcriptional inhibitor), stop of promotor induction in case of inducible promotors or after stop of pulse or label supply, e.g. after end of labelling.

Preferably, the second point in time is selected as late as possible during the mRNA decay process. However, if a plurality of mRNA species is considered, the second point in time is preferably selected such that still a considerable amount of the plurality of mRNA species, preferably at least 10% of the mRNA species, is present in a detectable amount, i.e. in an amount higher than 0. Preferably, the second point in time is at least 5 h, at least 6 h, at least 7 h, at least 8 h, at least 9 h, at least 10 h, at least 11 h, at least 12 h, at least 13 h, at least 14 h, or at least 15 h after the end of transcription or the end of the experimental labelling procedure.

Thus, the time span between the first point in time and the second point in time is preferably as large as possible within the above described limits. Therefore, the time span between the first point in time and the second point in time is preferably at least 4 h, at least 5 h, at least 6 h, at least 7 h, at least 8 h, at least 9 h, at least 10 h, at least 11 h, or at least 12 h.

Moreover, it is preferred that the at least one 3'-UTR element and/or the at least one 5'-UTR element in the artificial nucleic acid molecule according to the present invention, which is derived from a stable mRNA, is identified by a method for identifying a 3'-UTR element and/or a 5'-UTR element, which is derived from a stable mRNA, according to the present invention as described herein. It is particularly preferred that the at least one 3'-UTR element and/or the at least one 5'-UTR element in the artificial nucleic acid molecule according to the present invention, is identified by a method for identifying a 3'-UTR element and/or a 5'-UTR element, which prolongs and/or increases protein production from an artificial nucleic acid molecule and which is derived from a stable mRNA, according to the present invention as described herein.

Preferably, the at least one 3'-UTR element and/or the at least one 5'-UTR element in the artificial nucleic acid molecule according to the present invention comprises or consists of a nucleic acid sequence which is derived from the 3'-UTR and/or the 5'-UTR of a eukaryotic protein coding gene, preferably from the 3'-UTR and/or the 5'-UTR of a vertebrate protein coding gene, more preferably from the 3'-UTR and/or the 5'-UTR of a mammalian protein coding gene, e.g. from mouse and human protein coding genes, even more preferably from the 3'-UTR and/or the 5'-UTR of a primate or rodent protein coding gene, in particular the 3'-UTR and/or the 5'-UTR of a human or murine protein coding gene.

In general, it is understood that the at least one 3'-UTR element in the artificial nucleic acid molecule according to the present invention comprises or consists of a nucleic acid sequence which is preferably derived from a naturally (in nature) occurring 3'-UTR, whereas the at least one 5'-UTR element in the artificial nucleic acid molecule according to the present invention comprises or consists of a nucleic acid sequence which is preferably derived from a naturally

(in nature) occurring 5'-UTR.

Preferably, the at least one open reading frame is heterologous to the at least one 3'-UTR element and/or to the at least one 5'-UTR element. The term "heterologous" in this context means that two sequence elements comprised by the artificial nucleic acid molecule, such as the open reading frame and the 3'-UTR element and/or the open reading frame and the 5'-UTR element, do not occur naturally (in nature) in this combination. They are typically recombinant. Preferably, the 3'-UTR element and/or the 5'-UTR element are/is derived from a different gene than the open reading frame. For example, the ORF may be derived from a different gene than the 3'-UTR element and/or to the at least one 5'-UTR element, e.g. encoding a different protein or the same protein but of a different species etc. I.e. the open reading frame is derived from a gene which is distinct from the gene from which the 3'-UTR element and/or to the at least one 5'-UTR element is derived. In a preferred embodiment, the ORF does not encode a human or plant (e.g., Arabidopsis) ribosomal protein, preferably does not encode human ribosomal protein S6 (RPS6), human ribosomal protein L36a-like (RPL36AL) or Arabidopsis ribosomal protein S16 (RPS16). In a further preferred embodiment, the open reading frame (ORF) does not encode ribosomal protein S6 (RPS6), ribosomal protein L36a-like (RPL36AL) or ribosomal protein S16 (RPS16).

In specific embodiments it is preferred that the open reading frame does not code for a reporter protein, e.g., selected from the group consisting of globin proteins (particularly beta-globin), luciferase protein, GFP proteins or variants thereof, for example, variants exhibiting at least 70% sequence identity to a globin protein, a luciferase protein, or a GFP protein. Thereby, it is particularly preferred that the open reading frame does not code for a GFP protein. It is also particularly preferred that the open reading frame (ORF) does not encode a reporter gene or is not derived from a reporter gene, wherein the reporter gene is preferably not selected from group consisting of globin proteins (particularly beta-globin), luciferase protein, beta-glucuronidase (GUS) and GFP proteins or variants thereof, preferably not selected from EGFP, or variants of any of the above genes, typically exhibiting at least 70% sequence identity to any of these reporter genes, preferably a globin protein, a luciferase protein, or a GFP protein.

Even more preferably, the 3'-UTR element and/or the 5'-UTR element is heterologous to any other element comprised in the artificial nucleic acid as defined herein. For example, if the artificial nucleic acid according to the invention comprises a 3'-UTR element from a given gene, it does preferably not comprise any other nucleic acid sequence, in particular no functional nucleic acid sequence (e.g. coding or regulatory sequence element) from the same gene, including its regulatory sequences at the 5' and 3' terminus of the gene's ORF. Accordingly, for example, if the artificial nucleic acid according to the invention comprises a 5'-UTR element from a given gene, it does preferably not comprise any other nucleic acid sequence, in particular no functional nucleic acid sequence (e.g. coding or regulatory sequence element) from the same gene, including its regulatory sequences at the 5' and 3' terminus of the gene's ORF.

Moreover, it is preferred that the artificial nucleic acid according to the present invention comprises at least one open reading frame, at least one 3'-UTR (element) and at least one 5'-UTR (element), whereby either the at least one 3'-UTR (element) is a 3'-UTR element according to the present invention and/or the at least one 5'-UTR (element) is a 5'-UTR element according to the present invention. In such a preferred artificial nucleic acid according to the present invention, which comprises at least one open reading frame, at least one 3'-UTR (element) and at least one 5'-UTR (element), it is particularly preferred that each of the at least one open reading frame, the at least one 3'-UTR (element) and the at least one 5'-UTR (element) are heterologous, i.e. neither the at least one 3'-UTR (element) and the at least one 5'-UTR (element) nor the the open reading frame and the 3'-UTR (element) or the 5'-UTR (element), respectively, are occurring naturally (in nature) in this combination. This means that the artificial nucleic acid molecule comprises an ORF, a 3'-UTR (element) and a 5'-UTR (element), all of which are heterologous to each other, e.g. they are recombinant as each of them is derived from different genes (and their 5' and 3' UTR's). In another preferred embodiment, the 3'-UTR (element) is not derived from a 3'-UTR (element) of a viral gene or is not of viral origin.

Preferably, the artificial nucleic acid molecule according to the present invention:

(i) comprises at least one 3'-UTR element and at least one 5'-UTR element, wherein preferably (each of) the at least one 3'-UTR element and at least one 5'-UTR element comprises or consists of a nucleic acid sequence which is derived from the 3'-UTR, or the 5'-UTR respectively, of a human or murine protein coding gene;

(ii) the at least one 3'-UTR element, the at least one 5'-UTR element and the at least one open reading frame of the artificial nucleic acid molecule according to the present invention are all heterologous to each other;

(iii) the at least one 3' UTR element is derived from a gene selected from the group consisting of: housekeeping genes, genes coding for a membrane protein, genes involved in cellular metabolism, genes involved in transcription, translation and replication processes, genes involved in protein modification and genes involved in cell division; and

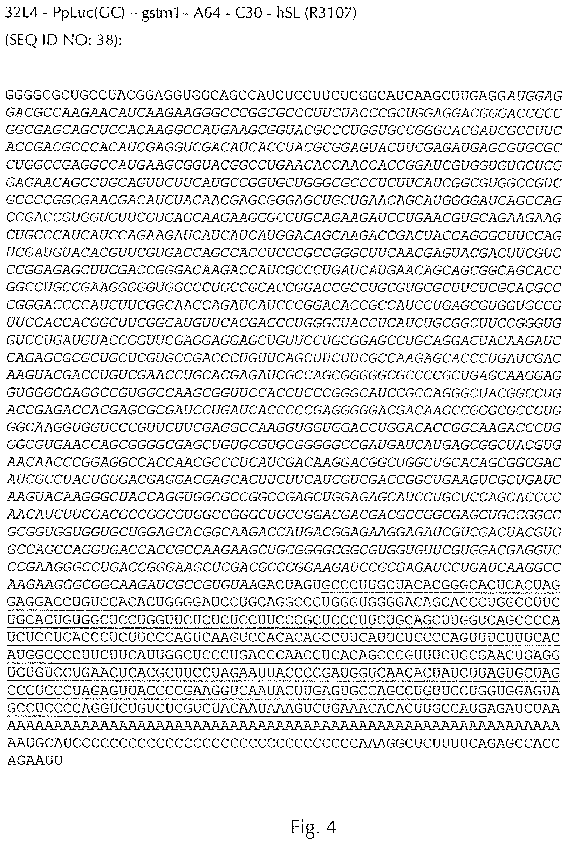

(iv) the 3'UTR is not derived from a gene coding for a ribosomal protein or from the Fig4 gene.

Housekeeping genes are typically constitutive genes that are required for the maintenance of basic cellular function and that are typically expressed in all cells of an organism under normal and patho-physiological conditions. Although some housekeeping genes are expressed at relatively constant levels in most non-pathological situations,

other housekeeping genes may vary depending on experimental conditions. Typically, housekeeping genes are expressed in at least 25 copies per cell and sometimes number in the thousands. Preferred examples of housekeeping genes in the context of the present invention are shown below in Table 10.

Table 10. List of abundant housekeeping genes (cf. WO 2007/068265 A1, Table 1).

| Acc | Definition | Symbol[a] | Length[b] | Abundance[c] |
|---|---|---|---|---|
| NM_ 001402 | Eukaryotic translation elongation factor 1 alpha 1 | EEF1A1 | 387 | 20011 |
| NM_ 001614 | Actin, gamma 1 | ACTG1 | 718 | 16084 |
| NM_ 002046 | Glyceraldehyde-3-phosphate dehydrogenase | GAPD | 201 | 15931 |
| NM_ 001101 | Actin, beta | ACTB | 593 | 15733 |
| NM_ 000967 | Ribosomal protein L3 | RPL3 | 74 | 10924 |
| NM_ 006082 | Tubulin, alpha, ubiquitous | K-ALPHA-1 | 174 | 10416 |
| NM_ 001428 | Enolase 1, (alpha) | ENO1 | 357 | 9816 |
| NM_ 006098 | Guanine nucleotide binding protein (G protein), beta polypeptide 2-like 1 | GNB2L1 | 45 | 8910 |
| NM_ 002032 | Ferritin, heavy polypeptide 1 | FTH1 | 138 | 8861 |
| NM_ 002654 | Pyruvate kinase, muscle | PKM2 | 643 | 7413 |
| NM_ 004048 | Beta-2-microglobulin | B2M | 568 | 7142 |
| NM_ 006597 | Heat shock 70kDa protein 8 | HSPA8 | 258 | 6066 |
| NM_ 000034 | Aldolase A, fructose-bisphosphate | ALDOA | 252 | 5703 |
| NM_ 021009 | Ubiquitin C | UBC | 67 | 5579 |
| NM_ 006013 | Ribosomal protein L10 | RPL10 | 1,503 | 5572 |
| NM_ 012423 | Ribosomal protein L13a | RPL13A | 509 | 5552 |
| NM_ 007355 | Heat shock 90kDa protein 1, beta | HSPCB | 309 | 5436 |
| NM_ 004046 | ATP synthase, H+ transporting, mitochondrial F1 complex, alpha subunit, isoform 1, cardiac muscle | ATP5A1 | 164 | 5434 |
| NM_ 000516 | GNAS complex locus | GNAS | 362 | 4677 |
| NM_ 001743 | Calmodulin 2 (phosphorylase kinase, delta) | CALM2 | 611 | 4306 |
| NM_ 005566 | Lactate dehydrogenase A | LDHA | 566 | 4186 |
| NM_ 000973 | Ribosomal protein L8 | RPL8 | 92 | 4042 |
| NM_ 002948 | Ribosomal protein L15 | RPL15 | 1,368 | 3861 |
| NM_ 000977 | Ribosomal protein L13 | RPL13 | 424 | 3774 |

(continued)

| Acc | Definition | Symbol[a] | Length[b] | Abundance[c] |
|---|---|---|---|---|
| NM_ 002952 | Ribosomal protein S2 | RPS2 | 86 | 3758 |
| NM_ 005507 | Cofilin 1 (non-muscle) | CFL1 | 508 | 3616 |
| NM_ 004039 | Annexin A2 | ANXA2 | 294 | 3560 |
| NM_ 021019 | Myosin, light polypeptide 6, alkali, smooth muscle and non-muscle | MYL6 | 209 | 3512 |
| NM_ 002300 | Lactate dehydrogenase B | LDHB | 230 | 3501 |
| NM_ 003217 | Testis enhanced gene transcript (BAX inhibitor 1) | TEGT | 1,847 | 3438 |
| NM_ 002568 | Poly(A) binding protein, cytoplasmic 1 | PABPC1 | 445 | 3241 |
| NM_ 001015 | Ribosomal protein S11 | RPS11 | 85 | 3220 |
| NM_ 003973 | Ribosomal protein L14 | RPL14 | 156 | 3198 |
| NM_ 000969 | Ribosomal protein L5 | RPL5 | 78 | 3167 |
| NM_ 007104 | Ribosomal protein L10a | RPL10A | 32 | 3079 |
| NM_ 001642 | Amyloid beta (A4) precursor-like protein 2 | APLP2 | 1,364 | 3002 |
| NM_ 001418 | Eukaryotic translation initiation factor 4 gamma, 2 | EIF4G2 | 791 | 2913 |
| NM_ 002635 | Solute carrier family 25 (mitochondrial carrier; phosphate carrier), member 3 | SLC25A3 | 197 | 2900 |
| NM_ 001009 | Ribosomal protein S5 | RPS5 | 58 | 2897 |
| NM_ 000291 | Phosphoglycerate kinase 1 | PGK1 | 1,016 | 2858 |
| NM_ 001728 | Basigin (OK blood group) | BSG | 769 | 2827 |
| NM_ 001658 | ADP-ribosylation factor 1 | ARF1 | 1,194 | 2772 |
| NM_ 001003 | Ribosomal protein, large, P1 | RPLP1 | 39 | 2770 |
| NM_ 018955 | Ubiquitin B | UBB | 144 | 2732 |
| NM_ 005998 | Chaperonin containing TCP1, subunit 3 (gamma) | CCT3 | 255 | 2709 |
| NM_ 001967 | Eukaryotic translation initiation factor 4A, isoform 2 | EIF4A2 | 626 | 2693 |
| NM_ 001469 | Thyroid autoantigen 70kDa (Ku antigen) | G22P1 | 259 | 2682 |
| NM_ 000918 | Procollagen-proline, 2-oxoglutarate 4-dioxygenase (proline 4-hydroxylase), beta polypeptide (protein disulfide isomerase; thyroid hormone binding protein p55) | P4HB | 868 | 2659 |
| NM_ 002574 | Peroxiredoxin 1 | PRDX1 | 323 | 2604 |

(continued)

| **Acc** | **Definition** | Symbol[a] | Length[b] | Abundance[c] |
|---|---|---|---|---|
| NM_ 001020 | Ribosomal protein S16 | RPS16 | 78 | 2573 |
| NM_ 007363 | Non-POU domain containing, octamer-binding | NONO | 1,119 | 2557 |
| NM_ 001022 | Ribosomal protein S19 | RPS19 | 63 | 2533 |
| NM_ 001675 | Activating transcription factor 4 (tax-responsive enhancer element | ATF4 | 85 | 2479 |
| NM_ 005617 | Ribosomal protein S14 | RPS14 | 78 | 2465 |
| NM_ 001664 | Ras homolog gene family, member A | RHOA | 1,045 | 2426 |
| NM_ 005801 | Putative translation initiation factor | SUI1 | 836 | 2425 |
| NM_ 0009B1 | Ribosomal protein L19 | RPL19 | 80 | 2381 |
| NM_ 000979 | Ribosomal protein L18 | RPL18 | 49 | 2362 |
| NM_ 001026 | Ribosomal protein S24 | RPS24 | 77 | 2355 |
| NM_ 000975 | Ribosomal protein L11 | RPL11 | 53 | 2314 |
| NM_ 002117 | Major histocompatibility complex, class I, C | HLA-C | 434 | 2278 |
| NM_ 004068 | Adaptor-related protein complex 2, mu 1 subunit | AP2M1 | 494 | 2230 |
| NM_ 006429 | Chaperonin containing TCP1, subunit 7 (eta) | CCT7 | 164 | 2216 |
| NM_ 022551 | Ribosomal protein S18 | RPS18 | 5,538 | 2208 |
| NM_ 001013 | Ribosomal protein S9 | RPS9 | 73 | 2113 |
| NM_ 005594 | Nascent-polypeptide-associated complex alpha polypeptide | NACA | 133 | 2075 |
| NM_ 001028 | Ribosomal protein S25 | RPS25 | 74 | 2066 |
| NM_ 032378 | Eukaryotic translation elongation factor 1 delta (guanine nucleotide exchange protein) | EEF1D | 76 | 2051 |
| NM_ 000999 | Ribosomal protein L38 | RPL38 | 50 | 2007 |
| NM_ 000994 | Ribosomal protein L32 | RPL32 | 64 | 2003 |
| NM_ 007008 | Reticulon 4 | RTN4 | 973 | 1969 |
| NM_ 001909 | Cathepsin D (lysosomal aspartyl protease) | CTSD | 834 | 1940 |
| NM_ 006325 | RAN, member RAS oncogene family | RAN | 892 | 1906 |
| NM_ 003406 | Tyrosine 3-monooxygenase/tryptophan 5-monooxygenase activation protein, zeta polypeptide | YWHAZ | 2,013 | 1892 |
| NM_ 006868 | Calmodulin 1 (phosphorylase kinase, delta) | CALM1 | 3,067 | 1880 |

(continued)

| Acc | Definition | Symbol[a] | Length[b] | Abundance[c] |
|---|---|---|---|---|
| NM_ 004339 | Pituitary tumor-transforming 1 interacting protein | PTTG11P | 1,985 | 1837 |
| NM_ 005022 | Profilin 1 | PFN1 | 289 | 1787 |
| NM_ 001961 | Eukaryotic translation elongation factor 2 | EEF2 | 504 | 1754 |
| NM_ 003091 | Small nuclear ribonucleoprotein polypeptides B and B1 | SNRPB | 295 | 1735 |
| NM_ 006826 | Tyrosine 3-monooxygenase/tryptophan 5-monooxygenase activation protein, theta polypeptide | YWHAQ | 1,310 | 1726 |
| NM_ 002140 | Heterogeneous nuclear ribonucleoprotein K | HNRPK | 1,227 | 1725 |
| NM_ 001064 | Transketolase (Wernicke-Korsakoff syndrome) | TKT | 167 | 1721 |
| NM_ 021103 | Thymosin, beta 10 | TMSB10 | 317 | 1714 |
| NM_ 004309 | Rho GDP dissociation inhibitor (GDI) alpha | ARHGDIA | 1,206 | 1702 |
| NM_ 002473 | Myosin, heavy polypeptide 9, non-muscle | MYH9 | 1,392 | 1692 |
| NM_ 000884 | IMP (inosine monophosphate) dehydrogenase 2 | IMPDH2 | 63 | 1690 |
| NM_ 001004 | Ribosomal protein, large P2 | RPLP2 | 59 | 1688 |
| NM_ 001746 | Calnexin | CANX | 2,302 | 1677 |
| NM_ 002819 | Polypyrimidine tract binding protein 1 | PTBP1 | 1,561 | 1663 |
| NM_ 000988 | Ribosomal protein L27 | RPL27 | 59 | 1660 |
| NM_ 004404 | Neural precursor cell expressed, developmentally down-regulated 5 | NEDD5 | 2,090 | 1654 |
| NM_ 005347 | Heat shock 70kDa protein 5 (glucose-regulated protein, 78kDa) | HSPA5 | 1,757 | 1651 |
| NM_ 000175 | Glucose phosphate isomerase | GPI | 296 | 1635 |
| NM_ 001207 | Basic transcription factor 3 | BTF3 | 300 | 1632 |
| NM_ 003186 | Transgelin | TAGLN | 405 | 1612 |
| NM_ 003334 | Ubiquitin-activating enzyme E1 (A1S9T and BN75 temperature sensitivity complementing) | UBE1 | 199 | 1590 |
| NM_ 001018 | Ribosomal protein S15 | RPS15 | 32 | 1574 |
| NM_ 003404 | Tyrosine 3-monooxygenase/tryptophan 5-monooxygenase activation protein, beta polypeptide | YWHAB | 2,088 | 1523 |
| NM_ 003753 | Eukaryotic translation initiation factor 3, subunit 7 zeta, 66/67kDa | EIF3S7 | 152 | 1509 |
| NM_ 005762 | Tripartite motif-containing 28 | TRIM28 | 193 | 1507 |
| NM_ 005381 | Nucleolin | NCL | 284 | 1501 |

(continued)

| Acc | Definition | Symbol[a] | Length[b] | Abundance[c] |
|---|---|---|---|---|
| NM_ 000995 | Ribosomal protein L34 | RPL34 | 450 | 1495 |
| NM_ 002823 | Prothymosin, alpha (gene sequence 28) | PTMA | 720 | 1462 |
| NM_ 002415 | Macrophage migration inhibitory factor (glycosylation-inhibiting factor) | MIF | 117 | 1459 |
| NM_ 002128 | High-mobility group box 1 | HMGB1 | 1,527 | 1457 |
| NM_ 006908 | Ras-related C3 botulinum toxin substrate 1 (rho family, small GTP protein Rac1) | RAC1 | 1,536 | 1437 |
| NM_ 002070 | Guanine nucleotide binding protein (G protein), alpha inhibiting activity polypeptide 2 | GNAI2 | 512 | 1435 |
| NM_ 001997 | Finket-Biskis-Reilly murine sarcoma virus (FBR-MuSV) ubiquitously expressed (fox derived); ribosomal protein S30 | FAU | 68 | 1428 |
| NM_ 014390 | Staphylococcal nuclease domain containing 1 | SND1 | 556 | 1422 |
| NM_ 014764 | DAZ associated protein 2 | DAZAP2 | 1,322 | 1419 |
| NM_ 005917 | Malate dehydrogenase 1, NAD (soluble) | MDH1 | 208 | 1396 |
| NM_ 001494 | GDP dissociation inhibitor 2 | GDI2 | 785 | 1395 |
| NM_ 014225 | Protein phosphatase 2 (formerly 2A), regulatory subunit A (PR 65), alpha isoform | PPP2R1A | 472 | 1391 |
| NM_ 001660 | ADP-ribosylation factor 4 | ARF4 | 858 | 1382 |
| NM_ 001823 | Creatine kinase, brain | CKB | 206 | 1381 |
| NM_ 003379 | Villin 2 (ezrin) | VIL2 | 1,272 | 1380 |
| NM_ 000182 | Hydroxyacyl-Coenzyme A dehydrogenase/3-ketoacyl-Coenzyme A thiolase/enoyl-Coenzyme A hydratase (trifunctional protein), alpha subunit | HADHA | 647 | 1379 |
| NM_ 003746 | Dynein, cytoplasmic, light polypeptide 1 | DNCL1 | 281 | 1375 |
| NM_ 007103 | NADH dehydrogenase (ubiquinone) flavoprotein 1, 51kDa | NDUFV1 | 103 | 1352 |
| NM_ 000992 | Ribosomal protein L29 | RPL29 | 164 | 1349 |
| NM_ 007209 | Ribosomal protein L35 | RPL35 | 35 | 1345 |
| NM_ 006623 | Phosphoglycerate dehydrogenase | PHGDH | 231 | 1340 |
| NM_ 002796 | Proteasome (prosome, macropain) subunit, beta type, 4 | PSMB4 | 108 | 1340 |
| NM_ 002808 | Proteasome (prosome, macropain) 26S subunit, non-ATPase, 2 | PSMD2 | 231 | 1326 |
| NM_ 000454 | Superoxide dismutase 1, soluble (amyotrophic lateral sclerosis 1 (adult)) | SOD1 | 346 | 1323 |
| NM_ 003915 | RNA binding motif protein 12 | RBM12 | 216 | 1323 |

(continued)

| Acc | Definition | Symbol[a] | Length[b] | Abundance[c] |
|---|---|---|---|---|
| NM_ 004924 | Actinin, alpha 4 | ACTN4 | 1,099 | 1316 |
| NM_ 006086 | Tubulin, beta 3 | TUBB3 | 296 | 1314 |
| NM_ 001016 | Ribosomal protein S12 | RPS12 | 56 | 1304 |
| NM_ 003365 | Ubiquinol-cytochrome c reductase core protein I | UQCRC1 | 126 | 1303 |
| NM_ 003016 | Splicing factor, arginine/serine-rich 2 | SFRS2 | 1,059 | 1301 |
| NM_ 007273 | Repressor of estrogen receptor activity | REA | 332 | 1281 |
| NM_ 014610 | Glucosidase, alpha; neutral AB | GANAB | 1,652 | 1280 |
| NM_ 001749 | Calpain, small subunit 1 | CAPNS1 | 514 | 1270 |
| NM_ 005080 | X-box binding protein 1 | XBP1 | 1,003 | 1269 |
| NM_ 005216 | Dolichyl-diphosphooligosaccharide-protein glycosyltransferase | DDOST | 616 | 1268 |
| NM_ 004640 | HLA-B associated transcript 1 | BAT1 | 237 | 1262 |
| NM_ 021983 | Major histocompatibility complex, class II, DR beta 4 | HLA-DRB1 | 313 | 1251 |
| NM_ 013234 | Eukaryotic translation initiation factor 3 subunit k | eIF3k | 84 | 1251 |
| NM_ 004515 | Interleukin enhancer binding factor 2, 45kDa | ILF2 | 384 | 1249 |
| NM_ 000997 | Ribosomal protein L37 | RPL37 | 50 | 1244 |
| NM_ 000801 | FK506 binding protein 1A. 12kDa | FKBP1A | 1,149 | 1243 |
| NM_ 000985 | Ribosomal protein L17 | RPL17 | 58 | 1243 |
| NM_ 001014 | Ribosomal protein S10 | RPS10 | 57 | 1232 |
| NM_ 001069 | Tubulin, beta 2 | TUBB2 | 194 | 1230 |
| NM_ 004960 | Fusion (involved in t(12;16) in malignant liposarcoma) | FUS | 166 | 1197 |
| NM_ 005165 | Aldolase C, fructose-bisphosphate | ALDOC | 432 | 1195 |
| NM_ 004930 | Capping protein (actin filament) muscle Z-line, beta | CAPZB | 259 | 1193 |
| NM_ 000239 | Lysozyme (renal amyloidosis) | LYZ | 1,016 | 1190 |
| NM_ 007263 | Coatomer protein complex, subunit epsilon | COPE | 263 | 1179 |
| NM_ 001861 | Cytochrome c oxidase subunit IV isoform 1 | COX411 | 129 | 1178 |
| NM_ 003757 | Eukaryotic translation initiation factor 3, subunit 2 beta, 36kDa | EIF3S2 | 408 | 1169 |

(continued)

| Acc | Definition | Symbol[a] | Length[b] | Abundance[c] |
|---|---|---|---|---|
| NM_ 005745 | B-cell receptor-associated protein 31 | BCAP31 | 438 | 1166 |
| NM_ 002743 | Protein kinase C substrate 80K-H | PRKCSH | 337 | 1158 |
| MM_ 004161 | RA81A, member RAS oncogene family | RAB1A | 638 | 1115 |
| NM_ 002080 | Glutamic-oxaloacetic transaminase 2, mitochondrial (aspartate aminotransferase 2) | GOT2 | 1,039 | 1114 |
| NM_ 005731 | Actin related protein 2/3 complex, subunit 2, 34kDa | ARPC2 | 448 | 1113 |
| NM_ 006445 | PRP8 pre-mRNA processing factor 8 homolog (yeast) | PRPF8 | 173 | 1110 |
| NM_ 001867 | Cytochrome c oxidase subunit VIIc | COX7C | 168 | 1106 |
| NM_ 002375 | Microtubule-associated protein 4 | MAP4 | 1,164 | 1102 |
| NM_ 003145 | Signal sequence receptor, beta (translocon-associated protein beta) | SSR2 | 492 | 1099 |
| NM_ 001788 | CDC10 cell division cycle 10 homolog (S. cerevisiae) | CDC10 | 1,015 | 1094 |
| NM_ 006513 | Seryl-tRNA synthetase | SARS | 323 | 1085 |
| NM_ 003754 | Eukaryotic translation initiation factor 3, subunit 5 epsilon, 47kDa | EIF3S5 | 152 | 1081 |
| NM_ 005112 | WD repeat domain 1 | WDR1 | 845 | 1080 |
| NM_ 004893 | H2A histone family, member Y | H2AFY | 635 | 1072 |
| NM_ 004494 | Hepatoma-derived growth factor (high-mobility group protein 1-like) | HDGF | 1,339 | 1069 |
| NM_ 001436 | Fibrillarin | FBL | 111 | 1069 |
| NM_ 003752 | Eukaryotic translation initiation factor 3, subunit 8, 110kDa | EIF3S8 | 201 | 1060 |
| NM_ 003321 | Tu translation elongation factor, mitochondrial | TUFM | 207 | 1038 |
| NM_ 001119 | Adducin 1 (alpha) | ADD1 | 1,569 | 1037 |
| NM_ 005273 | Guanine nucleotide binding protein (G protein), beta polypeptide 2 | GNB2 | 386 | 1030 |
| NM_ 006755 | Transaldolase 1 | TALDO1 | 256 | 1026 |
| NM_ 023009 | MARCKS-like 1 | MARCKSL 1 | 774 | 1014 |
| NM_ 002799 | Proteasome (prosome, macropain) subunit, beta type, 7 | PSMB7 | 162 | 1012 |
| NM_ 002539 | Omithine decarboxylase 1 | ODC1 | 343 | 1009 |
| NM_ 006801 | KDEL (Lys-Asp-Glu-Leu) endoplasmic reticulum protein retention receptor 1 | KDELR1 | 742 | 1007 |
| NM_ 014944 | Calsyntenin 1 | CLSTN1 | 1,481 | 1003 |

(continued)

| Acc | Definition | Symbol[a] | Length[b] | Abundance[c] |
|---|---|---|---|---|
| NM_ 007262 | Parkinson disease (autosomal recessive, early onset) 7 | PARK7 | 253 | 1002 |

The above table was obtained from WO 2007/068265 A1, Table 1 and is based on the list of the accession numbers as provided by Eisenberg, E. and E. Y. Levanon (2003): Human housekeeping genes are compact; Trends Genet. 19(7): 362-365. The accession numbers were used as input for a PERL (Programmed Extraction Report Language) computer program that extracts EST data from the Unigene database. The Unigene database was downloaded as a text file from the NCBI website. The length of the 3'UTR was derived by computationally extracting the 3'UTR (Bakheet, T., Frevel, M., Williams, BR, and K.S. Khabar, 2001. ARED: Human AU-rich element-containing mRNA database reveals unexpectedly diverse functional repertoire of encoded proteins. Nucleic Acids Research. 29:246-254). <a> is a commonly used abbreviation of the gene product; <b> is the length of the 3 'UTR; <c> is the number of ESTs.

Preferred housekeeping genes include LDHA, NONO, PGK1 and PPIH.

A gene coding for a membrane protein typically refers to such a gene, which codes for a protein that interacts with biological membranes. In most genomes, about 20 - 30% of all genes encode membrane proteins. Common types of proteins include - in addition to membrane proteins - soluble globular proteins, fibrous proteins and disordered proteins. Thus, genes coding for a membrane protein are typically different from genes coding for soluble globular proteins, fibrous proteins or disordered proteins. Membrane proteins include membrane receptors, transport proteins, membrane enzymes and cell adhesion molecules.

A gene involved in cellular metabolism typically refers to such a gene, which codes for a protein involved in cellular metabolism, i.e. in the set of life-sustaining chemical transformations within the cells of living organisms. These are typically enzyme-catalyzed reactions, which allow organisms to grow and reproduce, maintain their structures, and respond to their environments. Accordingly, preferred genes involved in cellular metabolism are such genes, which code for enzymes catalyzing a reaction, which allow organisms to grow and reproduce, maintain their structures, and respond to their environments. Other examples for a gene involved in cellular metabolism include genes coding for proteins having structural or mechanical function, such as those that form the cytoskeleton. Other proteins involved in cellular metabolism include proteins involved in cell signalling, immune responses, cell adhesion, active transport across membranes and in the cell cycle. Metabolism is usually divided into two categories: catabolism, the breaking down of organic matter by way of cellular respiration, and anabolism, the building up of components of cells such as proteins and nucleic acids.

A gene involved in transcription, translation and replication processes typically refers to such a gene, which codes for a protein involved in transcription, translation and replication processes. In particular, the term "replication", as used in this context, refers preferably to replication of nucleic acids, e.g. DNA replication. Preferred genes involved in transcription, translation and replication processes are genes coding for an enzyme involved in transcription, translation and/or (DNA) replication processes. Other preferred examples include genes coding for a transcription factor or for a translation factor. Ribosomal genes are other preferred examples of genes involved in transcription, translation and replication processes.

A gene involved in protein modification typically refers to such a gene, which codes for a protein involved in protein modification. Preferred examples of such genes code for enzymes involved in protein modification, in particular in post-translational modification processes. Preferred examples of enzymes involved in post-translational modification include (i) enzymes involved in the addition of hydrophobic groups, in particular for membrane localization, e.g. enzymes involed in myristoylation, palmitoylation, isoprenylation or prenylation, farnesylation, geranylation or in glypiation; (ii) enzymes involved in the addition of cofactors for enhanced enzymatic activity, e.g. enzymes involved in lipoylation, in the attachment of a flavin moiety, in the attachment of heme C, in phosphopantetheinylation or in retinylidene Schiff base formation; (iii) enzymes involved in the modification of translation factors, e.g. in diphtamide formation, in ethanolamine phosphoglycerol attachment or in hypusine formation; and (vi) enzymes involved in the addition of smaller chemical groups, e.g. acylation, such as acetylation and formylation, alkylation such as methylation, amide bond formation, such as amidation at C-terminus and amino acid addition (e.g. arginylation, polyglutamylation and polyglycylation), butyrylation, gamma-carboxylation, glycosylation, malonylation, hydroxylation, iodination, nucleotide addition, oxidation, phosphate ester or phosphoramidate formation such as phosphorylation and adenylation, propionylation, pyroglutamate formation, S-glutathionylation, S-nitrosylation, succinylation and sulfation.

A gene involved in cell division processes typically refers to such a gene, which codes for a protein involved in cell division. Cell division is the process by which a parent cell divides into two or more daughter cells. Cell division usually occurs as part of a larger cell cycle. In eukaryotes, there are two distinct types of cell division: a vegetative division, whereby each daughter cell is genetically identical to the parent cell (mitosis), and a reductive cell division, whereby the number of chromosomes in the daughter cells is reduced by half, to produce haploid gametes (meiosis).

Accordingly, preferred gene involved in cell division processes code for a protein involved in mitosis and/or meiosis.

Fig4 is an abbreviation for Factor-Induced Gene. The Fig4 gene codes for polyphosphoinositide phosphatase also known as phosphatidylinositol 3,5-bisphosphate 5-phosphatase or SAC domain-containing protein 3 (Sac3).

Preferably, the artificial nucleic acid molecule according to the present invention:

(i) comprises at least one 3'-UTR element and at least one 5'-UTR element, wherein preferably (each of) the at least one 3'-UTR element and at least one 5'-UTR element comprises or consists of a nucleic acid sequence which is derived from the 3'-UTR, or the 5'-UTR respectively, of a human or murine protein coding gene;
(ii) the at least one 3'-UTR element, the at least one 5'-UTR element and the at least one open reading frame are all heterologous to each other;
(iii) the at least one 5'-UTR element is derived from a gene selected from the group consisting of: housekeeping genes, genes coding for a membrane protein, genes involved in cellular metabolism, genes involved in transcription, translation and replication processes, genes involved in protein modification and genes involved in cell division;
(iv) the 5'-UTR is preferably not a 5' TOP UTR; and
(v) the 3'-UTR is preferably not derived from a gene coding for a ribosomal protein or for albumin or from the Fig4 gene.

More preferably, such an artificial nucleic acid molecule according to the present invention:

(i) comprises at least one 3'-UTR element and at least one 5'-UTR element, wherein preferably (each of) the at least one 3'-UTR element and at least one 5'-UTR element comprises or consists of a nucleic acid sequence which is derived from the 3'-UTR, or the 5'-UTR respectively, of a human or murine protein coding gene;
(ii) the at least one 3'-UTR element, the at least one 5'-UTR element and the at least one open reading frame are all heterologous to each other;
(iii) the at least one 3' UTR element is derived from a human or a murine gene selected from the group consisting of: housekeeping genes, genes coding for a membrane protein, genes involved in cellular metabolism, genes involved in transcription, translation and replication processes, genes involved in protein modification and genes involved in cell division;
(iv) the 3'UTR is not derived from a gene coding for a ribosomal protein or for albumin or from the Fig4 gene;
(v) the at least one 5'-UTR element is derived from a human or a murine gene selected from the group consisting of: housekeeping genes, genes coding for a membrane protein, genes involved in cellular metabolism, genes involved in transcription, translation and replication processes, genes involved in protein modification and genes involved in cell division; and
(vi) the 5'-UTR is not a 5' TOP UTR.

Thereby, it is preferred in the artificial nucleic acid molecule according to the present invention that the 3'-UTR and the 5'-UTR are derived from a human or a murine housekeeping gene. It is also preferred that the 3'-UTR and the 5'-UTR are derived from a human or a murine gene coding for a membrane protein. It is also preferred that the 3'-UTR and the 5'-UTR are derived from a human or a murine gene involved in cellular metabolism. It is also preferred that the 3'-UTR and the 5'-UTR are derived from a human or a murine gene involved in transcription, translation and replication processes. It is also preferred that the 3'-UTR and the 5'-UTR are derived from a human or a murine gene involved in protein modification. It is also preferred that the 3'-UTR and the 5'-UTR are derived from a human or a murine gene involved in cell division. In this context, the skilled person is aware that if (i) the 3'-UTR and the 5'-UTR are derived from genes belonging to the same gene class and (ii) the at least one 3'-UTR and the at least one 5'-UTR are heterologous to each other, that the the 3'-UTR and the 5'-UTR are not derived from the same gene, but from distinct genes belonging to the same gene class. Accordingly, it is preferred that the at least one 3'-UTR and the at least one 5'-UTR are derived from distinct genes belonging to the same gene class.

As used herein the term "gene class" refers to the classification of genes. Examples of gene classes include (i) housekeeping genes, (ii) genes coding for a membrane protein, (iii) genes involved in cellular metabolism, (iv) genes involved in transcription, translation and replication processes, (v) genes involved in protein modification and (vi) genes involved in cell division. In other words, "housekeeping genes" is one gene class, whereas "genes involved in transcription" is another gene class, "genes involved in cellular metabolism" is a further gene class, etc..

It is also preferred in the artificial nucleic acid molecule according to the present invention as described herein, that the 3'-UTR and the 5'-UTR are derived from a human or a murine gene selected from the group consisting of: genes coding for a membrane protein, genes involved in cellular metabolism, genes involved in transcription, translation and replication processes, genes involved in protein modification and genes involved in cell division, wherein the 3'-UTR and the 5'-UTR are selected from distinct gene classes.

Preferably, the at least one 3'-UTR element and/or to the at least one 5'-UTR element is functionally linked to the ORF. This means preferably that the 3'-UTR element and/or to the at least one 5'-UTR element is associated with

the ORF such that it may exert a function, such as an enhancing or stabilizing function on the expression of the encoded peptide or protein or a stabilizing function on the artificial nucleic acid molecule. Preferably, the ORF and the 3'-UTR element are associated in 5'→3' direction and/or the 5'-UTR element and the ORF are associated in 5'→3' direction. Thus, preferably, the artificial nucleic acid molecule comprises in general the structure 5'-[5'-UTR element]-(optional)-linker-ORF-(optional)-linker-[3'-UTR element]-3', wherein the artificial nucleic acid molecule may comprise only a 5'-UTR element and no 3'-UTR element, only a 3'-UTR element and no 5'-UTR element, or both, a 3'-UTR element and a 5'-UTR element. Furthermore, the linker may be present or absent. For example, the linker may be one or more nucleotides, such as a stretch of 1-50 or 1-20 nucleotides, e.g., comprising or consisting of one or more restriction enzyme recognition sites (restriction sites).

Preferably, the at least one 3'-UTR element and/or the at least one 5'-UTR element comprises or consists of a nucleic acid sequence which is derived from the 3'-UTR and/or the 5'-UTR of a transcript of a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), CNTN1 (contactin 1), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, Atp5e, Gstm5, Uqcr11, Ifi27l2a, Anapc13, Atp5l, Tmsb10, Nenf, Ndufa7, Atp5k, 1110008P14Rik, Cox4i1, Cox6a1, Ndufs6, Sec61b, Romo1, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufs5, 1810027O10Rik, Atp5o, Shfm1, Tspo, S100a6, Taldo1, Bloc1s1, Hexa, Ndufb11, Map1lc3a, Gpx4, Mif, Cox6b1, RIKEN cDNA2900010J23 (Swi5), Sec61g, 2900010M23Rik, Anapc5, Mars2, Phpt1, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, LMBRD2, TLR3, BCAT1, TOM1L1, SLC35A1, GLYATL2, STAT4, GULP1, EHHADH, NBEAL1, KIAA1598, HFE, KIAA1324L, and MANSC1.

In a particularly preferred embodiment the at least one 3'-UTR element and/or the at least one 5'-UTR element comprises or consists of a nucleic acid sequence which is derived from the 3'-UTR and/or the 5'-UTR of a transcript of a gene selected from the group consisting of GNAS (guanine nucleotide binding protein, alpha stimulating complex locus), MORN2 (MORN repeat containing 2), NDUFA1 (NADH dehydrogenase (ubiquinone) 1 alpha subcomplex), CBR2 (carbonyl reductase 2), MP68 (RIKEN cDNA 2010107E04 gene), NDUFA4 (NADH dehydrogenase (ubiquinone) 1 alpha subcomplex 4), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CASP1, SNX14, SKAP2, NDUFB6, EFHA1, BCKDHB, BBS2, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, Atp5e, Gstm5, Uqcr11, Ifi27l2a, Anapc13, Atp5l, Nenf, Ndufa7, Atp5k, 1110008P14Rik, Cox4i1, Cox6a1, Ndufs6, Sec61b, Romo1, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Ndufs5, 1810027O10Rik, Atp5o, Shfm1, Tspo, S100a6, Taldo1, Bloc1s1, Hexa, Ndufb11, Map1lc3a, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Ldhb, Nme2, Snrpg, Ndufa2, Serf1, Oaz1, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4,

GULP1, EHHADH, NBEAL1, KIAA1598, HFE, KIAA1324L, and MANSC1.

More preferably, the at least one 3'-UTR element and/or the at least one 5'-UTR element comprises or consists of a nucleic acid sequence which is derived from the 3'-UTR and/or the 5'-UTR of a transcript of a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), MP68 (RIKEN cDNA 2010107E04 gene), Ybx1 (Y-Box binding protein 1), Ndufb8 (NADH dehydrogenase (ubiquinone) 1 beta subcomplex 8), CNTN1 (contactin 1) and NDUFA4 (NADH dehydrogenase (ubiquinone) 1 alpha subcomplex 4).

Preferably, the at least one 3'-UTR element and/or the at least one 5'-UTR element of the artificial nucleic acid molecule according to the present invention comprises or consists of a "functional fragment", a "functional variant" or a "functional fragment of a variant" of the 3'-UTR and/or the 5'-UTR of a transcript of a gene.

Preferably, the at least one 3'-UTR element and/or the at least one 5'-UTR element comprises a nucleic acid sequence which is derived from the 3'-UTR and/or the 5'-UTR of a transcript of a human gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), MP68 (RIKEN cDNA 2010107E04 gene), NDUFA4 (NADH dehydrogenase (ubiquinone) 1 alpha subcomplex 4), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1.

Alternatively or additionally, it is also preferred that the at least one 3'-UTR element and/or the at least one 5'-UTR element comprises a nucleic acid sequence which is derived from the 3'-UTR and/or the 5'-UTR of a transcript of a murine gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), MP68 (RIKEN cDNA 2010107E04 gene), NDUFA4 (NADH dehydrogenase (ubiquinone) 1 alpha subcomplex 4), Atp5e, Gstm5, Uqcr11, Ifi27l2a, Anapc13, Atp5l, Tmsb10, Nenf, Ndufa7, Atp5k, 1110008P14Rik, Cox4i1, Cox6a1, Ndufs6, Sec61b, Romo1, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufs5, 1810027O10Rik, Atp5o, Shfm1, Tspo, S100a6, Taldo1, Bloc1s1, Hexa, Ndufb11, Map1lc3a, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, and Gaa.

Preferably, the at least one 3'-UTR element comprises a nucleic acid sequence which is derived from the 3'-UTR of a transcript of a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), SLC38A6, DECR1, PIGK, FAM175A, PHYH, TBC1D19, PIGB, ALG6, CRYZ, BRP44L, ACADSB, TMEM14A, GRAMD1C, C11orf80, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, HADHB, ANAPC4, PCCB, ABCB7, PGCP, NFU1, OMA1, HHLA3, ACAA2, GSTM4, ALG8, Atp5e, Gstm5, Uqcr11, Ifi27l2a, Cbr2, Atp5l, Tmsb10, Nenf, Atp5k, 1110008P14Rik, Cox4i1, Cox6a1, Ndufs6, Sec61b, Romo1, Gnas, Snrpd2, Mgst3, Aldh2, Ss4, Myl6, Prdx4, Ubl5, 1110001J03Rik, Ndufa13, Ndufa3, Gstp2, Tmem160, Ergic3, Pgcp, Slpi, Myeov2, Ndufa4, Ndufs5, Gstm1, 1810027O10Rik, Atp5o, Shfm1, Tspo, S100a6, Taldo1, Bloc1s1, Ndufb11, Map1lc3a, Morn2, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8,

ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1. More preferably, the at least one 3'-UTR element comprises or consists of a nucleic acid sequence which is derived from the 3'-UTR of a transcript of a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), Ybx1 (Y-Box binding protein 1), Ndufb8 (NADH dehydrogenase (ubiquinone) 1 beta subcomplex 8), and CNTN1 (contactin 1).

In a particularly preferred embodiment, the at least one 3'-UTR element comprises a nucleic acid sequence which is derived from the 3'-UTR of a transcript of a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), SLC38A6, DECR1, PIGK, FAM175A, PHYH, TBC1D19, PIGB, ALG6, CRYZ, BRP44L, ACADSB, TMEM14A, GRAMD1C, C11orf80, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, HADHB, ANAPC4, PCCB, ABCB7, PGCP, NFU1, OMA1, HHLA3, ACAA2, GSTM4, ALG8, Atp5e, Gstm5, Uqcr11, Ifi27l2a, Cbr2, Atp5l, Tmsb10, Nenf, Atp5k, 1110008P14Rik, Cox4i1, Cox6a1, Ndufs6, Sec61b, Romo1, Gnas, Snrpd2, Mgst3, Aldh2, Ss4, Myl6, Prdx4, Ubl5, 1110001J03Rik, Ndufa13, Ndufa3, Gstp2, Tmem160, Ergic3, Pgcp, Slpi, Myeov2, Ndufa4, Ndufs5, Gstm1, 1810027O10Rik, Atp5o, Shfm1, Tspo, S100a6, Taldo1, Bloc1s1, Ndufb11, Map1lc3a, Morn2, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1 324L, and MANSC1. More preferably, the at least one 3'-UTR element comprises or consists of a nucleic acid sequence which is derived from the 3'-UTR of a transcript of a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), Ybx1 (Y-Box binding protein 1), Ndufb8 (NADH dehydrogenase (ubiquinone) 1 beta subcomplex 8), and CNTN1 (contactin 1).

More preferably, the at least one 3'-UTR element comprises a nucleic acid sequence which is derived from the 3'-UTR of a transcript of a human gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), SLC38A6, DECR1, PIGK, FAM175A, PHYH, TBC1D19, PIGB, ALG6, CRYZ, BRP44L, ACADSB, TMEM14A, GRAMD1C, C11orf80, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, HADHB, ANAPC4, PCCB, ABCB7, PGCP, NFU1, OMA1, HHLA3, ACAA2, GSTM4, ALG8, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2L11, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1 324L, and MANSC1; preferably, the at least one 3'-UTR element comprises or consists of a nucleic acid sequence which is derived from the 3'-UTR of a transcript of a human gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), Ybx1 (Y-Box binding protein 1), Ndufb8 (NADH dehydrogenase (ubiquinone) 1 beta subcomplex 8), and CNTN1 (contactin 1).

Accordingly, it is also more preferable that the at least one 3'-UTR element comprises a nucleic acid sequence which is derived from the 3'-UTR of a transcript of a murine gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), Ybx1 (Y-Box binding protein 1), Ndufb8 (NADH dehydrogenase (ubiquinone) 1 beta subcomplex 8), and CNTN1 (contactin 1), Ndufa1, Atp5e, Gstm5, Uqcr11, Ifi27l2a, Cbr2, Atp5l, Tmsb10, Nenf, Atp5k, 1110008P14Rik, Cox4i1, Cox6a1, Ndufs6, Sec61b, Romo1, Gnas, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufa4, Ndufs5, Gstm1, 1810027O10Rik, Atp5o, Shfm1, Tspo, S100a6, Taldo1, Bloc1s1, Ndufb11, Map1lc3a, Morn2, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, and Gaa; preferably, the at least one 3'-UTR element comprises or consists of a nucleic acid sequence which is derived from the 3'-UTR of a transcript of a murine gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), Ybx1 (Y-Box binding protein 1), Ndufb8 (NADH dehydrogenase (ubiquinone) 1 beta subcomplex 8), and CNTN1 (contactin 1).

Preferably, the at least one 5'-UTR element comprises a nucleic acid sequence which is derived from the 5'-UTR of a transcript of a gene selected from the group consisting of MP68 (RIKEN cDNA 2010107E04 gene), NDUFA4 (NADH dehydrogenase (ubiquinone) 1 alpha subcomplex 4), LTA4H, DECR1, PIGK, TBC1D19, BRP44L, ACADSB, SUPT3H, TMEM14A, C9orf46, ANXA4, IFI6, C2orf34, ALDH6A1, CCDC53, CCDC104, CASP1, NDUFB6, BCKDHB, BBS2, HERC5, FAM175A, NT5DC1, RAB7A, AGA, TPK1, MBNL3, MCCC2, CAT, ANAPC4, PHKB, ABCB7, GPD2, TMEM38B, NFU1, LOC128322/NUTF2, NUBPL, LANCL1, PIR, CTBS, GSTM4, Ndufa1, Atp5e, Gstm5, Cbr2, Anapc13, Ndufa7, Atp5k, 1110008P14Rik, Cox4i1, Ndufs6, Sec61b, Snrpd2, Mgst3, Prdx4; Pgcp; Myeov2; Ndufa4; Ndufs5; Gstm1; Atp5o; Tspo; Taldo1; Bloc1s1; and Hexa. More preferably, the at least one 5'-UTR element comprises or consists of a nucleic acid sequence which is derived from the 5'-UTR of a transcript of MP68 (RIKEN cDNA 2010107E04 gene) or NDUFA4 (NADH dehydrogenase (ubiquinone) 1 alpha subcomplex 4).

In a particularly preferred embodiment, the at least one 5'-UTR element comprises a nucleic acid sequence which is derived from the 5'-UTR of a transcript of a gene selected from the group consisting of MP68 (RIKEN cDNA 2010107E04 gene), NDUFA4 (NADH dehydrogenase (ubiquinone) 1 alpha subcomplex 4), LTA4H, DECR1, PIGK, TBC1D19, BRP44L, ACADSB, SUPT3H, TMEM14A, C9orf46, ANXA4, IFI6, C2orf34, ALDH6A1, CCDC53, CASP1, NDUFB6, BCKDHB, BBS2, HERC5, FAM175A, NT5DC1, RAB7A, AGA, TPK1, MBNL3, MCCC2, CAT, ANAPC4, PHKB, ABCB7, GPD2, TMEM38B, NFU1, LOC128322/NUTF2, NUBPL, LANCL1, PIR, CTBS, GSTM4, Ndufa1, Atp5e, Gstm5, Cbr2, Anapc13, Ndufa7, Atp5k, 1110008P14Rik, Cox4i1, Ndufs6, Sec61b, Snrpd2, Mgst3, Prdx4; Pgcp; Ndufa4; Ndufs5; Atp5o; Tspo; Taldo1; Bloc1s1; and Hexa. More preferably, the at least one 5'-UTR element comprises or consists of a nucleic acid sequence which is derived from the 5'-UTR of a transcript of MP68 (RIKEN cDNA 2010107E04 gene) or NDUFA4 (NADH dehydrogenase (ubiquinone) 1 alpha subcomplex 4).

More preferably, the at least one 5'-UTR element comprises a nucleic acid sequence which is derived from the 5'-UTR of a transcript of a human gene selected from the group consisting of MP68 (RIKEN cDNA 2010107E04 gene), NDUFA4 (NADH dehydrogenase (ubiquinone) 1 alpha subcomplex 4), LTA4H, DECR1, PIGK, TBC1D19, BRP44L, ACADSB, SUPT3H, TMEM14A, C9orf46, ANXA4, IFI6, C2orf34, ALDH6A1, CCDC53, CCDC104, CASP1, NDUFB6, BCKDHB, BBS2, HERC5, FAM175A, NT5DC1, RAB7A, AGA, TPK1, MBNL3, MCCC2, CAT, ANAPC4, PHKB, ABCB7, GPD2, TMEM38B, NFU1, LOC128322/NUTF2, NUBPL, LANCL1, PIR, CTBS, and GSTM4; preferably, the at least one 5'-UTR element comprises or consists of a nucleic acid sequence which is derived from the 5'-UTR of a human transcript of MP68 (RIKEN cDNA 2010107E04 gene) or NDUFA4 (NADH dehydrogenase (ubiquinone) 1 alpha subcomplex 4).

Accordingly, it is also more preferable that the at least one 5'-UTR element comprises a nucleic acid sequence which is derived from the 5'-UTR of a transcript of a murine gene selected from the group consisting of MP68 (RIKEN cDNA 2010107E04 gene), NDUFA4 (NADH dehydrogenase (ubiquinone) 1 alpha subcomplex 4), Ndufa1, Atp5e, Gstm5, Cbr2, Anapc13, Ndufa7, Atp5k, 1110008P14Rik, Cox4i1, Ndufs6, Sec61b, Snrpd2, Mgst3, Prdx4; Pgcp; Myeov2; Ndufa4; Ndufs5; Gstm1; Atp5o; Tspo; Taldo1; Bloc1s1; and Hexa; preferably, the at least one 5'-UTR element comprises or consists of a nucleic acid sequence which is derived from the 5'-UTR of a murine transcript of MP68 (RIKEN cDNA 2010107E04 gene) or NDUFA4 (NADH dehydrogenase (ubiquinone) 1 alpha subcomplex 4).

The phrase "nucleic acid sequence which is derived from the 3'-UTR and/or the 5'-UTR of a of a transcript of a gene" preferably refers to a nucleic acid sequence which is based on the 3'-UTR sequence and/or on the 5'-UTR sequence of a transcript of a gene or a fragment or part thereof, preferably a naturally occurring gene or a fragment or part thereof. This phrase includes sequences corresponding to the entire 3'-UTR sequence and/or the entire 5'-UTR sequence, i.e. the full length 3'-UTR and/or 5'-UTR sequence of a transcript of a gene, and sequences corresponding

to a fragment of the 3'-UTR sequence and/or the 5'-UTR sequence of a transcript of a gene. Preferably, a fragment of a 3'-UTR and/or a 5'-UTR of a transcript of a gene consists of a continuous stretch of nucleotides corresponding to a continuous stretch of nucleotides in the full-length 3'-UTR and/or 5'-UTR of a transcript of a gene, which represents at least 5%, 10%, 20%, preferably at least 30%, more preferably at least 40%, more preferably at least 50%, even more preferably at least 60%, even more preferably at least 70%, even more preferably at least 80%, and most preferably at least 90% of the full-length 3'-UTR and/or 5'-UTR of a transcript of a gene. Such a fragment, in the sense of the present invention, is preferably a functional fragment as described herein. Preferably, the fragment retains a regulatory function for the translation of the ORF linked to the 3'-UTR and/or 5'-UTR or fragment thereof.

The terms "variant of the 3'-UTR and/or variant of the 5'-UTR of a of a transcript of a gene" and "variant thereof" in the context of a 3'-UTR and/or a 5'-UTR of a transcript of a gene refers to a variant of the 3'-UTR and/or 5'-UTR of a transcript of a naturally occurring gene, preferably to a variant of the 3'-UTR and/or 5'-UTR of a transcript of a vertebrate gene, more preferably to a variant of the 3'-UTR and/or 5'-UTR of a transcript of a mammalian gene, even more preferably to a variant of the 3'-UTR and/or 5'-UTR of a transcript of a primate gene, in particular a human gene as described above. Such variant may be a modified 3'-UTR and/or 5'-UTR of a transcript of a gene. For example, a variant 3'-UTR and/or a variant of the 5'-UTR may exhibit one or more nucleotide deletions, insertions, additions and/or substitutions compared to the naturally occurring 3'-UTR and/or 5'-UTR from which the variant is derived. Preferably, a variant of a 3'-UTR and/or variant of the 5'-UTR of a of a transcript of a gene is at least 40%, preferably at least 50%, more preferably at least 60%, more preferably at least 70%, even more preferably at least 80%, even more preferably at least 90%, most preferably at least 95% identical to the naturally occurring 3'-UTR and/or 5'-UTR the variant is derived from. Preferably, the variant is a functional variant as described herein.

The phrase "a nucleic acid sequence which is derived from a variant of the 3'-UTR and/or from a variant of the 5'-UTR of a of a transcript of a gene" preferably refers to a nucleic acid sequence which is based on a variant of the 3'-UTR sequence and/or the 5'-UTR of a transcript of a gene or on a fragment or part thereof as described above. This phrase includes sequences corresponding to the entire sequence of the variant of the 3'-UTR and/or the 5'-UTR of a transcript of a gene, i.e. the full length variant 3'-UTR sequence and/or the full length variant 5'-UTR sequence of a transcript of a gene, and sequences corresponding to a fragment of the variant 3'-UTR sequence and/or a fragment of the variant 5'-UTR sequence of a transcript of a gene. Preferably, a fragment of a variant of the 3'-UTR and/or the 5'-UTR of a transcript of a gene consists of a continuous stretch of nucleotides corresponding to a continuous stretch of nucleotides in the full-length variant of the 3'-UTR and/or the 5'-UTR of a transcript of a gene, which represents at least 20%, preferably at least 30%, more preferably at least 40%, more preferably at least 50%, even more preferably at least 60%, even more preferably at least 70%, even more preferably at least 80%, and most preferably at least 90% of the full-length variant of the 3'-UTR and/or the 5'-UTR of a transcript of a gene. Such a fragment of a variant, in the sense of the present invention, is preferably a functional fragment of a variant as described herein.

The terms "functional variant", "functional fragment", and "functional fragment of a variant" (also termed "functional variant fragment") in the context of the present invention, mean that the fragment of the 3'-UTR and/or the 5'-UTR, the variant of the 3'-UTR and/or the 5'-UTR, or the fragment of a variant of the 3'-UTR and/or the 5'-UTR of a transcript of a gene fulfils at least one, preferably more than one function of the naturally occurring 3'-UTR and/or 5'-UTR of a transcript of a gene of which the variant, the fragment, or the fragment of a variant is derived. Such function may be, for example, stabilizing mRNA and/or enhancing, stabilizing and/or prolonging protein production from an mRNA and/or increasing protein expression or total protein production from an mRNA, preferably in a mammalian cell, such as in a human cell. Preferably, the function of the 3'-UTR and/or the 5'-UTR concerns the translation of the protein encoded by the ORF. More preferably, the function comprises enhancing translation efficiency of the ORF linked to the 3'-UTR and/or the 5'-UTR or fragment or variant thereof. It is particularly preferred that the variant, the fragment, and the variant fragment in the context of the present invention fulfil the function of stabilizing an mRNA, preferably in a mammalian cell, such as a human cell, compared to an mRNA comprising a reference 3'-UTR and/or a reference 5'-UTR or lacking a 3'-UTR and/or a 5'-UTR, and/or the function of enhancing, stabilizing and/or prolonging protein production from an mRNA, preferably in a mammalian cell, such as in a human cell, compared to an mRNA comprising a reference 3'-UTR and/or a reference 5'-UTR or lacking a 3'-UTR and/or a 5'-UTR, and/or the function of increasing protein production from an mRNA, preferably in a mammalian cell, such as in a human cell, compared to an mRNA comprising a reference 3'-UTR and/or a reference 5'-UTR or lacking a 3'-UTR and/or a 5'-UTR. A reference 3'-UTR and/or a reference 5'-UTR may be, for example, a 3'-UTR and/or a 5'-UTR naturally occurring in combination with the ORF. Furthermore, a functional variant, a functional fragment, or a functional variant fragment of a 3'-UTR and/or a 5'-UTR of a transcript of a gene preferably does not have a substantially diminishing effect on the efficiency of translation of the mRNA which comprises such variant, fragment, or variant fragment of a 3'-UTR and/or a 5'-UTR compared to the wild type 3'-UTR and/or the wild-type 5'-UTR from which the variant, the fragment, or the variant fragment is derived. A particularly preferred function of a "functional fragment", a "functional variant" or a "functional fragment of a variant" of the 3'-UTR and/or the 5'-UTR of a transcript of a gene in the context of the present invention is the enhancement, stabilization and/or prolongation of protein production by expression of an mRNA carrying the functional fragment, functional variant or functional fragment

of a variant as described above.

Preferably, the efficiency of the one or more functions exerted by the functional variant, the functional fragment, or the functional variant fragment, such as mRNA and/or protein production stabilizing efficiency and/or the protein production increasing efficiency, is increased by at least 5%, more preferably by at least 10%, more preferably by at least 20%, more preferably by at least 30%, more preferably by at least 40%, more preferably by at least 50%, more preferably by at least 60%, even more preferably by at least 70%, even more preferably by at least 80%, most preferably by at least 90% with respect to the mRNA and/or protein production stabilizing efficiency and/or the protein production increasing efficiency exhibited by the naturally occurring 3'-UTR and/or 5'-UTR of a transcript of a gene from which the variant, the fragment or the variant fragment is derived.

In the context of the present invention, a fragment of the 3'-UTR and/or of the 5'-UTR of a transcript of a gene or of a variant of the 3'-UTR and/or of the 5'-UTR of a transcript of a gene preferably exhibits a length of at least about 3 nucleotides, preferably of at least about 5 nucleotides, more preferably of at least about 10, 15, 20, 25 or 30 nucleotides, even more preferably of at least about 50 nucleotides, most preferably of at least about 70 nucleotides. Preferably, such fragment of the 3'-UTR and/or of the 5'-UTR of a transcript of a gene or of a variant of the 3'-UTR and/or of the 5'-UTR of a transcript of a gene is a functional fragment as described above. In a preferred embodiment, the 3'-UTR and/or the 5'-UTR of a transcript of a gene or a fragment or variant thereof exhibits a length of between 3 and about 500 nucleotides, preferably of between 5 and about 150 nucleotides, more preferably of between 10 and 100 nucleotides, even more preferably of between 15 and 90, most preferably of between 20 and 70. Typically, the 5'-UTR element and/or the 3'-UTR element is characterized by less than 500, 400, 300, 200, 150 or less than 100 nucleotides.

Preferably, the at least one 3'-UTR element comprises or consists of a nucleic acid sequence which has an identity of at least about 1, 2, 3, 4, 5, 10, 15, 20, 30 or 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 1 to 24 and SEQ ID NOs: 49 to 318 or the corresponding RNA sequence, respectively, or wherein the at least one 3'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 1 to 24 and SEQ ID NOs: 49 to 318 or the corresponding RNA sequence, respectively:

Homo sapiens SLC38A6 3'-UTR
SLC38A6-001 ENST00000267488

AAGAAATATTTTCCTACTTCTTACAAGAATAATATACCCCTAGTTGCAAGAATGAATTATTCCGGA
AGACACCCTGGATGAAAAATAACATTTTAATAAAAATTATTAACAGAAAAGCAGAACAAAATGGCA
GTGGGTATGGGGAAGTAAGAGTGTGGCAGTTTTAATCAAAAAAAGAAACAAACTCGAAATGCTCTT
AAA

(SEQ ID NO:49)

Homo sapiens DECR1 3'-UTR
NM 001359.1

GACCACTTTGGCCTTCATCTTGGTTACAGAAAAGGGAATAGAAATGAAACAAATTATCTCTCATCT
TTTGACTATTTCAAGTCTAATAAATTCTTAATTAAC

(SEQ ID NO:50)

Homo sapiens PIGK 3'-UTR

ACTTGATGATGAATGAAGAATGCATGGAGGACTGCAAACTTGGATAATAATTTATGTCATTATATA
TTTTTAAAAATGTGTTTCTCTTGTATGAATTGGAAATAAGTATAAGGAAACTAAATTTGAATCAAC
TATTAATTTTATAACTTAAAGAAAAATAATTGTTAATGCAACTGCTTAATGGCACTAAATATATTC
CAGTTTTGTATTTTGTGTATTATAAAAGCGAATGAGACAGAGATCAGAATACATTGACTGTTTTG
AAAATAGTAATTTCCCCTTATCCCCTTTTCATTTGGAAAAGAAACAATTGTGAAGACATTAAATTC
TCACTAACAGAAGTAACTTTGGTTAATTATTTTTTGTAT

(SEQ ID NO:51)

Homo sapiens FAM175A 3'-UTR
FAM175A-009 ENST00000506553

TCCTTTTAACCTTACAAGGAGATTTTTTTATTTGGCTGATGGGTAAAGCCAAACATTTCTATTGTT
TTTACTATGTTGAGCTACTTGCAGTAAGTTCATTTGTTTTTACTATGTTCACCTGTTTGCAGTAAT
ACACAGATAACTCTTAGTGCATTTACTTCACAAAGTACTTTTTCAAACATCAGATGCTTTTATTTC
CAAACCTTTTTTTCACCTTTCACTAAGTTGTTGAGGGGAAGGCTTACACAGACACATTCTTTAGAA
TTGGAAAAGTGAGACCAGGCACAGTGGCTCACACCTGTAATCCCAGCACTTAGGGAAGACAAGTCA
GGAGGATTGATTGAAGTTAGGAGTTAGAGACCAGCCTGGGCAACGTATTGAGACCATGTCTATTAA
AAAATAAAATGGAAAAGCAAGAATAGCCTTATTTTCAAAATATGGAAAGAAATTTATATGAAAA

(SEQ ID NO:52)

Homo sapiens PHYH 3'-UTR
PHYH-002 ENST00000396913

AATAGCCATCTGCTATAACTCTTTCAACAGAAAACCAAAACCAAACGAAATGTCTAAGGAAAATGT
TTTCTTAATGAGATGATGTAACCTTTTCTATCACTTGTTAAAAGCAGAAAACATGTATCAGGTACT
TAATTGCATAGAGTTAGTTTTGCAGCACAATGGTGTTGCTTTAATGGAAAAAAAAAACAGTAAAAG

TGAAATATTACTGTTTTAAGGAAAACTAATTTAGGGTGGCAGCCAATAAAGGTGGTTGGTGTCTAA
TTTAAGTGTTAAATCAATTTCTTTCATTCAGTTAGCTCTTTACCCAAGAAGAAGTGAATGATTTGG
AGCTTAGGGTATGTTTTGTATCCCCTTTCTGATAAACCCATTCCCTACCAATTTTATGTCATAAGA
GATTTTTTTCCCCCAAATCTAGAACAATGTATAATACATTCACATCTAGTCAAGGGCATAGGAACG
GTGTCATGGAGTCCAAATAAAGTGGATATTCCTGCTCGGACAA

(SEQ ID NO:53)

Homo sapiens TBC1D19 3'-UTR
TBC1D19-001 ENST00000264866

TCTTCTTCACAGTCACTGGCAACACATCTAGTTTTTCATTAGAAACAAATCATGAACTATGCAAAC
TCTGCATAAAACCAAAATGAAACTTTGCATATAAGCCAATAAAGATCATGTTCCCTCTTCAGTTAA
ACCTAAGTAGTTTCTCACTTTTTGAAACAATAACTCTGCACCAAATATTGCATCGCATGCTGCTGA
TTTTCAAGAGAGAAGCAATAAACACAACTTCTGCTAAATTGAGCATTATATATATATAATATTATAAT
ATATATATAATCCTGACTTGTCAATGGCATGTAATAATATATGCAATAAGAACTAAAGATACTGTA
ATAAACTTCAAGAGGTAATGTAGCTTCTTGGATAATTCTTTTATGTCAGTTTATAAATTTATCTCT
AGATAATG

(SEQ ID NO:54)

Homo sapiens TBC1D19 NM 018317.2 3'-UTR

TCTTCTTCACAGTCACTGGCAACACATCTAGTTTTTCATTAGAAACAAATCATGAACTATGCAAAC
TCTGCATAAAACCAAAATGAAACTTTGCATATAAGCCAATAAAGATCATGTTCCCTCTTCAGTTAA
ACCTAAGTAGTTTCTCACTTTTTGAAACAATAACTCTGCACCAAATATTGCATCGCATGCTGCTGA
TTTTCAAGAGAGAAGCAATAAACACAACTTCTGCTAAATTGAGCATTATATATATAATATTATAAT
ATATATATAATCCTGACTTGTCAATGGCATGTAATAATATATGCAATAAGAACTAAAGATACTGTA
ATAAACTTCAAGAGGTAAAAAAA

(SEQ ID NO:55)

Homo sapiens PIGB 3'-UTR
PIGB-201 ENST00000539642

AAATTCAACATGAAGATGAAATTCTGAACTTTCCTAGATAAATTAACATTGCTGGGTGGAAATATT
CAGATGCTGCTTAAATACTTCGGTAAACACTGGGTAAGATTCATGGAACTTAGAAAAAAGCTGTAT
GAACTGCTTTACCAAATATCACTACTGAGGAAATGTATAAAATACCACATAGTATAAAATTACATG
TTAATACAATGCCAGATTTTAAATAAAGACCTTTAGTTTTCCTC

(SEQ ID NO:56)

Homo sapiens ALG6 3'-UTR
ALG6-006 ENST00000263440

CTGTATTCCTAAACAAATTGTTTCCTAAACAAATGTGAAAATGTGAACAGTGCTGAAAGGTTTTGT
GAACTTTTTGCTATGTATAAATGAAATTACCATTTTGAGAACCATGGAACCACAGGAAAGGAAATG
GTGAAAAGTCATTGTTGTCTACACA

(SEQ ID NO:57)

Homo sapiens CRYZ 3'-UTR
CRYZ-005 ENST00000370871

TGATTAATTCTTTCATGGATTTCCTATGTAATTAGAGGTACTGTCTTTCCCCCAGTTGTACTTACC
CTATCTTTTCTTTAATTAACATTCGATTCCATGAGCTTCTTATGTGAAAAAATAAGATTTTTCTTT
AGAGAGCAGAAGCAGAAGAGTAAAATTTATTGTATAGCTAGCAATATTTTTTTATGCCATCTGTCT
CAAATCAAAGAGTCATCATAGTAGGAAATAACATGTTAGTTGTCATTTGGCATGAGTGTGCATTCC
AGTAATTCTTAATTGATATTTGATTAATTCCATACCTTTGATTAAAACATGCTAGTTCAA

(SEQ ID NO:58)

Homo sapiens BRP44L 3'-UTR
BRP44L-001 ENST00000360961

CAATGGAAAAGGAAGAACAAGGTCTTGAAGGGACAGCATTGCCAGCTGCTGCTGAGTCACAGATTT
CATTATAAATAGCCTCCCTAAGGAAAATACACTGAATGCTATTTTTACTAACCATTCTATTTTTAT
AGAAATAGCTGAGAGTTTCTAAACCAACTCTCTGCTGCCTTACAAGTATTAAATATTTTACTTCTT
TCCATAAAGAGTAGCTCAAAATATGCAATTAATTTAATAATTTCTGATGATGGTTTTATCTGCAGT
AATATGTATATCATCTATTAGAATTTACTTAATGAAAAACTGAAGAGAACAAAATTTGTAACCACT
AGCACTTAAGTACTCCTGATTCTTAACATTGTCTTTAATGACCACAAGACAACCAACAGCTGGCCA
CGTACTTAAAATTTTGTCCCCACTGTTTAAAAATGTTACCTGTGTATTTCCATGCAGTGTATATAT
TGAGATGCTGTAACTTAATGGCAATAAATGATTTAAATATTTGTTAAA

(SEQ ID NO:59)

Homo sapiens ACADSB 3'-UTR
ACADSB-004

CGTCTATAGGAGTGGGACCCCTCCCTGGTGTCACTGCTGTAAAATTTTAAACGGTTGTGTCTTGTT
GGGAGTAAGTGCCTTGCGTGGGAATAAACTTCCACAGCATTCGAATATTTTAATGAAGCCCTTAGT
CAGGGTCCTGGTGTTGGCCTTTTTGGTTTTCTCTTTTCAGGCTGTTTAACTTAGGCACAGGAGATC
CACTTTTAAACTTGGGAAATAAGCACCTGTATTTTTTTCCAAAACTGTTTTTAAAGCTGTATACGC
ATACATATATATATTTTTACTCTGTCTTACTCTGTCACCCAGGCTAGAGTGCAGTGGCGCGATCTC
AGCTCACTGCAGCCTTGACCTCCT

(SEQ ID NO:60)

Homo sapiens TMEM14A 3'-UTR
NM 014051.3

GCATCTGGAGGAACAGAAAACTAAGTTCATGTCATCCTGCTGTAATGGGCAGAGCATATTTTTTTT
GTATTTAAAAGATAAACTTCAATATGGAATGCTAGAAACACAAATAGCACTGTCACCTCTAATATG
AACATTAGTTTGAGGTAGTTTTTTTCTAAAGCAAAAATTTTAACTGTTTTCTAATTGTCAAGCACT
ATTTTCATTAAAAGTGTCTAATGAATCATGATATACTCTTCCATTTGTTGTGTCTATTTTTTATAT
ATTTGGTATTTTTTTGAAAATTCCAAATACTCATGTCTCAAGTAAGCTTAAACTACAACTTGTCACA
TAAAGGAAGTCTTAAGTGGAGTTCACAGAATGATAATGTATCTATTTGTCATTTGTGTTATATTTG
AAATTATTAGAAATTATGCTTTTTCCATTTTAATTGTATTGCTGCCAGTGCTATTTTTTTCTTTAA
AAAATTTTATTCTTAGCACACTGTTATGTCCTAACTGAATGTATTCAGTATTCAAATAAAAGACAT
TTTGGTTCAAA

(SEQ ID NO:61)

Homo sapiens GRAMD1C 3'-UTR
GRAMD1C-005 ENST00000472026

TGATCTGAAGGACTAAAACCGCAGAGATACTTGGAACTTAAAGAAAATACCTGGAAGAAAACCAGA
CGAATGAAGGATTTTGGCATAGAACATTTCTATGTTTTTTCATTATTGAGATTTCTAATATGAACA
TTTCTTTCAGTAACATTTATTTGATAATTAGTTTCTGCTGGCCTTAATAATCCATCCTTTCACTTC
TTATAGATATTTTTAAGCTGTGAATTTCTTCAGTGAACCATGAAATATATTATAGAACTGAATTTC
TCTGATACAAAAAGAAAATGACACACCC

(SEQ ID NO:62)

Homo sapiens C11orf80 3'-UTR
C11orf80-201 ENST00000360962

GCCGGGTCCCCTTCCGCAAGCGCCCACCGATCCGGAGGCTGCGGGCAGCCGTTATCCCGTGGTTTA
ATAAAGCTGCCGCGCGCTCACCAAGTCC

(SEQ ID NO:63)

Homo sapiens ANXA4 3'-UTR
ANXA4-002 ENST00000409920

AATAAAAATCCCAGAAGGACAGGAGGATTCTCAACACTTTGAATTTTTTAACTTCATTTTTCTAC
ACTGCTATTATCATTATCTCAGAATGCTTATTTCCAATTAAAACGCCTACAGCTGCCTCCTAGAAT
ATAGACTGTCTGTATTATTATTCACCTATAATTAGTCATTATGATGCTTTAAAGCTGTACTTGCAT
TTCAAAGCTTATAAGATATAAATGGAGATTTTAAAGTAGAAATAAATATGTATTCCATGTTTTTAA
AA

(SEQ ID NO:64)

Homo sapiens TBCK 3'-UTR
TBCK-002 ENST00000361687

AGAACCAAGAGTGTGACTGCCAAAACTTAGTGTGGCATCAGCACCAACAGCACAGTTCTTCATATC
CACGCCACTCTCAGACAAAACTAGATGTCCAGATTGTTGCATTTCCGTAAAGTTTGTCACGAGACA
TTTTTTAAAATCTCATAACCCACATGTTCAGTTATCCATGCAAGAAACTTGACTCTACATGTATTG
CTGAAAGAATTTTCTTAACAGTGAAATCTGATCATATATTTTTACCACACTGCCACATAAAGCCCA
AGAAATTCAGCTGACAAGACAGATTTAGCATTATCAAGAAATCCCATTTGCCCTGAAAAAGCTGTC
CTCCATTGTACTGAACAGACAGTCCTGTCGATTGTGTTATTTAGAAACATACACTGAATGTGGGCT
GAAATCATCATCTTTCCATAATGAAAACTGAGAAACTATTCACAATGCATTCCTTATAAATAAATG
CTACATTTAGTAACTCATTTCACCCAAA

(SEQ ID NO:65)

Homo sapiens IFI6 3'-UTR
IFI6-001 ENST00000361157

CCAGCAGCTCCCAGAACCTCTTCTTCCTTCTTGGCCTAACTCTTCCAGTTAGGATCTAGAACTTTG
CCTTTTTTTTTTTTTTTTTTTTTTTGAGATGGGTTCTCACTATATTGTCCAGGCTAGAGTGCAGTG
GCTATTCACAGATGCGAACATAGTACACTGCAGCCTCCAACTCCTAGCCTCAAGTGATCCTCCTGT
CTCAACCTCCCAAGTAGGATTACAAGCATGCGCCGACGATGCCCAGAATCCAGAACTTTGTCTATC
ACTCTCCCCAACAACCTAGATGTGAAAACAGAATAAACTTCACCCAGAAAACACTT

(SEQ ID NO:66)

Homo sapiens CAMKMT 3'-UTR
(synonym C2orf34) ENST00000378494

AAGATTAAGCTTCTCAAAGACGAAGAAACGTATCAAGTGCATAGGGAATATTTTTACAAAAACGGA
AATCTGTAAGGGGTATAATCGCCTGCCTGCGCCCTTTGCAGCATTTCACGTGTGGGCTATGGACTC
CACCTGTCCTCACCCACGTTATTCCCCAGCTGCCCTCTCCAGCTCCCTCCCCGCCTCTTTTTACAC
TCTGCTTGTTGCTCGTCCTGCCCTAAACCTTTGTTTGTCTTTAAATGTGTATAAGCTGCCTGTCTG
TGACTTGAATTTGACTGGTGAACAAACTAAATATTTTTCCCTGTAATTGAGACAGAATTTCTTTTG
ATGATACCCATCCCTCCTTCATTTTTTTTTTTTTTTTGGTCTTTGTTCTGTTTTGGTGGTGGTAGT
TTTTAATCAGTAAACCCAGCAAATATCATGATTCTTTCCTGGTTAGAAAAATAAATAAAGTGTATC
TTTTTATCTCCCTCCAA

(SEQ ID NO:67)

Homo sapiens ALDH6A1 3'-UTR
NM 005589.2

AAACAAGTTTGTTTAAGACTGACTCCATCCTGAGTAATCTCCCTTTATTTTTGACCAGCTTCATTT
GTCAGCTTTGCTCAGATCAGATCGATGGGATTGGAATACATTGTAACTAAAATCTTCCTCAGGACT
ATTAACCCCCGCAAAGTTTCTATAGGGAACTGCCTAGTGTAACAATGAAACCAGATTTCTCACTTG
CTCTTCATACTTCTATTTTGAGGTAACTGTTGTAACTATGAAATGCTTATCTGAAAGTAGTGCTTA
AACCTGATTTCTAAAAATTATCCCATTTTCTGATGATTTGAAGGGGAGAAAAGCCAGTGTATGTAA
AGAAAATGTTCCAGCCAGGCGCGGTGGCTCACGCCTGTAATTCCATCATTTTGGGAGGCCACAGTG
GGCAGATTGCTTGAGCCCAGGAGTTGAAGAACGTGGCGAAACCCCGTATCTATTATTTAAAAAAAT
TGAAAAAGTAAAAA

(SEQ ID NO:68)

Homo sapiens AGTPBP1 3'-UTR
AGTPBP1-004 ENST00000357081

GCCCGCTGCCATCTCTTGTTAACTGCAAAGAATAAATGAAATATCTTGGTTTTTATTTCCCAGGAA
GCTTGAGAGAAATGAGTTTATACAGAGCTGACTCAAAAAGACAAAAAGTAACTTGGGCCAGTTTGG
TTTCAAGATAATAAATGTGTTATTAATTAATGATAAAATTGGCGCTTGTTTTATTTTCGATATTCA
ATGCACTTTATGTAGCATTGAATGATCAAATATTGGATTTACCTTTAAAAAAAAAACCTGAGTATC
ATTGCATGAATTTTTATCTCCCTATGGTTATATCCTGCATCAAGTGGATAATTTTGAAGTGTGTTC
AGAATATAAAATTGAAATTTTAGAGTTGTTGAAAATCCTGACTTGTTGAAAACTAATATATATGTA
CATGGATTTCTATAGATGTGTTTGTTTAGAAGTGGGTAGATATTGCAGATAAGACTGTTCTTCAGA
ATCATGTTAACTATTGGGTTGTGACTGAAGTAGTCCAGGGTTTGCCTTGAAACCATTACATTCTAC
ATTTACCAAATTAAACAAATAAAAACTGTATTAAATGTT

(SEQ ID NO:69)

Homo sapiens CCDC53 3'-UTR
CCDC53-001 ENST00000240079

GCTTAATTTTGATAAGAATTACATATGCATGCATAGGGGTACATTTACATTCTGTAAGAGATTGAG
CCTGAACTCTCTTAGTCATAAAAACATCAAATGGCCACATGTCCACTACCAAGCTTCTTCTATGTT
AAAAAAATAATAATAAAGCAGTTTTAACCTGCCAGTA

(SEQ ID NO:70)

Homo sapiens LRRC28 3'-UTR
LRRC28-002 ENST00000331450

TAAACACTCAAGAACCTCAGGAGCGCTGCCAGCTTGACACTGGGGAATCCAGCCAGTCCAGCACAC
TCTTCCATCCTGTCCTGTCCAATGCGGGGGCACTGCAGAACTCTCTAGAAATGTCATGATTGAGCT
TCAGAGCTAAAATGCCTTCACCCTTCCCCCAAGTTGGAATATATCCTCCCCCAAATTAAGGA

(SEQ ID NO:71)

Homo sapiens CCDC109B 3'-UTR
NM_017918.4

TCTTACAGTTTTAAATGTCGTCAGATTTTCCATTATGTATTGATTTTGCAACTTAGGATGTTTTTG
AGTCCCATGGTTCATTTTGATTGTTTAATCTTTGTTATTAAATTCTTGTAAAAC

(SEQ ID NO:72)

Homo sapiens PUS10 3'-UTR

PUS10-001 ENST00000316752

CTTTCAAATTTGGAGACAAAGAGTATGGTTTTCCTGGCATGATGTGGACATCCATGGAGCACATGC
CGTAAAATGGCTGTTTACCCACCATAACGGTGTCTTGAAAACTATTTGGATCATGTTGATCTATAT
AATTGTTAATTTGTTGTAACATCTCAGGATCTATATATGTGTATATTTTGTGTTAAATTGTTCCAA
GGATGTCTTAGGATTTTTCTCATTCCCTCTTTCACCCCCACAAACCAAACTATGAATAATGAAATA
ATTCTCCTTAATTCTTTCATTTAGAGAGGTGCACAAACAGGACACATTCTCTGTTAACCTAAGAAG
CTGTAATTTCAGCAAGATTTCCCTCCACAAGAGATATACCACCTTTAAAATCATGTTCTAATTTTT
GTAAATTATCTGAATAAAAGTTATATCTAG

(SEQ ID NO:73)

Homo sapiens CCDC104 3'-UTR
CCDC104-002 ENST00000339012

TAATTAAGAACAATTTAACAAAATGGAAGTTCAAATTGTCTTAAAAATAAATTATTTAGTCCTTAC
ACTGA

(SEQ ID NO:74)

Homo sapiens CASP1 3'-UTR
CASP1-007 ENST00000527979

AATAAGGAAACTGTATGAATGTCTGTGGGCAGGAAGTGAAGAGATCCTTCTGTAAAGGTTTTTGGA
ATTATGTCTGCTGAATAATAAACTTTTTTGAAATAATAAATCTGGTAGAAAAATGAAAA

(SEQ ID NO:75)

Homo sapiens SNX14 3'-UTR
SNX14-007 ENST00000513865

ACACTTGGATTTGGTATAGAATAACCCATTGAAATTTCTGCTGTGCGAGGGTGGTAGAAATTTACT
TTTTTGGGTATATTCTTATATATATTATGTACATCGCTGTCTGAAATTTAGTTATTTTTTGTTTT
TAATAAAGACTAACACAAACTTAATGATTAAAAGTGATTGAG

(SEQ ID NO:76)

Homo sapiens SKAP2 3'-UTR
SKAP2-201 (part of SKAP2.001 ENST00000345317)

GAGTCCTGGAAAAGGAAAATTCTTCTGCTTGTCTGCAAATGCTTTGGATTTAGAAGCGTCATGAAA
GCACGAGTGACAGCTCCTAACCTCTCCTTGTTTTATTAAACATTACTTATCTTTGACTGTTATTTT
ATGCAGTCGCTCATTAAAATATTCCTCTGATGTGAAATTAAATGAAGGATATTAATGTAAATTAGA
TGCAACCAGTTAAGTTATACCTGTTGCTATTTTGCAAAG

(SEQ ID NO:77)

Homo sapiens NDUFB6 3'-UTR
NM 182739.2

AGATTATGTAAAAAGTTAAAAGGCTTATGAGCCTAAGTTTGTTCCTATATTACCATATTTACTGAA
TTTTCTGGAAAAGTAACTTTAATAAAGTTTAATCTCAGAAATTGTCATATCTGTTTTCAAGCATTG
TACAATTTGAGACTGAGTAATTTAACAATAAGTAAAAAGTGGACATGCTAAACAAATATGAGAGAC
TACCTACTTTTTCTGGTCATTCTTGACTTGGAAAACGGTATGGAAAAGTATTTAGTTACATGTTTG
TTTGTTTTTTTCTTACACAGTACTTACACTAATTTGGTATCAGGGTATGCAACAGTGAAATATCAC
AATAAACAAATGTAAGAACAAAAAAAAAAAAA

(SEQ ID NO:78)

Homo sapiens EFHA1 3'-UTR
EFHA1-001 ENST00000382374

TAAAAGATATAATAGTATGGCAATTATATTGTTCCAAATGTCAAAATTTGTGATTTTTTAGAAGTA
CTTGCTATTTATCTTCTTAAGTCTTCATTGATATTCTGTGTGAAATAAGCATGTCTTGTACTTGCT
TTCTGATTCATAATTTTATTAAAGAACTTAGTAGAAAGAAAAGTAAGTATAAAAATAGATATTGGA
TTCTGTCAGAAGGCCTAGATTTGAAATAATGTTTTGTACTTCGGTAAGATGGAAAACTTAGTGATT
CACTGATTTCTTAGACACTCTAATATGATATGCTTTCTGGAAGGATAAAACAAATACATATGGGAA
AAAGTACTTGAGACCAAGGCCAGCATCAATTCCAGACATCTTCATGTTCCTAATAGGCTAAATGAA
GTTAAAAACTTATTTCAGATTTTTCTCATCTGTACCTTATATCTCATAAATTTATTGCATATTTTA
TGTCAGTAGCTTAGCTGTTTATTGTCTTTAAAATAACATGTAAACTTCAATGTTCTATCTGGAAGC
AGAATAAAATATTTACATAGA

(SEQ ID NO:79)

Homo sapiens BCKDHB 3'-UTR
BCKDHB-005 ENST00000356489

CCATATAGAAAAGCTGGAAGATTATGACTAGATATGGAAATATTTTTTCTGAATTTTTTTTTATAT
TTCCTCCGACTTACCTCTTTTTGAAAAGAGAGTTTTTATTAAGTGAACCATCACGATATTGGCTGA
AAAGTTCTACATTCTATTATTGTATTGTAACACACATGTATTGATGATTTTCATTAAGAGTTTCAG
ATTAACTTTGAAAAATATTCCACATGGTAATCTTATAAATTCTGTTTAATTACATCTGTAAATATT
ATGTGTGTGATAGTATTCAATAAA

(SEQ ID NO:80)

Homo sapiens BCKDHB 3'-UTR
NM_001164783.1

GACCTGCTCAGCCCACCCCCACCCATCCTCAGCTACCCCGAGAGGTAGCCCCACTCTAAGGGGAGC
AGGGGGACCTGACAGCACACCACTGTCTTCCCCAGTCAGCTCCCTCTAAAATACTCAGCGGCCAGG
GCGGCTGCCACTCTTCACCCCTGCTCCTCCCGGCTGTTACATTGTCAGGGGACAGCATCTGCAGCA
GTTGCTGAGGCTCCGTCAGCCCCCTCTTCACCTGTTGTTACAGTGCCTTCTCCCAGGGGCTGGGTG
AGGGCACATTCAGGACTAGAAGCCCCTCTGGGCATGGGGTGGACATGGCAGGTCAGCCTGTGGAAC
TTGCGCAGGTGCGAGTGGCCAGCAGAGGTCACGAATAAACTGCATCTCTGCGCCTGGCTCTCTACC
AAAAAAAAAAAAAAAAAA

(SEQ ID NO:81)

Homo sapiens BBS2 3'-UTR
NM 031885.3

GTGAGGAAAATACAGGTCATGAAGTTCCTGGCAAAGATTTTCTGTTAAAAACCTATGCTGGTTTGC
TTTGGATCACACCCTGGTGAACCCCGGGTGCTAAGAATGAAAATAACCTTGGTGAGTTGTACAAAT
TAAAGACAAAGAACTACATGTGAAGATAGACTTGCTTTCTATTTTTAAATCAGTAGTAGTACTGTT
GCTGAATAATACTAGGTTTTTATGGAATAGGATGAATGCTTTTGAAGTATTAGGGCTTCAGAGTCC
AATTTTGCTTATTTATGGTATATAAATACATATTTTTTTCTTGAAATTGCAATTGAGTTTGTACTT
TTCAAATAGATTATCTACTTTTTCATTAAAATGTAAAGATGTTAAACTTTGTGTTGATTGATTATA
AAATCACCACCAAATCAG

(SEQ ID NO:82)

Homo sapiens LMBRD1 3'UTR
NM 018368.3

CAGCCTTCTGTCTTAAAGGTTTTATAATGCTGACTGAATATCTGTTATGCATTTTTAAAGTATTAA
ACTAACATTAGGATTTGCTAACTAGCTTTCATCAAAAATGGGAGCATGGCTATAAGACAACTATAT
TTTATTATATGTTTTCTGAAGTAACATTGTATCATAGATTAACATTTTAAATTACCATAATCATGC
TATGTAAATATAAGACTACTGGCTTTGTGAGGGAATGTTTGTGCAAAATTTTTTCCTCTAATGTAT
AATAGTGTTAAATTGATTAAAAATCTTCCAGAATTAATATTCCCTTTTGTCACTTTTTGAAAACAT
AATAAATCATCTGTATCTGTGCCTTAGGTTCTCCAGAGTGATGTGGAATTTTAAAGTGTCTCTCTC
TGATTGCCTCCAA

(SEQ ID NO:83)

Homo sapiens ITGA6 3'-UTR
ITGA6-003 ENST00000409532

TATTGATCTACTTCTGTAATTGTGTGGATTCTTTAAACGCTCTAGGTACGATGACAGTGTTCCCCG
ATACCATGCTGTAAGGATCCGGAAAGAAGAGCGAGAGATCAAAGATGAAAAGTATATTGATAACCT
TGAAAAAAAACAGTGGATCACAAAGTGGAACGAAAATGAAAGCTACTCATAGCGGGGGCCTAAAAA
AAAAAAGCTTCACAGTACCCAAACTGCTTTTTCCAACTCAGAAATTCAATTTGGATTTAAAAGCCT
GCTCAATCCCTGAGGACTGATTTCAGAGTGACTACACACAGTACGAACCTACAGTTTTAACTGTGG
ATATTGTTACGTAGCCTAAGGCTCCTGTTTTGCACAGCCAAATTTAAAACTGTTGGAATGGATTTT
TCTTTAACTGCCGTAATTTAACTTTCTGGGTTGCCTTTATTTTTGGCGTGGCTGACTTACATCATG
TGTT

(SEQ ID NO:84)

Homo sapiens HERC5 3'-UTR
HERC5-001 ENST00000264350

CCAGCTTGCTTGTCCAACAGCCTTATTTTGTTGTTGTTATCGTTGTTGTTGTTGTTGTTGTTGTTG
TTTCTCTACTTTGTTTTGTTTTAGGCTTTTAGCAGCCTGAAGCCATGGTTTTTCATTTCTGTCTCT
AGTGATAAGCAGGAAAGAGGGATGAAGAAGAGGGTTTACTGGCCGGTTAGAACCCGTGACTGTATT

CTCTCCCTTGGATACCCCTATGCCTACATCATATTCCTTACCTCTTTTGGGAAATATTTTTCAAAA
ATAAAATAACCGAAAAATTAA

(SEQ ID NO:85)

Homo sapiens HADHB 3'-UTR
HADHB-001 ENST00000317799

TAGATCCAGAAGAAGTGACCTGAAGTTTCTGTGCAACACTCACACTAGGCAATGCCATTTCAATGC
ATTACTAAATGACATTTGTAGTTCCTAGCTCCTCTTAGGAAAACAGTTCTTGTGGCCTTCTATTAA
ATAGTTTGCACTTAAGCCTTGCCAGTGTTCTGAGCTTTTCAATAATCAGTTTACTGCTCTTTCAGG
GATTTCTAAGCCACCAGAATCTCACATGAGATGTGTGGGTGGTTGTTTTTGGTCTCTGTTGTCACT
AAAGACTAAATGAGGGTTTGCAGTTGGGAAAGAGGTCAACTGAGATTTGGAAATCATCTTTGTAAT
ATTTGCAAATTATACTTGTTCTTATCTGTGTCCTAAAGATGTGTTCTCTATAAAATACAAACCAAC
GTGCCTAATTAATTATGGAAAAATAATTCAGAATCTAAACACCACTGAAAACTTATAAAAAATGTT
TAGATACATAAATATGGTGGTCAGCGTTAATAAAGTGGAGAAATATTGGAGAA

(SEQ ID NO:86)

Homo sapiens ANAPC4 3'-UTR
ANAPC4-001 ENST00000315368

TCTAGCTTGCCATTATTGTGTGTGTAATTATGGCCAAAAGGACATAGGAGATGGACTAAGATGTCT
TGGACCACCTTTGTGTAACAAAGAAATAAACAGTAAATTTTATTTTTTCA

(SEQ ID NO:87)

Homo sapiens PCCB 3'-UTR
NM_000532.4

ACAAATCAAAGGAAAAGAAACCAAGAACTGAATTACTGTCTGCCCATTCACATCCCATTCCTGCCT
TTTGCAATCATGAAACCTGGGAATCCAAATAGTTGGATAACTTAGAATAACTAAGTTTATTAAATT
CTAGAAAGATCTCAAAAAAAAA

(SEQ ID NO:88)

Homo sapiens ABCB7 3'-UTR
ABCB7-001 ENST00000253577

GTCACATAAGACATTTTCTTTTTTTGTTGTTTTGGACTACATATTTGCACTGAAGCAGAATTGTTT
TATTAAAAAAATCATACATTCCCA

(SEQ ID NO:89)

Homo sapiens PGCP 3'-UTR
CPQ-001 ENST00000220763

AAACAGTAAGAAAGAAACGTTTTCATGCTTCTGGCCAGGAATCCTGGGTCTGCAACTTTGGAAAAC
TCCTCTTCACATAACAATTTCATCCAATTCATCTTCAAAGCACAACTCTATTTCATGCTTTCTGTT
ATTATCTTTCTTGATACTTTCCAAATTCTCTGATTCTAGAAAAAGGAATCATTCTCCCCTCCCTCC
CACCACATAGAATCAACATATGGTAGGGATTACAGTGGGGGCATTTCTTTATATCACCTCTTAAAA
ACATTGTTTCCACTTTAAAAGTAAACACTTAATAAATTTTTGGAAGATCTCTGA

(SEQ ID NO:90)

Homo sapiens NFU1 3'-UTR
NM 001002755.2

AATAATCTGGATTTTCTTTGGGCATAACAGTCAGACTTGTTGATAATATATATCAAGTTTTTATTA
TTAATATGCTGAGGAACTTGAAGATTAATAAAATATGCTCTTCAGAGAATGATATATAAATATTGC
A

(SEQ ID NO:91)

Homo sapiens OMA1 3'-UTR
OMA1-001 ENST00000371226

ATTAAAATTTATGAGACACAAGATATATGAAGAATGTTGCAGTCCTTATCATTTTATGTTACTTTT
TAAAAAATGATGTTTGAAGTGAAAAAAAAAAGGATATTCAGGGTCAAATCATGTACATTACAGATA
TTATCTAAATTCTTCTAGAATTTATTTTTCATGAAATATTGATGTATTTTAATCTATGTTAAAATA
TCTTCAATGAGGAAAATGTCACAGAATAAATTTATATTACACATTTTA

(SEQ ID NO:92)

Homo sapiens HHLA3 3'-UTR
NM 001036646.1

GGCGAATCCATAGAGTAAGCTTAGTGATGTGTGTCAGACCTCTGAGCCCAAGCAAAGCCATCATAT
CCCCTGTGACCTGCATGTATACATCCAGATGGCCTGAAGCAAGTGAAGAATCACAAAAGAAGTGAA
AAGGGCCGGTTCCTGCCTTAACTGATGACATTCCACCATTGTGATTTGTTCCTGCCCCACCTTAAC
TGAGCGATTAACCTGTGAACTTCCTTCTCCTGGCTCAGAAGCTTCCCCACTGAGCACCTTGTGACC
CCCGCCCCTGCCTGCCATAGAACAACCCCCTTTGATTGTAATTTTCCTTTACCTACCCAAATCCTA
TAAAACGGCCCCACCCCTATCTCCCTTCGCTGACACTCTCTTTGGACTCAGCCTGCCTGCACCTAG
GTGATTAAAAAGCTTTATTGCTCACGC

(SEQ ID NO:93)

Homo sapiens HHLA3 3'-UTR
NM 001031693.2

AAAGGGCCGGTTCCTGCCTTAACTGATGACATTCCACCATTGTGATTTGTTCCTGCCCCACCTTAA
CTGAGCGATTAACCTGTGAACTTCCTTCTCCTGGCTCAGAAGCTTCCCCACTGAGCACCTTGTGAC
CCCCGCCCCTGCCTGCCATAGAACAACCCCCTTTGATTGTAATTTTCCTTTACCTACCCAAATCCT
ATAAAACGGCCCCACCCCTATCTCCCTTCGCTGACACTCTCTTTGGACTCAGCCTGCCTGCACCTA
GGTGATTAAAAAGCTTTATTGCTCACGC

(SEQ ID NO:94)

Homo sapiens ACAA2 3'-UTR NM 006111.2

AGAGACCAGTGAGCTCACTGTGACCCATCCTTACTCTACTTGGCCAGGCCACAGTAAAACAAGTGA
CCTTCAGAGCAGCTGCCACAACTGGCCATGCCCTGCCATTGAAACAGTGATTAAGTTTGATCAAGC
CATGGTGACACAAAAATGCATTGATCATGAATAGGAGCCCATGCTAGAAGTACATTCTCTCAGATT
TGAACCAGTGAAATATGATGTATTTCTGAGCTAAAACTCAACTATAGAAGACATTAAAAGAAATCG
TATTCTTGCCAAGTAACCACCACTTCTGCCTTAGATAATATGATTATAAGGAAATCAAATAAATGT
TGCCTTAACTTC

(SEQ ID NO:95)

Homo sapiens GSTM4 3'-UTR
GSTM4-001 ENST00000369836

TGCCTTGAAGGCCAGGAGGTGGGAGTGAGGAGCCCATACTCAGCCTGCTGCCCAGGCTGTGCAGCG
CAGCTGGACTCTGCATCCCAGCACCTGCCTCCTCGTTCCTTTCTCCTGTTTATTCCCATCTTTACC
CCCAAGACTTTATTGGGCCTCTTCACTTCCCCTAAACCCCTGTCCCATGCAGGCCCTTTGAAGCCT
CAGCTACCCACTTTCCTTCATGAACATCCCCCTCCCAACACTACCCTTCCCTGCACTAAAGCCAGC
CTGACCTTCCTTCCTGTTAGTGGTTGTATCTGCTTTGAAGGGCCTACCTGGCCCCTCGCCTGTGGA
GCTCAGCCCTGAGCTGTCCCCGTGTTGCATGACAGCATTGACTGGTTTACAGGCCCTGCTCCTGCA
GCATGGCCCCTGCCTTAGGCCTACCTGATCAAAATAAAGCCTCAGCCACA

(SEQ ID NO:96)

Homo sapiens GSTM4 3'-UTR
GSTM4-003 ENST00000326729

TGGTCAATTTTCTGCATCAACTTGACTGGGCTAAGGGATGCTCAGATGGCAGGTAAAATCATTGTG
CTTGTGAGGGTGTTTCCAGAAGAGATTTGCCTTTGAATCAGAAGACAGCAAAGATTTCCTTCAGCA

ATGAAGGAGGCATCCACCAAACTGTCAGGGCCCAGAGAGAAGAAAAAGACAGGAAGGGTGAATTTG
ACCTCTCTGACTGGGACATCCATCTCTGCCTATCCTGGGACCTCCACACTCCTGGTTCTCTGGCCT
TCAGACTTGATCAGGGACTAACACCATCGCCTCCCACCCCCACCTTTGTTCTGAGGCCTTTAGCCT
CTGAATGATACCACTGGCTTTCCTGCTTCTCTATCCTGCAGTCGGCAGATCATGGGACTTCTTCAC
TCCAAAATTGTGTGAGCCAATTCCCATAACAGATAGATAAATTTATAAATAAACACACAAATTTCC
TAC

(SEQ ID NO:97)

Homo sapiens ALG8 3'-UTR
NM_001007027.2

CTGAAACCTCCGCCTCCCAGAAAAGAAAAACCTCTTTTTAATTGGATGGAAACTTTCTACCTGCTT
GGCCTGGGGCCTCTGGAAGTCTGCTGTGAATTTGTATTCCCTTTCACCTCCTGGAAGGTGAAGTAC
CCCTTCATCCCTTTGTTACTAACCTCAGTGTATTGTGCAGTAGGCATCACATATGCTTGGTTCAAA
CTGTATGTTTCAGTATTGATTGACTCTGCTATTGGCAAGACAAAGAAACAATGAATAAAGGAACTG
CTTAGATATG

(SEQ ID NO:98)

Homo sapiens C11orf74 3'UTR

TTCACAGAGGCATTTTGTGTGTGTGTGCTTATTTTAATTTTGTTCTTATTCTAGCAACATTAGAAT
AAAAGATAAACCTACTATAATTCCCTTTGTGGAAATTTAAAAAAAAAAAAAAAAA

(SEQ ID NO:99)

Mus musculus Ndufa1 3'-UTR
Ndufa1-001 ENSMUST00000016571

GGAAGCATTTTCCTGGCTGATTAAAAGAAATTACTCAGCTATGGTCATCTGTTCCTGTTAGAAGGC
TATGCAGCATATTATATACTATGCGCATGTTATGAAATGCATAATAAAAAATTTTAAAAAATCTAA
A

(SEQ ID NO:100)

Mus musculus Atp5e 3'-UTR
NM 025983

CTGAATCTGAAGCCTGAAGTGCTGAGTCTTGAAGGTGAAGCATGTGGGCCCCTGTTCTGGCAGATG
GAAATCAACCTCACCTCCTGGGGGACAGGCTGCCCATCTCGTTGATAAATTGACTATGCCAATAAA
TTAACATGGTTCACTTTCAAAAA

(SEQ ID NO:101)

Mus musculus Gstm5 3'-UTR
NM 010360

GCCAGAGCTCGCTGCTGCTGAGCCATCTTGCCCTGAGGGGCCCACACTCTTAGCTCACTGTCAGTC
TTGTTCCATCCTGTCCTGAGGGCCCCCACTCTGTCTCCTCTGCTCTTTCTAATAAACAGCAGTTGC
ATTA

(SEQ ID NO:102)

Mus musculus Uqcr11 3'-UTR
NM_025650

GCAGCCCCTCCCCCACCACAGGCCTCGATGGTACCATGTGCCGAGGCCTCAGACACAGCGTAGTCC
TGTGGAAGACACTGAGGAAGCTGGACACTGGAGAGGTCTGCACCGCTCAGGGAGCTTCCATGTTGA
CAGACACTAGGGCTGCCTTGATGGGTGCAGCATTAAACCTTATTCTTATGCCTTGGA

(SEQ ID NO:103)

Mus musculus IFi27l2a 3'-UTR
IFi27l2a-001 ENSMUST00000055071; NM_029803

GCTTAGGAGATGACACTTCTATCAGCTCAACTCAAAGCCTGTACAGACTACGCAGGAGATGAAGTT
CCAAAAGGCACCTTCAGAACCCTCACTGATGTCAAAGAATGATGAAAACAACAAAGTATATGGGCT
GGTGTTCCTAA

(SEQ ID NO:104)

Mus musculus Cbr2 3'-UTR
NM 007621

TCTGCTCAGTTGCCGCGGACATCTGAGTGGCCTTCTTAGCCCCACCCTCAGCCAAAGCATTTACTG
ATCTCGTGACTCCGCCCTCATGCTACAGCCACGCCCACCACGCAGCTCACAGTTCCACCCCCATGT
TACTGTCGATCCCACAACCACTCCAGGCGCAGACCTTGTTCTCTTTGTCCACTTTGTTGGGCTCAT
TTGCCTAAATAAACGGGCCACCGCGTTACCTTTAACTAT

(SEQ ID NO:105)

Mus musculus Atp51 3'-UTR
Atp51-201 ENSMUST00000043675

AGACCAATCTTTAACTTCTGATTTGAGTTCTTATTTGAATGTTCTTGGACCATGTGTAACAGGACT
GCTATCTGAATAAAATACTAGGTGTTGAAAACACTGCTGTGTTTTCTCTGTC

(SEQ ID NO:106)

Mus musculus Tmsb10 3'-UTR
NM_025284

AAGCCTAGGAAGATTTCCCCACCCCACCCCACCCCGCCCCATCATCTCCAAGACCCCCTCGTGATG
TGGAGGAAGAGCCACCTGCAAGATGGACGCGAGCCACAAGCTGCACTGTGAAACCCGGGCACTCCG
AGCCGATGCCACCGGCCCGCGGGTCTCTGAAGGGGACCCCTCCACTAATCGGACTGCCAAATTTCA
CCGGTTTGCCCTGGGATATTATAGAAAATTATTTGTATGATTGATGAAAATAAAAACACCTCGTGG
CATGGTT

(SEQ ID NO:107)

Mus musculus Nenf 3'-UTR
NM 025424

TGTCTAGCTGAGAAGCAGCCGGTTCTAGGGAGAAGTGAGGGGACAGGAGTTAAGTGTCCCTCGGAA
CAAGCGGAGGAAGCCTCCGAGTGCCCTGCAGCTGAATAAAGCGAATGTTT

(SEQ ID NO:108)

Mus musculus Atp5k 3'-UTR
NM 007507

GGCGTCAGCGAGCTTGCTTTTCTCTAGTCGTTGAGAACGAATAAAGCTTCATTGTGTGAAAAAAAA
AAAAAAAAAAAAAAAAAAAAAAAAAAAAAAAAAAAAAAAAAAAAAAAAAAAAAAAAA

(SEQ ID NO:109)

Mus musculus 1110008P14Rik 3'-UTR
1110008P14Rik-001 ENSMUST00000048792

GTGCCGGGAGCCCCCATCCAGGCCCTACCCTCACCTCTCTAGGCCATGTTCTGGCCTGGGTAGATA
CTACTTGGCTTAGACACCATCTCGGGTACTGGCCTCCAGATCCTAGTGGGTCTACCAGCCTGGACC
AGTCCCCATTCACTGCCCATCACCCTTCCTGGAGTCAGGTGCAATCCTACAGTTCTCCCACTTGTC
TGTCTTCTTTCCCCTCCATCCAGACTGAGAGTCCGAATTAAAGATGTCTCCCACACCACTGC

(SEQ ID NO:110)

Mus musculus Cox4i1 3'-UTR
NM_009941

GAGCCCGCTGCCTGCCGGCTCCCTGCCTCCCTCACTCCCTCGGCATGCTGGAAGCTGCCGTATCCA
ATGGTCCATGCTAATAAAAGACCAGTTTACGTGGTG

(SEQ ID NO:111)

Mus musculus Cox6a1 3'-UTR
NM 007748

AGAGAACCTGGCCTCCCCCAGGCAACAAAGGGACCACAGCACTGGTTTTGGACCCTTACTCTGTGT
GGACCACGAAAACCCTTTGGATGCTAAGCTCGTGTCTCCTTTCCTCAGATGGCGACCATTACTCTG
ATCTTCCATCCCTTCTGCTTGTAAGAGGAGATGCCTTAAATAAATAACTTAAACTCA

(SEQ ID NO:112)

Mus musculus Ndufs6 3'-UTR
NM 010888

TGTGGGCTGTGTCCTGGTCCTCTGACTCCTATGGAACATCTCCACGCTGGGTGTTCTGTGTGAGGC
CACTGCTCTGTGAATGGTGTCCCTTGTTTTGAATAAAGGATGCTCCCACCATGAAAAAAAAAAAAA
AAAAAAA

(SEQ ID NO:113)

Mus musculus Sec61b 3'-UTR
NM 024171

ATTGGGCTACATCCATCTGTCATCTGAAGAAGAAGAAGAAGGAAAAAAACCCAACATATCTTGGAC
CAAAAGTGTAGTGATTTTCTGTTCACGTGTATTATTTTACAGAGAATAAGAATTGACTTTGAGAAA
TCAGTTTTTTCTATGGCTAATAAACTTTGGAATTGCTTT

(SEQ ID NO:114)

Mus musculus Romo1 3'-UTR
NM 025946

TTAGGGCTAGGATGCCCTGCAATACCTAAACTTCCCCATCCATTTCGACCCTTGTACAATAATAAA
GTTGTTTTCTTCTCGTTAAAAAAAAAAAAAAAAAA

(SEQ ID NO:115)

Mus musculus Gnas 3'-UTR
NM 010309

GAAGGGAACACCCAAATTTAATTCAGCCTTAAGCACAATTAATTAAGAGTGAAACGTAATTGTACA
AGCAGTTGGTCACCCACCATAGGGCATGATCAACACCGCAACCTTTCCTTTTTCCCCCAGTGATTC
TGAAAAACCCCTCTTCCCTTCAGCTTGCTTAGATGTTCCAAATTTAGTAAGCTTAAGGCGGCCTAC
AGAAGAAAAAGAAAAAAAAGGCCACAAAAGTTCCCTCTCACTTTCAGTAAATAAAATAAAAGCAGC
AACAGAAATAAAGAAATAAATGAAATTCAAAATGAAATAAATATTGTGTTGTGCAGCATTAAAAAA
TCAATAAAAATTAAAAATGAGCAAAAAAAAAAAAAAAAAA

(SEQ ID NO:116)

Mus musculus Snrpd2 3'-UTR
NM 026943

AGCCTGCTCCCTGCCCTGCGAAGGCCTGCAGAACCCTGCCCAGTGGGCGAGAAATAAAACCCTGTG
CTTTTTGGTTAAAAAAAAAAAAAAAAAAAA

(SEQ ID NO:117)

Mus musculus Mgst3 3'-UTR
NM 025569

GGTGTGGAGGGCCTTCCGACTCTCACTCACCTCCAGCGACTCACCCTGATTTCCAGTTGCACTGGT
TTTTTTTTTTTTTTAATATAATAAAAACTTATCTGGCATCAGCCTCATACCT

(SEQ ID NO:118)

Mus musculus Aldh2 3'-UTR
NM_009656

AGCGGCATGCCTGCTTCCTCAGCCCGCACCCGAAAACCCAACAAGATATACTGAGAAAAACCGCCA
CACACACTGCGCCTCCAAAGAGAAACCCCTTCACCAAAGTGTCTTGGGTCAAGAAAGAATTTTATA
AACAGGGCGGGGCTGGTGGGGGGGAAAGCTCCTGATAAACTGGGTAGGGGATGAAGCTCAATGCAG
ACCGATCACGCGTCCAGATGTGCAGGATGCTGCCTTCAACCTGCAGTCCCTAAGCAGCAAATGAGC
AATAAAAATCAGCAGATCAAAGCCACGGGGTCAGTTCTCT

(SEQ ID NO:119)

Mus musculus Mp68 (2010107E04Rik) 3'-UTR
NM_027360

CTGCTCCGAATCCACAAGATGAAGACGTCGGCTAAACTTGAGCAAGCTTTGTTAGATGGGAACATG
GAACATCACTGTACACTTATCTAAGTACCATTTATAATGTGGCATTAATAAATGTATCTGTGAATA
CC

(SEQ ID NO:120)

Mus musculus Ssr4 3'-UTR
NM 001166480

GGGCAGCAACTTCAGCCGTCCATTGCTTCTTTCAATAAACAGTCACTATTTGACATGAGTACATTC
AAGAAAAAAAAAAAAAAAAAA

(SEQ ID NO:121)

Mus musculus Myl6 3'-UTR
NM_010860

GGACATTCTGTATCCCGAGTCTGTTCCTTGCCCAGTGTGATTTCTGTGTGGCTCCAGAGGCTCCCC
TGTCACAGCACCTTGCCCATTTGGTTTCTTTTGGATGATGTTTGCCTTCCCCAAATAAAATTTGCT
CTCTTTGCCCTCCAAAAAAAAAAAAAAAAAAAAAAAAAAAAAAAAAAAAAAAA

(SEQ ID NO:122)

Mus musculus Prdx4 3'-UTR
Prdx4-001, NM_016764
AAAGTACTTCAGTTATGATGTTTGGACCTTCTCAATAAAGGTCATTGTGTTATTACCA
(SEQ ID NO:123)

Mus musculus Ub15 3'-UTR
NM 025401

AGGGGGATTCCTTCTCCTCCTCGCCCTGCTCTGCCCTGCCCTCCTCTCCCATCCTCATCTGACACT
GGTGTAGATGGTCATTTTTAACAGTTCACATGAATAAAAACTTGGCTGCTGCTTTGCTGCTGTC

(SEQ ID NO:124)

Mus musculus 1110001J03Rik 3'-UTR
NM 025363

TGCAGAGAGTCCTCAGATGTTCCTTCATTCAAGAGTTTAACCATTTCTAACAATATGTAGTTATCA
TTAAATCTTTTTTAAAGTGTG

(SEQ ID NO:125)

Mus musculus Ndufa13 3'-UTR
Ndufa13-201 ENSMUST00000110167
GGCCTGAGCCAACGCACATAATAAAGAGTGGTC
(SEQ ID NO:126)

Mus musculus Ndufa3 3'-UTR
NM_025348

ATGCCTCTGCTGATGGAAGAGGCCCCTTCCCTGTTGCTCTCCAATAAAAATGTGAAAACTAATAAC
CCC

(SEQ ID NO:127)

Mus musculus Gstp2 3'-UTR
NM 181796

TGGACTGAAGAGACAAGAGCTTCTTGTCCCCGTTTTCCCAGCACTAATAAAGTTTGTAAGACAAAA
AAAAAAAAAAAAAAAAAAAAAAAAAAAAAA

(SEQ ID NO:128)

Mus musculus Tmem160 3'-UTR
NM 026938

ACAACAGGGCTGTGGGGACTGGCTGGGCCTGACGACTGGGACATTAAAACCTGACCCTTCCGCAAA
AAAAAAAAAAAAAAAAAAAAAAAA

(SEQ ID NO:129)

Mus musculus Ergic3 3'-UTR
NM 025516

CTCTCTCCCTTCCCCACAGCTTGTCCTGCCCTCTCTTCCCCTGTGGGTTTACCCTCCAGCCTGTCA
ACTACCCATATCCTCTCCTCAGCCAGCCCAGCCCAGGGCAATAAATATGAATTGTGATAGGAA

(SEQ ID NO:130)

Mus musculus Pgcp 3'-UTR
NM_018755

GGAGAACAAGAAGAGAGGACCTTGTTCTCTGTAGTTGGGAATCCCAACTCTGAATCTTTACAACAT
CCATCGTCACAAAAGAGTGTTATACATTTAATCCACAGGGCATAGTTTTCTTTATACCTTCTGTTA
ATCATCTTTCCTTAATACTTTCTTATCTGTTTCTAGAATAAATCATGATCCCTACTGCACCACCTT
GAAAATGTTGTTTCCAGTTTTAAAATAAGCAATAAATATTTGAAATGCTTCTGATTTTTCATTTTC
ATTTAAAAACATTAAATTAAATGTAATGAGA

(SEQ ID NO:131)

Mus musculus Slpi 3'-UTR
NM_011414

GCCTGATCCCTGACATTGGCGCCGGCTCTGGACTCGTGCTCGGTGTGCTCTGGAAACTACTTCCCT
GCTCCCAGGCGTCCCTGCTCCGGGTTCCATGGCTCCCGGCTCCCTGTATCCCAGGCTTGGATCCTG
TGGACCAGGGTTACTGTTTTACCACTAACATCTCCTTTTGGCTCAGCATTCACCGATCTTTAGGGA
AATGCTGTTGGAGAGCAAATAAATAAACGCATTCATTTCTCTATGCAAAAAAAAAAAAAAAAAAA

(SEQ ID NO:132)

Mus musculus Myeov2 3'-UTR
NM 001163425

GGCCGCCCGGTCCTATGTGCTCCATGTCTGTGATGTGTCTGGAGTCTCTCGGGACACGACCAGCTG
ATTGTAGACACCGTGTTGATATCACTAGAAATGAAGACCTTGTCAACCAATAGAGGAACTGTCTGA
ACCAACTGGGTACTGATGTCTCTGGGAATGCCAGCCCGTGTCCTTGTTTAAGTTAATAAAGAACAC
TGTAACACGCAGGGTGATTTTAAAAAAAAAAAAAAAAAAAAAA

(SEQ ID NO:133)

Mus musculus Ndufa4 3'-UTR
NM_010886

ACTATGAAGTTCACTGTAAAGCTGCTGATAATGAAGGTCTTTCAGAAGCCATCCGCACAATTTTCC
ACTTAAGCAGGAAATATGTCTCTGAATGCATGAAATCATGTTGATTTTTTTTTTTTTGGAGTTTA
TTACACTGATGAATAAATCTCTGAAACTTG

(SEQ ID NO:134)

Mus musculus Ndufs5 3'-UTR
NM 001030274

GCGGGGCAGCTGGAGGCCGCTGTCATGCTCTGTTTTCCCCTGGAGAGAATATTTAAGGAAAGCTCC
TTCATTAAGTATTAAGTATGTGGAAATAAAGAATTACTCAGTCTTAAAAAAAAAAAAAAAAAAAA
AAAAAAAAAAA

(SEQ ID NO:135)

Mus musculus Gstm1 3'-UTR
NM 010358

GCCCTTGCTACACGGGCACTCACTAGGAGGACCTGTCCACACTGGGGATCCTGCAGGCCCTGGGTG
GGGACAGCACCCTGGCCTTCTGCACTGTGGCTCCTGGTTCTCTCTCCTTCCCGCTCCCTTCTGCAG
CTTGGTCAGCCCCATCTCCTCACCCTCTTCCCAGTCAAGTCCACACAGCCTTCATTCTCCCCAGTT
TCTTTCACATGGCCCCTTCTTCATTGGCTCCCTGACCCAACCTCACAGCCCGTTTCTGCGAACTGA
GGTCTGTCCTGAACTCACGCTTCCTAGAATTACCCCGATGGTCAACACTATCTTAGTGCTAGCCCT
CCCTAGAGTTACCCCGAAGGTCAATACTTGAGTGCCAGCCTGTTCCTGGTGGAGTAGCCTCCCCAG
GTCTGTCTCGTCTACAATAAAGTCTGAAACACACTTGCCATGAAAAAAAAAAAAAAAAA

(SEQ ID NO:136)

Mus musculus 1810027010Rik 3'-UTR
1810027010Rik-001 ENSMUST00000094065

AGTCTCTTGTTTAAGCGCCCAGTCCTGGCCTTTCTGGGTAATTGGGCGCAGAGGGAAGGAGCCAAT
GTTGAAGCAGAAAAGAAATTAAAAGAAAAAGGCATATAAAGAA

(SEQ ID NO:137)

Mus musculus 1810027010Rik 3'-UTR
BC117077
AGTCTCTTGTTTAAGCGCCCAGTCCTGGCCTTTCTGGGTAATTGGGCGC
(SEQ ID NO:138)

Mus musculus Atp5o 3'-UTR
NM_138597
GAGACTGTCACCTGTGTGAGCTCTTGTCCTTGGAGCAACAATAAAATGCTTCCTG
(SEQ ID NO:139)

Mus musculus Shfm1 3'-UTR
NM 009169

CATCTGGGAATGTCCCAGGAACCTCAATCATGGACTCTACCACAGTCTAGGACAGAGAAAGCAGGA
CGGGATACTTTAAAGAACATGTTTATTTCATTATCTGCTTCAATTTATTTTTGTTTTATAACAAAA
AAAATAAGTAAATAAATGTTTTGATTTAATCTTTTTGGTTCA

(SEQ ID NO:140)

Mus musculus Tspo 3'-UTR
NM_009775

AGGCACCCAGCCATCAGGAATGCAGCCCTGCCAGCCAGGCACCATGGGTGGCAGCCATCATGCTTT
TATGACCATTGGGCCTGCTGGTCTACCTGGTCTTAGCCCAGGAAGCCACCAGGTAGGTTAGGGTGG
TCAGTGCCGAGTCTCCTGCAGACACAGTTATACCTGCCTTTCTGCACTGCTCCAGGCATGCCCTTA
GAGCATGGTGTTTTAAAGCTAAATAAAGTCTCTAACTTCATGTGTAAAAAAAAAAAAAAAAA

(SEQ ID NO:141)

Mus musculus S100a6 3'-UTR
NM_011313

AATGGGACCGTTGAGATGACTTCCGGGGGCCTCTCTCGGTCAAATCCAGTGGTGGGTAGTTATACA
ATAAATATTTCGTTTTTGTTATGCCT

(SEQ ID NO:142)

Mus musculus Taldo1 3'-UTR
NM 011528

TGCAACACCCGAGGCCCCAGTCCTGCACCGAGGCTGACCCCAGACCTGCACTGCCTTTGAGCTGGG
TCCTAATTGCACATGGCTTGTGACGAATGAATCTTGCATTTTTTAGTGATCGGAGAAGGGATGGAT
CATAGGATTCTGATTTTATGTGAAATTTTGTCTAATTCATTAAAGCAGTTGCTTTTCCTATGCTGT
TT

(SEQ ID NO:143)

Mus musculus Bloc1s1 3'-UTR
NM 015740

ACTAAAACCCACCCCTCTTACTTCACCCTCCTGGACAGGAGGGAAACTGGTGAGCCACGAATAAAA
ACACAAGCTTCCATTCT

(SEQ ID NO:144)

Mus musculus Ndufb11 3'-UTR
NM 019435

TGGCTTACCGAGCAGGGCCTAAGAAGCATTACTCATCCGCTGCTTGTTATTTACCTGGTTCCTCAG
AACACCTTATTAAAGGAATTGAAAGTA

(SEQ ID NO:145)

Mus musculus Map11c3a 3'-UTR
NM 025735

GTCAAGAGGAGGGGAGGGGGGTGGCTGGGAGTTCTGGTCAGGTTCTCCCCAGGGAGGTCCTGGCTC
CTAAACTAAGCTATTTCAGTCCCCAGTGGATTAGGCAGAGATGTGACACCCACTCCCCCCCCCAGG
TAGGGGCCACCAGCCAGCCTACCACATCCTGGGTAGGTCCTGGGCCAGTCATGTTCGGGTTGCTCT
TTTGGGTGCTGGCTGGGTTGGGAGTGGGTGGGGAGCAGCATCCCTGCTCTGTGGGGTTTGTCATTT
TGTTAGGCCCTTGCCTGTCTGCCCATCTTGCCCCTCATCCACCTGAGGCTTTGCCTCCTGCCAGGA
CCTGCCCCACCCCTGAAAGGCTGGCTCCCCTTGTCCTGACTCGGTGTATGGATCTGTGGTCATTTC
CTCTGCAGAAAGAATAAAGACTGCTCAGGCCTGCCTGGCCAAAAAAAAAAAAAAAAA

(SEQ ID NO:146)

Mus musculus Morn2 3'-UTR
NM_194269

ACCTGCTGCCTTAACGCTGAGATGTGGCCTCTGCAACCCCCCTTAGGCAAAGCAACTGAACCTTCT
GCTAAAGTGACCTGCCCTCTTCCGTAAGTCCAATAAAGTTGTCATGCACCCACAAAAAAAAAAAAA
AAA

(SEQ ID NO:147)

Mus musculus Gpx4 3'-UTR
NM_008162.2

CTAGCCCTACAAGTGTGTGCCCCTACACCGAGCCCCCCTGCCCTGTGACCCCTGGAGCCTTCCACC
CCGGCACTCATGAAGGTCTGCCTGAAAACCAGCCTGCTGGTGGGGCAGTCCTGAGGACCTGGCGTG
CATCCCTGCCGGAGGAAGGTCCAGAGGCCTGTGGCCCTGGGCTCGAGCTTCACCCTGGCTGCCTTG
TGGGAATAAAATGTAGAAATGTGAAAAAAAAAAAAAAAAA

(SEQ ID NO:148)

Mus musculus Mif 3'-UTR
NM 010798.2

GTCCTGGCCCCACTTACCTGCACCGCTGTTCTTTGAGCCTCGCTCCACGTAGTGTTCTGTGTTTAT
CCACCGGTAGCGATGCCCACCTTCCAGCCGGGAGAAATAAATGGTTTATAAGAGACCA

(SEQ ID NO:149)

Mus musculus Cox6b1 3'-UTR
NM_025628

CCTGGCTCCGCCCACCTCTCCTCTGTTCTTTGTCTTTCTCCCCGGATAGAAAAGGGGGACCTCAGC
ATATGATGGTCCTTACCCTGGGACCCTGAATCATGATGCAACTACTAATAAAAACTCACTGGAAAA
GTT

(SEQ ID NO:150)

Mus musculus RIKEN cDNA2900010J23 (Swi5) 3'-UTR
NM 175190

GCAGCTTCTTGGAGATTTTCATCTACAGCCCACAGGGACAGGAGGATGGGGGCATAAAAGGCAGAG
TCTAGACAGTATGTTCATATGGTTTTCAGATTTTAAAAGATGCTAGAAGCCCTCCAAAGTTTGGGG
TGGGTTCTAGAGAAGAGGAGTATTGGGAGGGGTGGGTATTGTCAATGTTAAGGTTCCTAAACATAC
TTGTGAGTAGGTGTGTGTGGTTGTCCCTTTTGTTAATAAACATATGAGCAGTCAAAAAAAAAAAAA
AAA

(SEQ ID NO:151)

Mus musculus Sec61g 3'-UTR
NM 011343.3

GTCCTTCTCATCATGGGACGAGTGAGCCAGAGCGGGGGAAAGGGCATGAAGTAAAGCGTTGCCTGA
ATGCTGTGTGGTGTTTTGTTTCTTCCTCCTTCCTATGAGGTTTTCTACTTCTCAATTAAAATAATT
TCAAAATAAACACTTTTTCCATAACAGA

(SEQ ID NO:152)

Mus musculus 2900010M23Rik 3'-UTR
BC_030629
CCGTGGGGTCTGATACTCATCAATAAAACTGCCTGGTTTCTCCCACAAAAAAAAAAAAAAA
(SEQ ID NO:153)

Mus musculus Anapc5 3'-UTR
Anapc5-201 ENSMUST00000086216

CCAGGACTCCCTGCTTGATGGTGTGCATTTAGGGGTGGGTCATTACATGCTATCTTGTCAATAAAC
TGTTCTGATCAGTTTGTCTGAAGTGGGTTTTTTTTTATTTTTCTGGGTTGAATTGTCAGTATCTTT
GTTAAGAACTGTGTATCTAGGGGCTGGAGAGATGGCTTAGCAGTTAAGAGCACTAACTGTTCTTCT
AAAGGACCTGGGTTCAATTCCTAGCACCCTCATGACAGCTCACAGCTGTCTGTAACTCCTGTTCCA
GGGACTCTGACACCCTCAGGCAGACATAAAAGCAGTCAAAACACCGATGTACATAAAATTAAAATA
AATTATTT

(SEQ ID NO:154)

Mus musculus Mars2 3'-UTR
BC132343.1

GAACTCAGCTCTTACTGACTGGTAGTAAAAGATCAAATGTATTCTTTTTGCGTTTTTAAGTAAAGT
CATGC

(SEQ ID NO:155)

Mus musculus Phpt1 3'-UTR
NM_029293

AGCTCTGCCCCACCCCCCACCCCCCGGACTAAGTCAGGTCTCTGCTCTTGCTGTGTTCTGTTTTGA

GGGGCTGGCCCTGTGCTTTCCTTTTGTACCTTAGGCAGCATAGCACCTGCCAGGCCTTAGAGGCCA
GACCAATCTGGTCCATAGGAATTAAAAGCATTGATATGCCTACT

(SEQ ID NO:156)

Mus musculus Ndufb8 3'-UTR
NM_026061
GGAGGCTTGATGGGCTTTTTGCCCTCGTTCCTAGAGGCTTAACCATAATAAAATCCCTAATAAAGC
(SEQ ID NO:157)

Mus musculus Pfdn5 3'-UTR
NM 027044

GAGTGCACTGCAGAAATGAAGCAGAGTGAGGGACCCTTCTTCAAGGGGCCTGGACTTTTTCCGGC
AATGGCCTCCTGGGAAAGTGGCCTGGGAAGAGAGTGTTTTGTGTTTAATGTTAATAAATGTGACCG
CTGCGCAAAAAAAAAAAAAAAAAAAAAAAAAAAAAAAA

(SEQ ID NO:158)

Mus musculus Arpc3 3'-UTR
NM_019824

GAGGAGCCTGGGCAGCACCATCACGTGGAGACACATCATAGGACACACAGGCCAATGTGTCTGTTC
ATACCTACCGTATCAAGGAGAGAAGAGAGCCTGTCTTTGCTGGAAAAGCTCTTGGTCAAGAATTGG
GAGGGTGGGTGTTGGGCGATTTCGATTTTTGGCAGTTTTAAGCTGGTACTTAATATATAATAAATG
TCACTGCTTATGTTAGACATTGAATTAAAACATTTTTGAGAAAAAGCTTTAAAAAAAAAAAAAAAA
AAAAAAAAAAAAAA

(SEQ ID NO:159)

Mus musculus Ndufb7 3'-UTR
NM_025843
GGATTACCCGCCAGCCTGTGGACCTATCAGTGAAATAAAAGCTTTGGGTCACCTGCCT
(SEQ ID NO:160)

Mus musculus Atp5h 3'-UTR
NM_027862
AGCAGCCTGGGACGGAGCCCCGGCCGACATGAAATAAAACATTTAAATAGT
(SEQ ID NO:161)

Mus musculus Mrp123 3'-UTR
NM 011288

CCTATGACAGCAGGATTTGGACCACAGACCCTAGTGAGCACAGTGGTTCTGACAAGCCCAAATAAA
AATTCTTTGTGGAG

(SEQ ID NO:162)

Mus musculus Tomm6 3'-UTR
NM_025365.3

CCAGAGAATGGAACTCCTGTGTATTCAGACTTTCCAAAGACAGCCTACTGTCTGTGACCACAAGAT
CCTACCTGAGTGGCAGCTGAAGTTGACTCCCTCTCCTTGCCTGAACCCCCCCCCACTGCCCCCCCA
TCCCCCAGTGTCGGCTGAGATGTTGCCTCTGCACGGTTCTGTGTGCAGTTCCCAACTTTCTGCAGA
AGATGGTCCTTGCCCTTGTCCTGAAGAGTAGTAATGGTTCTTGAAAAAGATTTCAAATAAAGCCTG
CACATAAAAGACAGGTATTTTATTCTTTTAATAAGAAACTTATTACAAAAACAAGGTGTAAAAAGT
CCGCTTACAAAAATCAAATAAACATGACTTGTATTTCAAAAAAAAAAAAAAAAAAA

(SEQ ID NO:163)

Mus musculus Tomm6 3'-UTR
Tomm6-002 ENSMUST00000113301

CCAGGTGAGAGCAGTTCTCCTGTGTTTCCCCGTTTCTGATGCTGTTATCTGCTTACAGAGAATGGA
ACTCCTGTGTATTCAGACTTTCCAAAGACAGCCTACTGTCTGTGACCACAAGATCCTACCTGAGTG
GCAGCTGAAGTTGACTCCCTCTCCTTGCCTGAACCCCCCCCCACTGCCCCCCCATCCCCCAGTGTC
GGCTGAGATGTTGCCTCTGCACGGTTCTGTGTGCAGTTCCCAACTTTCTGCAGAAGATGGTCCTTG
CCCTTGTCCTGAAGAGTAGTAATGGTTCTTGAAAAAGATTTCAAATAAAGCCTGCACATAAAA

(SEQ ID NO:164)

Mus musculus Tomm6 3'-UTR

CCAGAGAATGGAACTCCTGTGTATTCAGACTTTCCAAAGACAGCCTACTGTCTGTGACCACAAGAT
CCTACCTGAGTGGCAGCTGAAGTTGACTCCCTCTCCTTGCCTGAACCCCCCCCCACTGCCCCCCCA
TCCCCCAGTGTCGGCTGAGATGTTGCCTCTGCACGGTTCTGTGTGCAGTTCCCAACTTTCTGCAGA
AGATGGTCCTTGCCCTTGTCCTGAAGAGTAGTAATGGTTCTTGAAAAAGATTTCAAATAAAGCCTG
CACATAAAA

(SEQ ID NO:165)

Mus musculus Mtch1 3'-UTR
NM_019880

CCTAAGCTGCCCGACCAAACATTTATGGGGTCTTAGCCTACCCCTGGTGAGGACCCATCATCTCAG
ATGCCCAAGGGTGACTCCAGCCCAGCCTGGCTTCATGTCCATATTTGCCATGTGTCTGTCCAGATG
TGGGCTGGTGGAGGTGGGTCACCTGGGACCTGGGGAAGCCTGGGGGAGCAGTGTTGGGGTGGCATC
CCCTTCCTGCCTAGAGGTACTGGAGTCCATCTTGTACTCAGGCAGAGGCAGGCTGCAGAGGCAAAC
GTCACTCAGTGGCAAGGCTTCCCTGCACCTCTAGCCCAGCTCATCCTGCCAGTCAGCCAGAAGCAC
CCCCGCCCCCCACTTCCTGCTTTGTAAATTGGGCGCCATCACACCTGGGCCATGGGAGGCTGGAGC
TATGTTCCCAACACTAATTTTCTTATACAAGGGTGGTGCCTTCTCCTGAATAGGAAATCATGTTCT
CCTCAGACCATCCCCTCATCTGCTTGTCTGTGCTGGTGACGCCAGGTGTGAGGGTTCAGTCACTGT
GCTGGGTGCGAATACGCACAGGTTACATAGGCCGACATCTAGTCCTCCCCTCGTGGTAAGATAGAC
CCATCTCCTCGAATAAATGTATTGGTGGTGATTTGGA

(SEQ ID NO:166)

Mus musculus Pcbd2 3'-UTR
NM_028281

TCTGCGCCTGCCTTGTCTGCAGCGTTGTTTGCAAGCCACTTATGTTAATAAATTGTCATAAAGTAG
TTCATAGTTACATGTATACATTGTTGTATGATTGATGCTCAAATACAGAATGATTTGAAGCCAAAA
AAAAAAAAAAAAAAAAAAAAAAAAA

(SEQ ID NO:167)

Mus musculus Ecml 3'-UTR
NM 007899

GTCACCCTGAGCCTCAGAGGATTAGATGGGGGAACTCCGCCCTACTCCACCCTCCTCGAACACTCA
TTACAATAAATGCCTCTTGGATTTGGC

(SEQ ID NO:168)

Mus musculus Hrsp12 3'-UTR
Hrsp12-001 ENSMUST00000022946

CTATAAGTAGCCATGCTGATGTTGACTCCGGAGGTTTTAGAATGTCTTTCACACTTTAATTTTTAC
AAATGATGCTGGGAAGTATAAAAATGACCAGAGTGGTTGAAGTTATTGTGGAAGTGATCAAATATG
TGGAGATTTGACATTAATTGGAGATTATTCAGTATAGTGACTGATGTTCTAATTTCACTTATGTTG
CTGGGTGTGAGAGAAGAGGTGCACAGCTACTGAGATGGGAAGCAGAAGGAAAGATGGGCTGTTGTA
CATGAGAAATAGTAAGGAGCACATCTACTTAAATCATATTAATTTGCTCATGTGAAATACTTAGTT
CTTATGTTAGATATAAGAAACTAAATTGAAATATTCAAACTTGAATAGTACCAGGAGAACAAGTGG
ACCAAAATCTTATACAGATAATATTACTTTAATTGAAATAAAAAATAGATGTGTAACTTTCC

(SEQ ID NO:169)

Mus musculus Mecr 3'-UTR
NM_025297

TTGCTCCAGAGGACCAGGAGGAAAGCAGGAGAGGCAAGACTGGCTGTCTGCTGGCCCCTCCATGAG
AACCCCAGCCTTCCCAGACTGCCTCACCCATATTGTCTCTTCCTACCAGGAGGGTGGGGGACCAAC
TCTAGGCTCCCTAATAAACCCTTAACTTCCCGAGTGGAGGATGAAGAGTAC

(SEQ ID NO:170)

Mus musculus Uqcrq 3'-UTR

NM 025352

ACGGCCTGCACCTGGGTGACAGTCCCCTGCCTCTGAAAGACCCTTCTCTGGGAGAGGAATCCACAC
TGTAGTCTTGAAGACAATAAACTACTTATGGACTTCCCTTTGAAAAAAAAAAAA

(SEQ ID NO:171)

Mus musculus Gstm3 3'-UTR
NM_010359

GCCCCTGCCATGCTGTCACTCAGAGTGGGGGACCTGTCCATACTGCGGATCCTGCAGGCTCTGGGT
GGGGACAGCACCCTGGCCTTCTGCACTGTGGCTCCCGGTTCTCTCTCCTTCCCGCTCCCTTCTGCA
GCTTGGTCAGCCCCATCTCCTCATCCTCACCCCAGTCAAGCCCATGCAGCCTTTATTCTCCCCATT
TTTTTTTCACATGGCCCCTTCTTCATTGGTGCCCAGACCCAACCTCACAGCCCTTTTCTGCAATCT
GAGGTCTGTCCTGAACTCAGGCTCCCTAGAGTTACCCCAATGGTCAACACTATCTTAGTGCCAGCC
CTCCCTAGAGATACCCTGATGGTCAATACTATCTTAGTGACGGCCCTCCCTAGAGTTACCCTGAAG
GTCAATACTCGAGTGCCAGCCTGTTCCTGTTTAAGGAGCTGCCCCAGGCCTGTCTCATGTACAATA
AAGCCTGAAACACACTTGAAACACAATAAACACTGAACACTTGCTGTGA

(SEQ ID NO:172)

Mus musculus Lsm4 3'-UTR
NM 015816

TCACTCCCTGCCTGAGCCGAGCCCAGAACGGTGGGTGAGGCCTCAGGGCACCTTTGTGTGAAGCCC
CACTTGGCGTCTGGTCCAGTGAAGTCCCTCGCTGGCCACTGACTCAGTTTCTGGAAGGTTCCGAGT
CTGAGGTGCCTGTGGAGCCTTAGATGCCCTTTGAAGGGCTGACTTCTTCCAGGCATGTTTGAGTTT
CAGTTGGAGCTGCAGGCTCAGCCCATGGCGGCTCACCTGTCCTTTACCAGCCATACCCTGTACATC
TTCTGTTTGAAAAATAAAAGCAAACACCATAGAAAGAAAAAAAAAAAA

(SEQ ID NO:173)

Mus musculus Park7 3'-UTR
NM 020569

AGCCCAAGCCCTGGGCCCCACGCTTGAGCAGGCATTGGAAGCCCACTGGTGTGTCCAGAGCCCAGG
GAACCTCAGCAGTAGTATGTGAAGCAGCCGCCACACGGGGCTCTCATCCCGGGTCTGTATGTTTCT
GAACCTTGCTAGTAGAATAAACAGTTTACCAAGCTCCTGCCAGCTAAAAAAAAAAAAAAAAA

(SEQ ID NO:174)

Mus musculus Usmg5 3'-UTR
NM 023211

ATGGATTTTGAAATGTCTGACCTCACCTGTTAAGTCCCATGCCTGAAGAAGCTGATGTGAACTCAT
CATGTAATACTCAATTTGTACAATAAATTATGAACCCAAAAAAAAAAAAAAAAAAAAAAAAAAA

(SEQ ID NO:175)

Mus musculus Cox8a 3'-UTR
NM_007750 AGGGAGCAGTCTTCCCTCATCCTTTGACTAGACCACTTTTGCCAGCCCACCTTGATCAT-GTTGCCT

GCATTCCTGGCTGGCCTTCCCCGGGATCATGTTATTCAATTCCAGTCACCTCTTCTGCAATCATGA
CCTCTCGATGTCTCCATGGTGACAACTGGGACCACATGTATTGGCTCTGCTTGGTGGGGTCCCCCT
TTGTAACAATAAAGTCTATTTAAACCTTGCTCC

(SEQ ID NO:176)

Mus musculus Ly6cl 3'-UTR
NM 010741

TGGTCCTTCCAATGACCCCCACCCTTTTCCTTTTATCTTCATGTGCAACCACTCTTTCCTGGAGTC
CTCTAGTGACAAATTATATGTTATAGAAGGTCCAATGTGGGGATAGTGTGTGGAACACCCTGTTTC
ACCTTTATAGCCCCTGCTGGGTAAGTGCCCGACTCCTCTCTAGGGCTTTCAAATCTGTACTTCTTG
CAATGCCATTTAGTTGTGGATTTCTATTCTTGGCCCTGGAGGCATGTGGCCAGCACATGCAACAGG
CAGTATTCCAAGGTATTATAGTATCACCATCCACACATAAGTATCTGGGGTCCTGCAGGGTTCCCA
TGTATGCCTGTCAATGACCCCTGTTGAGTCCAATAAAAGCTTTGTTCTCCCAGCCAAAAAAAAAAA
AAAAAAA

(SEQ ID NO:177)

Mus musculus Ly6cl 3'-UTR
NM_001252058.1

ACTCATAAAAATGCTCCTGCCTCGGTCTTCCAAGTTCTAGGATTGCAAGTCTGACTTCAACATGCC
TTACAGACAACTCTGGGACATCCAGGCCTAGTGGCATGTTGCCCAGATATGGGGATGCTCTGTGGC
CCCTGCATAAGAAGTGAGTCACTCCCTGATTTCTTGCAGACTCTCAAAGAAGGAAACTAAAGACCC
GTCAGTGCCTTTCTTTCTGCCCTGCTGGTGTGCCAATCAGGGATCCTAACATCAGGGAGAGGACTT
CCTGTTGCAGCGAAGACCTCTGCAATGCAGCAGTTCCCACTGCAG

(SEQ ID NO:178)

Mus musculus Cox7b 3'-UTR
NM_025379

TCGTGCCAGCTGGTACAATAATCAAGGAATTGTTTAAAACCAACTTATAAGTGAATGCCAAGTCAA
AGAATCATGTACTCATTATACTATGGCAGATTGAAGAACAAATAAAGAAATAAAGTACCTTAACCT
TCATTCTAGGCTTTGTTTTTTTCCTTTGTAAATGAAGCCCAAGCATGGTGACTTCTCATTTATTTA
AGCTGTATTGTCTCTTAAAATGGCTTTTTACCCTATGAGGTGGTATGAGGGAAATCTATGATCAGG
AGGGCACCTTTATAGTAAGCTGAAATTACAGAGAATGAAGAAATAAGCACAGAGCTGTTTTAGGAG
CCCACTGGGTCATTGGCCATATAGGTTATGCTTACTGCCCTCTACCTCGTGGTTATATTTGGAATT
GCCATTAGCTCCCTTCTGCTTAGAGACTGGACTGTCACCAAACCCAAGGGGATAGTGATCCTGTAA
TGATCCTGTGTGAACTAGGTTTGCTAAAGACTACCACCTCCTTACACTGTATGGCATATTCATCTG
AAATAGGTGCTAATTTTTCAGCATAATCCTTAATCTTTAGGATCTGTCATACTTCCTAGTAATTTA
ACTGTTGCTGAAGAAATAAAGGCTATCTGTTAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAA
AAAAAAAAAA

(SEQ ID NO:179)

Mus musculus Ppib 3'-UTR
NM_011149

AGAGCCTGGGGGACCTCATCCCTCTAAGCAGCTGTCTGTGTGGGTCCTGTCAATCCCCACACAGAC
GAAGGTAGCCAGTCACAAGGTTCTGTGCCACCCTGGCCCTAGTGCTTCCATCTGATGGGGTGACCA
CACCCCTCACATTCCACAGGCCTGATTTTTATAAAAAACTACCAATGCTGATCAATAAAGTGGGTT
TTTTTTATAGCTTGAAAAAAAAAAAAAAAAAAA

(SEQ ID NO:180)

Mus musculus Bag1 3'-UTR
NM 009736

AGTGCAGTGGAGAGTGGCTGTACTGGCCTGAAGAGCAGCTTTACAGCCCTGCCCTCTCTGGAACAG
AAGTCGCCTGTTTCTCCATGGCTGCCAGGGGCAACTAGCCAAATGTCAATTTCCCTGCTCCTCCGT
CGGTTCTCAATGAAAAAGTCCTGTCTTTGCAACCTGAATTAGACTTGTGTTTTCTCAAAAAAAAAA
AAAAAAA

(SEQ ID NO:181)

Mus musculus S100a4 3'-UTR
S100a4-201 ENSMUST00000001046

AGACTCCTCAGATGAAGTGTTGGGGTGTAGTTTGCCAGTGGGGGATCTTCCCTGTTGGCTGTGAGC
ATAGTGCCTTACTCTGGCTTCTTCGCACATGTGCACAGTGCTGAGCAAATTCAATAAAAGGTTTTG
AAACTATT

(SEQ ID NO:182)

Mus musculus Bcap31 3'-UTR
NM_012060

AGGCTTGGTGTTTCCCTGCCTGCCGCTGGCTTCTACCTGACCCATGCTTACTGCTTCCTTGGAGCC
CAGACTATCCCTCTGGTACTTGGGTTTATTCCCTACTTCCCCAATTTTCTTCCATGGCTTATAGAT
CATTATTTTGGCACCATTACACATACTGCTCTTATACCAAAAGGGACCTGATTGTTGTTTATTCAG
AGTACTTTTGCCACTGTTCTGCCTGGCTAGGGCACTTTCCACTCCTGGAAGTGTAGAAAAGCACTG
GTGACCTGGCCTGCAGTTTGAACCCCTTTTTATTTTGCAATGTACCCTAAAGGAGGCTGCTGTGAA
GCAGGTCAACTGTTTTATCCTGAGGGGAATAAATGTTGTTATGT

(SEQ ID NO:183)

Mus musculus Tecr 3'-UTR
NM_134118

GCAGCTCCTCACGGCTCTGCCCAGTAATACTCTCCACCCCTCACTGCCCCTGTCCTGATGTGTGGC
TGGCCATGGCTCTCCAGCAGCAACAATAAAACCTGCTTACCCAAAAAAAAAAAAAAAAAA

(SEQ ID NO:184)

Mus musculus Rabac1 3'-UTR
NM_010261

AGTGTCCTCCAGGACCTGCCGGCCTCTCCTGCCGGCCGGCTGTCCCATCTCTGTCTGTTCTCGTCC
TACCTGGCCTTGCTGCTCAGCTCCGAGCCTTCCACCTGAGGCCTCAAACCCAGGGAGGGGCTTTTG
TCTTTGGAAATAAAGCTGTTACAATTGCTATTTGGCCAA

(SEQ ID NO:185)

Mus musculus Robld3 3'-UTR
NM_031248 (Lamtor2)

CAGCGTGATGGAGGCTGGAGTAGAAAAGGGATGATGATCTGGAGGGAGGGGCGGGGCCCTAGAAAC
GCCATATCGGGCGAGGTACAGGAAGGGGGGGTTGCTTTTTTCTGAATAAATTTTCAACTCTTAAAA
AAAAAAAAAAAA

(SEQ ID NO:186)

Mus musculus Sod1 3'-UTR
NM_011434

ACATTCCCTGTGTGGTCTGAGTCTCAGACTCATCTGCTACCCTCAAACCATTAAACTGTAATCTGA
AAAAAAAAAAAAAA

(SEQ ID NO:187)

Mus musculus Nedd8 3'-UTR
NM_008683

AGAAACTTGGTTCCGTTTACCTCCTTGCCCTGCCAATCATAATGTGGCATCACATATCCTCTCACT
CTCTGGGACACCAGAGCCACTGCCCCCTCTCTTGGATGCCCAATCTTGTGTGTCTACTGGTGGGAG
AATGTGAGGACCCCAGGGTGCAGTGTTCCTGGCCCAGATGGCCCCTGCTGGCTATTGGGTTTTAGT
TTGCAGTCATGTGTGCTTCCCTGTCTTATGGCTGTATCCTTGGTTATCAATAAAATATTTCCTG

(SEQ ID NO:188)

Mus musculus Higd2a 3'-UTR
NM 025933

GTATAGCCGGGTCTTAAAGCGCCATGGAAACCATTACAAAACCCAGGAACAACAGACATCCCTGTC
AGACTTGCTCCCTCCGTTTCAGACCGGACCTTATTGTCATTTGGGTGAGGAAGTGGCCGATTTTGT
AACTGATTTGCGCTTCCACCGCTGCCCCCTCCCGCTCCCAAAATCCCAGGTTCATTTCAGTTGGGT
TGCATGCTTCTATTTGTGATGCGTCCCCTTAATTACTTAATAAAAGCTTATTACACTTG

(SEQ ID NO:189)

Mus musculus Trappc6a 3'-UTR
Trappc6a-001 ENSMUST00000002112

GGACCCCAGACCCCAGGCTTGCCCTTCCCTAAGCTTAGCCTCGGAATGTGGCACCTGACCCTGCCT
CACTGCTCACCTTTGCAGGTCGCCTTGAAGCTGGAGCTCACAGGCTCTGGGGAGGTCACATGTGCT
TCAGACAAGGGAATGAAAGGGCCGGGAGGGTCCCGGGAGGTGGGACCATCCCCTGAGTTCCAAGTC
AGCATGGAGGGACATTAGGGCATCACCCAGATGACAGATGTTCAGTAAAGGTTCTTTATGTGCAAA
CAGA

(SEQ ID NO:190)

Mus musculus Ldhb 3'-UTR
Ldhb-001 ENSMUST00000032373

CTGCCAGTCTCTAGGCTGTAGAACACAAACCTCCAATGTGACCATGAACCTTTAGTCTTCAGCCAT
GTATGTAGGTCACAGTTTGCTTCTTCCCTGACATGTGATATGAGCTCACAGATCAAAGCCCAGGCT
TGTTTGATGTTTGCACTAGGAGCTCCTGATCAAATAAAGTTAGCAATTGCAGCATA

(SEQ ID NO:191)

Mus musculus Nme2 3'-UTR
Nme2-001 ENSMUST00000021217

ACATGAAGAAACCAGAATCCTTTTCAGCACTACTGATGGGTTTCTGGACAGAGCTCTTCATCCCAC
TGACAGGATGGATCATCTTTTCTAAAACAATAAAGACTTTGGAACT

(SEQ ID NO:192)

Mus musculus Snrpg 3'-UTR
NM_026506

CCTGTGCTCAGCAAGCAGTGTCCACATCCCTCCCCAAAGGCCTGTTTGATTGTGATGTAGAATTAG
GTCATGTACATTTTCATATGGAACTTTTTACTAAATAAACTTTTGTGATACTC

(SEQ ID NO:193)

Mus musculus Ndufa2 3'-UTR
NM_010885

AGGTCTCCACTGAGGACTGTGAGCGAGAGCAGCTGAACCTGCTGGACTGAAGACAGTGTGGGGAAA
TGTGTGCTTTGGGTCCTTATAAAGCTTACGCTGTACAGTGTCCCTTCAGAATGTCCTCTTCATTAC
CTTCTCCCTCTTACTGCGCAACACTGAGGCAAAGTAGTTTTATATAAAAATACTCCTTTATTTCTC
CTCAAAAAAAAAAAAAAAAAAAACCCACCAGGTGCCA

(SEQ ID NO:194)

Mus musculus Serf1 3'-UTR
Serf1-003 ENSMUST00000142155

TGACTGGCTTTTTGGAAAACCTGGGTGCTATTGCCAGTGGGTGCATCATACGCTCTAAGATTAAAA
TTTCACAGTGACTAATCATTATATGTGTTATAACTTGTCCTTATAAAACTATTTTAAACTTTACTC
TTCAGCCTATCTTAATGTGATGTTTTAAGACCATCAAAAAATAAAGTACTGACCTTGCATGTAA

(SEQ ID NO:195)

Mus musculus Oaz1 3'-UTR
Oaz1-001 ENSMUST00000180036

GTGCCAGCCCTGCCCAGTGTCCCTGTGCCCTCTCCTGGGTTAGTCCACATGTCGTGATTGTGCAGA
ATAAACGCTCACTCCATTAGCGGGGTGCTTCTTCGAGCTGAATGCTGTGTTTGTCACACTCAAGTG
TTGGCTTTAATTCTAAATAAAGGTTTCTATTTTACTTTTTTATTGCTGTTTAAGATGGTCAGGTGA
CCTATGCTATAGCAGTCTCCTTTGAAGTCTGGAAAAATAGTGTCACCTCCCCTGGCTCAAATCCAA
TAAAGTGATCTCGTTCATTGGC

(SEQ ID NO:196)

Mus musculus Ybx1 3'-UTR
Ybx1-001 ENSMUST00000079644

ATGCCGGCTTACCATCTCTACCATCATCCGGTTTGGTCATCCAACAAGAAGAAATGAATATGAAAT
TCCAGCAATAAGAAATGAACAAAGATTGGAGCTGAAGACCTTAAGTGCTTGCTTTTTGCCCTCTGA
CCAGATAACATTAGAACTATCTGCATTATCTATGCAGCATGGGGTTTTATTATTTTTACCTAAAG
ATGTCTCTTTTTGGTAATGACAAACGTGTTTTTTAAGAAAAAAAAAAAAAAAGGCCTGGTTTTTCTC
AATACACCTTTAACGGTTTTTAAATTGTTTCATATCTGGTCAAGTTGAGATTTTTAAGAACTTCAT
TTTTAATTTGTAATAAAGTTTACAACTTGATTTTTTCAAAAAAGTCAACAAACTGCAAGCACCTGT
TAATAAAGGTCTTAAATAATAA

(SEQ ID NO:197)

Mus musculus Ybx1(v2) 3'-UTR
with mutation T128bpG and deletion de1236-237bp

*TTT*TTATGCCGGCTTACCATCTCTACCATCA
TCCGGTTTGGTCATCCAACAAGAAGAAATGAATATGAAATTCCAGCAATAAGAAATGAAC
AAAGATTGGAGCTGAAGACCTTAAGTGCTTGCTTTTTGCCCGCTGACCAGATAACATTAG
AACTATCTGCATTATCTATGCAGCATGGGGTTTTATTATTTTTACCTAAAGATGTCTCT
TTTTGGTAATGACAAACGTGTTTTTTAAGAAAAAAAAAAAAAGGCCTGGTTTTTCTCAATA
CACCTTTAACGGTTTTTAAATTGTTTCATATCTGGTCAAGTTGAGATTTTTAAGAACTTC
ATTTTTAATTTGTAATAAAGTTTACAACTTGATTTTTTCAAAAAAGTCAACAAACTGCAA
GCACCTGTTAATAAAGGTCTTAAATAATAA

(SEQ ID NO:198)

Mus musculus Sepp1 3'-UTR
NM_009155

ATTATTTAAAACAAGGCATACCTCTCCCCAACTCAGTCTAAAGACACAATTTCATTTTGAGAATGT
TTACAGCCCATTTAATTAATCAGTGAACTAAAAGTCATAGAAATTGGATTTGTGCAAATGTAGAGA
AATCTACCATATTGGCTTCCAAAATTTAAAAATTTTATGCCACAGAACATTTCATCCAAATCAGAT
TTGTACAATAGGGCACCTGAAAAGTGACTGCAGCCTTTGGTTAATATGTCTTTCTTTTTCCTTTTT
CCAGTGTTCTAGTTACATTAATGAGAACAGAAACATAAACTATGACCTAGGGGTTTCTGTTGGATA
GCTTGTAATTAAGAACGGAGAAAGAACAACAAAGACATATTTTCCAGTTTTTTTTTTCTTTACTTA
AACTCTGAAAACAACAGAAACTTTGTCTTCCTACTCTTACATTCTAAACCGATGAAATCTTTAACA
GATTACACTTTAAATATCTACTCATCATTTTCTCTCTCAGAGTCCTAGCTTGAGTTGCACTGCATG
TATCTGTGCATCTTGTTCTCTTCATTTAATGCTGTACTGTTCTGCTGAGCTCTGAGGGACTATCTT
GAGAGATGTAATGGAAGGAAAGCGTGGTGTTAATCTGCGTACTGCTTAAGACAGTATTTCCATAAT
CAATGATGGTTTCATAGAGAAACTAAGTCCTATGAACCTGACCTCTTTTATGGCTAATACGACTAA

GCAAGAATGGAGTACAGAATTAAGTGGCTACAGTACACACTTATCAAAATAAATGCAATTTTAAAA
CCTTTC

(SEQ ID NO:199)

Mus musculus Gaa 3'-UTR
Gaa-001 ENSMUST00000106259

GAGAGTCCGTCGTTTACAGAGGCCTCCAGGGAGGCAGAGGGAGCTTGAGCTGGCTCTGGCTGGTGG
CTCCTGTAAGGACCTGCGTCCTGCTCTCCTGACACATCTTTGAGCTTTTCCCACCGTGTTACTGCA
TGCGCCCCTGAAGCTCTGTGTTCTTAGGAGAGTGAGGCTCGCCTCACCTGCCCCACCCCAGCTGTC
TGTCCCTCACCTGGCACTAGAGAATGTGGAGCTCGGCGTGGGGACATCGTGTCTGCACCAACATCA
GGCTGTGCAGCCACTGCAGCCGCAACCCTGCAGAGACAGAGCTGGTGCCTTCACCAGGTTCCCAAG
ACTCGAGAAACTTACTGTGAAGTGTACTTACTTTTAATAAAAAGGATATTGTTTGGAAGC

(SEQ ID NO:200)

Homo sapiens ACTR10 3'-UTR
ACTR10-002 ENST00000254286

AAGTTTGATTAAAAATCAACCTTGCTTCATATCAAATATTTAACCAATTATAAGCAAATTGTACAA
AGTATGTAGGATGTTTTGTTATAGAGGACTATAGTGGAAGTGAAAGCATTCTGTGTTTACTCTTTG
CATTAATATATAATTCTTTTGACTTTGTTTCTCTTGTGTAGTGGTAAAATGGTAGCTGGTGCTTAT
TGAGATTTGCTGTATTTATATCAATAAAGTATAGTAAAGCAGTTTGATTTTGGAAGTTTGTTATGT
GGCTTTTTTTTTTTTTTTTTTTTTGAGACGGAGTCTCGCTCTGTCACTTAGGCTGGAGTGCAGTGG
CACAATCTCTACTCATTGCAAGCTCCGCCTCCCGGGTTTACGCCATTCTGTCTCAGCCTCCTGAGT
AGCTGGGACTATAGGCATACGCCACCCCGCCCGGCTAATTTTTTGTATATTTAGTAGAGACGGGGT
TTCACCATGTTAGCCAGGA

(SEQ ID NO:201)

Homo sapiens PIGF 3'-UTR
NM_173074

GTAACTTAATCCTGACAACCGTAGTGCAAGGTATGGCCCATCTCCTGTACGCTTGGAGCGACCTTT
GGCTACGTGGCTGGCCTTGTTATTTCACCACTCTGGATATACTGGAATAGAAAGCAACTTACATAC
AAGAACAATTAACTGGAGCAAAGGGAGATATTTCTTTGTGCAGATTCTGTAAGGGCTGGGCAGAAA
TGTGTATGGTCAAAGCCAAGCAGTTCCATTTACAGCTCTGTTTTTTACGTAGTTACAACATGATGT
GATTGTAGCTTTTTAAACTATGAAACCCCTGAGAGATTGTACCTTCTAGTTGAAATAAAGTATTTA
TAATAGATTGTGGCTTC

(SEQ ID NO:202)

Homo sapiens PIGF 3'-UTR
NM_002643.3

CTGGAGCAAAGGGAGATATTTCTTTGTGCAGATTCTGTAAGGGCTGGGCAGAAATGTGTATGGTCA
AAGCCAAGCAGTTCCATTTACAGCTCTGTTTTTTACGTAGTTACAACATGATGTGATTGTAGCTTT
TTAAACTATGAAACCCCTGAGAGATTGTACCTTCTAGTTGAAATAAAGTATTTATAATAGATTGTG
GCTTCAAAAAAAAAAAAAAAAAAAAAAAAAAAAAA

(SEQ ID NO:203)

Homo sapiens MGST3 3'-UTR
MGST3-001 ENST00000367889

AGAATTATAGGGGTTTAAAAACTCTCATTCATTTTAAATGACTTACCTTTATTTCCAGTTACATTT
TTTTTCTAAATATAATAAAAACTTACCTGGCATCAGCCTCATACCTAAAA

(SEQ ID NO:204)

Homo sapiens SCP2 3'-UTR
NM_001193599

AGAACTCCCTTTGGCTACTTTTGAAAATCAAGATGAGATATATAGATATATATCCATACATTTTAT
TGTCAGAATTTAGACTGAAACTACACATTGGCAAATAGCGTGGGATAGATTTGTTTCTTAATGGGT
GTGACCAATCCTGTTTTTCCTATGCTCTGGGTGAATAGAGCCTGATGGTATACTACTGCTTTGCGG
AATTGCATACAACTGTGCATTACAAAGTTAATATGGTAATTATGGTCTGGGGTAAAATTGAGTTTC
AGAATAAAATTAGGAACAGTAAAATCCAAAGAACTATGTAAACAAAAAAGCTTTTGTTTTGCTTAC
AAAGTATATTTAAGGATTATTCTGCTGAAGATTCAGTTTAAGAGTTTTCCTTGGGAGAACTAAGTA
AGAAACACAATGCCAACAGCTGGCCAGTAATTAGTGTTGTGCACTTCATGTCATTAATCAATTTCT
CAATAGTTCTTAAAATTAGTGAGATTAAAAATCTAAAAATTTTGCATTTCATGCTATCAGAAACAG
TATTTTCTTCCCAAATCAAAATAAAAGAAATATGATCAGAGCTTGAACACAGGCTTATTTTTAAAA
TAAAAATATTTTTAACATGGGTTTCCTTATTGAAAAATCAGTGTATTAGTCATAAAACACCATCAT
TAAGAATAATTGAACAATAAAGTTTGCTTTCAGATGCAGTTTTCAAATTATAATCTCATTTCAATT
TATAACGTTCTCAGTCCTTTGTTATAATTTTCCTTTTTCATGTAAGTTTAATTATCTGCATTTATC
TTTTTTCCTAGTTTTTCTAATACTAATGTTATTTCTTAAAATTCAGTGAGATATAGGATAAAATAA
TGCTTTGAGAAGAATGTTTAATAGAAAATTAAAATAACTTTTTCTGGCCTCTCTT

(SEQ ID NO:205)

Homo sapiens SCP2 3'-UTR
SCP2-015 ENST00000435345

AGAACTCCCTTTGGCTACTTTTGAAAATCAAGATGAGATATATAGATATATATCCATACATTTTAT
TGTCAGAATTTAGACTGAAACTACACATTGGCAAATAGCGTGGGATAGATTTGTTTCTTAATGGGT
GTGACCAATCCTGTTTTTCCTATGCTCTGGGTGAATAGAGCCTGATGGTATACTACTGCTTTGCGG
AATTGCATACAACTGTGCATTACAAAGTTAATATGGTAATTATGGTCTGGGGTAAAATTGAGTTTC
AGAATAAAATTAGGAACAGTAAAATCCAAAGAACTATGTAAACAAAAAAGCTTTTGTTTTGCTTAC
AAAGTATATTTAAGGATTATTCTGCTGAAGATTCAGTTTAAGAGTTTTCCTTGGGAGAACTAAGTA
AGAAACACAATGC

(SEQ ID NO:206)

Homo sapiens HPRT1 3'-UTR
HPRT1-001 ENST00000298556

GATGAGAGTTCAAGTTGAGTTTGGAAACATCTGGAGTCCTATTGACATCGCCAGTAAAATTATCAA
TGTTCTAGTTCTGTGGCCATCTGCTTAGTAGAGCTTTTTGCATGTATCTTCTAAGAATTTTATCTG
TTTTGTACTTTAGAAATGTCAGTTGCTGCATTCCTAAACTGTTTATTTGCACTATGAGCCTATAGA
CTATCAGTTCCCTTTGGGCGGATTGTTGTTTAACTTGTAAATGAAAAAATTCTCTTAAACCACAGC
ACTATTGAGTGAAACATTGAACTCATATCTGTAAGAAATAAAGAGAAGATATATTAGTTTTTTAAT
TGGTATTTTAATTTTTATATATGCAGGAAAGAATAGAAGTGATTGAATATTGTTAATTATACCACC
GTGTGTTAGAAAAGTAAGAAGCAGTCAATTTTCACATCAAAGACAGCATCTAAGAAGTTTTGTTCT
GTCCTGGAATTATTTTAGTAGTGTTTCAGTAATGTTGACTGTATTTTCCAACTTGTTCAAATTATT
ACCAGTGAATCTTTGTCAGCAGTTCCCTTTTAAATGCAAATCAATAAATTCCCAAAAATTTAA

(SEQ ID NO:207)

ACSF2
Homo sapiens

ATAAAGCAGCAGGCCTGTCCTGGCCGGTTGGCTTGACTCTCTCCTGTCAGAATGCAACCTGGCTTT
ATGCACCTAGATGTCCCCAGCACCCAGTTCTGAGCCAGGCACATCAAATGTCAAGGAATTGACTGA
ACGAACTAAGAGCTCCTGGATGGGTCCGGGAACTCGCCTGGGCACAAGGTGCCAAAAGGCAGGCAG
CCTGCCCAGGCCCTCCCTCCTGTCCATCCCCCACATTCCCCTGTCTGTCCTTGTGATTTGGCATAA
AGAGCTTCTGTTTTCTTTG

(SEQ ID NO:208)

Homo sapiens VPS13A 3'-UTR
NM_033305

AATTCATATGTTCTTTATTTTACTTGGAATGTTTCATTAACATGTTTTGTATGACTTATACCATAA
TGCCCATATGTCCATTTATAGGGAGGTAAAACACATTTTCTTTTAAAATGTTTTCCTACACATTTT
CATAAAGCAAAATAATTGTATTATTTAAGCACAGAAAAAAAATGTATCTTACATCCAAAGTAGGGAG
GGCATCCAACATATTATAGATTTGCTTTTATATATTTTATAGCTTTGTATTGCATAGTTTGTCTTT
AAGAGTTCAAGTTAGACTTAAATATAATTTTGATGTTCACTGGTTTTATTTTAAATTGCCTTCTTA
TTTGTTAGCAAAATGCCTTTTTTTAATGGTCTCTGTAAATTTTCTGGGCTTTAATGTAATGCCACT
GTGTAAAAAAAAAGGAAGAAAATAGTAATAGCCATTTAATGTTTTATATTTATCATTTTAAAGATA
TTTTTGTCAAATTTCTTTTAATAATAATAAACATATGTAATCTAAAAAAAAAAAAAAAAAAAAAAA
AAAAAAAAAAAAAAAAAAAAAAAAAAA

(SEQ ID NO:209)

Homo sapiens CTH 3'-UTR
NM_001190463.1

TATTCCAGAGCTGCTATTAGAAGCTGCTTCCTGTGAAGATCAAATCTTCCTGAGTAATTAAATGGA
CCAACAATGAGCCTTTGCAAAATTTTCAAGCGGAAATTTTAAGGCACCTCATTATCTTTCATAACT
GTAATTTTCTTAGGGATCATCTCTGTTAAAAAGTTTTCTGTATGTCATGTTATAATTACAGGTCAA
TTCTGTTAATATCTTTTTGTTAATTTTGCTCTATGTTTGCCTCTGAAGGAGGTGAGATTTGTGCTA
CTTTGGGAGATTATGTTCTTTTTTCATGTCTAAGATTTATTTTGATCATGTTTATAATATAATGGT
AATTCATTTTTGATGTTTTGTGAAGAATTTAAATTTAAACGAATGTTCTTAAATCAAGTGTGATTT
TTTTGCATATCATTGAAAAGAACATTAAAAGCAATGGTTTACACTTAGTTACCATAAGCCGAAAAT
CAAATACTTGAAAAGTTTACTGTGAAATTCTACTGATTTAAGACTATACTTAATATTTTTAAAAAA
ATAAATCAGCTGGGCGCGGTGGCTCACGCATGTAATGCCAGCACTTTTGGAGGATAAGGCGGGCGG
ATCACGAGGTCAGGAGATTGAGACCATCCTGGCTAGCGCAGTGAAACCCCCATCTCTACTAAAAAT
GCAAAAAAAATTAGACGGACGTGGTGGCGGGTGCCTGTAGTCCCAGCTACTTGGGAGGCTGAGG

(SEQ ID NO:210)

Homo sapiens CTH 3'-UTR
CTH-001 ENST00000370938

TATTCCAGAGCTGCTATTAGAAGCTGCTTCCTGTGAAGATCAAATCTTCCTGAGTAATTAAATGGA
CCAACAATGAGCCTTTGCAAAATTTTCAAGCGGAAATTTTAAGGCACCTCATTATCTTTCATAACT
GTAATTTTCTTAGGGATCATCTCTGTTAAAAAGTTTTCTGTATGTCATGTTATAATTACAGGTCAA
TTCTGTTAATATCTTTTTGTTAATTTTGCTCTATGTTTGCCTCTGAAGGAGGTGAGATTTGTGCTA
CTTTGGGAGATTATGTTCTTTTTTCATGTCTAAGATTTATTTTGATCATGTTTATAATATAATGGT
AATTCATTTTTGATGTTTTGTGAAGAATTTAAATTTAAACGAATGTTCTTAAATCAAGTGTGATTT
TTTTGCATATCATTGAAAAGAACATTAAAAGCAATGGTTTACACTTA

(SEQ ID NO:211)

Homo sapiens CTH 3'-UTR

CTH-002 ENST00000346806

TATTCCAGAGCTGCTATTAGAAGCTGCTTCCTGTGAAGATCAAATCTTCCTGAGTAATTAAATGGA
CCAACAATGAG

(SEQ ID NO:212)

Homo sapiens NXT2 3'-UTR
NXT2-004 ENST00000372107

AGGGGCAAAAGTCCATTCTCATTTGGTCCATTAGTTCCAGCAATTGAAATTTATGTGAATTATTTT
GATTGTAGAAGCACTATAATATGTGCTGAAACTAAATTTCTTTAATATTTTCTATTCCTGTCAGCA
CCTTTTCTAGCAGCTGCCAGTTTGGAGCATTGCCCTCTAAGAGCTTTAAAACTATTTTTTTACATG
CCTTATATACATTCCACTAATGACATTCTTATAATAATATTAAACACATGATCTTGGTACTAACAT
ACTCACTGTGAACCCAGCCTAT

(SEQ ID NO:213)

Homo sapiens MGST2 3'-UTR
NM 002413

CTTTTTCTCTTCCCTTTAATGCTTGCAGAAGCTGTTCCCACCATGAAGGTAATATGGTATCATTTG
TTAAATAAAAATAAAGTCTTTATTCTGTTTTTCTTGAAAAAAAAAAAAAAAAAAA

(SEQ ID NO:214)

Homo sapiens MGST2 3'-UTR
NM 001204366.1

CTTTTTCTCTTCCCTTTAATGCTTGCAGAAGCTGTTCCCACCATGAAGGCTTGAAGCCACAGTGCA
TGGCCAGAACCAGCCAGACCTTTGGAGTTCAAGAACTCGAGAGGTGGGTGAAAACTGCCATTGCCT
CCACAGACTGTCTTCTCCGTGGAAAGAAGACCTGAGTCACCAGGGCTGGGAAACCTGCACCACTGA
GACGAGCACAGCCTCTGCCGGCATGCAAGTGGCCGCTGTCAGGACACATGGACTGAAAGTGGTTTG
TCAGCTGCTCCATTAGGTTTTTTTTACCCATATGTTTGCTACCTTTCTTTCCTTGATTTAAAAATA
GGGAGGGGGAGCAGTCTCAGCTGTCTTCAGCTGCTAGGGAGATTTTTTTCCCCCTCCTGAGCTACT
GTTTCCCCCAACCCGAGCCTTTCTCTCTTATTGTACCCACCCTTTCTGATGAAGTCATCAAAGCAA
AGATTGCATAACTGATGCATAGGCCTATCTTGTGTTATACTGGGAGACAGGCCAATGTTTCCATTA
ATAGACAAGAGCACCACCACGCTGCCAAATGGAGCTCTCTGCTGCAACCACTAC

(SEQ ID NO:215)

Homo sapiens C11orf67 3'-UTR
AAMDC-005 ENST00000526415
TGGAGCCTTAAGAGGAGAATAAATCACTAAGTGCCTA
(SEQ ID NO:216)

Homo sapiens PCCA 3'-UTR
NM 000282

AGGATTTATAACCTTTCAGTCATCACCCAATTTAATTAGCCATTTGCATGATGCTTTCACACACAA
TTGATTCAAGCATTATACAGGAACACCCCTGTGCAGCTACGTTTACGTCGTCATTTATTCCACAGA
GTCAAGACCAATATTCTGCCAAAAAATCACCAATGGAAATTTTCATTGATATAAATACTTGTACAT
ATGATTTGTACTTCTGCTGTGAGATTCCCTAGTGTCAAAATTAAATCAATAAAACTGAGCATTTGT
CT

(SEQ ID NO:217)

Homo sapiens GLMN 3'-UTR
NM 053274

AAGTTCCATTTCCTAAATAAAAACTAATAAAATATAGTACTTTCCATTATGATTCATTTAATACCT
TTATAAAAAATTTTTCTGTAAAAATTTACTGCTTGAAAAATAAATGTAGCTTTTCTCATTTATCAA
AAAAAAAAAA

(SEQ ID NO:218)

Homo sapiens DHRS1 3'-UTR
NM_001136050

CCCTCCTGGTCTGACACTACGTCTCTGCTTGTCTTCTCATTTGGACTTGGTGGTTCGTCCTGTCTC
AGTGAAACAGCAGCCTTTCTTGTTTACCCATACCCTTGATATGAAGAGAAGCCCTCTGCTGTGTGT
CCGTGGTGAGTTCTGGGGTGCGCCTAGGTCCCTTCTTTGTGCCTTGGTTTTCCTTGTCCTTCTTTT
TACTTTTTGCCTTAGTATTGAAAAATGCTCTTGGAGCTAATAAAAGTCTCATTTCTCTTTCAAAAA
AAAAAAAAAAAA

(SEQ ID NO:219)

Homo sapiens PON2 3'-UTR
PON2-001 ENST00000433091

ATTGTACTTTTGGCATGAAAGTGCGATAACTTAACAATTAATTTTCTATGAATTGCTAATTCTGAG
GGAATTTAACCAGCAACATTGACCCAGAAATGTATGGCATGTGTAGTTAATTTTATTCCAGTAAGG
AACGGCCCTTTTAGTTCTTAGAGCACTTTTAACAAAAAAGGAAAATGAACAGGTTCTTTAAAATGC
CAAGCAAGGGACAGAAAAGAAAGCTGCTTTCGAATAAAGTGAATACATTTTGCACAAAGTAAGCCT
CACCTTTGCCTTCCAACTGCCAGAACATGGATTCCACTGAAATAGAGTGAATTATATTTCCTTAAA
ATGTGAGTGACCTCACTTCTGGCACTGTGACTACTATGGCTGTTTAGAACTACTGATAACGTATTT
TGATGTTTTGTACTTACATCTTTGTTTACCATTAAAAAGTTGGAGTTATATTAA

(SEQ ID NO:220)

Homo sapiens NME7 3'-UTR
NM_013330

TGGTGTGGAAAGTAAAGAAGTCACAGGTTGGGACATTTAGACAAGAGTGAATCACACACGAGGAAT
GTGTTCATTCTTTTATTGTCCGTTGTTTTAACCTGACTGAATACAAGATCAACAAGAGCACTGTAC
TCCTGGCAATTATTACATATGTTAGAACATGGATTTTGCACTGTAGACAACATTTAACACCAGTCT
ATGGGGTACTGCATTGCTTTTTATAAAGTTCAAAATAAAGATTTATTTTCAAACAAAAAAAAAAAA
AAAAAAAAAAAAA

(SEQ ID NO:221)

Homo sapiens ETFDH 3'-UTR

NM_004453

ACTGCAGCTAGCCAGTTTCTTTCAAGTATGGCAAGCTAACGTTAAAATGTTTAGAGATTAACAGAT
TTCAGAATGTCTTTCTGCATATTACTGAACAGAATAGTCACAAAATGATTATCAAATAAAAATTTT
ATACTATATGTAAGATTGTCCCATAAAGAAA

(SEQ ID NO:222)

Homo sapiens ALG13 3'-UTR
BC117377

GATCCAGCAGTATGAAGTATTCTTGCACTGCCATTTTCTTGCTGTTTTTGTTTTTAAAAAGTATTT
TATGTTAGTGGTTAAATGATTTAGGTGATTAGTGTTTACTATTGTATTTGTCTTTAAAATTATTTT
ATCTTTTGATTTAAAATAGTACTTTAAAATTAAGGGGTATTATTTTGGGCTGTGACTAAGGAAATT
GAGATGGATGTACAACTAGCCCCATATTGAGCATACTTCATTGTATTCAGCTGTTTTCCTGTCAGC
CATTTGTCAGC

(SEQ ID NO:223)

Homo sapiens ALG13 3'-UTR
NM 001099922.2

GATCCAGCAGTATGAAGTATTCTTGCACTGCCATTTTCTTGCTGTTTTTGTTTTTAAAAAGTATTT
TATGTTAGTGGTTAAATGATTTAGGTGATTAGTGTTTACTATTGTATTTGTCTTTAAAATTATTTT
ATCTTTTGATTTAAAATAGTACTTTAAAATTAAGGGGTATTATTTTGGGCTGTGACTAAGGAAATT
GAGATGGATGTACAACTAGCCCCATATTGAGCATACTTCATTGTATTCAGCTGTTTTCCTGTCAGC
CATTTGTCAGCTTTATATTAGCTGATGGTACCAATTGATAAAATGAATATAAAGTATTTCATTGGT
TCAAAAATCACACATCATATTAAACCATGCAGAATTGGAGTAACTTCCACTTTTTTCTAGAAAGTA
AAACCAAGAGCCTTTGCTTCTGGATAACTCACTTAATATTAAATTAAAGAGCTCTTCACGTTTCTT
GAGAATTATCTGAAGCCAGTTGCATTCTGTGATATCAGTTTTGAAGGCACATGGTTCTCTGCTTTA
GATTTATCCCATATGCTATTGTTTAATACTGGATGTATGTAAGTGTTTTACTGCACTGTATTGAAT
TGGTGTCTTTTGCACAGTTAGCAGTAAATAAAAATTAGCATTTAAAATTGCCAAAAAAAAAAAAAA
AAAA

(SEQ ID NO:224)

Homo sapiens DDX60 3'-UTR
DDX60-001 ENST00000393743

AAACAAAGTCTATGCAAACCACTTAAAAATAATTCCATAGTAGTTTTTCAGGTCACGTTTTTGATT
CTTATGCTTCTTGCCAGAAATACATTATGATAAAGTGGAAATACATTACGATGAAGTGGAAAGAGC
AAACACTTTGGAATCAAACAGAGTTGCAATCAAACCTGCCATGTTCTGTCATGAATACTCACAAAT
TATTTAGTATACCTGAATCTTGGTTTCTTTTTATAACTGAGTAATAATGGTTACATCTCAGGTAGT
TTGAGGATTGACTAAAAAAAATGCGAGAATGTTGTATGTGACTGAATAACAATTTTTACTCTGCGAA
GCCAAAGTAAATATAATATTATCAGTAACTTTATCCCCAGTGTCAGTATTTATAAAATGTTTATTA
AGGCTAGAAAAAAATGAATACAATATCCTGAAGGTGAAATATATTCTCTTCAATTAGCATAAATATG
ATTTACATAAGTTAGCTATACAGCTATTGAGATAGTACTTTCTAGTAAACTTAAACTACTTTTTAA
ACATACATTTTGTGATGATTTAACAAAAATATAGAGAATGATTTGCTTTATTGTAATTGTATATAA
GTGACTGGAAAAGCACAAAGAAATAAAGTGGGTTCGATCTGTTTAC

(SEQ ID NO:225)

Homo sapiens DYNC2LI1 3'-UTR
NM 015522.3

AATTCATTTGATGTAGATGAACCTGTTCACTGGAAAATTACAGCAATTTATTAAAACCTCAGTAAG
AGCAAAACAAGGAAGAAGATTCCTTATATCTTCTTGTTAGACATCTTCTGTGATTGTTATGGCATA
TTACACCAATCAGAGAAATAGAGTTTTAAAGTAGTGGTTTGATATTGATTTTATAATCTCTGTAAA
AATGAAGATAAAAAGCCAGATTGTACAAAAGTCACCTGACAAAGACTAGATGAAGCTACAACTTTA
AGCAAGGGGTAGAGTTGTAATAGCCTTCACCATCACTCTGTATTTTACATTCATTTCGTTTCTGTC
ACTTATTCAGTATCTTTTTATCATCTGACAGCTAATTAAATTATAAAGTTGCTATGATGGTAACAC
AAGTTCTTCAAATACAATAATAAATATCATCATCTGGAAAAAAAAAAAAAAAAAA

(SEQ ID NO:226)

Homo sapiens VPS8 3'-UTR
NM_001009921, NM_015303

TGACTCCATGGAGCCTGGCCCAGGAGAACCAGAGATGATCCCGAGGCAGCTGGGGAGAGGCCCCGC
CTCTGGTGGGCTTGGCCTCCACCACCTCCCACGCTTCTGAGAAGAGGTTCCAAATTGGGCTTCTGT
GCCCAGAGCGTCCACAGCACCATTCCCAGTGTAGACTCCCAGTCTTCTCCACATTGCTGTCATGGC
GTCAGTTCACCAGACTCATTGATTTTGTTTTGCTTGTTAAGCAAAGGAATGTCACATACCTCTGTC
CAGCTTTTTAGGAAATACATTTCGCCTATTGCGACTTTTTCCATTTACCCTGAAGCCTAGAAAGTA
GGTGGAACTCACACAAATGGCATTCCAGAGTCTGCCATACTCCGTCTCCTCCAGCTGCTGGATAAT
ACAGAGGAACTTCAACTTCTACAGGGAACAGTGGTTGGCCAGGCTGCAGTATAACTGAAGCATGCC
TTGGAGAGAGCAGACACTGTGGGGGCCAGGGCCATCTCCCTTTAATGTGTTCATGTTAAAACCTAT
TTGAGTGTAAGACTTGCCCTTTCTAACAATAAATGCTCCGTGTTTAAGTTCTGCAGGTCTCAAAAA
AAAAAAAAAAAA

(SEQ ID NO:227)

Homo sapiens ITFG1 3'-UTR
NM 030790

CTTGCCTTTAATATTACATAATGGAATGGCTGTTCACTTGATTAGTTGAAACACAAATTCTGGCTT
GAAAAAATAGGGGAGATTAAATATTATTTATAAATGATGTATCCCATGGTAATTATTGGAAAGTAT
TCAAATAAATATGGTTTGAATATGTCACAAGGTCTTTTTTTTTAAAGCACTTTGTATATAAAAATT
TGGGTTCTCTATTCTGTAGTGCTGTACATTTTTGTTCCTTTGTGGAATGTGTTGCATGTACTCCAG
TGTTTGTGTATTTATAATCTTATTTGCATCATGATGATGGAAAAAGTTGTGTAAATAAAAATAATT
AAAAAAAAAAAAAAAAAAAAAAAAAAAAAA

(SEQ ID NO:228)

Homo sapiens CDK5 3'-UTR
NM_004935

GCCCCGGGACCCCCGGCCTCCAGGCTGGGGCCTGGCCTATTTAAGCCCCCTCTTGAGAGGGGTGAG
ACAGTGGGGGTGCCTGGTGCGCTGTGCTCCAGCAGTGCTGGGCCCAGCCGGGGTGGGGTGCCTGAG
CCCGAATTTCTCACTCCCTTTGTGGACTTTATTTAATTTCATAAATTGGCTCCTTTCCCACAGTCA
AAAAAAAAAAAAAAAAA

(SEQ ID NO:229)

Homo sapiens Clorf112 3'-UTR
BC091516

AACTTATCACTAGGCAGAACTGGGTTTGATGCTTTGTCAACTGAAAATACTTATGTCTGTACATTT
TCTAACAGATATAAAACAAATTTTGTAAAGTTGAAAAAAAAAAAAAAAAAAAAAAAAAAAAAAAA
AAAAAAAAAAAAAA

(SEQ ID NO:230)

Homo sapiens IFT52 3'-UTR
NM_016004

AGACCATGCCTCTTGAAGCTTTTTCTGCCTCCTGATTCTCTCTTTGTAAACTATTTTCAAATTGTT
TTTCAACTCCTTATCAAAATTGTTTATACACTCTTTCCTCCATGAGCTCTGGAAGGTATATGCATC
TTCTGTAATACTCAGATAGGTATAAGATTTTTCACAAAATCCTTATGTAAGATACATTCCATTTTT
AAAAATTAAATGTATGGTTGCATCTGTCTTTTTATACCCTA

(SEQ ID NO:231)

Homo sapiens CLYBL 3'-UTR
CLYBL-003 ENST00000339105

TCTGTTAAATGAAGCTGTCATCAGGCTAAAGGGTATTGAAGCTGCAGAGGGATCAACTTGTGCTTG
CCAGAGGACGCCAATGAAGTTTGAAACACCAACAATCAGAGATTTTGTTTCTGTTCCTCATTAAAT
CATGAGCTTTTGTG

(SEQ ID NO:232)

Homo sapiens FAM114A2 3'-UTR
FAM114A2-006 ENST00000520667

AGAATGGAGACGTTTTGACCTGGGACTTGTGACGGCCAAGGAATGCCACCTTATTCTGGCTACTCC
TGCAGAAATGAAGGAGTGGGGTTATTTTAGTATATAAAAATTCAGGCAGGAGAGATGGTTTAAAGA
GGAAGATTGTTGCCTTCAGTGTTTGATTGAAGTATTCAGGTTCTCACAGTATTCTTTCCAGTTGTT
GTAATTCATAAATTATTTGAAAAGAAACTTTTGTAGAAAGTCCAAGAATAATAACTCTAGATAAAG
ATTAGTGGGACACTCAGGCAAAAATGTTGGTCTTTCTTTGACATGTTGCAAAATGTTATCAATTTT
GTCATGGATATAATTTGCAGCCCATGGATATAACTGGTTGATAAGCCAGAGAAAAATAATTTAGTG
TTCTAAAATTCATGGCATGTGTGGTTTATTAATGCCATGTACTTTCTCCTTTCTGGAATAAAATCT
ATGGCTTTAAGAAAA

(SEQ ID NO:233)

Homo sapiens NUDT7 3'-UTR
NM 001243661

TTTACTAGAGCAAGAGACAAAGAACTATTCACGAGGATTCTGTGTGTGCTTATTCGTAGAACAACA
ACAATGCCAGCTGTTGGAATTTGACAGGTGTGAATATTTTTTCTGCAGTATGTAGTTAGAATCCTT
GCCTCTTTTCCAGTTGCCTTCTATTGTCTGAAAAAGTAAAAGCCATTCAAAAATGAAAACTATGTT
CATAGTGTTGCATATTTTCACCCACAATATGTTAATAATATTTTTCTTACACATATAATAAAGAAT
ATCTGGCACATACTAGGCCCTTAATAAAGATTTTTTGAATATATAA

(SEQ ID NO:234)

Homo sapiens AKD1 3'-UTR
NM_001145128

TTTACTTAGGTGATAGCAGCCTGAATCTCAAGAGTTATCTGAAAGTGATAGAGGGAAACTGAGAGA
AGTAGATTGAAAATCTGGGCCTCTTGGAAGTACTTTTGCCTCCTGAGCAAGGTACCATGGCTGCCA
GACTTCAGGTGAACTCAAAGGTCTGCCAGCCAGGAAGGAGCACTCTTATGGAAACAAGTTTTAATA
CAATTTTAAAATGTATTGCTCTTTGCCTGAACTTTGATGCTTTAACAAAATAAACATTCTATTTAT
AATTCCATATAGAAAAGTTAAGTGACTTATTTAATAAATGTATTATTTTCCTTTTTAACATTTTCA
GTAGAAAAGTCAGTCTCTGTTAAAATTACTCATTAAATGTTAGAAAGCTTTAAGACATTTAACATT
GTTATAAATGAAACCAAAATATGGGTTATACATTTTACATACAAAACTGTTTGTGAACTTTGTGAA
CATAAGATACTATCATTTTCCCAATAAAATAAATGGATTTTGCAACAACTT

(SEQ ID NO:235)

Homo sapiens MAGED2 3'-UTR
NM 014599

GATTTTAGATATTGTTAATCCTGCCAGTCTTTCTCTTCAAGCCAGGGTGCATCCTCAGAAACCTAC
TCAACACAGCACTCTAGGCAGCCACTATCAATCAATTGAAGTTGACACTCTGCATTAAATCTATTT
GCCATTTCAAAAAAAAAAAAAAAAAA

(SEQ ID NO:236)

Homo sapiens HRSP12 3'-UTR
HRSP12-001 ENST00000254878

GTGGGCCCAGTGCTGTGTAGTCTGGAATTGTTAACATTTTAATTTTTACAATTGATGTAACATCTT
AATTAACCTTTTAATTTTCACAATTGATGACAGTGTGAGTTTGATGAAAATATCTGAAGCTATTAT
GGAAATACCATGTAATAGGGAGAGTTGAACATGAATATTAGAGAAGGAATCCAGTTACTTTTTTAA
ATTACACCTGTGTGCACCTGTATTACTGAATATAGGAAAGAGATACCCATTACATAGTTACTCAGT
AAACAAAAGAGAAATACCAGGTAGGAAAGAAGAGTTACTATTCCTGAGAAATAATCAAGAACATAT
TTAATTTAAACTAATGATGTGAACTATTTAGTTTTGATGTCCGTTATGTGATTCTGCTTTTACTTG
AGTAAAATTAAAGTGTTTAAATTTGAGATCAAGGAGAAGATAGTGGAACAAAATGTTATATAGATA
ATATTTTTCTAATGGAAATAAAATAGGCAGATTTCC

(SEQ ID NO:237)

Homo sapiens STX8 NM_004853 3'-UTR

TGGCAGTAAAGAGACCACCAGCAGTGACACCTGCCAATGACAGATGCAAGCCCAACACCCTTTTGG
TACGCAAAACCTGCTCTCAATAAATTCCCCCAAAGCTCTGAAAAAAAAAAAAAAAA

(SEQ ID NO:238)

Homo sapiens ACAT1 3'-UTR
ACAT1-001 ENST00000265838

ACAACCTCTGCTATTTAAGGAGACAACCCTATGTGACCAGAAGGCCTGCTGTAATCAGTGTGACTA
CTGTGGGTCAGCTTATATTCAGATAAGCTGTTTCATTTTTTATTATTTTCTATGTTAACTTTTAAA
AATCAAAATGATGAAATCCCAAAACATTTTGAAATTAAAAATAAATTTCTTCTTCTGCTTTTTTCT
TGGTAACCTTGAAAAGTTTGATACATTTTTGCATTCTGAGTCTATACTTATCGAAATATGGTAGAA
ATACCAATGTGTAATATTAGTGACTTACATAAGTAGCTAGAAGTTTCCATTTGTGAGAACACATTT
ATATTTTTGAGGATTGTTAAAGGTCAAGTGAATGCTCTTTATAGGTAATTTACATT

(SEQ ID NO:239)

Homo sapiens IFT74 3'-UTR
IFT74-201 ENST00000433700

GTTTAAGTCCACTGAAAGTCTCTAAGGAAGTATCCTCTTGCTGCTAAACTTGGTACAAGTTGACTA
CCAAAAAAAAAAAAAGCTTACTTTTGGAGTTTACCTAAAATTTCTGAATGTTATAATTTTTGTGGC
CTCTTTTAAGAATGATATTTTAAAATAGTAAATAGTTCAATAAATGGTTTGCATATT

(SEQ ID NO:240)

Homo sapiens KIFAP3 3'-UTR
NM_014970

TAAAGTATCTGTTTCCATGTGTAATCTCAGCTTAGAAGAAATCTGTGTGGGTTGGGTTAATTTTGG
ATCTTTGCCTAATAATGCATGTTGATGTTATTGTGGGTCTGTGTTTGTTTTTATTTTTATATGTTG
TTAGCTGCAGATTAACCCCAGCCCCTCTGTCTTCTGTTAAGTACAGTTGATACTGACATTGTTCAC
TCATCAAACCACATCTTGATGCTAAGTAACATTTCCCATGAGCCACAAAACTGAATGCTGAAAAGC
TACTAGACTGGAAAACAAACACTGCATTATGTATGTTAAGTGACTAATTTAATTTCAATTAAAAAG
CGTAAAGTGAAAATGAAAAAAAAAAAAAAAA

(SEQ ID NO:241)

Homo sapiens CAPN1 3'-UTR
NM 005186

GGCAGGGACTCGGTCCCCCTTGCCGTGCTCCCCTCCCTCCTCGTCTGCCAAGCCTCGCCTCCTACC
ACACCACACCAGGCCACCCCAGCTGCAAGTGCCTTCCTTGGAGCAGAGAGGCAGCCTCGTCCTCCT
GTCCCCTCTCCTCCCAGCCACCATCGTTCATCTGCTCCGGGCAGAACTGTGTGGCCCCTGCCTGTG
CCAGCCATGGGCTCGGGATGGACTCCCTGGGCCCCACCCATTGCCAAGCCAGGAAGGCAGCTTTCG
CTTGTTCCTGCCTCGGGACAGCCCCGGGTTTCCCCAGCATCCTGATGTGTCCCCTCTCCCCACTTC
AGAGGCCACCCACTCAGCACCACCGGCCTGGCCTTGCCTGCAGACTATAAACTATAACCACTAGCT
CGACACAGTCTGCAGTCCAGGCGTGTGGAGCCGCCTCCCGGCTCGGGGAGGCCCCGGGGCTGGGAA
CGCCTGTGCCTTCCTGCGCCGAAGCCAACGCCCCCTCTGTCCTTCCCTGGCCCTGCTGCCGACCAG
GAGCTGCCCAGCCTGTGGGCGGTCGGCCTTCCCTCCTTCGCTCCTTTTTTATATTAGTGATTTTAA
AGGGGACTCTTCAGGGACTTGTGTACTGGTTATGGGGGTGCCAGAGGCACTAGGCTTGGGGTGGGG
AGGTCCCGTGTTCCATATAGAGGAACCCCAAATAATAAAAGGCCCCACATCTGTCTGTGAAAAAAA
AAAAAAAAAAAAAAAAAAAAAAAAAAAAAAAAAAAAAAAAAAAAAAAAAAAAAAAAA

(SEQ ID NO:242)

Homo sapiens COX11 3'-UTR
NM 001162861

AGAGTTGGCACCTTTGATGTGGTAGTGAGCTGATCATCCACTTTCTTCTAAAATAAAGAGAAGAAA
ATGGCCAGTAAAAAAAAAAAAA

(SEQ ID NO:243)

Homo sapiens GLT8D4 3'-UTR
BC127733

ATATTTTGTCTTGTTGCAAGTCAATTAGGTGTCTTGTGAACAAGGAAATACTAATCTCTAAGCTGC
CTGGGTCTTTT

(SEQ ID NO:244)

Homo sapiens GLT8D4 3'-UTR
NM_001080393

ATATTTTGTCTTGTTGCAAGTCAATTAGGTGTCTTGTGACCAAGGAAATACTAATCTCTAAGCTGC
CTGGGTCTTTTTGTGTGAATATTTAATGGTGCTCCATGACTGTTGAGTTTTAAAAACCTCGTTAAA
TTTTGCCAAATCAGTTGCCCCCAAAAGGGAATATGCTTTTCCTTATTTTTTTTTCTAAAATGCTAT
TTATCTCTAAGGAAAAA

(SEQ ID NO:245)

Homo sapiens HACL1 3'-UTR
NM_012260

ATAAAGACGCCAGTTGGTGGTCTTGAGTTTTCTCTTTCTTGCAAGATGAAATTTTATTTTCCACAG
CAAAATTACTCTACTGTTAAAATTGTGCAAAATAAAATAAACATTTAAAATGACATTTTACAGTAA
AAAAAAAA

(SEQ ID NO:246)

Homo sapiens IFT88 3'-UTR
NM 175605

TATTCACTTTAATATTTATTAAAGGAAAGAAATTGCCTTATGAGATCATCCTCATGTTAAACCTTG
GATTAAATATCTAACCTGTAATTATTTTTTTTCACTGTCAAAACTTAAGTAAGTGTATTCTATTCT
GTATGTATGCATTTAAGTTGTTTTTTTCTTTTAAGGAATAAAAACAGGTAAAACTAATACTTTAGG
CCAGTGACTTCCTTAGCTTTTTGAAAACATTGACACACAGGAAGAAATAAATTTCATAACACAAAA
AAAAAAAAA

(SEQ ID NO:247)

Homo sapiens IFT88 3'-UTR
IFT88-001 ENST00000351808

TATTCACTTTAATATTTATTAAAGGAAAGAAATTGCCTTATGAGATCATCCTCATGTTAAACCTTG
GATTAAATATCTAACCTGTAATTATTTTTTTTCACTGTCAAAACTTAAGTAAGTGTATTCTATTCT
GTATGTATGCATTTAAGTTGTTTTTTTCTTTTAAGGAATAAAAACAGGTAAAACT

(SEQ ID NO:248)

Homo sapiens NDUFB3 3'-UTR
NM_002491

AGATAATACCTGGAAGCATCATAGTGGTTTCTTAACTCTCCAAAATAAGATTTCTTCTCTGTAGCC
TACTTGTCTGGTTTATCCCTTACAGAATATTAGTAAGATTTAATCAATTAAAATATATATATATGC
CAAAAAAAAAAAAAAAAA

(SEQ ID NO:249)

Homo sapiens ANO10 3'-UTR
NM_018075

GTGCCCAGCGTGCCCAGCTGCCCTGTTGGCAGAGGCCTGTGTCTGTGCCACACCTGCCACGGTGGC
AGGGGGGGTACCCGGGGCAGCATCGTGGCTCCTGAACCCAGACCCAATGCTTAGCCAAACGAAGTG
GCTCCCATGTGGCAAGCACCCTTCTCAGTTTCGCAGTGGCTTGGCTCGGGATCCTTGGCAGTTCCC
CCAGCCCCACCCTGTCTGCTCCTTCCCAGTTCCTTCCCGGGCCCCACACGCTGCTCCAGCTGCCAA
CTTTGCTGCAGAGCCACTGCCGCCCTTGAGCCTCTCACCATGAGTGAGCCACCAGCTCTCCACGTT
CCCCTCATAGCAGTGTCACTCCCAACCCCACCATGGCCCAGGGACCCGTGGACAGGTTGGGGATGG
GGTGTGTGCCCACTGTGCTCATCACAGGAGCCTCAGTTGAGAGTGAGCGGGGTACAGTAAGGCAGT
GCTTCCCACACTGGACCTCTTTCCTGGTTCTCTTTTGCAATACATTAACAGACCCTTTATCAACAT
AAACAATAGTAACTGAGCTATTAAAGGCAACCTCTCTGACTCCTTCTGCCTAAAAAAAAAA

(SEQ ID NO:250)

Homo sapiens ANO10 3'-UTR
ANO10-005 ENST00000451430

GTGCCCAGCGTGCCCAGCTGCCCTGTTGGCAGAGGCCTGTGTCTGTGCCACACCTGCCACGGTGGC
AGGGGGGGTACCCGGGGCAGCATCGTGGCTCCTGAACCCAGACCCAATGCTTAGCCAAACGAAGTG
GCTCCCATGTGGCAAGCACCCTTCTCAGTTTCGCAGTGGCTTGGCTCGGGATCCTTGGCAGTTCCC
CCAGCCCCACCCTGTCTGCTCCTTCCCAGTTCCTTCCCGGGCCCCACACGCTGCTCCAGCTGCCA

(SEQ ID NO:251)

Homo sapiens ARL6 3'-UTR
NM 032146

AAAGATAATAGTTGGAAACCTCAGCAATTTTCAATTCAAGGAATCTATCTAAGACAAATAGAATAC
ATTTTGTAAAAGATGTTTATGCATCAAAAAATATAATTTTCTGCTTGCATTTATGGACTCTGACCT
TTTTAAGAACATAGGACTTCAGGTATGCTAATTTGGCCATTAATTATTTAAAAACTAAATATTCCC
TCAAAAGGGCTCCCTAGAATTATCAAGTTCTTAGTGAAGGTCTACATTTGATTGTACGTAGAATGT
TTAAAAGTCAGTTATAAGCCATCTCATCCCATCATAATTTATGATATGTTTAATATATTTTATTTT

TTAATTGTCTTTTTAAAAAAATTTAGTTTATGACTTTGCAGTATGAATTGTGCTTGTGAAAAAGAAC
TTTAAATATTTATAAGGGACCATGGGTAATTAATATATATTCAATTTTTACTATGTGTCACTGTCA
ATAAAATGTAAAATATAATGTGCC

(SEQ ID NO:252)

Homo sapiens LPCAT3 3'-UTR
NM_005768

TCCATTTCCCTGGTGGCCTGTGCGGGACTGGTGCAGAAACTACTCGTCTCCCTTTTCACAGCACTC
CTTTGCCCCAGAGCAGAGAATGGAAAAGCCAGGGAGGTGGAAGATCGATGCTTCCAGCTGTGCCTC
TGCTGCCAGCCAAGTCTTCATTTGGGGCCAAAGGGGAAACTTTTTTTTGGAGAAGGCGTCTTGCTT
TGTCACCCACGCTGGAATGCAGTGGCGGGATCTCAGCTCACCGCAACCTCCACCTCCTGGGTTCAA
GTGATTTTCCTGCCTCAGCCTCCCAAGTAGCTGGGAATACAGGCACGCCACCATGCCCAGCTAATT
TTTGTATTTTCAGTAGAAACGGGATTTCACCACGTTGGCCAGGCTGGTCTCGAACTCCTGACCGCA
AGTGATCCACCCGCCTCCGCCTCCCAAAGTGCTGGGATTACAGGCGTGAGCCACCGTGCCCGGCCC
AAAGGGGAAACTCTTGTGGGAGGAGCAGAGGGGCTCACATCTCCCCTCTGATTCCCCCATGCACAT
TGCCTTATCTCTCCCCATCTAGCCAGGAATCTATTGTGTTTTTCTTCTGCCAATTTACTATGATTG
TGTATGTGCCGCTACCACCACCCCCCCCATGGGGGGGTGGAGAGGGGTGCAAGGCCCTGCCTGCTC
CACTTTTTCTACCTTGGAACTGTATTAGATAAAATCACTTCTGTTTGTTCAGTTTTTCA

(SEQ ID NO:253)

Homo sapiens ABCD3 3'-UTR
NM_001122674

AAACCAGACAAATGTATTGGCCAGGCGTGGTGGCTCATGCCTGTAATCCCAGCACTTTGGGAGGCT
GAGATGGGAGGATCGCTTGAATCCAGGAGTTCGAGACAAGCCTGGACAAAAAGCGAGACCCGCTTC
TTTAAAAAATAATAATAAAACA

(SEQ ID NO:254)

Homo sapiens COPG2 3'-UTR
NM_012133

ATGCTTACTGGACAAGAGGAAACTGATGCACACTACATGGTCAGTGGGCTTTTAGGCTAGTGGCAT
CAGTTTCCCAGAATCAGACTTTTGAAGATGAATGACTTTGGAGAAGCAAATTAAACATTTGGCCCT
GAGCCAGCAGATCAAGCAAATGTCTATCTTTGCGCATGGGTTGTTTTTTTTTTTTTTCTTTTTATT
CTACTTGGTCAGCTTTGGGACGATAGTGCAGCTTTGGGTGATCTTGAAAATCAAATACTATCCTAT
ACTCCAGCTGCTTAACTTCATTTTATTCTTTAATGTGTACCTGAAAGCTCCTGGCAATGCTGGAAA
ATTTTTATCCCAGAGGGGTGGGGGGGAGGGGGGAGGGGAAGCCAGAGTCCACTTTTGTCACAATTC
ATTTTTATTAATAGAAAATAAACACTTATTCCAGTTTCAAAAAAAAAAAAAA

(SEQ ID NO:255)

Homo sapiens MIPEP 3'-UTR
NM_005932

AAGAAACACTCTACACCTCTTAAATCAAGGTCATGTAGATAATGACTTTGTTATAAATGCTACAGC
TGTGAGAGCTTGTTTCTGATTTCATTGTTCGCTTCTGTAATTCTGAAAAACTTTAAACTGGTAGAA
CTTGGAATAAATAATTTGTTTTAATTAAAAAAAAAAAAAAAAAA

(SEQ ID NO:256)

Homo sapiens LEPR 3'-UTR
NM 002303

TTTCACTGAAGAAACCTTCAGATTTGTGTTATAATGGGTAATATAAAGTGTAATAGATTATAGTTG
TGGGTGGGAGAGAGAAAAGAAACCAGAGTCAAATTTGAAAATAATTGTTCCAAATGAATGTTGTCT
GTTTGTTCTCTCTTAGTAACATAGACAAAAAATTTGAGAAAGCCTTCATAAGCCTACCAATGTAGA
CACGCTCTTCTATTTTATTCCCAAGCTCTAGTGGGAAGGTCCCTTGTTTCCAGCTAGAAATAAGCC
CAACAGACACCATCTTTTGTGAGATGTAATTGTTTTTTCAGAGGGCGTGTTGTTTTACCTCAAGTT

TTTGTTTTGTACCAACACACACACACACACACACATTCTTAACACATGTCCTTGTGTGTTTTGAGAGT
ATATTATGTATTTATATTTTGTGCTATCAGACTGTAGGATTTGAAGTAGGACTTTCCTAAATGTTT
AAGATAAACAGAATTC

(SEQ ID NO:257)

Homo sapiens LEPR 3'-UTR
NM_001198688
GAAATGCTTGTAGACTACGTCCTACCTCGCTGCCGCACCTGCTCTCCCTGAGGTGTGCACAATG
(SEQ ID NO:258)

Homo sapiens C2orf76 3'-UTR

NM_001017927
AAACATCTCGAGGGCTTCCTTTTTGCAT
(SEQ ID NO:259)

Homo sapiens C2orf76 3'-UTR
C2orf76-001 ENST00000409466

AAACATCTCGAGGGCTTCCTTTTTGCATACCTGTATTAAGCTCTTTATTCCACTGCTGAATTTTTG
AAATTGACAAACAAATCTTAAAAAATTAATCCCAGGCTATACTCTTTGAGCTAAAATCTGGTTATT
TCTTTCTCTTCAGGTCTTTCCTTCTCTCTTTCTTTTTCTTTGTTGTTGTAAAATAATATATTATGA
GAAAAACATTTGATCTTTTTAAAGGGAAATAAATTGTTATTAAAAA

(SEQ ID NO:260)

Homo sapiens ABCA6 3'-UTR
NM 080284.2

AACCTCAAACCTAGTAATTTTTTGTTGATCTCCTATAAACTCATGTTTTATGTAATAATTAATAGT
ATGTTTAATTTTAAAGATCATTTAAAATTAACATCAGGTATATTTTGTAAATTTAGTTAACAAATA
CATAAATTTTAAAATTATTCTTCCTCTCAAACATAGGGGTGATAGCAAACCTGTGATAAAGGCAAT
ACAAAATATTAGTAAAGTCACCCAAAGAGTCAGGCACTGGGTATTGTGGAAATAAAACTATATAAA
CTT

(SEQ ID NO:261)

Homo sapiens LY96 3'-UTR
NM_015364.4
AATAAATTGAGTATTTAAAAAAAAAAAAAAAAAAAAAAAAAAAAA
(SEQ ID NO:262)

Homo sapiens CROT 3'-UTR
NM 001243745.1

TGATGATGTTTAAAGAATGATAAATAAAAAGTGCATAGTTTTTATTTTTAAATTATTGCTGTAAAA
ATTTTTACAGTTATTATTGTTATTTTCATAATCCAAAAGAAGGAATGAATCACTTAACTTTGGGAG
TTTTCAGTGGGTGGATTCGGGAACTTGTTAAAATGCAGATTTGCTGGGATAAGTGATTCTGATTCA
CATGGCTGGAATGAGGCCCAGAGATTCTTATTTTAACAATCACTTCATGTGGTTTGGCTGCAGGTA
ATCTGTAGACCATGCTGAAGGAAAACATTTTGTCCAGGTGACTAGCTTGAAAAATCAGAAACACTA
AAATAGACATGTCACATAGGTGGCATAGAAATATTTTCGTAGTACAATGGAGAAAGGGAATCATTA
AAAATCAGAGTGGAGAATGGTTATGTATATTGTATATTTCAGTTAGATAAATTGAGGAAGCTAGTA
TAATAATTATTGAAGGTCTCAATAATTTTCCACAAAATTCTTTAACTTCTTCAGCTCAACCATTTC
TGTACTTCTCTACTATGAATCAGAGGATGAGGTTGTATAATTCAAAAGCATTGCCTTAGTCTAGAA
ATAATTATTGTACCTATCATTTAGTTTTAGAAATAAAAAGCAAGCTGATTTTTTTTGATGAACCAT
TTATATCTGTGATGGAATAATAAAATTTCACACTTCCGGATTCCTTTGTTCTCAATTTTGAGCCTT
GAGTTGTTTTAATTAAAGAGGGGTAAAGG

(SEQ ID NO:263)

Homo sapiens ENPP5 3'-UTR
ENPP5-002 ENST00000230565

TGTTACTTTGAAGTGGATTTGCATATTGAAGTGGAGATTCCATAATTATGTCAGTGTTTAAAGGTT
TCAAATTCTGGGAAACCAGTTCCAAACATTTGCAGAAACCATTAAGCAGTTACATATTTAGGTATA
CACACACACACACACACACATACACACACACGGACCAAAATACTTACACCTGCAAAGGAATAAAGA
TGTGAGAGTATGTCTCCATTGTTCACTGTAGCATAGGGATAGATAAGATCCTGCTTTATTTGGACT
TGGCGCAGATAATGTATATATTTAGCAACTTTGCACTATGTAAAGTACCTTATGTATTGCACTTTA
AATTTCTCTCCTGATGGGTACTTTAATTTGAAATGCACTTTATGCACAGTTATGTCTTATAACTTG
ATTGAAAATGACAACTTTTTGCACCCATGTCACAGAATACTTGTTACGCATTGTTCAAACTGAAGG
AAATTTCTAATAATCCCGAATAATGAACGTAGAAATCTATCTCCATAAATTGAGAGAAGAAGAAGG
TGATAAGTGTTGAAAATTAAATGTGATAACCTTTGAACCTTGAATTTTGGAGATGTATTCCCAACA
GCAGAATGCAACTGTGGGCATTTCTTGTCTTATTTCTTTCCAGAGAACGTGGTTTTCATTTATTTT
TCCCTCAAAAGAGAGTCAAATACTGACAGATTCGTTCTAAATATATTGTTTCTGTCATAAAATTAT
TGTGATTTCCTGATGAGTCATATTACTGTGATTTTCATAATAATGAAGACACCATGAATATACTTT
TTTTCTATATAGTTCAGCAATGGCCTGAATAGAAGCAACCAGGCACCATCTCAGCAATGTTTTCTC
TTGTTTGTAATTATTTGCTCCTTTGAAAATTAAATCACTATTAATTACATTAA

(SEQ ID NO:264)

Homo sapiens SERPINB7 3'-UTR
SERPINB7-203 ENST00000546027

AAATCCAATTGGTTTCTGTTATAGCAGTCCCCACAACATCAAAGAACCACCACAAGTCAATAGATT
TGAGTTTAATTGGAAAAATGTGGTGTTTCCTTTGAGTTTATTTCTTCCTAACATTGGTCAGCAGAT
GACACTGGTGACTTGACCCTTCCTAGACACCTGGTTGATTGTCCTGATCCCTGCTCTTAGCATTCT
ACCACCATGTGTCTCACCCATTTCTAATTTCATTGTCTTTCTTCCCACGCTCATTTCTATCATTCT
CCCCCATGACCCGTCTGGAAATTATGGAGAGTGCTC

(SEQ ID NO:265)

Homo sapiens TCP11L2 3'-UTR
NM_152772

AGAAGAACTGACATTGGACGAGAGATTGGAAATCCAGTACTTTGGTATCCAGTCCACTTCCATTGA
TGGCATTAGAGATCCAGCACATTCTCAGTACTGTGGTGCAGTATTAGCCCAAATCTGTGTAATGGG
TAATATTAGCATTACAGAAGACACACACATCACATAGACCCTCAGAAGACGTAAACATCACATAGA
CCCTATTTGTGCATCATTTTCAAGTTTAAAACAGATATTTGTAATGAACAGAAAACAATTTGTAAT
TAATTATATTACCTATATAATACTTGTAAATGTTTTCTTAACCATTTATATTTGGCTTATGACATT
TAACCCCTAAGGAGTTGTTTTTCTCACTTGTTATTATCAAACCTAATGGTTTTTAATTTTGGTACA
ACTCCTTAAAGGGTTGAAGGTTGTGACAATAACTGAGGGAACTGATGTTCTGAATAAATGATGTGA
AGTAAACACAATTGTATTTGAAAAAAAAAAAAAAAAAAAAAAA

(SEQ ID NO:266)

Homo sapiens IRAK1BP1 3'-UTR
NM_001010844

AATTCCAAACAAATTATATTGTACTTGTATCTTTTTACCTATTTTTATACTTTTTATAATGTTTAC
GTTTGTCCTGAATATATA

(SEQ ID NO:267)

Homo sapiens CDKL2 3'-UTR
CDKL2-002 ENST00000307465

GAACCATTTTGGTTCTGAACTGGATGATGCTCTTGCACTTGAGATGACATCTTCTTGCAGCAAGAG
TGCTGATATCCCAAGAGGAGAGATTCATGGTTTTGATCATTTCCTTCTGAACTGCCTGCATTTTCT
GAGGAAGGCCTTCTAGAAGAAGGAAAGACAAAGACTTCCAAATGTTTCAAAGGAAGATTGAACAAA
TGGCCCTCCCCAACTGTTATCCCATTACCTTTCACGTCCACCGATGCTATTTCAAGACATATCCAG

TGGAATAACAGTGATATGGTTCTTGTTACATGAATGTGTATTTACTGTTAGGAGATTGTATATTTT
AAGTTACC

(SEQ ID NO:268)

Homo sapiens GHR 3'-UTR
GHR-202 ENST00000537449

CCTTTCTTTGGTTTCCCAAGAGCTACGTATTTAATAGCAAAGAATTGACTGGGGCAATAACGTTTA
AGCCAAAACAATGTTTAAACCTTTTTTGGGGGAGTGACAGGATGGGGTATGGATTCTAAAATGCCT
TTTCCCAAAATGTTGAAATATGATGTTAAAAAAATAAGAAGAATGCTTAATCAGATAGATATTCCT
ATTGTGCAATGTAAATATTTTAAAGAATTGTGTCAGACTGTTTAGTAGCAGTGATTGTCTTAATAT
TGTGGGTGTTAATTTTTGATACTAAGCATTGAATGGCTATGTTTTTAATGTATAGTAAATCACGCT
TTTTGAAAAAGCGAAAAAATCAGGTGGCTTTTGCGGT

(SEQ ID NO:269)

Homo sapiens KIAA1107 3'-UTR
NM 015237

GTGTTAACATTTTGGAAAAATTTATGCCACTCCTTTATTTTTTGATGCCTATATTATATCCAAATG
ATAATTGCATTAGCCGGATATAAACTTTCTTTAATATTGAGTCTTTCCAATTTAATGAGGTAAACA
TAGTTTATTTATTAATATATCACATATAGAAAAATGTTTTTCTAAAGTTTTTGAGCATGTTTTCTC
TAATTATTAGAGAAATTAGAAGACTTATAAGGAAACCCTAGCTTCAGTTTTCCTTTCCTAGCTGAT
GATTTGTTCACTTAATCATTATTCAAGAATTTAAAATGTGAATGCAGAAGTAGATCAGTCCCTTTA
CTTTTTGCTCTGCATAGGGTAACATAGTAATTTAACAATAAAAACTTACCGTGCTTGTGTCCAAAA
AAAAAAAAAA

(SEQ ID NO:270)

Homo sapiens RPS6KA6 3'-UTR
RPS6KA6-001 ENST00000262752

GATTTGTGGTGTTCCTAGGCCAAACTGGATGAAGATGAAATTAAATGTGTGGCTTTTTTCCTATTC
TTATCAAAGGCATCGTTGTCTGCTAAATTACTTGAATATTAAGTAATATTAAATCCCCATTTTTAG
GGGAAGTGAGATTTAAAAAACCATTCACAGGTCCACAATATTCATACTATGTGTTTGCAGTAGTGT
TCAAGTGTTTATTTAAGCATATAATTGGTGTCCACCAGGTCCTCACAACTTCTCTGCACACAAGCT
TCTAAAATTCCTTTCAAATAAAGTTACTTTAATATTT

(SEQ ID NO:271)

Homo sapiens CLGN 3'-UTR
NM 004362, NM 001130675

ACTAGATTGAAATATTTTTAATTCCCGAGAGGGATGTTTGGCATTGTAAAAATCAGCATGCCAGAC
CTGAACTTTAATCAGTCTGCACATCCTGTTTCTAATATCTAGCAACATTATATTCTTTCAGACATT
TATTTTAGTCCTTCATTTCAGAGGAAAAAGAAGCAACTTTGAAGTTACCTCATCTTTGAATTTAGA
ATAAAAGTGGCACATTACATATCGGATCTAAGAGATTAATACCATTAGAAGTTACACAGTTTTAGT
TGTTTGGAGATAGTTTTGGTTTGTACAGAACAAAATAATATGTAGCAGCTTCATTGCTATTGGAAA
AATCAGTTATTGGAATTTCCACTTAAATGGCTATACAACAATATAACTGGTAGTTCTATAATAAAA
ATGAGCATATGTTCTGTTGTGAAGAGCTAAATGCAATAAAGTTTCTGTATGGTTGTTTGATTCTAT
CAACAATTGAAAGTGTTGTATATGACCCACATTTACCTAGTTTGTGTCAAATTATAGTTACAGTGA
GTTGTTTGCTTAAATTATAGATTCCTTTAAGGACATGCCTTGTTCATAAAATCACTGGATTATATT
GCAGCATATTTTACATTTGAATACAAGGATAATGGGTTTTATCAAAACAAAATGATGTACAGATTT
TTTTTCAAGTTTTTATAGTTGCTTTATGCCAGAGTGGTTTACCCCATTCACAAAATTTCTTATGCA
TACATTGCTATTGAAAATAAAATTTAAATATTTTTTCATCCTGAAAAAAAA

(SEQ ID NO:272)

Homo sapiens CLGN-202 3'-UTR
NM_004362, NM_001130675
ENST00000325617

ACTAGATTGAAATATTTTTAATTCCCGAGAGGGATGTTTGGCATTGTAAAAATCAGCATGCCAGAC
CTGAACTTTAATCAGTCTGCACATCCTGTTTCTAATATCTAGCAACATTATATTCTTTCAGACATT
TATTTTAGTCCTTCATTTCAGAGGAAAAAGAAGCAACTTTGAAGTTACCTCATCTTTGAATTTAGA
ATAAAAGTGGCACATTACATATCGGATCTAAGAGATTAATACCATTAGAAGTTACACAGTTTTAGT
TGTTTGGAGATAGTTTTGGTTTGTACAGAACAAAATAATATGTAGCAGCTTCATTGCTATTGGAAA
AATCAGTTATTGGAATTTCCACTTAAATGGCTATACAACAATATAACTGGTAGTTCTATAATAAAA
ATGAGCATATGTTCTGTTGTGAAGAGCTAAATGCAATAAAGTTTCTGTATGGTTGTTTGATTCTAT
CAAC

(SEQ ID NO:273)

Homo sapiens TMEM45A 3'-UTR
NM_018004

CTTTGATGAGCTTCCAGTTTTTCTAGATAAACCTTTTCTTTTTTACATTGTTCTTGGTTTTGTTTC
TCGATCTTTTGTTTGGAGAACAGCTGGCTAAGGATGACTCTAAGTGTACTGTTTGCATTTCCAATT
TGGTTAAAGTATTTGAATTTAAATATTTTCTTTTTAGCTTTGAAAATATTTTGGGTGATACTTTCA
TTTTGCACATCATGCACATCATGGTATTCAGGGGCTAGAGTGATTTTTTTCCAGATTATCTAAAGT
TGGATGCCCACACTATGAAAGAAATATTTGTTTTATTTGCCTTATAGATATGCTCAAGGTTACTGG
GCTTGCTACTATTTGTAACTCCTTGACCATGGAATTATACTTGTTTATCTTGTTGCTGCAATGAGA
AATAAATGAATGTATGTATTTTGGTGC

(SEQ ID NO:274)

Homo sapiens TBC1D8B 3'-UTR
TBC1D8B-007 ENST00000276175

ATCCCTAGGAATTGCCTATCATAGACAAGTTTACTAACATTCCTGTAGCTGTCAGTTTGATTCCTG
TGAGTAGGGCTCAGGGATTTATCTTGTTACCAATGTGTCTGAAGGCCAAAATATATATCCAGAAGC
ACAATGCATCATTCCTTTGT

(SEQ ID NO:275)

Homo sapiens ACP6 3'-UTR
NM_016361

CTGATTTATAAAAGCAGGATGTGTTGATTTTAAAATAAAGTGCCTTTATACAATGCCAAAAAAAA
AAAAAAAAAAAAAAA

(SEQ ID NO:276)

Homo sapiens RP6-213H19.1 3'-UTR
MST4-003 (RBM4B-003 ENST00000496850)

GAAACTTATTATTGGCTTCTGTTTCATATGGACCCAGAGAGCCCCACCAAACCTACGTCAAGATTA
ACAATGCTTAACCCATGAGCTCCATGTGCCTTTTGGATCTTTGCA

(SEQ ID NO:277)

Homo sapiens SNRPN 3'-UTR
NM 022807

CATACTGTTGATCCATCTCAGTCACTTTTTCCCCTGCAATGCGTCTTGTGAAATTGTGTAGAGTGT
TTGTGAGCTTTTTGTTCCCTCATTCTGCATTAATAATAGCTAATAATAAATGCATAGAGCAATTAA
ACTGTG

(SEQ ID NO:278)

Homo sapiens GLRB 3'-UTR
GLRB-005 ENST00000512619

GATCTAATGACTTCAGCATTGTTGGAAGCTTACCAAGAGATTTTGAACTATCCAATTATGACTGCT
ATGGAAAACCCATTGAAGTTAACAACGGACTTGGGAAATCTCAGGCTAAGAACAACAAGAAGCCTC
CCCCTGCGAAACCTGTTATTCCAACAGCAGCAAAGCGAATTGATCTTTATGCAAGAGCATTGTTTC
CTTTCTGCTTCTTGTTCTTCAATGTTATATATTGGTCTATATATTTATGATAAATCTTTTCCATTT
GTACAAAATAAAATTCCATTTCATTGTGACCTACTCCTTTCATAAATGCCAATCTGTGAGAACTTT
TGAATTTTCATAGCAACATTGCATTTTGGATGCCATTTGATTGTAATAAAACTGTGGCACCTTAAT
TTTGAATGGCAGCATGATCATGTAATATC

(SEQ ID NO:279)

Homo sapiens HERC6 3'-UTR
NM 017912

TCACCTCTGAGAGACTCAGGGTGGGCTTTCTCACACTTGGATCCTTCTGTTCTTCCTTACACCTAA
ATAATACAAGAGATTAATGAATAGTGGTTAGAAGTAGTTGAGGGAGAGATTGGGGGAATGGGGAGA
TGATGATGATGGTCAAAGGGTGCAAAATCTCACACAAGACTGAGGCAGGAGAATAGGGTACAGAGA
TAGGGATCTAAGGATGACTTGGACACACTCCCTGGCACTGAAGAGTCTGAACACTGGCCTGTGATT
GGTCCATTCCAGGACCTTCATTTGCATAAGGTATCAAACCACATCAGCCTCTGATTGGCCATGGGC
CAGACCTGCACTCTGGCCAATGATTGGTTCATTCCAGGACATTCATTTGCATAAGGAGTCAAACCA
CACCAGTCTTGGATTGGCTGTGAGCCAATTCACCTCAGTCTCTAATTGGCTGTGAGTCAGTCTTTC
ATTTACATAGGGTGTAACCATCAAGAAACCTCTACAGGGTACTTAAGCCCCAGAAGATTTTGCTAC
CAGGGCTCTTGAGCCACTTGCTCTAGCCCACTCCCACCCTGTGGAATGTACTTTCACTTTTGCTGC
TTCACTGCCTTGTGCTCCAATAAATCCACTCCTTCACCACCCAAAAAAAAAAAAAAA

(SEQ ID NO:280)

Homo sapiens CFH 3'-UTR
NM_000186

AATCAATCATAAAGTGCACACCTTTATTCAGAACTTTAGTATTAAATCAGTTCTCAATTTCATTTT
TTATGTATTGTTTTACTCCTTTTTATTCATACGTAAAATTTTGGATTAATTTGTGAAAATGTAATT
ATAAGCTGAGACCGGTGGCTCTCTTCTTAAAAGCACCATATTAAATCCTGGAAAACTAAAAAAAAA
AAAAAAAAAAAAAAAAAAAAAAAAAAAAAAAAAAAAAAAAAAAAAAAAAAAAAAAAAAAAAAAAAA

(SEQ ID NO:281)

Homo sapiens GALC 3'-UTR
GALC-002 ENST00000393569

TACTTAACAGGGCATCATAGAATACTCTGGATTTTCTTCCCTTCTTTTTGGTTTTGGTTCAGAGCC
AATTCTTGTTTCATTGGAACAGTATATGAGGCTTTTGAGACTAAAAATAATGAAGAGTAAAAGGGG
AGAGAAATTTATTTTTAATTTACCCTGTGGAAGATTTTATTAGAATTAATTCCAAGGGGAAAACTG
GTGAATCTTTAACATTACCTGGTGTGTTCCCTAACATTCAAACTGTGCATTGGCCATACCCTTAGG
AGTGGTTTGAGTAGTACAGACCTCGAAGCCTTGCTGCTAACACTGAGGTAGCTCTCTTCATCTTAT
TTGCAAGCGGTCCTGTAGATGGCAGTAACTTGATCATCACTGAGATGTATTTATGCATGCTGACCG
TGTGT

(SEQ ID NO:282)

Homo sapiens GALC 3'-UTR
GALC-005 ENST00000393568
TACTTAACAGGGCATCATAGAATACTCTGGATTTTCTTCCCTTCTTTTTGG
(SEQ ID NO:283)

Homo sapiens PDE1A 3'-UTR
NM_001003683.2

ACACCTTTAAGTAAAACCTCGTGCATGGTGGCAGCTCTAATTTGACCAAAAGACTTGGAGATTTTG
ATTATGCTTGCTGGAAATCTACCCTGTCCTGTGTGAGACAGGAAATCTATTTTTGCAGATTGCTCA
ATAAGCATCATGAGCCACATAAATAACAGCTGTAAACTCCTTAATTCACCGGGCTCAACTGCTACC
GAACAGATTCATCTAGTGGCTACATCAGCACCTTGTGCTTTCAGATATCTGTTTCAATGGCATTTT
GTGGCATTTGTCTTTACCGAGTGCCAATAAATTTTCTTTGAGCAGCTAATTGCTAATTTTGTCATT
TCTACAATAAAGCTTGGTCCACCTGTTTTC

(SEQ ID NO:284)

Homo sapiens PDE1A 3'-UTR
PDE1A-003 ENST00000410103

ACACCTTTAAGTAAAACCTCGTGCATGGTGGCAGCTCTAATTTGACCAAAAGACTTGGAGATTTTG
ATTATGCTTGCTGGAAATCTACCCTGTCCTGTGTGAGACAGGAAATCTATTTTTGCAGATTGCTCA
ATAAGCATCATGAGCCACATAAATAACAGCTGTAAACTCCTTAATTCACCGGGCTCAACTGCTACC
GAACAGATTCATCTAGTGGCTACATCAGCACCTTGTGCTTTCAGATATCTGTTTCAATGGCATTTT
GTGGCATTTGTCTTTACCGAGTGCCAATAAATTTTCTTTGAGCA

(SEQ ID NO:285)

Homo sapiens GSTM5 3'-UTR
NM_000851

GGCCCAGTGATGCCAGAAGATGGGAGGGAGGAGCCAACCTTGCTGCCTGCGACCCTGGAGGACAGC
CTGACTCCCTGGACCTGCCTTCTTCCTTTTTCCTTCTTTCTACTCTCTTCTCTTCCCCAAGGCCTC
ATTGGCTTCCTTTCTTCTAACATCATCCCTCCCCGCATCGAGGCTCTTTAAAGCTTCAGCTCCCCA
CTGTCCTCCATCAAAGTCCCCCTCCTAACGTCTTCCTTTCCCTGCACTAACGCCAACCTGACTGCT
TTTCCTGTCAGTGCTTTTCTCTTCTTTGAGAAGCCAGACTGATCTCTGAGCTCCCTAGCACTGTCC
TCAAAGACCATCTGTATGCCCTGCTCCCTTTGCTGGGTCCCTACCCCAGCTCCGTGTGATGCCCAG
TAAAGCCTGAACCATGCCTGCCATGTCTTGTCTTATTCCCTGAGGCTCCCTTGACTCAGGACTGTG
CTCGAATTGTGGGTGGTTTTTTGTCTTCTGTTGTCCACAGCCAGAGCTTAGTGGATGGGTGTGTGT
GTGTGTGTGTTGGGGGTGGTGATCAGGCAGGTTCATAAATTTCCTTGGTCATTTCTGCCCTCTAGC
CACATCCCTCTGTTCCTCACTGTGGGGATTACTACAGAAAGGTGCTCTGTGCCAAGTTCCTCACTC
ATTCGCGCTCCTGTAGGCCGTCTAGAACTGGCATGGTTCAAAGAGGGGCTAGGCTGATGGGGAAGG
GGGCTGAGCAGCTCCCAGGCAGACTGCCTTCTTTCACCCTGTCCTGATAGACTTCCCTGATCTAGA
TATCCTTCGTCATGACACTTCTCAATAAAACGTATCCCACCGTATTGTAAAAAAAAAAAAAAA

(SEQ ID NO:286)

Homo sapiens CADPS2 3'-UTR
CADPS2-002 ENST00000412584

TATCACACAGCTTTGCAGAAGGAAGGAAGACCTTGATCGACATTGTTTTTTATTTTTTTAACCTTG
TCCTTGTAATTACATTCATTGTTTGTTTTGGCCAAATAAAAATGCTTGTATTTCTTTAAAAAGTAA
GCCTGAATGTAGAGTAAAAGGGGAAATGCCAAGATTTTGGGGTTTTTTTGTTTCCTTTTTTTGTTT
GTTTGTTTGTTTGTTTTTTTGGAGAAGAGCATCCTCTTTTGTGTAGTTTGACCTAAAAATGAACCT
TGGCTCTGCTTGTGATCAGAACATGAACTTTTTTTTTTAAAGAAGATTTGAGCATTTTTCTGTAAT
CACATCAAAATGATGTTTTCTGTGTAAAGCGAGATACATATTTCTCATAATGCAGCATTGTGAGAA
GTCAGTTCGGACCACTGCACCAA

(SEQ ID NO:287)

Homo sapiens CADPS2 3'-UTR
CADPS2-001 ENST00000449022

TATCACACAGCTTTGCAGAAGGAAGGAAGACCTTGATCGACATTGTTTTTTATTTTTTTAACCTTG
TCCTTGTAATTACATTCATTGTTTGTTTTGGCCAAATAAAAATGCTTGTATTTCTTTAAAAAGTAA
GCCTGAATGTAGAGTAAAAGGGGAAATGCC

(SEQ ID NO:288)

Homo sapiens AASS 3'-UTR
AASS-001 ENST00000417368

TTGGGAATTATATTTTGTTTTTTTCTTCCCAGGCAATACACCTCTGAACATGTGTGTGATAAATGG
GTTTGCTAATGTGCTGTTTTAAAGTATAAAGCATAATATGTTTTGGTTAACACAATGTACTTTTTG
AACTATAAATCTTTATTTTAATATGGAAATGTTTGGAACAGGAGATGCAAGCCACTAACAGAGAAC
TTTAATAATTCTACCCTGTATTTTATAAATACGTATGTGAAAGTGATGA

(SEQ ID NO:289)

Homo sapiens TRIM6-TRIM34 3'-UTR
NM 001003819

ATTTTCTCATTTCTTCACCTACAACCCTTTGTCTTGACTTATCTCCTGCAACTGACTCATCTGCAA
CATTCACACCATTGCTTCCTTGTGGTTTCCCTTCTTTAGAACTTTTACTCATCCTTGAGATGTATG
GTGTATTTGGCTTGAGTTATGAGAGATGCTTATTTATTCATTTACTCTTTTTCATATTTTCAGAGA
AAGTTACCTAATCCCTCCTAAAGACACAGCAGTATGGGTATAACATCCTTGCCTTCCCATTTATCC
ATGTTTCACTTTATCACTGATATGAAGAGGCCCAAAGCCTGTTAGCCACCATCCATGCTACCTAGG
TAGTCCATAGGAACCACCCCCATGACCACCACCAACATCAACTAAAGGTTCTTGGAGGGTATGTCA
GTGTGTTGCTCAGGATACCCCAGGTACATCAAGGAATCAAGGAGAGGAAAATATGAGCAATATGTG
TATTCAGAGTGAAGATTTTATGTCCAGAGTATTTGAGCTCAAACCTTGCCTGTTGTTTTCTAATCA
TGATGAATACTTTCTCAGTTTCTTTTTCCTGAAATATAAATTGGGATTTAAGACTGTACCTAACTA
TTAAGATCACTGTGTAAAACTAAGTGTCTCTAAATGTAATGCATCGATTTAGTGTCTGGAACATAA
TAAATATTTGCTCTCATGATTGCTAAAAAAAAAAA

(SEQ ID NO:290)

Homo sapiens SEPP1 3'-UTR
NM_005410

ATATTTAAAATAGGACATACTCCCCAATTTAGTCTAGACACAATTTCATTTCCAGCATTTTTATAA
ACTACCAAATTAGTGAACCAAAAATAGAAATTAGATTTGTGCAAACATGGAGAAATCTACTGAATT
GGCTTCCAGATTTTAAATTTTATGTCATAGAAATATTGACTCAAACCATATTTTTTATGATGGAGC
AACTGAAAGGTGATTGCAGCTTTTGGTTAATATGTCTTTTTTTTTCTTTTTCCAGTGTTCTATTTG
CTTTAATGAGAATAGAAACGTAAACTATGACCTAGGGGTTTCTGTTGGATAATTAGCAGTTTAGAA
TGGAGGAAGAACAACAAAGACATGCTTTCCATTTTTTTCTTTACTTATCTCTCAAAACAATATTAC
TTTGTCTTTTCAATCTTCTACTTTTAACTAATAAAATAAGTGGATTTTGTATTTTAAGATCCAGAA
ATACTTAACACGTGAATATTTTGCTAAAAAAGCATATATAACTATTTTAAATATCCATTTATCTTT
TGTATATCTAAGACTCATCCTGATTTTTACTATCACACATGAATAAAGCCTTTGTATCTTTCTTTC
TCTAATGTTGTATCATACTCTTCTAAAACTTGAGTGGCTGTCTTAAAAGATATAAGGGGAAAGATA
ATATTGTCTGTCTCTATATTGCTTAGTAAGTATTTCCATAGTCAATGATGGTTTAATAGGTAAACC
AAACCCTATAAACCTGACCTCCTTTATGGTTAATACTATTAAGCAAGAATGCAGTACAGAATTGGA
TACAGTACGGATTTGTCCAAATAAATTCAATAAAAACCTTAAAGCTGAAAAAAAAAAAAAAAAAA
AAAAAAAAAAAAAAAAAAAAAAAAAAAAAAAAAAAAAAAAAAAAAAAAAAAAAAAAAAAAA

(SEQ ID NO:291)

Homo sapiens SEPP1 3'-UTR
SEPP1-004 ENST00000506577

ATATTTAAAATAGGACATACTCCCCAATTTAGTCTAGACACAATTTCATTTCCAGCATTTTTATAA
ACTACCAAATTAGTGAACCAAAAATAGAAATTAGATTTGTGCAAACATGGAGAAATCTACTGAATT
GGCTTCCAGATTTTAAATTTTATGTCATAGAAATATTGACTCAAACCATATTTTTTATGATGGAGC
AACTGAAAGGTGATTGCAGCTTTTGGTTAATATGTCTTTTTTTTTCTTTTTCCAGTGTTCTATTTG
CTTTAATGAGAATAGAAACGTAAACTATGACCTAGGGGTTTCTGTTGGATAATTAGCAGTTTAGAA
TGGAGGAAGAACAACAAAGACATGCTTTCCATTTTTTTCTTTACTTATCTCTCAAAACAATATTAC
TTTGTCTTTTCAATCTTCTACTTTTAACTAATAAAATAAGTGGATTTTGTATTTTAAGATCCAGAA
ATACTTAACACGTGAATATTTTGCTAAAAAAGCATATATAACTATTTTAAATATCCATTTATCTTT
TGTATATCTAAGACTCATCCTGATTTTTACTATCACACATGAATAAAGCCTTTGTATCTTT

(SEQ ID NO:292)

Homo sapiens PDE5A 3'-UTR
PDE5A-002 ENST00000264805
GTGGCCTATTTCATGCAGAGTTGAAGTTTACAGAGATGGTGTGTTCTGCAATATGCCTAG
(SEQ ID NO:293)

Homo sapiens SATB1 3'-UTR

SATB1-004 ENST00000417717

GATAAAAGTATTTGTTTCGTTCAACAGTGCCACTGGTATTTACTAACAAAATGAAAAGTCCACCTT
GTCTTCTCTCAGAAAACCTTTGTTGTTCATTGTTTGGCCAATGAATCTTCAAAAACTTGCACAAAC
AGAAAAGTTGGAAAAGGATAATACAGACTGCACTAAATGTTTTCCTCTGTTTTACAAACTGCTTGG
CAGCCCCAGGTGAAGCATCAAGGATTGTTTGGTATTAAAATTTGTGTTCACGGGATGCACCAAAGT
GTGTACCCCGTAAGCATGAAACCAGTGTTTTTTGTTTTTTTTTTAGTTCTTATTCCGGAGCCTCAA
ACAAGCATTATACCTTCTGTGATTATGATTTCCTCTCCTATAATTATTTCTGTAGCACTCCACACT
GATCTTTGGAAACTTGCCCCTTATTT

(SEQ ID NO:294)

Homo sapiens CCPG1 3'-UTR
CCPG1-002 ENST00000442196

TTCACAATTGAGTTAAATTAGACAACTGTAAGAGAAAAATTTATGCTTTGTATAATGTTTGGTATT
GAAACTAATGAAATTACCAAGATGACAATGTCTTTTCTTTTGTTTCTAAGTATCAGTTTGATAACT
TTATATTATTCCTCAGAAGCATTAGTTAAAAGTCTACTAACCTGCATTTTCCTGTAGTTTAGCTTC
GTTGAATTTTTTTTGACACTGGAAATGTTCAACTGTAGTTTTATTAAGGAAGCCAGGCATGCAACA
GATTTTGTGCATGAAATGAGACTTCCTTTCAGTGTAAGAGCTTAAAGCAAGCTCAGTCATACATGA
CAAAGTGTAATTAACACTGATGTTTGTGTTAAATTTGCAGCAGAGCTTGAGAAAAGTACATTGTTC
TGGAATTTCATCATTAACATTTTATAATCTTACACTCACTTCTTGTCTTTTTGTGGGTTCAAGAGC
CCTCTGACTTGTGAAGAATTTGCTGCCCTCTTAAGAGCTTGCTGACTTGTTTTCTTGTGAAATTTT
TTGCACATCTGAATATCGTGGAAGAAACAATAAAACTACACCATGAGGAAAACTAAAGGTCTTTAT
TTAAAATCTGGCATTGTATTAACATGTAATTTTATACTATGTGGTATTTTATACATTTCCTCAGTA
GTGATATTTGGTAAAGCAGTTCATACAGCTTTTTTCTAAGTTCCATGAATCTTACCCAGTGTTTAC
CGAAGTATTTAAGCAGCATCTGAATATTTCCACCCAGCAATGTTAATTTATCTAGGAAAGTTCAGA
ATTTCATCTTCATGTTGAATTTCCCTTTTAACTTCCGTTCATAGACATATATGTGACTTCCAATTC
GACCCTCTGGCAAGTGAGTGTGGAAGAAAACAGCAGTTCTTTTATAATTGCTTGAAATTAGGAAAG
CGCTTATTTCCTAGAAGCAAATAAATGTTTAAGTAAATAAAGGCTACATTTTGCTGA

(SEQ ID NO:295)

Homo sapiens CCPG1 3'-UTR
CCPG1-004 ENST00000425574

TTCACAATTGAGTTAAATTAGACAACTGTAAGAGAAAAATTTATGCTTTGTATAATGTTTGGTATT
GAAACTAATGAAATTACCAAGATGACAATGTCTTTTCTTTTGTTTCTAAGTATCAGTTTGATAACT
TTATATTATTCCTCAGAAGCATTAGTTAAAAGTCTACTAACCTGCATTTTCCTGTAGTTTAGCTTC
GTTGAATTTTTTTTGACACTGGAAATGTTCAACTGTAGTTTTATTAAGGAAGCCAGGCATGCAACA
GATTTTGTGCATGAAATGAGACTTCCTTTCAGTGTAAGAGCTTAAAGCAAGCTCAGTCATACATGA
CAAAGTGTAATTAACACTGATGTTTGTGTTAAATTTGCAGCAGAGCTTGAGAAAAGTACATTGTTC
TGGAATTTCATCATTAACATTTTATAATCTTACACTCACTTCTTGTCTTTTTGTGGGTTCAAGAGC
CCTCTGACTTGTGAAGAATTTGCTGCCCTCTTAAGAGCTTGCTGACTTGTTTTCTTGTGAAATTTT
TTGCACATCTGAATATCGTGGAAGAAACAATAAAACTACACCATGAG

(SEQ ID NO:296)

Homo sapiens CNTN1 3'-UTR
CNTN1-002 ENST00000348761

ATGTGTTGTGACAGCTGCTGTTCCCATCCCAGCTCAGAAGACACCCTTCAACCCTGGGATGACCAC
AATTCCTTCCAATTTCTGCGGCTCCATCCTAAGCCAAATAAATTATACTTTAACAAACTATTCAAC
TGATTTACAACACACATGATGACTGAGGCATTCGGGAACCCCTTCATCCAAAAGAATAAACTTTTA
AATGGATATAAATGATTTTTAACTCGTTCCAA

(SEQ ID NO:297)

Homo sapiens CNTN1 3’-UTR
CNTN1-004 ENST00000547849

TCGTTGACACTCACCATTTCTGTGAAAGACTTTTTTTTTTTTAACATATTATACTAGATTTGACTA
ACTCAATCTTGTAGCTTCTGCAGTTCTCCCCACCCCCAACCTAGTTCTTAGAGTATGTTTCCCCTT
TTGAAACATGTAAACATACTTTGGGCATAAATATTTTTTAAAATATAACTATAATGCTTCACTAAT
ACCTTAAAAATGCCTAGTGAACTAACTCAGTACATTATATAATGGCCAAGTGAAAGTTTTGTGTTT
TCATGTCCTGTTTTTCTTTGAAATTATATAGCCCAGAAATTAGCTCATTATCTGAAAAACGTATGA
AGAACTGATGAATTGTATAATACAGGAGTATTGCCATTGAATGTACTGTTTGATTTATTCAAGCAG
GTAATGAACAATGTTGTCAAACTCTCTAATGAGACATCATAATTAGGACATAAGCTAAAAGGGGCA
TTACTCCGGCAGTCTTTTTTTCTTAATCCTAGTACCATACATATTCTTTGGCATGAAAGAATGAAA
AGCATTAGTAAACAACTGAAGTCCTACCATGGCTCTGTAGGGTTTTTGGAACAATTCCTGGAATTG
GAAAGTGAAAATGGATAGCATGTGGGGGAAACCCTCATCTGAGTAGCAAGATTTTAGTAAAGATGA
CTAAGCCATTAACAGCATGCATTCATATTTAATTTTATTGACTCCTGCCATCAGCTTTTGTAGATC
GTTTGGGTGGAAGGTTGTGATTTTTACTGGGAGGACTTGAGTAGAAGTGGATGATTAAAATTGAGG
AGTATATAATTCTTTCTGGGACTGCTTAAATGTTATTGTTTGAAAATACCTTCACTTTCCCCCTTT
GGTCAAAGAGATGTGCTTAAAATTCTTATTCCTTCACAATAAATAATTTTGATTTTCTTAGACA

(SEQ ID NO:298)

Homo sapiens CNTN1 3’-UTR
CNTN1-004 ENST00000547849
+T at pos. 30bp, mutations G727bpT, A840bpG

*TTT*TTTCGTTGACACTCACCATTTCTGTGAA
AGACTTTTTTTTTTTTTAACATATTATACTAGATTTGACTAACTCAATCTTGTAGCTTCT
GCAGTTCTCCCCACCCCCAACCTAGTTCTTAGAGTATGTTTCCCCTTTTGAAACATGTAA
ACATACTTTGGGCATAAATATTTTTTAAAATATAACTATAATGCTTCACTAATACCTTAA
AAATGCCTAGTGAACTAACTCAGTACATTATATAATGGCCAAGTGAAAGTTTTGTGTTTT
CATGTCCTGTTTTTCTTTGAAATTATATAGCCCAGAAATTAGCTCATTATCTGAAAAACG
TATGAAGAACTGATGAATTGTATAATACAGGAGTATTGCCATTGAATGTACTGTTTGATT
TATTCAAGCAGGTAATGAACAATGTTGTCAAACTCTCTAATGAGACATCATAATTAGGAC
ATAAGCTAAAAGGGGCATTACTCCGGCAGTCTTTTTTTCTTAATCCTAGTACCATACATA
TTCTTTGGCATGAAAGAATGAAAAGCATTAGTAAACAACTGAAGTCCTACCATGGCTCTG
TAGGGTTTTTGGAACAATTCCTGGAATTGGAAAGTGAAAATGGATAGCATGTGGGGGAAA
CCCTCATCTGAGTAGCAAGATTTTAGTAAAGATGACTAAGCCATTAACAGCATGCATTCA
TATTTAATTTTATTGACTCCTGCCATCAGCTTTTGTAGATCTTTTGGGTGGAAGGTTGTG
ATTTTTACTGGGAGGACTTGAGTAGAAGTGGATGATTAAAATTGAGGAGTATATAATTCT
TTCTGGGACTGCTTAAATGTTATTGTTTGAAAATGCCTTCACTTTCCCCCTTTGGTCAAA
GAGATGTGCTTAAAATTCTTATTCCTTCACAATAAATAATTTTGATTTTCTTAGACA

(SEQ ID NO:299)

Homo sapiens LMBRD2 3’-UTR

AGTCTGAAAAAGTTTGTGGGACCACTAACCAAGGTCAACACATCAGTTCAGTCTTGATGAACATCT
GTGTACCCTAGAATTTCCTCTATACACAGTGAAAAGTGTCAAGATAACAAAAAAGGCACTGAGAAT
TAATTATATCTTAGGAATAATAGTTTAATGTGCATTGAATAGAGTATCACCTTTTTCAACAAGATT
TATTACATATCATTTCCTAAGCATCTGCCTTAGAAATACAGTTACAGTGGAAGGACTTTAAGAAAG

ATCAACATATGTTAAGAACATGCAGTTCAGTTTGTTTCAGATTAATTTTTTTTCAAGAGAGTTATT
TTAAAGATTCAAGGAAGCCATAAGTCATACTAAATAATATTATATACAGTTTTGTTATTGTGACTT
ACATTTTTGTTACTTCTAAAAAGTATATTCAACCTGTATTTCCCAAAGAAATGTAAGTGAATGGAG
ACCTCAAATAATAACTGTATTCATAAAACTCGTGTCTTAAAACAAGGCTTACTTACTAGACATAAC
TGAATGTAAAAAGTGCTTTTTCAAATCTGTTTGCAAACTCGTGGGGGATTTTTGCATGTATAAGAT
TAAGATTATACTTCAAGTGATGCGTGTCTGTGTATTTAGCATGTGTACTATAATCAGGTGATATAG
TATTCCTTCAGTCTTTGTAGTAACTGGATTTTTTTATGCTTCTGGTATTGCTTTATAAAAGATTTT
CATTTCAG

(SEQ ID NO:300)

Homo sapiens TLR3 3'-UTR
NM 003265

ATTTATTTAAATATTCAATTAGCAAAGGAGAAACTTTCTCAATTTAAAAAGTTCTATGGCAAATTT
AAGTTTTCCATAAAGGTGTTATAATTTGTTTATTCATATTTGTAAATGATTATATTCTATCACAAT
TACATCTCTTCTAGGAAAATGTGTCTCCTTATTTCAGGCCTATTTTTGACAATTGACTTAATTTTA
CCCAAAATAAAACATATAAGCACGTAAAAAAAAAAAAAAAAAA

(SEQ ID NO:301)

Homo sapiens BCAT1 3'-UTR
BCAT1-002 ENST00000342945

ATGGAAAATAGAGGATACAATGGAAAATAGAGGATACCAACTGTATGCTACTGGGACAGACTGTTG
CATTTGAATTGTGATAGATTTCTTTGGCTACCTGTGCATAATGTAGTTTGTAGTATCAATGTGTTA
CAAGAGTGATTGTTTCTTCATGCCAGAGAAAATGAATTGCAATCATCAAATGGTGTTTCATAACTT
GGTAGTAGTAACTTACCTTACCTTACCTAGAAAAACATTAATGTAAGCCATATAACATGGGATTTT
CCTCAATGATTTTAGTGCCTCCTTTTGTACTTCACTCAGATACTAAATAGTAGTTTATTCTTTAAT
ATAAGTTACATTCTGCTCCTCAAACAAATGCAATTTTTTGTGTGTGTTTGAAAGCTAATTTGAGAA
AATTTCATAGGTTACATTTCCTGCAGCCTATCTTTATCCACAGAAAGTGTTTTCTTTTTTTTAAAT
CAAGACTTTTAAAACTGGATTTCCTCCCATCACTGTTTTTTGAAGGTCCTCCAAGTCCGTGTTAA

(SEQ ID NO:302)

Homo sapiens BCAT1 3'-UTR

ATGGAAAATAGAGGATACAATGGAAAATAGAGGATACCAACTGTATGCTACTGGGACAGACTGTTG
CATTTGAATTGTGATAGATTTCTTTGGCTACCTGTGCATAATGTAGTTTGTAGTATCAATGTGTTA
CAAGAGTGATTGTTTCTTCATGCCAGAGAAAATGAATTGCAATCATCAAATGGTGTTTCATAACTT
G

(SEQ ID NO:303)

Homo sapiens TOM1L1 3'-UTR
TOM1L1-001 ENST00000575882

GAAGAAAGTGGATGATCAGCTCACTACCACATCAAAGGTGCCAACTCTCTAAAACGTAGACTCTGT
GCAGCTTTGAAGCCTGGAAGACAATACCTACCAACATGTCAAAGCCATGGTGGCACATTTCTGCTA
TAATGAAGATTAAATAGAATAACAGTTCCAGGATAACACTGATTCCTGACAACAGCGTGAGATTTC
AACAGAACTTGTTTGGAACAAATACTCACTTAAAACTTCAGCAGAAGAAAAATTACTTAGTCCTTA
GGCCAACCAATTTAACTGCAGTGTCATGTTTCACAGGCCTTCCTACATTTAGAAATCGTCACACAG
CTGTGATAAGAGTAGATTATTTTACTATGAAATAATTCTGAATAGATGAAAGCATAAAATGTGAGA
AACTGAATGTATTATTCAGGAAGAATACTGAGTGCCTTCATTTAACTAAAGTTGAATGTAAAAGTC
AATTTGCACTTCTTTATAATCCTCTGGTTTAGAATTATAAATTGTTAAAACCTTGATAATTGTCAT
TTAATTATATTTCAGGTGTCCTGAACAGGTCACTAGACTCTACATTGGGCAGCCTTTAAATATGAT
TCTTTGTAATGCTAAATAGCCTTTTTTTCTCTTTTTACTGCAACTTAATATTTCTATTTAGAACAC
AGAAAATGAAAATATTTAGAATAAGTTGTACATTTGATGACAAATAAATCACTATT

(SEQ ID NO:304)

Homo sapiens SLC35A1 3’-UTR
SLC35A1-201 ENST00000369556

TTTTAGCCTCACGTGAGACTCCTTTTAAGACTAAACCATTTGCATTAAACTAGAGCCTTAAGTCAA
TCTCAGAAGGTAGCATAAACAAATAAAAATTAACTGTATGGCATGATCAGTGCGGTTATGTGGAAA
CAACAACAAACAAACGAAGCTATCTGAGTGAACTGCTAATACAGAAACTTAATGTAGACCTGTTTG
GGGTCTACTATTGTTTTAGAATGAAGGAATTGTATTATTGTGTGTATATATAATTTGTAAATAAAA
AGTATGGAGATGATACGGTGTTAAAAAAAATCATGGTAAGGCTACAATACTCAAGTAACAAGGTTT
GGGACAATGTCTAAGGGTTAAAGTGCCAAAGCCATTTCTGTACTAACTGTTCTCTTGTTCCGGTAC
CGGGGAGAAGGATGACCCCTCCTTATTCTCCAATTCATGTACAGTATTTTGTCCTAGCAGCATAAA
GACCTAGCTCTTTTCTTACAAGAGGCAGAAACAAGACAGGCTAGTTCATAAACAAACTGTGTAACT
TCTCAAAATGAATCTATTTCATAACTCGGACAATTTCTGGGTGGTGACTGAGTACCCCTTTAGTGA
GTACCCCTTTAGTGCTATATTTGTGCCATTCATTATCTGGTTCATATTTCTTTTCTGTTAGATGAT
ACACATTTCTTCAAAAAAATTTCTAATGTCACTTTTGTACTTTTTTAAATAAAGTATGTTTAACTG
TTGGGCTCTCAATAATTTGTGAAATTTCAGTGTTTTCTATAATGTTAATGGGGAAATTCAGCAATA
AACTTTATTTGT

(SEQ ID NO:305)

Homo sapiens GLYATL2 3’-UTR
GLYATL2-003 ENST00000532258

TTGATTCCACTGTCCATTTCAAATCTTTCTTATCAGTAAAAAAACATTAATTCAAACACAAGCATT
GTGATCTACATTAGCACAAAATGCAACTGATTATCTAGGATCTGTGTATTACTTAAGCTCACCCTT
AACAGTTTTACCTTCCTTCTCCTCTGTATTCTTACAGAAAATTAGAAGCTCAATTTTATGGTCTCA
TAATTTCCTTTATGACAGACATCTCAGAATTAAAATCACCCAAAGCCAATCATTAGTGCCAAGATA
ACCCTTTAACGGCAACACTTTCTTAAATGAAGACTATTTCTTTCATGAAAAAATTCACTTTTATGA
CT

(SEQ ID NO:306)

Homo sapiens STAT4 3’-UTR
STAT4-002 ENST00000392320

CAGGATAAACTCTGACGCACCAAGAAAGGAAGCAAATGAAAAAGTTTAAAGACTGTTCTTTGCCCA
ATAACCACATTTTATTTCTTCAGCTTTGTAAATACCAGGTTCTAGGAAATGTTTGACATCTGAAGC
TCTCTTCACACTCCCGTGGCACTCCTCAATTGGGAGTGTTGTGACTGAAATGCTTGAAACCAAAGC
TTCAGATAAACTTGCAAGATAAGACAACTTTAAGAAACCAGTGTTAATAACAATATTAACAG

(SEQ ID NO:307)

Homo sapiens GULP1 3'-UTR
GULP1-002 ENST00000409609

CATCAAGAACAAGAAATCCTGATTCATGTTAAATGTGTTTGTATACACATGTCATTTATTATTATT
ACTTTAAGATAGGTATTATTCATGTGTCAATGTTTTTGAATATTTTAATATTTTGAAAATTTTCTC
AGTTAAATTTCCTCACCTTCACTATTGATCTGTAATTTTTATTTTAAAAACAGCTTACTGTAAAGT
AGATCATACTTTTATGTTCCTTTCTGTTTCTACTGTAGATGAATTTGTAATTGAAAGACATATTAT
ACAAAT

(SEQ ID NO:308)

Homo sapiens GULP1 3'-UTR
GULP1-010 ENST00000409805

CATCAAGAACAAGAAATCCTGATTCATGTTAAATGTGTTTGTATACACATGTCATTTATTATTATT
ACTTTAAGATAGG

(SEQ ID NO:309)

Homo sapiens EHHADH 3'-UTR
EHHADH-002 ENST00000456310

TTCAGTCTTCCAGATTATGCCTCACATGCTAGCATCAGGTAATGCTGACTGAATTTCAGTGAAATT
AAATCAAAAATCCAAAGTAAGATTGTTCTGAAATACAAAGCAAAATAAATAATCATTAGAATCTTC
TGTGTAACGACTCTAATGGTCAAATCTTTAGGAATGTGCTTCCTATGCCTCTGAATCTGTCCTTAT
CAGATAAATTCAATGCATGAACTTGTGTGAATATAATACCATAATAGCTAATGAAAGA

(SEQ ID NO:310)

Homo sapiens NBEAL1 3'-UTR
NM 001114132.1

TTGTTATTTCCATTTTCTGTTATGATTACTGAAACCTGATTTATTGCTTTGTCACTTTAACCACAT
CTCTCAACTCTCTGCAATGTTGCAAGGCTTTTATCCCTGAAAATCATTTACAGATAACCACAATTT
GCTGTGGTATATAAACTAATTCTTGGTCTATACTAAGATGTATTTGAGAAAATACATTTGATTTGA
TTTTGTGGCCCATTCCTAAAGGTCATTGTATCCATTTTTAAAACAAACTAAAATGAGAACATTAGG
TTCAATTTTCTTATTATTCCAAATGATAAAATTTAAGATTTTTCTAATAAAAGAGTACAGATAATG
GGACAGTTGAGAGAGATGGCTTTAAATACATTCTTAAGTAATCATTTTCCTATTTACTGACCACTG
TAATGAAAATATATCAATTTATTTATGGAACTCCTGATTGGGGATAATATTTTAAAGGTATCTGTT
GCACACTTGGATTTTCAAAACTCGGTGAAAGTTACAAGTTTGCATGGTAAGAATAAAATAAGAATA
TTGAAACTGGTACATTAGCTAATTCTATTACTACTTAGCGTGTTTCTAATGAGAAGTTACTGAAAT
CTATTACTGTCCTTAATAAAAATTGAGTAGAAAAAAGTGGAACTAG

(SEQ ID NO:311)

Homo sapiens KIAA1598 3'-UTR
NM_001258299.1

TCTGAATCAGAAAATACTGCAACTCCTTCCTCCTTTTGTCTGCCTTTTGTTCTCCAAAAGTAAGTG
GAAATTACATTTCCAAGAAAGGAAATGAAATAATTGCAGGCCCAAGGTCTGCAAAATATGTGTTGA
ATTGACAGTGAAAAGGATCCATGTGTTGACAGACACAGTTGTTAGATGCCATAAAGGCAGATGTGA
AGCTCAATTTATTTCTCATCTTGCTTG

(SEQ ID NO:312)

Homo sapiens HFE 3'-UTR
HFE-006 ENST00000317896

CACGCAGCCTGCAGACTCACTGTGGGAAGGAGACAAAACTAGAGACTCAAAGAGGGAGTGCATTTA
TGAGCTCTTCATGTTTCAGGAGAGAGTTGAACCTAAACATAGAAATTGCCTGACGAACTCCTTGAT
TTTAGCCTTCTCTGTTCATTTCCTCAAAAAGATTTCCCCATTTAGGTTTCTGAGTTCCTGCATGCC
GGTGATCCCTAGCTGTGACCTCTCCCCTGGAACTGTCTCTCATGAACCTCAAGCTGCATCTAGAGG
CTTCCTTCATTTCCTCCGTCACCTCAGAGACATACACCTATGTCATTTCATTTCCTATTTTTGGAA
GAGGACTCCTTAAATTTGGGGGACTTACATGATTCATTTTAACATCTGAGAAAAGCTTTGAACCCT
GGGACGTGGCTAGTCATAACCTTACCAGATTTTTACACATGTATCTATGCATTTTCTGGACCCGTT
CAACTTTTCCTTTGAATCCTCTCTCTGTGTTACCCAGTAACTCATCTGTCACCAAGCCTTGGGGAT
TCTTCCATCTGATTGTGATGTGAGTTGCACAGCTATGAAGGCTGTACACTGCACGAATGGAAGAGG
CACCTGTCCCAGAAAAAGCATCATGGCTATCTGTGGGTAGTATGATGGGTGTTTTTAGCAGGTAGG
AGGCAAATATCTTGAAAGGGGTTGTGAAGAGGTGTTTTTTCTAATTGGCATGAAGGTGTCATACAG
ATTTGCAAAGTTTAATGGTGCCTTCATTTGGGATGCTACTCTAGTATTCCAGACCTGAAGAATCAC
AATAATTTTCTACCTGGTCTCTCCTTGTTCTGATAATGAAAATTATGATAAGGATGATAAAAGCAC
TTACTTCGTGTCCGACTCTTCTGAGCACCTACTTACATGCATTACTGCATGCACTTCTTACAATAA
TTCTATGAGATAGGTACTATTATCCCCATTTCTTTTTTAAATGAAGAAAGTGAAGTAGGCCGGGCA
C

(SEQ ID NO:313)

Homo sapiens HFE 3'-UTR
HFE-004 ENST00000349999

CACGCAGCCTGCAGACTCACTGTGGGAAGGAGACAAAACTAGAGACTCAAAGAGGGAGTGCATTTA
TGAGCTCTTCATGTTTCAGGAGAGAGTTGAACCTAAACATAGAAATTGCCTGACGAACTCCTTGAT
TTTAGCCTTCTCTGTTCATTTCCTCAAAAAGATTTCCCCATTTAGGTTTCTGAGTTCCTGCATGCC
GGTGATCCCTAGCTGTGACCTCTCCCCTGGAACTGTCTCTCATGAACCTCAAGCTGCATCTAGAGG
CTTCCTTCATTTCCTCCGTCACCTCAGAGACATACACCTATGTCATTTCATTTCCTATTTTTGGAA
GAGGACTCCTTAAATTTGGGGGACTTACATGATTCATTTTAACATCTGAGAAAAGCTTTGAACCCT
GGGACGTGGCTAGTCATAACCTTACCAGATTTTTACACATGTATCTATGCATTTTCTGGACCCGTT
CAACTTTTCCTTTGAATCCTCTCTCTGTGTTACCCAGTAACTCATCTGTCACCAAGCCTTGGGGAT
TCTTCCATCTGATTGTGATGTGAGTTGCACAGCTATGAAGGCTGTACACTGCACGAATGGAAGAGG
CACCTGTCCCAGAAAAAGCATCATGGCTATCTGTGGGTAGTATGATGGGTGTTTTTAGCAGGTAGG
AGGCAAATATCTTGAAAGGGGTTGTGAAGAGGTGTTTTTTCTAATTGGCATGAAGGTGTCATACAG
ATTTGCAAAGTTTAATGGTGCCTTCATTTGGGATG

(SEQ ID NO:314)

Homo sapiens HFE 3'-UTR
HFE-005 ENST00000397022

CACGCAGCCTGCAGACTCACTGTGGGAAGGAGACAAAACTAGAGACTCAAAGAGGGAGTGCATTTA
TGAGCTCTTCATGTTTCAGGAGAGAGTTGAACCTAAACATAGAAATTGCCTGACGAACTCCTTGAT
TTTAGCCTTC

(SEQ ID NO:315)

Homo sapiens HFE 3'-UTR
HFE-012 ENST00000336625
CACGCAGCCTGCAGACTCACTGTGGGAAGGA
(SEQ ID NO:316)

Homo sapiens KIAA1324L 3'-UTR
KIAA1324L-005 ENST00000416314

AGAGACAGTGCTGTAGCCTTGAGACTAATGAACAAAGAAACCTGCTCTAGTTTTACAGGACCATAT
TTTAGGGTCTGTCCTCATACCTGTCACATTGGTGATCTCACAGAGGAGGGCCATGCCGCTGAAAAG
GGAAGGAGATTGAAACATTTGATTGCCTTATCACATGGTCAAGTACCTTGCCAAATAAAGGAAAGC
AAATGATTTGGGTCTCAACTGAAGATGAAGCTCAACTCAGGAAGAGATTTATCTGTATATACACAT
AACTGAAAACCAAGTTTAAGCCCACCAATGCACTGCTGATGCATGCCATATAATTAATGGGTAACT
TTTATTCTTTATGATGTCTACATAACAAGTGTGATTTGGAAGGCACATGTGAGCATATGCATTA

(SEQ ID NO:317)

Homo sapiens MANSC1 NM_018050 3'-UTR

GGATGGAACTCGGTGTCTCTTAATTCATTTAGTAACCAGAAGCCCAAATGCAATGAGTTTCTGCTG
ACTTGCTAGTCTTAGCAGGAGGTTGTATTTTGAAGACAGGAAAATGCCCCCTTCTGCTTTCCTTTT
TTTTTTTTGGAGACAGAGTCTTGCTTTGTTGCCCAGGCTGGAGTGCAGTAGCACGATCTCGGCTCT
CACCGCAACCTCCGTCTCCTGGGTTCAAGCGATTCTCCTGCCTCAGCCTCCTAAGTATCTGGGATT
ACAGGCATGTGCCACCACACCTGGGTGATTTTTGTATTTTTAGTAGAGACGGGGTTTCACCATGTT
GGTCAGGCTGGTCTCAAACTCCTGACCTAGTGATCCACCCTCCTCGGCCTCCCAAAGTGCTGGGAT
TACAGGCATGAGCCACCACAGCTGGCCCCCTTCTGTTTTATGTTTGGTTTTTGAGAAGGAATGAAG
TGGGAACCAAATTAGGTAATTTTGGGTAATCTGTCTCTAAAATATTAGCTAAAAACAAAGCTCTAT
GTAAAGTAATAAAGTATAATTGCCATATAAATTTCAAAATTCAACTGGCTTTTATGCAAAGAAACA
GGTTAGGACATCTAGGTTCCAATTCATTCACATTCTTGGTTCCAGATAAAATCAACTGTTTATATC
AATTTCTAATGGATTTGCTTTTCTTTTTATATGGATTCCTTTAAAACTTATTCCAGATGTAGTTCC
TTCCAATTAAATATTTG

(SEQ ID NO:318)

Preferably, the at least one 5'-UTR element comprises or consists of a nucleic acid sequence which has an identity of at least about 1, 2, 3, 4, 5, 10, 15, 20, 30 or 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NO: 25 or SEQ ID NO: 30 and SEQ ID NOs: 319 to 382 or the corresponding DNA or RNA sequence, respectively, or wherein the at least one 5'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NO: 25 or SEQ ID NO: 30 and SEQ ID NOs: 319 to 382 or the corresponding DNA or RNA sequence, respectively:

Homo sapiens LTA4H 5'-UTR
LTA4H-001 ENST00000228740

AAGAAACTTCCTTTCCCGGCGTGCACCGCGAATCCCTCCTCCTCTTCTTTACCTCTCTCCCTCCTC
CTCAGGTTCTCTATCGACGAGTCTGGTAGCTGAGCGTTGGGCTGTAGGTCGCTGTGCTGTGTGATC
CCCCAGAGCC

(SEQ ID NO:319)

Homo sapiens DECR1 5'-UTR
DECR1-001 ENST00000220764

TCCAGCCCCGAGAACTTTGTTCTTTTTGTCCCGCCCCCTGCGCCCAACCGCCTGCGCCGCCTTCCG
GCCCGAGTTCTGGAGACTCAAC

(SEQ ID NO:320)

Homo sapiens PIGK 5'-UTR
ACTGCCTCCGCCCCTTCAGGTGCGGGAAGTCTGAAGCCGGTAAAC
(SEQ ID NO:321)

Homo sapiens BRP44L 5'-UTR
BRP44L-001

GTCGTGAGGCGGGCCTTCGGGCTGGCTCGCCGTCGGCTGCCGGGGGGTTGGCCGGGGTGTCATTGG
CTCTGGGAAGCGGCAGCAGAGGCAGGGACCACTCGGGGTCTGGTGTCGGCACAGCC

(SEQ ID NO:322)

Homo sapiens ACADSB 5'-UTR
ACADSB-004 NM_001609.3 ENST00000368869

AGGGATTAAGGGGGGGTGTGTGCGGGGCGGGTACTGAGTGGGCGGGGCCTTGCTCGGGTAACTCCC
AGGGGCTGGCTAGAGACCCAGAGGCGCAGAGCGGAGAGGCCTGCGGCGAGG

(SEQ ID NO:323)

Homo sapiens SUPT3H 5'-UTR
SUPT3H-006 ENST00000371459

CACAGCCGAGTCACCTTTTCCCTTTCTACACTCCACACTCTCAGTCCCCCACCCCGCCCCTTTCCA
AGCGTGTCCCGGGCCGCAGCAGCAGAAACCGCACCATCTCCACCCCCACATTCTCCTCGCGGGAAG
CGCAGCAGTGCCTCCAAGGGTTCTTAAAGCAGAG

(SEQ ID NO:324)

Homo sapiens TMEM14A 5'-UTR
NM 014051.3

GTTTCCAGGAGGGAGCGGCCTTTGCTCAGCGCGAGACGGCTGGGCGCCGAGTGGGACAGCGCTGGT
GCGGAGACTGCTTCCGGACTCCAGGTACCGCGCTTGGCGGCAGCTGGCCCCAGACTTCTGTCTTTT
CAGCTGCAGTGAAGGCTCGGGGCTGCAGAATTGCAACCTTGCCA

(SEQ ID NO:325)

Homo sapiens C9orf46 5'-UTR
AF225420.1

GAGCGAGGCCCGGTCCCTGCAGCGGGCGAAAGGAGCCCGGGCCTGGAGGTTTGCGTACCGGTCGCC
TGGTCCCGGCACCAGCGCCGCCCAGTGTGGTTTCCCATAAGGAAGCTCTTCTTCCTGCTTGGCTTC
CACCTTTAACCCTTCCACCTGGGAGCGTCCTCTAACACATTCAGACTACAAGTCCAGACCCAGGAG
AGCAAGGCCCAGAAAGAGGTCAAA

(SEQ ID NO:326)

Homo sapiens ANXA4 5'-UTR
NM 001153.3

GCCCCAGGTGCGCTTCCCCTAGAGAGGGATTTTCCGGTCTCGTGGGCAGAGGAACAACCAGGAACT
TGGGCTCAGTCTCCACCCCACAGTGGGGCGGATCCGTCCCGGATAAGACCCGCTGTCTGGCCCTGA
GTAGGGTGTGACCTCCGCAGCCGCAGAGGAGGAGCGCAGCCCGGCCTCGAAGAACTTCTGCTTGGG
TGGCTGAACTCTGATCTTGACCTAGAGTC

(SEQ ID NO:327)

Homo sapiens IFI6 5'-UTR
NM_022873.2

CCAGCCTTCAGCCGGAGAACCGTTTACTCGCTGCTGTGCCCATCTATCAGCAGGCTCCGGGCTGAA
GATTGCTTCTCTTCTCTCCTCCAAGGTCTAGTGACGGAGCCCGCGCGCGGCGCCACC

(SEQ ID NO:328)

Homo sapiens C2orf34 5'-UTR
CAMKMT -008 ENST00000402247
TCCTGGCAGGGGACGAGCTGCGGCGGTGGCACCTCCGGGTGTGGAAGGCTCCAGTGAG
(SEQ ID NO:329)

Homo sapiens C2orf34 5'-UTR
NM 024766.3

GAGGGTGCCGGGCGTCACAGGTCCTGACAGGGAAGAAGTTGGCAGGTCCTGGCAGGGGACGAGCTG
CGGCGGTGGCACCTCCGGGTGTGGAAGGCTCCAGTGAG

(SEQ ID NO:330)

Homo sapiens ALDH6A1 5'-UTR
ALDH6A1-002 ENST00000350259
AGTGCTTCTGGGCAGTAGAGGCGCGGGGTGCGGAGCTAGGGCGGCCGAGAGCC
(SEQ ID NO:331)

Homo sapiens CCDC53 5'-UTR
CCDC53-002 ENST00000545679

GGAAGGGCCCCGGAGGCGGGCACTTGGGGGGAAAGTTGAGACGTGATTACCGGGTTGGGCGGGCCC
CATCTGGGAGGGGTTTGTGGGTGAACTCGGGGTCCACCGCCCGCTGAGGAG

(SEQ ID NO:332)

Homo sapiens CASP1 5'-UTR
NM_001257119.1
ATACTTTCAGTTTCAGTCACACAAGAAGGGAGGAGAGAAAAGCC
(SEQ ID NO:333)

Homo sapiens NDUFB6 5'-UTR
NM 182739.2

GTAATAACCGCGCGGCGCTCGGCGTTCCCGCAAGGTCGCTTTGCAGAGCGGGAGCGCGCTTAAG
TAACTAGTCCGTAGTTCGAGGGTGCGCCGTGTCCTTTTGCGTTGGTACCAGCGGCGAC

(SEQ ID NO:334)

Homo sapiens BCKDHB 5'-UTR
BCKDHB-002 ENST00000369760
AGGCGGCGTGCGGCTGCATAGCCTGAGAATCCCGGTGGTGAGCGGGG
(SEQ ID NO:335)

Homo sapiens BCKDHB 5'-UTR
NM_001164783.1
CTACGTGAGTGCCGGACCGCTGAGTGGTTGTTAGCCAAG
(SEQ ID NO:336)

Homo sapiens BBS2 5'-UTR
NM_031885.3

CACAGAAGGCGCCGAGGCTCCACCGCGCAGCCGCAAAAAGAGCGGACGGGTCTGCGCCGCCGCAGG
AGGAGCAGGCGGTACCTGGACGGGTTCGTCCCGGGCTGTTTCGCGTCCGGCCTGAGGCGGCTGGGG
CCGCGCAGGTAGTGTCCCTGCACTTCTTGCCCGGGCGCGTGAGGCCAGCTCCGCTGCGCTTGTCTC
CAGCTTCCAGCCCTCCTCCCCTAAGCCGCCGCCATC

(SEQ ID NO:337)

Homo sapiens HERC5 5'-UTR
HERC5-001 ENST00000264350

TCAGTAGCTGAGGCTGCGGTTCCCCGACGCCACGCAGCTGCGCGCAGCTGGTTCCCGCTCTGCAGC
GCAACGCCTGAGGCAGTGGGCGCGCTCAGTCCCGGGACCAGGCGTTCTCTCCTCTCGCCTCTGGGC
CTGGGACCCCGCAAAGCGGCG

(SEQ ID NO:338)

Homo sapiens FAM175A 5'-UTR
NM_139076.2
ACCACAGGGTCTTGCCTCCGCGCGCCCCGCCCTCGTCCTCTTGTGTAGCCTGAGGCGGCGGTAGC
(SEQ ID NO:339)

Homo sapiens NT5DC1 5'-UTR
NT5DC1-002 ENST00000319550

CGGTCCTGTCCCGCAGCGTCCCGCCAGCCAGCTCCTTGCACCCTTCGCGGCCGAGGCGCTCCCTGG
TGCTCCCCGCGCAGCC

(SEQ ID NO:340)

Homo sapiens RAB7A 5'-UTR
RAB7A-001 ENST00000265062

GTCTCGTGACAGGTACTTCCGCTCGGGGCGGCGGCGGTGGCGGAAGTGGGAGCGGGCCTGGAGTCT
TGGCCATAAAGCCTGAGGCGGCGGCAGCGGCGGAGTTGGCGGCTTGGAGAGCTCGGGAGAGTTCCC
TGGAACCAGAACTTGGACCTTCTCGCTTCTGTCCTCCGTTTAGTCTCCTCCTCGGCGGGAGCCCTC
GCGACGCGCCCGGCCCGGAGCCCCCAGCGCAGCGGCCGCGTTTGAAGG

(SEQ ID NO:341)

Homo sapiens AGA 5'-UTR
AGA-001 ENST00000264595

AGGGACGCCTGAGCGAACCCCCGAGAGAGCGGGCGTGGGCGCCAGGCGGGCGGGGCACTGGGGATT
AATTGTTCGGCGATCGCTGGCTGCCGGGACTTTTCTCGCGCTGGTCTCTTCGGTGGTCAGGG

(SEQ ID NO:342)

Homo sapiens TPK1 5'-UTR
TPK1-001 ENST00000360057

AAGGCTCCTCAGCCGAGCGCCGAGCGGTCGATCGCCGTAGCTCCCGCAGCCTGCGATCTCCAGTCT
GTGGCTCCTACCAGCCATTGTAGGCCAATAATCCGTT

(SEQ ID NO:343)

Homo sapiens MBNL3 5'-UTR
MBNL3-001 ENST00000370839

AATTCATTTTTAATCCTTTAATAGTCCACAGTAATATTGTCCTAAAGAGGGTACATTGGATTTTAA
TTTTGCTTTCAAT

(SEQ ID NO:344)

Homo sapiens MCCC2 5'-UTR
MCCC2-001 ENST00000340941

AGAATCAGAGAAACCTTCTCTGGGGCTGCAAGGACCTGAGCTCAGCTTCCGCCCCAGCCAGGGAAG
CGGCAGGGGAAAGCACCGGCTCCAGGCCAGCGTGGGCCGCTCTCTCGCTCGGTGCCCGCCGCC

(SEQ ID NO:345)

Homo sapiens CAT 5'-UTR
CAT-001 ENST00000241052

ACTCGGGGCAACAGGCAGATTTGCCTGCTGAGGGTGGAGACCCACGAGCCGAGGCCTCCTGCAGTG
TTCTGCACAGCAAACCGCACGCT

(SEQ ID NO:346)

Homo sapiens ANAPC4 5'-UTR
ANAPC4-001 ENST00000315368

CCCGACGCCGGAAGTGCCTGGAGCGCGCGACAGCGGCGGGGCGGGGCGGCCTGGAGGCTGTGGCGC
GCGGCCGGCAGAGGGAGGGGAGAGGCCACTGGGGCCGTGTTAGTCTGCCGGTGGGGACTCTTGCAG
GGCCGTCCCC

(SEQ ID NO:347)

Homo sapiens PHKB 5'-UTR
PHKB-002 ENST00000323584
GGCCAAGGCGGCGACCGGAGCGCG
(SEQ ID NO:348)

Homo sapiens ABCB7 5'-UTR
ABCB7-001 ENST00000253577
CTCGGTTCCTCTTTCCTCGCTCAAG
(SEQ ID NO:349)

Homo sapiens GPD2 5'-UTR
GPD2-002 ENST00000438166

CCCGCGCGCCTCGCTGGGAGCACCCGGGCCGAGGCTCTGATTCTGGGGGGAGGCCGACTCCACCCT
GGCTGGAGGAACTGGGTGCTCCTGCCCGCTGGCCCCTCGCGCGTGAGGATCTATCTCAGGCTAAGA
A

(SEQ ID NO:350)

Homo sapiens TMEM38B 5'-UTR
TMEM38B-001 ENST00000374692

GCTGGAGCCGGCGCGGAGGAGCGGGCGGCCGCGGCTGTGCCCTCTCCTACTCCTCACCGCGCGAGC
GCGGGGAACCAGTAGCCGCGGCTGCTTCGGTTGCCGCGGTCGGTGGTCGTT

(SEQ ID NO:351)

Homo sapiens NFU1 5'-UTR
NM 001002755.2

GGGAAAGGTTCCCCGGCCTCTCTTGGTCAGGGTGACGCAGTAGCCTGCAAACCTCGGCGCGTAGGC
CACCGCACTTATCCGCAGCAGGACCGCCCGCAGCCGGTAGGGTGGGCTCTTCCCAGTGCCCGCCCA
GCTACCGGCCAGCCTGCGGCTGCGCAGATCTTTCGTGGTTCTGTCAGGGAGACCCTTAGGCACTCC
GGACTAAG

(SEQ ID NO:352)

Homo sapiens LOC128322/NUTF2 5'-UTR
NM_005796.1

GGAAGGGACAGTCGGCCGCAGACCGCGCTGGGTTGCCGCTGCCGCTGCCGCCATCGTGCCAGCCCC
TCGGGTCTCCGTGAGGCCGGGTGACGCTCCAGA

(SEQ ID NO:353)

Homo sapiens NUBPL 5'-UTR
NM_025152.2

ACTCCGCGCCACCCGCGACAGTTTCCCAGCAGGGCTCACAGCAGCGTTCCGCGTC
(SEQ ID NO:354)

Homo sapiens LANCL1 5'-UTR
LANCL1-004 ENST00000233714

GAGAAGGGCTTCAGGACGCGGGAGGCGCACTTGCTTCAAGTCGCGGGCGTGGGAACGGGGCTTGCT
TCCGGCGTC

(SEQ ID NO:355)

Homo sapiens PIR 5'-UTR
PIR-002 ENST00000380420

CCTCCCGCCTCCTCTAGGCCGCCGGCCGCGAAGCGCTGAGTCACGGTGAGGCTACTGGACCCACAC
TCTCTTAACCTGCCCTCCCTGCACTCGCTCCCGGCGGCTCTTCGCGTCACCCCCGCCGCTAAGGCT

CCAGGTGCCGCTACCGCAGCCCCTCCATCCTCTACAGCTCAGCATCAGAACACTCTCTTTTTAGAC
TCCGAT

(SEQ ID NO:356)

Homo sapiens CTBS 5'-UTR
NM_004388.2
GACGCGCAGCAGGCCCCGCCCACCCAGGCGGTAGGAACCCACTCCGGCCCGCTAGACCTGCTGCT
(SEQ ID NO:357)

Homo sapiens GSTM4 5'-UTR
NM 000850.4

AAGCTGGCGAGGCCGAGCCCCTCCTAGTGCTTCCGGACCTTGCTCCCTGAACACTCGGAGGTGGCG
GTGGATCTTACTCCTTCCAGCCAGTGAGGATCCAGCAACCTGCTCCGTGCCTCCCGCGCCTGTTGG
TTGGAAGTGACGACCTTGAAGATCGGCCGGTTGGAAGTGACGACCTTGAAGATCGGCGGGCGCAGC
GGGGCCGAGGGGGCGGGTCTGGCGCTAGGTCCAGCCCCTGCGTGCCGGGAACCCCAGAGGAGGTCG
CAGTTCAGCCCAGCTGAGGCCTGTCTGCAGAATCGACACCAACCAGCATC

(SEQ ID NO:358)

Mus musculus Ndufa1 5'-UTR
Ndufa1-001 ENSMUST00000016571

GCCGGAAGAGAGGTAAAGCCGGGTCACCTCTGAGGAGCCGGTGACGGGTTGGCGTGCGAGTAACGG
TGCGGAG

(SEQ ID NO:359)

Mus musculus Atp5e 5'-UTR
NM_025983

CCCACCCCTTCCGCTACTCAGGCCTGACCTTCCTGCTGCCGGGCCGGTTTGAGGCTACTCTGAAGC
GACCCAGCGGTTCTGCCCGACGCGCCCGCTCGAGACACC

(SEQ ID NO:360)

Mus musculus Gstm5 5'-UTR
NM_010360

GAGACAGTTCGGTCGCGTCAGCCCGGCCCACAGCGTCCAGTATAAAGTTAGCCGCCCACAGTCCAT
CGCTGTATCCCCGAAGGGGCTAAGATCGCCCAAA

(SEQ ID NO:361)

Mus musculus Cbr2 5'-UTR
NM_007621
ATAAAAGCTGAGCCCATCTCTTGCTTCGGAAGAAGCTGGTGTCAGCAGC
(SEQ ID NO:362)

Mus musculus Anapc13 5'-UTR
NM_181394

GTGACCCAGAAGAAGGGCGGGGCCGGGAGGAAGCCGACGCGCGCGCAGTGGGCCTGACAAGATCAA
AGCTGCAGGAGG

(SEQ ID NO:363)

Mus musculus Ndufa7 5'-UTR
NM_023202
TCGGAGCGGAAGGAAT
(SEQ ID NO:364)

Mus musculus Atp5k 5'-UTR
NM_007507
CGAAGGTCACGGACAAA
(SEQ ID NO:365)

Mus musculus Cox4il 5'-UTR
NM 009941

CTTCCGGTCGCGAGCACCCCAGGGTGTAGAGGGCGGTCGCGGCGGTCGCCTGGGCAGCGGTGGCAG
A

(SEQ ID NO:366)

Mus musculus Ndufs6 5'-UTR
NM_010888
TTGGTACGACGCGTGGGGTCAAGGGTCACCGGCAAG
(SEQ ID NO:367)

Mus musculus Sec61b 5'-UTR
NM 024171

AGAGCCTGTATCTACGAGAGTTCTGAGTGCTCGGCAACTTCACGACTTCCCTCTTCCTGCCTCCTG
TGCCCACCGTTCTTAGGCATCAGC

(SEQ ID NO:368)

Mus musculus Snrpd2 5'-UTR
NM_026943
AAGGCTGGAGCAACGCGCTTGGAGGCGGGAGTGATCTGCGAGCGAAACCTACACC
(SEQ ID NO:369)

Mus musculus Mgst3 5'-UTR
NM_025569
ACTGCTGTGCTTCTCAGGTCTGTACCAGGCGCACGAAGGTGAGCCAGAGCCAAG
(SEQ ID NO:370)

Mus musculus Mp68 (2010107E04Rik) 5'-UTR
NM_027360
CTTTCCCATTCTGTAGCAGAATTTGGTGTTGCCTGTGGTCTTGGTCCCGCGGAG
(SEQ ID NO:371)

Mus musculus Prdx4-001, 5'-UTR
NM 016764

GCGCGGTCTCCAGCGCGCCGTTTTAGCTGGCTGCCTGGCGGCAGGGGACTCTGTGCTTTAGCAGAG
GGACGTGTTTTCGCGCTTGCTTGGTC

(SEQ ID NO:372)

Mus musculus Pgcp 5'-UTR
NM_176073

GCTGTCCTGGCACACAAAGAAGCCAGGCCTGCAGACTACTGGGGCTCCGGGCTGTTCCTGAGGCCT
CTGGAGGCCCGCCCTGTGGCTCCAGTGCGCTCTGAGGACCTTCCTGGTCCCGCCCCCGAACGTGCC
TGTGGTCTGCAGGCCTCACCGGGTGTTGTGGCCGCTGCTGCTCCGCAGAGCCTCGTGATCAGGAAG
AAAAGCAACTAGGAACA

(SEQ ID NO:373)

Mus musculus Myeov2 5'-UTR
NM_001163425
AGAAGGGGCTGGCCGGAAGTGAGCGCAACGCCGCCTTGTCGAG
(SEQ ID NO:374)

Mus musculus Ndufa4 5'-UTR
NM 010886

GTCCGCTCAGCCAGGTTGCAGAAGCGGCTTAGCGTGTGTCCTAATCTTCTCTCTGCGTGTAGGTAG
GCCTGTGCCGCAAAC

(SEQ ID NO:375)

Mus musculus Ndufs5 5'-UTR
NM 001030274

ACGGCAGGCGTCTGCGTCCTCCCGCAGCCGGCGGTCGGGAATTGCACCAGGGACCTGACAAGGGCA
CTGCAGAGCC

(SEQ ID NO:376)

Mus musculus Gstm1 5'-UTR
NM_010358

CTGCCTTCCGCTTTAGGGTCTGCTGCTCTGGTTACAGACCTAGGAAGGGGAGTGCCTAATTGGGAT
TGGTGCAGGGTTGGGAGGGACCCGCTGTTTTGTCCTGCCCACGTTTCTCTAGTAGTCTGTATAAAG
TCACAACTCCAAACACACAGGTCAGTCCTGCTGAAGCCAGTTTGAGAAGACCACAGCACCAGCACC

(SEQ ID NO:377)

Mus musculus Atp5o 5'-UTR
NM 138597

CTGGCGCGCGCGCGTGCGCTCTGGCGCCAGTAGTCTCTTTTCATTTGGGTTTGACCTACAGCCGCC
CGGGAAAAG

(SEQ ID NO:378)

Mus musculus Tspo 5'-UTR
NM_009775

GTCAGCGGCTACCAACCTCTGTGCGCAGTGTCCTTCACGGAACAACCAGCGACTGCGTGAGCGGGG
CTGTGGATCTTTCCAGAACATCAGTTGCAATCACC

(SEQ ID NO:379)

Mus musculus Taldo1 5'-UTR
NM_011528
GACGCGCGGGGCATTGTGGGTTAGCACGCACCGGCTACCGCCTCAGCTGTTCGCGTTTCGCC
(SEQ ID NO:380)

Mus musculus Bloc1s1 5'-UTR
NM_015740

GTGACGCCTTCCGGGTGAGCCAAGGCATAGTCCAGTTCCTGCAGCCTTAGGGAGGGGTCCGCCGTG
CCCACACCCAGCCAGACTCGACC

(SEQ ID NO:381)

Mus musculus Hexa 5'-UTR
NM_010421
AGCTGACCGGGGCTCACGTGGGCTCAGCCTGCTGGAAGGGGAGCTGGCCGGTGGGCC
(SEQ ID NO:382)

Preferably, the at least one 3'-UTR element of the artificial nucleic acid molecule according to the present invention comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the 3'-UTR sequence of a transcript of a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), Ybxl (Y-Box binding protein 1), Ndufb8 (NADH dehydrogenase (ubiquinone) 1 beta subcomplex 8), and CNTN1 (contactin 1; whereby CNTN1-004 is particularly preferred). Most preferably, the at least one 3'-UTR element of the artificial nucleic acid molecule according to the present invention comprises or consists of a nucleic acid sequence which has an identity of at least about 40%, preferably of at least about 50%, preferably of

at least about 60%, preferably of at least about 70%, more preferably of at least about 80%, more preferably of at least about 90%, even more preferably of at least about 95%, even more preferably of at least about 99%, most preferably of 100% to a sequence selected from the group consisting of SEQ ID NO: 1 to SEQ ID NO: 24, or the corresponding RNA sequences, respectively:

SEQ ID NO: 1

GAAGGGAACACCCAAATTTAATTCAGCCTTAAGCACAATTAATTAAGAGTGAAACGTAATGTACAA
GCAGTTGGTCACCCACCATAGGGCATGATCAACACCGCAACCTTTCCTTTTTCCCCCAGTGATTCT
GAAAAACCCCTCTTCCCTTCAGCTTGCTTAGATGTTCCAAATTTAGTAAGCTTAAGGCGGCCTACA
GAAGAAAAAGAAAAAAAAGGCCACAAAAGTTCCCTCTCACTTTCAGTAAATAAAATAAAAGCAGCA
ACAGAAATAAAGAAATAAATGAAATTCAAAATGAAATAAATATTGTTTGTGCAGCATTAAAAAATC
AATAAAAATTAAAAATGAGCA

(Mus musculus GNAS 3'-UTR)
SEQ ID NO: 2

GAAGGGAACACCCAAATTTAATTCAGCCTTAAGCACAATTAATTAAGAGTGAAACGTAATTGTACA
AGCAGTTGGTCACCCACCATAGGGCATGATCAACACCGCAACCTTTCCTTTTTCCCCCAGTGATTC
TGAAAAACCCCTCTTCCCTTCAGCTTGCTTAGATGTTCCAAATTTAGTAAGCTTAAGGCGGCCTAC
AGAAGAAAAAGAAAAAAAAGGCCACAAAAGTTCCCTCTCACTTTCAGTAAATAAAATAAAAGCAGC
AACAGAAATAAAGAAATAAATGAAATTCAAAATGAAATAAATATTGTGTTGTGCAGCATTAAAAAA
TCAATAAAAATTAAAAATGAGCA

(Mus musculus GNAS 3'-UTR)
SEQ ID NO: 3

GAAGGGAACCCCCAAATTTAATTAAAGCCTTAAGCACAATTAATTAAAAGTGAAACGTAATTGTAC
AAGCAGTTAATCACCCACCATAGGGCATGATTAACAAAGCAACCTTTCCCTTCCCCCGAGTGATTT
TGCGAAACCCCCTTTTCCCTTCAGCTTGCTTAGATGTTCCAAATTTAGAAAGCTTAAGGCGGCCTA
CAGAAAAAGGAAAAAAGGCCACAAAAGTTCCCTCTCACTTTCAGTAAAAATAAATAAAACAGCAGC
AGCAAACAAATAAAATGAAATAAAAGAAACAAATGAAATAAATATTGTGTTGTGCAGCATTAAAAA
AAATCAAAATAAAAATTAAATGTGAGCAAAGAATGAAAAAAAAAAAAAAAAAAA

(Homo sapiens GNAS 3'-UTR)
SEQ ID NO: 4

TGGAGGACGCCGTCCAGATTCTCCTTGTTTTCATGGATTCAGGTGCTGGAGAATCTGGTAAAAGCA
CCATTGTGAAGCAGATGAGGATCCTGCATGTTAATGGGTTTAATGGAGAGGGCGGCGAAGAGGACC
CGCAGGCTGCAAGGAGCAACAGCGATGGCAGTGAGAAGGCAACCAAAGTGCAGGACATCAAAAACA
ACCTGAAAGAGGCGATTGAAACCATTGTGGCCGCCATGAGCAACCTGGTGCCCCCCGTGGAGCTGG
CCAACCCCGAGAACCAGTTCAGAGTGGACTACATCCTGAGTGTGATGAACGTGCCTGACTTTGACT
TCCCTCCCGAATTCTATGAGCATGCCAAGGCTCTGTGGGAGGATGAAGGAGTGCGTGCCTGCTACG
AACGCTCCAACGAGTACCAGCTGATTGACTGTGCCCAGTACTTCCTGGACAAGATCGACGTGATCA
AGCAGGCTGACTATGTGCCGAGCGATCAGGACCTGCTTCGCTGCCGTGTCCTGACTTCTGGAATCT
TTGAGACCAAGTTCCAGGTGGACAAAGTCAACTTCCACATGTTTGACGTGGGTGGCCAGCGCGATG
AACGCCGCAAGTGGATCCAGTGCTTCAACGATGTGACTGCCATCATCTTCGTGGTGGCCAGCAGCA
GCTACAACATGGTCATCCGGGAGGACAACCAGACCAACCGCCTGCAGGAGGCTCTGAACCTCTTCA
AGAGCATCTGGAACAACAGATGGCTGCGCACCATCTCTGTGATCCTGTTCCTCAACAAGCAAGATC
TGCTCGCTGAGAAAGTCCTTGCTGGGAAATCGAAGATTGAGGACTACTTTCCAGAATTTGCTCGCT
ACACTACTCCTGAGGATGCTACTCCCGAGCCCGGAGAGGACCCACGCGTGACCCGGGCCAAGTACT
TCATTCGAGATGAGTTTCTGAGGATCAGCACTGCCAGTGGAGATGGGCGTCACTACTGCTACCCTC
ATTTCACCTGCGCTGTGGACACTGAGAACATCCGCCGTGTGTTCAACGACTGCCGTGACATCATTC
AGCGCATGCACCTTCGTCAGTACGAGCTGCTCTAAGAAGGGAACCCCCAAATTTAATTAAAGCCTT
AAGCACAATTAATTAAAAGTGAAACGTAATTGTACAAGCAGTTAATCACCCACCATAGGGCATGAT
TAACAAAGCAACCTTTCCCTTCCCCCGAGTGATTTTGCGAAACCCCCTTTTCCCTTCAGCTTGCTT
AGATGTTCCAAATTTAGAAAGCTTAAGGCGGCCTACAGAAAAAGGAAAAAAGGCCACAAAAGTTCC
CTCTCACTTTCAGTAAAAATAAATAAAACAGCAGCAGCAAACAAATAAAATGAAATAAAAGAAACA
AATGAAATAAATATTGTGTTGTGCAGCATTAAAAAAAATCAAAATAAAAATTAAATGTGAGCAAAG
AATGAAAAAAAAAAAAAAAAAAA

(Homo sapiens GNAS 3'-UTR)
SEQ ID NO: 5

ACCTGCTGCCTTAACGCTGAGATGTGGCCTCTGCAACCCCCCTTAGGCAAAGCAACTGAACCTTCT
GCTAAAGTGACCTGCCCTCTTCCGTAAGTCCAATAAAGTTGTCATGCACCC

(Mus musculus MORN2 3'-UTR)
SEQ ID NO: 6

ACCTGCTGCCTTAACGCTGAGATGTGGCCTCTGCAACCCCCCTTAGGCAAAGCAACTGAACCTTCT
GCTAAAGTGACCTGCCCTCTTCCGTAAGTCCAATAAAGTTGTCATGCACCCACAAAAAAAAAAAAA
AAA

(Mus musculus MORN2 3'-UTR)
SEQ ID NO: 7

CATGTAGATGTGATGTTAAATTAAAGTTGAAATGTAGTAATTGAAGCTTTTAGTTGTAAGGAAAGC
AACTTAATCTGTTATTTGAAATGACTTCATACACTACCCCTATAAGTTTGCCAATAAAACCATCAC
CTGCTTACACCTTTTTGAACTTTATATTCATTGTCTTACAATTAGTTTAAAATAAATGACATGATT
CAAAAAAAAAAA

(Homo sapiens MORN2 3'-UTR)
SEQ ID NO: 8

GCCCTTGCTACACGGGCACTCACTAGGAGGACCTGTCCACACTGGGGATCCTGCAGGCCCTGGGTG
GGGACAGCACCCTGGCCTTCTGCACTGTGGCTCCTGGTTCTCTCTCCTTCCCGCTCCCTTCTGCAG
CTTGGTCAGCCCCATCTCCTCACCCTCTTCCCAGTCAAGTCCACACAGCCTTCATTCTCCCCAGTT
TCTTTCACATGGCCCCTTCTTCATTGGCTCCCTGACCCAACCTCACAGCCCGTTTCTGCGAACTGA
GGTCTGTCCTGAACTCACGCTTCCTAGAATTACCCCGATGGTCAACACTATCTTAGTGCTAGCCCT
CCCTAGAGTTACCCCGAAGGTCAATACTTGAGTGCCAGCCTGTTCCTGGTGGAGTAGCCTCCCCAG
GTCTGTCTCGTCTACAATAAAGTCTGAAACACACTTGCCATG

(Mus musculus GSTM1 3'-UTR)
SEQ ID NO: 9

GCCCTTGCTACACGGGCACTCACTAGGAGGACCTGTCCACACTGGGGATCCTGCAGGCCCTGGGTG
GGGACAGCACCCTGGCCTTCTGCACTGTGGCTCCTGGTTCTCTCTCCTTCCCGCTCCCTTCTGCAG
CTTGGTCAGCCCCATCTCCTCACCCTCTTCCCAGTCAAGTCCACACAGCCTTCATTCTCCCCAGTT
TCTTTCACATGGCCCCTTCTTCATTGGCTCCCTGACCCAACCTCACAGCCCGTTTCTGCGAACTGA
GGTCTGTCCTGAACTCACGCTTCCTAGAATTACCCCGATGGTCAACACTATCTTAGTGCTAGCCCT
CCCTAGAGTTACCCCGAAGGTCAATACTTGAGTGCCAGCCTGTTCCTGGTGGAGTAGCCTCCCCAG
GTCTGTCTCGTCTACAATAAAGTCTGAAACACACTTGCCATGAAAAAAAAAAAAAAAAA

(Mus musculus GSTM1 3'-UTR)
SEQ ID NO: 10

GGCCTTGAAGGCCAGGAGGTGGGAGTGAGGAGCCCATACTCAGCCTGCTGCCCAGGCTGTGCAGCG
CAGCTGGACTCTGCATCCCAGCACCTGCCTCCTCGTTCCTTTCTCCTGTTTATTCCCATCTTTACT
CCCAAGACTTCATTGTCCCTCTTCACTCCCCCTAAACCCCTGTCCCATGCAGGCCCTTTGAAGCCT
CAGCTACCCACTATCCTTCGTGAACATCCCCTCCCATCATTACCCTTCCCTGCACTAAAGCCAGCC
TGACCTTCCTTCCTGTTAGTGGTTGTGTCTGCTTTAAAGGGCCTGCCTGGCCCCTCGCCTGTGGAG
CTCAGCCCCGAGCTGTCCCCGTGTTGCATGAAGGAGCAGCATTGACTGGTTTACAGGCCCTGCTCC
TGCAGCATGGTCCCTGCCTTAGGCCTACCTGATGGAAGTAAAGCCTCAACCACAAAAAAAAAAAAA
AAAAAAAAAAAAAAAAAAAAAAAAAAAAAAAAAAAAAAAAAAAAAAAAAAAAAAAAAAAAAAAAAA
AAA

(Homo sapiens GSTM1 3'-UTR)
SEQ ID NO: 11

GGAAGCATTTTCCTGGCTGATTAAAAGAAATTACTCAGCTATGGTCATCTGTTCCTGTTAGAAGGC
TATGCAGCATATTATATACTATGCGCATGTTATGAAATGCATAATAAAAAATTTTAAAAAATCTAA
A

(Mus musculus NDUFA1 3'-UTR)
SEQ ID NO: 12

GGAAGCATTTTCCTGATTGATGAAAAAAATAACTCAGTTATGGCCATCTACCCCTGCTAGAAGGTT
ACAGTGTATTATGTAGCATGCAATGTGTTATGTAGTGCTTAATAAAAATAAAATGAAAAAAATGCA
AAAAAAAAAAAAAAAA

(Homo sapiens NDUFA1 3'-UTR)
SEQ ID NO: 13

TCTGCTCAGTTGCCGCGGACATCTGAGTGGCCTTCTTAGCCCCACCCTCAGCCAAAGCATTTACTG
ATCTCGTGACTCCGCCCTCATGCTACAGCCACGCCCACCACGCAGCTCACAGTTCCACCCCCATGT
TACTGTCGATCCCACAACCACTCCAGGCGCAGACCTTGTTCTCTTTGTCCACTTTGTTGGGCTCAT
TTGCCTAAATAAACGGGCCACCGCGTTACCTTTAACTAT

(Mus musculus CBR2 3'-UTR)
SEQ ID NO: 14

ATGCCGGCTTACCATCTCTACCATCATCCGGTTTGGTCATCCAACAAGAAGAAATGAATATGAAAT
TCCAGCAATAAGAAATGAACAAAGATTGGAGCTGAAGACCTTAAGTGCTTGCTTTTTGCCCGCTGA
CCAGATAACATTAGAACTATCTGCATTATCTATGCAGCATGGGGTTTTTATTATTTTTACCTAAAG
ATGTCTCTTTTTGGTAATGACAAACGTGTTTTTTAAGAAAAAAAAAAAAAGGCCTGGTTTTTCTCAA
TACACCTTTAACGGTTTTTAAATTGTTTCATATCTGGTCAAGTTGAGATTTTTAAGAACTTCATTT
TTAATTTGTAATAAAGTTTACAACTTGATTTTTTCAAAAAAGTCAACAAACTGCAAGCACCTGTTA
ATAAAGGTCTTAAATAATAA

(Mus musculus YBX1 3'-UTR)
SEQ ID NO: 15

ATGCCGGCTTACCATCTCTACCATCATCCGGTTTGGTCATCCAACAAGAAGAAATGAATATGAAAT
TCCAGCAATAAGAAATGAACAAAGATTGGAGCTGAAGACCTTAAGTGCTTGCTTTTTGCCCTCTGA
CCAGATAACATTAGAACTATCTGCATTATCTATGCAGCATGGGGTTTTTATTATTTTTACCTAAAG
ATGTCTCTTTTTGGTAATGACAAACGTGTTTTTTAAGAAAAAAAAAAAAAAGGCCTGGTTTTTCTC
AATACACCTTTAACGGTTTTTAAATTGTTTCATATCTGGTCAAGTTGAGATTTTTAAGAACTTCAT
TTTTAATTTGTAATAAAGTTTACAACTTGATTTTTTCAAAAAAGTCAACAAACTGCAAGCACCTGT
TAATAAAGGTCTTAAATAATAA

(Mus musculus YBX1 3'-UTR)
SEQ ID NO: 16

ATGCCGGCTTACCATCTCTACCATCATCCGGTTTAGTCATCCAACAAGAAGAAATATGAAATTCCA
GCAATAAGAAATGAACAAAAGATTGGAGCTGAAGACCTAAAGTGCTTGCTTTTTGCCCGTTGACCA
GATAAATAGAACTATCTGCATTATCTATGCAGCATGGGGTTTTTATTATTTTTACCTAAAGACGTC
TCTTTTTGGTAATAACAAACGTGTTTTTTAAAAAAGCCTGGTTTTTCTCAATACGCCTTTAAAGGT
TTTTAAATTGTTTCATATCTGGTCAAGTTGAGATTTTTAAGAACTTCATTTTTAATTTGTAATAAA
AGTTTACAACTTGATTTTTTCAAAAAAGTCAACAAACTGCAAGCACCTGTTAATAAAGGTCTTAAA
TAATAAAAAAAAAAAAAAA

(Homo sapiens YBX1 3'-UTR)
SEQ ID NO: 17
GGAGGCTTGATGGGCTTTTTGCCCTCGTTCCTAGAGGCTTAACCATAATAAAATCCCTAATAAAGC
(Mus musculus Ndufb8 3'-UTR)
SEQ ID NO: 18

GGAGGCTTCGTGGGCTTTTGGGTCCTCTAACTAGGACTCCCTCATTCCTAGAAATTTAACCTTAAT
GAAATCCCTAATAAAACTCAGTGCTGTGTTATTTGTGCCTCAAAAAAAAAAAAAAAAAA

(Homo sapiens Ndufb8 3'-UTR)
SEQ ID NO: 19

GTGAGGAAGAGGAGTGCTGTTCCTGCCTTCCTAGCCCAGCTGGGTCTGACCAGAGGCTACTGTGTA
CCCATTTACCATGCGTGATTGTTAACTCAGAGTGGGGTGTAGCCAGGTATTGACTGAATGTATGTT
CTTGCTGACCTGTGTTTTTTTCTGTAGGGACCAAAGCAGTATCCTTACAATAATCTGTACCTGGAA
CGAGGCGGTGATCCCTCCAAAGAACCAGAGCGGGTGGTTCACTATGAGATCTGAGGAGGCTTCGTG
GGCTTTTGGGTCCTCTAACTAGGACTCCCTCATTCCTAGAAATTTAACCTTAATGAAATCCCTAAT
AAAACTCAGTGCTGTGTTATTTGTGCCTCAAAAAAAAAAAAAAAAAA

(Homo sapiens Ndufb8 3'-UTR)
SEQ ID NO: 20

TCGTTGACACTCACCATTTCTGTGAAAGACTTTTTTTTTTTTTAACATATTATACTAGATTTGACT
AACTCAATCTTGTAGCTTCTGCAGTTCTCCCCACCCCCAACCTAGTTCTTAGAGTATGTTTCCCCT
TTTGAAACATGTAAACATACTTTGGGCATAAATATTTTTTAAAATATAACTATAATGCTTCACTAA
TACCTTAAAAATGCCTAGTGAACTAACTCAGTACATTATATAATGGCCAAGTGAAAGTTTTGTGTT
TTCATGTCCTGTTTTTCTTTGAAATTATATAGCCCAGAAATTAGCTCATTATCTGAAAAACGTATA
AGAACTGATGAATTGTATAATACAGGAGTATTGCCATTGAATGTACTGTTTGATTTATTCAAGCAG
GTAATGAACAATGTTGTCAAACTCTCTAATGAGACATCATAATTAGGACATAAGCTAAAAGGGGCA
TTACTCCGGCAGTCTTTTTTTCTTAATCCTAGTACCATACATATTCTTTGGCATGAAAGAATGAAA
AGCATTAGTAAACAACTGAAGTCCTACCATGGCTCTGTAGGGTTTTTGGAACAATTCCTGGAATTG
GAAAGTGAAAATGGATAGCATGTGGGGGAAACCCTCATCTGAGTAGCAAGATTTTAGTAAAGATGA
CTAAGCCATTAACAGCATGCATTCATATTTAATTTTATTGACTCCTGCCATCAGCTTTTGTAGATC
TTTTGGGTGGAAGGTTGTGATTTTTACTGGGAGGACTTGAGTAGAAGTGGATGATTAAAATTGAGG
AGTATATAATTCTTTCTGGGACTGCTTAAATGTTATTGTTTGAAAATGCCTTCACTTTCCCCCTTT
GGTCAAAGAGATGTGCTTAAAATTCTTATTCCTTCACAATAAATAATTTTGATTTTCTTAGACA

(Homo sapiens CNTN1-004 3'-UTR)
SEQ ID NO: 21

TCGTTGACACTCACCATTTCTGTGAAAGACTTTTTTTTTTTTTAACATATTATACTAGATTTGACTA
ACTCAATCTTGTAGCTTCTGCAGTTCTCCCCACCCCCAACCTAGTTCTTAGAGTATGTTTCCCCTT
TTGAAACATGTAAACATACTTTGGGCATAAATATTTTTTAAAATATAACTATAATGCTTCACTAAT
ACCTTAAAAATGCCTAGTGAACTAACTCAGTACATTATATAATGGCCAAGTGAAAGTTTTGTGTTT
TCATGTCCTGTTTTTCTTTGAAATTATATAGCCCAGAAATTAGCTCATTATCTGAAAAACGTATGA
AGAACTGATGAATTGTATAATACAGGAGTATTGCCATTGAATGTACTGTTTGATTTATTCAAGCAG
GTAATGAACAATGTTGTCAAACTCTCTAATGAGACATCATAATTAGGACATAAGCTAAAAGGGGCA
TTACTCCGGCAGTCTTTTTTTCTTAATCCTAGTACCATACATATTCTTTGGCATGAAAGAATGAAA
AGCATTAGTAAACAACTGAAGTCCTACCATGGCTCTGTAGGGTTTTTGGAACAATTCCTGGAATTG
GAAAGTGAAAATGGATAGCATGTGGGGGAAACCCTCATCTGAGTAGCAAGATTTTAGTAAAGATGA
CTAAGCCATTAACAGCATGCATTCATATTTAATTTTATTGACTCCTGCCATCAGCTTTTGTAGATC
GTTTGGGTGGAAGGTTGTGATTTTTACTGGGAGGACTTGAGTAGAAGTGGATGATTAAAATTGAGG

AGTATATAATTCTTTCTGGGACTGCTTAAATGTTATTGTTTGAAAATACCTTCACTTTCCCCCTTT
GGTCAAAGAGATGTGCTTAAAATTCTTATTCCTTCACAATAAATAATTTTGATTTTCTTAGACA

(Homo sapiens CNTN1-004 3'-UTR)
SEQ ID NO: 22

*TTT*TTTCGTTGACACTCACCATTTCTGTGAAAGACTTTTTTTTTTTTTAACATATTATACTAGATT
TGACTAACTCAATCTTGTAGCTTCTGCAGTTCTCCCCACCCCCAACCTAGTTCTTAGAGTATGTTT
CCCCTTTTGAAACATGTAAACATACTTTGGGCATAAATATTTTTTAAAATATAACTATAATGCTTC
ACTAATACCTTAAAAATGCCTAGTGAACTAACTCAGTACATTATATAATGGCCAAGTGAAAGTTTT
GTGTTTTCATGTCCTGTTTTTCTTTGAAATTATATAGCCCAGAAATTAGCTCATTATCTGAAAAAC
GTATGAAGAACTGATGAATTGTATAATACAGGAGTATTGCCATTGAATGTACTGTTTGATTTATTC
AAGCAGGTAATGAACAATGTTGTCAAACTCTCTAATGAGACATCATAATTAGGACATAAGCTAAAA
GGGGCATTACTCCGGCAGTCTTTTTTTCTTAATCCTAGTACCATACATATTCTTTGGCATGAAAGA
ATGAAAAGCATTAGTAAACAACTGAAGTCCTACCATGGCTCTGTAGGGTTTTTGGAACAATTCCTG
GAATTGGAAAGTGAAAATGGATAGCATGTGGGGGAAACCCTCATCTGAGTAGCAAGATTTTAGTAA
AGATGACTAAGCCATTAACAGCATGCATTCATATTTAATTTTATTGACTCCTGCCATCAGCTTTTG
TAGATCTTTTGGGTGGAAGGTTGTGATTTTTACTGGGAGGACTTGAGTAGAAGTGGATGATTAAAA
TTGAGGAGTATATAATTCTTTCTGGGACTGCTTAAATGTTATTGTTTGAAAATGCCTTCACTTTCC
CCCTTTGGTCAAAGAGATGTGCTTAAAATTCTTATTCCTTCACAATAAATAATTTTGATTTTCTTA
GACA

(Homo sapiens CNTN1-004 3'-UTR)
SEQ ID NO: 23

ATGTGTTGTGACAGCTGCTGTTCCCATCCCAGCTCAGAAGACACCCTTCAACCCTGGGATGACCAC
AATTCCTTCCAATTTCTGCGGCTCCATCCTAAGCCAAATAAATTATACTTTAACAAACTATTCAAC
TGATTTACAACACACATGATGACTGAGGCATTCGGGAACCCCTTCATCCAAAAGAATAAACTTTTA
AATGGATATAAATGATTTTTAACTCGTTCCAATATGCCTTATAAACCACTTAACCTGATTCTGTGA
CAGTTGCATGATTTAACCCAATGGGACAAGTTACAGTGTTCAATTCAATACTATAGGCTGTAGAGT
GAAAGTCAAATCACCATATACAGGTGCTTTAAATTTAATAACAAGTTGTGAAATATAATAGAGATT
GAAATGTTGGTTGTATGTGGTAAATGTAAGAGTAATACAGTCTCTTGTACTTTCCTCACTGTTTTG
GGTACTGCATATTATTGAATGGCCCCTATCATTCATGACATCTTGAGTTTTCTTGAAAAGACAATA
GAGTGTAACAAATATTTTGTCAGAAATCCCATTATCAAATCATGAGTTGAAAGATTTTGACTATTG
AAAACCAAATTCTAGAACTTACTATCAGTATTCTTATTTTCAAAGGAAATAATTTTCTAAATATTT
GATTTTCAGAATCAGTTTTTTAATAGTAAAGTTAACATACCATATAGATTTTTTTTTACTTTTATA
TTCTACTCTGAAGTTATTTTATGCTTTTCTTATCAATTTCAAATCTCAAAAATCACAGCTCTTATC
TAGAGTATCATAATATTGCTATATTTGTTCATATGTGGAGTGACAAATTTTGAAAAGTAGAGTGCT
TCCTTTTTTATTGAGATGTGACAGTCTTTACATGGTTAGGAATAAGTGACAGTTAAGTGAATATCA
CAATTACTAGTATGTTGGTTTTTCTGCTTCATTCCTAAGTATTACGTTTCTTTATTGCAGATGTCA
GATCAAAAAGTCACCTGTAGGTTGAAAAAGCTACCGTATTCCATTTTGTAAAAATAACAATAATAA
TAATAATAATAATTAGTTTTAAGCTCATTTCCCACTTCAATGCAATACTGAAAACTGGCTAAAAAT
ACCAAATCAATATACTGCTAATGGTACTTTGAAGAGTATGCAAAACTGGAAGGCCAGGAGGAGGCA
AATAATATGTCTTTCCGATGGTGTCTCCCAAGTGTTGGTGCTTTGGGTTTTTATAAGTTGTGAAAA
GGAAGATGCACATTTCTTCATTCTCCATGGTGTGCATGGAAATGTGTTTGAGTGTGGATGTAAAAG
AAATCGAGTAATAAAGAATTAGCTGGCTTGTGAAATAGTGCAGTGTTGGATGCTTCAAGAGGTATA
ATCCTATTTTATTAGCACAAACTTGCTAGCTAATTAGAGTTTATCTTTTTAGAAAGGACACCGTAT
AGGTTCGTAAAAAATATTTACAGGAAGCAAAATAGATCTATTACTACTTTACCGACTTTACCCCCT
TTCTTTAATTTGTATAATTTTTGTACTATATATCGATGTGTAAATGTTTAGAGTCTTCATTATGAA
AATATCAATAAATATTTCATTAGTTTACATTTAACTCTGGTATAAAATGAAACTTTTAAAAATAAG
TGAAATGGATGATTTCCCAGTGGAAGTATGTCAACAGTCTTAAGATCATTGCCAGATTTCATAAAA
TATTTAAGTATTTGAAAAAGAAACAAAATGTCTTCATACTTTAGGGAAACGAATACCCTGTATACC

TTCTGTACAAATGTTTGTGTTTTCATTGTTACACTTTGGGGTTTTACTTTTGCAATGTGACCCATG
TTGGGCATTTTTATATAATCAACAACTAAATCTTTTGCCAAATGCATGCTTGCCTTTTATTTTCTA
ATATATGATAATAACGAGCAAAACTGGTTAGATTTTGCATGAAATGGTTCTGAAAGGTAAGAGGAA
AACAGACTTTGGAGGTTGTTTAGTTTTGAATTTCTGACAGAGATAAAGTAGTTTAAAATCTCTCGT
ACACTGATAACTCAAGCTTTTCATTTTCTCATACAGTTGTACAGATTTAACTGGGACCATCAGTTT
TAAACTGTTGTCAAGCTAACTAATAATCATCTGCTTTAAGACGCAAGATTCTGAATTAAACTTTAT
ATAGGTATAGATACATCTGTTGTTTCTTTGTATTTCAGGAAAGGTGATAGTAGTTTTATTTGATAC
TGATAAATATTGAATTGATTTTTTAGTTATTTTTTATCATTTTTTCAATGGAGTAGTATAGGACTG
TGCTTTGTCCTTTTTATGAATGAAAAAATTAGTATAAAGTAATAAATGTCTTATGTTACCCAAGAA
AAAA

(Homo sapiens CNTN1-004 3'-UTR)
SEQ ID NO: 24

TCGTTGACACTCACCATTTCTGTGAAAGACTTTTTTTTTTTTAACATATTATACTAGATTTGACTA
ACTCAATCTTGTAGCTTCTGCAGTTCTCCCCACCCCCAACCTAGTTCTTAGAGTATGTTTCCCCTT
TTGAAACATGTAAACATACTTTGGGCATAAATATTTTTTAAAATATAACTATAATGCTTCACTAAT
ACCTTAAAAATGCCTAGTGAACTAACTCAGTACATTATATAATGGCCAAGTGAAAGTTTTGTGTTT
TCATGTCCTGTTTTTCTTTGAAATTATATAGCCCAGAAATTAGCTCATTATCTGAAAAACGTATGA
AGAACTGATGAATTGTATAATACAGGAGTATTGCCATTGAATGTACTGTTTGATTTATTCAAGCAG
GTAATGAACAATGTTGTCAAACTCTCTAATGAGACATCATAATTAGGACATAAGCTAAAAGGGGCA
TTACTCCGGCAGTCTTTTTTTCTTAATCCTAGTACCATACATATTCTTTGGCATGAAAGAATGAAA
AGCATTAGTAAACAACTGAAGTCCTACCATGGCTCTGTAGGGTTTTTGGAACAATTCCTGGAATTG
GAAAGTGAAAATGGATAGCATGTGGGGGAAACCCTCATCTGAGTAGCAAGATTTTAGTAAAGATGA
CTAAGCCATTAACAGCATGCATTCATATTTAATTTTATTGACTCCTGCCATCAGCTTTTGTAGATC
GTTTGGGTGGAAGGTTGTGATTTTTACTGGGAGGACTTGAGTAGAAGTGGATGATTAAAATTGAGG
AGTATATAATTCTTTCTGGGACTGCTTAAATGTTATTGTTTGAAAATACCTTCACTTTCCCCCTTT
GGTCAAAGAGATGTGCTTAAAATTCTTATTCCTTCACAATAAATAATTTTGATTTTCTTAGACAGG
TTTGTGTTTAGGTATGAGTTTCTCTTTTACTTCATCTAGCAATTCTCTCTGTGGTCAGAAGAACTC
TGAAGAAAGCTTTGAGGGAAATGAATATAACTCTTAAATTATTATATGTGTGTGTATATATATAGT
TTAACTTTAAAAATAATTTATTAGTCATCATAAAGAAATAAATGTCTCTGGCTCAAGATGTTACTT
ATTTCCTTCTTTTATATTTTCTAGTCTCAATTACTGTTCCAAAAGGAGCTATCTTAGAACTTAGAC
TAGAGATCCAGATTAA

(Homo sapiens CNTN1-004 3'-UTR)

Preferably, the at least one 5'-UTR element of the artificial nucleic acid molecule according to the present invention comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the 5'-UTR sequence of a transcript of MP68 (RIKEN cDNA 2010107E04 gene), or NDUFA4 (NADH dehydrogenase (ubiquinone) 1 alpha subcomplex 4). Most preferably, the at least one 5'-UTR element of the artificial nucleic acid molecule according to the present invention comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to a sequence according to SEQ ID NO: 25 or SEQ ID NO: 30, or the corresponding RNA sequence, respectively:

SEQ ID NO: 25
CTTTCCCATTCTGTAGCAGAATTTGGTGTTGCCTGTGGTCTTGGTCCCGCGGAG
(Mus musculus MP68 5'-UTR)

SEQ ID NO: 26

CTTCCCGGCATCCCCTGCGCGCGCCTGCGCGCTCGGTGACCTTTCCGAGTTGGCTGCAGATTTGTG
GTGCGTTCTGAGCCGTCTGTCCTGCGCCAAG

(Homo sapiens MP68 5'-UTR)

SEQ ID NO: 27

CTTCCCGGCATCCCCTGCGCGCGCCTGCGCGCTCGGTGACCTTTCCGAGTTGGCTGCAGATTTGTG
GTGCGTTCTGAGCCGTCTGTCCTGCGCCAAGGGAGCGTACCTTGGCCTTGAGAGGTTCAGCTGCCT
AACCCAGAGGCTACGCAGAGTTAGAGAAGCCAGAGTCCAAGCCAAGAACTCTGACTCCACATCCAG
TCCCTTCTCTCCTTTATAACTCAAGTTTCCTTGCGCCACACTGCCCTCCACGTTATGCTGTACATG
ACAACTTGGGTGAGGCAACAGGGAAGCTGAAAAGAGATCATACGGTGCTGA

(Homo sapiens MP68 5'-UTR)

SEQ ID NO: 28

GTCCGCTCAGCCAGGTTGCAGAAGCGGCTTAGCGTGTGTCCTAATCTTCTCTCTGCGTGTAGGTA
GGCCTGTGCCGCAAAC

(Mus musculus NDUFA4 5'-UTR)

SEQ ID NO: 29

GUCCGCUCAGCCAGGUUGCAGAAGCGGCUUAGCGUGUGUCCUAAUCUUCUCUCUGCGUGUAGGUA
GGCCUGUGCCGCAAAC

(Homo sapiens NDUFA4 5'-UTR)

SEQ ID NO: 30

GGGTCCTTCAGGTAGGAGGTCCTGGGTGACTTTGGAAGTCCGTAGTGTCTCATTGCAGATAATTTT
TAGCTTAGGGCCTGGTGGCTAGGTCGGTTCTCTCCTTTCCAGTCGGAGACCTCTGCCGCAAAC

(Homo sapiens NDUFA4 5'-UTR)

The at least one 3'-UTR element of the artificial nucleic acid molecule according to the present invention may also comprise or consist of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the nucleic acid sequence of the 3'-UTR of a transcript of a gene, such as to the 3'-UTR of a sequence according to SEQ ID NOs: 1 to 24 and SEQ ID NOs: 49 to 318, wherein the fragment is preferably a functional fragment or a functional variant fragment as described above. Such fragment preferably exhibits a length of at least about 3 nucleotides, preferably of at least about 5 nucleotides, more preferably of at least about 10, 15, 20, 25 or 30 nucleotides, even more preferably of at least about 50 nucleotides, most preferably of at least about 70 nucleotides. In a preferred embodiment, the fragment or variant thereof exhibits a length of between 3 and about 500 nucleotides, preferably of between 5 and about 150 nucleotides, more preferably of between 10 and 100 nucleotides, even more preferably of between 15 and 90, most preferably of between 20 and 70. Preferably, said variants, fragments or variant fragments are functional variants, functional fragments, or functional variant fragments of the 3'-UTR, prolong protein production from the artificial nucleic acid molecule according to the invention with an efficiency of at least 30%, preferably with an efficiency of at least 40%, more preferably of at least 50%, more preferably of at least 60%, even more preferably of at least 70%, even more preferably of at least 80%, most preferably of at least 90% of the protein production prolonging efficiency exhibited by an artificial nucleic acid molecule comprising the nucleic acid sequence

selected from the group consisting of SEQ ID NO: 1 to SEQ ID NO: 24.

The at least one 5'-UTR element of the artificial nucleic acid molecule according to the present invention may also comprise or consist of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the nucleic acid sequence of the 5'-UTR of a transcript of a gene, such as to the 5'-UTR of a sequence according to SEQ ID NO: 25 or SEQ ID NO: 30 and SEQ ID NOs: 319 to 382, wherein the fragment is preferably a functional fragment or a functional variant fragment as described above. Such fragment preferably exhibits a length of at least about 3 nucleotides, preferably of at least about 5 nucleotides, more preferably of at least about 10, 15, 20, 25 or 30 nucleotides, even more preferably of at least about 50 nucleotides, most preferably of at least about 70 nucleotides. In a preferred embodiment, the fragment or variant thereof exhibits a length of between 3 and about 500 nucleotides, preferably of between 5 and about 150 nucleotides, more preferably of between 10 and 100 nucleotides, even more preferably of between 15 and 90, most preferably of between 20 and 70. Preferably, said variants, fragments or variant fragments are functional variants, functional fragments, or functional variant fragments of the 5'-UTR, increase protein production from the artificial nucleic acid molecule according to the invention with an efficiency of at least 30%, preferably with an efficiency of at least 40%, more preferably of at least 50%, more preferably of at least 60%, even more preferably of at least 70%, even more preferably of at least 80%, most preferably of at least 90% of the protein production increasing efficiency exhibited by an artificial nucleic acid molecule comprising the nucleic acid sequence selected from the group consisting of SEQ ID NO: 25 to SEQ ID NO: 30.

Preferably, the at least one 3'-UTR element and/or the at least one 5'-UTR element of the artificial nucleic acid molecule according to the present invention exhibits a length of at least about 3 nucleotides, preferably of at least about 5 nucleotides, more preferably of at least about 10, 15, 20, 25 or 30 nucleotides, even more preferably of at least about 50 nucleotides, most preferably of at least about 70 nucleotides. The upper limit for the length of the at least one 3'-UTR element and/or the at least one 5'-UTR element may be 500 nucleotides or less, e.g. 400, 300, 200, 150 or 100 nucleotides. For other embodiments the upper limit may be chosen within the range of 50 to 100 nucleotides. For example, the fragment or variant thereof may exhibit a length of between 3 and about 500 nucleotides, preferably of between 5 and about 150 nucleotides, more preferably of between 10 and 100 nucleotides, even more preferably of between 15 and 90, most preferably of between 20 and 70.

Furthermore, the artificial nucleic acid molecule according to the present invention may comprise more than one 3'-UTR elements and/or more than one 5'-UTR elements as described above. For example, the artificial nucleic acid molecule according to the present invention may comprise one, two, three, four or more 3'-UTR elements, and/or one, two, three, four or more 5'-UTR elements, wherein the individual 3'-UTR elements may be the same or they may be different, and similarly, the individual 5'-UTR elements may be the same or they may be different. For example, the artificial nucleic acid molecule according to the present invention may comprise two essentially identical 3'-UTR elements as described above, e.g. two 3'-UTR elements comprising or consisting of a nucleic acid sequence, which is derived from the 3'-UTR of a transcript of a gene, such as from a sequence according to SEQ ID NOs: 1 to 24 and SEQ ID NO: 49 to 318, or from a fragment or variant of the 3'-UTR of a transcript of a gene, functional variants thereof, functional fragments thereof, or functional variant fragments thereof as described above. Accordingly, for example, the artificial nucleic acid molecule according to the present invention may comprise two essentially identical 5'-UTR elements as described above, e.g. two 5'-UTR elements comprising or consisting of a nucleic acid sequence, which is derived from the 5'-UTR of a transcript of a gene, such as from a sequence according to SEQ ID NOs: 25 to 30 and SEQ ID NO: 319 to 382, or from a fragment or variant of the 5'-UTR of a transcript of a gene, functional variants thereof, functional fragments thereof, or functional variant fragments thereof as described above.

Surprisingly, the inventors found that an artificial nucleic acid molecule comprising a 3'-UTR element as described above and/or a 5'-UTR element as described above may represent or may provide an mRNA molecule, which allows for increased, prolonged and/or stabilized protein production. Thus, a 3'-UTR element as described herein and/or a 5'-UTR element as described herein may improve stability of protein expression from an mRNA molecule and/or improve translational efficiency.

In particular, the artificial nucleic acid molecule according to the invention may comprise (i) at least one 3'-UTR element and at least one 5'-UTR element, which prolongs and/or increases protein production; (ii) at least one 3'-UTR element, which prolongs and/or increases protein production, but no 5'-UTR element, which prolongs and/or increases protein production; or (iii) at least one 5'-UTR element, which prolongs and/or increases protein production, but no 3'-UTR element, which prolongs and/or increases protein production.

However, in particular in case (ii) and (iii), but possibly also in case (i), the artificial nucleic acid molecule according to the present invention may further comprise one or more "further 3'-UTR elements and/or 5'-UTR elements", i.e. 3'-UTR elements and/or 5'-UTR elements which do not fulfil the requirements as described above. For example, an artificial nucleic acid molecule according to the invention, which comprises a 3'-UTR element according to the present invention, i.e. a 3'-UTR element which prolongs and/or increases protein production from said artificial nucleic acid

molecule, may additionally comprise any further 3'-UTR and/or any further 5'-UTR, in particular a further 5'-UTR, e.g. a 5'-TOP UTR, or any other 5'-UTR or 5'-UTR element. Similarly for example, an artificial nucleic acid molecule according to the invention, which comprises a 5'-UTR element according to the present invention, i.e. a 5'-UTR element which prolongs and/or increases protein production from said artificial nucleic acid molecule, may additionally comprise any further 3'-UTR and/or any further 5'-UTR, in particular a further 3'-UTR, e.g. a 3'-UTR derived from a 3'-UTR of an albumin gene, particularly preferably a 3'-UTR comprising a sequence according to SEQ ID NO. 31 or 32, in particular to SEQ ID NO. 32, or any other 3'-UTR or 3'-UTR element.

If additionally to the inventive at least one 5'-UTR element and/or to the inventive at least one 3'-UTR element, which prolongs and/or increases protein production, a further 3'-UTR (element) and/ or a further 5'-UTR (element) are present in the artificial nucleic acid molecule according to the invention, the further 5'-UTR (element) and/or the further 3'-UTR (element) may interact with the inventive 3'-UTR element and/or inventive 5'-UTR element and, thus, support the increasing and/or prolonging effect of the inventive 3'-UTR element and/or of the inventive 5'-UTR element, respectively. Such further 3'-UTR and/or 5'-UTR (elements) may further support stability and translational efficiency. Moreover, if both, an inventive 3'-UTR element and an inventive 5'-UTR element are present in the artificial nucleic acid molecule according to the invention, the prolonging and/or increasing effect of the inventive 5'-UTR element and the inventive 3'-UTR element result preferably in enhanced and prolonged protein production in a synergistic way.

Preferably, the further 3'-UTR comprises or consists of a nucleic acid sequence which is derived from a 3'-UTR of a gene selected from the group consisting of an albumin gene, an $\alpha$-globin gene, a $\beta$-globin gene, a tyrosine hydroxylase gene, a lipoxygenase gene, and a collagen alpha gene, such as a collagen alpha 1(I) gene, or from a variant of a 3'-UTR of a gene selected from the group consisting of an albumin gene, an $\alpha$-globin gene, a $\beta$-globin gene, a tyrosine hydroxylase gene, a lipoxygenase gene, and a collagen alpha gene, such as a collagen alpha 1(I) gene according to SEQ ID No. 1369-1390 of the patent application WO2013/143700 whose disclosure is incorporated herein by reference. In a particularly preferred embodiment, the further 3'-UTR comprises or consists of a nucleic acid sequence which is derived from a 3'-UTR of an albumin gene, preferably a vertebrate albumin gene, more preferably a mammalian albumin gene, most preferably a human albumin gene according to SEQ ID NO. 31:

SEQ ID NO. 31:

```
CATCACATTT AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG TCTAAAAAAC
ATAAATTTCT TTAATCATTT TGCCTCTTTT CTCTGTGCTT CAATTAATAA AAAATGGAAA
GAATCT
```

(Human albumin 3'-UTR; corresponding to SEQ ID No: 1369 of the patent application WO2013/143700)

In this context it is particularly preferred that the inventive nucleic acid molecule comprises a further 3'-UTR element derived from the nucleic acids according to SEQ ID No. 1369-1390 of the patent application WO2013/143700 or a fragment, homolog or variant thereof.

Most preferably the further 3'-UTR comprises the nucleic acid sequence derived from a fragment of the human albumin gene according to SEQ ID NO. 32:

SEQ ID NO. 32:

```
CATCACATTTAAAAGCATCTCAGCCTACCATGAGAATAAGAGAAAGAAAATGAAGATCAATAGCTT
ATTCATCTCTTTTTCTTTTTCGTTGGTGTAAAGCCAACACCCTGTCTAAAAAACATAAATTTCTTT
AATCATTTTGCCTCTTTTCTCTGTGCTTCAATTAATAAAAAATGGAAAGAACCT
```

(albumin7 3'-UTR; corresponding to SEQ ID No: 1376 of the patent application WO2013/143700)

In this context it is particularly preferred that the further 3'-UTR of the inventive artificial nucleic acid molecule comprises or consists of the nucleic acid sequence according to SEQ ID NO. 32, or a corresponding RNA sequence.

The further 3'-UTR may also comprise or consist of a nucleic acid sequence derived from a ribosomal protein coding gene, whereby ribosomal protein coding genes from which a further 3'-UTR may be derived include, but are not limited to, ribosomal protein L9 (RPL9), ribosomal protein L3 (RPL3), ribosomal protein L4 (RPL4), ribosomal protein L5 (RPL5), ribosomal protein L6 (RPL6), ribosomal protein L7 (RPL7), ribosomal protein L7a (RPL7A), ribosomal protein L11 (RPL11), ribosomal protein L12 (RPL12), ribosomal protein L13 (RPL13), ribosomal protein L23 (RPL23), ribosomal

protein L18 (RPL18), ribosomal protein L18a (RPL18A), ribosomal protein L19 (RPL19), ribosomal protein L21 (RPL21), ribosomal protein L22 (RPL22), ribosomal protein L23a (RPL23A), ribosomal protein L17 (RPL17), ribosomal protein L24 (RPL24), ribosomal protein L26 (RPL26), ribosomal protein L27 (RPL27), ribosomal protein L30 (RPL30), ribosomal protein L27a (RPL27A), ribosomal protein L28 (RPL28), ribosomal protein L29 (RPL29), ribosomal protein L31 (RPL31), ribosomal protein L32 (RPL32), ribosomal protein L35a (RPL35A), ribosomal protein L37 (RPL37), ribosomal protein L37a (RPL37A), ribosomal protein L38 (RPL38), ribosomal protein L39 (RPL39), ribosomal protein, large, P0 (RPLP0), ribosomal protein, large, P1 (RPLP1), ribosomal protein, large, P2 (RPLP2), ribosomal protein S3 (RPS3), ribosomal protein S3A (RPS3A), ribosomal protein S4, X-linked (RPS4X), ribosomal protein S4, Y-linked 1 (RPS4Y1), ribosomal protein S5 (RPS5), ribosomal protein S6 (RPS6), ribosomal protein S7 (RPS7), ribosomal protein S8 (RPS8), ribosomal protein S9 (RPS9), ribosomal protein S10 (RPS10), ribosomal protein S11 (RPS11), ribosomal protein S12 (RPS12), ribosomal protein S13 (RPS13), ribosomal protein S15 (RPS15), ribosomal protein 515a (RPS15A), ribosomal protein 516 (RP516), ribosomal protein 519 (RPS19), ribosomal protein S20 (RPS20), ribosomal protein S21 (RPS21), ribosomal protein S23 (RPS23), ribosomal protein S25 (RPS25), ribosomal protein S26 (RPS26), ribosomal protein S27 (RPS27), ribosomal protein S27a (RPS27a), ribosomal protein S28 (RPS28), ribosomal protein S29 (RPS29), ribosomal protein L15 (RPL15), ribosomal protein S2 (RPS2), ribosomal protein L14 (RPL14), ribosomal protein S14 (RPS14), ribosomal protein L10 (RPL10), ribosomal protein L10a (RPL10A), ribosomal protein L35 (RPL35), ribosomal protein L13a (RPL13A), ribosomal protein L36 (RPL36), ribosomal protein L36a (RPL36A), ribosomal protein L41 (RPL41), ribosomal protein S18 (RPS18), ribosomal protein S24 (RPS24), ribosomal protein L8 (RPL8), ribosomal protein L34 (RPL34), ribosomal protein S17 (RPS17), ribosomal protein SA (RPSA), ubiquitin A-52 residue ribosomal protein fusion product 1 (UBA52), Finkel-Biskis-Reilly murine sarcoma virus (FBR-MuSV) ubiquitously expressed (FAU), ribosomal protein L22-like 1 (RPL22L1), ribosomal protein S17 (RPS17), ribosomal protein L39-like (RPL39L), ribosomal protein L10-like (RPL10L), ribosomal protein L36a-like (RPL36AL), ribosomal protein L3-like (RPL3L), ribosomal protein S27-like (RPS27L), ribosomal protein L26-like 1 (RPL26L1), ribosomal protein L7-like 1 (RPL7L1), ribosomal protein L13a pseudogene (RPL13AP), ribosomal protein L37a pseudogene 8 (RPL37AP8), ribosomal protein S10 pseudogene 5 (RPS10P5), ribosomal protein S26 pseudogene 11 (RPS26P11), ribosomal protein L39 pseudogene 5 (RPL39P5), ribosomal protein, large, P0 pseudogene 6 (RPLPOP6) and ribosomal protein L36 pseudogene 14 (RPL36P14).

Preferably, the further 5'-UTR comprises or consists of a nucleic acid sequence which is derived from the 5'-UTR of a TOP gene or which is derived from a fragment, homolog or variant of the 5'-UTR of a TOP gene.

It is particularly preferred that the 5'-UTR element does not comprise a TOP-motif or a 5'TOP, as defined above. In particular, it is preferred that a 5'-UTR of a TOP gene is a 5'-UTR of a TOP gene lacking the TOP motif.

The nucleic acid sequence which is derived from the 5'-UTR of a TOP gene is derived from a eukaryotic TOP gene, preferably a plant or animal TOP gene, more preferably a chordate TOP gene, even more preferably a vertebrate TOP gene, most preferably a mammalian TOP gene, such as a human TOP gene.

For example, the further 5'-UTR is preferably selected from 5'-UTR elements comprising or consisting of a nucleic acid sequence which is derived from a nucleic acid sequence selected from the group consisting of SEQ ID NOs. 1-1363, SEQ ID NO. 1395, SEQ ID NO. 1421 and SEQ ID NO. 1422 of the patent application WO2013/143700 whose disclosure is incorporated herein by reference, from the homologs of SEQ ID NOs. 1-1363, SEQ ID NO. 1395, SEQ ID NO. 1421 and SEQ ID NO. 1422 of the patent application WO2013/143700, from a variant thereof, or preferably from a corresponding RNA sequence. The term "homologs of SEQ ID NOs. 1-1363, SEQ ID NO. 1395, SEQ ID NO. 1421 and SEQ ID NO. 1422 of the patent application WO2013/143700" refers to sequences of other species than homo sapiens, which are homologous to the sequences according to SEQ ID NOs. 1-1363, SEQ ID NO. 1395, SEQ ID NO. 1421 and SEQ ID NO. 1422 of the patent application WO2013/143700.

In a preferred embodiment, the further 5'-UTR comprises or consists of a nucleic acid sequence which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or a corresponding RNA sequence. It is particularly preferred that the further 5'-UTR is derived from a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or a corresponding RNA sequence.

In a particularly preferred embodiment, the further 5'-UTR comprises or consists of a nucleic acid sequence which is derived from a 5'-UTR of a TOP gene encoding a ribosomal protein or from a variant of a 5'-UTR of a TOP gene encoding a ribosomal protein. For example, the 5'-UTR element comprises or consists of a nucleic acid sequence

which is derived from a 5'-UTR of a nucleic acid sequence according to any of SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360 of the patent application WO2013/143700, a corresponding RNA sequence, a homolog thereof, or a variant thereof as described herein, preferably lacking the 5'-TOP motif. As described above, the sequence extending from position 5 to the nucleotide immediately 5' to the ATG (which is located at the 3' end of the sequences) corresponds to the 5'-UTR of said sequences.

Preferably, the further 5'-UTR comprises or consists of a nucleic acid sequence which is derived from a 5'-UTR of a TOP gene encoding a ribosomal Large protein (RPL) or from a homolog or variant of a 5'-UTR of a TOP gene encoding a ribosomal Large protein (RPL). For example, the 5'-UTR element comprises or consists of a nucleic acid sequence which is derived from a 5'-UTR of a nucleic acid sequence according to any of SEQ ID NOs: SEQ ID NOs: 67, 259, 1284-1318, 1344, 1346, 1348-1354, 1357, 1358, 1421 and 1422 of the patent application WO2013/143700, a corresponding RNA sequence, a homolog thereof, or a variant thereof as described herein, preferably lacking the 5'TOP motif.

In a particularly preferred embodiment, the 5'-UTR element comprises or consists of a nucleic acid sequence which is derived from the 5'-UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, or from a variant of the 5'-UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, wherein preferably the further 5'-UTR does not comprise the 5'TOP of said gene.

Accordingly, in a particularly preferred embodiment, the further 5'-UTR comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO. 33 (5'-UTR of human ribosomal protein Large 32 lacking the 5' terminal oligopyrimidine tract: GGCGCTGCCTACGGAGGTGGCAGCCATCTCCTTCTCGGCATC (SEQ ID NO. 33); corresponding to SEQ ID NO. 1368 of the patent application WO2013/143700) or preferably to a corresponding RNA sequence, or wherein the further 5'-UTR comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO. 33 or more preferably to a corresponding RNA sequence, wherein, preferably, the fragment is as described above, i.e. being a continuous stretch of nucleotides representing at least 20% etc. of the full-length 5'-UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In some embodiments, the artificial nucleic acid molecule comprises a further 5'-UTR which comprises or consists of a nucleic acid sequence which is derived from the 5'-UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB or from a homolog or variant thereof, wherein preferably the further 5'-UTR does not comprise a TOP-motif or the 5'TOP of said genes, and wherein optionally the further 5'-UTR starts at its 5'-end with a nucleotide located at position 1, 2, 3, 4, 5, 6, 7, 8, 9 or 10 downstream of the 5'terminal oligopyrimidine tract (TOP) and wherein further optionally the further 5'-UTR which is derived from a 5'-UTR of a TOP gene terminates at its 3'-end with a nucleotide located at position 1, 2, 3, 4, 5, 6, 7, 8, 9 or 10 upstream of the start codon (A(U/T)G) of the gene it is derived from.

The artificial nucleic acid molecule according to the present invention may be RNA, such as mRNA or viral RNA or a replicon, DNA, such as a DNA plasmid or viral DNA, or may be a modified RNA or DNA molecule. It may be provided as a double-stranded molecule having a sense strand and an anti-sense strand, for example, as a DNA molecule having a sense strand and an anti-sense strand.

The artificial nucleic acid molecule according to the present invention may further comprise optionally a 5'-cap.

The optional 5'-cap is preferably located 5' to the ORF, more preferably 5' to the at least one 5'-UTR or to any further 5'-UTR within the artificial nucleic acid molecule according to the present invention.

Preferably, the artificial nucleic acid molecule according to the present invention further comprises a poly(A) sequence and/or a polyadenylation signal. Preferably, the optional poly(A) sequence is located 3' to the at least one 3'-UTR element or to any further 3'-UTR, more preferably the optional poly(A) sequence is connected to the 3'-end of an 3'-UTR element. The connection may be direct or indirect, for example, via a stretch of 2, 4, 6, 8, 10, 20 etc. nucleotides, such as via a linker of 1-50, preferably of 1-20 nucleotides, e.g. comprising or consisting of one or more restriction sites. However, even if the artificial nucleic acid molecule according to the present invention does not comprise a 3'-UTR, for example if it only comprises at least one 5'-UTR element, it preferably still comprises a poly(A) sequence and/or a polyadenylation signal.

In one embodiment, the optional polyadenylation signal is located downstream of the 3' of the 3'-UTR element. Preferably, the polyadenylation signal comprises the consensus sequence NN(U/T)ANA, with N = A or U, preferably AA(U/T)AAA or A(U/T)(U/T)AAA. Such consensus sequence may be recognised by most animal and bacterial cell-systems, for example by the polyadenylation-factors, such as cleavage/polyadenylation specificity factor (CPSF) cooperating with CstF, PAP, PAB2, CFI and/or CFII. Preferably, the polyadenylation signal, preferably the consensus sequence NNUANA, is located less than about 50 nucleotides, more preferably less than about 30 bases, most preferably less than about 25 bases, for example 21 bases, downstream of the 3'-end of the 3'-UTR element or of the ORF, if no 3'-UTR element is present.

Transcription of an artificial nucleic acid molecule according to the present invention, e.g. of an artificial DNA molecule, comprising a polyadenylation signal downstream of the 3'-UTR element (or of the ORF) will result in a premature-RNA containing the polyadenylation signal downstream of its 3'-UTR element (or of the ORF).

Using an appropriate transcription system will then lead to attachment of a poly(A) sequence to the premature-RNA. For example, the inventive artificial nucleic acid molecule may be a DNA molecule comprising a 3'-UTR element as described above and a polyadenylation signal, which may result in polyadenylation of an RNA upon transcription of this DNA molecule. Accordingly, a resulting RNA may comprise a combination of the inventive 3'-UTR element followed by a poly(A) sequence.

Potential transcription systems are *in vitro* transcription systems or cellular transcription systems etc. Accordingly, transcription of an artificial nucleic acid molecule according to the invention, e.g. transcription of an artificial nucleic acid molecule comprising an open reading frame, a 3'-UTR element and/or a 5'-UTR element and optionally a polyadenylation-signal, may result in an mRNA molecule comprising an open reading frame, a 3'-UTR element and optionally a poly(A) sequence.

Accordingly, the invention also provides an artificial nucleic acid molecule, which is an mRNA molecule comprising an open reading frame, a 3'-UTR element as described above and/or a 5'-UTR element as described above and optionally a poly(A) sequence.

In another embodiment, the 3'-UTR of the artificial nucleic acid molecule according to the invention does not comprise a polyadenylation signal or a poly(A) sequence. Further preferably, the artificial nucleic acid molecule according to the invention does not comprise a polyadenylation signal or a poly(A) sequence. More preferably, the 3'-UTR of the artificial nucleic acid molecule, or the inventive artificial nucleic acid molecule as such, does not comprise a polyadenylation signal, in particular it does not comprise the polyadenylation signal AAU/TAAA.

In a preferred embodiment, the invention provides an artificial nucleic acid molecule which is an artificial RNA molecule comprising an open reading frame and an RNA sequence corresponding to a DNA sequence selected from the group consisting of sequences according to SEQ ID NOs: 1 to 30, preferably from the group consisting of sequences according to SEQ ID NO. 1, SEQ ID NO. 5, SEQ ID NO. 8, SEQ ID NO. 11, SEQ ID NO. 13, SEQ ID NO. 14, SEQ ID NO. 17, SEQ ID NO. 20, SEQ ID NO. 25 and SEQ ID NO. 28, or sequences having an identity of at least about 40 % or more to SEQ ID NOs: 1 to 30, preferably to SEQ ID NO. 1, SEQ ID NO. 5, SEQ ID NO. 8, SEQ ID NO. 11, SEQ ID NO. 13, SEQ ID NO. 14, SEQ ID NO. 17, SEQ ID NO. 20, SEQ ID NO. 25 and SEQ ID NO. 28 or a fragment thereof as described above. Moreover, a corresponding artificial DNA molecule is also provided.

In another preferred embodiment, the invention provides an artificial nucleic acid molecule which is an artificial DNA molecule comprising an open reading frame and a sequence selected from the group consisting of sequences according to SEQ ID NOs: 1 to 30, preferably from the group consisting of sequences according to SEQ ID NO. 1, SEQ ID NO. 5, SEQ ID NO. 8, SEQ ID NO. 11, SEQ ID NO. 13, SEQ ID NO. 14, SEQ ID NO. 17, SEQ ID NO. 20, SEQ ID NO. 25 and SEQ ID NO. 28, or sequences having an identity of at least about 40% or more to SEQ ID NOs: 1 to 30, preferably to SEQ ID NO. 1, SEQ ID NO. 5, SEQ ID NO. 8, SEQ ID NO. 11, SEQ ID NO. 13, SEQ ID NO. 14, SEQ ID NO. 17, SEQ ID NO. 20, SEQ ID NO. 25 and SEQ ID NO. 28.

Accordingly, the invention provides an artificial nucleic acid molecule which may serve as a template for an RNA molecule, preferably for an mRNA molecule, which is stabilised and optimized with respect to translation efficiency. In other words, the artificial nucleic acid molecule may be a DNA which may be used as a template for production of an mRNA. The obtainable mRNA, may, in turn, be translated for production of a desired peptide or protein encoded by the

open reading frame. If the artificial nucleic acid molecule is a DNA, it may, for example, be used as a double-stranded storage form for continued and repetitive *in vitro* or *in vivo* production of mRNA. Thereby, *in vitro* refers in particular to ("living") cells and/or tissue, including tissue of a living subject. Cells include in particular cell lines, primary cells, cells in tissue or subjects. In specific embodiments cell types allowing cell culture may be suitable for the present invention. Particularly preferred are mammalian cells, e.g. human cells and mouse cells. In particularly preferred embodiments the human cell lines HeLa, and U-937 and the mouse cell lines NIH3T3, JAWSII and L929 are used. Furthermore primary cells are particularly preferred, in particular preferred embodiments human dermal fibroblasts (HDF) may be used. Alternatively also a tissue of a subject may be used.

In one embodiment, the artificial nucleic acid molecule according to the present invention further comprises a poly(A) sequence. For example, a DNA molecule comprising an ORF, optionally followed by a 3' UTR, may contain a stretch of thymidine nucleotides which can be transcribed into a poly(A) sequence in the resulting mRNA. The length of the poly(A) sequence may vary. For example, the poly(A) sequence may have a length of about 20 adenine nucleotides up to about 300 adenine nucleotides, preferably of about 40 to about 200 adenine nucleotides, more preferably from about 50 to about 100 adenine nucleotides, such as about 60, 70, 80, 90 or 100 adenine nucleotides. Most preferably, the inventive nucleic acid comprises a poly(A) sequence of about 60 to about 70 nucleotides, most preferably 64 adenine nucleotides.

Artificial RNA-molecules may also be obtainable *in vitro* by common methods of chemical-synthesis without being necessarily transcribed from a DNA-progenitor.

In a particularly preferred embodiment, the artificial nucleic acid molecule according to the present invention is an RNA molecule, preferably an mRNA molecule comprising in 5'-to-3'-direction an open reading frame, a 3'-UTR element as described above and a poly(A) sequence or comprising in 5'-to-3'-direction a 5'-UTR element as described above, an open reading frame and a poly(A) sequence.

In a preferred embodiment, the open reading frame is derived from a gene, which is distinct from the gene from which the 3'-UTR element and/or the 5'-UTR element of the inventive artificial nucleic acid is derived. In some further preferred embodiments, the open reading frame does not code for a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), CNTN1 (contactin 1), preferably CNTN1-004 or variants thereof, provided that the 3'-UTR element and/or the 5'-UTR element is a sequence which is selected from the group consisting of sequences according to SEQ ID NO. 1 to SEQ ID NO. 30.

In a preferred embodiment, the ORF does not encode human or plant, in particular Arabidopsis, ribosomal proteins, in particular does not encode human ribosomal protein S6 (RPS6), human ribosomal protein L36a-like (RPL36AL) or Arabidopsis ribosomal protein S16 (RPS16). In a further preferred embodiment, the open reading frame (ORF) does not encode ribosomal protein S6 (RPS6), ribosomal protein L36a-like (RPL36AL) or ribosomal protein S16 (RPS16) of whatever origin.

In one embodiment, the invention provides an artificial DNA molecule comprising an open reading frame, preferably an open reading frame derived from a gene, which is distinct from the gene from which the 3'-UTR element and/or the 5'-UTR element is derived; a 3'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to a DNA sequence selected from the group consisting of sequences according to SEQ ID NO. 1 to 24, and/or a 5'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to a DNA sequence selected from the group consisting of sequences according to SEQ ID NO. 25 to 30; and a polyadenylation signal and/or a poly(A) sequence.

In a further embodiment there is provided a composition comprising a plurality of RNA molecules of the embodiments in pharmaceutically acceptable carrier, wherein at least about 90%, 91%, 92%, 93%, 94%, 95%, 96%, 97%, 98%, 99% or greater of the RNA in the composition comprises a poly(A) sequence that differs in length by no nor that 10 nucleotides. In a preferred embodiment at least about 90%, 91%, 92%, 93%, 94%, 95%, 96%, 97%, 98%, 99% or greater of the RNA in the composition comprises a poly(A) sequence of identical length. In certain embodiments, the poly(A) sequence is positioned at the 3' end of the RNA, with no other nucleotides positioned 3' relative the poly(A) sequence. In still a further embodiment, there is provided a composition comprising a plurality of RNA molecules of the embodiments in pharmaceutically acceptable carrier, wherein said plurality of RNA molecules comprise both capped and uncapped RNAs. For example, in some aspects, a composition comprises a plurality of RNA molecules wherein no more than 95%, 90%, 80%, 70% or 60% of the RNAs comprise a cap and the remaining RNA molecules are uncapped. Furthermore, the invention provides an artificial RNA molecule, preferably an artificial mRNA molecule or an artificial viral RNA molecule, comprising an open reading frame, preferably an open reading frame is derived from a gene, which

is distinct from the gene from which the 3'-UTR element and/or the 5'-UTR element is derived; a 3'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to an RNA sequence corresponding to a DNA sequence selected from the group consisting of sequences according to SEQ ID NO. 1 to 24, and/or a 5'-UTR element comprising or consisting of a sequence which has at least about 60%, preferably at least about 70%, more preferably at least about 80%, more preferably at least about 90%, even more preferably at least about 95%; even more preferably at least 99%; even more preferably 100% sequence identity to an RNA sequence corresponding to a DNA sequence selected from the group consisting of sequences according to SEQ ID NO. 25 to 30; and a polyadenylation signal and/or a poly(A) sequence.

The invention provides an artificial nucleic acid molecule, preferably an artificial mRNA, which may be characterized by increased and/or prolonged expression of the encoded peptide or protein. Without being bound by any theory, enhanced stability of protein expression and thus prolonged protein expression may result from reduction in degradation of the artificial nucleic acid molecule, such as an artificial mRNA molecule according to the present invention. Accordingly, the inventive 3'-UTR element and/or the inventive 5'-UTR element may prevent the artificial nucleic acid from degradation and decay.

Preferably, the artificial nucleic acid molecule may additionally comprise a histone stem-loop. Thus, an artificial nucleic acid molecule according to the present invention may, for example, comprise in 5'-to-3'-direction an ORF, a 3'-UTR element, an optional histone stem-loop sequence, an optional poly(A) sequence or polyadenylation signal and an optional poly(C) sequence or in 5'-to-3'-direction an 5'-UTR element, an ORF, an optional histone stem-loop sequence, an optional poly(A) sequence or polyadenylation signal and an optional poly(C) sequence or in 5'-to-3'-direction an 5'-UTR element, an ORF, a 3'-UTR element, an optional histone stem-loop sequence, an optional poly(A) sequence or polyadenylation signal and an optional poly(C) sequence. It may also comprise in 5'-to-3'-direction an ORF, an 3'-UTR element, an optional poly(A) sequence, an optional poly (C) sequence and an optional histone stem-loop sequence, or in 5'-to-3'-direction an 5'-UTR element, an ORF, an optional poly(A) sequence, an optional poly(C) sequence and an optional histone stem-loop sequence, or in 5'-to-3'-direction an 5'-UTR element, an ORF, a 3'-UTR element, an optional poly(A) sequence, an optional poly(C) sequence and an optional histone stem-loop sequence.

In a preferred embodiment, the artificial nucleic acid molecule according to the invention further comprises at least one histone stem-loop sequence.

Such histone stem-loop sequences are preferably selected from histone stem-loop sequences as disclosed in WO 2012/019780, whose disclosure is incorporated herewith by reference.

A histone stem-loop sequence, suitable to be used within the present invention, is preferably selected from at least one of the following formulae (I) or (II):

formula (I) (stem-loop sequence without stem bordering elements):

$$\underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}$$

formula (II) (stem-loop sequence with stem bordering elements):

$$\underbrace{N_{1-6}}_{\substack{\text{stem1}\\ \text{bordering element}}}\ \underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}\ \underbrace{N_{1-6}}_{\substack{\text{stem2}\\ \text{bordering element}}}$$

wherein:

stem1 or stem2 bordering elements $N_{1-6}$ — is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue there-

of;

| | |
|---|---|
| stem1 [$N_{0-2}GN_{3-5}$] | is reverse complementary or partially reverse complementary with element stem2, and is a consecutive sequence between of 5 to 7 nucleotides;<br>wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof;<br>wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof, and<br>wherein G is guanosine or an analogue thereof, and may be optionally replaced by a cytidine or an analogue thereof, provided that its complementary nucleotide cytidine in stem2 is replaced by guanosine; |
| loop sequence [$N_{0-4}(U/T)N_{0-4}$] | is located between elements stem1 and stem2, and is a consecutive sequence of 3 to 5 nucleotides, more preferably of 4 nucleotides;<br>wherein each $N_{0-4}$ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and wherein U/T represents uridine, or optionally thymidine; |
| stem2 [$N_{3-5}CN_{0-2}$] | is reverse complementary or partially reverse complementary with element stem1, and is a consecutive sequence between of 5 to 7 nucleotides;<br>wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof;<br>wherein $N_{0-2}$ is a consecutive sequence of 0 to 2, preferably of 0 to 1, more preferably of 1 N, wherein each N is independently from another selected from a nucleotide selected from A, U, T, G or C or a nucleotide analogue thereof; and<br>wherein C is cytidine or an analogue thereof, and may be optionally replaced by a guanosine or an analogue thereof provided that its complementary nucleoside guanosine in stem1 is replaced by cytidine; |

wherein
stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one ore more bases in one stem do not have a complementary base in the reverse complementary sequence of the other stem.

According to a further preferred embodiment the histone stem-loop sequence may be selected according to at least one of the following specific formulae (Ia) or (IIa):

formula (Ia) (stem-loop sequence without stem bordering elements):

$$\underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}$$

formula (IIa) (stem-loop sequence with stem bordering elements):

$$\underbrace{N_{2-5}}_{\text{stem1 bordering element}}\ \underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}\ \underbrace{N_{2-5}}_{\text{stem2 bordering element}}$$

wherein:

N, C, G, T and U are as defined above.

According to a further more particularly preferred embodiment of the first aspect, the artificial nucleic acid molecule sequence may comprise at least one histone stem-loop sequence according to at least one of the following specific formulae (Ib) or (IIb):

formula (Ib) (stem-loop sequence without stem bordering elements):

$$\underbrace{[N_1GN_4]}_{\text{stem1}}\ \underbrace{[N_2(U/T)N_1]}_{\text{loop}}\ \underbrace{[N_4CN_1]}_{\text{stem2}}$$

formula (IIb) (stem-loop sequence with stem bordering elements):

$$\underbrace{N_{4-5}}_{\text{stem1 bordering element}}\ \underbrace{[N_1GN_4]}_{\text{stem1}}\ \underbrace{[N_2(U/T)N_1]}_{\text{loop}}\ \underbrace{[N_4CN_1]}_{\text{stem2}}\ \underbrace{N_{4-5}}_{\text{stem2 bordering element}}$$

wherein:

N, C, G, T and U are as defined above.

A particular preferred histone stem-loop sequence is the sequence according to SEQ ID NO: 34: CAAAGGCTCTTTTCAGAGCCACCA or more preferably the corresponding RNA sequence of the nucleic acid sequence according to SEQ ID NO: 34.

As an example, the single elements may be present in the artificial nucleic acid molecule in the following order:

5'-cap - 5'-UTR (element) - ORF - 3'-UTR (element) - histone stem-loop - poly(A)/(C) sequence;
5'-cap - 5'-UTR (element) - ORF - 3'-UTR (element) - poly(A)/(C) sequence - histone stem-loop;
5'-cap - 5'-UTR (element) - ORF - IRES - ORF - 3'-UTR (element) - histone stem-loop - poly(A)/(C) sequence;
5'-cap - 5'-UTR (element) - ORF - IRES - ORF - 3'-UTR (element) - histone stem-loop - poly(A)/(C) sequence - poly(A)/(C) sequence;
5'-cap - 5'-UTR (element) - ORF - IRES - ORF - 3'-UTR (element) - poly(A)/(C) sequence - histone stem-loop;
5'-cap - 5'-UTR (element) - ORF - IRES - ORF - 3'-UTR (element) - poly(A)/(C) sequence - poly(A)/(C) sequence - histone stem-loop;
5'-cap - 5'-UTR (element) - ORF - 3'-UTR (element) - poly(A)/(C) sequence - poly(A)/(C) sequence;

5'-cap - 5'-UTR (element) - ORF - 3'-UTR (element) - poly(A)/(C) sequence - poly(A)/(C) sequence - histone stem loop; etc.

In some embodiments, the artificial nucleic acid molecule comprises further elements such as a 5'-cap, a poly(C) sequence and/or an IRES-motif. A 5'-cap may be added during transcription or post-transcriptionally to the 5'end of an RNA. Furthermore, the inventive artificial nucleic acid molecule, particularly if the nucleic acid is in the form of an mRNA or codes for an mRNA, may be modified by a sequence of at least 10 cytidines, preferably at least 20 cytidines, more preferably at least 30 cytidines (so-called "poly(C) sequence"). In particular, the inventive artificial nucleic acid molecule may contain, especially if the nucleic acid is in the form of an (m)RNA or codes for an mRNA, a poly(C) sequence of typically about 10 to 200 cytidine nucleotides, preferably about 10 to 100 cytidine nucleotides, more preferably about 10 to 70 cytidine nucleotides or even more preferably about 20 to 50 or even 20 to 30 cytidine nucleotides. Most preferably, the inventive nucleic acid comprises a poly(C) sequence of 30 cytidine residues. Thus, preferably the artificial nucleic acid molecule according to the present invention comprises, preferably in 5'-to-3' direction, at least one 5'-UTR element as described above, an ORF, at least one 3'-UTR element as described above, a poly(A) sequence or a polyadenylation signal, and a poly(C) sequence or, in 5'-to-3' direction, optionally a further 5'-UTR, an ORF, at least one 3'-UTR element as described above, a poly(A) sequence or a polyadenylation signal, and a poly(C) sequence, or, in 5'-to-3' direction, at least one 5'-UTR element as described above, an ORF, optionally a further 3'-UTR, a poly(A) sequence or a polyadenylation signal, and a poly(C) sequence .

An internal ribosome entry site (IRES) sequence or IRES-motif may separate several open reading frames, for example if the artificial nucleic acid molecule encodes for two or more peptides or proteins. An IRES-sequence may be particularly helpful if the artificial nucleic acid molecule is a bi- or multicistronic nucleic acid molecule.

Furthermore, the artificial nucleic acid molecule may comprise additional 5'-elements, preferably a promoter or a promoter containing-sequence. The promoter may drive and or regulate transcription of the artificial nucleic acid molecule according to the present invention, for example of an artificial DNA-molecule according to the present invention.

Preferably, the artificial nucleic acid molecule according to the present invention, preferably the open reading frame, is at least partially G/C modified. Thus, the inventive artificial nucleic acid molecule may be thermodynamically stabilized by modifying the G (guanosine)/C (cytidine) content of the molecule. The G/C content of the open reading frame of an artificial nucleic acid molecule according to the present invention may be increased compared to the G/C content of the open reading frame of a corresponding wild type sequence, preferably by using the degeneration of the genetic code. Thus, the encoded amino acid sequence of the artificial nucleic acid molecule is preferably not modified by the G/C modification compared to the coded amino acid sequence of the particular wild type sequence. The codons of the coding sequence or the whole artificial nucleic acid molecule, e.g. an mRNA, may therefore be varied compared to the wild type coding sequence, such that they include an increased amount of G/C nucleotides while the translated amino acid sequence is maintained. Due to the fact that several codons code for one and the same amino acid (so-called degeneration of the genetic code), it is feasible to alter codons while not altering the encoded peptide/protein sequence (so-called alternative codon usage). Hence, it is possible to specifically introduce certain codons (in exchange for the respective wild-type codons encoding the same amino acid), which are more favourable with respect to stability of RNA and/or with respect to codon usage in a subject (so-called codon optimization).

Depending on the amino acid to be encoded by the coding region of the inventive artificial nucleic acid molecule as defined herein, there are various possibilities for modification of the nucleic acid sequence, e.g. the open reading frame, compared to its wild type coding region. In the case of amino acids, which are encoded by codons which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U/T is present.

In contrast, codons which contain A and/or U/T nucleotides may be modified by substitution of other codons which code for the same amino acids but contain no A and/or U/T. For example
the codons for Pro can be modified from CC(U/T) or CCA to CCC or CCG;
the codons for Arg can be modified from CG(U/T) or CGA or AGA or AGG to CGC or CGG;
the codons for Ala can be modified from GC(U/T) or GCA to GCC or GCG;
the codons for Gly can be modified from GG(U/T) or GGA to GGC or GGG.

In other cases, although A or (U/T) nucleotides cannot be eliminated from the codons, it is however possible to decrease the A and (U/T) content by using codons which contain a lower content of A and/or (U/T) nucleotides. Examples of these are:

The codons for Phe can be modified from (U/T)(U/T)(U/T) to (U/T) (U/T)C;
the codons for Leu can be modified from (U/T) (U/T)A, (U/T) (U/T)G, C(U/T) (U/T) or C(U/T)A to C(U/T)C or C(U/T)G;
the codons for Ser can be modified from (U/T)C(U/T) or (U/T)CA or AG(U<T) to (U/T)CC, (U/T)CG or AGC;
the codon for Tyr can be modified from (U/T)A(U/T) to (U/T)AC;
the codon for Cys can be modified from (U/T)G(U/T) to (U/T)GC;

the codon for His can be modified from CA(U/T) to CAC;
the codon for Gln can be modified from CAA to CAG;
the codons for Ile can be modified from A(U/T)(U/T) or A(U/T)A to A(U/T)C;
the codons for Thr can be modified from AC(U/T) or ACA to ACC or ACG;
the codon for Asn can be modified from AA(U/T) to AAC;
the codon for Lys can be modified from AAA to AAG;
the codons for Val can be modified from G(U/T)(U/T) or G(U/T)A to G(U/T)C or G(U/T)G;
the codon for Asp can be modified from GA(U/T) to GAC;
the codon for Glu can be modified from GAA to GAG;
the stop codon (U/T)AA can be modified to (U/T)AG or (U/T)GA.

In the case of the codons for Met (A(U/T)G) and Trp ((U/T)GG), on the other hand, there is no possibility of sequence modification without altering the encoded amino acid sequence. The substitutions listed above can be used either individually or in all possible combinations to increase the G/C content of the open reading frame of the inventive artificial nucleic acid molecule as defined herein, compared to its particular wild type open reading frame (i.e. the original sequence). Thus, for example, all codons for Thr occurring in the wild type sequence can be modified to ACC (or ACG).

Preferably, the G/C content of the open reading frame of the inventive artificial nucleic acid molecule as defined herein is increased by at least 7%, more preferably by at least 15%, particularly preferably by at least 20%, compared to the G/C content of the wild type coding region without altering the encoded amino acid sequence, i.e. using the degeneracy of the genetic code. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the open reading frame of the inventive artificial nucleic acid molecule or a fragment, variant or derivative thereof are substituted, thereby increasing the G/C content of said open reading frame.

In this context, it is particularly preferable to increase the G/C content of the open reading frame of the inventive artificial nucleic acid molecule as defined herein, to the maximum (i.e. 100% of the substitutable codons), compared to the wild type open reading frame, without altering the encoded amino acid sequence.

Furthermore, the open reading frame is preferably at least partially codon-optimized. Codon-optimization is based on the finding that the translation efficiency may be determined by a different frequency in the occurrence of transfer RNAs (tRNAs) in cells. Thus, if so-called "rare codons" are present in the coding region of the inventive artificial nucleic acid molecule as defined herein, to an increased extent, the translation of the corresponding modified nucleic acid sequence is less efficient than in the case where codons coding for relatively "frequent" tRNAs are present.

Thus, the open reading frame of the inventive artificial nucleic acid molecule is preferably modified compared to the corresponding wild type coding region such that at least one codon of the wild type sequence which codes for a tRNA which is relatively rare in the cell is exchanged for a codon which codes for a tRNA which is comparably frequent in the cell and carries the same amino acid as the relatively rare tRNA. By this modification, the open reading frame of the inventive artificial nucleic acid molecule as defined herein, is modified such that codons for which frequently occurring tRNAs are available may replace codons which correspond to rare tRNAs. In other words, according to the invention, by such a modification all codons of the wild type open reading frame which code for a rare tRNA may be exchanged for a codon which codes for a tRNA which is more frequent in the cell and which carries the same amino acid as the rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. Accordingly, preferably, the open reading frame is codon-optimized, preferably with respect to the system in which the artificial nucleic acid molecule according to the present invention is to be expressed, preferably with respect to the system in which the artificial nucleic acid molecule according to the present invention is to be translated. Preferably, the codon usage of the open reading frame is codon-optimized according to mammalian codon usage, more preferably according to human codon usage. Preferably, the open reading frame is codon-optimized and G/C-content modified.

For further improving degradation resistance, e.g. resistance to *in vivo* (or *in vitro* as defined above) degradation by an exo- or endonuclease, and/or for further improving stability of protein expression from the artificial nucleic acid molecule according to the present invention, the artificial nucleic acid molecule may further comprise modifications, such as backbone modifications, sugar modifications and/or base modifications, e.g., lipid-modifications or the like. Preferably, the transcription and/or the translation of the artificial nucleic acid molecule according to the present invention is not significantly impaired by said modifications.

Generally, the artificial nucleic acid molecule of the present invention may comprise any native (= naturally occurring) nucleotide, e.g. guanosine, uracil, adenosine, and/or cytosine or an analogue thereof. In this respect, nucleotide analogues are defined as natively and non-natively occurring variants of the naturally occurring nucleotides adenosine, cytosine, thymidine, guanosine and uridine. Accordingly, analogues are e.g. chemically derivatized nucleotides with non-natively occurring functional groups, which are preferably added to or deleted from the naturally occurring nucleotide or which substitute the naturally occurring functional groups of a nucleotide. Accordingly, each component of the naturally

occurring nucleotide may be modified, namely the base component, the sugar (ribose) component and/or the phosphate component forming the backbone (see above) of the RNA sequence. Analogues of guanosine, uridine, adenosine, thymidine and cytosine include, without implying any limitation, any natively occurring or non-natively occurring guanosine, uridine, adenosine, thymidine or cytosine that has been altered e.g. chemically, for example by acetylation, methylation, hydroxylation, etc., including 1-methyl-adenosine, 1-methyl-guanosine, 1-methyl-inosine, 2,2-dimethyl-guanosine, 2,6-diaminopurine, 2'-Amino-2'-deoxyadenosine, 2'-Amino-2'-deoxycytidine, 2'-Amino-2'-deoxyguanosine, 2'-Amino-2'-deoxyuridine, 2-Amino-6-chloropurineriboside, 2-Aminopurine-riboside, 2'-Araadenosine, 2'-Aracytidine, 2'-Arauridine, 2'-Azido-2'-deoxyadenosine, 2'-Azido-2'-deoxycytidine, 2'-Azido-2'-deoxyguanosine, 2'-Azido-2'-deoxyuridine, 2-Chloroadenosine, 2'-Fluoro-2'-deoxyadenosine, 2'-Fluoro-2'-deoxycytidine, 2'-Fluoro-2'-deoxyguanosine, 2'-Fluoro-2'-deoxyuridine, 2'-Fluorothymidine, 2-methyl-adenosine, 2-methyl-guanosine, 2-methyl-thio-N6-isopenenyl-adenosine, 2'-O-Methyl-2-aminoadenosine, 2'-O-Methyl-2'-deoxyadenosine, 2'-O-Methyl-2'-deoxycytidine, 2'-O-Methyl-2'-deoxyguanosine, 2'-O-Methyl-2'-deoxyuridine, 2'-O-Methyl-5-methyluridine, 2'-O-Methylinosine, 2'-O-Methylpseudouridine, 2-Thiocytidine, 2-thio-cytosine, 3-methyl-cytosine, 4-acetyl-cytosine, 4-Thiouridine, 5-(carboxyhydroxymethyl)-uracil, 5,6-Dihydrouridine, 5-Aminoallylcytidine, 5-Aminoallyl-deoxy-uridine, 5-Bromouridine, 5-carboxymehtylaminomethyl-2-thio-uracil, 5-carboxymethylamonomethyl-uracil, 5-Chloro-Ara-cytosine, 5-Fluoro-uridine, 5-Iodouridine, 5-methoxycarbonylmethyl-uridine, 5-methoxy-uridine, 5-methyl-2-thio-uridine, 6-Azacytidine, 6-Azauridine, 6-Chloro-7-deaza-guanosine, 6-Chloropurineriboside, 6-Mercapto-guanosine, 6-Methyl-mercaptopurine-riboside, 7-Deaza-2'-deoxy-guanosine, 7-Deazaadenosine, 7-methyl-guanosine, 8-Azaadenosine, 8-Bromo-adenosine, 8-Bromo-guanosine, 8-Mercapto-guanosine, 8-Oxoguanosine, Benzimidazole-riboside, Beta-D-mannosyl-queosine, Dihydro-uracil, Inosine, N1-Methyladenosine, N6-([6-Aminohexyl]carbamoylmethyl)-adenosine, N6-isopentenyl-adenosine, N6-methyl-adenosine, N7-Methyl-xanthosine, N-uracil-5-oxyacetic acid methyl ester, Puromycin, Queosine, Uracil-5-oxyacetic acid, Uracil-5-oxyacetic acid methyl ester, Wybutoxosine, Xanthosine, and Xylo-adenosine. The preparation of such analogues is known to a person skilled in the art, for example from US Patents 4,373,071, US 4,401,796, US 4,415,732, US 4,458,066, US 4,500,707, US 4,668,777, US 4,973,679, US 5,047,524, US 5,132,418, US 5,153,319, US 5,262,530 and 5,700,642. In the case of an analogue as described above, particular preference may be given according to certain embodiments of the invention to those analogues that increase the protein expression of the encoded peptide or protein or that increase the immunogenicity of the artificial nucleic acid molecule of the invention and/or do not interfere with a further modification of the artificial nucleic acid molecule that has been introduced.

According to a particular embodiment, the artificial nucleic acid molecule of the present invention can contain a lipid modification.

In a preferred embodiment, the artificial nucleic acid molecule comprises, preferably from 5' to 3' direction, the following elements:

a 5'-UTR element which prolongs and/or increases protein production from said artificial nucleic acid molecule, preferably from a nucleic acid sequence according to any of SEQ ID NO: 25 to 30 and SEQ ID NOs: 319 to 382, more preferably of the 5'-UTR of MP68 or NDUFA4; or a further 5'-UTR, preferably a 5'-TOP UTR;

at least one open reading frame (ORF), wherein the ORF preferably comprises at least one modification with respect to the wild type sequence;

a 3'-UTR element which prolongs and/or increases protein production from said artificial nucleic acid molecule, preferably from a nucleic acid sequence according to any of SEQ ID NO: 1 to 24 and SEQ ID NOs: 49 to 318, more preferably of the 3'-UTR of GNAS, MORN2, GSTM1, NDUFA1, CBR2, YBX1, NDUFB8, or CNTN1; or a further 3'-UTR, preferably an albumin7 3'-UTR;

a poly(A) sequence, preferably comprising 64 adenylates;

a poly(C) sequence, preferably comprising 30 cytidylates;

a histone stem-loop sequence.

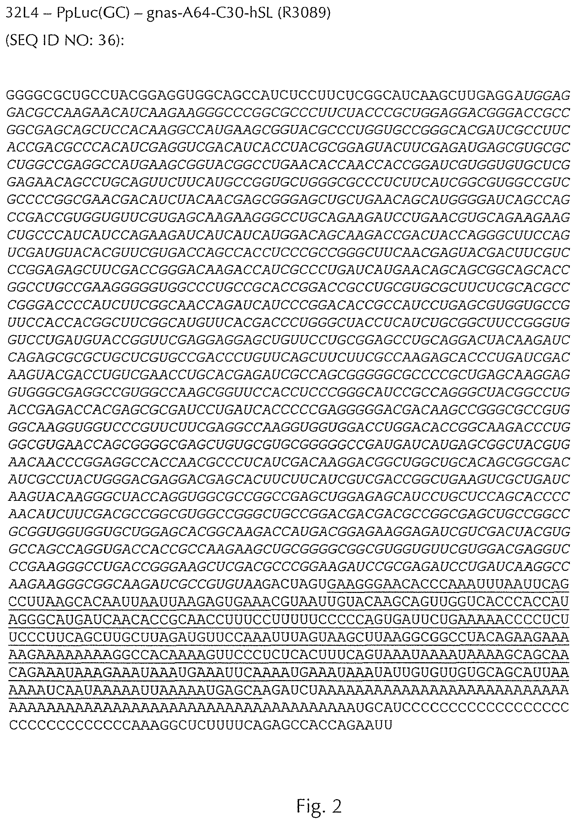

In another preferred embodiment, the artificial nucleic acid molecule comprises or consists of a nucleotide sequence selected from the group consisting of nucleic acid sequences according to SEQ ID NOs: 36 to 40, SEQ ID NOs: 42 and 43, SEQ ID NOs: 45 to 48, and SEQ ID NOs: 384 to 388 (see Fig. 2 to 6, Fig. 8, 9, 11, Fig. 19 to 21 and Fig. 26 to 30) or the complementary DNA sequence.

In a particularly preferred embodiment, the artificial nucleic acid molecule according to the invention may further comprise one or more of the modifications described in the following:

<u>Chemical modifications:</u>

The term "modification" as used herein with regard to the artificial nucleic acid molecule may refer to chemical modifications comprising backbone modifications as well as sugar modifications or base modifications.

In this context, the artificial nucleic acid molecule, preferably an RNA molecule, as defined herein may contain

nucleotide analogues/modifications, e.g. backbone modifications, sugar modifications or base modifications. A backbone modification in connection with the present invention is a modification, in which phosphates of the backbone of the nucleotides contained in a nucleic acid molecule as defined herein are chemically modified. A sugar modification in connection with the present invention is a chemical modification of the sugar of the nucleotides of the nucleic acid molecule as defined herein. Furthermore, a base modification in connection with the present invention is a chemical modification of the base moiety of the nucleotides of the nucleic acid molecule of the nucleic acid molecule. In this context, nucleotide analogues or modifications are preferably selected from nucleotide analogues which are applicable for transcription and/or translation.

Sugar Modifications:

The modified nucleosides and nucleotides, which may be incorporated into the artificial nucleic acid molecule, preferably an RNA, as described herein, can be modified in the sugar moiety. For example, the 2' hydroxyl group (OH) of an RNA molecule can be modified or replaced with a number of different "oxy" or "deoxy" substituents. Examples of "oxy" -2' hydroxyl group modifications include, but are not limited to, alkoxy or aryloxy (-OR, e.g., R = H, alkyl, cycloalkyl, aryl, aralkyl, heteroaryl or sugar); polyethyleneglycols (PEG), -0(CH2CH2o)nCH2CH2OR; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH2; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified nucleic acid molecule can include nucleotides containing, for instance, arabinose as the sugar.

Backbone Modifications:

The phosphate backbone may further be modified in the modified nucleosides and nucleotides, which may be incorporated into the artificial nucleic acid molecule, preferably an RNA, as described herein. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

Base Modifications:

The modified nucleosides and nucleotides, which may be incorporated into the artificial nucleic acid molecule, preferably an RNA molecule, as described herein, can further be modified in the nucleobase moiety. Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogues/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-Iodo-2'-deoxycytidine-5'-triphosphate, 5-iodouridine-5'-triphosphate, 5-Iodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

In some embodiments, modified nucleosides include pyridin-4-one ribonucleoside, 5-azauridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, l-taurinomethyl-4-thio-uridine, 5-methyluridine, 1-methyl-pseudouridine, 4-thio-1-methyl-pseudouridine, 2-thio-l-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio- 1-methyl-pseudoisocytidine, 4-thio- 1 -methyl- 1 -deaza-pseudoisocytidine, 1 -methyl-1 -deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine.

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, l-methyl-6-thio-guanosine, N2-methyl-6-thio-guanosine, and N2,N2-dimethyl-6-thio-guanosine.

In some embodiments, the nucleotide can be modified on the major groove face and can include replacing hydrogen on C-5 of uracil with a methyl group or a halo group.

In specific embodiments, a modified nucleoside is 5'-0-(l-Thiophosphate)-Adenosine, 5'-0-(1-Thiophosphate)-Cytidine, 5'-0-(1-Thiophosphate)-Guanosine, 5'-0-(1-Thiophosphate)-Uridine or 5'-0-(l-Thiophosphate)-Pseudouridine.

In further specific embodiments the artificial nucleic acid molecule, preferably an RNA molecule, may comprise nucleoside modifications selected from 6-aza-cytidine, 2-thio-cytidine, alpha-thio-cytidine, Pseudo-iso-cytidine, 5-aminoallyl-uridine, 5-iodo-uridine, N1-methyl-pseudouridine, 5,6-dihydrouridine, alpha-thio-uridine, 4-thio-uridine, 6-aza-uridine, 5-hydroxy-uridine, deoxy-thymidine, 5-methyl-uridine, Pyrrolo-cytidine, inosine, alpha-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-amino-purine, Pseudo-iso-cytidine, 6-Chloro-purine, N6-methyl-adenosine, alpha-thio-adenosine, 8-azido-adenosine, 7-deaza-adenosine.

Lipid modification:

According to a further embodiment, the artificial nucleic acid molecule, preferably an RNA, as defined herein can contain a lipid modification. Such a lipid-modified RNA typically comprises an RNA as defined herein. Such a lipid-modified RNA molecule as defined herein typically further comprises at least one linker covalently linked with that RNA molecule, and at least one lipid covalently linked with the respective linker. Alternatively, the lipid-modified RNA molecule comprises at least one RNAmolecule as defined herein and at least one (bifunctional) lipid covalently linked (without a linker) with that RNA molecule. According to a third alternative, the lipid-modified RNA molecule comprises an artificial nucleic acid molecule, preferably an RNA molecule, as defined herein, at least one linker covalently linked with that RNA molecule, and at least one lipid covalently linked with the respective linker, and also at least one (bifunctional) lipid covalently linked (without a linker) with that RNA molecule. In this context, it is particularly preferred that the lipid modification is present at the terminal ends of a linear RNA sequence.

Modification of the 5'-end of the modified RNA:

According to another preferred embodiment of the invention, the artificial nucleic acid molecule, preferably an RNA molecule, as defined herein, can be modified by the addition of a so-called "5' CAP" structure.
A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. m7GpppN is the 5'-CAP structure which naturally occurs in mRNA transcribed by polymerase II and is therefore not considered as modification comprised in the modified RNA according to the invention. This means the artificial nucleic acid molecule, preferably an RNA molecule, according to the present invention may comprise a m7GpppN as 5'-CAP, but additionally the artificial nucleic acid molecule, preferably an RNA molecule, comprises at least one further modification as defined herein.

Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-CAP structures are regarded as at least one modification comprised in the artificial nucleic acid molecule, preferably in an RNA molecule, according to the present invention.

Particularly preferred modified 5'-CAP structures are CAP1 (methylation of the ribose of the adjacent nucleotide of m7G), CAP2 (methylation of the ribose of the 2$^{nd}$ nucleotide downstream of the m7G), CAP3 (methylation of the ribose of the 3$^{rd}$ nucleotide downstream of the m7G), CAP4 (methylation of the ribose of the 4$^{th}$ nucleotide downstream of the m7G), ARCA (anti-reverse CAP analogue, modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In a preferred embodiment, the at least one open reading frame encodes a therapeutic protein or peptide. In another embodiment, an antigen is encoded by the at least one open reading frame, such as a pathogenic antigen, a tumour antigen, an allergenic antigen or an autoimmune antigen. Therein, the administration of the artificial nucleic acid molecule encoding the antigen is used in a genetic vaccination approach against a disease involving said antigen.

In an alternative embodiment, an antibody or an antigen-specific T cell receptor or a fragment thereof is encoded by the at least one open reading frame of the artificial nucleic acid molecule according to the invention.

Antigens:

Pathogenic antigens:

The artificial nucleic acid molecule according to the present invention may encode a protein or a peptide, which comprises a pathogenic antigen or a fragment, variant or derivative thereof. Such pathogenic antigens are derived from pathogenic organisms, in particular bacterial, viral or protozoological (multicellular) pathogenic organisms, which evoke an immunological reaction in a subject, in particular a mammalian subject, more particularly a human. More specifically, pathogenic antigens are preferably surface antigens, e.g. proteins (or fragments of proteins, e.g. the exterior portion of a surface antigen) located at the surface of the virus or the bacterial or protozoological organism.

Pathogenic antigens are peptide or protein antigens preferably derived from a pathogen associated with infectious disease which are preferably selected from antigens derived from the pathogens Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia and other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheriae, Coxiella burnetii, Crimean-Congo hemorrhagic fever virus, Cryptococcus neoformans, Cryptosporidium genus, Cytomegalovirus (CMV), Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and Enterovirus 71 (EV71), Epidermophyton spp, Epstein-Barr Virus (EBV), Escherichia coli O157:H7, O111 and O104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenzae, Helicobacter pylori, Henipavirus (Hendra virus Nipah virus), Hepatitis A Virus, Hepatitis B Virus (HBV), Hepatitis C Virus (HCV), Hepatitis D Virus, Hepatitis E Virus,

Herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV-7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family (Influenza), Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, rhinoviruses, Rickettsia akari, Rickettsia genus, Rickettsia prowazekii, Rickettsia rickettsii, Rickettsia typhi, Rift Valley fever virus, Rotavirus, Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yellow fever virus, Yersinia enterocolitica, Yersinia pestis, and Yersinia pseudotuberculosis.

In this context particularly preferred are antigens from the pathogens selected from Influenza virus, respiratory syncytial virus (RSV), Herpes simplex virus (HSV), human Papilloma virus (HPV), Human immunodeficiency virus (HIV), Plasmodium, Staphylococcus aureus, Dengue virus, Chlamydia trachomatis, Cytomegalovirus (CMV), Hepatitis B virus (HBV), Mycobacterium tuberculosis, Rabies virus, and Yellow Fever Virus.

Tumour antigens:

In a further embodiment the artificial nucleic acid molecule according to the present invention may encode a protein or a peptide, which comprises a peptide or protein comprising a tumour antigen, a fragment, variant or derivative of said tumour antigen, preferably, wherein the tumour antigen is a melanocyte-specific antigen, a cancer-testis antigen or a tumour-specific antigen, preferably a CT-X antigen, a non-X CT-antigen, a binding partner for a CT-X antigen or a binding partner for a non-X CT-antigen or a tumour-specific antigen, more preferably a CT-X antigen, a binding partner for a non-X CT-antigen or a tumour-specific antigen or a fragment, variant or derivative of said tumour antigen; and wherein each of the nucleic acid sequences encodes a different peptide or protein; and wherein at least one of the nucleic acid sequences encodes for 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-BIO, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PAP, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP-1, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGFR1, VEGFR-2/FLK-1, WT1 and a immunoglobulin

idiotype of a lymphoid blood cell or a T cell receptor idiotype of a lymphoid blood cell, or a fragment, variant or derivative of said tumour antigen; preferably survivin or a homologue thereof, an antigen from the MAGE-family or a binding partner thereof or a fragment, variant or derivative of said tumour antigen. Particularly preferred in this context are the tumour antigens NY-ESO-1, 5T4, MAGE-C1, MAGE-C2, Survivin, Muc-1, PSA, PSMA, PSCA, STEAP and PAP.

In a preferred embodiment, the artificial nucleic acid molecule encodes a protein or a peptide, which comprises a therapeutic protein or a fragment, variant or derivative thereof.

Therapeutic proteins as defined herein are peptides or proteins, which are beneficial for the treatment of any inherited or acquired disease or which improves the condition of an individual. Particularly, therapeutic proteins play an important role in the creation of therapeutic agents that could modify and repair genetic errors, destroy cancer cells or pathogen infected cells, treat immune system disorders, treat metabolic or endocrine disorders, among other functions. For instance, Erythropoietin (EPO), a protein hormone can be utilized in treating patients with erythrocyte deficiency, which is a common cause of kidney complications. Furthermore adjuvant proteins, therapeutic antibodies are encompassed by therapeutic proteins and also hormone replacement therapy which is e.g. used in the therapy of women in menopause. In more recent approaches, somatic cells of a patient are used to reprogram them into pluripotent stem cells, which replace the disputed stem cell therapy. Also these proteins used for reprogramming of somatic cells or used for differentiating of stem cells are defined herein as therapeutic proteins. Furthermore, therapeutic proteins may be used for other purposes, e.g. wound healing, tissue regeneration, angiogenesis, etc. Furthermore, antigen-specific B cell receptors and fragments and variants thereof are defined herein as therapeutic proteins.

Therefore therapeutic proteins can be used for various purposes including treatment of various diseases like e.g. infectious diseases, neoplasms (e.g. cancer or tumour diseases), diseases of the blood and blood-forming organs, endocrine, nutritional and metabolic diseases, diseases of the nervous system, diseases of the circulatory system, diseases of the respiratory system, diseases of the digestive system, diseases of the skin and subcutaneous tissue, diseases of the musculoskeletal system and connective tissue, and diseases of the genitourinary system, independently if they are inherited or acquired.

In this context, particularly preferred therapeutic proteins which can be used inter alia in the treatment of metabolic or endocrine disorders are selected from (in brackets the particular disease for which the therapeutic protein is used in the treatment): Acid sphingomyelinase (Niemann-Pick disease), Adipotide (obesity), Agalsidase-beta (human galactosidase A) (Fabry disease; prevents accumulation of lipids that could lead to renal and cardiovascular complications), Alglucosidase (Pompe disease (glycogen storage disease type II)), alpha-galactosidase A (alpha-GAL A, Agalsidase alpha) (Fabry disease), alpha-glucosidase (Glycogen storage disease (GSD), Morbus Pompe), alpha-L-iduronidase (mucopolysaccharidoses (MPS), Hurler syndrome, Scheie syndrome), alpha-N-acetylglucosaminidase (Sanfilippo syndrome), Amphiregulin (cancer, metabolic disorder), Angiopoietin ((Ang1, Ang2, Ang3, Ang4, ANGPTL2, ANGPTL3, ANGPTL4, ANGPTL5, ANGPTL6, ANGPTL7) (angiogenesis, stabilize vessels), Betacellulin (metabolic disorder), Beta-glucuronidase (Sly syndrome), Bone morphogenetic protein BMPs (BMP1, BMP2, BMP3, BMP4, BMP5, BMP6, BMP7, BMP8a, BMP8b, BMP10, BMP15) (regenerative effect, bone-related conditions, chronic kidney disease (CKD)), CLN6 protein (CLN6 disease - Atypical Late Infantile, Late Onset variant, Early Juvenile, Neuronal Ceroid Lipofuscinoses (NCL)), Epidermal growth factor (EGF) (wound healing, regulation of cell growth, proliferation, and differentiation), Epigen (metabolic disorder), Epiregulin (metabolic disorder), Fibroblast Growth Factor (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23) (wound healing, angiogenesis, endocrine disorders, tissue regeneration), Galsulphase (Mucopolysaccharidosis VI), Ghrelin (irritable bowel syndrome (IBS), obesity, Prader-Willi syndrome, type II diabetes mellitus), Glucocerebrosidase (Gaucher's disease), GM-CSF (regenerative effect, production of white blood cells, cancer), Heparin-binding EGF-like growth factor (HB-EGF) (wound healing, cardiac hypertrophy and heart development and function), Hepatocyte growth factor HGF (regenerative effect, wound healing), Hepcidin (iron metabolism disorders, Beta-thalassemia), Human albumin (Decreased production of albumin (hypoproteinaemia), increased loss of albumin (nephrotic syndrome), hypovolaemia, hyperbilirubinaemia), Idursulphase (Iduronate-2-sulphatase) (Mucopolysaccharidosis II (Hunter syndrome)), Integrins $\alpha V\beta 3$, $\alpha V\beta 5$ and $\alpha 5\beta 1$ (Bind matrix macromolecules and proteinases, angiogenesis), Iuduronate sulfatase (Hunter syndrome), Laronidase (Hurler and Hurler-Scheie forms of mucopolysaccharidosis I), N-acetylgalactosamine-4-sulfatase (rhASB; galsulfase, Arylsulfatase A (ARSA), Arylsulfatase B (ARSB)) (arylsulfatase B deficiency, Maroteaux-Lamy syndrome, mucopolysaccharidosis VI), N-acetylglucosamine-6-sulfatase (Sanfilippo syndrome), Nerve growth factor (NGF, Brain-Derived Neurotrophic Factor (BDNF), Neurotrophin-3 (NT-3), and Neurotrophin 4/5 (NT-4/5) (regenerative effect, cardiovascular diseases, coronary atherosclerosis, obesity, type 2 diabetes, metabolic syndrome, acute coronary syndromes, dementia, depression, schizophrenia, autism, Rett syndrome, anorexia nervosa, bulimia nervosa, wound healing, skin ulcers, corneal ulcers, Alzheimer's disease), Neuregulin (NRG1, NRG2, NRG3, NRG4) (metabolic disorder, schizophrenia), Neuropilin (NRP-1, NRP-2) (angiogenesis, axon guidance, cell survival, migration), Obestatin (irritable bowel syndrome (IBS), obesity, Prader-Willi syndrome, type II diabetes mellitus), Platelet Derived Growth factor (PDGF (PDFF-A, PDGF-B, PDGF-C, PDGF-D) (regenerative effect, wound healing, disorder in angiogenesis, Arteriosclerosis, Fibrosis, cancer), TGF beta receptors (endoglin, TGF-beta

1 receptor, TGF-beta 2 receptor, TGF-beta 3 receptor) (renal fibrosis, kidney disease, diabetes, ultimately end-stage renal disease (ESRD), angiogenesis), Thrombopoietin (THPO) (Megakaryocyte growth and development factor (MGDF)) (platelets disorders, platelets for donation, recovery of platelet counts after myelosuppressive chemotherapy), Transforming Growth factor (TGF (TGF-alpha, TGF-beta (TGFbeta1, TGFbeta2, and TGFbeta3))) (regenerative effect, wound healing, immunity, cancer, heart disease, diabetes, Marfan syndrome, Loeys-Dietz syndrome), VEGF (VEGF-A, VEGF-B, VEGF-C, VEGF-D, VEGF-E, VEGF-F und PIGF) (regenerative effect, angiogenesis, wound healing, cancer, permeability), Nesiritide (Acute decompensated congestive heart failure), Trypsin (Decubitus ulcer, varicose ulcer, debridement of eschar, dehiscent wound, sunburn, meconium ileus), adrenocorticotrophic hormone (ACTH) ("Addison's disease, Small cell carcinoma, Adrenoleukodystrophy, Congenital adrenal hyperplasia, Cushing's syndrome, Nelson's syndrome, Infantile spasms), Atrial-natriuretic peptide (ANP) (endocrine disorders), Cholecystokinin (diverse), Gastrin (hypogastrinemia), Leptin (Diabetes, hypertriglyceridemia, obesity), Oxytocin (stimulate breastfeeding, non-progression of parturition), Somatostatin (symptomatic treatment of carcinoid syndrome, acute variceal bleeding, and acromegaly, polycystic diseases of the liver and kidney, acromegaly and symptoms caused by neuroendocrine tumors), Vasopressin (antidiuretic hormone) (diabetes insipidus), Calcitonin (Postmenopausal osteoporosis, Hypercalcaemia, Paget's disease, Bone metastases, Phantom limb pain, Spinal Stenosis), Exenatide (Type 2 diabetes resistant to treatment with metformin and a sulphonylurea), Growth hormone (GH), somatotropin (Growth failure due to GH deficiency or chronic renal insufficiency, Prader-Willi syndrome, Turner syndrome, AIDS wasting or cachexia with antiviral therapy), Insulin (Diabetes mellitus, diabetic ketoacidosis, hyperkalaemia), Insulin-like growth factor 1 IGF-1 (Growth failure in children with GH gene deletion or severe primary IGF1 deficiency, neurodegenerative disease, cardiovascular diseases, heart failure), Mecasermin rinfabate, IGF-1 analog (Growth failure in children with GH gene deletion or severe primary IGF1 deficiency, neurodegenerative disease, cardiovascular diseases, heart failure), Mecasermin, IGF-1 analog (Growth failure in children with GH gene deletion or severe primary IGF1 deficiency, neurodegenerative disease, cardiovascular diseases, heart failure), Pegvisomant (Acromegaly), Pramlintide (Diabetes mellitus, in combination with insulin), Teriparatide (human parathyroid hormone residues 1-34) (Severe osteoporosis), Becaplermin (Debridement adjunct for diabetic ulcers), Dibotermin-alpha (Bone morphogenetic protein 2) (Spinal fusion surgery, bone injury repair), Histrelin acetate (gonadotropin releasing hormone; GnRH) (Precocious puberty), Octreotide (Acromegaly, symptomatic relief of VIP-secreting adenoma and metastatic carcinoid tumours), and Palifermin (keratinocyte growth factor; KGF) (Severe oral mucositis in patients undergoing chemotherapy, wound healing).

These and other proteins are understood to be therapeutic, as they are meant to treat the subject by replacing its defective endogenous production of a functional protein in sufficient amounts. Accordingly, such therapeutic proteins are typically mammalian, in particular human proteins.

For the treatment of blood disorders, diseases of the circulatory system, diseases of the respiratory system, cancer or tumour diseases, infectious diseases or immunedeficiencies following therapeutic proteins may be used: Alteplase (tissue plasminogen activator; tPA) (Pulmonary embolism, myocardial infarction, acute ischaemic stroke, occlusion of central venous access devices), Anistreplase (Thrombolysis), Antithrombin III (AT-III) (Hereditary AT-III deficiency, Thromboembolism), Bivalirudin (Reduce blood-clotting risk in coronary angioplasty and heparin-induced thrombocytopaenia), Darbepoetin-alpha (Treatment of anaemia in patients with chronic renal insufficiency and chronic renal failure (+/- dialysis)), Drotrecogin-alpha (activated protein C) (Severe sepsis with a high risk of death), Erythropoietin, Epoetin-alpha, erythropoetin, erthropoyetin (Anaemia of chronic disease, myleodysplasia, anaemia due to renal failure or chemotherapy, preoperative preparation), Factor IX (Haemophilia B), Factor VIIa (Haemorrhage in patients with haemophilia A or B and inhibitors to factor VIII or factor IX), Factor VIII (Haemophilia A), Lepirudin (Heparin-induced thrombocytopaenia), Protein C concentrate (Venous thrombosis, Purpura fulminans), Reteplase (deletion mutein of tPA) (Management of acute myocardial infarction, improvement of ventricular function), Streptokinase (Acute evolving transmural myocardial infarction, pulmonary embolism, deep vein thrombosis, arterial thrombosis or embolism, occlusion of arteriovenous cannula), Tenecteplase (Acute myocardial infarction), Urokinase (Pulmonary embolism), Angiostatin (Cancer), Anti-CD22 immunotoxin (Relapsed CD33+ acute myeloid leukaemia), Denileukin diftitox (Cutaneous T-cell lymphoma (CTCL)), Immunocyanin (bladder and prostate cancer), MPS (Metallopanstimulin) (Cancer), Aflibercept (Non-small cell lung cancer (NSCLC), metastatic colorectal cancer (mCRC), hormone-refractory metastatic prostate cancer, wet macular degeneration), Endostatin (Cancer, inflammatory diseases like rheumatoid arthritis as well as Crohn's disease, diabetic retinopathy, psoriasis, and endometriosis), Collagenase (Debridement of chronic dermal ulcers and severely burned areas, Dupuytren's contracture, Peyronie's disease), Human deoxy-ribonuclease I, dornase (Cystic fibrosis; decreases respiratory tract infections in selected patients with FVC greater than 40% of predicted), Hyaluronidase (Used as an adjuvant to increase the absorption and dispersion of injected drugs, particularly anaesthetics in ophthalmic surgery and certain imaging agents), Papain (Debridement of necrotic tissue or liquefication of slough in acute and chronic lesions, such as pressure ulcers, varicose and diabetic ulcers, burns, postoperative wounds, pilonidal cyst wounds, carbuncles, and other wounds), L-Asparaginase (Acute lymphocytic leukaemia, which requires exogenous asparagine for proliferation), Peg-asparaginase (Acute lymphocytic leukaemia, which requires exogenous asparagine for proliferation), Rasburicase (Paediatric patients with leukaemia, lymphoma, and solid tumours who are undergoing

anticancer therapy that may cause tumour lysis syndrome), Human chorionic gonadotropin (HCG) (Assisted reproduction), Human follicle-stimulating hormone (FSH) (Assisted reproduction), Lutropin-alpha (Infertility with luteinizing hormone deficiency), Prolactin (Hypoprolactinemia, serum prolactin deficiency, ovarian dysfunction in women, anxiety, arteriogenic erectile dysfunction, premature ejaculation, oligozoospermia, asthenospermia, hypofunction of seminal vesicles, hypoandrogenism in men), alpha-1-Proteinase inhibitor (Congenital antitrypsin deficiency), Lactase (Gas, bloating, cramps and diarrhoea due to inability to digest lactose), Pancreatic enzymes (lipase, amylase, protease) (Cystic fibrosis, chronic pancreatitis, pancreatic insufficiency, post-Billroth II gastric bypass surgery, pancreatic duct obstruction, steatorrhoea, poor digestion, gas, bloating), Adenosine deaminase (pegademase bovine, PEG-ADA) (Severe combined immunodeficiency disease due to adenosine deaminase deficiency), Abatacept (Rheumatoid arthritis (especially when refractory to TNFalpha inhibition)), Alefacept (Plaque Psoriasis), Anakinra (Rheumatoid arthritis), Etanercept (Rheumatoid arthritis, polyarticular-course juvenile rheumatoid arthritis, psoriatic arthritis, ankylosing spondylitis, plaque psoriasis, ankylosing spondylitis), Interleukin-1 (IL-1) receptor antagonist, Anakinra (inflammation and cartilage degradation associated with rheumatoid arthritis), Thymulin (neurodegenerative diseases, rheumatism, anorexia nervosa), TNF-alpha antagonist (autoimmune disorders such as rheumatoid arthritis, ankylosing spondylitis, Crohn's disease, psoriasis, hidradenitis suppurativa, refractory asthma), Enfuvirtide (HIV-1 infection), and Thymosin $\alpha 1$ (Hepatitis B and C).
(in brackets is the particular disease for which the therapeutic protein is used in the treatment)

In a further aspect, the present invention provides a vector comprising

a. an open reading frame (ORF) and/or a cloning site, e.g. for insertion of an open reading frame or a sequence comprising an open reading frame; and
b. at least one 3'-untranslated region element (3'-UTR element) and/or at least one 5'-untranslated region element (5'-UTR element), wherein the at least one 3'-UTR element and/or the at least one 5'-UTR element prolongs and/or increases protein production from said artificial nucleic acid molecule and wherein the at least one 3'-UTR element and/or the at least one 5'-UTR element is derived from a stable mRNA.

In general, the vector according to the present invention may comprise an artificial nucleic acid molecule according to the present invention as described above. In particular, the preferred embodiments described above for an artificial nucleic acid molecule according to the present invention also apply for an artificial nucleic acid molecule according to the present invention, which is comprised by a vector according to the present invention. For example, in the inventive vector the at least one 3'-UTR element and/or the at least one 5'-UTR element and the ORF are as described above for the artificial nucleic acid molecule according to the present invention, including the preferred embodiments. For example, in the vector according to the present invention, the stable mRNA from which the at least one 3'-UTR element and/or the at least one 5'-UTR element is derived may be preferably characterized by an mRNA decay wherein the ratio of the amount of said mRNA at a second point in time to the amount of said mRNA at a first point in time is at least 0.5 (50%), at least 0.6 (60%), at least 0.7 (70%), at least 0.75 (75%), at least 0.8 (80%), at least 0.85 (85%), at least 0.9 (90%), or at least 0.95 (95%).

The cloning site may be any sequence that is suitable for introducing an open reading frame or a sequence comprising an open reading frame, such as one or more restriction sites. Thus, the vector comprising a cloning site is preferably suitable for inserting an open reading frame into the vector, preferably for inserting an open reading frame 3' to the 5'-UTR element and/or 5' to the 3'-UTR element. Preferably the cloning site or the ORF is located 3' to the 5'-UTR element and/or 5' to the 3'-UTR element, preferably in close proximity to the 3'-end of the 5'-UTR element and/or to the 5'-end of the 3'-UTR element. For example, the cloning site or the ORF may be directly connected to the 3'-end of the 5'-UTR element and/or to the 5'-end of the 3'-UTR element or they may be connected via a stretch of nucleotides, such as by a stretch of 2, 4, 6, 8, 10, 20 etc. nucleotides as described above for the artificial nucleic acid molecule according to the present invention.

Preferably, the vector according to the present invention is suitable for producing the artificial nucleic acid molecule according to the present invention, preferably for producing an artificial mRNA according to the present invention, for example, by optionally inserting an open reading frame or a sequence comprising an open reading frame into the vector and transcribing the vector. Thus, preferably, the vector comprises elements needed for transcription, such as a promoter, e.g. an RNA polymerase promoter. Preferably, the vector is suitable for transcription using eukaryotic, prokaryotic, viral or phage transcription systems, such as eukaryotic cells, prokaryotic cells, or eukaryotic, prokaryotic, viral or phage *in vitro* transcription systems. Thus, for example, the vector may comprise a promoter sequence, which is recognized by a polymerase, such as by an RNA polymerase, e.g. by a eukaryotic, prokaryotic, viral, or phage RNA polymerase. In a preferred embodiment, the vector comprises a phage RNA polymerase promoter such as an SP6, T3 or T7, preferably a T7 promoter. Preferably, the vector is suitable for *in vitro* transcription using a phage based *in vitro* transcription system, such as a T7 RNA polymerase based *in vitro* transcription system. In another preferred embodiment, the vector may be used directly for expression of the encoded peptide or protein in cells or tissue. For this purpose, the vector comprises particular elements, which are necessary for expression in those cells/tissue e.g. particular promoter

sequences, such as a CMV promoter.

The vector may further comprise a poly(A) sequence and/or a polyadenylation signal as described above for the artificial nucleic acid molecule according to the present invention.

The vector may be an RNA vector or a DNA vector. Preferably, the vector is a DNA vector. The vector may be any vector known to the skilled person, such as a viral vector or a plasmid vector. Preferably, the vector is a plasmid vector, preferably a DNA plasmid vector.

In a preferred embodiment, the vector according to the present invention comprises the artificial nucleic acid molecule according to the present invention.

Preferably, a DNA vector according to the invention comprises a nucleic acid sequence which has an identity of at least about 1, 2, 3, 4, 5, 10, 15, 20, 30 or 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the nucleic acid sequence of a 3'-UTR of a transcript of a gene, such as to the nucleic acid sequences according to SEQ ID NOs: 1 to 24 and SEQ ID NOs: 49 to 318.

Preferably, a DNA vector according to the invention comprises a nucleic acid sequence which has an identity of at least about 1, 2, 3, 4, 5, 10, 15, 20, 30 or 40%, preferably of at least about 50%, preferably of at least about 60%, preferably of at least about 70%, more preferably of at least about 80%, more preferably of at least about 90%, even more preferably of at least about 95%, even more preferably of at least about 99%, most preferably of 100% to the nucleic acid sequence of a 5'-UTR of a transcript of a gene, such as to the nucleic acid sequences according to SEQ ID NOs: 25 to 30 and SEQ ID NOs: 319 to 382.

Preferably, a DNA vector according to the present invention comprises a sequence selected from the group consisting of DNA sequences according to SEQ ID NOs. 1 to 30 or a sequence having an identity of at least about 40%, preferably of at least about 50%, preferably of at least about 60%, preferably of at least about 70%, more preferably of at least about 80%, more preferably of at least about 90%, even more preferably of at least about 95%; even more preferably of at least about 99% sequence identity to the DNA sequences according to SEQ ID NOs. 1 to 30 or a fragment thereof as described above, preferably a functional fragment thereof.

Preferably, an RNA vector according to the present invention comprises a sequence selected from the group consisting of the sequences according to RNA sequences corresponding to DNA sequences according to SEQ ID NOs: 1 to 30 or a sequence having an identity of at least about 40%, preferably of at least about 50%, preferably of at least about 60%, preferably of at least about 70%, more preferably of at least about 80%, more preferably of at least about 90%, even more preferably of at least about 95%; even more preferably of at least about 99% sequence identity to the RNA sequences corresponding to the DNA sequences according to SEQ ID NOs: 1 to 30 or to a fragment thereof, preferably a functional fragment thereof.

Preferably, the vector is a circular molecule. Preferably, the vector is a double-stranded molecule, such as a double-stranded DNA molecule. Such circular, preferably double stranded DNA molecule may be used conveniently as a storage form for the inventive artificial nucleic acid molecule. Furthermore, it may be used for transfection of cells, for example, cultured cells. Also it may be used for *in vitro* transcription for obtaining an artificial RNA molecule according to the invention.

Preferably, the vector, preferably the circular vector, is linearizable, for example, by restriction enzyme digestion. In a preferred embodiment, the vector comprises a cleavage site, such as a restriction site, preferably a unique cleavage site, located immediately 3' to the ORF, or - if present - located immediately 3' to the 3'-UTR element, or - if present - located 3' to the poly(A) sequence or polyadenylation signal, or - if present - located 3' to the poly(C) sequence, or - if present - located 3' to the histone stem-loop. Thus, preferably, the product obtained by linearizing the vector terminates at the 3'end with the 3'-end of the ORF, or - if present - with the 3'-end of the 3'-UTR element, or - if present - with the 3'-end of the poly(A) sequence or polyadenylation signal, or - if present - with the 3'-end of the poly(C) sequence. In the embodiment, wherein the vector according to the present invention comprises the artificial nucleic acid molecule according to the present invention, a restriction site, preferably a unique restriction site, is preferably located immediately 3' to the 3'-end of the artificial nucleic acid molecule.

In a further aspect, the present invention relates to a cell comprising the artificial nucleic acid molecule according to the present invention or the vector according to present invention. The cell may be any cell, such as a bacterial cell, insect cell, plant cell, vertebrate cell, e.g. a mammalian cell. Such cell may be, e.g., used for replication of the vector of the present invention, for example, in a bacterial cell. Furthermore, the cell may be used for transcribing the artificial nucleic acid molecule or the vector according to the present invention and/or translating the open reading frame of the artificial nucleic acid molecule or the vector according to the present invention. For example, the cell may be used for recombinant protein production.

The cells according to the present invention are, for example, obtainable by standard nucleic acid transfer methods, such as standard transfection, transduction or transformation methods. For example, the artificial nucleic acid molecule or the vector according to the present invention may be transferred into the cell by electroporation, lipofection,

e.g. based on cationic lipids and/or liposomes, calcium phosphate precipitation, nanoparticle based transfection, virus based transfection, or based on cationic polymers, such as DEAE-dextran or polyethylenimine etc.

Preferably, the cell is a mammalian cell, such as a cell of human subject, a domestic animal, a laboratory animal, such as a mouse or rat cell. Preferably the cell is a human cell. The cell may be a cell of an established cell line, such as a CHO, BHK, 293T, COS-7, HELA, HEK, etc. or the cell may be a primary cell, such as a human dermal fibroblast (HDF) cell etc., preferably a cell isolated from an organism. In a preferred embodiment, the cell is an isolated cell of a mammalian subject, preferably of a human subject. For example, the cell may be an immune cell, such as a dendritic cell, a cancer or tumor cell, or any somatic cell etc., preferably of a mammalian subject, preferably of a human subject.

In a further aspect, the present invention provides a pharmaceutical composition comprising the artificial nucleic acid molecule according to the present invention, the vector according the present invention, or the cell according to the present invention. The pharmaceutical composition according to the invention may be used, e.g., as a vaccine, for example, for genetic vaccination. Thus, the ORF may, e.g., encode an antigen to be administered to a patient for vaccination. Thus, in a preferred embodiment, the pharmaceutical composition according to the present invention is a vaccine. Furthermore, the pharmaceutical composition according to the present invention may be used, e.g., for gene therapy.

Preferably, the pharmaceutical composition further comprises one or more pharmaceutically acceptable vehicles, diluents and/or excipients and/or one or more adjuvants. In the context of the present invention, a pharmaceutically acceptable vehicle typically includes a liquid or non-liquid basis for the inventive pharmaceutical composition. In one embodiment, the pharmaceutical composition is provided in liquid form. In this context, preferably, the vehicle is based on water, such as pyrogen-free water, isotonic saline or buffered (aqueous) solutions, e.g phosphate, citrate etc. buffered solutions. The buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of mammalian cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

One or more compatible solid or liquid fillers or diluents or encapsulating compounds suitable for administration to a patient may be used as well for the inventive pharmaceutical composition. The term "compatible" as used herein preferably means that these components of the inventive pharmaceutical composition are capable of being mixed with the inventive artificial nucleic acid, vector or cells as defined herein in such a manner that no interaction occurs which would substantially reduce the pharmaceutical effectiveness of the inventive pharmaceutical composition under typical use conditions.

The pharmaceutical composition according to the present invention may optionally further comprise one or more additional pharmaceutically active components. A pharmaceutically active component in this context is a compound that exhibits a therapeutic effect to heal, ameliorate or prevent a particular indication or disease. Such compounds include, without implying any limitation, peptides or proteins, nucleic acids, (therapeutically active) low molecular weight organic or inorganic compounds (molecular weight less than 5000, preferably less than 1000), sugars, antigens or antibodies, therapeutic agents already known in the prior art, antigenic cells, antigenic cellular fragments, cellular fractions, cell wall components (e.g. polysaccharides), modified, attenuated or de-activated (e.g. chemically or by irradiation) pathogens (virus, bacteria etc.).

Furthermore, the inventive pharmaceutical composition may comprise a carrier for the artificial nucleic acid molecule or the vector. Such a carrier may be suitable for mediating dissolution in physiological acceptable liquids, transport and cellular uptake of the pharmaceutical active artificial nucleic acid molecule or the vector. Accordingly, such a carrier may be a component which may be suitable for depot and delivery of an artificial nucleic acid molecule or vector according to the invention. Such components may be, for example, cationic or polycationic carriers or compounds which may serve as transfection or complexation agent.

Particularly preferred transfection or complexation agents in this context are cationic or polycationic compounds, including protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, proline-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*), pAntp, pIsl, FGF, Lactoferrin, Transportan, Buforin-2, Bac715-24, SynB, SynB(1), pVEC, hCT-derived peptides, SAP, or histones.

Furthermore, such cationic or polycationic compounds or carriers may be cationic or polycationic peptides or proteins, which preferably comprise or are additionally modified to comprise at least one -SH moiety. Preferably, a cationic or polycationic carrier is selected from cationic peptides having the following sum formula (I):

$\{(Arg)_l;(Lys)_m;(His)_n;(Orn)_o;(Xaa)_x\}$; formula (I)

wherein l + m + n + o + x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred.

Further, the cationic or polycationic peptide or protein, when defined according to formula $\{(Arg)_l;(Lys)_m;(His)_n;(Orn)_o;(Xaa)_x\}$ (formula (I)) as shown above and which comprise or are additionally modified to comprise at least one -SH moeity, may be, without being restricted thereto, selected from subformula (Ia):

$\{(Arg)_l;(Lys)_m;(His)_n;(Orn)_o;(Xaa')_x(Cys)_y\}$ subformula (Ia)

wherein $(Arg)_l;(Lys)_m;(His)_n;(Orn)_o$; and x are as defined herein, Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide. Further, the cationic or polycationic peptide may be selected from subformula (Ib):

$Cys_1\{(Arg)_l;(Lys)_m;(His)_n;(Orn)_o;(Xaa)_x\}Cys_2$ subformula (Ib)

wherein empirical formula $\{(Arg)_l;(Lys)_m;(His)_n;(Orn)_o;(Xaa)_x\}$ (formula (III)) is as defined herein and forms a core of an amino acid sequence according to (semiempirical) formula (III) and wherein $Cys_1$ and $Cys_2$ are Cysteines proximal to, or terminal to $(Arg)_l;(Lys)_m;(His)_n;(Orn)_o;(Xaa)_x$.

Further preferred cationic or polycationic compounds, which can be used as transfection or complexation agent may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]-trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g polyethyleneglycole); etc.

According to another embodiment, the pharmaceutical composition according to the invention may comprise an adjuvant in order to enhance the immunostimulatory properties of the pharmaceutical composition. In this context, an adjuvant may be understood as any compound, which is suitable to support administration and delivery of the components such as the artificial nucleic acid molecule or vector comprised in the pharmaceutical composition according to the invention. Furthermore, such an adjuvant may, without being bound thereto, initiate or increase an immune response of the innate immune system, i.e. a non-specific immune response. With other words, when administered, the pharmaceutical composition according to the invention typically initiates an adaptive immune response directed to the antigen encoded by the artificial nucleic acid molecule. Additionally, the pharmaceutical composition according to the invention may generate an (supportive) innate immune response due to addition of an adjuvant as defined herein to the pharmaceutical composition according to the invention.

Such an adjuvant may be selected from any adjuvant known to a skilled person and suitable for the present

case, i.e. supporting the induction of an immune response in a mammal. Preferably, the adjuvant may be selected from the group consisting of, without being limited thereto, TDM, MDP, muramyl dipeptide, pluronics, alum solution, aluminium hydroxide, ADJUMERTM (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINETM (propanediamine); BAY R1005TM ((N-(2-deoxy-2-L-leucylamino-b-D-glucopyranosyl)-N-octadecyl-dodecanoyl-amide hydroacetate); CALCITRIOLTM (1-alpha,25-dihydroxy-vitamin D3); calcium phosphate gel; CAPTM (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D-glutamine (GMDP), ii) dimethyldioctadecylammonium chloride (DDA), iii) zinc-L-proline salt complex (ZnPro-8); GM-CSF); GMDP (N-acetylglucosaminyl-(b1-4)-N-acetylmuramyl-L-alanyl-D-isoglutamine); imiquimod (1-(2-methypropyl)-1H-imidazo[4,5-c]quinoline-4-amine); ImmTherTM (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-1beta; interleukin-2; interleukin-7; interleukin-12; ISCOMSTM; ISCOPREP 7.0.3. TM; liposomes; LOXORIBINETM (7-allyl-8-oxoguanosine); LT oral adjuvant (E.coli labile enterotoxin-protoxin); microspheres and microparticles of any composition; MF59TM; (squalene-water emulsion); MONTANIDE ISA 51TM (purified incomplete Freund's adjuvant); MONTANIDE ISA 720TM (metabolisable oil adjuvant); MPLTM (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDETM (Nac-Mur-L-Ala-D-Gln-OCH3); MURAPALMITINETM and D-MURAPALMITINETM (Nac-Mur-L-Thr-D-isoGln-sn-glyceroldipalmitoyl); NAGO (neuraminidase-galactose oxidase); nanospheres or nanoparticles of any composition; NISVs (non-ionic surfactant vesicles); PLEURANTM (-glucan); PLGA, PGA and PLA (homo- and co-polymers of lactic acid and glycolic acid; microspheres/nanospheres); PLURONIC L121TM; PMMA (polymethyl methacrylate); PODDSTM (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULONTM (QS-21); Quil-A (Quil-A saponin); S-28463 (4-amino-otec-dimethyl-2-ethoxymethyl-1H-imidazo[4,5 c]quinoline-1-ethanol); SAF-1TM ("Syntex adjuvant formulation"); Sendai proteoliposomes and Sendai-containing lipid matrices; Span-85 (sorbitan trioleate); Specol (emulsion of Marcol 52, Span 85 and Tween 85); squalene or Robane® (2,6,10,15,19,23-hexamethyltetracosan and 2,6,10,15,19,23-hexamethyl-2,6,10,14,18,22-tetracosahexane); stearyltyrosine (octadecyltyrosine hydrochloride); Theramid® (N-acetylglucosaminyl-N-acetylmuramyl-L-Ala-D-isoGlu-L-Ala-dipalmitoxypropylamide); Theronyl-MDP (TermurtideTM or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin,; microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Suitable adjuvants may also be selected from cationic or polycationic compounds wherein the adjuvant is preferably prepared upon complexing the artificial nucleic acid molecule or the vector of the pharmaceutical composition with the cationic or polycationic compound. Association or complexing the artificial nucleic acid molecule or the vector of the pharmaceutical composition with cationic or polycationic compounds as defined herein preferably provides adjuvant properties and confers a stabilizing effect to the artificial nucleic acid molecule or the vector of the pharmaceutical composition. Particularly such preferred, such cationic or polycationic compounds are selected from cationic or polycationic peptides or proteins, including protamine, nucleoline, spermin or spermidine, or other cationic peptides or proteins, such as poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs, PpT620, prolin-rich peptides, arginine-rich peptides, lysine-rich peptides, MPG-peptide(s), Pep-1, L-oligomers, Calcitonin peptide(s), Antennapedia-derived peptides (particularly from Drosophila antennapedia), pAntp, pIsl, FGF, Lactoferrin, Transportan, Buforin-2, Bac715-24, SynB, SynB(1), pVEC, hCT-derived peptides, SAP, protamine, spermine, spermidine, or histones. Further preferred cationic or polycationic compounds may include cationic polysaccharides, for example chitosan, polybrene, cationic polymers, e.g. polyethyleneimine (PEI), cationic lipids, e.g. DOTMA: 1-(2,3-sioleyloxy)propyl)-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]-trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as -aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, Chitosan, etc., silan backbone based polymers , such as PMOXA-PDMS copolymers, etc., Blockpolymers consisting of a combination of one or more cationic blocks (e.g. selected of a cationic polymer as mentioned above) and of one or more hydrophilic- or hydrophobic blocks (e.g polyethyleneglycole); etc.

Additionally, preferred cationic or polycationic proteins or peptides, which can be used as an adjuvant by complexing the artificial nucleic acid molecule or the vector, preferably an RNA, of the composition, may be selected from following proteins or peptides having the following total formula (I): (Arg)l;(Lys)m;(His)n;(Orn)o;(Xaa)x, wherein l + m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide. Particularly preferred oligoarginines in this context are e.g. Arg7, Arg8, Arg9, Arg7, H3R9, R9H3, H3R9H3, YSSR9SSY, (RKH)4, Y(RKH)2R, etc.

The ratio of the artificial nucleic acid or the vector to the cationic or polycationic compound may be calculated on the basis of the nitrogen/phosphate ratio (N/P-ratio) of the entire nucleic acid complex. For example, 1 μg RNA typically contains about 3 nmol phosphate residues, provided the RNA exhibits a statistical distribution of bases. Additionally, 1 μg peptide typically contains about x nmol nitrogen residues, dependent on the molecular weight and the number of basic amino acids. When exemplarily calculated for (Arg)9 (molecular weight 1424 g/mol, 9 nitrogen atoms), 1 μg (Arg)9 contains about 700 pmol (Arg)9 and thus 700 × 9=6300 pmol basic amino acids = 6.3 nmol nitrogen atoms. For a mass ratio of about 1:1 RNA/(Arg)9 an N/P ratio of about 2 can be calculated. When exemplarily calculated for protamine (molecular weight about 4250 g/mol, 21 nitrogen atoms, when protamine from salmon is used) with a mass ratio of about 2:1 with 2 μg RNA, 6 nmol phosphate are to be calulated for the RNA; 1 μg protamine contains about 235 pmol protamine molecues and thus 235 x 21 = 4935 pmol basic nitrogen atoms = 4.9 nmol nitrogen atoms. For a mass ratio of about 2:1 RNA/protamine an N/P ratio of about 0.81 can be calculated. For a mass ratio of about 8:1 RNA/protamine an N/P ratio of about 0.2 can be calculated. In the context of the present invention, an N/P-ratio is preferably in the range of about 0.1-10, preferably in a range of about 0.3-4 and most preferably in a range of about 0.5-2 or 0.7-2 regarding the ratio of nucleic acid:peptide in the complex, and most preferably in the range of about 0.7-1.5.

Patent application WO2010/037539, the disclosure of which is incorporated herein by reference, describes an immunostimulatory composition and methods for the preparation of an immunostimulatory composition. Accordingly, in a preferred embodiment of the invention, the composition is obtained in two separate steps in order to obtain both, an efficient immunostimulatory effect and efficient translation of the artificial nucleic acid molecule according to the invention. Therein, a so called "adjuvant component" is prepared by complexing - in a first step - the artificial nucleic acid molecule or vector, preferably an RNA, of the adjuvant component with a cationic or polycationic compound in a specific ratio to form a stable complex. In this context, it is important, that no free cationic or polycationic compound or only a neglibly small amount remains in the adjuvant component after complexing the nucleic acid. Accordingly, the ratio of the nucleic acid and the cationic or polycationic compound in the adjuvant component is typically selected in a range that the nucleic acid is entirely complexed and no free cationic or polycationic compound or only a neclectably small amount remains in the composition. Preferably the ratio of the adjuvant component, i.e. the ratio of the nucleic acid to the cationic or polycationic compound is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w).

According to a preferred embodiment, the artificial nucleic acid molecule or vector, preferably an RNA molecule, according to the invention is added in a second step to the complexed nucleic acid molecule, preferably an RNA, of the adjuvant component in order to form the (immunostimulatory) composition of the invention. Therein, the artificial acid molecule or vector, preferably an RNA, of the invention is added as free nucleic acid, i.e. nucleic acid, which is not complexed by other compounds. Prior to addition, the free artificial nucleic acid molecule or vector is not complexed and will preferably not undergo any detectable or significant complexation reaction upon the addition of the adjuvant

component.

Suitable adjuvants may furthermore be selected from nucleic acids having the formula (II): GlXmGn, wherein: G is guanosine, uracil or an analogue of guanosine or uracil; X is guanosine, uracil, adenosine, thymidine, cytosine or an analogue of the above-mentioned nucleotides; l is an integer from 1 to 40, wherein when l = 1 G is guanosine or an analogue thereof, when l > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof.

Other suitable adjuvants may furthermore be selected from nucleic acids having the formula (III): ClXmCn, wherein: C is cytosine, uracil or an analogue of cytosine or uracil; X is guanosine, uracil, adenosine, thymidine, cytosine or an analogue of the above-mentioned nucleotides; l is an integer from 1 to 40, wherein when l = 1 C is cytosine or an analogue thereof, when l > 1 at least 50% of the nucleotides are cytosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 C is cytosine or an analogue thereof, when n > 1 at least 50% of the nucleotides are cytosine or an analogue thereof.

The pharmaceutical composition according to the present invention preferably comprises a "safe and effective amount" of the components of the pharmaceutical composition, particularly of the inventive artificial nucleic acid molecule, the vector and/or the cells as defined herein. As used herein, a "safe and effective amount" means an amount sufficient to significantly induce a positive modification of a disease or disorder as defined herein. At the same time, however, a "safe and effective amount" preferably avoids serious side-effects and permits a sensible relationship between advantage and risk. The determination of these limits typically lies within the scope of sensible medical judgment.

In a further aspect, the present invention provides the artificial nucleic acid molecule according to the present invention, the vector according to the present invention, the cell according to the present invention, or the pharmaceutical composition according to the present invention for use as a medicament, for example, as vaccine (in genetic vaccination) or in gene therapy.

The artificial nucleic acid molecule according to the present invention, the vector according to the present invention, the cell according to the present invention, or the pharmaceutical composition according to the present invention are particularly suitable for any medical application which makes use of the therapeutic action or effect of peptides, polypeptides or proteins, or where supplementation of a particular peptide or protein is needed. Thus, the present invention provides the artificial nucleic acid molecule according to the present invention, the vector according to the present invention, the cell according to the present invention, or the pharmaceutical composition according to the present invention for use in the treatment or prevention of diseases or disorders amenable to treatment by the therapeutic action or effect of peptides, polypeptides or proteins or amenable to treatment by supplementation of a particular peptide, polypeptide or protein. For example, the artificial nucleic acid molecule according to the present invention, the vector according to the present invention, the cell according to the present invention, or the pharmaceutical composition according to the present invention may be used for the treatment or prevention of genetic diseases, autoimmune diseases, cancerous or tumour-related diseases, infectious diseases, chronic diseases or the like, e.g., by genetic vaccination or gene therapy.

In particular, such therapeutic treatments which benefit from an increased and prolonged presence of therapeutic peptides, polypeptides or proteins in a subject to be treated are especially suitable as medical application in the context of the present invention, since the inventive 3'-UTR element provides for a stable and prolonged expression of the encoded peptide or protein of the inventive artificial nucleic acid molecule or vector and/or the inventive 5'-UTR element provides for an increased expression of the encoded peptide or protein of the inventive artificial nucleic acid molecule or vector. Thus, a particularly suitable medical application for the artificial nucleic acid molecule according to the present invention, the vector according to the present invention, the cell according to the present invention, or the pharmaceutical composition according to the present invention is vaccination. Thus, the present invention provides the artificial nucleic acid molecule according to the present invention, the vector according to the present invention, the cell according to the present invention, or the pharmaceutical composition according to the present invention for vaccination of a subject, preferably a mammalian subject, more preferably a human subject. Preferred vaccination treatments are vaccination against infectious diseases, such as bacterial, protozoal or viral infections, and anti-tumour-vaccination. Such vaccination treatments may be prophylactic or therapeutic.

Depending on the disease to be treated or prevented, the ORF may be selected. For example, the open reading frame may code for a protein that has to be supplied to a patient suffering from total lack or at least partial loss of function of a protein, such as a patient suffering from a genetic disease. Additionally the open reading frame may be chosen from an ORF coding for a peptide or protein which beneficially influences a disease or the condition of a subject. Furthermore, the open reading frame may code for a peptide or protein which effects down-regulation of a pathological overproduction of a natural peptide or protein or elimination of cells expressing pathologically a protein or peptide. Such lack, loss of function or overproduction may, e.g., occur in the context of tumour and neoplasia, autoimmune diseases,

allergies, infections, chronic diseases or the like. Furthermore, the open reading frame may code for an antigen or immunogen, e.g. for an epitope of a pathogen or for a tumour antigen. Thus, in preferred embodiments, the artificial nucleic acid molecule or the vector according to the present invention comprises an ORF encoding an amino acid sequence comprising or consisting of an antigen or immunogen, e.g. an epitope of a pathogen or a tumour-associated antigen, a 3'-UTR element as described above and/or a 5'-UTR element as described above, and optional further components, such as a poly(A) sequence etc.

In the context of medical application, in particular, in the context of vaccination, it is preferred that the artificial nucleic acid molecule according to the present invention is RNA, preferably mRNA, since DNA harbours the risk of eliciting an anti-DNA immune response and tends to insert into genomic DNA. However, in some embodiments, for example, if a viral delivery vehicle, such as an adenoviral delivery vehicle is used for delivery of the artificial nucleic acid molecule or the vector according to the present invention, e.g., in the context of gene therapeutic treatments, it may be desirable that the artificial nucleic acid molecule or the vector is a DNA molecule.

The artificial nucleic acid molecule according to the present invention, the vector according to the present invention, the cell according to the present invention, or the pharmaceutical composition according to the present invention may be administered orally, parenterally, by inhalation spray, topically, rectally, nasally, buccally, vaginally, via an implanted reservoir or via jet injection. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques. In a preferred embodiment, the artificial nucleic acid molecule according to the present invention, the vector according to the present invention, the cell according to the present invention, or the pharmaceutical composition according to the present invention is administered via needle-free injection (e.g. jet injection).

Preferably, the artificial nucleic acid molecule according to the present invention, the vector according to the present invention, the cell according to the present invention, or the pharmaceutical composition according to the present invention is administered parenterally, e.g. by parenteral injection, more preferably by subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, sublingual injection or via infusion techniques. Particularly preferred is intradermal and intramuscular injection. Sterile injectable forms of the inventive pharmaceutical composition may be aqueous or oleaginous suspension. These suspensions may be formulated according to techniques known in the art using suitable dispersing or wetting agents and suspending agents. Preferably, the solutions or suspensions are administered via needle-free injection (e.g. jet injection).

The artificial nucleic acid molecule according to the present invention, the vector according to the present invention, the cell according to the present invention, or the pharmaceutical composition according to the present invention may also be administered orally in any orally acceptable dosage form including, but not limited to, capsules, tablets, aqueous suspensions or solutions.

The artificial nucleic acid molecule according to the present invention, the vector according to the present invention, the cell according to the present invention, or the pharmaceutical composition according to the present invention may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the artificial nucleic acid molecule according to the present invention, the vector according to the present invention, the cell according to the present invention, or the pharmaceutical composition according to the present invention may be formulated in a suitable ointment suspended or dissolved in one or more carriers.

In one embodiment, the use as a medicament comprises the step of transfection of mammalian cells, preferably *in vitro* or *ex vivo* transfection of mammalian cells, more preferably *in vitro* transfection of isolated cells of a subject to be treated by the medicament. If the use comprises the *in vitro* transfection of isolated cells, the use as a medicament may further comprise the readministration of the transfected cells to the patient. The use of the inventive artificial nucleic acid molecules or the vector as a medicament may further comprise the step of selection of successfully transfected isolated cells. Thus, it may be beneficial if the vector further comprises a selection marker. Also, the use as a medicament may comprise *in vitro* transfection of isolated cells and purification of an expression-product, i.e. the encoded peptide or protein from these cells. This purified peptide or protein may subsequently be administered to a subject in need thereof.

The present invention also provides a method for treating or preventing a disease or disorder as described above comprising administering the artificial nucleic acid molecule according to the present invention, the vector according to the present invention, the cell according to the present invention, or the pharmaceutical composition according to the present invention to a subject in need thereof.

Furthermore, the present invention provides a method for treating or preventing a disease or disorder comprising transfection of a cell with an artificial nucleic acid molecule according to the present invention or with the vector according to the present invention. Said transfection may be performed *in vitro, ex vivo* or *in vivo.* In a preferred embodiment, transfection of a cell is performed *in vitro* and the transfected cell is administered to a subject in need thereof, preferably

to a human patient. Preferably, the cell which is to be transfected *in vitro* is an isolated cell of the subject, preferably of the human patient. Thus, the present invention provides a method of treatment comprising the steps of isolating a cell from a subject, preferably from a human patient, transfecting the isolated cell with the artificial nucleic acid according to the present invention or the vector according to the present invention, and administering the transfected cell to the subject, preferably the human patient.

The method of treating or preventing a disorder according to the present invention is preferably a vaccination method or a gene therapy method as described above.

As described above, the inventive 3'-UTR element and/or the inventive 5'-UTR element are capable of prolonging and/or increasing the protein production from an mRNA. Thus, in a further aspect, the present invention relates to a method for increasing and/or prolonging protein production from an artificial nucleic acid molecule, preferably from an mRNA molecule or a vector, the method comprising the step of associating an open reading frame with a 3'-UTR element and/or a 5'-UTR element, wherein the 3'-UTR element and/or the 5'-UTR element prolongs and/or increases protein production from a resulting artificial nucleic acid molecule and wherein the at least one 3'-UTR element and/or the at least one 5'-UTR element is derived from a stable mRNA, to obtain an artificial nucleic acid molecule, preferably an mRNA molecule, according to the present invention as described above or a vector according to the present invention as described above.

Preferably, in the method for increasing and/or prolonging protein production from an artificial nucleic acid molecule, preferably from an mRNA molecule or a vector, according to the present invention the 3'-UTR element and/or the 5'-UTR element comprises or consists of a nucleic acid sequence which is derived from the 3'-UTR and/or the 5'-UTR of a transcript of a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), MP68 (RIKEN cDNA 2010107E04 gene), NDUFA4 (NADH dehydrogenase (ubiquinone) 1 alpha subcomplex 4), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5,NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, Atp5e, Gstm5, Uqcr11, Ifi27l2a, Anapc13, Atp5l, Tmsb10, Nenf, Ndufa7, Atp5k, 1110008P14Rik, Cox4i1, Cox6a1, Ndufs6, Sec61b, Romo1, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufs5, 1810027O10Rik, Atp5o, Shfm1, Tspo, S100a6, Taldo1, Bloc1s1, Hexa, Ndufb11, Map1lc3a, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1; preferably from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), and CNTN1 (contactin 1).

The term "associating the artificial nucleic acid molecule or the vector with a 3'-UTR element and/or a 5'-UTR element" in the context of the present invention preferably means functionally associating or functionally combining the artificial nucleic acid molecule or the vector with the 3'-UTR element and/or with the 5'-UTR element. This means that the artificial nucleic acid molecule or the vector and the 3'-UTR element and/or the 5'-UTR element, preferably the 3'-UTR element and/or the 5'-UTR element as described above, are associated or coupled such that the function of the 3'-UTR element and/or of the 5'-UTR element, e.g., the RNA and/or protein production prolonging and/or increasing function, is exerted. Typically, this means that the 3'-UTR element and/or the 5'-UTR element is integrated into the artificial nucleic acid molecule or the vector, preferably the mRNA molecule, 3' and/or 5', respectively, to an open reading frame, preferably immediately 3' to an open reading frame and/or immediately 5' to an open reading frame, the 3'-UTR element preferably between the open reading frame and a poly(A) sequence or a polyadenylation signal. Preferably, the 3'-UTR element and/or the 5'-UTR element is integrated into the artificial nucleic acid molecule or the vector, preferably

the mRNA, as 3'-UTR and/or as 5'-UTR respectively, i.e. such that the 3'-UTR element and/or the 5'-UTR element is the 3'-UTR and/or the 5'-UTR, respectively, of the artificial nucleic acid molecule or the vector, preferably the mRNA, i.e., such that the 5'-UTR ends immediately before the 5'-end of the ORF and the 3'-UTR extends from the 3'-side of the open reading frame to the 5'-side of a poly(A) sequence or a polyadenylation signal, optionally connected via a short linker, such as a sequence comprising or consisting of one or more restriction sites. Thus, preferably, the term "associating the artificial nucleic acid molecule or the vector with a 3'-UTR element and/or a 5'-UTR element" means functionally associating the 3'-UTR element and/or the 5'-UTR element with an open reading frame located within the artificial nucleic acid molecule or the vector, preferably within the mRNA molecule. The 3'-UTR and/or the 5'-UTR and the ORF are as described above for the artificial nucleic acid molecule according to the present invention, for example, preferably the ORF and the 3'-UTR are heterologous and/or the ORF and the 5'-UTR are heterologous,respectively, e.g. derived from different genes, as described above.

In a further aspect, the present invention provides the use of a 3'-UTR element and/or of a 5'-UTR element, preferably the 3'-UTR element as described above and/or the 5'-UTR element as described above, for increasing and/or prolonging protein production from an artificial nucleic acid molecule, preferably from an mRNA molecule or a vector, wherein the 3'-UTR element and/or the 5'-UTR element comprises or consists of a nucleic acid sequence which is derived from the 3'-UTR and/or the 5'-UTR of a transcript of a gene selected from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), MP68 (RIKEN cDNA 2010107E04 gene), NDUFA4 (NADH dehydrogenase (ubiquinone) 1 alpha subcomplex 4), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, Atp5e, Gstm5, Uqcr11, Ifi27l2a, Anapc13, Atp5l, Tmsb10, Nenf, Ndufa7, Atp5k, 1110008P14Rik, Cox4i1, Cox6a1, Ndufs6, Sec61b, Romo1, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufs5, 1810027O10Rik, Atp5o, Shfm1, Tspo, S100a6, Taldo1, Bloc1s1, Hexa, Ndufb11, Map1lc3a, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1; preferably from the group consisting of GNAS (guanine nucleotide binding protein, alpha stimulating complex locus), MORN2 (MORN repeat containing 2), GSTM1 (glutathione S-transferase, mu 1), NDUFA1 (NADH dehydrogenase (ubiquinone) 1 alpha subcomplex), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), and CNTN1 (contactin 1).

The uses according to the present invention preferably comprise associating the artificial nucleic acid molecule, the vector, or the RNA with the 3'-UTR element as described above and/or with the 5'-UTR element as described above.

The compounds and ingredients of the inventive pharmaceutical composition may also be manufactured and traded separately of each other. Thus, the invention relates further to a kit or kit of parts comprising an artificial nucleic acid molecule according to the invention, a vector according to the invention, a cell according to the invention, and/or a pharmaceutical composition according to the invention. Preferably, such kit or kits of parts may, additionally, comprise instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector, the cells or the pharmaceutical composition.

In a further aspect the present invention provides a method for identifying a 3'-untranslated region element (3'-UTR element) and/or a 5'-untranslated region element (5'-UTR element), which is derived from a stable mRNA, comprising the following steps:

a) Analyzing the stability of an mRNA comprising the following sub-steps:

i. Determining the amount of said mRNA at a first point in time during a decay process of said mRNA,
ii. Determining the amount of said mRNA at a second point in time during a decay process of said mRNA, and
iii. Calculating the ratio of the amount of said mRNA determined in step (i) to the the amount of said mRNA determined in step (ii);

b) Selecting a stable mRNA having a ratio calculated in sub-step (iii) of at least 0.5 (50%), at least 0.6 (60%), at least 0.7 (70%), at least 0.75 (75%), at least 0.8 (80%), at least 0.85 (85%), at least 0.9 (90%), or at least 0.95 (95%); and
c) Determining the nucleotide sequence of a 3'- and/or 5'-UTR element of said stable mRNA.

Thereby, the stability of the mRNA is preferably assessed under standard conditions, for example standard conditions (standard medium, incubation, etc.) for a certain cell line or cell type used.
In order to analyze the stability of an mRNA, the decay process of this mRNA is assessed by determining the amount or concentration of said mRNA at a first and at a second point in time during the decay process of said mRNA (cf. steps a) i. and a) ii.).
To determine the amount or concentration of mRNA during the RNA decay process *in vivo* or *in vitro* as defined above (i.e. *in vitro* referring in particular to ("living") cells and/or tissue, including tissue of a living subject; cells include in particular cell lines, primary cells, cells in tissue or subjects, preferred are mammalian cells, e.g. human cells and mouse cells and particularly preferred are the human cell lines HeLa, and U-937 and the mouse cell lines NIH3T3, JAWSII and L929 are used; furthermore primary cells are particularly preferred, in particular preferred embodiments human dermal fibroblasts (HDF)), various methods may be used, which are known to the skilled person. Non-limiting examples of such methods include general inhibition of transcription, e.g. with a transcription inhibitor such as Actinomycin D, use of inducible promotors to specifically promote transient transcription, e.g. c-fos serum-inducible promotor system and Tet-off regulatory promotor system, and kinetic labelling techniques, e.g. pulse labelling.
For example, if transcriptional inhibitor-mediated transcriptional arrest is used in step a) to determine the amount or concentration of mRNA during the RNA decay process *in vivo* or *in vitro* as defined above, transcriptional inhibitors such as Actinomycin D (ActD), 5,6-dichloro-1-D-ribofuranosyl-benzimidazole (DRB) or -amanitin ($\alpha$-Am) may be used. Hereby, to assess mRNA decay, the transcriptional inhibitors are usually added to the cells and, thereby the transcription is generally inhibited and RNA decay can be observed without interferences of ongoing transcription.
Alternatively, inducible promotors to specifically promote transient transcription may be used in step a), whereby the rationale is to provide a stimulus that activates transcription and leads to a burst of mRNA synthesis, then remove the stimulus to shut off transcription and monitor the decay of mRNA. Thereby, the inducible promoter enables a stringent control, so that induction and silencing of transcription is accomplished within a narrow window of time. In mammalian cells, the c*fos* promoter is known to be valuable for this purpose, because it can be induced in response to serum addition quickly and transiently, thereby providing a reliable and simple way of achieving a transient burst in transcription. The Tet-off promotor system offers another option that further broadens the application of a transcriptional pulsing approach to study mRNA turnover in mammalian cells.
However, in the present invention kinetic labelling techniques are preferred in step a) for determining the amount of mRNA during the RNA decay process *in vivo* or *in vitro* as defined above. In kinetic labelling RNA is usually labelled, whereby labels include in particular labelled nucleotides and labelled nucleosides and labelled uridine and labelled uracil are particularly preferred. Examples of preferred labels include 4-thiouridine (4sU), 2-thiouridine, 6-thioguanosine, 5-ethynyluridine (EU), 5-bromo-uridine (BrU), Biotin-16-Aminoallyluridine, 5-Aminoallyluridine, 5-Aminoallylcytidine, etc., whereby 4-Thiouridine (4sU), 5-Ethynyluridine (EU) or 5'-Bromo-Uridine (BrU) are more preferred. Particularly preferred is 4-thiouridine (4sU). 4-Thiouridine (4sU) is preferably used in a concentration of 100-500 $\mu$M. Moreover, also radioactively labelled nucleotides may be used, e.g. with Uridine-$^3$H. Also combinations of the above mentioned labelled nucleotides may be used, whereby a combination of 4-thiouridine and 6-thioguanosine is particularly preferred.
In kinetic labelling, usually the emerging RNA is labelled, e.g. by incorporation of labelled uridine or uracil during transcription. After a while, the provision of label is stopped and RNA decay may then be observed by assessing specifically labelled RNA without generally inhibiting transcription.
For determining the amount of mRNA during the RNA decay process in step a), pulse labelling is preferred, and a pulse-chase methodology is particularly preferred. As used herein, the term "pulse labelling refers to a technique in which a label, e.g. the labels described above, is used for the measurement of the rates of synthesis and/or decay of compounds within living cells. Typically, cells are exposed to a small quantity of a label for a brief period, hence the term 'pulse'. In the pulse-chase methodology, after pulse-labelling usually a much larger quantity of an unlabeled compound corresponding to the "pulse" (e.g. unlabelled uridine, if labelled uridine is used as pulse) is added following the required period of exposure to the label. The effect of competition between the labelled and the unlabeled compound is to reduce to a negligible level the further uptake of the labelled compound, hence the term "chase".
To determine the amount or concentration of mRNA usually the mRNA has to be isolated. Different techniques

for RNA isolation are known to the skilled person, e.g. by Guanidinium thiocyanate-phenol-chloroform extraction or by silica-column based extraction. Also commercially available kits may be used, e.g. RNeasy Kit from Qiagen.

Furthermore, an extraction step may be required, in particular if kinetic labelling is used (in contrast to a transcription inhibitor, wherein the total RNA represents "decaying" RNA since transcription is generally inhibited). In the extraction step, labelled RNA (i.e. representing "decaying" RNA) is extracted from total isolated RNA. Thus, the means of extraction may be selected depending on the label used. For example, immunopurification with antibodies to the label may be used.

Furthermore, for example, for extraction of thio-labelled, e.g. 4-thiouridine (4sU)-labelled, RNA, HPDP-Biotin (pyridyldithiol-activated, sulfhydryl-reactive biotinylation reagent that conjugates via a cleavable (reversible) disulfide bond) may be incubated with the isolated "total RNA". This reagent specifically reacts with the reduced thiols (-SH) in the 4-thiouridine (4sU)-labelled RNA to form reversible disulfide bonds. The biotinylation allows for binding of the thio-labelled e.g. 4-thiouridine (4sU)-labelled RNA to streptavidin and therefore can be extracted from the total RNA by reduction of the disulfide bond with dithiothreitol or betamercaptoethanol (or any other reduction agent).

In case biotin-labelled nucleotides, e.g. Biotin-16-Aminoallyluridine, streptavidin can directly be used to extract the labelled RNA from total RNA.

For example, for extraction of newly transcribed 5-ethynyluridine (EU)-labelled cellular RNAs from total RNA, biotinylation of EU in a copper-catalyzed cycloaddition reaction (often referred to as click chemistry) may be used, which is followed by purification by streptavidin affinity. This method is commercially available as the Click-iT Nascent RNA Capture Kit (Catalog no. C10365, Invitrogen). The manufacturer's instruction of this kit recommends that the pulse labeling time is 30 to 60 min for a 0.5 mM EU dose, or 1 to 24 h for a 0.1 or 0.2 mM EU dose.

For example, BrU-labeled RNA molecules may be extracted by immunopurification with an anti-Bromodeoxyuridine antibody (e.g. Clone. 2B1,Catalog no. MI-11-3, MBL), and Protein G Sepharose.

The amount or concentration of mRNA, i.e. the transcript level, may then be measured by various methods known to the person skilled in the art. Non-limiting examples for such methods include micro array analysis, Northern Blot analysis, quantitative PCR or by next generation sequencing (high throughput sequencing). Particularly preferred are micro array analysis and next generation sequencing. Moreover, whole-genome approaches/whole transcriptome approaches are particularly preferred, e.g. in micro array analysis whole genome micro array analysis, e.g. Affymetrix Human Gene 1.0 ST or 2.0 ST or Affymetrix Mouse Gene 1.0 ST or 2.0 ST or whole transcriptome analysis by next generation sequencing.

In substeps i. and ii. of step a), the amount of mRNA is determined at a first and at a second point in time during a decay process of the mRNA. Typically, this means that mRNA is in particular isolated at a first and at a second point in time during a decay process of the mRNA to determine the respective amounts. Therefore, "the first point in time" and "the second point in time" are in particular points in time during the RNA decay process, at which RNA is isolated to determine the RNA amount. In general, "the second point in time" is later in the RNA decay process than the "the first point in time".

Preferably, the first point in time is selected such, that only mRNA undergoing a decay process is considered, i.e. emerging mRNA - e.g. in ongoing transcription - is avoided. For example, if kinetic labelling techniques, e.g. pulse labelling, are used, the first point in time is preferably selected such that the incorporation of the label into mRNA is completed, i.e. no ongoing incorporation of the label into mRNA occurs. Thus, if kinetic labelling is used, the first point in time may be at least 10 min, at least 20 min, at least 30 min, at least 40 min, at least 50 min, at least 60 min, at least 70 min, at least 80 min, or at least 90 min after the end of the experimental labelling procedure, e.g. after the end of the incubation of cells with the label.

For example, the first point in time may be preferably from 0 to 6 h after the stop of transcription (e.g. by a transcriptional inhibitor), stop of promotor induction in case of inducible promotors or after stop of pulse or label supply, e.g. after end of labelling. More preferably, the first point in time may be from 30 min to 5 h, even more preferably from 1 h to 4 h and particularly preferably about 3 h after the stop of transcription (e.g. by a transcriptional inhibitor), stop of promotor induction in case of inducible promotors or after stop of pulse or label supply, e.g. after end of labelling.

Preferably, the second point in time is selected as late as possible during the mRNA decay process. However, if a plurality of mRNA species is considered, the second point in time is preferably selected such that still a considerable amount of the plurality of mRNA species, preferably at least 10% of the mRNA species, is present in a detectable amount, i.e. in an amount higher than 0. Preferably, the second point in time is at least 5 h, at least 6 h, at least 7 h, at least 8 h, at least 9 h, at least 10 h, at least 11 h, at least 12 h, at least 13 h, at least 14 h, or at least 15 h after the stop of transcription (e.g. by a transcriptional inhibitor), stop of promotor induction in case of inducible promotors or after stop of pulse or label supply, e.g. after end of labelling.

For example, the second point in time may be preferably from 3 to 48 h after the stop of transcription (e.g. by a transcriptional inhibitor), stop of promotor induction in case of inducible promotors or after stop of pulse or label supply, e.g. after end of labelling. More preferably, the second point in time may be from 6 min to 36 h, even more preferably from 10 h to 24 h and particularly preferably about 15 h after the stop of transcription (e.g. by a transcriptional inhibitor),

stop of promotor induction in case of inducible promotors or after stop of pulse or label supply, e.g. after end of labelling.

Thus, the time span between the first point in time and the second point in time is preferably as large as possible within the above described limits. Therefore, the time span between the first point in time and the second point in time is preferably at least 4 h, at least 5 h, at least 6 h, at least 7 h, at least 8 h, at least 9 h, at least 10 h, at least 11 h, or at least 12 h, whereby a time span of about 12 h is particularly preferred. In general, the second later point in time is at least 10 minutes later than the first point in time.

In sub-step iii. of step a) the ratio of the amount of the mRNA determined in step (i) to the the amount of the mRNA determined in step (ii) is calculated. To this end, the amount of the mRNA (transcript level) determined as described above at the second point in time is divided by the amount of the mRNA (transcript level) determined as described above at the first point in time. This ratio prevents that stable mRNAs, which are already at the first point in time present only in very low amounts, are disregarded in respect to mRNAs, which are present in high amounts.

In step b), such an mRNA is selected, which has a ratio calculated in sub-step (iii) of step a) of at least 0.5 (50%), at least 0.6 (60%), at least 0.7 (70%), at least 0.75 (75%), at least 0.8 (80%), at least 0.85 (85%), at least 0.9 (90%), or at least 0.95 (95%). Such mRNA is in the present invention considered as a particular stable mRNA.

In step c), the nucleotide sequence of a 3'- and/or 5'-UTR element of said mRNA, i.e. the mRNA selected in step b), is determined. To this end, different methods known to the skilled person may be applied, e.g. sequencing or selection from a publicly available database, such as e.g. NCBI (National Center for Biotechnology Information). For example, the mRNA sequence of the mRNA selected in step b) may be searched in a database and the 3'- and/or 5'-UTR may then be extracted from the mRNA sequence present in the database.

In particular, in the above described method for identifying a 3'-untranslated region element (3'-UTR element) and/or a 5'-untranslated region element (5'-UTR element), which is derived from a stable mRNA, the term "mRNA" and/or "stable mRNA", respectively, may also refer to an mRNA species as defined herein and/or to a stable mRNA species, respectively.

Furthermore, it is preferred in the present invention that a "stable mRNA" may have a slower mRNA decay compared to average mRNA decay, preferably assessed *in vivo* or *in vitro* as defined above. Thereby, "average mRNA decay" may be assessed by investigating mRNA decay of a plurality of mRNA species.

Accordingly, the present invention provides in a further aspect a method for identifying a 3'-untranslated region element (3'-UTR element) and/or a 5'-untranslated region element (5'-UTR element), which is derived from a stable mRNA, comprising the following steps:

a) Analyzing the stability of a plurality of mRNA species comprising the following sub-steps:

  i. Determining the amount of each mRNA species of said plurality of mRNA species at a first point in time during a decay process of said mRNA species,
  ii. Determining the amount of each mRNA species of said plurality of mRNA species at a second point in time during a decay process of said mRNA species, and
  iii. Calculating for each mRNA species of said plurality of mRNA species the ratio of the amount of said mRNA species determined in step (i) to the the amount of said mRNA species determined in step (ii);

b) Ranking of the mRNA species of the plurality of mRNA species according to the ratio calculated in sub-step (iii) for each mRNA species;
c) Selecting one or more mRNA species having the highest ratio or the highest ratios calculated in sub-step (iii); and
d) Determining the nucleotide sequence of a 3'- and/or 5'-UTR element of said mRNA.

An "mRNA species", as used herein, corresponds to a genomic transcription unit, i.e. usually to a gene. Thus, within one "mRNA species" different transcripts may occur, for example, due to mRNA processing. For example, an mRNA species may be represented by a spot on a microarray. Accordingly, a microarray provides an advantageous tool to determine the amount of a plurality of mRNA species, e.g. at a certain point in time during mRNA decay. However, also other techniques known to the skilled person, e.g. RNA-seq (also called Whole Transcriptome Shotgun Sequencing which is a technology that uses the capabilities of next-generation sequencing to reveal a snapshot of RNA presence and quantity from a genome at a given moment in time), quantitative PCR etc. may be used.

Preferably, "a plurality of mRNA species", refers to at least 100, at least 300, at least 500, at least 1000, at least 2000, at least 3000, at least 4000, at least 5000, at least 6000, at least 7000, at least 8000, at least 9000, at least 10000, at least 11000, at least 12000, at least 13000, at least 14000, at least 15000, at least 16000, at least 17000, at least 18000, at least 19000, at least 20000, at least 21000, at least 22000, at least 23000, at least 24000, at least 25000, at least 26000, at least 27000, at least 28000, at least 29000, or at least 30000 mRNA species. It is particularly preferred that the whole transcriptome is assessed, or as many mRNA species of the transcriptome as possible. This may be achieved, for example, by using a micro array providing whole transcript coverage.

Step a) of this method with its sub-steps i. to iii. corresponds essentially to step a) with its sub-steps i. to iii. of the previously described inventive method, but differs only in that the amount of each mRNA species of a plurality of mRNA species is determined at a first and at a second point in time and in that the ratio is calculated for each mRNA species. Accordingly, the detailed methods and preferred embodiments outlined above apply here as well and the ratio for a single mRNA species (and each single mRNA species, respectively) may be determined as outlined above for "an mRNA".

However, in contrast to the above method, the stability of the mRNA is not assessed by the absolute value of the ratio, but by a ranking of the mRNA species of the plurality of mRNA species according to the ratio calculated in sub-step (iii) of step a) for each mRNA species. In sub-step c) one or more mRNA species having the highest ratio or the highest ratios calculated in sub-step (iii) of step a) are then selected.

In this context it is particularly preferred to select the 0.1%, 0.2%, 0.3%, 0.4%, 0.5%, 0.6%, 0.7%, 0.8%, 0.9%, 1%, 2%, 3%, 4%, 5%, 10%, 15%, 20% most stable mRNA species in step c). Alternatively or additionally, in step c) such mRNA species may be selected which show a ratio calculated in sub-step iii. of step a) corresponding to a least 100% of the average ratio calculated from all mRNA species analyzed. More preferably such mRNA species are selected showing a ratio of at least 150%, even more preferably of at least 200% and most preferably of at least 300% of the average ratio calculated from all mRNA species analyzed.

In step d) the nucleotide sequence of a 3'- and/or 5'-UTR element of the mRNA selected in step c) is determined as described above, for step c) of the previously described inventive method.

Preferably, in both of the above described methods for identifying a 3'-UTR element and/or a 5'-UTR element according to the present invention, the time period between the first point in time and the second point in time is at least 5h, preferably at least 6h, preferably at least 7h, more preferably at least 8h, more preferably at least 9h, even more preferably at least 10h, even more preferably at least 11h, and particularly preferably at least 12h.

Preferably, in both of the above described methods for identifying a 3'-UTR element and/or a 5'-UTR element according to the present invention, the stability of an mRNA is analysed by pulse labelling, preferably using a pulse-chase methodology.

In a further aspect, the present invention also provides a method for identifying a 3'-untranslated region element (3'-UTR element) and/or a 5'-untranslated region element (5'-UTR element), which prolongs and/or increases protein production from an artificial nucleic acid molecule and which is derived from a stable mRNA comprising the following steps:

a) identifying a 3'-UTR element and/or a 5'-UTR element which is derived from a stable mRNA by a method for identifying a 3'-UTR element and/or a 5'-UTR element according to any of the methods described above;

b) synthesizing an artificial nucleic acid molecule comprising at least one open reading frame and at least one 3'-UTR element and/or at least one 5'-UTR element which corresponds to or is comprised by the 3'-UTR element and/or the 5'-UTR element identified in step a);

c) analyzing the expression of the protein encoded by the at least one open reading frame (ORF) of the artificial nucleic acid molecule synthesized in step b);

d) analyzing the expression of a protein encoded by at least one open reading frame of a reference artificial nucleic acid molecule lacking a 3'-UTR element and/or a 5'-UTR element;

e) comparing the protein expression from the artificial nucleic acid molecule analysed in step c) to the protein expression from the reference artificial nucleic acid molecule analysed in step d); and

f) selecting the 3'-UTR element and/or the 5'-UTR element if the protein expression from the artificial nucleic acid molecule analysed in step c) is prolonged and/or increased in comparison to the protein expression from the reference artificial nucleic acid molecule analysed in step d).

In this method, at first a 3'-UTR element and/or a 5'-UTR element are identified by a method according to the present invention as described above. This enables synthesis of the 3'- and/or the 5'-UTR element by methods known to the skilled person, e.g. by PCR amplification. The primers used for such a PCR may preferably comprise restriction sites for cloning. Alternatively, the 3'- and/or 5'-UTR element may be synthesized e.g. by chemical synthesis or oligo annealing. Accordingly, in step b), an artificial nucleic acid molecule is synthesized comprising at least one open reading frame and at least one 3'-UTR element and/or at least one 5'-UTR element which corresponds to or is comprised by the 3'-UTR element and/or the 5'-UTR element identified in step a). In particular, the at least one 3'-UTR element and/or at least one 5'-UTR element is usually combined with an open reading frame, which results in an artificial nucleic acid comprising a 3'- and/or 5'-UTR element according to the present invention, if the 3'- and/or 5'-UTR element fulfil the respective requirements, i.e. if they prolong and/or increase protein expression. To test this, the 3'- and/or the 5'-UTR element identified in step a), or a PCR fragment or synthesized sequence thereof respectively, may be cloned into a particular vector, preferably in an expression vector, in order to assess protein expression from the respective ORF.

The protein expression from the artificial nucleic acid molecule comprising the at least one 3'-UTR element and/or the at least one 5'-UTR element is then assessed in step c) as described herein and compared to the protein

expression assessed in step d) from a respective reference artificial nucleic acid molecule lacking a 3'-UTR element and/or a 5'-UTR element as described herein in step e).

Thereafter, in step f), such a 3'-UTR element and/or 5'-UTR element is selected, which prolongs and/or increases the protein expression from the artificial nucleic acid molecule analysed in step c) in comparison to the protein expression from the reference artificial nucleic acid molecule analysed in step d). The comparison of the protein expression of the inventive nucleic acid molecule to the reference nucleic acid molecule is carried out as described herein, in particular in the context of the inventive artificial nucleic acid molecule.

Furthermore, the present invention provides a particularly preferred method for identifying a 3'-untranslated region element (3'-UTR element) and/or a 5'-untranslated region element (5'-UTR element), which prolongs and/or increases protein production from an artificial nucleic acid molecule and which is derived from a stable mRNA comprising the following steps:

a) feeding/incubating cells with a labelled nucleotide for incorporation in newly transcribed RNA molecules (pulse-chase labelling);
b) isolating total RNA of the cells at a first point in time and at at least one second later point in time;
c) extracting of the labelled RNA molecules from the total RNA isolated in step b);
d) measuring of the amount/transcript level of the different mRNA species comprised in the labelled RNA;
e) calculating the ratio of the amount/transcript level of an mRNA species present at the at least one second later point in time to the amount/transcript level of the mRNA species present at the first point in time;
f) ranking of the mRNA species according to the ratio determined in step e);
g) selecting the most stable mRNA species;
h) determinating the nucleotide sequence of the 3'- and/or 5'-UTR of the most stable mRNA species selected in step g);
i) synthesizing a 3'- and/or a 5'-UTR element comprised in the 3'- and/or 5'-UTR determined in step h);
j) combination of the 3'- and/or 5'-UTR element synthesized in step i) with an open reading frame to get a nucleic acid according to the invention as described herein; and
k) optionally comparing the expression of the open reading frame present in the inventive nucleic acid compared to the expression of the open reading frame present in a reference nucleic acid without a 3'- and/or 5'-UTR element as described herein.

Thereby, the details and preferred embodiments described for the inventive methods above also apply herein, within the respective limitation outlined in steps a) to k).

In particular, the following labelled nucleotides are preferred for feeding the cells in step a) of the inventive method: 4-thiouridine (4sU), 2-thiouridine, 6-thioguanosine, 5-ethynyluridine (EU), 5-bromo-uridine (BrU), Biotin-16-Aminoallyluridine, 5-Aminoallyluridine, 5-Aminoallylcytidine, etc. Particularly preferred is 4-thiouridine (4sU). 4-thiouridine is preferably used in a concentration of 100-500 μM. Alternatively radioactively labelled nucleotides may be used, e.g. Uridine-$^3$H. Combinations of the above mentioned labelled nucleotides may be used. Particularly preferred is the combination of 4-thiouridine and 6-thioguanosine

The incubation of the cells with the labelled nucleotide in step a) can be varied. Particularly preferred is an incubation (feeding time) from 10 minutes to 24 hours. Particularly preferred are 2 to 6 hours, more preferably 2 to 3 hours.

Cells, which can be used for the inventive method, include in particular cell lines, primary cells, cells in tissue or subjects. In specific embodiments cell types allowing cell culture may be suitable for the inventive method. Particularly preferred are mammalian cells, e.g. human cells and mouse cells. In particularly preferred embodiments the human cell lines HeLa, and U-937 and the mouse cell lines NIH3T3, JAWSII and L929 are used. Furthermore primary cells are particularly preferred; in particular preferred embodiments particularly human dermal fibroblasts (HDF) can be used. Alternatively the labelled nucleotide may also be applied to a tissue of a subject and after the incubation time the RNA of the tissue is isolated according to step c).

For determination of the most stable mRNAs of a cell (type), total RNA is extracted at a first point in time as described above, e.g. 0 to 6 h after labelling, preferably 3 h after labelling and at a second later point in time as described above, e.g. 3 to 48 h after labelling, preferably 10 to 24 h, most preferably 15 h after labelling. The second later point in timeis at least 10 minutes later than the first time.

In step f) the mRNA species are ranked according to the ratio calculated in step e). In this context it is particularly preferred to select the 0.1 %, 0.2%, 0.3%, 0.4%, 0.5%, 0.6%, 0.7%, 0.8%, 0.9%, 1%, 2%, 3%, 4%, 5%, 10%, 15%, 20% most stable mRNA species.

In this context it is further preferred to select these mRNA species showing at least 50% (0,5 fold), at least 60% (0,6 fold), at least 70% (0,7 fold), at least 90% (0,9 fold) or at least 95% (0,95 fold) transcript level/amount of the mRNA species at the second later time compared to the first time. This embodiment is particularly preferred if the RNA is isolated at 3 hours (first point in time) and at 15 hours (second point in time) after labelling.

Alternatively or additionally, these mRNA species are selected showing a ratio calculated in step e) corresponding to a least 100% of the average ratio calculated from all mRNA species analyzed. More preferably these mRNA species are selected showing a ratio of at least 150% and more preferably of at least 200% and most preferably of at least 300% of the average ratio calculated from all mRNA species analyzed.

In a further step of the inventive method the nucleotide sequence of the 3'- and/or 5'-UTR of the most stable mRNA species selected in step g) is determined and in step i) the 3'- and/or 5'-UTR element is synthesized e.g. by PCR amplification. The primers used for the PCR may preferably comprise restriction sites for cloning.Alternatively the 3'- and/or 5'-UTR element may be synthesized (e.g. by chemical synthesis or oligo annealing).

In step j) of the inventive method the resulting PCR fragment or synthesized sequence is combined with an open reading frame resulting in an artificial nucleic acid comprising a 3'- and/or 5'-UTR element according to the invention. Preferably, the PCR fragment or sequence may be cloned into a vector.

In a particularly preferred embodiment the invention provides a method comprising the steps a) to k) for identifying 3'-untranslated region elements (3'-UTR elements) and/or 5'- untranslated region elements (5'-UTR elements), wherein the 3'-UTR elements and/or the 5'-UTR elements prolong protein production from an artificial nucleic acid molecule comprising at least one of the 3'-UTR elements and/or at least one of the 5'-UTR elements.

In a further aspect, the present invention also provides a method for generating an artificial nucleic acid molecule, wherein an artificial nucleic acid molecule comprising at least one open reading frame and at least one 3'-UTR element and/or at least one 5'-UTR element identified by a method for identifying a 3'-UTR element and/or a 5'-UTR element according to the present invention as described above is synthesized. Synthesizing of such an artificial nucleic acid molecule is typically carried out by methods known to the skilled person, e.g. cloning methods for example as generally known or described herein.

Preferably, a vector according to the present invention as described herein is used in such an inventive method for generating an artificial nucleic acid molecule.

Preferably, the artificial nucleic acid molecule generated by such a method for generating an artificial nucleic acid molecule is a nucleic acid molecule according to the present invention as described herein.

In addition, the present invention also provides an artificial nucleic acid molecule obtainable by a method for generating an artificial nucleic acid molecule according to the present invention as described herein.

The following Figures, Sequences and Examples are intended to illustrate the invention further. They are not intended to limit the subject matter of the invention thereto.

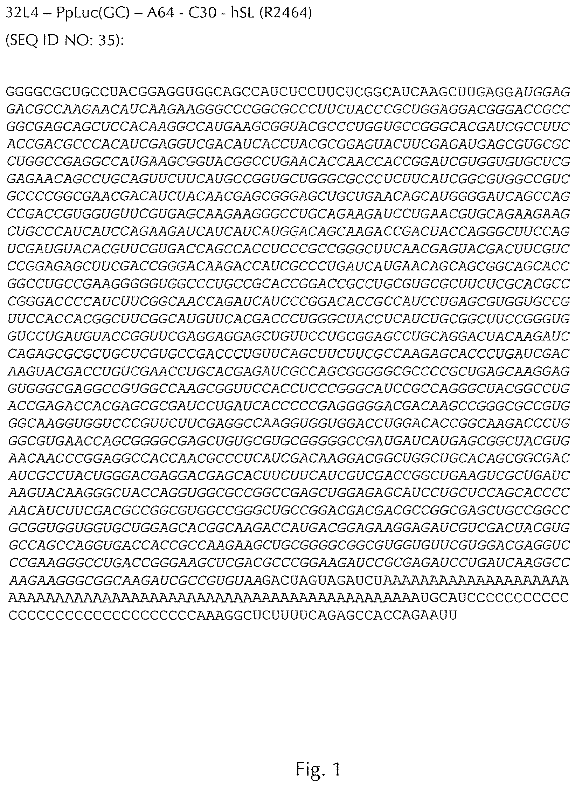

Figures 1 to 11, 19 to 21 and 25 to 30 show sequences encoding mRNAs that can be obtained by in vitro transcription. The following abbreviations are used:

- PpLuc (GC): GC-enriched mRNA sequence coding for *Photinus pyralis* luciferase
- A64: poly(A)-sequence with 64 adenylates
- C30: poly(C)-sequence with 30 cytidylates
- hSL: a histone stem-loop sequence taken from (Cakmakci, Lerner, Wagner, Zheng, & William F Marzluff, 2008. Mol. Cell. Biol. 28(3):1182-94)
- 32L4: 5'-UTR of human ribosomal protein Large 32 lacking the 5' terminal oligopyrimidine tract
- albumin7: 3'-UTR of human albumin with three single point mutations introduced to remove a T7 termination signal as well as a HindIII and XbaI restriction site
- gnas: 3'-UTR element derived from the 3'-UTR of murine gnas; Mus musculus GNAS (guanine nucleotide binding protein, alpha stimulating) complex locus (Gnas), mRNA
- morn2: 3'-UTR element derived from the 3'-UTR of murine morn2; Mus musculus MORN repeat containing 2 (Morn2), mRNA
- gstm1: 3'-UTR element derived from the 3'-UTR of murine gstm1; Mus musculus glutathione S-transferase, mu 1 (Gstm1), mRNA
- ndufa1: 3'-UTR element derived from the 3'-UTR of murine ndufa1; Mus musculus NADH dehydrogenase (ubiquinone) 1 alpha subcomplex, (Ndufa1), mRNA
- cbr2: 3'-UTR element derived from the 3'-UTR of murine cbr2; Mus musculus carbonyl reductase 2 (Cbr2), mRNA
- mp68: 5'-UTR element derived from the 5'-UTR of murine mp68; Mus musculus RIKEN cDNA 2010107E04 gene (2010107E04Rik), mRNA
- ndufa4: 5'-UTR element derived from the 5'-UTR of murine nudfa4; Mus musculus NADH dehydrogenase (ubiquinone) 1 alpha subcomplex,4, (Ndufa4), mRNA
- Ybx1: 3'-UTR element derived from the 3'-UTR of murine Ybx1 (Y-Box binding protein 1)
- Ndufb8: 3'-UTR element derived from the 3'-UTR of murine Ndufb8 (NADH dehydrogenase (ubiquinone) 1 beta subcomplex 8)
- CNTN1: 3'-UTR element derived from the 3'-UTR of human CNTN1 (contactin 1)

Fig. 1: shows SEQ ID NO. 35, i.e. the mRNA sequence of 32L4 - PpLuc(GC) -A64 -C30 - hSL. (R2464). The 5'-UTR is derived of human ribosomal protein Large 32 mRNA lacking the 5' terminal oligopyrimidine tract. The PpLuc(GC) ORF is highlighted in italics.

Fig. 2: shows SEQ ID NO. 36, i.e. the mRNA sequence of 32L4 - PpLuc(GC) - gnas-A64-C30-hSL. (R3089). The PpLuc(GC) ORF is highlighted in italics. The 3'-UTR element, which is derived from mouse Gnas transcript, is underlined.

Fig. 3: shows SEQ ID NO. 37, i.e. the mRNA sequence of 32L4 - PpLuc(GC) - morn2-A64 - C30 - hSL. (R3106). The PpLuc(GC) ORF is highlighted in italics. The 3'-UTR element, which is derived from mouse morn2, is underlined.

Fig. 4: shows SEQ ID NO. 38, i.e. the mRNA sequence of 32L4 - PpLuc(GC) - gstm1-A64 - C30 - hSL. (R3107). The PpLuc(GC) ORF is highlighted in italics. The 3'-UTR element, which is derived from mouse gstm1, is underlined.

Fig. 5: shows SEQ ID NO. 39, i.e. the mRNA sequence of 32L4 - PpLuc(GC) - ndufa1-A64 - C30 - hSL. (R3108). The PpLuc(GC) ORF is highlighted in italics. The 3'-UTR element, which is derived from mouse ndufa1, is underlined.

Fig. 6: shows SEQ ID NO. 40, i.e. the mRNA sequence of 32L4 - PpLuc(GC) - cbr2-A64 - C30 - hSL. (R3109). The PpLuc(GC) ORF is highlighted in italics. The 3'-UTR element, which is derived from mouse cbr2, is underlined.

Fig. 7: shows SEQ ID NO. 41, i.e. the mRNA sequence of PpLuc(GC) - albumin7- A64-C30 - hSL. (R2463). The 3'-UTR is derived from human albumin with three single point mutations introduced to remove a T7 termination signal as well as a HindIII and XbaI restriction site (albumin7). The PpLuc(GC) ORF is highlighted in italics.

Fig. 8: shows SEQ ID NO. 42, i.e. the mRNA sequence of Mp68 - PpLuc(GC) - albumin7-A64 - C30 - hSL. (R3111). The PpLuc(GC) ORF is highlighted in italics. The 5'-UTR element, which is derived from mouse mp68, is underlined.

Fig. 9: shows SEQ ID NO. 43, i.e. the mRNA sequence of Ndufa4 - PpLuc(GC)-albumin7- A64 - C30 - hSL. (R3112). The PpLuc(GC) ORF is highlighted in italics. The 5'-UTR element, which is derived from mouse Ndufa4, is underlined.

Fig. 10: shows SEQ ID NO. 44, i.e. the mRNA sequence of PpLuc(GC) - A64 - C30 - hSL (R2462) The PpLuc(GC) ORF is highlighted in italics.

Fig. 11: shows SEQ ID NO. 45, i.e. the mRNA sequence of PpLuc(GC) - gnas- A64 - C30 - hSL (R3116). The PpLuc(GC) ORF is highlighted in italics. The 3'-UTR element, which is derived from mouse Gnas, is underlined.

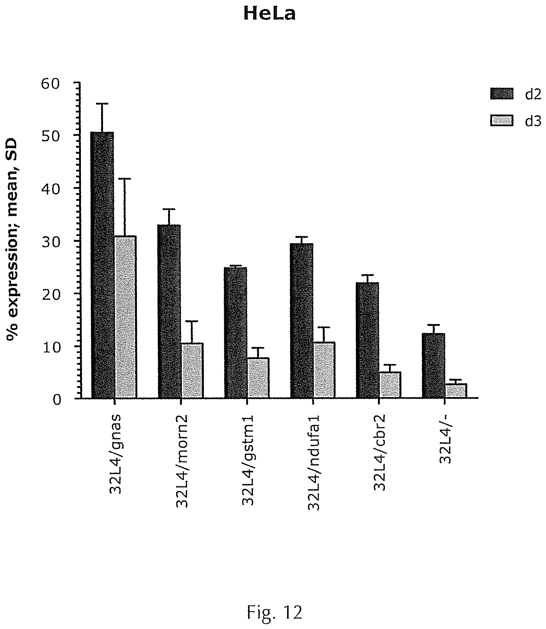

Fig. 12: shows that different 3'-UTR elements, namely 3'-UTR elements derived from gnas, morn2, gstm1, ndufa1 and cbr2 markedly prolong protein expression from mRNA. The effect of the inventive 3'-UTR elements derived from gnas, morn2, gstm1, ndufa1 and cbr2 3'-UTRs on luciferase expression from mRNA was examined, compared to luciferase expression from mRNA lacking a 3'-UTR. To this end, human HeLa were transfected with different mRNAs by lipofection. Luciferase levels were measured at different times after transfection. The PpLuc signal was corrected for transfection efficiency by the signal of cotransfected RrLuc. Normalized PpLuc levels at 24h were set to 100% and relative expression to 24h was calculated. The 3'-UTRs prolong luciferase expression. Mean values from three independent experiments are shown. Values are summarized in Example 7.a.

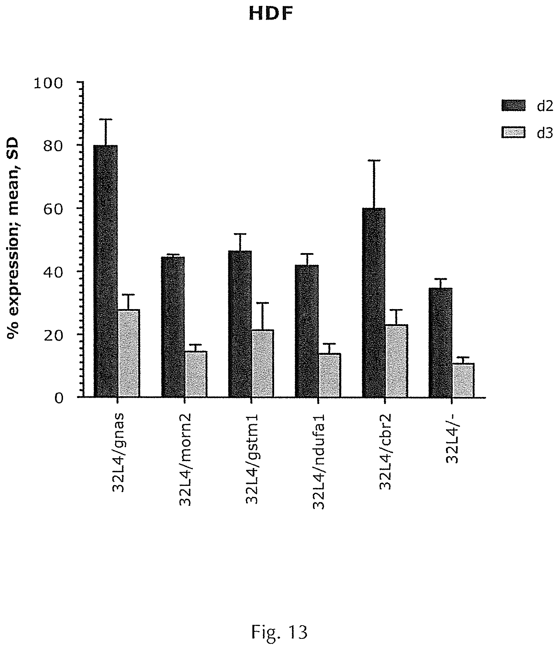

Fig. 13: shows that different 3'-UTR elements, namely 3'-UTR elements derived from gnas, morn2, gstm1, ndufa1 and cbr2 markedly prolong protein expression from mRNA. The effect of the inventive 3'-UTR elements derived from gnas, morn2, gstm1, ndufa1 and cbr2 3'-UTRs on luciferase expression from mRNA was examined, compared to luciferase expression from mRNA lacking a 3'-UTR. To this end, HDF (human dermal fibroblasts) cells were transfected with different mRNAs by lipofection. Luciferase levels were measured at different times after transfection. The PpLuc signal was corrected for transfection efficiency by the signal of cotransfected RrLuc. Normalized PpLuc levels at 24h were set to 100% and relative expression to 24h was

calculated. The 3'-UTRs prolong luciferase expression. Mean values from three independent experiments are shown. Values are summarized in Example 7.a.

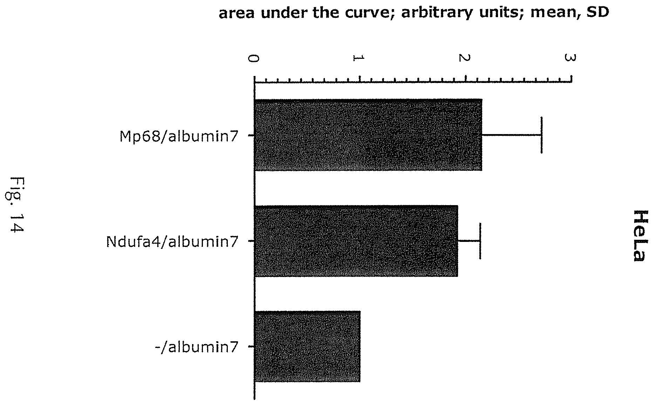

Fig. 14: shows that different 5'-UTR elements, namely 5'-UTR elements derived from Mp68 and ndufa4 markedly increase total protein expression from mRNA. The effect of the inventive 5'-UTR elements derived from Mp68 and ndufa4 on luciferase expression from mRNA was examined. To this end, human HeLa cells were transfected with different mRNAs by lipofection. Luciferase levels were measured 6, 24, 48, and 72 hours after transfection. The PpLuc signal was corrected for transfection efficiency by the signal of cotransfected RrLuc. Total protein expression (area under the curve) was calculated. To compare the expression levels of the mRNAs containing the inventive 5'-UTR elements to an mRNA lacking a 5'-UTR, expression levels of the control construct without 5' UTR was set to 1. Mean values from three independent experiments are shown. Values are summarized in Example 7.b.

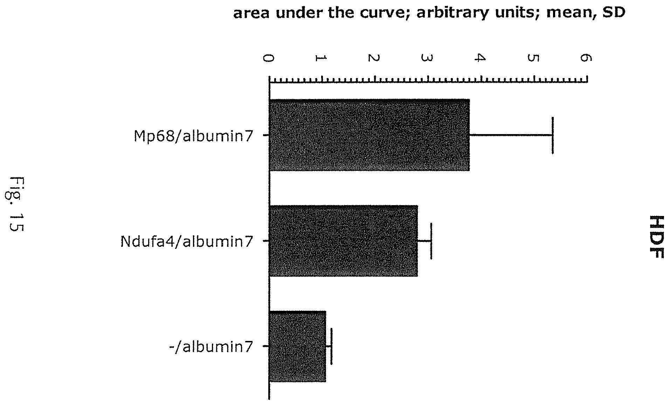

Fig. 15: shows that different 5'-UTR elements, namely 5'-UTR elements derived from Mp68 and ndufa4 markedly increase total protein expression from mRNA. The effect of the inventive 5'-UTR elements derived from Mp68 and ndufa4 on luciferase expression from mRNA was examined. To this end, HDF cells were transfected with different mRNAs by lipofection. Luciferase levels were measured 6, 24, 48, and 72 hours after transfection. The PpLuc signal was corrected for transfection efficiency by the signal of cotransfected RrLuc. Total protein expression (area under the curve) was calculated. To compare the expression levels of the mRNAs containing the inventive 5'-UTR elements to an mRNA lacking a 5'-UTR, expression levels of the control construct without 5' UTR was set to 1. Mean values from three independent experiments are shown. Values are summarized in Example 7.b.

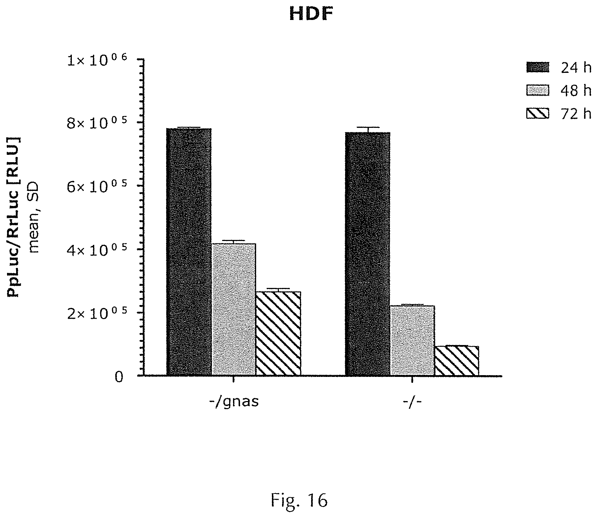

Fig. 16: shows that the 3'-UTR element derived from gnas markedly prolongs protein expression from mRNA. The effect of the inventive 3'-UTR element derived from gnas 3'-UTR on luciferase expression from mRNA was examined, compared to luciferase expression from mRNA lacking a 3'-UTR. To this end, HDF cells were transfected with respective mRNAs by lipofection. Luciferase levels were measured at 24, 48, and 72 hours after transfection. The PpLuc signal was corrected for transfection efficiency by the signal of cotransfected RrLuc. Normalized PpLuc levels at 24h were set to 100% and relative expression to 24h was calculated. The gnas 3'-UTR prolongs luciferase expression. Values are summarized in Example 7.c.

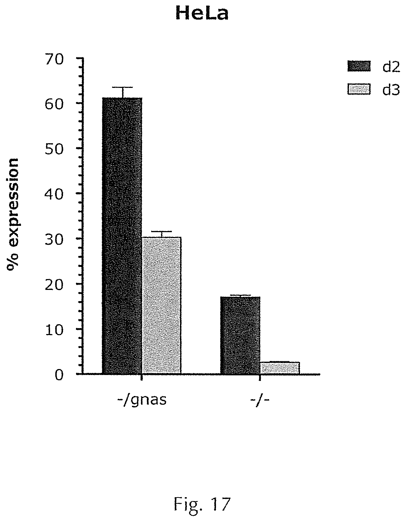

Fig. 17: shows that the 3'-UTR element derived from gnas markedly prolongs protein expression from mRNA.
The effect of the inventive 3'-UTR element derived from gnas 3'-UTR on luciferase expression from mRNA was examined, compared to luciferase expression from mRNA lacking a 3'-UTR. To this end, HeLa cells were transfected with respective mRNAs by lipofection. Luciferase levels were measured at d2 and d3 after transfection. The PpLuc signal was corrected for transfection efficiency by the signal of cotransfected RrLuc. Normalized PpLuc levels at 24h were set to 100% and relative expression to 24h was calculated. The gnas 3'-UTR prolongs luciferase expression. Values are summarized in Example 7.c.

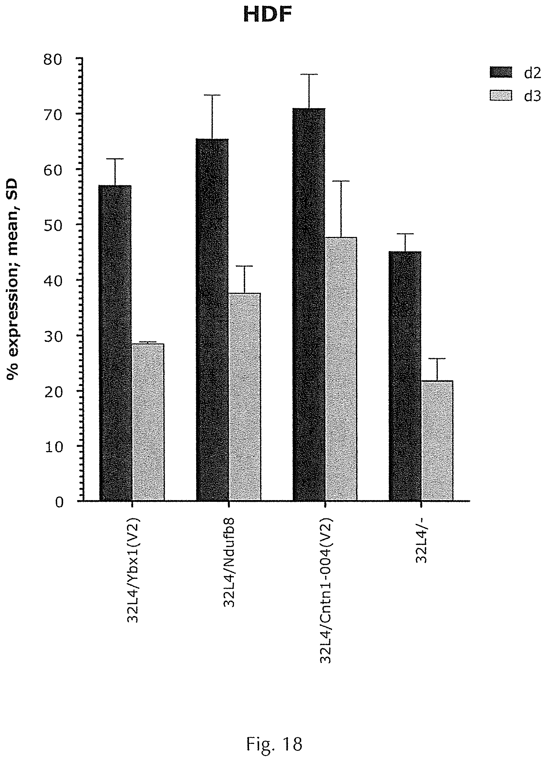

Fig. 18: shows that different 3'-UTR elements, namely 3'-UTR elements derived from ybx1(V2), ndufb8, and cntn1-004(V2) markedly prolong protein expression from mRNA.
The effect of the inventive 3'-UTR elements derived from ybx1(V2), ndufb8, and cntn1-004(V2) 3'-UTRs on luciferase expression from mRNA was examined, compared to luciferase expression from mRNA lacking a 3'-UTR. To this end, HDF cells were transfected with the different mRNAs by lipofection. Luciferase levels were measured at different times after transfection. The PpLuc signal was corrected for transfection efficiency by the signal of cotransfected RrLuc. Normalized PpLuc levels at 24h were set to 100% and relative expression to 24h was calculated. The 3'-UTRs prolong luciferase expression. Values are summarized in Example 7.d.

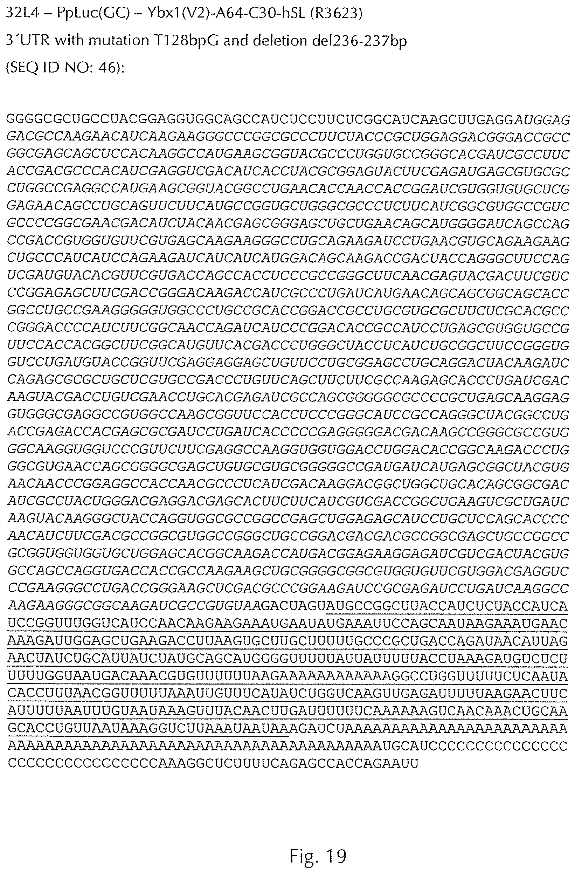

Fig. 19: shows SEQ ID NO. 46 , i.e. the mRNA sequence of 32L4 - PpLuc(GC) - Ybx1-001(V2)-A64-C30-hSL (R3623) mus musculus 3'UTR with mutation T128bpG and deletion del236-237bp. The PpLuc(GC) ORF is highlighted in italics. The 3'-UTR element, which is derived from mouse Ybx1 transcript, is underlined.

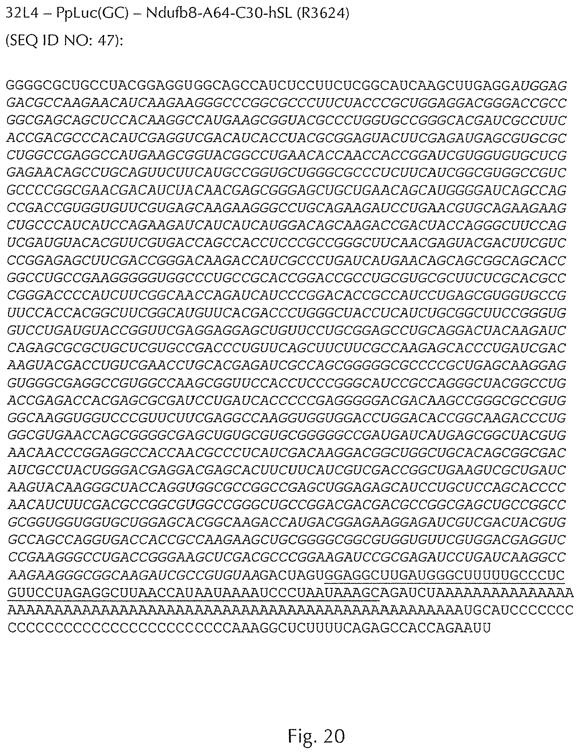

Fig. 20: shows SEQ ID NO. 47, i.e. the mRNA sequence of 32L4 - PpLuc(GC) - Ndufb8-A64-C30-hSL (R3624) The PpLuc(GC) ORF is highlighted in italics. The 3'-UTR element, which is derived from mouse Ndufb8 transcript, is underlined.

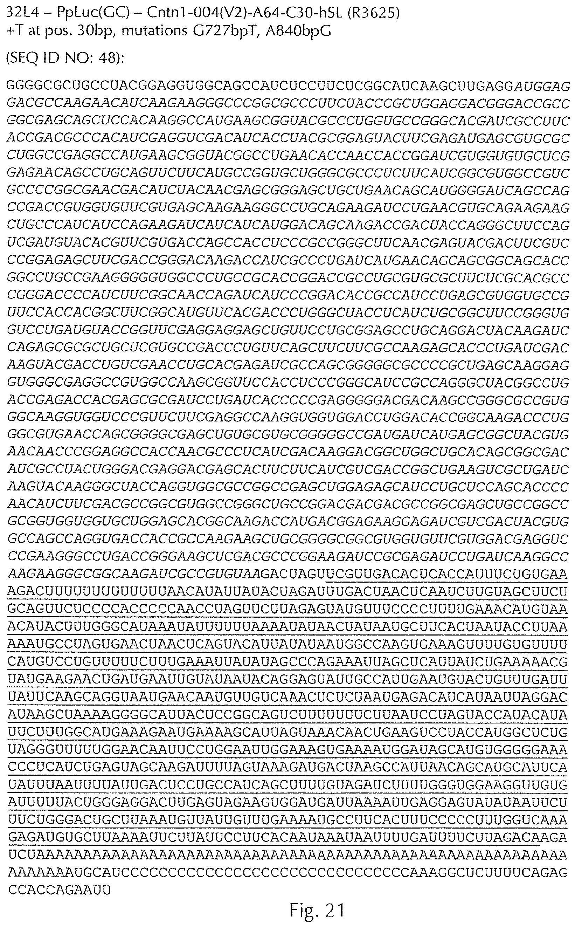

Fig. 21: shows SEQ ID NO. 48, i.e. the mRNA sequence of 32L4 - PpLuc(GC) - Cntn1-004(V2)-A64-C30-hSL (R3625) +T at pos. 30bp, mutations G727bpT, A840bpG. The PpLuc(GC) ORF is highlighted in italics. The 3'-UTR

element, which is derived from human Cntn1 transcript, is underlined.

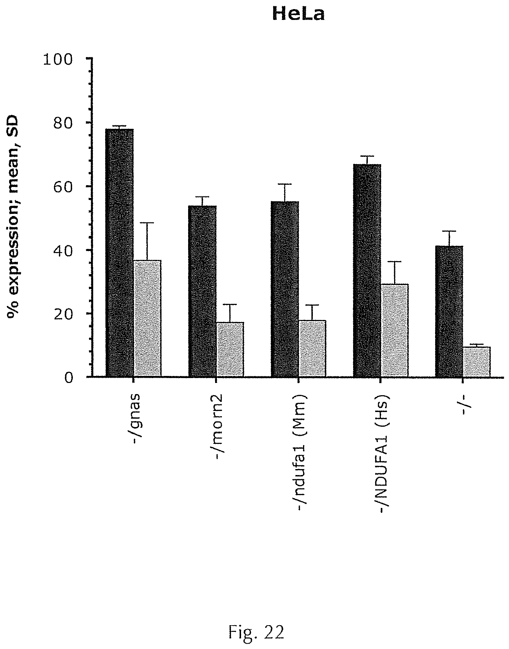

Fig. 22: shows that different 3'-UTR elements, namely 3'-UTR elements derived from gnas, morn2, ndufa1 (Mm; mus musculus), and NDUFA1 (Hs; homo sapiens) markedly prolong protein expression from mRNA. The effect of the inventive 3'-UTR elements derived from gnas, morn2, ndufa1 (Mm; mus musculus), and NDUFA1 (Hs; homo sapiens) on luciferase expression from mRNA was examined, compared to luciferase expression from mRNA lacking a 3'-UTR. To this end, human Hela cells were transfected with respective mRNAs by lipofection. Luciferase levels were measured at different times after transfection. The PpLuc signal was corrected for transfection efficiency by the signal of cotransfected RrLuc. Normalized PpLuc levels at 24h were set to 100% and relative expression to 24h is calculated. The 3'UTRs prolong luciferase expression. Mean values from 3 independent experiments are shown. Values are summarized in Table 8.

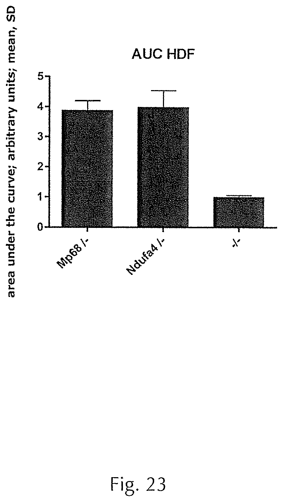

Fig. 23: shows that different 5'-UTR elements, namely 5'-UTR elements derived from Mp68 and ndufa4, markedly increase total protein expression from mRNA. The effect of the inventive 5'-UTR elements derived from Mp68 and ndufa4 on luciferase expression from mRNA was examined. To this end, human HeLa cells were transfected with different mRNAs by lipofection. Luciferase levels were measured 6, 24, 48, and 72 hours after transfection. The PpLuc signal was corrected for transfection efficiency by the signal of cotransfected RrLuc. Total protein expression (area under the curve) was calculated. To compare the expression levels of the mRNAs containing the inventive 5'-UTR elements to an mRNA lacking a 5'-UTR, expression levels of the control construct without 5' UTR was set to 1. Mean values are shown. Values are summarized in Table 9.

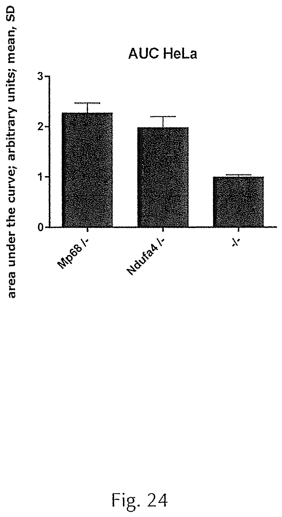

Fig. 24: shows that different 5'-UTR elements, namely 5'-UTR elements derived from Mp68 and ndufa4, markedly increase total protein expression from mRNA. The effect of the inventive 5'-UTR elements derived from Mp68 and ndufa4 on luciferase expression from mRNA was examined. To this end, human Hela cells were transfected with different mRNAs by lipofection. Luciferase levels were measured 24, 48, and 72 hours after transfection. The PpLuc signal was corrected for transfection efficiency by the signal of cotransfected RrLuc. Total protein expression (area under the curve) was calculated. To compare the expression levels of the mRNAs containing the inventive 5'-UTR elements to an mRNA lacking a 5'-UTR, expression levels of the control construct without 5' UTR was set to 1. Mean values are shown. Values are summarized in Table 9.

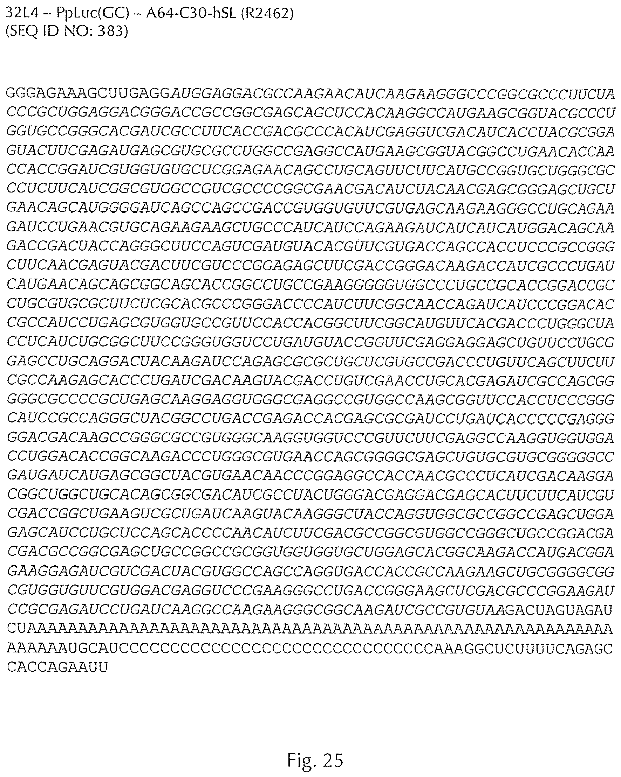

Fig. 25: shows SEQ ID NO. 383, i.e. the mRNA sequence of 32L4 - PpLuc(GC) - A64-C30-hSL (R2462). The PpLuc(GC) ORF is highlighted in italics.

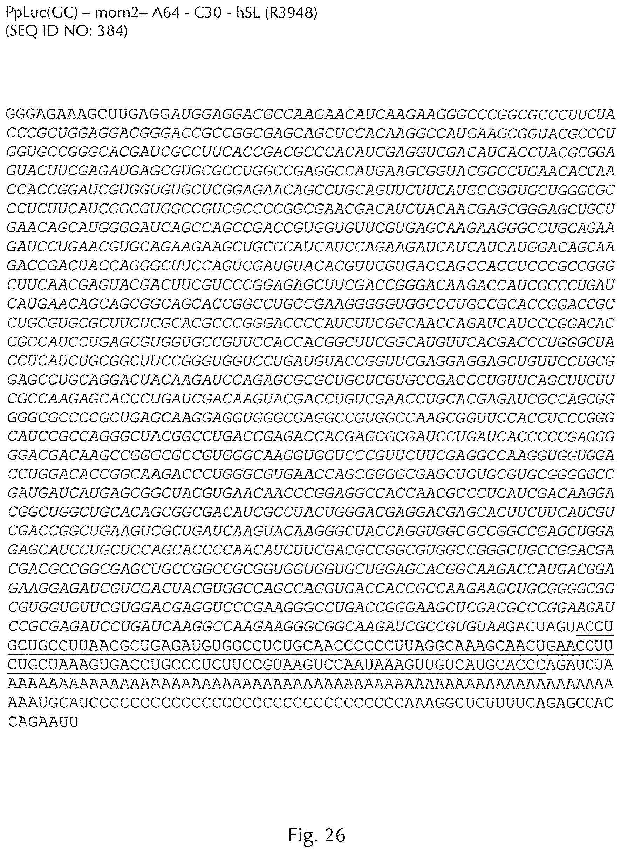

Fig. 26: shows SEQ ID NO. 384, i.e. the mRNA sequence of PpLuc(GC) - morn2- A64-C30 - hSL. (R3948). The PpLuc(GC) ORF is highlighted in italics. The 3'-UTR element, which is derived from murine morn2 is underlined.

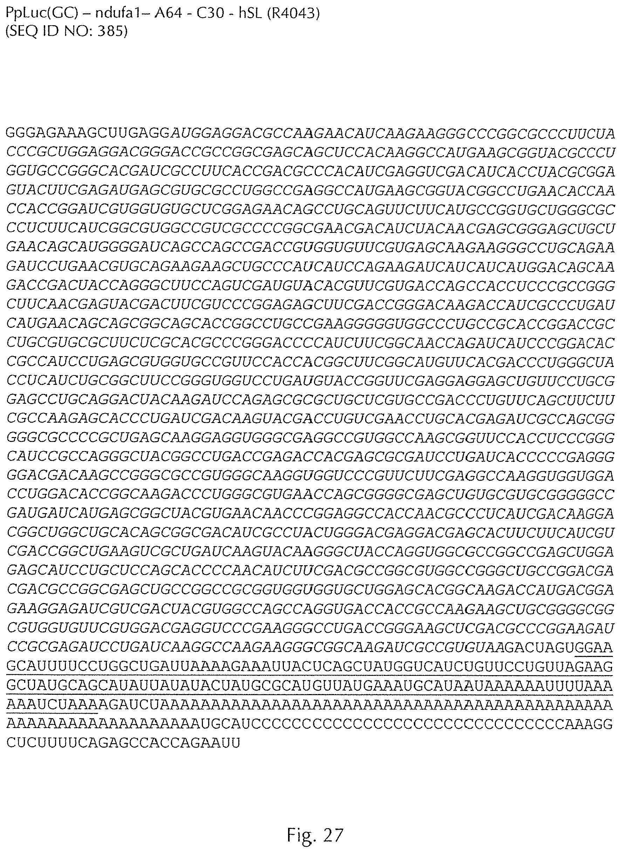

Fig. 27: shows SEQ ID NO. 385, i.e. the mRNA sequence of PpLuc(GC) - ndufa1- A64-C30 - hSL. (R4043). The PpLuc(GC) ORF is highlighted in italics. The 3'-UTR element, which is derived from murine ndufa1 is underlined.

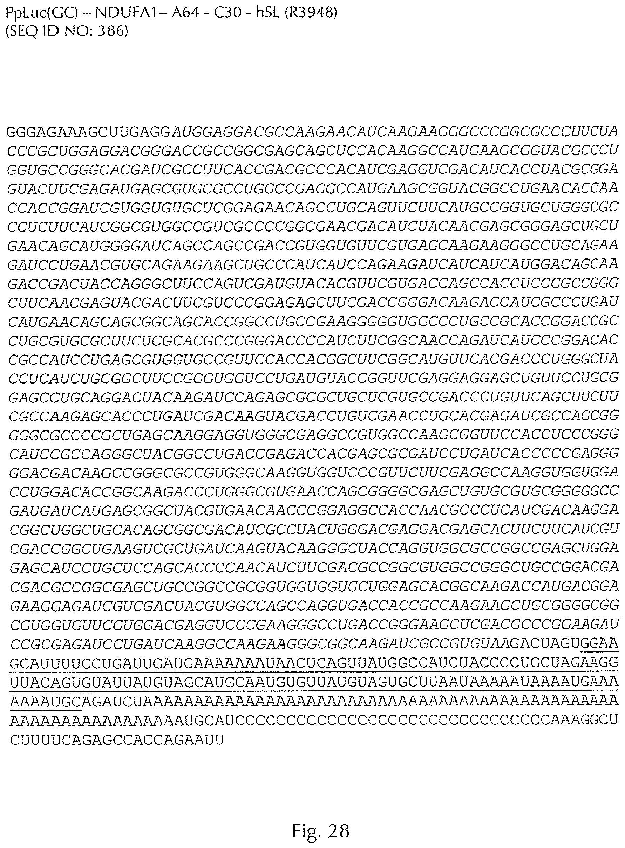

Fig. 28: shows SEQ ID NO. 386, i.e. the mRNA sequence of PpLuc(GC) - NDFUA1-A64 - C30 - hSL. (R3948). The PpLuc(GC) ORF is highlighted in italics. The 3'-UTR element, which is derived from human NDUFA1 is underlined.

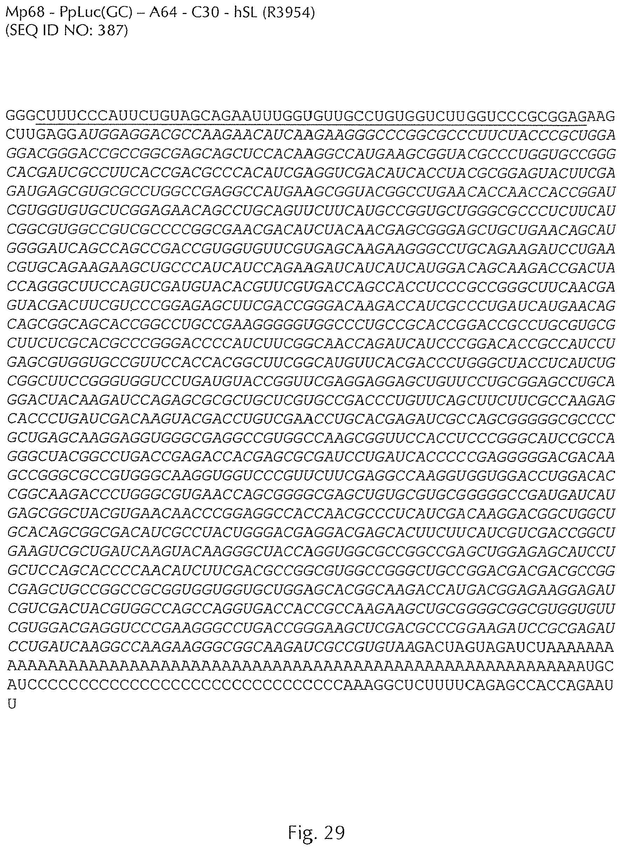

Fig. 29: shows SEQ ID NO. 387, i.e. the mRNA sequence of Mp68 - PpLuc(GC) - A64-C30 - hSL. (R3954). The PpLuc(GC) ORF is highlighted in italics. The 5'-UTR element, which is derived from murine mp68 is underlined.

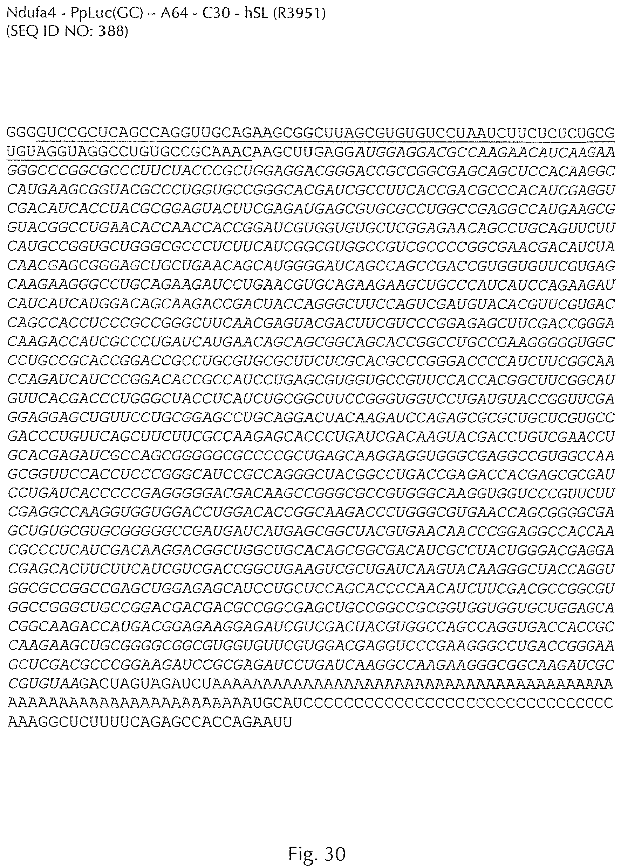

Fig. 30: shows SEQ ID NO. 388, i.e. the mRNA sequence of Ndufa4 - PpLuc(GC) - A64-C30 - hSL (R3951). The PpLuc(GC) ORF is highlighted in italics. The 5'-UTR element, which is derived from murine ndufa4 is underlined.

Examples

1. Identification of 3'-untranslated region elements (3'-UTR elements) and/or 5'-untranslated region elements (5'-UTR elements) prolonging and/or increasing protein production:

mRNA decay in different human and murine cell types was assessed by pulse-chase methodology. To this

end, three different human cell types (HeLa, HDF and U-937) and three different mouse cell types (NIH3T3, JAWSII and L929) were plated over night in their respective medium: HeLa, U-937, L929 in RPMI medium, JAWSII und NIH3T3 in DMEM and HDF in Fibroblast Growth Medium 2. The cells were incubated for 3 h with the respective medium containing 200 μM 4-thiouridine (4sU) for labelling of newly synthesized RNA ("pulse"). After incubation (labelling), cells are washed once and the medium was replaced by fresh medium supplemented with 2mM Uridine ("chase"). Cells were incubated further for 3 h (1st point in time) or 15 h ($2^{nd}$ point in time) before harvesting.

Accordingly, cells were harvested 3 h (1st point in time) and 15 h ($2^{nd}$ point in time) after end of labelling. The total RNA was isolated from these cells using RNeasy Mini Kit (Qiagen).

HPDP-Biotin (EZ-Link Biotin-HPDP, Thermo Scientific; pyridyldithiol-activated, sulfhydryl-reactive biotinylation reagent that conjugates via a cleavable (reversible) disulfide bond) was then incubated with the total RNA in order to extract the 4-thiouridine (4sU)-labelled RNA. HPDP-Biotin specifically reacts with the reduced thiols (-SH) in the 4-thiouridine (4sU)-labelled RNA to form reversible disulfide bonds. The biotinylated RNA was ultrafiltrated using an Amicon-30 device, incubated with streptavidin-coupled dynabeads (Life Technologies) and recovered from streptavidin by DTT. Subsequently, the RNA was purified using RNeasy Mini Kit. For each cell line 3 independent experiments were performed.

The extracted 4sU-labelled RNA was used in a micro array analysis in order to determine the transcript levels of a large variety of mRNA species (i.e. the amounts of the mRNA species) present at a first point in time (3 h after labelling) and the transcript levels of a large variety of mRNA species (i.e. the amounts of the mRNA species) present at a second point in time (15 h after labelling). Affymetrix Human Gene 1.0 ST and Affymetrix Mouse Gene 1.0 ST micro arrays were used. Affymetrix Human Gene 1.0 ST comprises 36079 mRNA species. Affymetrix Mouse Gene 1.0 ST comprises 26166 mRNA species.

Since these micro arrays provide a whole transcript coverage, i.e. they provide a complete expression profile of mRNA, the ratio of the transcript level of a certain mRNA species at the second point in time to the transcript level of the same mRNA species at the first point in time was accordingly determined for a large number of mRNA species. The ratios thus reflect the x-fold transcript level of the mRNA species (shown as Gene Symbol) at the second point in time as compared to the first point in time.

The results from these experiments are shown in Tables 1 - 3 below. Each of the Tables 1 - 3 shows a ranking of the most stable mRNA species, i.e. according to the ratio of the transcript level of this mRNA species at the second point in time to the transcript level of this mRNA species at the first point in time (Table 1: combined analysis of human cell types (HeLa, HDF and U-937); Table 2: combined analysis of mouse cell lines (NIH3T3, JAWSII and L929); Table 3: human cell line HDF (human dermal fibroblasts)). Such mRNA species were considered as "most stable mRNA species", which show a value for the ratio of the transcript level at the first point in time/ the transcript level at the second point in time of at least 0,549943138 (approximately 55%; Table 1), 0,676314425 (approximately 68%, Table 2) or 0,8033973 (approximately 80%, Table 3).

Furthermore, the relationship of the ratio of a certain mRNA species to the average ratio (i.e. the average of the ratios of all mRNA species determined, which is shown in the Tables as "Average of the ratio") was calculated and given as % of average.

Table 1: stable mRNAs resulting from the combined analysis of human cell types (HeLa, HDF and U-937) with the Affymetrix Human Gene 1.0 ST micro array. 113 mRNA species of the 36079 mRNA species on the micro array were selected as "most stable" mRNA species. This corresponds to 0,31 % of the mRNA species present on the micro array.

| Gene symbol | Ratio of the transcript level at the 2nd time to the transcript level at the 1st time | Average of the ratio | % of average |
|---|---|---|---|
| LTA4H | 0,982490359 | 0,258826017 | 379,5948991 |
| SLC38A6 | 0,953694877 | | 368,4694789 |
| DECR1 | 0,927429689 | | 358,3216631 |
| PIGK | 0,875178367 | | 338,1338462 |
| FAM175A | 0,849392515 | | 328,1712266 |
| PHYH | 0,827905031 | | 319,8693239 |
| NT5DC1 | 0,815986179 | | 315,2643572 |
| TBC1D19 | 0,805960687 | | 311,3909086 |
| PIGB | 0,805108608 | | 311,0616997 |
| ALG6 | 0,804875859 | | 310,9717748 |

(continued)

| Gene symbol | Ratio of the transcript level at the 2nd time to the transcript level at the 1st time | Average of the ratio | % of average |
|---|---|---|---|
| CRYZ | 0,797694475 | | 308,1971756 |
| BRP44L | 0,796150905 | | 307,6008021 |
| ACADSB | 0,792385554 | | 306,1460216 |
| SUPT3H | 0,792305264 | | 306,1150005 |
| TMEM14A | 0,792128439 | | 306,0466827 |
| GRAMD1C | 0,78766459 | | 304,3220303 |
| C11orf80 | 0,778391775 | | 300,739386 |
| C9orf46 | 0,776061355 | | 299,8390053 |
| ANXA4 | 0,765663559 | | 295,8217134 |
| RAB7A | 0,757621668 | | 292,7146492 |
| TBCK | 0,753324047 | | 291,0542204 |
| AGA | 0,751782245 | | 290,4585303 |
| IFI6 | 0,742389518 | | 286,829557 |
| C2orf34 | 0,737633511 | | 284,9920263 |
| TPK1 | 0,731359535 | | 282,5680135 |
| ALDH6A1 | 0,731062569 | | 282,4532776 |
| AGTPBP1 | 0,725606511 | | 280,3452757 |
| CCDC53 | 0,725535697 | | 280,3179158 |
| LRRC28 | 0,722761729 | | 279,2461657 |
| MBNL3 | 0,716905277 | | 276,9834674 |
| CCDC109B | 0,713320794 | | 275,5985668 |
| PUS10 | 0,70905743 | | 273,9513739 |
| CCDC104 | 0,706185858 | | 272,8419137 |
| CASP1 | 0,699081435 | | 270,0970494 |
| SNX14 | 0,689529842 | | 266,4066965 |
| SKAP2 | 0,686417578 | | 265,2042424 |
| NDUFB6 | 0,683568924 | | 264,1036366 |
| EFHA1 | 0,680321463 | | 262,8489478 |
| BCKDHB | 0,679714289 | | 262,6143601 |
| BBS2 | 0,677825758 | | 261,8847077 |
| LMBRD1 | 0,676629332 | | 261,4224565 |
| ITGA6 | 0,660264393 | | 255,0996998 |
| HERC5 | 0,654495807 | | 252,8709496 |
| HADHB | 0,651220796 | | 251,6056164 |
| MCCC2 | 0,650460461 | | 251,3118537 |
| CAT | 0,647218183 | | 250,0591672 |

(continued)

| Gene symbol | Ratio of the transcript level at the 2nd time to the transcript level at the 1st time | Average of the ratio | % of average |
|---|---|---|---|
| ANAPC4 | 0,646761056 | | 249,8825517 |
| PCCB | 0,641145931 | | 247,7130926 |
| PHKB | 0,639806797 | | 247,1957046 |
| ABCB7 | 0,639415266 | | 247,0444329 |
| PGCP | 0,636830107 | | 246,0456309 |
| GPD2 | 0,63484437 | | 245,2784217 |
| TMEM38B | 0,634688463 | | 245,2181856 |
| NFU1 | 0,63202654 | | 244,1897253 |
| OMA1 | 0,631592924 | | 244,0221934 |
| LOC128322 | 0,630915328 | | 243,7603974 |
| NUBPL | 0,627949735 | | 242,6146113 |
| LANCL1 | 0,627743069 | | 242,5347636 |
| HHLA3 | 0,62723119 | | 242,3369941 |
| PIR | 0,625871255 | | 241,8115696 |
| ACAA2 | 0,624054189 | | 241,1095284 |
| CTBS | 0,621758355 | | 240,22251 |
| GSTM4 | 0,618559637 | | 238,9866536 |
| ALG8 | 0,617468882 | | 238,5652294 |
| ACTR10 | 0,614629804 | | 237,4683237 |
| PIGF | 0,612863425 | | 236,7858655 |
| MGST3 | 0,607459796 | | 234,6981198 |
| SCP2 | 0,604745109 | | 233,6492735 |
| HPRT1 | 0,604586436 | | 233,5879689 |
| ACSF2 | 0,603568827 | | 233,1948052 |
| VPS13A | 0,60079506 | | 232,1231332 |
| CTH | 0,598492068 | | 231,2333494 |
| NXT2 | 0,597938464 | | 231,0194589 |
| MGST2 | 0,596121512 | | 230,3174615 |
| C11orf67 | 0,59596274 | | 230,2561181 |
| PCCA | 0,595915054 | | 230,2376943 |
| GLMN | 0,594596168 | | 229,7281295 |
| DHRS1 | 0,594391166 | | 229,6489249 |
| PON2 | 0,594025719 | | 229,5077308 |
| NME7 | 0,593140523 | | 229,1657265 |
| ETFDH | 0,59290737 | | 229,0756456 |
| ALG13 | 0,591519568 | | 228,5394547 |

(continued)

| Gene symbol | Ratio of the transcript level at the 2nd time to the transcript level at the 1st time | Average of the ratio | % of average |
|---|---|---|---|
| DDX60 | 0,590567649 | | 228,1716714 |
| DYNC2LI1 | 0,590400874 | | 228,1072359 |
| VPS8 | 0,586233686 | | 226,4972016 |
| ITFG1 | 0,585791975 | | 226,3265424 |
| CDK5 | 0,584517109 | | 225,8339853 |
| C1orf112 | 0,58415003 | | 225,6921603 |
| IFT52 | 0,579757269 | | 223,9949738 |
| CLYBL | 0,577777391 | | 223,230028 |
| FAM114A2 | 0,575975081 | | 222,533688 |
| NUDT7 | 0,575398988 | | 222,3111085 |
| AKD1 | 0,57519887 | | 222,233791 |
| MAGED2 | 0,575157132 | | 222,217665 |
| HRSP12 | 0,574805797 | | 222,0819235 |
| STX8 | 0,573508131 | | 221,5805571 |
| ACAT1 | 0,569067306 | | 219,8648003 |
| IFT74 | 0,568627867 | | 219,695019 |
| KIFAP3 | 0,567709483 | | 219,3401921 |
| CAPN1 | 0,567537877 | | 219,2738902 |
| COX11 | 0,566354405 | | 218,8166442 |
| GLT8D4 | 0,566035014 | | 218,6932442 |
| HACL1 | 0,56371793 | | 217,7980159 |
| IFT88 | 0,562663344 | | 217,3905661 |
| NDUFB3 | 0,561240987 | | 216,8410243 |
| ANO10 | 0,561096127 | | 216,7850564 |
| ARL6 | 0,560155258 | | 216,4215424 |
| LPCAT3 | 0,559730076 | | 216,2572689 |
| ABCD3 | 0,55747212 | | 215,3848853 |
| COPG2 | 0,557180095 | | 215,2720583 |
| MIPEP | 0,554396343 | | 214,1965281 |
| LEPR | 0,551799358 | | 213,1931572 |
| C2orf76 | 0,549943138 | | 212,4759882 |

Table 2: stable mRNAs resulting from the combined analysis of mouse cell lines (NIH3T3, JAWSII and L929) with the Affymetrix Mouse Gene 1.0 ST micro array: 99 mRNA species of the 26166 mRNA species on the micro array were selected as the "most stable" mRNA species. This corresponds to 0,38% of the mRNA species present on the micro array.

| ene symbol | Ratio of the transcript level at the 2nd time to the transcript level at the 1 st time | Average of the ratio | % of average |
|---|---|---|---|
| Ndufa1 | 1,571557917 | 0,209425963 | 750,4121719 |
| Atp5e | 1,444730129 | | 689,8524465 |
| Gstm5 | 1,436992822 | | 686,1579154 |
| Uqcr1 1 | 1,221605816 | | 583,3115431 |
| Ifi2712a | 1,203811772 | | 574,8149632 |
| Cbr2 | 1,162403907 | | 555,0428852 |
| Anapc13 | 1,153679871 | | 550,8771953 |
| Atp51 | 1,126858713 | | 538,0702074 |
| Tmsb10 | 1,048459674 | | 500,6350022 |
| Nenf | 1,045891853 | | 499,4088786 |
| Ndufa7 | 1,03898238 | | 496,1096349 |
| Atp5k | 1,03623698 | | 494,7987179 |
| 1110008P14Rik | 1,029513775 | | 491,5884162 |
| Cox4i1 | 0,991815573 | | 473,5876865 |
| Cox6a1 | 0,991620272 | | 473,4944312 |
| Ndufs6 | 0,989419978 | | 472,4438002 |
| Sec61b | 0,984420709 | | 470,0566705 |
| Romo1 | 0,981642576 | | 468,7301241 |
| Gnas | 0,969128675 | | 462,7547898 |
| Snrpd2 | 0,962862199 | | 459,7625743 |
| Mgst3 | 0,96060161 | | 458,6831531 |
| Aldh2 | 0,949761281 | | 453,5069425 |
| 2010107E04Rik | 0,933570825 | | 445,776069 |
| Ssr4 | 0,930263069 | | 444,1966294 |
| Myl6 | 0,920572238 | | 439,5692993 |
| Prdx4 | 0,914830854 | | 436,8278128 |
| Ubl5 | 0,902505176 | | 430,9423544 |
| 1110001J03Rik | 0,888041155 | | 424,0358468 |
| Ndufa13 | 0,881735594 | | 421,0249684 |
| Ndufa3 | 0,880861551 | | 420,6076163 |
| Gstp2 | 0,87970004 | | 420,0529997 |
| Tmem160 | 0,878001416 | | 419,2419142 |
| Ergic3 | 0,87481135 | | 417,7186716 |
| Pgcp | 0,870441149 | | 415,6319192 |

(continued)

| ene symbol | Ratio of the transcript level at the 2nd time to the transcript level at the 1 st time | Average of the ratio | % of average |
|---|---|---|---|
| Slpi | 0,868909664 | | 414,9006418 |
| Myeov2 | 0,868175997 | | 414,5503186 |
| Ndufa4 | 0,862009116 | | 411,6056594 |
| Ndufs5 | 0,857586364 | | 409,4938143 |
| Gstm1 | 0,856672742 | | 409,0575637 |
| 1810027010Rik | 0,855929863 | | 408,7028424 |
| Atp50 | 0,848957424 | | 405,3735324 |
| Shfm1 | 0,841951399 | | 402,0281856 |
| Tspo | 0,840567742 | | 401,3674952 |
| S100a6 | 0,840163495 | | 401,1744691 |
| Taldo1 | 0,8400757 | | 401,1325475 |
| Bloc1s1 | 0,838838894 | | 400,541978 |
| Hexa | 0,826597959 | | 394,6969835 |
| Ndufb11 | 0,821601877 | | 392,311376 |
| Map1lc3a | 0,816696063 | | 389,968871 |
| Morn2 | 0,810862522 | | 387,18338 |
| Gpx4 | 0,808459051 | | 386,0357329 |
| Mif | 0,804105552 | | 383,9569558 |
| Cox6b1 | 0,803409855 | | 383,6247633 |
| 2900010J23Rik | 0,802900813 | | 383,3816981 |
| Sec61g | 0,797138268 | | 380,6301077 |
| 2900010M23Rik | 0,793618387 | | 378,9493795 |
| Anapc5 | 0,793224505 | | 378,7613023 |
| Mars2 | 0,787395376 | | 375,9779182 |
| Phpt1 | 0,785668786 | | 375,153479 |
| Ndufb8 | 0,784300334 | | 374,5000492 |
| Pfdn5 | 0,779021933 | | 371,9796349 |
| Arpc3 | 0,77876305 | | 371,8560197 |
| Ndufb7 | 0,774103875 | | 369,6312833 |
| Atp5h | 0,772255845 | | 368,7488573 |
| Mrpl23 | 0,77034041 | | 367,834245 |
| Tomm6 | 0,75481818 | | 360,4224467 |
| Mtch1 | 0,752594518 | | 359,3606576 |
| Pcbd2 | 0,752256847 | | 359,199421 |
| Ecm1 | 0,752254099 | | 359,1981094 |
| Hrsp12 | 0,749135357 | | 357,708923 |

(continued)

| ene symbol | Ratio of the transcript level at the 2nd time to the transcript level at the 1 st time | Average of the ratio | % of average |
|---|---|---|---|
| Mecr | 0,746269148 | | 356,3403207 |
| Uqcrq | 0,734462177 | | 350,7025426 |
| Gstm3 | 0,733839044 | | 350,4049993 |
| Lsm4 | 0,732100345 | | 349,5747779 |
| Park7 | 0,7307842 | | 348,9463242 |
| Usmg5 | 0,724562823 | | 345,9756436 |
| Cox8a | 0,720194618 | | 343,8898445 |
| Ly6c1 | 0,716087602 | | 341,9287619 |
| Cox7b | 0,713519017 | | 340,7022736 |
| Ppib | 0,706106711 | | 337,1629288 |
| Bag1 | 0,70488561 | | 336,5798584 |
| S100a4 | 0,701675201 | | 335,046902 |
| Bcap31 | 0,700846929 | | 334,6514056 |
| Tecr | 0,699592215 | | 334,0522852 |
| Rabac1 | 0,699161282 | | 333,8465165 |
| Robld3 | 0,694068018 | | 331,4145049 |
| Sod1 | 0,691852987 | | 330,356837 |
| Nedd8 | 0,691415017 | | 330,1477083 |
| Higd2a | 0,689498548 | | 329,2326025 |
| Trappc6a | 0,688046277 | | 328,5391491 |
| Ldhb | 0,686084572 | | 327,6024437 |
| Nme2 | 0,685974394 | | 327,5498339 |
| Snrpg | 0,684247073 | | 326,7250454 |
| Ndufa2 | 0,683350661 | | 326,2970129 |
| Serfl | 0,681148053 | | 325,2452768 |
| Oaz1 | 0,681139695 | | 325,2412861 |
| Ybx1 | 0,678927132 | | 324,1847964 |
| Sepp1 | 0,677551422 | | 323,5279009 |
| Gaa | 0,676314425 | | 322,9372402 |

Table 3: stable mRNAs resulting from the analysis of the human cell line HDF (human dermal fibroblasts) with the Affymetrix Human Gene 1.0 ST micro array: 46 mRNA species of the 36079 mRNA species on the micro array were selected as the "most stable" mRNA species. This corresponds to 0,13% of the mRNA species present on the micro array.

| Gene symbol | Ratio of the transcript level at the 2nd time to the transcript level at the 1st time | Average of the ratio | % of average |
|---|---|---|---|
| ABCA6 | 2,062835692 | 0,278262352 | 741,3276273 |

(continued)

| Gene symbol | Ratio of the transcript level at the 2nd time to the transcript level at the 1st time | Average of the ratio | % of average |
|---|---|---|---|
| LY96 | 1,719983635 | | 618,1158256 |
| CROT | 1,422424006 | | 511,1809038 |
| ENPP5 | 1,315849211 | | 472,880791 |
| SERPINB7 | 1,12288882 | | 403,5360196 |
| TCP11L2 | 1,103519648 | | 396,5752607 |
| IRAK1BP1 | 1,05490107 | | 379,1030521 |
| CDKL2 | 1,042002646 | | 374,4677057 |
| GHR | 1,039327135 | | 373,5061992 |
| KIAA1107 | 1,020519239 | | 366,7471477 |
| RPS6KA6 | 1,017695602 | | 365,7324085 |
| CLGN | 1,007943464 | | 362,2277524 |
| TMEM45A | 1,006063873 | | 361,5522781 |
| TBC1D8B | 0,979626826 | | 352,0515148 |
| ACP6 | 0,964241225 | | 346,5223439 |
| RP6-213H19.1 | 0,960702414 | | 345,2505905 |
| C11orf74 | 0,960086216 | | 345,0291458 |
| SNRPN | 0,939315038 | | 337,5645433 |
| GLRB | 0,923441342 | | 331,8599644 |
| HERC6 | 0,919865006 | | 330,5747254 |
| CFH | 0,908835974 | | 326,6111879 |
| GALC | 0,90862766 | | 326,5363257 |
| PDE1A | 0,908445187 | | 326,4707497 |
| GSTM5 | 0,902862912 | | 324,4646303 |
| CADPS2 | 0,89753131 | | 322,5485959 |
| AASS | 0,894768872 | | 321,5558503 |
| TRIM6-TRIM34 | 0,892150571 | | 320,6149031 |
| SEPP1 | 0,891344657 | | 320,3252795 |
| PDE5A | 0,890221551 | | 319,9216656 |
| SATB1 | 0,885139895 | | 318,0954552 |
| CCPG1 | 0,88148167 | | 316,7807873 |
| CNTN1 | 0,87246423 | | 313,5401621 |
| LMBRD2 | 0,871500964 | | 313,1939903 |
| TLR3 | 0,86777981 | | 311,8567077 |
| BCAT1 | 0,864255836 | | 310,5902863 |
| TOM1L1 | 0,86240499 | | 309,925142 |
| SLC35A1 | 0,857201353 | | 308,055095 |

(continued)

| Gene symbol | Ratio of the transcript level at the 2nd time to the transcript level at the 1st time | Average of the ratio | % of average |
|---|---|---|---|
| GLYATL2 | 0,85132258 | | 305,9424223 |
| STAT4 | 0,840572034 | | 302,0789653 |
| GULP1 | 0,839518351 | | 301,7003001 |
| EHHADH | 0,82971807 | | 298,1783427 |
| NBEAL1 | 0,82554089 | | 296,6771768 |
| KIAA1598 | 0,820341324 | | 294,8085928 |
| HFE | 0,815037603 | | 292,9025779 |
| KIAA1324L | 0,808279102 | | 290,4737547 |
| MANSC1 | 0,8033973 | | 288,7193664 |

2. Cloning of 5'- and 3'-UTR elements of stably expressed mRNAs:

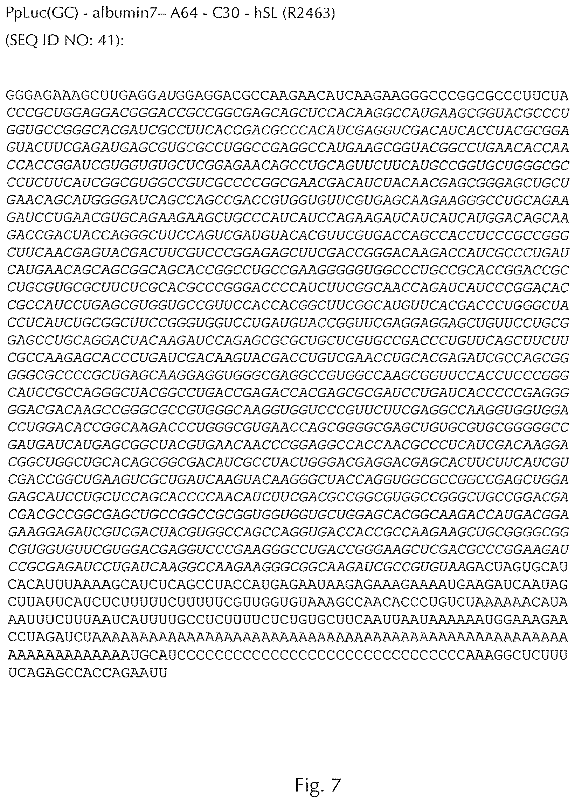

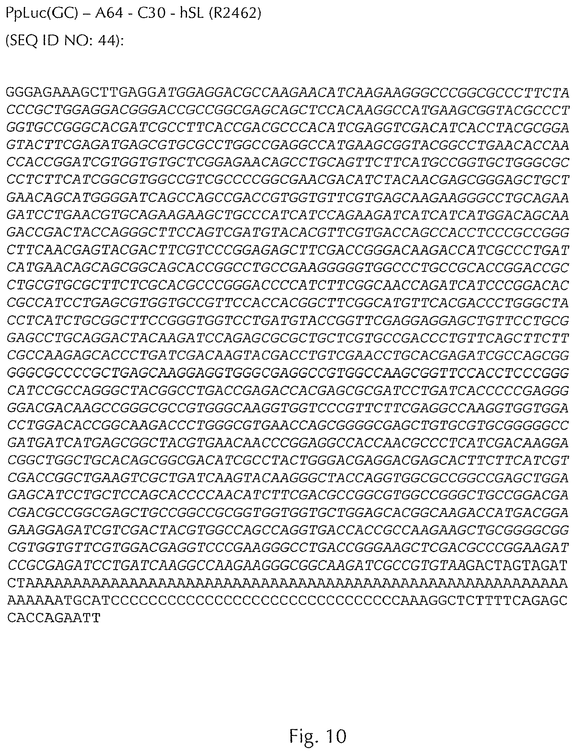

The nucleotide sequence of the 5'- and/or 3'-UTRs of the mRNA species shown in Table 1 - 3 were determined by data base search and amplified by PCR or synthesized by oligo annealing. The resulting PCR fragments were cloned into a vector as described in detail in Example 3 below. 5'-UTR elements were cloned into the vector PpLuc(GC) -albumin7-A64 - C30 - hSL (SEQ ID NO. 41, Fig. 7); and 3'-UTR elements were cloned into the vector 32L4 - PpLuc(GC) - A64 - C30 - hSL (SEQ ID NO. 35, Fig. 1) or into the vector PpLuc(GC) - A64 - C30 - hSL (SEQ ID NO. 44, Fig. 10).

3. Preparation of DNA-templates

A vector for *in vitro* transcription was constructed containing a T7 promoter and a GC-enriched sequence coding for *Photinus pyralis* luciferase (PpLuc(GC)). An A64 poly(A) sequence, followed by C30 and a histone stem-loop sequence, was inserted 3' of PpLuc(GC). The histone stem-loop sequence was followed by a restriction site used for linearization of the vector before in vitro transcription.

To investigate the effect of different 3'-UTR elements on protein expression, a vector as described above was used (control) and this vector was modified to include a 3'-UTR element of interest. Alternatively, a vector was constructed as described above, whereby the 5' untranslated region (5'-UTR) of 32L4 (ribosomal protein Large 32) was inserted 5' of PpLuc(GC). This vector was then modified to include either different 3'-UTR elements or no 3'-UTR (control).

Particularly, the following mRNAs were obtained from these vectors accordingly by *in vitro* transcription (the mRNA sequences are depicted in Figures 1 to 6, Figures 10, 11 and Figures 19 to 21):

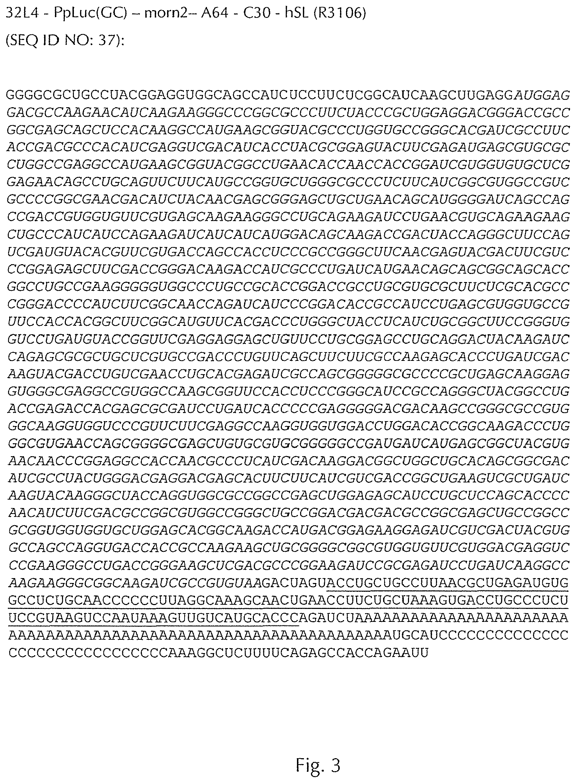

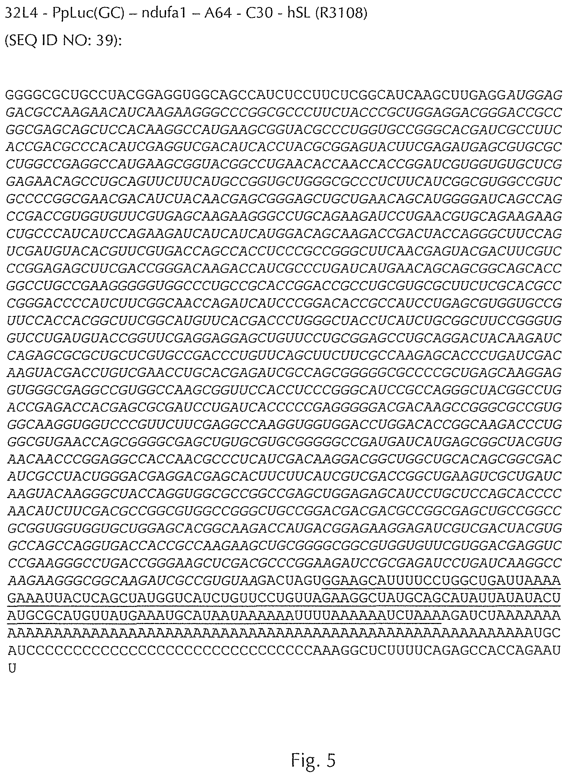

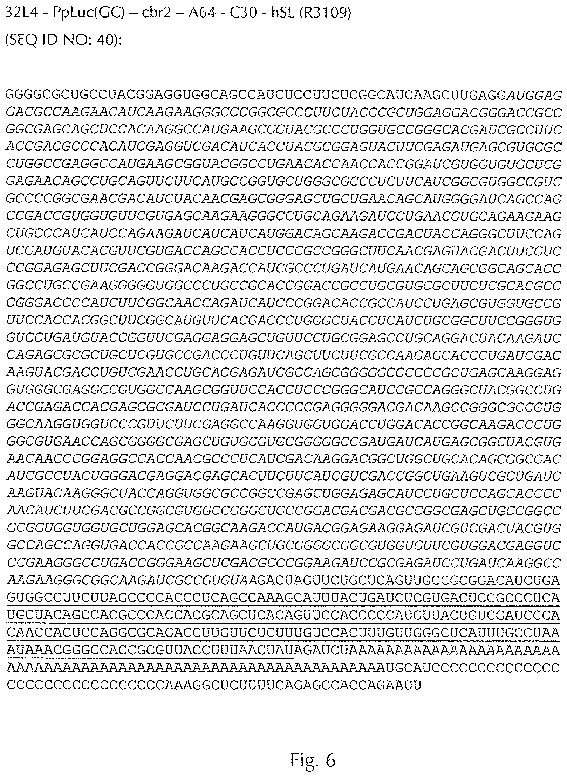

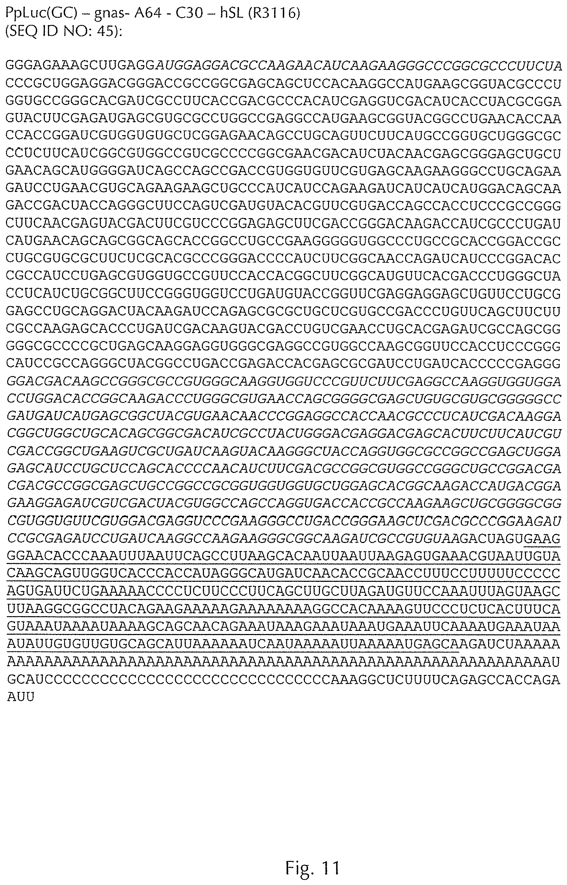

32L4 - PpLuc(GC) - A64 - C30 - hSL (SEQ ID NO. 35, Fig. 1);
32L4 - PpLuc(GC) - gnas - A64 - C30 - hSL (SEQ ID NO. 36, Fig. 2);
32L4 - PpLuc(GC) - morn2 - A64 - C30 - hSL (SEQ ID NO. 37, Fig. 3);
32L4 - PpLuc(GC) - gstm1 - A64 - C30 - hSL (SEQ ID NO. 38, Fig. 4);
32L4 - PpLuc(GC) - ndufa1 - A64 - C30 - hSL (SEQ ID NO. 39, Fig. 5);
32L4 - PpLuc(GC) - cbr2 - A64 - C30 - hSL (SEQ ID NO. 40, Fig. 6);
PpLuc(GC) - A64 - C30 - hSL (SEQ ID NO. 44, Fig. 10);
PpLuc(GC) - gnas - A64 - C30 - hSL (SEQ ID NO. 45, Fig. 11);
32L4 - PpLuc(GC) - Ybx1 (V2)-A64-C30-hSL (SEQ ID NO. 46, Fig. 19);
32L4 - PpLuc(GC) - Ndufb8-A64-C30-hSL (SEQ ID NO. 47, Fig. 20); and
32L4 - PpLuc(GC) - Cntn1 -004(V2)-A64-C30-hSL (SEQ ID NO. 48, Fig. 21).

An alternative sequence for the construct 32L4 - PpLuc(GC) - A64 - C30 - hSL is shown in Fig. 25 (SEQ ID NO. 383). However, SEQ ID NO. 35, Fig. 1 was used in the Examples as described herein and is, thus, preferred for the construct 32L4 - PpLuc(GC) - A64 - C30 - hSL.

To investigate the effect of different 5'-UTR elements on protein expression, a vector was constructed as described above, whereby the 3' untranslated region (3'-UTR) of albumin7 (3'-UTR of human albumin with three single

point mutations introduced to remove a T7 termination signal as well as a HindIII and XbaI restriction site) was inserted 3' of PpLuc(GC). This vector was modified to include either different 5'-UTR elements or no 5'-UTR (control).

Particularly, the following mRNAs were obtained from these vectors accordingly by *in vitro* transcription (the mRNA sequences are depicted in Figures 7 to 9):

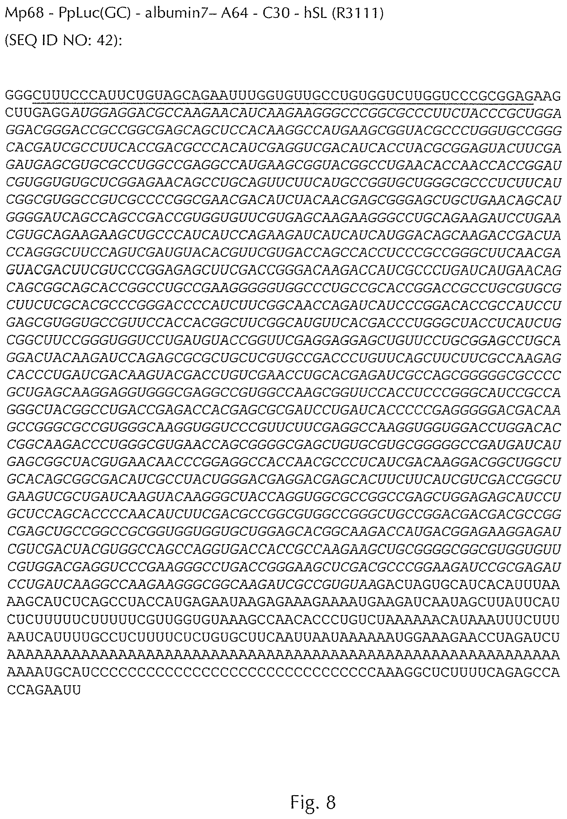

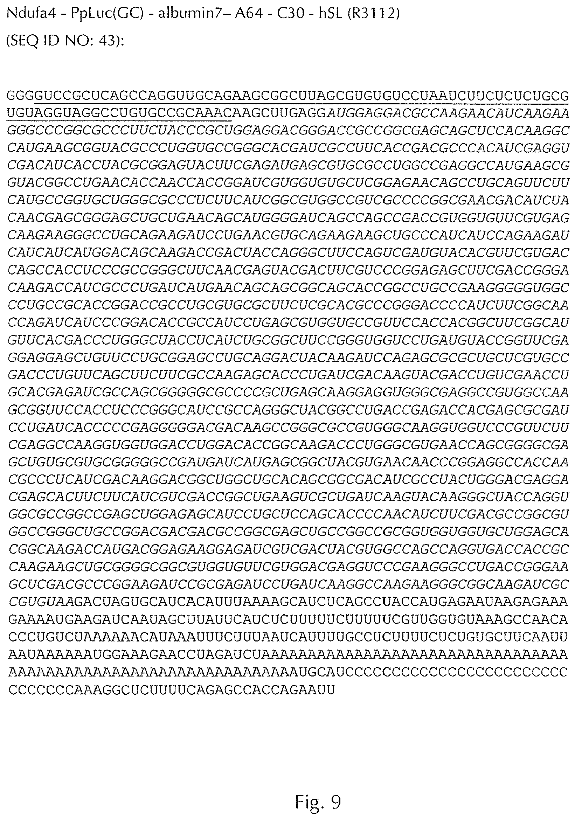

PpLuc(GC) - albumin7 - A64 - C30 - hSL (SEQ ID NO. 41, Fig. 7);
Mp68 - PpLuc(GC) - albumin7 - A64 - C30 - hSL (SEQ ID NO. 42, Fig. 8); and
Ndufa4 - PpLuc(GC) - albumin7 - A64 - C30 - hSL (SEQ ID NO. 43, Fig. 9);

4. *In vitro* transcription

The DNA templates according to Example 2 and 3 were linearized and transcribed *in vitro* using T7-RNA polymerase. The DNA templates were then digested by DNase-treatment. mRNA transcripts contained a 5'-CAP structure obtained by adding an excess of N7-Methyl-Guanosine-5'-Triphosphate-5'-Guanosine to the transcription reaction. mRNA thus obtained was purified and resuspended in water.

5. Luciferase expression by mRNA lipofection

Human dermal fibroblasts (HDF) and HeLa cells were seeded in 96 well plates at a density of $1x10^4$ cells per well. The following day, cells were washed in Opti-MEM and then transfected with 12.5 ng per well of Lipofectamine2000-complexed PpLuc-encoding mRNA in Opti-MEM. Untransfected cells served as control. mRNA coding for *Renilla reniformis* luciferase (RrLuc) was transfected together with PpLuc mRNA to control for transfection efficiency (1 ng of RrLuc mRNA per well). 90 minutes after start of transfection, Opti-MEM was exchanged for medium. 6, 24, 48, 72 hours after transfection, medium was aspirated and cells were lysed in 100 μl of Passive Lysis buffer (Promega). Lysates were stored at -80°C until luciferase activity was measured.

6. Luciferase measurement

Luciferase activity was measured as relative light units (RLU) in a Hidex Chameleon plate reader. The activities of Ppluc and Rrluc are measured sequentially from a single sample in a dual luciferase assay. The PpLuc activity was measured first at 2 seconds measuring time using 20 μl of lysate and 50 μl of Beetle juice (pjk GmbH). After 1500ms delay RrLuc activity is measured with 50 μl Renilla juice (pjk GmbH).

7. Results

a. Protein expression from mRNA containing 3'-UTR elements according to the invention is increased and/or prolonged.
To investigate the effect of various 3'-UTR elements on protein expression from mRNA, mRNAs containing different 3'-UTR elements were compared to an mRNA lacking a 3'-UTR.
Human HeLa and HDF cells were transfected with Luciferase encoding mRNAs and Luciferase levels (in RLU) were measured 6, 24, 48, and 72 hours after transfection. The PpLuc signal was corrected for transfection efficiency by the signal of cotransfected RrLuc. Normalized PpLuc levels at 24h were set to 100% and relative expression to 24h is calculated (see following Table 4 and Figures 12 (HeLa cells) and 13 (HDF cells)).

Table 4:

| **mRNA** | **HeLa** | | | **HDF** | | |
|---|---|---|---|---|---|---|
| | **24h** | **48h** | **72h** | **24h** | **48h** | **72h** |
| 32L4-PpLuc(GC)-A64-C30-hSL | 100 | 12,3 | 2,7 | 100 | 34,8 | 10,9 |
| 32L4-PpLuc(GC)-gnas-A64-C30-hsL | 100 | 50,5 | 30,9 | 100 | 79,8 | 27,8 |
| 32L4-PpLuc(GC)-mom2-A64-C30-hSL | 100 | 32,9 | 10,5 | 100 | 44,5 | 14,6 |
| 32L4-PpLuc(GC)-gstm 1-A64-C3 0-hSL | 100 | 24,8 | 7,6 | 100 | 46,5 | 21,4 |
| 32 L4-PpLuc(GC)-ndufa 1-A64-C30-hSL | 100 | 29,4 | 10,6 | 100 | 41,9 | 13,9 |
| 32L4-PpLuc(GC)-cbr2-A64-C30-hSL | 100 | 21,9 | 4,9 | 100 | 60,0 | 23,2 |

Table 4 shows relative PpLuc expression normalized to RrLuc (mean values of three independent experiments are given). Luciferase was expressed from mRNA lacking a 3'-UTR. However, the inventive 3'-UTR elements gnas, morn2, gstm1, ndufa and cbr2 significantly prolonged luciferase expression.

b. Protein expression from mRNA containing 5'-UTR elements according to the invention is increased and/or prolonged.

To investigate the effect of various 5'-UTR elements on protein expression from mRNA, mRNAs containing different 5'-UTRs were compared to an mRNA lacking a 5'-UTR.

Human HeLa and HDF cells were transfected with Luciferase encoding mRNAs and Luciferase levels were measured 6, 24, 48, and 72 hours after transfection. The PpLuc signal was corrected for transfection efficiency by the signal of cotransfected RrLuc. Total protein expression from 0 to 72 hours was calculated as the area under the curve (AUC). The levels of the control construct without 5' UTR was set to 1 (see following Table 5 and Figure 14 (HeLa cells) and 15 (HDF cells)).

Table 5:

| **mRNA** | **AUC HeLa** | **AUC HDF** |
|---|---|---|
| PpLuc(GC)-albumin7-A64-C30-hSL | 1,00 | 1,07 |
| Mp68-PpLuc(GC)-albumin7-A64-C30-hSL | 1,79 | 3,03 |
| Ndufa4-PLuc(GC-albumin7-A64-C30-hSL | 1,92 | 2,83 |

Table 5 shows the total PpLuc expression normalized to RrLuc (mean values of three independent experiments are given). Luciferase was expressed from mRNA lacking a 5'-UTR. However, the inventive 5'-UTR elements mp68 and ndufa4 significantly increased luciferase expression.

c. Protein expression from mRNA containing 3'-UTR elements according to the invention is prolonged.

To investigate the effect of various 3'UTRs on protein expression from mRNA, mRNAs containing different 3'UTRs were compared to an mRNA lacking a 3'UTR.

Human HeLa and HDF cells were transfected with Luciferase encoding mRNAs and Luciferase levels (in RLU) were measured 24, 48, and 72 hours after transfection. The PpLuc signal was corrected for transfection efficiency by the signal of cotransfected RrLuc. Normalized PpLuc levels at 24h were set to 100% and relative expression to 24h is calculated (see following Table 6 and Figures 16 (HeLa cells) and 17 (HDF cells)).

Table 6:

| mRNA | HeLa | | | HDF | | |
|---|---|---|---|---|---|---|
| | 24h | 48h | 72h | 24h | 48h | 72h |
| PpLuc(GC)-gnas-A64-C30-hSL | 100 | 61,1 | 30,3 | 100 | 53,6 | 34,2 |
| PpLuc(GC)-A64-C30-hSL | 100 | 17,1 | 2,7 | 100 | 29,0 | 12,4 |

Table 6 shows relative PpLuc expression normalized to RrLuc (mean values of three independent experiments are given).

d. Protein expression from mRNA containing 3'-UTR elements according to the invention is prolonged.

To investigate the effect of various 3'UTRs on protein expression from mRNA, mRNAs containing different 3'UTRs were compared to an mRNA lacking a 3'UTR.

Human HeLa and HDF cells were transfected with Luciferase encoding mRNAs and Luciferase levels were measured 6, 24, 48, and 72 hours after transfection. The PpLuc signal was corrected for transfection efficiency by the signal of cotransfected RrLuc. Total protein expression from 0 to 72 hours was calculated as the area under the curve (AUC). The levels of the control construct without 5' UTR was set to 1 (see following Table 7 and Figure 18 (HDF cells) and 17 (HeLa cells)).

Human HeLa and HDF cells were transfected with Luciferase encoding mRNAs and Luciferase levels (in RLU) were measured 24, 48, and 72 hours after transfection. The PpLuc signal was corrected for transfection efficiency by the signal of cotransfected RrLuc. Normalized PpLuc levels at 24h were set to 100% and relative expression to 24h is calculated (see following Table 7 and Figure 18 (HDF cells)).

Table 7:

| mRNA | HDF | | |
|---|---|---|---|
| | 24h | 48h | 72h |
| 32L4-PpLuc(GC)-Ybx1-001 (V2)-A64-C30-hSL | 100 | 57,0 | 28,5 |
| 32L4-PpLuc(GC)-Ndufb8-A64-C30-hSL | 100 | 65,4 | 37,6 |
| 32L4-PpLuc(GC)-Cntn1004(V2)-A64-C30-hSL | 100 | 71,0 | 47,7 |
| 32L4-PpLuc(GC)-A64-C30-hSL | 100 | 45,2 | 21,87 |

Table 7 shows relative PpLuc expression normalized to RrLuc (mean values of three independent experiments are given).

8. Effect of further 3'UTRs on protein expression

To further investigate the effect of various 3'UTRs on protein expression from mRNA, new mRNA constructs were prepared and those mRNAs containing different 3'UTRs were compared to an mRNA lacking a 3'UTR.

To this end, selected 3'-UTR elements (gnas, morn2, ndufa1 and NDUFA1) were cloned into the vector PpLuc(GC) - A64 - C30 - hSL (SEQ ID NO. 44, Fig. 10), which was constructed containing a T7 promoter and a GC-enriched sequence coding for *Photinus pyralis* luciferase (PpLuc(GC)). An A64 poly(A) sequence, followed by C30 and a histone stem-loop sequence, was inserted 3' of PpLuc(GC). The histone stem-loop sequence was followed by a restriction site used for linearization of the vector before in vitro transcription.

In particular, the following mRNAs were obtained from such vectors by *in vitro* transcription (the mRNA sequences are depicted in Figures 11 and 26 to 28:

PpLuc(GC) - gnas - A64 - C30 - hSL (SEQ ID NO. 45, Fig. 11);
PpLuc(GC) - morn2 - A64 - C30 - hSL (SEQ ID NO. 384, Fig. 26);
PpLuc(GC) - ndufa1 - A64 - C30 - hSL (SEQ ID NO. 385, Fig. 27); and
PpLuc(GC) - NDUFA1 - A64 - C30 - hSL (SEQ ID NO. 386, Fig. 28).

Human HeLa cells were transfected with Luciferase encoding mRNAs and Luciferase levels were measured 24, 48, and 72 hours after transfection. The PpLuc signal was corrected for transfection efficiency by the signal of cotransfected RrLuc (see following Table 8 and Fig. 22).

Table 8: relative PpLuc expression normalized to RrLuc (mean values of 3 independent experiments are given).

| mRNA | HeLa (expression in %) | | |
|---|---|---|---|
| | 24h | 48h | 72h |
| PpLuc(GC)-gnas-A64-C30-hSL | 100 | 77,9 | 36,7 |
| PpLuc(GC)-morn2-A64-C30-hSL | 100 | 53,8 | 17,2 |
| PpLuc(GC)-ndufa1-A64-C30-hSL | 100 | 55,2 | 17,9 |
| PpLuc(GC)-NDUFA1-A64-C30-hSL | 100 | 66,9 | 29,4 |
| PpLuc(GC)-A64-C30-hSL | 100 | 41,5 | 9,6 |

These data and the data shown in Fig. 22 show that protein expression from mRNA containing 3'-UTR elements according to the invention is prolonged.

9. Effect of further 5'UTRs on protein expression

To further investigate the effect of various 5'UTRs on protein expression from mRNA, new mRNA constructs were prepared and those mRNAs containing different 5'UTRs were compared to an mRNA lacking a 5'UTR.

To this end, selected 5'-UTR elements (mp68 and ndufa4) were cloned into the vector PpLuc(GC) - A64 - C30 - hSL (SEQ ID NO. 44, Fig. 10), which was constructed containing a T7 promoter and a GC-enriched sequence coding for *Photinus pyralis* luciferase (PpLuc(GC)). An A64 poly(A) sequence, followed by C30 and a histone stem-loop sequence, was inserted 3' of PpLuc(GC). The histone stem-loop sequence was followed by a restriction site used for linearization of the vector before in vitro transcription.

In particular, the following mRNAs were obtained from such vectors by *in vitro* transcription (the mRNA sequences are depicted in Figures 29 and 30:

Mp68 - PpLuc(GC) - A64 - C30 - hSL (SEQ ID NO. 387, Fig. 29); and
Ndufa4 - PpLuc(GC) -A64 - C30 - hSL (SEQ ID NO. 388, Fig. 30).

Human HDF and HeLa cells were transfected with Luciferase encoding mRNAs and Luciferase levels were measured 24, 48, and 72 hours after transfection. The PpLuc signal was corrected for transfection efficiency by the signal of cotransfected RrLuc. Total protein expression (area under the curve) was calculated. The levels of the control construct without 5' UTR was set to 1 (see following Table 9 and Figures 23 and 24).

Table 9: total PpLuc expression normalized to RrLuc (mean RLU values are given).

| mRNA | AUC HDF | AUC HeLa |
|---|---|---|
| PpLuc(GC) -A64-C30-hSL | 1,0 | 1,0 |
| Mp68-PpLuc(GC)-A64-C30-hSL | 3,9 | 2,3 |
| Ndufa4-PpLuc(GC)-A64-C30-hSL | 4,0 | 2,0 |

These data and the data shown in Figures 23 and 24 show that protein expression from mRNA containing 5'-UTR elements according to the invention is increased.

Items

1. An artificial nucleic acid molecule comprising

a. at least one open reading frame (ORF); and

b. at least one 3'-untranslated region element (3'-UTR element) and/or at least one 5'-untranslated region element (5'-UTR element), wherein the at least one 3'-UTR element and/or the at least one 5'-UTR element prolongs and/or increases protein production from said artificial nucleic acid molecule and wherein the at least one 3'-UTR element and/or the at least one 5'-UTR element is derived from a stable mRNA.

2. The artificial nucleic acid molecule according to item 1, wherein the open reading frame is derived from a gene, which is distinct from a gene from which the at least one 3'-UTR element and/or the at least one 5'-UTR element is derived.

3. The artificial nucleic acid molecule according to item 1 or 2 comprising at least one 3'-UTR element and at least one 5'-UTR element.

4. The artificial nucleic acid molecule according to item 3, wherein each of the at least one open reading frame, the at least one 3'-UTR element and the at least one 5'-UTR element are heterologous to each other.

5. The artificial nucleic acid molecule according to any of items 1 to 4, wherein the stable mRNA from which the at least one 3'-UTR element and/or the at least one 5'-UTR element is derived is characterized by an mRNA decay wherein the ratio of the amount of said mRNA at a second point in time to the amount of said mRNA at a first point in time is at least 0.5 (50%), at least 0.6 (60%), at least 0.7 (70%), at least 0.75 (75%), at least 0.8 (80%), at least 0.85 (85%), at least 0.9 (90%), or at least 0.95 (95%).

6. The artificial nucleic acid molecule according to any of items 1 to 5, wherein the artificial nucleic acid molecule does not comprise a 3'-UTR and/or a 5'-UTR of ribosomal protein S6, of RPL36AL, of rps16 or of ribosomal protein L9 and wherein the open reading frame of the artificial nucleic acid molecule does not code for a GFP protein.

7. The artificial nucleic acid molecule according to item 6, wherein the open reading frame of the artificial nucleic acid molecule does not code for a reporter protein.

8. The artificial nucleic acid molecule according to any one of items 1 to 7, wherein the at least one 3'-UTR element and/or the at least one 5'-UTR element comprises or consists of a nucleic acid sequence which is derived from the 3'-UTR and/or the 5'-UTR of a eukaryotic protein coding gene, preferably from the 3'-UTR and/or the 5'-UTR of a vertebrate protein coding gene, more preferably from the 3'-UTR and/or the 5'-UTR of a mammalian protein coding gene, even more preferably from the 3'-UTR and/or the 5'-UTR of a primate protein coding gene.

9. The artificial nucleic acid molecule according to item 8, wherein the at least one 3'-UTR element and/or the at least one 5'-UTR element comprises or consists of a nucleic acid sequence which is derived from the 3'-UTR and/or the 5'-UTR of a human or murine protein coding gene.

10. The artificial nucleic acid molecule according to item 8 or 9, wherein:

(i) the nucleic acid molecule comprises at least one 3'-UTR element and at least one 5'-UTR element;
(ii) the at least one 3'-UTR element, the at least one 5'-UTR element and the at least one open reading frame are all heterologous to each other;
(iii) the at least one 3' UTR element is derived from a gene selected from the group consisting of: housekeeping genes, genes coding for a membrane protein, genes involved in cellular metabolism, genes involved in transcription, translation and replication processes, genes involved in protein modification and genes involved in cell division; and
(iv) the 3'UTR is not derived from a gene coding for a ribosomal protein or from the Fig4 gene.

11. The artificial nucleic acid molecule according to any of items 8 to 10, wherein:

(i) the nucleic acid molecule comprises at least one 3'-UTR element and at least one 5'-UTR element;
(ii) the at least one 3'-UTR element, the at least one 5'-UTR element and the at least one open reading frame are all heterologous to each other;
(iii) the at least one 5'-UTR element is derived from a gene selected from the group consisting of: housekeeping genes, genes coding for a membrane protein, genes involved in cellular metabolism, genes involved in transcription, translation and replication processes, genes involved in protein modification and genes involved in cell division;
(iv) the 5'-UTR is preferably not a 5' TOP UTR; and
(v) the 3'-UTR is preferably not derived from a gene coding for a ribosomal protein or for albumin or from the Fig4 gene.

12. The artificial nucleic acid molecule according to item 10 or 11, wherein:

(i) the nucleic acid molecule comprises at least one 3'-UTR element and at least one 5'-UTR element;
(ii) the at least one 3'-UTR element, the at least one 5'-UTR element and the at least one open reading frame are all heterologous to each other;
(iii) the at least one 3' UTR element is derived from a human or a murine gene selected from the group consisting of: housekeeping genes, genes coding for a membrane protein, genes involved in cellular metabolism, genes involved in transcription, translation and replication processes, genes involved in protein modification and genes involved in cell division;
(iv) the 3'UTR is not derived from a gene coding for a ribosomal protein or for albumin or from the Fig4 gene;
(v) the at least one 5'-UTR element is derived from a human or a murine gene selected from the group consisting of: housekeeping genes, genes coding for a membrane protein, genes involved in cellular metabolism, genes involved in transcription, translation and replication processes, genes involved in protein modification and genes involved in cell division; and
(vi) the 5'-UTR is not a 5' TOP UTR.

13. The artificial nucleic acid molecule according to any of items 10 to 12, wherein the 3'-UTR and the 5'-UTR are derived from a human or a murine housekeeping gene.

14. The artificial nucleic acid molecule according to item 12, wherein the 3'-UTR and the 5'-UTR are derived from a human or a murine gene selected from the group consisting of: genes coding for a membrane protein, genes

involved in cellular metabolism, genes involved in transcription, translation and replication processes, genes involved in protein modification and genes involved in cell division and wherein the 3'-UTR and the 5'-UTR are selected from distinct gene classes.

15. The artificial nucleic acid molecule according to any of items 1 to 14, wherein the at least one 3'-UTR element and/or the at least one 5'-UTR element prolongs protein production from said artificial nucleic acid molecule at least 1.2 fold, preferably at least 1.5 fold, more preferably at least 2 fold, even more preferably at least 2.5 fold, compared to the protein production from a reference nucleic acid molecule lacking a 3'-UTR and/or the at least one 5'-UTR, respectively, and/or wherein the at least one 3'-UTR element and/or the at least one 5'-UTR element increases protein production from said artificial nucleic acid molecule at least 1.5 fold, preferably at least 2 fold, more preferably at least 2.5 fold, compared to the protein production from a reference nucleic acid molecule lacking a 3'-UTR and/or the at least one 5'-UTR, respectively.

16. The artificial nucleic acid molecule according to any one of items 1 to 15, wherein the at least one 3'-UTR element and/or the at least one 5'-UTR element comprises or consists of a nucleic acid sequence which is derived from the 3'-UTR and/or the 5'-UTR of a transcript of a gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, Ndufa1, Atp5e, Gstm5, Uqcr11, Ifi27l2a, Cbr2, Anapc13, Atp5l, Tmsb10, Nenf, Ndufa7, Atp5k, 1110008P14Rik, Cox4i1, Cox6a1, Ndufs6, Sec61b, Romo1, Gnas, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufa4, Ndufs5, Gstm1, 1810027O10Rik, Atp5o, Shfm1, Tspo, S100a6, Taldo1, Bloc1s1, Hexa, Ndufb11, Map1lc3a, Morn2, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1; preferably from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), and CNTN1 (contactin 1).

17. The artificial nucleic acid molecule according to item 16, wherein the at least one 3'-UTR element and/or the at least one 5'-UTR element comprises a nucleic acid sequence which is derived from the 3'-UTR and/or the 5'-UTR of a transcript of a human gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), CNTN1 (contactin 1), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3,, PIR, ACAA2, CTBS, GSTM4, ALG8, ACTR10, PIGF, MGST3, SCP2, HPRT1, ACSF2, VPS13A, CTH, NXT2, MGST2, C11orf67, PCCA,

GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11 L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, LMBRD2, TLR3, BCAT1, TOM1L1, SLC35A1, GLYATL2, STAT4, GULP1, EHHADH, NBEAL1, KIAA1598, HFE, KIAA1324L, and MANSC1.

18. The artificial nucleic acid molecule according to item 16, wherein the at least one 3'-UTR element and/or the at least one 5'-UTR element comprises a nucleic acid sequence which is derived from the 3'-UTR and/or the 5'-UTR of a transcript of a murine gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), CNTN1 (contactin 1), Atp5e, Gstm5, Uqcr11, Ifi27l2a, Anapc13, Atp5l, Tmsb10, Nenf, Ndufa7, Atp5k, 1110008P14Rik, Cox4i1, Cox6a1, Ndufs6, Sec61b, Romo1, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufs5, 1810027010Rik, Atp5o, Shfm1, Tspo, S100a6, Taldo1, Bloc1s1, Hexa, Ndufb11, Map1lc3a, Gpx4, Mif, Cox6b1, RIKEN cDNA2900010J23 (Swi5), Sec61g, 2900010M23Rik, Anapc5, Mars2, Phpt1, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Sepp1, and Gaa.

19. The artificial nucleic acid molecule according to any one of items 16 to 18, wherein the at least one 3'-UTR element comprises a nucleic acid sequence which is derived from the 3'-UTR of a transcript of a gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), SLC38A6, DECR1, PIGK, FAM175A, PHYH, TBC1D19, PIGB, ALG6, CRYZ, BRP44L, ACADSB, TMEM14A, GRAMD1C, C11orf80, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, HADHB, ANAPC4, PCCB, ABCB7, PGCP, NFU1, OMA1, HHLA3, ACAA2, GSTM4, ALG8, Ndufa1, Atp5e, Gstm5, Uqcr11, Ifi27l2a, Cbr2, Atp5l, Tmsb10, Nenf, Atp5k, 1110008P14Rik, Cox4i1, Cox6a1, Ndufs6, Sec61b, Romo1, Gnas, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufa4, Ndufs5, Gstm1, 1810027010Rik, Atp5o, Shfm1, Tspo, S100a6, Taldol, Bloc1s1, Ndufb11, Map1lc3a, Morn2, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1; preferably, the at least one 3'-UTR element comprises or consists of a nucleic acid sequence which is derived from the 3'-UTR of a transcript of a gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), Ybx1 (Y-Box binding protein 1), Ndufb8 (NADH dehydrogenase (ubiquinone) 1 beta subcomplex 8), and CNTN1 (contactin 1).

20. The artificial nucleic acid molecule according to item 19, wherein the at least one 3'-UTR element comprises a nucleic acid sequence which is derived from the 3'-UTR of a transcript of a human gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), SLC38A6, DECR1, PIGK, FAM175A, PHYH, TBC1D19, PIGB, ALG6, CRYZ,

BRP44L, ACADSB, TMEM14A, GRAMD1C, C11orf80, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, HADHB, ANAPC4, PCCB, ABCB7, PGCP, NFU1, OMA1, HHLA3, ACAA2, GSTM4, ALG8, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1; preferably, the at least one 3'-UTR element comprises or consists of a nucleic acid sequence which is derived from the 3'-UTR of a transcript of a human gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), Ybx1 (Y-Box binding protein 1), Ndufb8 (NADH dehydrogenase (ubiquinone) 1 beta subcomplex 8), and CNTN1 (contactin 1).

21. The artificial nucleic acid molecule according to item 19, wherein the at least one 3'-UTR element comprises a nucleic acid sequence which is derived from the 3'-UTR of a transcript of a murine gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), Ybx1 (Y-Box binding protein 1), Ndufb8 (NADH dehydrogenase (ubiquinone) 1 beta subcomplex 8), and CNTN1 (contactin 1), Ndufa1, Atp5e, Gstm5, Uqcr11, Ifi27l2a, Cbr2, Atp5l, Tmsb10, Nenf, Atp5k, 1110008P14Rik, Cox4i1, Cox6a1, Ndufs6, Sec61b, Romo1, Gnas, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufa4, Ndufs5, Gstm1, 1810027O10Rik, Atp5o, Shfm1, Tspo, S100a6, Taldo1, Bloc1s1, Ndufb11, Map1lc3a, Morn2, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, and Gaa; preferably, the at least one 3'-UTR element comprises or consists of a nucleic acid sequence which is derived from the 3'-UTR of a transcript of a murine gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), Ybx1 (Y-Box binding protein 1), Ndufb8 (NADH dehydrogenase (ubiquinone) 1 beta subcomplex 8), and CNTN1 (contactin 1).

22. The artificial nucleic acid molecule according to any one of items 16 to 18, wherein the at least one 5'-UTR element comprises a nucleic acid sequence which is derived from the 5'-UTR of a transcript of a gene selected from the group consisting of MP68 (RIKEN cDNA 2010107E04 gene), NDUFA4 (NADH dehydrogenase (ubiquinone) 1 alpha subcomplex 4), LTA4H, DECR1, PIGK, TBC1D19, BRP44L, ACADSB, SUPT3H, TMEM14A, C9orf46, ANXA4, IFI6, C2orf34, ALDH6A1, CCDC53, CCDC104, CASP1, NDUFB6, BCKDHB, BBS2, HERC5, FAM175A, NT5DC1, RAB7A, AGA, TPK1, MBNL3, MCCC2, CAT, ANAPC4, PHKB, ABCB7, GPD2, TMEM38B, NFU1, LOC128322/NUTF2, NUBPL, LANCL1, PIR, CTBS, GSTM4, Ndufa1, Atp5e, Gstm5, Cbr2, Anapc13, Ndufa7, Atp5k, 1110008P14Rik, Cox4i1, Ndufs6, Sec61b, Snrpd2, Mgst3, Prdx4; Pgcp; Myeov2; Ndufa4; Ndufs5; Gstm1; Atp5o; Tspo; Taldo1; Btods1; and Hexa; preferably, the at least one 5'-UTR element comprises or consists of a nucleic acid sequence which is derived from the 5'-UTR of a transcript of MP68 (RIKEN cDNA 2010107E04 gene) or NDUFA4 (NADH dehydrogenase (ubiquinone) 1 alpha subcomplex 4).

23. The artificial nucleic acid molecule according to item 22, wherein the at least one 5'-UTR element comprises a nucleic acid sequence which is derived from the 5'-UTR of a transcript of a human gene selected from the group consisting of MP68 (RIKEN cDNA 2010107E04 gene), NDUFA4 (NADH dehydrogenase (ubiquinone) 1 alpha subcomplex 4), LTA4H, DECR1, PIGK, TBC1D19, BRP44L, ACADSB, SUPT3H, TMEM14A, C9orf46, ANXA4, IFI6, C2orf34, ALDH6A1, CCDC53, CCDC104, CASP1, NDUFB6, BCKDHB, BBS2, HERC5, FAM175A, NT5DC1, RAB7A, AGA, TPK1, MBNL3, MCCC2, CAT, ANAPC4, PHKB, ABCB7, GPD2, TMEM38B, NFU1, LOC128322/NUTF2, NUBPL, LANCL1, PIR, CTBS, and GSTM4; preferably, the at least one 5'-UTR element comprises or consists of a nucleic acid sequence which is derived from the 5'-UTR of a human transcript of MP68 (RIKEN cDNA 2010107E04 gene) or NDUFA4 (NADH dehydrogenase (ubiquinone) 1 alpha subcomplex 4).

24. The artificial nucleic acid molecule according to item 22, wherein the at least one 5'-UTR element comprises a nucleic acid sequence which is derived from the 5'-UTR of a transcript of a murine gene selected from the group consisting of MP68 (RIKEN cDNA 2010107E04 gene), NDUFA4 (NADH dehydrogenase (ubiquinone) 1 alpha subcomplex 4), Ndufa1, Atp5e, Gstm5, Cbr2, Anapc13, Ndufa7, Atp5k, 1110008P14Rik, Cox4i1, Ndufs6, Sec61b, Snrpd2, Mgst3, Prdx4; Pgcp; Myeov2; Ndufa4; Ndufs5; Gstm1; Atp5o; Tspo; Taldo1; Bloc1s1; and Hexa; preferably, the at least one 5'-UTR element comprises or consists of a nucleic acid sequence which is derived from the 5'-UTR of a murine transcript of MP68 (RIKEN cDNA 2010107E04 gene) or NDUFA4 (NADH dehydrogenase (ubiquinone) 1 alpha subcomplex 4).

25. The artificial nucleic acid molecule according to any one of items 1 - 21, wherein the at least one 3'-UTR element comprises or consists of a nucleic acid sequence which has an identity of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 1 to 24 and SEQ ID NOs: 49 to 318 or wherein the at least one 3'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to fragment of a nucleic acid sequence selected from the group consisting of SEQ ID NOs: 1 to 24 and SEQ ID NOs: 49 to 318.

26. The artificial nucleic acid molecule according to any one of items 1 - 18 and 22 to 24, wherein the at least one 5'-UTR element comprises or consists of a nucleic acid sequence which has an identity of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 25 to 30 and SEQ ID NOs: 319 to 382 or wherein the at least one 5'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 25 to 30 and SEQ ID NOs: 319 to 382.

27. The artificial nucleic acid molecule according to item 25 or 26, wherein the fragment exhibits a length of between 3 and about 500 nucleotides, preferably of between 5 and about 150 nucleotides, more preferably of between 10 and 100 nucleotides, even more preferably of between 15 and 90, most preferably of between 20 and 70.

28. The artificial nucleic acid molecule according to any one of items 1 - 27, wherein the at least one 3'-UTR element and/or the at least one 5'-UTR element exhibits a length of between 3 and about 500 nucleotides, preferably of between 5 and about 150 nucleotides, more preferably of between 10 and 100 nucleotides, even more preferably of between 15 and 90, most preferably of between 20 and 70.

29. The artificial nucleic acid molecule according to any one of items 1 - 28 further comprising

c. a poly(A) sequence and/or a polyadenylation signal.

30. The artificial nucleic acid molecule according to item 29, wherein the poly(A) sequence or the polyadenylation signal is located 3' of the 3'-UTR element.

31. The artificial nucleic acid molecule according to item 29 or 30, wherein the polyadenylation signal comprises the consensus sequence NN(U/T)ANA, with N = A or U, preferably AA(U/T)AAA or A(U/T)(U/T)AAA.

32. The artificial nucleic acid molecule according to any one of items 29 - 31, wherein the polyadenylation signal, preferably the consensus sequence NNUANA, is located less than about 50 nucleotides downstream of the 3'-end of the 3'-UTR element.

33. The artificial nucleic acid molecule according to any one of items 29 - 32, wherein the poly(A) sequence has a length of about 20 to about 300 adenine nucleotides, preferably of about 40 to about 200 adenine nucleotides, more preferably of about 50 to about 100 adenine nucleotides, even more preferably of about 60 to about 70 adenine nucleotides.

34. The artificial nucleic acid molecule according to any one of items 1 - 33, further comprising a 5'-cap structure, a poly(C) sequence, a histone stem-loop, and/or an IRES-motif.

35. The artificial nucleic acid molecule according to any one of items 1 - 34, wherein the histone stem-loop comprises a sequence according to SEQ ID NO: 34.

36. The artificial nucleic acid molecule according to any one of items 1 - 35, wherein the nucleic acid comprises a promoter.

37. The artificial nucleic acid molecule according to any one of items 1 - 36, wherein the nucleic acid comprises a 5'-TOP UTR.

38. The artificial nucleic acid molecule according to any one of items 1 - 37, wherein the nucleic acid comprises a 3'-UTR, which comprises or consists of a nucleic acid sequence which is derived from a 3'-UTR of an albumin gene.

39. The artificial nucleic acid molecule according to any one of items 1 - 38, wherein the artificial nucleic acid molecule, preferably the open reading frame, is at least partially G/C modified, preferably wherein the G/C content of the open reading frame is increased compared to the wild type open reading frame.

40. The artificial nucleic acid molecule according to any one of items 1 - 39, wherein the open reading frame comprises a codon-optimized region, preferably, wherein the open reading frame is codon-optimized.

41. The artificial nucleic acid molecule according to any one of items 1 - 40, which is an RNA, preferably an mRNA molecule.

42. A vector comprising an artificial nucleic acid molecule according to any one of items 1 - 41.

43. The vector according to item 42, which is a DNA vector.

44. The vector according to item 42 or 43, which is a plasmid vector or a viral vector, preferably a plasmid vector.

45. The vector according to any one of items 42 - 44, which is a circular molecule.

46. The vector according to item 42, wherein the poly(A) sequence, the poly(C) sequence, the histone stem loop or the 3'-UTR element of the coding strand is followed in 5'$\rightarrow$3' direction by a restriction site for linearization of the circular vector molecule.

47. A cell comprising the artificial nucleic acid molecule according to any one of items. 1 - 39 or the vector according to any one of items 42 - 46.

48. The cell according to item 47, which is a mammalian cell.

49. The cell according to item 47 or 48, which is a cell of a mammalian subject, preferably an isolated cell of a mammalian subject, preferably of a human subject.

50. A pharmaceutical composition comprising the artificial nucleic acid molecule according to any one of items 1 - 41, the vector according to any one of items 42 - 46, or the cell according to any one of items 47 - 49.

51. The pharmaceutical composition according to item 50, further comprising one or more pharmaceutically acceptable vehicles, diluents and/or excipients and/or one or more adjuvants.

52. The artificial nucleic acid molecule according to any one of items 1 - 41, the vector according to any one of items 42 - 46, the cell according to any one of items 47 - 49, or the pharmaceutical composition according to item 50 or 51 for use as a medicament.

53. The artificial nucleic acid molecule according to any one of items 1 - 41, the vector according to any one of items 42 - 46, the cell according to any one of items 47 - 49, or the pharmaceutical composition according to item 50 or 51 for use as a vaccine or for use in gene therapy.

54. A method for treating or preventing a disorder comprising administering the artificial nucleic acid molecule according to any one of items 1 - 41, the vector according to any one of items 42 - 46, the cell according to any one of items 47 - 49, or the pharmaceutical composition according to item 50 or 51 to a subject in need thereof.

55. A method of treating or preventing a disorder comprising transfection of a cell with an artificial nucleic acid molecule according to any one of items 1 - 41 or the vector according to any one of items 42 - 46.

56. The method according to item 55, wherein transfection of a cell is performed *in vitro*/*ex vivo* and the transfected cell is administered to a subject in need thereof, preferably to a human patient.

57. The method according to item 56, wherein the cell which is to be transfected *in vitro* is an isolated cell of the subject, preferably of the human patient.

58. The method according to any one of items 54 - 57, which is a vaccination method or a gene therapy method.

59. A method for increasing and/or prolonging protein production from an artificial nucleic acid molecule, preferably from an mRNA molecule or a vector, the method comprising the step of associating an open reading frame with a 3'-UTR element and/or a 5'-UTR element, wherein the 3'-UTR element and/or the 5'-UTR element prolongs and/or increases protein production from a resulting artificial nucleic acid molecule and wherein the at least one 3'-UTR element and/or the at least one 5'-UTR element is derived from a stable mRNA, to obtain an artificial nucleic acid molecule, preferably an mRNA molecule, according to any of items 1 - 41 or a vector according to any of items 42 - 46.

60. A method for increasing and/or prolonging protein production from an artificial nucleic acid molecule, preferably from an mRNA molecule or a vector, according to item 59, wherein the 3'-UTR element and/or the 5'-UTR element comprises or consists of a nucleic acid sequence which is derived from the 3'-UTR and/or the 5'-UTR of a transcript of a gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, Atp5e, Gstm5, Uqcr11, Ifi27l2a, Anapc13, Atp5l, Tmsb10, Nenf, Ndufa7, Atp5k, 1110008P14Rik, Cox4i1, Cox6a1, Ndufs6, Sec61b, Romo1, Snrpd2, Mgst3, Aldh2, , Ssr4, Myl6, Prdx4, Ubl5, 1110001J03Rik, Ndufa13, Ndufa3, Gstp2, Tmem160, Ergic3, Pgcp, Slpi, Myeov2, Ndufs5, 1810027O10Rik, Atp5o, Shfm1, Tspo, S100a6, Taldo1, Bloc1s1, Hexa, Ndufb11, Map1lc3a, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1; preferably from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), and CNTN1 (contactin 1).

61. Use of a 3'-UTR element and/or a 5'-UTR element for increasing and/or prolonging protein production from an artificial nucleic acid molecule, preferably from an mRNA molecule or a vector, wherein the 3'-UTR element and/or the 5'-UTR element comprises or consists of a nucleic acid sequence which is derived from the 3'-UTR and/or the

5'-UTR of a transcript of a gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, Atp5e, Gstm5, Uqcr11, Ifi27l2a, Anapc13, Atp5l, Tmsb10, Nenf, Ndufa7, Atp5k, 1110008P14Rik, Cox4i1, Cox6a1, Ndufs6, Sec61b, Romo1, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufs5, 1810027O10Rik, Atp5o, Shfm1, Tspo, S100a6, Taldo1, Bloc1s1, Hexa, Ndufb11, Map1lc3a, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1; preferably from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), and CNTN1 (contactin 1).

62. The method according to item 60 or the use according to item 61, wherein the at least one 3'-UTR element and/or the at least one 5'-UTR element comprises a nucleic acid sequence which is derived from the 3'-UTR and/or the 5'-UTR of a transcript of a human gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), CNTN1 (contactin 1), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, LMBRD2, TLR3, BCAT1, TOM1L1, SLC35A1, GLYATL2, STAT4, GULP1, EHHADH, NBEAL1, KIAA1598, HFE, KIAA1324L, and MANSC1.

63. The method according to item 60 or the use according to item 61, wherein the at least one 3'-UTR element and/or the at least one 5'-UTR element comprises a nucleic acid sequence which is derived from the 3'-UTR and/or the 5'-UTR of a transcript of a murine gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), CNTN1 (contactin 1), Atp5e, Gstm5, Uqcr11, Ifi27l2a, Anapc13, Atp5l, Tmsb10, Nenf, Ndufa7, Atp5k, 1110008P14Rik, Cox4i1, Cox6a1, Ndufs6, Sec61b, Romo1, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufs5, 1810027O10Rik, Atp5o, Shfm1, Tspo, S100a6, Taldo1, Bloc1s1, Hexa, Ndufb11, Map1lc3a, Gpx4, Mif, Cox6b1, RIKEN cDNA2900010J23 (Swi5), Sec61g, 2900010M23Rik, Anapc5, Mars2, Phpt1, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabac1, Robld3, Sod1, Nedd8, Higd2a, Trappc6a, Ldhb, Nme2, Snrpg, Ndufa2, Serf1, Oaz1, Rps4x, Rps13, Sepp1, and Gaa.

64. The method or the use according to any one of the items 60 - 63, wherein the at least one 3'-UTR element comprises a nucleic acid sequence which is derived from the 3'-UTR of a transcript of a gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), SLC38A6, DECR1, PIGK, FAM175A, PHYH, TBC1D19, PIGB, ALG6, CRYZ, BRP44L, ACADSB, TMEM14A, GRAMD1C, C11orf80, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, HADHB, ANAPC4, PCCB, ABCB7, PGCP, NFU1, OMA1, HHLA3, ACAA2, GSTM4, ALG8, Ndufa1, Atp5e, Gstm5, Uqcr11, Ifi27l2a, Cbr2, Atp5l, Tmsb10, Nenf, Atp5k, 1110008P14Rik, Cox4i1, Cox6a1, Ndufs6, Sec61b, Romo1, Gnas, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufa4, Ndufs5, Gstm1, 1810027O10Rik, Atp5o, Shfm1, Tspo, S100a6, Taldo1, Bloc1s1, Ndufb11, Map1lc3a, Morn2, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabacl, Robld3, Sod1, Nedd8, Higd2a, Trappc6a, Ldhb, Nme2, Snrpg, Ndufa2, Serf1, Oaz1, Rps4x, Rps13, Ybxl, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1; preferably, the at least one 3'-UTR element comprises or consists of a nucleic acid sequence which is derived from the 3'-UTR of a transcript of a gene selected from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), Ybx1 (Y-Box binding protein 1), Ndufb8 (NADH dehydrogenase (ubiquinone) 1 beta subcomplex 8), and CNTN1 (contactin 1).

65. The method or the use according to any one of the items 60 - 63, wherein the at least one 5'-UTR element comprises a nucleic acid sequence which is derived from the 5'-UTR of a transcript of a gene selected from the group consisting of MP68 (RIKEN cDNA 2010107E04 gene), NDUFA4 (NADH dehydrogenase (ubiquinone) 1 alpha subcomplex 4), LTA4H, DECR1, PIGK, TBC1D19, BRP44L, ACADSB, SUPT3H, TMEM14A, C9orf46, ANXA4, IFI6, C2orf34, ALDH6A1, CCDC53, CCDC104, CASP1, NDUFB6, BCKDHB, BBS2, HERC5, FAM175A, NT5DC1, RAB7A, AGA, TPK1, MBNL3, MCCC2, CAT, ANAPC4, PHKB, ABCB7, GPD2, TMEM38B, NFU1, LOC128322/NUTF2, NUBPL, LANCL1, PIR, CTBS, GSTM4, Ndufa1, Atp5e, Gstm5, Cbr2, Anapc13, Ndufa7, Atp5k, 1110008P14Rik, Cox4i1, Ndufs6, Sec61b, Snrpd2, Mgst3, , Prdx4; Pgcp; Myeov2; Ndufa4; Ndufs5; Gstm1; Atp5o; Tspo; Taldo1; Bloc1s1; and Hexa; preferably, the at least one 5'-UTR element comprises or consists of a nucleic acid sequence which is derived from the 5'-UTR of a transcript of MP68 (RIKEN cDNA 2010107E04 gene) or NDUFA4 (NADH dehydrogenase (ubiquinone) 1 alpha subcomplex 4).

66. The method or the use according to any one of the items 60 - 64, wherein the 3'-UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a sequence selected from the group consisting of SEQ ID NOs: 1 to 24 and SEQ ID NOs: 49 to 318 or wherein the 3'-UTR element comprises or consists of a fragment of a nucleic acid sequence that has an identity of at least about 40%, preferably of at least about 50%, preferably of at least about 60%, preferably of at least about

70%, more preferably of at least about 80%, more preferably of at least about 90%, even more preferably of at least about 95%, even more preferably of at least about 99% to a sequence selected from the group consisting of SEQ ID NOs: 1 to 24 and SEQ ID NOs: 49 to 318.

67. The method or the use according to any one of the items 60 - 63 and 65, wherein the 5'-UTR element comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a sequence selected from the group consisting of SEQ ID NOs: 25 to 30 and SEQ ID NOs: 319 to 382 or wherein the 5'-UTR element comprises or consists of a fragment of a nucleic acid sequence that has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a sequence selected from the group consisting of SEQ ID NOs: 25 to 30 and SEQ ID NOs: 319 to 382.

68. The method or the use according to item 66 or 67, wherein the fragment exhibits a length of between 3 and about 500 nucleotides, preferably of between 5 and about 150 nucleotides, more preferably of between 10 and 100 nucleotides, even more preferably of between 15 and 90, most preferably of between 20 and 70.

69. The method or the use according to any one of items 60 - 68, wherein the 3'-UTR element and/or the 5'-UTR element exhibits a length of between 3 and about 500 nucleotides, preferably of between 5 and about 150 nucleotides, more preferably of between 10 and 100 nucleotides, even more preferably of between 15 and 90, most preferably of between 20 and 70.

70. A kit or kit of parts comprising an artificial nucleic acid molecule according to any one of items 1 - 41, a vector according to any one of items 42 - 46, a cell according to any one of items 47 - 49, and/or a pharmaceutical composition according to item 50 or 51.

71. The kit according to item 70 further comprising instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector, the cells or the pharmaceutical composition.

72. A method for identifying a 3'-untranslated region element (3'-UTR element) and/or a 5'-untranslated region element (5'-UTR element), which is derived from a stable mRNA comprising the following steps:

a) Analyzing the stability of an mRNA comprising the following sub-steps:

i. Determining the amount of said mRNA at a first point in time during a decay process of said mRNA,
ii. Determining the amount of said mRNA at a second point in time during a decay process of said mRNA, and
iii. Calculating the ratio of the amount of said mRNA determined in step (i) to the the amount of said mRNA determined in step (ii);

b) Selecting a stable mRNA having a ratio calculated in sub-step (iii) of at least 0.5 (50%), at least 0.6 (60%), at least 0.7 (70%), at least 0.75 (75%), at least 0.8 (80%), at least 0.85 (85%), at least 0.9 (90%), or at least 0.95 (95%); and
c) Determining the nucleotide sequence of a 3'- and/or 5'-UTR element of said stable mRNA.

73. A method for identifying a 3'-untranslated region element (3'-UTR element) and/or a 5'-untranslated region element (5'-UTR element), which is derived from a stable mRNA comprising the following steps:

a) Analyzing the stability of a plurality of mRNA species comprising the following sub-steps:

i. Determining the amount of each mRNA species of said plurality of mRNA species at a first point in time during a decay process of said mRNA species,
ii. Determining the amount of each mRNA species of said plurality of mRNA species at a second point in time during a decay process of said mRNA species, and
iii. Calculating for each mRNA species of said plurality of mRNA species the ratio of the amount of said

mRNA species determined in step (i) to the the amount of said mRNA species determined in step (ii);

b) Ranking of the mRNA species of the plurality of mRNA species according to the ratio calculated in sub-step (iii) for each mRNA species;
c) Selecting one or more mRNA species having the highest ratio or the highest ratios calculated in sub-step (iii); and
d) Determining the nucleotide sequence of a 3'- and/or 5'-UTR element of said mRNA.

74. The method for identifying a 3'-UTR element and/or a 5'-UTR element according to item 72 or 73, wherein the time period between the first point in time and the second point in time is at least 5h, preferably at least 6h, preferably at least 7h, more preferably at least 8h, more preferably at least 9h, even more preferably at least 10h, even more preferably at least 11h, and particularly preferably at least 12h.

75. The method for identifying a 3'-UTR element and/or a 5'-UTR element according to any of items 72 - 74, wherein the stability of an mRNA is analysed by pulse labelling, preferably using a pulse-chase methodology.

76. A method for identifying a 3'-untranslated region element (3'-UTR element) and/or a 5'-untranslated region element (5'-UTR element), which prolongs and/or increases protein production from an artificial nucleic acid molecule and which is derived from a stable mRNA comprising the following steps:

a) identifying a 3'-UTR element and/or a 5'-UTR element which is derived from a stable mRNA by a method for identifying a 3'-UTR element and/or a 5'-UTR element according to any of items 72 - 75;
b) synthesizing an artificial nucleic acid molecule comprising at least one open reading frame and at least one 3'-UTR element and/or at least one 5'-UTR element which corresponds to or is comprised by the 3'-UTR element and/or the 5'-UTR element identified in step a);
c) analyzing the expression of the protein encoded by the at least one open reading frame of the artificial nucleic acid molecule synthesized in step b);
d) analyzing the expression of a protein encoded by at least one open reading frame (ORF) of a reference artificial nucleic acid molecule lacking a 3'-UTR element and/or a 5'-UTR element;
e) comparing the protein expression from the artificial nucleic acid molecule analysed in step c) to the protein expression from the reference artificial nucleic acid molecule analysed in step d); and
f) selecting the 3'-UTR element and/or the 5'-UTR element if the protein expression from the artificial nucleic acid molecule analysed in step c) is prolonged and/or increased in comparison to the protein expression from the reference artificial nucleic acid molecule analysed in step d).

77. A method for generating an artificial nucleic acid molecule, wherein an artificial nucleic acid molecule comprising at least one open reading frame and at least one 3'-UTR element and/or at least one 5'-UTR element identified by a method for identifying a 3'-UTR element and/or a 5'-UTR element according to any of items 72 - 76 is synthesized.

78. The method for generating an artificial nucleic acid molecule according to item 77, wherein a vector according to any of items 42 - 46 is used for synthesizing the artificial nucleic acid molecule.

79. The method for generating an artificial nucleic acid molecule according to item 77 or 78, wherein the artificial nucleic acid molecule is an artificial nucleic acid molecule according to any of items 1 to 41.

80. An artificial nucleic acid molecule obtainable by a method for generating an artificial nucleic acid molecule according to any of items 77 - 79.

## Claims

1. An artificial nucleic acid molecule comprising

   a. at least one open reading frame (ORF); and
   b. at least one 3'-untranslated region element (3'-UTR element) and/or at least one 5'-untranslated region element (5'-UTR element), wherein the at least one 3'-UTR element and/or the at least one 5'-UTR element prolongs and/or increases protein production from said artificial nucleic acid molecule and wherein the at least one 3'-UTR element and/or the at least one 5'-UTR element is derived from a stable mRNA,
   wherein the at least one 3'-UTR element and/or the at least one 5'-UTR element comprises or consists of a nucleic acid sequence which is derived from the 3'-UTR and/or the 5'-UTR of a transcript of GNAS (guanine nucleotide binding protein, alpha stimulating complex locus).

2. The artificial nucleic acid molecule according to claim 1, wherein the open reading frame is derived from a gene, which is distinct from a gene from which the at least one 3'-UTR element and/or the at least one 5'-UTR element is derived.

3. The artificial nucleic acid molecule according to claim 2, wherein each of the at least one open reading frame, the at least one 3'-UTR element and the at least one 5'-UTR element are heterologous to each other.

4. The artificial nucleic acid molecule according to any of claims 1 to 3, wherein the stable mRNA from which the at least one 3'-UTR element and/or the at least one 5'-UTR element is derived is **characterized by** an mRNA decay wherein the ratio of the amount of said mRNA at a second point in time to the amount of said mRNA at a first point in time is at least 0.5 (50%), at least 0.6 (60%), at least 0.7 (70%), at least 0.75 (75%), at least 0.8 (80%), at least 0.85 (85%), at least 0.9 (90%), or at least 0.95 (95%).

**5.** The artificial nucleic acid molecule according to any of claims 1 to 4, wherein the artificial nucleic acid molecule does not comprise a 3'-UTR and/or a 5'-UTR of ribosomal protein S6, of RPL36AL, of rps16 or of ribosomal protein L9 and wherein the open reading frame of the artificial nucleic acid molecule does not code for a GFP protein and wherein the open reading frame of the artificial nucleic acid molecule preferably does not code for a reporter protein.

**6.** The artificial nucleic acid molecule according to any of claims 1 to 5, wherein the at least one 3'-UTR element and/or the at least one 5'-UTR element prolongs protein production from said artificial nucleic acid molecule at least 1.2 fold, preferably at least 1.5 fold, more preferably at least 2 fold, even more preferably at least 2.5 fold, compared to the protein production from a reference nucleic acid molecule lacking a 3'-UTR and/or the at least one 5'-UTR, respectively, and/or wherein the at least one 3'-UTR element and/or the at least one 5'-UTR element increases protein production from said artificial nucleic acid molecule at least 1.5 fold, preferably at least 2 fold, more preferably at least 2.5 fold, compared to the protein production from a reference nucleic acid molecule lacking a 3'-UTR and/or the at least one 5'-UTR, respectively.

**7.** The artificial nucleic acid molecule according to any one of claims 1 to 6, wherein the at least one 3'-UTR element and/or the at least one 5'-UTR element additionally comprises a nucleic acid sequence which is derived from the 3'-UTR and/or the 5'-UTR of a transcript of a gene selected from the group consisting of NDUFA1 (NADH dehydrogenase (ubiquinone) 1 alpha subcomplex), , MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), LTA4H, SLC38A6, DECR1, PIGK, FAM175A, PHYH, TBC1D19, PIGB, ALG6, CRYZ, BRP44L, ACADSB, SUPT3H, TMEM14A, GRAMD1C, C11orf80, C9orf46, ANXA4, TBCK, IFI6, C2orf34, ALDH6A1, AGTPBP1, CCDC53, LRRC28, CCDC109B, PUS10, CCDC104, CASP1, SNX14, SKAP2, NDUFB6, EFHA1, BCKDHB, BBS2, LMBRD1, ITGA6, HERC5, NT5DC1, RAB7A, AGA, TPK1, MBNL3, HADHB, MCCC2, CAT, ANAPC4, PCCB, PHKB, ABCB7, PGCP, GPD2, TMEM38B, NFU1, OMA1, LOC128322/NUTF2, NUBPL, LANCL1, HHLA3, PIR, ACAA2, CTBS, GSTM4, ALG8, Ndufa1, Atp5e, Gstm5, Uqcr11, Ifi27l2a, Cbr2, Anapc13, Atp5l, Tmsb10, Nenf, Ndufa7, Atp5k, 1110008P14Rik, Cox4i1, Cox6a1, Ndufs6, Sec61b, Romo1, Gnas, Snrpd2, Mgst3, Aldh2, Ssr4, Myl6, Prdx4, Ubl5, 1110001J03Rik, Ndufa13, Ndufa3, Gstp2, Tmem160, Ergic3, Pgcp, Slpi, Myeov2, Ndufa4, Ndufs5, Gstm1, 1810027O10Rik, Atp5o, Shfm1, Tspo, S100a6, Taldo1, Bloc1s1, Hexa, Ndufb11, Map1lc3a, Morn2, Gpx4, Mif, Cox6b1, RIKEN cDNA2900010J23 (Swi5), Sec61g, 2900010M23Rik, Anapc5, Mars2, Phpt1, Ndufb8, Pfdn5, Arpc3, Ndufb7, Atp5h, Mrpl23, Uba52, Tomm6, Mtch1, Pcbd2, Ecm1, Hrsp12, Mecr, Uqcrq, Gstm3, Lsm4, Park7, Usmg5, Cox8a, Ly6c1, Cox7b, Ppib, Bag1, S100a4, Bcap31, Tecr, Rabacl, Robld3, Sod1, Nedd8, Higd2a, Trappc6a, Ldhb, Nme2, Snrpg, Ndufa2, Serf1, Oaz1, Rps4x, Rps13, Ybx1, Sepp1, Gaa, ACTR10, PIGF, MGST3, SCP2, HPRT1, ACSF2, VPS13A, CTH, NXT2, MGST2, C11orf67, PCCA, GLMN, DHRS1, PON2, NME7, ETFDH, ALG13, DDX60, DYNC2LI1, VPS8, ITFG1, CDK5, C1orf112, IFT52, CLYBL, FAM114A2, NUDT7, AKD1, MAGED2, HRSP12, STX8, ACAT1, IFT74, KIFAP3, CAPN1, COX11, GLT8D4, HACL1, IFT88, NDUFB3, ANO10, ARL6, LPCAT3, ABCD3, COPG2, MIPEP, LEPR, C2orf76, ABCA6, LY96, CROT, ENPP5, SERPINB7, TCP11L2, IRAK1BP1, CDKL2, GHR, KIAA1107, RPS6KA6, CLGN, TMEM45A, TBC1D8B, ACP6, RP6-213H19.1, SNRPN, GLRB, HERC6, CFH, GALC, PDE1A, GSTM5, CADPS2, AASS, TRIM6-TRIM34 (readthrough transcript), SEPP1, PDE5A, SATB1, CCPG1, CNTN1, LMBRD2, TLR3, BCAT1, TOM1L1, SLC35A1, GLYATL2, STAT4, GULP1, EHHADH, NBEAL1, KIAA1598, HFE, KIAA1324L, and MANSC1; preferably from the group consisting of NDUFA1 (NADH dehydrogenase (ubiquinone) 1 alpha subcomplex), GNAS (guanine nucleotide binding protein, alpha stimulating complex locus), MORN2 (MORN repeat containing 2), GSTM1 (glutathione S-transferase, mu 1), CBR2 (carbonyl reductase 2), MP68 (RIKEN cDNA 2010107E04 gene), NDUFA4 (NADH dehydrogenase (ubiquinone) 1 alpha subcomplex 4), Ybx1 (Y-Box binding protein 1), Ndufb8 (NADH dehydrogenase (ubiquinone) 1 beta subcomplex 8), and CNTN1 (contactin 1).

**8.** The artificial nucleic acid molecule according to any one of claims 1 to 6, wherein the at least one 3'-UTR element comprises a nucleic acid sequence which is derived from the 3'-UTR of a transcript of a gene selected from the group consisting of human or murine GNAS (guanine nucleotide binding protein, alpha stimulating complex locus).

**9.** The artificial nucleic acid molecule according to claim 7, wherein the at least one 5'-UTR element additionally comprises a nucleic acid sequence which is derived from the 5'-UTR of a transcript of a gene selected from the group consisting of MP68 (RIKEN cDNA 2010107E04 gene), NDUFA4 (NADH dehydrogenase (ubiquinone) 1 alpha subcomplex 4), LTA4H, DECR1, PIGK, TBC1D19, BRP44L, ACADSB, SUPT3H, TMEM14A, C9orf46, ANXA4, IFI6, C2orf34, ALDH6A1, CCDC53, CCDC104, CASP1, NDUFB6, BCKDHB, BBS2, HERC5, FAM175A, NT5DC1, RAB7A, AGA, TPK1, MBNL3, MCCC2, CAT, ANAPC4, PHKB, ABCB7, GPD2, TMEM38B, NFU1, LOC128322/NUTF2, NUBPL, LANCL1, PIR, CTBS, GSTM4, Ndufa1, Atp5e, Gstm5, Cbr2, Anapc13, Ndufa7, Atp5k, 1110008P14Rik, Cox4i1, Ndufs6, Sec61b, Snrpd2, Mgst3, Prdx4; Pgcp; Myeov2; Ndufa4; Ndufs5; Gstm1; Atp5o;

Tspo; Taldo1; Bloc1s1; and Hexa; preferably, the at least one 5'-UTR element comprises or consists of a nucleic acid sequence which is derived from the 5'-UTR of a transcript of MP68 (RIKEN cDNA 2010107E04 gene) or NDUFA4 (NADH dehydrogenase (ubiquinone) 1 alpha subcomplex 4).

**10.** The artificial nucleic acid molecule according to any one of claims 1 - 8, wherein the at least one 3'-UTR element comprises or consists of a nucleic acid sequence which has an identity of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 1 to 24 and SEQ ID NOs: 49 to 318 or wherein the at least one 3'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to fragment of a nucleic acid sequence selected from the group consisting of SEQ ID NOs: 1 to 24 and SEQ ID NOs: 49 to 318.

**11.** The artificial nucleic acid molecule according to any one of claims 1 - 7 and 9, wherein the at least one 5'-UTR element comprises or consists of a nucleic acid sequence which has an identity of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 25 to 30 and SEQ ID NOs: 319 to 382 or wherein the at least one 5'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence selected from the group consisting of SEQ ID NOs: 25 to 30 and SEQ ID NOs: 319 to 382.

**12.** The artificial nucleic acid molecule according to any one of claims 1 - 11 further comprising

c. a poly(A) sequence and/or a polyadenylation signal,
wherein the poly(A) sequence or the polyadenylation signal is preferably located 3' of the 3'-UTR element and/or wherein the polyadenylation signal preferably comprises the consensus sequence NN(U/T)ANA, with N = A or U, preferably AA(U/T)AAA or A(U/T)(U/T)AAA and/or wherein the polyadenylation signal, preferably the consensus sequence NNUANA, is preferably located less than about 50 nucleotides downstream of the 3'-end of the 3'-UTR element and/or wherein the poly(A) sequence has preferably a length of about 20 to about 300 adenine nucleotides, preferably of about 40 to about 200 adenine nucleotides, more preferably of about 50 to about 100 adenine nucleotides, even more preferably of about 60 to about 70 adenine nucleotides.

**13.** The artificial nucleic acid molecule according to any one of claims 1 - 12, further comprising a 5'-cap structure, a poly(C) sequence, a histone stem-loop, and/or an IRES-motif, wherein the histone stem-loop preferably comprises a sequence according to SEQ ID NO: 34.

**14.** The artificial nucleic acid molecule according to any one of claims 1 - 13, wherein the nucleic acid comprises a 5'-TOP UTR and/or
wherein the nucleic acid comprises a 3'-UTR, which comprises or consists of a nucleic acid sequence which is derived from a 3'-UTR of an albumin gene and/or
wherein the artificial nucleic acid molecule, preferably the open reading frame, is at least partially G/C modified, preferably wherein the G/C content of the open reading frame is increased compared to the wild type open reading frame and/or
wherein the open reading frame comprises a codon-optimized region, preferably, wherein the open reading frame is codon-optimized and/or
wherein the artificial nucleic acid molecule is an RNA, preferably an mRNA molecule.

**15.** A vector, preferably a circular molecule, comprising an artificial nucleic acid molecule according to any one of claims 1 - 14, which is preferably a DNA vector,
which is more preferably a plasmid vector or a viral vector, most preferably a plasmid vector.

**16.** A cell comprising the artificial nucleic acid molecule according to any one of claims 1 - 14 or the vector according to claim 15,

which is preferably a mammalian cell.

**17.** A pharmaceutical composition comprising the artificial nucleic acid molecule according to any one of claims 1 - 14, the vector according to claim 15, or the cell according to claim 16, preferably comprising one or more pharmaceutically acceptable vehicles, diluents and/or excipients and/or one or more adjuvants.

**18.** The artificial nucleic acid molecule according to any one of claims 1 - 14, the vector according to claim 15, the cell according to any one of claim 16, or the pharmaceutical composition according to claim 17 for use as a medicament, preferably for use as a vaccine or for use in gene therapy.

**19.** An in vitro method for increasing and/or prolonging protein production from an artificial nucleic acid molecule, preferably from an mRNA molecule or a vector, the method comprising the step of associating an open reading frame with a 3'-UTR element and/or a 5'-UTR element, wherein the 3'-UTR element and/or the 5'-UTR element prolongs and/or increases protein production from a resulting artificial nucleic acid molecule and wherein the at least one 3'-UTR element and/or the at least one 5'-UTR element is derived from a stable mRNA, to obtain an artificial nucleic acid molecule, preferably an mRNA molecule, according to any of claims 1 - 14 or a vector according to claim 15.

**20.** Use of a 3'-UTR element and/or a 5'-UTR element for increasing and/or prolonging protein production from an artificial nucleic acid molecule, preferably from an mRNA molecule or a vector, wherein the 3'-UTR element and/or the 5'-UTR element comprises or consists of a nucleic acid sequence which is derived from the 3'-UTR and/or the 5'-UTR of a transcript of GNAS (guanine nucleotide binding protein, alpha stimulating complex locus).

**21.** A kit or kit of parts comprising an artificial nucleic acid molecule according to any one of claims 1 - 14, a vector according to claim 15, a cell according to claim 16, and/or a pharmaceutical composition according to claim 17.

**22.** The kit according to claim 21 further comprising instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector, the cells or the pharmaceutical composition.

32L4 – PpLuc(GC) – A64 - C30 - hSL (R2464)

(SEQ ID NO: 35):

GGGGCGCUGCCUACGGAGGUGGCAGCCAUCUCCUUCUCGGCAUCAAGCUUGAGGAUGGAG
GACGCCAAGAACAUCAAGAAGGGCCCGGCGCCCUUCUACCCGCUGGAGGACGGGACCGCC
GGCGAGCAGCUCCACAAGGCCAUGAAGCGGUACGCCCUGGUGCCGGGCACGAUCGCCUUC
ACCGACGCCCACAUCGAGGUCGACAUCACCUACGCGGAGUACUUCGAGAUGAGCGUGCGC
CUGGCCGAGGCCAUGAAGCGGUACGGCCUGAACACCAACCACCGGAUCGUGGUGUGCUCG
GAGAACAGCCUGCAGUUCUUCAUGCCGGUGCUGGGCGCCCUCUUCAUCGGCGUGGCCGUC
GCCCCGGCGAACGACAUCUACAACGAGCGGGAGCUGCUGAACAGCAUGGGGAUCAGCCAG
CCGACCGUGGUGUUCGUGAGCAAGAAGGGCCUGCAGAAGAUCCUGAACGUGCAGAAGAAG
CUGCCCAUCAUCCAGAAGAUCAUCAUCAUGGACAGCAAGACCGACUACCAGGGCUUCCAG
UCGAUGUACACGUUCGUGACCAGCCACCUCCCGCCGGGCUUCAACGAGUACGACUUCGUC
CCGGAGAGCUUCGACCGGGACAAGACCAUCGCCCUGAUCAUGAACAGCAGCGGCAGCACC
GGCCUGCCGAAGGGGGUGGCCCUGCCGCACCGGACCGCCUGCGUGCGCUUCUCGCACGCC
CGGGACCCCAUCUUCGGCAACCAGAUCAUCCCGGACACCGCCAUCCUGAGCGUGGUGCCG
UUCCACCACGGCUUCGGCAUGUUCACGACCCUGGGCUACCUCAUCUGCGGCUUCCGGGUG
GUCCUGAUGUACCGGUUCGAGGAGGAGCUGUUCCUGCGGAGCCUGCAGGACUACAAGAUC
CAGAGCGCGCUGCUCGUGCCGACCCUGUUCAGCUUCUUCGCCAAGAGCACCCUGAUCGAC
AAGUACGACCUGUCGAACCUGCACGAGAUCGCCAGCGGGGGCGCCCCGCUGAGCAAGGAG
GUGGGCGAGGCCGUGGCCAAGCGGUUCCACCUCCCGGGCAUCCGCCAGGGCUACGGCCUG
ACCGAGACCACGAGCGCGAUCCUGAUCACCCCCGAGGGGGACGACAAGCCGGGCGCCGUG
GGCAAGGUGGUCCCGUUCUUCGAGGCCAAGGUGGUGGACCUGGACACCGGCAAGACCCUG
GGCGUGAACCAGCGGGGCGAGCUGUGCGUGCGGGGGCCGAUGAUCAUGAGCGGCUACGUG
AACAACCCGGAGGCCACCAACGCCCUCAUCGACAAGGACGGCUGGCUGCACAGCGGCGAC
AUCGCCUACUGGGACGAGGACGAGCACUUCUUCAUCGUCGACCGGCUGAAGUCGCUGAUC
AAGUACAAGGGCUACCAGGUGGCGCCGGCCGAGCUGGAGAGCAUCCUGCUCCAGCACCCC
AACAUCUUCGACGCCGGCGUGGCCGGGCUGCCGGACGACGACGCCGGCGAGCUGCCGGCC
GCGGUGGUGGUGCUGGAGCACGGCAAGACCAUGACGGAGAAGGAGAUCGUCGACUACGUG
GCCAGCCAGGUGACCACCGCCAAGAAGCUGCGGGGCGGCGUGGUGUUCGUGGACGAGGUC
CCGAAGGGCCUGACCGGGAAGCUCGACGCCCGGAAGAUCCGCGAGAUCCUGAUCAAGGCC
AAGAAGGGCGGCAAGAUCGCCGUGUAAGACUAGUAGAUCUAAAAAAAAAAAAAAAAAAAA
AAAAAAAAAAAAAAAAAAAAAAAAAAAAAAAAAAAAAAAAAAAAUGCAUCCCCCCCCCCC
CCCCCCCCCCCCCCCCCCCAAAGGCUCUUUUCAGAGCCACCAGAAUU

Fig. 1

32L4 – PpLuc(GC) – gnas-A64-C30-hSL (R3089)

(SEQ ID NO: 36):

GGGGCGCUGCCUACGGAGGUGGCAGCCAUCUCCUUCUCGGCAUCAAGCUUGAGGAUGGAG
GACGCCAAGAACAUCAAGAAGGGCCCGGCGCCCUUCUACCCGCUGGAGGACGGGACCGCC
GGCGAGCAGCUCCACAAGGCCAUGAAGCGGUACGCCCUGGUGCCGGGCACGAUCGCCUUC
ACCGACGCCCACAUCGAGGUCGACAUCACCUACGCGGAGUACUUCGAGAUGAGCGUGCGC
CUGGCCGAGGCCAUGAAGCGGUACGGCCUGAACACCAACCACCGGAUCGUGGUGUGCUCG
GAGAACAGCCUGCAGUUCUUCAUGCCGGUGCUGGGCGCCCUCUUCAUCGGCGUGGCCGUC
GCCCCGGCGAACGACAUCUACAACGAGCGGGAGCUGCUGAACAGCAUGGGGAUCAGCCAG
CCGACCGUGGUGUUCGUGAGCAAGAAGGGCCUGCAGAAGAUCCUGAACGUGCAGAAGAAG
CUGCCCAUCAUCCAGAAGAUCAUCAUCAUGGACAGCAAGACCGACUACCAGGGCUUCCAG
UCGAUGUACACGUUCGUGACCAGCCACCUCCCGCCGGGCUUCAACGAGUACGACUUCGUC
CCGGAGAGCUUCGACCGGGACAAGACCAUCGCCCUGAUCAUGAACAGCAGCGGCAGCACC
GGCCUGCCGAAGGGGGUGGCCCUGCCGCACCGGACCGCCUGCGUGCGCUUCUCGCACGCC
CGGGACCCCAUCUUCGGCAACCAGAUCAUCCCGGACACCGCCAUCCUGAGCGUGGUGCCG
UUCCACCACGGCUUCGGCAUGUUCACGACCCUGGGCUACCUCAUCUGCGGCUUCCGGGUG
GUCCUGAUGUACCGGUUCGAGGAGGAGCUGUUCCUGCGGAGCCUGCAGGACUACAAGAUC
CAGAGCGCGCUGCUCGUGCCGACCCUGUUCAGCUUCUUCGCCAAGAGCACCCUGAUCGAC
AAGUACGACCUGUCGAACCUGCACGAGAUCGCCAGCGGGGGCGCCCCGCUGAGCAAGGAG
GUGGGCGAGGCCGUGGCCAAGCGGUUCCACCUCCCGGGCAUCCGCCAGGGCUACGGCCUG
ACCGAGACCACGAGCGCGAUCCUGAUCACCCCCGAGGGGGACGACAAGCCGGGCGCCGUG
GGCAAGGUGGUCCCGUUCUUCGAGGCCAAGGUGGUGGACCUGGACACCGGCAAGACCCUG
GGCGUGAACCAGCGGGGCGAGCUGUGCGUGCGGGGGCCGAUGAUCAUGAGCGGCUACGUG
AACAACCCGGAGGCCACCAACGCCCUCAUCGACAAGGACGGCUGGCUGCACAGCGGCGAC
AUCGCCUACUGGGACGAGGACGAGCACUUCUUCAUCGUCGACCGGCUGAAGUCGCUGAUC
AAGUACAAGGGCUACCAGGUGGCGCCGGCCGAGCUGGAGAGCAUCCUGCUCCAGCACCCC
AACAUCUUCGACGCCGGCGUGGCCGGGCUGCCGGACGACGACGCCGGCGAGCUGCCGGCC
GCGGUGGUGGUGCUGGAGCACGGCAAGACCAUGACGGAGAAGGAGAUCGUCGACUACGUG
GCCAGCCAGGUGACCACCGCCAAGAAGCUGCGGGGCGGCGUGGUGUUCGUGGACGAGGUC
CCGAAGGGCCUGACCGGGAAGCUCGACGCCCGGAAGAUCCGCGAGAUCCUGAUCAAGGCC
AAGAAGGGCGGCAAGAUCGCCGUGUAAGACUAGUGAAGGGAACACCCAAAUUUAAUUCAG
CCUUAAGCACAAUUAAUUAAGAGUGAAACGUAAUUGUACAAGCAGUUGGUCACCCACCAU
AGGGCAUGAUCAACACCGCAACCUUUCCUUUUUCCCCCAGUGAUUCUGAAAAACCCCUCU
UCCCUUCAGCUUGCUUAGAUGUUCCAAAUUUAGUAAGCUUAAGGCGGCCUACAGAAGAAA
AAGAAAAAAAAGGCCACAAAAGUUCCCUCUCACUUUCAGUAAAUAAAAUAAAAGCAGCAA
CAGAAAUAAAGAAAUAAAUGAAAUUCAAAAUGAAAUAAAUAUUGUGUUGUGCAGCAUUAA
AAAAUCAAUAAAAAUUAAAAAUGAGCAAGAUCUAAAAAAAAAAAAAAAAAAAAAAAAAAA
AAAAAAAAAAAAAAAAAAAAAAAAAAAAAAAAAAAAAUGCAUCCCCCCCCCCCCCCCCCC
CCCCCCCCCCCCAAAGGCUCUUUUCAGAGCCACCAGAAUU

Fig. 2

32L4 - PpLuc(GC) – morn2– A64 - C30 - hSL (R3106)

(SEQ ID NO: 37):

GGGGCGCUGCCUACGGAGGUGGCAGCCAUCUCCUUCUCGGCAUCAAGCUUGAGGAUGGAG
GACGCCAAGAACAUCAAGAAGGGCCCGGCGCCCUUCUACCCGCUGGAGGACGGGACCGCC
GGCGAGCAGCUCCACAAGGCCAUGAAGCGGUACGCCCUGGUGCCGGGCACGAUCGCCUUC
ACCGACGCCCACAUCGAGGUCGACAUCACCUACGCGGAGUACUUCGAGAUGAGCGUGCGC
CUGGCCGAGGCCAUGAAGCGGUACGGCCUGAACACCAACCACCGGAUCGUGGUGUGCUCG
GAGAACAGCCUGCAGUUCUUCAUGCCGGUGCUGGGCGCCCUCUUCAUCGGCGUGGCCGUC
GCCCCGGCGAACGACAUCUACAACGAGCGGGAGCUGCUGAACAGCAUGGGGAUCAGCCAG
CCGACCGUGGUGUUCGUGAGCAAGAAGGGCCUGCAGAAGAUCCUGAACGUGCAGAAGAAG
CUGCCCAUCAUCCAGAAGAUCAUCAUCAUGGACAGCAAGACCGACUACCAGGGCUUCCAG
UCGAUGUACACGUUCGUGACCAGCCACCUCCCGCCGGGCUUCAACGAGUACGACUUCGUC
CCGGAGAGCUUCGACCGGGACAAGACCAUCGCCCUGAUCAUGAACAGCAGCGGCAGCACC
GGCCUGCCGAAGGGGGUGGCCCUGCCGCACCGGACCGCCUGCGUGCGCUUCUCGCACGCC
CGGGACCCCAUCUUCGGCAACCAGAUCAUCCCGGACACCGCCAUCCUGAGCGUGGUGCCG
UUCCACCACGGCUUCGGCAUGUUCACGACCCUGGGCUACCUCAUCUGCGGCUUCCGGGUG
GUCCUGAUGUACCGGUUCGAGGAGGAGCUGUUCCUGCGGAGCCUGCAGGACUACAAGAUC
CAGAGCGCGCUGCUCGUGCCGACCCUGUUCAGCUUCUUCGCCAAGAGCACCCUGAUCGAC
AAGUACGACCUGUCGAACCUGCACGAGAUCGCCAGCGGGGGCGCCCCGCUGAGCAAGGAG
GUGGGCGAGGCCGUGGCCAAGCGGUUCCACCUCCCGGGCAUCCGCCAGGGCUACGGCCUG
ACCGAGACCACGAGCGCGAUCCUGAUCACCCCCGAGGGGGACGACAAGCCGGGCGCCGUG
GGCAAGGUGGUCCCGUUCUUCGAGGCCAAGGUGGUGGACCUGGACACCGGCAAGACCCUG
GGCGUGAACCAGCGGGGCGAGCUGUGCGUGCGGGGGCCGAUGAUCAUGAGCGGCUACGUG
AACAACCCGGAGGCCACCAACGCCCUCAUCGACAAGGACGGCUGGCUGCACAGCGGCGAC
AUCGCCUACUGGGACGAGGACGAGCACUUCUUCAUCGUCGACCGGCUGAAGUCGCUGAUC
AAGUACAAGGGCUACCAGGUGGCGCCGGCCGAGCUGGAGAGCAUCCUGCUCCAGCACCCC
AACAUCUUCGACGCCGGCGUGGCCGGGCUGCCGGACGACGACGCCGGCGAGCUGCCGGCC
GCGGUGGUGGUGCUGGAGCACGGCAAGACCAUGACGGAGAAGGAGAUCGUCGACUACGUG
GCCAGCCAGGUGACCACCGCCAAGAAGCUGCGGGGCGGCGUGGUGUUCGUGGACGAGGUC
CCGAAGGGCCUGACCGGGAAGCUCGACGCCCGGAAGAUCCGCGAGAUCCUGAUCAAGGCC
AAGAAGGGCGGCAAGAUCGCCGUGUAAGACUAGUACCUGCUGCCUUAACGCUGAGAUGUG
GCCUCUGCAACCCCCCUUAGGCAAAGCAACUGAACCUUCUGCUAAAGUGACCUGCCCUCU
UCCGUAAGUCCAAUAAAGUUGUCAUGCACCCAGAUCUAAAAAAAAAAAAAAAAAAAAAAA
AAAAAAAAAAAAAAAAAAAAAAAAAAAAAAAAAAAAAAAAAUGCAUCCCCCCCCCCCCCC
CCCCCCCCCCCCCCCCAAAGGCUCUUUUCAGAGCCACCAGAAUU

Fig. 3

32L4 - PpLuc(GC) – gstm1– A64 - C30 - hSL (R3107)
(SEQ ID NO: 38):

GGGGCGCUGCCUACGGAGGUGGCAGCCAUCUCCUUCUCGGCAUCAAGCUUGAGGAUGGAG
GACGCCAAGAACAUCAAGAAGGGCCCGGCGCCCUUCUACCCGCUGGAGGACGGGACCGCC
GGCGAGCAGCUCCACAAGGCCAUGAAGCGGUACGCCCUGGUGCCGGGCACGAUCGCCUUC
ACCGACGCCCACAUCGAGGUCGACAUCACCUACGCGGAGUACUUCGAGAUGAGCGUGCGC
CUGGCCGAGGCCAUGAAGCGGUACGGCCUGAACACCAACCACCGGAUCGUGGUGUGCUCG
GAGAACAGCCUGCAGUUCUUCAUGCCGGUGCUGGGCGCCCUCUUCAUCGGCGUGGCCGUC
GCCCCGGCGAACGACAUCUACAACGAGCGGGAGCUGCUGAACAGCAUGGGGAUCAGCCAG
CCGACCGUGGUGUUCGUGAGCAAGAAGGGCCUGCAGAAGAUCCUGAACGUGCAGAAGAAG
CUGCCCAUCAUCCAGAAGAUCAUCAUCAUGGACAGCAAGACCGACUACCAGGGCUUCCAG
UCGAUGUACACGUUCGUGACCAGCCACCUCCCGCCGGGCUUCAACGAGUACGACUUCGUC
CCGGAGAGCUUCGACCGGGACAAGACCAUCGCCCUGAUCAUGAACAGCAGCGGCAGCACC
GGCCUGCCGAAGGGGGUGGCCCUGCCGCACCGGACCGCCUGCGUGCGCUUCUCGCACGCC
CGGGACCCCAUCUUCGGCAACCAGAUCAUCCCGGACACCGCCAUCCUGAGCGUGGUGCCG
UUCCACCACGGCUUCGGCAUGUUCACGACCCUGGGCUACCUCAUCUGCGGCUUCCGGGUG
GUCCUGAUGUACCGGUUCGAGGAGGAGCUGUUCCUGCGGAGCCUGCAGGACUACAAGAUC
CAGAGCGCGCUGCUCGUGCCGACCCUGUUCAGCUUCUUCGCCAAGAGCACCCUGAUCGAC
AAGUACGACCUGUCGAACCUGCACGAGAUCGCCAGCGGGGGCGCCCCGCUGAGCAAGGAG
GUGGGCGAGGCCGUGGCCAAGCGGUUCCACCUCCCGGGCAUCCGCCAGGGCUACGGCCUG
ACCGAGACCACGAGCGCGAUCCUGAUCACCCCCGAGGGGGACGACAAGCCGGGCGCCGUG
GGCAAGGUGGUCCCGUUCUUCGAGGCCAAGGUGGUGGACCUGGACACCGGCAAGACCCUG
GGCGUGAACCAGCGGGGCGAGCUGUGCGUGCGGGGGCCGAUGAUCAUGAGCGGCUACGUG
AACAACCCGGAGGCCACCAACGCCCUCAUCGACAAGGACGGCUGGCUGCACAGCGGCGAC
AUCGCCUACUGGGACGAGGACGAGCACUUCUUCAUCGUCGACCGGCUGAAGUCGCUGAUC
AAGUACAAGGGCUACCAGGUGGCGCCGGCCGAGCUGGAGAGCAUCCUGCUCCAGCACCCC
AACAUCUUCGACGCCGGCGUGGCCGGGCUGCCGGACGACGACGCCGGCGAGCUGCCGGCC
GCGGUGGUGGUGCUGGAGCACGGCAAGACCAUGACGGAGAAGGAGAUCGUCGACUACGUG
GCCAGCCAGGUGACCACCGCCAAGAAGCUGCGGGGCGGCGUGGUGUUCGUGGACGAGGUC
CCGAAGGGCCUGACCGGGAAGCUCGACGCCCGGAAGAUCCGCGAGAUCCUGAUCAAGGCC
AAGAAGGGCGGCAAGAUCGCCGUGUAAGACUAGUGCCCUUGCUACACGGGCACUCACUAG
GAGGACCUGUCCACACUGGGGAUCCUGCAGGCCCUGGGUGGGGACAGCACCCUGGCCUUC
UGCACUGUGGCUCCUGGUUCUCUCUCCUUCCCGCUCCCUUCUGCAGCUUGGUCAGCCCCA
UCUCCUCACCCUCUUCCCAGUCAAGUCCACACAGCCUUCAUUCUCCCCAGUUUCUUUCAC
AUGGCCCCUUCUUCAUUGGCUCCCUGACCCAACCUCACAGCCCGUUUCUGCGAACUGAGG
UCUGUCCUGAACUCACGCUUCCUAGAAUUACCCCGAUGGUCAACACUAUCUUAGUGCUAG
CCCUCCCUAGAGUUACCCCGAAGGUCAAUACUUGAGUGCCAGCCUGUUCCUGGUGGAGUA
GCCUCCCCAGGUCUGUCUCGUCUACAAUAAAGUCUGAAACACACUUGCCAUGAGAUCUAA
AAAAAAAAAAAAAAAAAAAAAAAAAAAAAAAAAAAAAAAAAAAAAAAAAAAAAAAAAAAA
AAUGCAUCCCCCCCCCCCCCCCCCCCCCCCCCCCCCCAAAGGCUCUUUUCAGAGCCACC
AGAAUU

Fig. 4

32L4 - PpLuc(GC) – ndufa1 – A64 - C30 - hSL (R3108)

(SEQ ID NO: 39):

GGGGCGCUGCCUACGGAGGUGGCAGCCAUCUCCUUCUCGGCAUCAAGCUUGAGG*AUGGAG*
*GACGCCAAGAACAUCAAGAAGGGCCCGGCGCCCUUCUACCCGCUGGAGGACGGGACCGCC*
*GGCGAGCAGCUCCACAAGGCCAUGAAGCGGUACGCCCUGGUGCCGGGCACGAUCGCCUUC*
*ACCGACGCCCACAUCGAGGUCGACAUCACCUACGCGGAGUACUUCGAGAUGAGCGUGCGC*
*CUGGCCGAGGCCAUGAAGCGGUACGGCCUGAACACCAACCACCGGAUCGUGGUGUGCUCG*
*GAGAACAGCCUGCAGUUCUUCAUGCCGGUGCUGGGCGCCCUCUUCAUCGGCGUGGCCGUC*
*GCCCCGGCGAACGACAUCUACAACGAGCGGGAGCUGCUGAACAGCAUGGGGAUCAGCCAG*
*CCGACCGUGGUGUUCGUGAGCAAGAAGGGCCUGCAGAAGAUCCUGAACGUGCAGAAGAAG*
*CUGCCCAUCAUCCAGAAGAUCAUCAUCAUGGACAGCAAGACCGACUACCAGGGCUUCCAG*
*UCGAUGUACACGUUCGUGACCAGCCACCUCCCGCCGGGCUUCAACGAGUACGACUUCGUC*
*CCGGAGAGCUUCGACCGGGACAAGACCAUCGCCCUGAUCAUGAACAGCAGCGGCAGCACC*
*GGCCUGCCGAAGGGGGUGGCCCUGCCGCACCGGACCGCCUGCGUGCGCUUCUCGCACGCC*
*CGGGACCCCAUCUUCGGCAACCAGAUCAUCCCGGACACCGCCAUCCUGAGCGUGGUGCCG*
*UUCCACCACGGCUUCGGCAUGUUCACGACCCUGGGCUACCUCAUCUGCGGCUUCCGGGUG*
*GUCCUGAUGUACCGGUUCGAGGAGGAGCUGUUCCUGCGGAGCCUGCAGGACUACAAGAUC*
*CAGAGCGCGCUGCUCGUGCCGACCCUGUUCAGCUUCUUCGCCAAGAGCACCCUGAUCGAC*
*AAGUACGACCUGUCGAACCUGCACGAGAUCGCCAGCGGGGGCGCCCCGCUGAGCAAGGAG*
*GUGGGCGAGGCCGUGGCCAAGCGGUUCCACCUCCCGGGCAUCCGCCAGGGCUACGGCCUG*
*ACCGAGACCACGAGCGCGAUCCUGAUCACCCCCGAGGGGGACGACAAGCCGGGCGCCGUG*
*GGCAAGGUGGUCCCGUUCUUCGAGGCCAAGGUGGUGGACCUGGACACCGGCAAGACCCUG*
*GGCGUGAACCAGCGGGGCGAGCUGUGCGUGCGGGGGCCGAUGAUCAUGAGCGGCUACGUG*
*AACAACCCGGAGGCCACCAACGCCCUCAUCGACAAGGACGGCUGGCUGCACAGCGGCGAC*
*AUCGCCUACUGGGACGAGGACGAGCACUUCUUCAUCGUCGACCGGCUGAAGUCGCUGAUC*
*AAGUACAAGGGCUACCAGGUGGCGCCGGCCGAGCUGGAGAGCAUCCUGCUCCAGCACCCC*
*AACAUCUUCGACGCCGGCGUGGCCGGGCUGCCGGACGACGACGCCGGCGAGCUGCCGGCC*
*GCGGUGGUGGUGCUGGAGCACGGCAAGACCAUGACGGAGAAGGAGAUCGUCGACUACGUG*
*GCCAGCCAGGUGACCACCGCCAAGAAGCUGCGGGGCGGCGUGGUGUUCGUGGACGAGGUC*
*CCGAAGGGCCUGACCGGGAAGCUCGACGCCCGGAAGAUCCGCGAGAUCCUGAUCAAGGCC*
*AAGAAGGGCGGCAAGAUCGCCGUGUAA*GACUAGUGGAAGCAUUUUCCUGGCUGAUUAAAA
GAAAUUACUCAGCUAUGGUCAUCUGUUCCUGUUAGAAGGCUAUGCAGCAUAUUAUAUACU
AUGCGCAUGUUAUGAAAUGCAUAAUAAAAAAUUUUAAAAAAUCUAAAAGAUCUAAAAAAA
AAAAAAAAAAAAAAAAAAAAAAAAAAAAAAAAAAAAAAAAAAAAAAAAAAAAAAAAAUGC
AUCCCCCCCCCCCCCCCCCCCCCCCCCCCCCCCAAAGGCUCUUUUCAGAGCCACCAGAAU
U

Fig. 5

32L4 - PpLuc(GC) – cbr2 – A64 - C30 - hSL (R3109)

(SEQ ID NO: 40):

GGGGCGCUGCCUACGGAGGUGGCAGCCAUCUCCUUCUCGGCAUCAAGCUUGAGG*AUGGAG*
*GACGCCAAGAACAUCAAGAAGGGCCCGGCGCCCUUCUACCCGCUGGAGGACGGGACCGCC*
*GGCGAGCAGCUCCACAAGGCCAUGAAGCGGUACGCCCUGGUGCCGGGCACGAUCGCCUUC*
*ACCGACGCCCACAUCGAGGUCGACAUCACCUACGCGGAGUACUUCGAGAUGAGCGUGCGC*
*CUGGCCGAGGCCAUGAAGCGGUACGGCCUGAACACCAACCACCGGAUCGUGGUGUGCUCG*
*GAGAACAGCCUGCAGUUCUUCAUGCCGGUGCUGGGCGCCCUCUUCAUCGGCGUGGCCGUC*
*GCCCCGGCGAACGACAUCUACAACGAGCGGGAGCUGCUGAACAGCAUGGGGAUCAGCCAG*
*CCGACCGUGGUGUUCGUGAGCAAGAAGGGCCUGCAGAAGAUCCUGAACGUGCAGAAGAAG*
*CUGCCCAUCAUCCAGAAGAUCAUCAUCAUGGACAGCAAGACCGACUACCAGGGCUUCCAG*
*UCGAUGUACACGUUCGUGACCAGCCACCUCCCGCCGGGCUUCAACGAGUACGACUUCGUC*
*CCGGAGAGCUUCGACCGGGACAAGACCAUCGCCCUGAUCAUGAACAGCAGCGGCAGCACC*
*GGCCUGCCGAAGGGGGUGGCCCUGCCGCACCGGACCGCCUGCGUGCGCUUCUCGCACGCC*
*CGGGACCCCAUCUUCGGCAACCAGAUCAUCCCGGACACCGCCAUCCUGAGCGUGGUGCCG*
*UUCCACCACGGCUUCGGCAUGUUCACGACCCUGGGCUACCUCAUCUGCGGCUUCCGGGUG*
*GUCCUGAUGUACCGGUUCGAGGAGGAGCUGUUCCUGCGGAGCCUGCAGGACUACAAGAUC*
*CAGAGCGCGCUGCUCGUGCCGACCCUGUUCAGCUUCUUCGCCAAGAGCACCCUGAUCGAC*
*AAGUACGACCUGUCGAACCUGCACGAGAUCGCCAGCGGGGGCGCCCCGCUGAGCAAGGAG*
*GUGGGCGAGGCCGUGGCCAAGCGGUUCCACCUCCCGGGCAUCCGCCAGGGCUACGGCCUG*
*ACCGAGACCACGAGCGCGAUCCUGAUCACCCCCGAGGGGGACGACAAGCCGGGCGCCGUG*
*GGCAAGGUGGUCCCGUUCUUCGAGGCCAAGGUGGUGGACCUGGACACCGGCAAGACCCUG*
*GGCGUGAACCAGCGGGGCGAGCUGUGCGUGCGGGGGCCGAUGAUCAUGAGCGGCUACGUG*
*AACAACCCGGAGGCCACCAACGCCCUCAUCGACAAGGACGGCUGGCUGCACAGCGGCGAC*
*AUCGCCUACUGGGACGAGGACGAGCACUUCUUCAUCGUCGACCGGCUGAAGUCGCUGAUC*
*AAGUACAAGGGCUACCAGGUGGCGCCGGCCGAGCUGGAGAGCAUCCUGCUCCAGCACCCC*
*AACAUCUUCGACGCCGGCGUGGCCGGGCUGCCGGACGACGACGCCGGCGAGCUGCCGGCC*
*GCGGUGGUGGUGCUGGAGCACGGCAAGACCAUGACGGAGAAGGAGAUCGUCGACUACGUG*
*GCCAGCCAGGUGACCACCGCCAAGAAGCUGCGGGGCGGCGUGGUGUUCGUGGACGAGGUC*
*CCGAAGGGCCUGACCGGGAAGCUCGACGCCCGGAAGAUCCGCGAGAUCCUGAUCAAGGCC*
*AAGAAGGGCGGCAAGAUCGCCGUGUAA*GACUAGUUCUGCUCAGUUGCCGCGGACAUCUGA
GUGGCCUUCUUAGCCCCACCCUCAGCCAAAGCAUUUACUGAUCUCGUGACUCCGCCCUCA
UGCUACAGCCACGCCCACCACGCAGCUCACAGUUCCACCCCCAUGUUACUGUCGAUCCCA
CAACCACUCCAGGCGCAGACCUUGUUCUCUUUGUCCACUUUGUUGGGCUCAUUUGCCUAA
AUAAACGGCCACCGCGUUACCUUUAACUAUAGAUCUAAAAAAAAAAAAAAAAAAAAAAAA
AAAAAAAAAAAAAAAAAAAAAAAAAAAAAAAAAAAAAAAAUGCAUCCCCCCCCCCCCCCC
CCCCCCCCCCCCCCCCAAAGGCUCUUUCAGAGCCACCAGAAUU

Fig. 6

PpLuc(GC) - albumin7– A64 - C30 - hSL (R2463)

(SEQ ID NO: 41):

GGGAGAAAGCUUGAGGAUGGAGGACGCCAAGAACAUCAAGAAGGGCCCGGCGCCCUUCUA
CCCGCUGGAGGACGGGACCGCCGGCGAGCAGCUCCACAAGGCCAUGAAGCGGUACGCCCU
GGUGCCGGGCACGAUCGCCUUCACCGACGCCCACAUCGAGGUCGACAUCACCUACGCGGA
GUACUUCGAGAUGAGCGUGCGCCUGGCCGAGGCCAUGAAGCGGUACGGCCUGAACACCAA
CCACCGGAUCGUGGUGUGCUCGGAGAACAGCCUGCAGUUCUUCAUGCCGGUGCUGGGCGC
CCUCUUCAUCGGCGUGGCCGUCGCCCCGGCGAACGACAUCUACAACGAGCGGGAGCUGCU
GAACAGCAUGGGGAUCAGCCAGCCGACCGUGGUGUUCGUGAGCAAGAAGGGCCUGCAGAA
GAUCCUGAACGUGCAGAAGAAGCUGCCCAUCAUCCAGAAGAUCAUCAUCAUGGACAGCAA
GACCGACUACCAGGGCUUCCAGUCGAUGUACACGUUCGUGACCAGCCACCUCCCGCCGGG
CUUCAACGAGUACGACUUCGUCCCGGAGAGCUUCGACCGGGACAAGACCAUCGCCCUGAU
CAUGAACAGCAGCGGCAGCACCGGCCUGCCGAAGGGGGUGGCCCUGCCGCACCGGACCGC
CUGCGUGCGCUUCUCGCACGCCCGGGACCCCAUCUUCGGCAACCAGAUCAUCCCGGACAC
CGCCAUCCUGAGCGUGGUGCCGUUCCACCACGGCUUCGGCAUGUUCACGACCCUGGGCUA
CCUCAUCUGCGGCUUCCGGGUGGUCCUGAUGUACCGGUUCGAGGAGGAGCUGUUCCUGCG
GAGCCUGCAGGACUACAAGAUCCAGAGCGCGCUGCUCGUGCCGACCCUGUUCAGCUUCUU
CGCCAAGAGCACCCUGAUCGACAAGUACGACCUGUCGAACCUGCACGAGAUCGCCAGCGG
GGGCGCCCCGCUGAGCAAGGAGGUGGGCGAGGCCGUGGCCAAGCGGUUCCACCUCCCGGG
CAUCCGCCAGGGCUACGGCCUGACCGAGACCACGAGCGCGAUCCUGAUCACCCCCGAGGG
GGACGACAAGCCGGGCGCCGUGGGCAAGGUGGUCCCGUUCUUCGAGGCCAAGGUGGUGGA
CCUGGACACCGGCAAGACCCUGGGCGUGAACCAGCGGGGCGAGCUGUGCGUGCGGGGGCC
GAUGAUCAUGAGCGGCUACGUGAACAACCCGGAGGCCACCAACGCCCUCAUCGACAAGGA
CGGCUGGCUGCACAGCGGCGACAUCGCCUACUGGGACGAGGACGAGCACUUCUUCAUCGU
CGACCGGCUGAAGUCGCUGAUCAAGUACAAGGGCUACCAGGUGGCGCCGGCCGAGCUGGA
GAGCAUCCUGCUCCAGCACCCCAACAUCUUCGACGCCGGCGUGGCCGGGCUGCCGGACGA
CGACGCCGGCGAGCUGCCGGCCGCGGUGGUGGUGCUGGAGCACGGCAAGACCAUGACGGA
GAAGGAGAUCGUCGACUACGUGGCCAGCCAGGUGACCACCGCCAAGAAGCUGCGGGGCGG
CGUGGUGUUCGUGGACGAGGUCCCGAAGGGCCUGACCGGGAAGCUCGACGCCCGGAAGAU
CCGCGAGAUCCUGAUCAAGGCCAAGAAGGGCGGCAAGAUCGCCGUGUAAGACUAGUGCAU
CACAUUUAAAAGCAUCUCAGCCUACCAUGAGAAUAAGAGAAAGAAAAUGAAGAUCAAUAG
CUUAUUCAUCUCUUUUUCUUUUUCGUUGGUGUAAAGCCAACACCCUGUCUAAAAAACAUA
AAUUUCUUUAAUCAUUUUGCCUCUUUUCUCUGUGCUUCAAUUAAUAAAAAAUGGAAAGAA
CCUAGAUCUAAAAAAAAAAAAAAAAAAAAAAAAAAAAAAAAAAAAAAAAAAAAAAAAAAA
AAAAAAAAAAAAAUGCAUCCCCCCCCCCCCCCCCCCCCCCCCCCCCCCAAAGGCUCUUU
UCAGAGCCACCAGAAUU

Fig. 7

Mp68 - PpLuc(GC) - albumin7– A64 - C30 - hSL (R3111)

(SEQ ID NO: 42):

GGGCUUUCCCAUUCUGUAGCAGAAUUUGGUGUUGCCUGUGGUCUUGGUCCCGCGGAGAAG
CUUGAGGAUGGAGGACGCCAAGAACAUCAAGAAGGGCCCGGCGCCCUUCUACCCGCUGGA
GGACGGGACCGCCGGCGAGCAGCUCCACAAGGCCAUGAAGCGGUACGCCCUGGUGCCGGG
CACGAUCGCCUUCACCGACGCCCACAUCGAGGUCGACAUCACCUACGCGGAGUACUUCGA
GAUGAGCGUGCGCCUGGCCGAGGCCAUGAAGCGGUACGGCCUGAACACCAACCACCGGAU
CGUGGUGUGCUCGGAGAACAGCCUGCAGUUCUUCAUGCCGGUGCUGGGCGCCCUCUUCAU
CGGCGUGGCCGUCGCCCCGGCGAACGACAUCUACAACGAGCGGGAGCUGCUGAACAGCAU
GGGGAUCAGCCAGCCGACCGUGGUGUUCGUGAGCAAGAAGGGCCUGCAGAAGAUCCUGAA
CGUGCAGAAGAAGCUGCCCAUCAUCCAGAAGAUCAUCAUCAUGGACAGCAAGACCGACUA
CCAGGGCUUCCAGUCGAUGUACACGUUCGUGACCAGCCACCUCCCGCCGGGCUUCAACGA
GUACGACUUCGUCCCGGAGAGCUUCGACCGGGACAAGACCAUCGCCCUGAUCAUGAACAG
CAGCGGCAGCACCGGCCUGCCGAAGGGGGUGGCCCUGCCGCACCGGACCGCCUGCGUGCG
CUUCUCGCACGCCCGGGACCCCAUCUUCGGCAACCAGAUCAUCCCGGACACCGCCAUCCU
GAGCGUGGUGCCGUUCCACCACGGCUUCGGCAUGUUCACGACCCUGGGCUACCUCAUCUG
CGGCUUCCGGGUGGUCCUGAUGUACCGGUUCGAGGAGGAGCUGUUCCUGCGGAGCCUGCA
GGACUACAAGAUCCAGAGCGCGCUGCUCGUGCCGACCCUGUUCAGCUUCUUCGCCAAGAG
CACCCUGAUCGACAAGUACGACCUGUCGAACCUGCACGAGAUCGCCAGCGGGGGCGCCCC
GCUGAGCAAGGAGGUGGGCGAGGCCGUGGCCAAGCGGUUCCACCUCCCGGGCAUCCGCCA
GGGCUACGGCCUGACCGAGACCACGAGCGCGAUCCUGAUCACCCCCGAGGGGGACGACAA
GCCGGGCGCCGUGGGCAAGGUGGUCCCGUUCUUCGAGGCCAAGGUGGUGGACCUGGACAC
CGGCAAGACCCUGGGCGUGAACCAGCGGGGCGAGCUGUGCGUGCGGGGGCCGAUGAUCAU
GAGCGGCUACGUGAACAACCCGGAGGCCACCAACGCCCUCAUCGACAAGGACGGCUGGCU
GCACAGCGGCGACAUCGCCUACUGGGACGAGGACGAGCACUUCUUCAUCGUCGACCGGCU
GAAGUCGCUGAUCAAGUACAAGGGCUACCAGGUGGCGCCGGCCGAGCUGGAGAGCAUCCU
GCUCCAGCACCCCAACAUCUUCGACGCCGGCGUGGCCGGGCUGCCGGACGACGACGCCGG
CGAGCUGCCGGCCGCGGUGGUGGUGCUGGAGCACGGCAAGACCAUGACGGAGAAGGAGAU
CGUCGACUACGUGGCCAGCCAGGUGACCACCGCCAAGAAGCUGCGGGGCGGCGUGGUGUU
CGUGGACGAGGUCCCGAAGGGCCUGACCGGGAAGCUCGACGCCCGGAAGAUCCGCGAGAU
CCUGAUCAAGGCCAAGAAGGGCGGCAAGAUCGCCGUGUAAGACUAGUGCAUCACAUUUAA
AAGCAUCUCAGCCUACCAUGAGAAUAAGAGAAAGAAAAUGAAGAUCAAUAGCUUAUUCAU
CUCUUUUUCUUUUUCGUUGGUGUAAAGCCAACACCCUGUCUAAAAAACAUAAAUUUCUUU
AAUCAUUUUGCCUCUUUUCUCUGUGCUUCAAUUAAUAAAAAAUGGAAAGAACCUAGAUCU
AAAAAAAAAAAAAAAAAAAAAAAAAAAAAAAAAAAAAAAAAAAAAAAAAAAAAAAAAAAA
AAAAUGCAUCCCCCCCCCCCCCCCCCCCCCCCCCCCCCCCAAAGGCUCUUUUCAGAGCCA
CCAGAAUU

Fig. 8

Ndufa4 - PpLuc(GC) - albumin7– A64 - C30 - hSL (R3112)

(SEQ ID NO: 43):

GGGGUCCGCUCAGCCAGGUUGCAGAAGCGGCUUAGCGUGUGUCCUAAUCUUCUCUCUGCG
UGUAGGUAGGCCUGUGCCGCAAACAAGCUUGAGGAUGGAGGACGCCAAGAACAUCAAGAA
GGGCCCGGCGCCCUUCUACCCGCUGGAGGACGGGACCGCCGGCGAGCAGCUCCACAAGGC
CAUGAAGCGGUACGCCCUGGUGCCGGGCACGAUCGCCUUCACCGACGCCCACAUCGAGGU
CGACAUCACCUACGCGGAGUACUUCGAGAUGAGCGUGCGCCUGGCCGAGGCCAUGAAGCG
GUACGGCCUGAACACCAACCACCGGAUCGUGGUGUGCUCGGAGAACAGCCUGCAGUUCUU
CAUGCCGGUGCUGGGCGCCCUCUUCAUCGGCGUGGCCGUCGCCCCGGCGAACGACAUCUA
CAACGAGCGGGAGCUGCUGAACAGCAUGGGGAUCAGCCAGCCGACCGUGGUGUUCGUGAG
CAAGAAGGGCCUGCAGAAGAUCCUGAACGUGCAGAAGAAGCUGCCCAUCAUCCAGAAGAU
CAUCAUCAUGGACAGCAAGACCGACUACCAGGGCUUCCAGUCGAUGUACACGUUCGUGAC
CAGCCACCUCCCGCCGGGCUUCAACGAGUACGACUUCGUCCCGGAGAGCUUCGACCGGGA
CAAGACCAUCGCCCUGAUCAUGAACAGCAGCGGCAGCACCGGCCUGCCGAAGGGGGUGGC
CCUGCCGCACCGGACCGCCUGCGUGCGCUUCUCGCACGCCCGGGACCCCAUCUUCGGCAA
CCAGAUCAUCCCGGACACCGCCAUCCUGAGCGUGGUGCCGUUCCACCACGGCUUCGGCAU
GUUCACGACCCUGGGCUACCUCAUCUGCGGCUUCCGGGUGGUCCUGAUGUACCGGUUCGA
GGAGGAGCUGUUCCUGCGGAGCCUGCAGGACUACAAGAUCCAGAGCGCGCUGCUCGUGCC
GACCCUGUUCAGCUUCUUCGCCAAGAGCACCCUGAUCGACAAGUACGACCUGUCGAACCU
GCACGAGAUCGCCAGCGGGGGCGCCCCGCUGAGCAAGGAGGUGGGCGAGGCCGUGGCCAA
GCGGUUCCACCUCCCGGGCAUCCGCCAGGGCUACGGCCUGACCGAGACCACGAGCGCGAU
CCUGAUCACCCCCGAGGGGGACGACAAGCCGGGCGCCGUGGGCAAGGUGGUCCCGUUCUU
CGAGGCCAAGGUGGUGGACCUGGACACCGGCAAGACCCUGGGCGUGAACCAGCGGGGCGA
GCUGUGCGUGCGGGGGCCGAUGAUCAUGAGCGGCUACGUGAACAACCCGGAGGCCACCAA
CGCCCUCAUCGACAAGGACGGCUGGCUGCACAGCGGCGACAUCGCCUACUGGGACGAGGA
CGAGCACUUCUUCAUCGUCGACCGGCUGAAGUCGCUGAUCAAGUACAAGGGCUACCAGGU
GGCGCCGGCCGAGCUGGAGAGCAUCCUGCUCCAGCACCCCAACAUCUUCGACGCCGGCGU
GGCCGGGCUGCCGGACGACGACGCCGGCGAGCUGCCGGCCGCGGUGGUGGUGCUGGAGCA
CGGCAAGACCAUGACGGAGAAGGAGAUCGUCGACUACGUGGCCAGCCAGGUGACCACCGC
CAAGAAGCUGCGGGGCGGCGUGGUGUUCGUGGACGAGGUCCCGAAGGGCCUGACCGGGAA
GCUCGACGCCCGGAAGAUCCGCGAGAUCCUGAUCAAGGCCAAGAAGGGCGGCAAGAUCGC
CGUGUAAGACUAGUGCAUCACAUUUAAAAGCAUCUCAGCCUACCAUGAGAAUAAGAGAAA
GAAAAUGAAGAUCAAUAGCUUAUUCAUCUCUUUUUCUUUUUCGUUGGUGUAAAGCCAACA
CCCUGUCUAAAAAACAUAAAUUUCUUUAAUCAUUUUGCCUCUUUUCUCUGUGCUUCAAUU
AAUAAAAAAUGGAAAGAACCUAGAUCUAAAAAAAAAAAAAAAAAAAAAAAAAAAAAAAAAA
AAAAAAAAAAAAAAAAAAAAAAAAAAAAAAUGCAUCCCCCCCCCCCCCCCCCCCCCCC
CCCCCCCAAAGGCUCUUUUCAGAGCCACCAGAAUU

Fig. 9

PpLuc(GC) – A64 - C30 - hSL (R2462)

(SEQ ID NO: 44):

GGGAGAAAGCTTGAGGATGGAGGACGCCAAGAACATCAAGAAGGGCCCGGCGCCCTTCTA
CCCGCTGGAGGACGGGACCGCCGGCGAGCAGCTCCACAAGGCCATGAAGCGGTACGCCCT
GGTGCCGGGCACGATCGCCTTCACCGACGCCCACATCGAGGTCGACATCACCTACGCGGA
GTACTTCGAGATGAGCGTGCGCCTGGCCGAGGCCATGAAGCGGTACGGCCTGAACACCAA
CCACCGGATCGTGGTGTGCTCGGAGAACAGCCTGCAGTTCTTCATGCCGGTGCTGGGCGC
CCTCTTCATCGGCGTGGCCGTCGCCCCGGCGAACGACATCTACAACGAGCGGGAGCTGCT
GAACAGCATGGGGATCAGCCAGCCGACCGTGGTGTTCGTGAGCAAGAAGGGCCTGCAGAA
GATCCTGAACGTGCAGAAGAAGCTGCCCATCATCCAGAAGATCATCATCATGGACAGCAA
GACCGACTACCAGGGCTTCCAGTCGATGTACACGTTCGTGACCAGCCACCTCCCGCCGGG
CTTCAACGAGTACGACTTCGTCCCGGAGAGCTTCGACCGGGACAAGACCATCGCCCTGAT
CATGAACAGCAGCGGCAGCACCGGCCTGCCGAAGGGGGTGGCCCTGCCGCACCGGACCGC
CTGCGTGCGCTTCTCGCACGCCCGGGACCCCATCTTCGGCAACCAGATCATCCCGGACAC
CGCCATCCTGAGCGTGGTGCCGTTCCACCACGGCTTCGGCATGTTCACGACCCTGGGCTA
CCTCATCTGCGGCTTCCGGGTGGTCCTGATGTACCGGTTCGAGGAGGAGCTGTTCCTGCG
GAGCCTGCAGGACTACAAGATCCAGAGCGCGCTGCTCGTGCCGACCCTGTTCAGCTTCTT
CGCCAAGAGCACCCTGATCGACAAGTACGACCTGTCGAACCTGCACGAGATCGCCAGCGG
GGGCGCCCCGCTGAGCAAGGAGGTGGGCGAGGCCGTGGCCAAGCGGTTCCACCTCCCGGG
CATCCGCCAGGGCTACGGCCTGACCGAGACCACGAGCGCGATCCTGATCACCCCCGAGGG
GGACGACAAGCCGGGCGCCGTGGGCAAGGTGGTCCCGTTCTTCGAGGCCAAGGTGGTGGA
CCTGGACACCGGCAAGACCCTGGGCGTGAACCAGCGGGGCGAGCTGTGCGTGCGGGGGCC
GATGATCATGAGCGGCTACGTGAACAACCCGGAGGCCACCAACGCCCTCATCGACAAGGA
CGGCTGGCTGCACAGCGGCGACATCGCCTACTGGGACGAGGACGAGCACTTCTTCATCGT
CGACCGGCTGAAGTCGCTGATCAAGTACAAGGGCTACCAGGTGGCGCCGGCCGAGCTGGA
GAGCATCCTGCTCCAGCACCCCAACATCTTCGACGCCGGCGTGGCCGGGCTGCCGGACGA
CGACGCCGGCGAGCTGCCGGCCGCGGTGGTGGTGCTGGAGCACGGCAAGACCATGACGGA
GAAGGAGATCGTCGACTACGTGGCCAGCCAGGTGACCACCGCCAAGAAGCTGCGGGGCGG
CGTGGTGTTCGTGGACGAGGTCCCGAAGGGCCTGACCGGGAAGCTCGACGCCCGGAAGAT
CCGCGAGATCCTGATCAAGGCCAAGAAGGGCGGCAAGATCGCCGTGTAAGACTAGTAGAT
CTAAAAAAAAAAAAAAAAAAAAAAAAAAAAAAAAAAAAAAAAAAAAAAAAAAAAAAAAAA
AAAAAATGCATCCCCCCCCCCCCCCCCCCCCCCCCCCCCCCCAAAGGCTCTTTTCAGAGC
CACCAGAATT

Fig. 10

PpLuc(GC) – gnas- A64 - C30 – hSL (R3116)
(SEQ ID NO: 45):

GGGAGAAAGCUUGAGG*AUGGAGGACGCCAAGAACAUCAAGAAGGGCCCGGCGCCCUUCUA*
CCCGCUGGAGGACGGGACCGCCGGCGAGCAGCUCCACAAGGCCAUGAAGCGGUACGCCCU
GGUGCCGGGCACGAUCGCCUUCACCGACGCCCACAUCGAGGUCGACAUCACCUACGCGGA
GUACUUCGAGAUGAGCGUGCGCCUGGCCGAGGCCAUGAAGCGGUACGGCCUGAACACCAA
CCACCGGAUCGUGGUGUGCUCGGAGAACAGCCUGCAGUUCUUCAUGCCGGUGCUGGGCGC
CCUCUUCAUCGGCGUGGCCGUCGCCCCGGCGAACGACAUCUACAACGAGCGGGAGCUGCU
GAACAGCAUGGGGAUCAGCCAGCCGACCGUGGUGUUCGUGAGCAAGAAGGGCCUGCAGAA
GAUCCUGAACGUGCAGAAGAAGCUGCCCAUCAUCCAGAAGAUCAUCAUCAUGGACAGCAA
GACCGACUACCAGGGCUUCCAGUCGAUGUACACGUUCGUGACCAGCCACCUCCCGCCGGG
CUUCAACGAGUACGACUUCGUCCCGGAGAGCUUCGACCGGGACAAGACCAUCGCCCUGAU
CAUGAACAGCAGCGGCAGCACCGGCCUGCCGAAGGGGGUGGCCCUGCCGCACCGGACCGC
CUGCGUGCGCUUCUCGCACGCCCGGGACCCCAUCUUCGGCAACCAGAUCAUCCCGGACAC
CGCCAUCCUGAGCGUGGUGCCGUUCCACCACGGCUUCGGCAUGUUCACGACCCUGGGCUA
CCUCAUCUGCGGCUUCCGGGUGGUCCUGAUGUACCGGUUCGAGGAGGAGCUGUUCCUGCG
GAGCCUGCAGGACUACAAGAUCCAGAGCGCGCUGCUCGUGCCGACCCUGUUCAGCUUCUU
CGCCAAGAGCACCCUGAUCGACAAGUACGACCUGUCGAACCUGCACGAGAUCGCCAGCGG
GGGCGCCCCGCUGAGCAAGGAGGUGGGCGAGGCCGUGGCCAAGCGGUUCCACCUCCCGGG
CAUCCGCCAGGGCUACGGCCUGACCGAGACCACGAGCGCGAUCCUGAUCACCCCCGAGGG
*GGACGACAAGCCGGGCGCCGUGGGCAAGGUGGUCCCGUUCUUCGAGGCCAAGGUGGUGGA*
*CCUGGACACCGGCAAGACCCUGGGCGUGAACCAGCGGGGCGAGCUGUGCGUGCGGGGGCC*
*GAUGAUCAUGAGCGGCUACGUGAACAACCCGGAGGCCACCAACGCCCUCAUCGACAAGGA*
*CGGCUGGCUGCACAGCGGCGACAUCGCCUACUGGGACGAGGACGAGCACUUCUUCAUCGU*
*CGACCGGCUGAAGUCGCUGAUCAAGUACAAGGGCUACCAGGUGGCGCCGGCCGAGCUGGA*
*GAGCAUCCUGCUCCAGCACCCCAACAUCUUCGACGCCGGCGUGGCCGGGCUGCCGGACGA*
*CGACGCCGGCGAGCUGCCGGCCGCGGUGGUGGUGCUGGAGCACGGCAAGACCAUGACGGA*
*GAAGGAGAUCGUCGACUACGUGGCCAGCCAGGUGACCACCGCCAAGAAGCUGCGGGGCGG*
*CGUGGUGUUCGUGGACGAGGUCCCGAAGGGCCUGACCGGGAAGCUCGACGCCCGGAAGAU*
*CCGCGAGAUCCUGAUCAAGGCCAAGAAGGGCGGCAAGAUCGCCGUGUAA*GACUAGUGAAG
GGAACACCCAAAUUUAAUUCAGCCUUAAGCACAAUUAAUUAAGAGUGAAACGUAAUUGUA
CAAGCAGUUGGUCACCCACCAUAGGGCAUGAUCAACACCGCAACCUUUCCUUUUUCCCCC
AGUGAUUCUGAAAAACCCCUCUUCCCUUCAGCUUGCUUAGAUGUUCCAAAUUUAGUAAGC
UUAAGGCGGCCUACAGAAGAAAAAGAAAAAAAAGGCCACAAAAGUUCCCUCUCACUUUCA
GUAAAUAAAAUAAAAGCAGCAACAGAAAUAAAGAAAUAAAUGAAAUUCAAAAUGAAAUAA
AUAUUGUGUUGUGCAGCAUUAAAAAAUCAAUAAAAAUUAAAAAUGAGCAAGAUCUAAAAA
AAAAAAAAAAAAAAAAAAAAAAAAAAAAAAAAAAAAAAAAAAAAAAAAAAAAAAAAAAAU
GCAUCCCCCCCCCCCCCCCCCCCCCCCCCCCCCCAAAGGCUCUUUUCAGAGCCACCAGA
AUU

Fig. 11

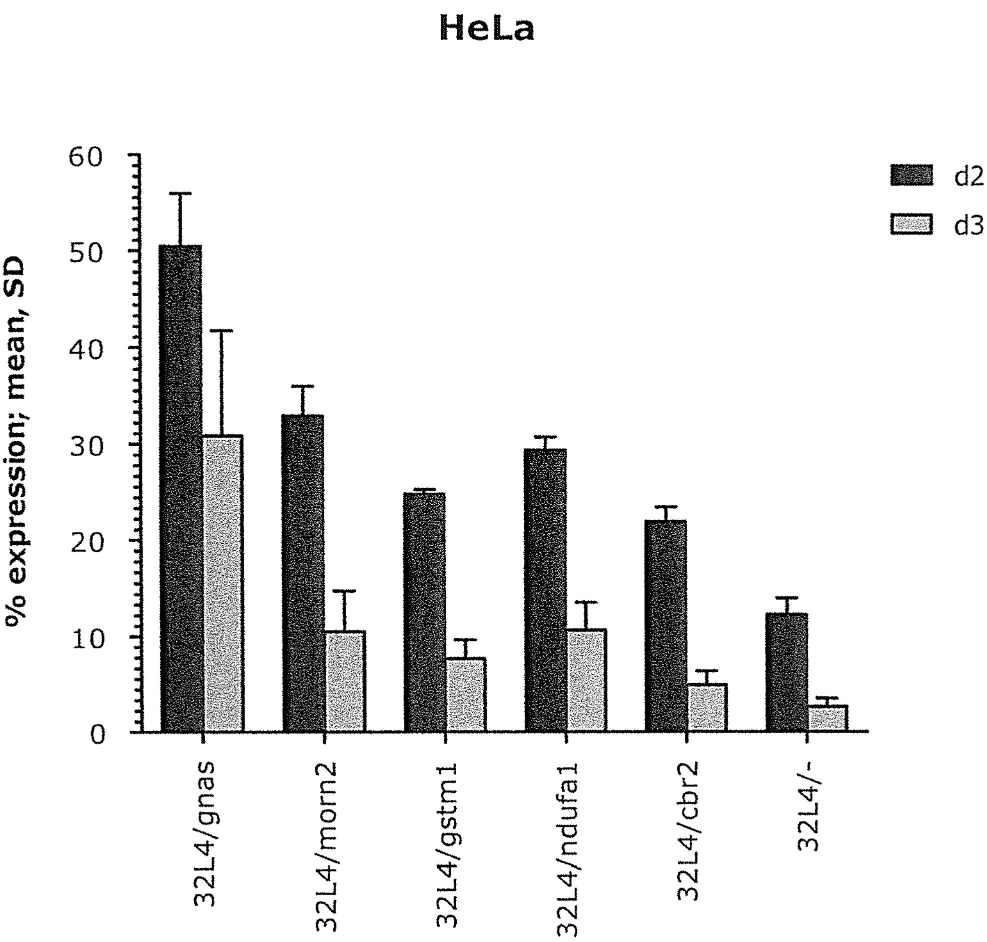
HeLa
% expression; mean, SD
60
50
40
30
20
10
0
d2
d3
32L4/gnas
32L4/morn2
32L4/gstm1
32L4/ndufa1
32L4/cbr2
32L4/-

Fig. 12

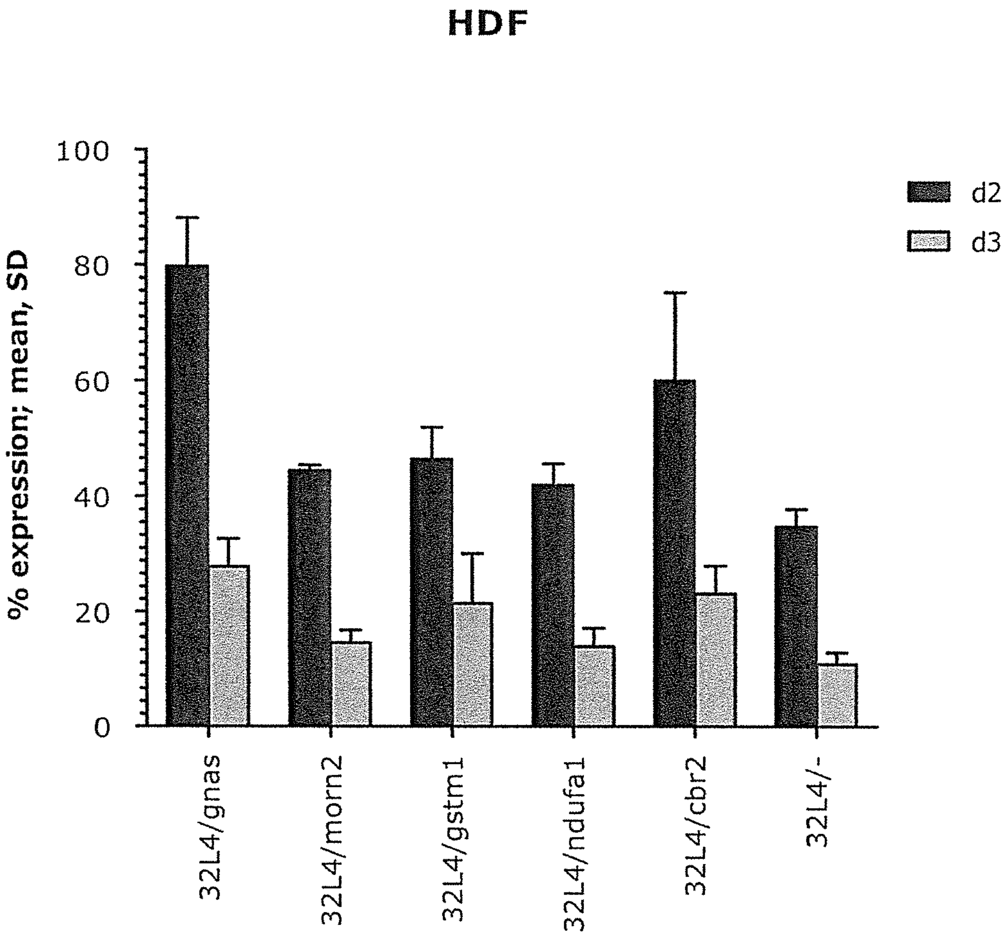
HDF
% expression; mean, SD
100
80
60
40
20
0
d2
d3
32L4/gnas
32L4/morn2
32L4/gstm1
32L4/ndufa1
32L4/cbr2
32L4/-

Fig. 13

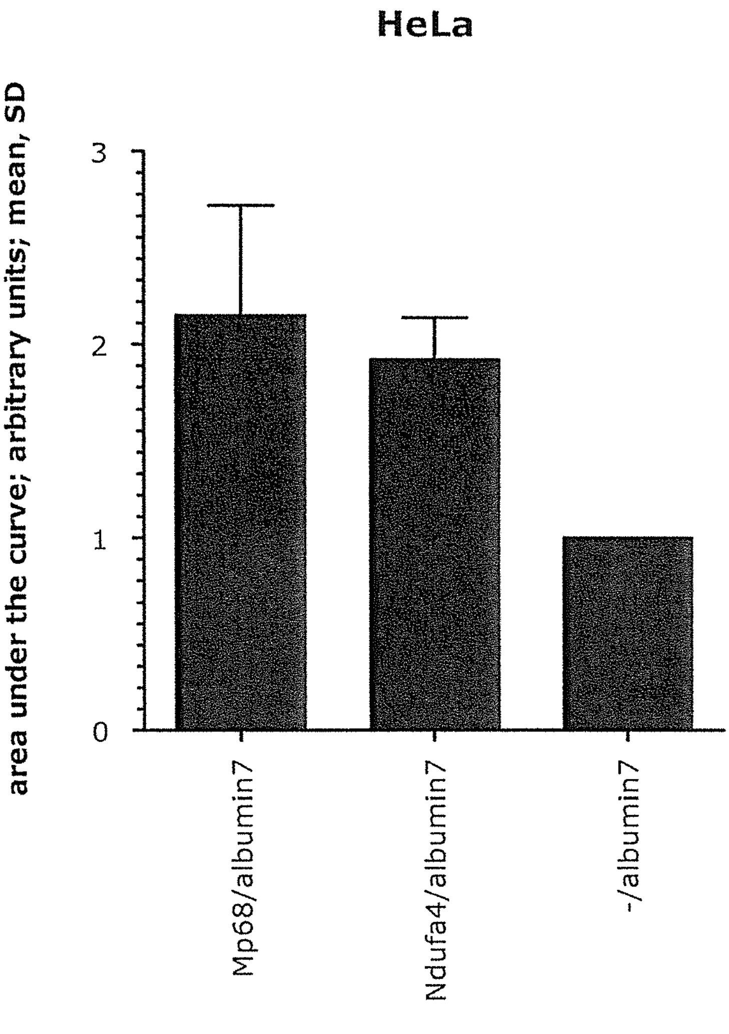
HeLa
area under the curve; arbitrary units; mean, SD
3
2
1
0
Mp68/albumin7
Ndufa4/albumin7
-/albumin7

Fig. 14

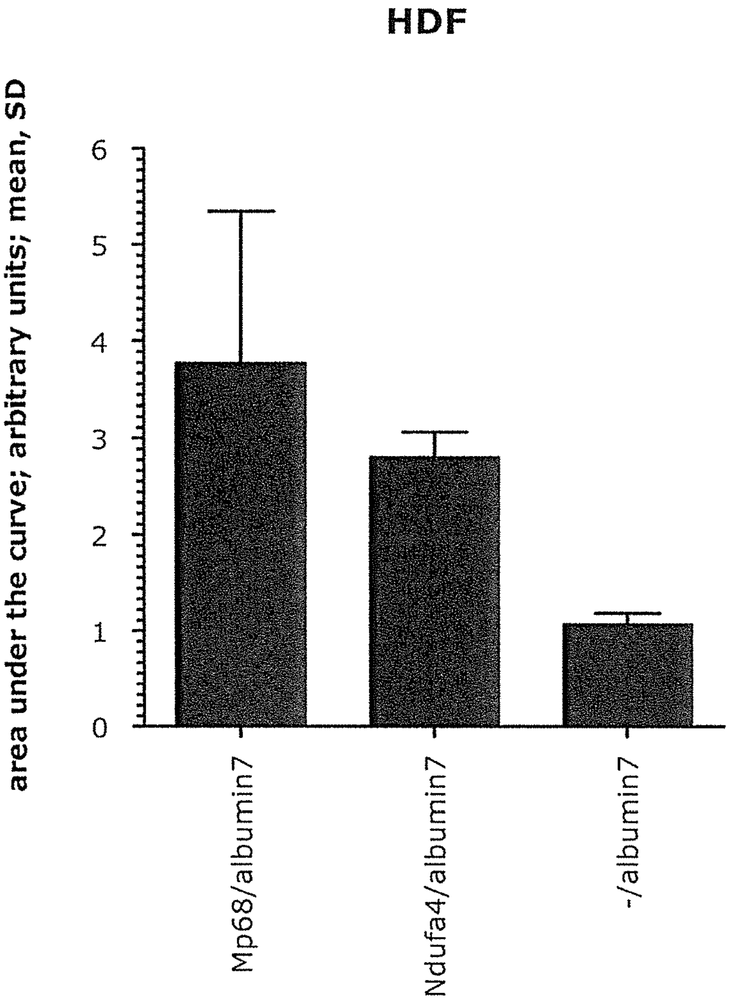
HDF
area under the curve; arbitrary units; mean, SD
6
5
4
3
2
1
0
Mp68/albumin7
Ndufa4/albumin7
-/albumin7

Fig. 15

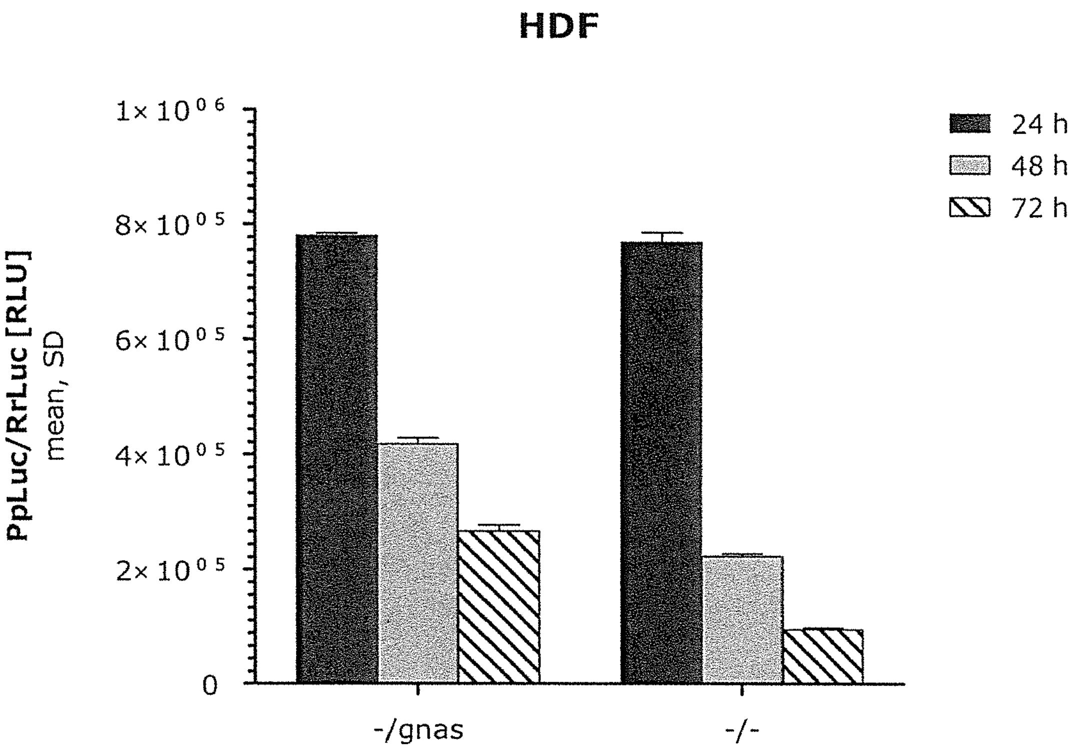
HDF
PpLuc/RrLuc [RLU]
mean, SD
1×10^06
8×10^05
6×10^05
4×10^05
2×10^05
0
24 h
48 h
72 h
-/gnas
-/-

Fig. 16

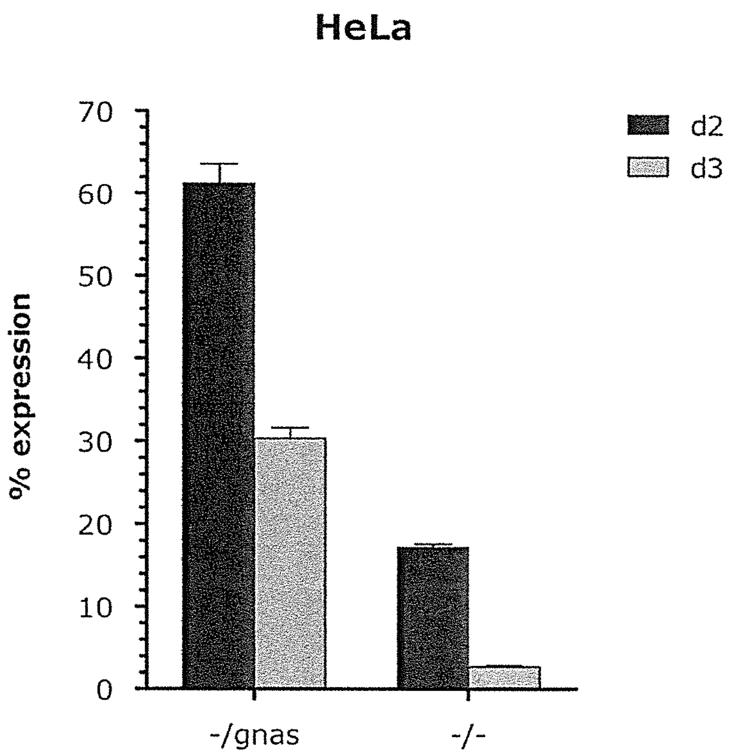


Fig. 17

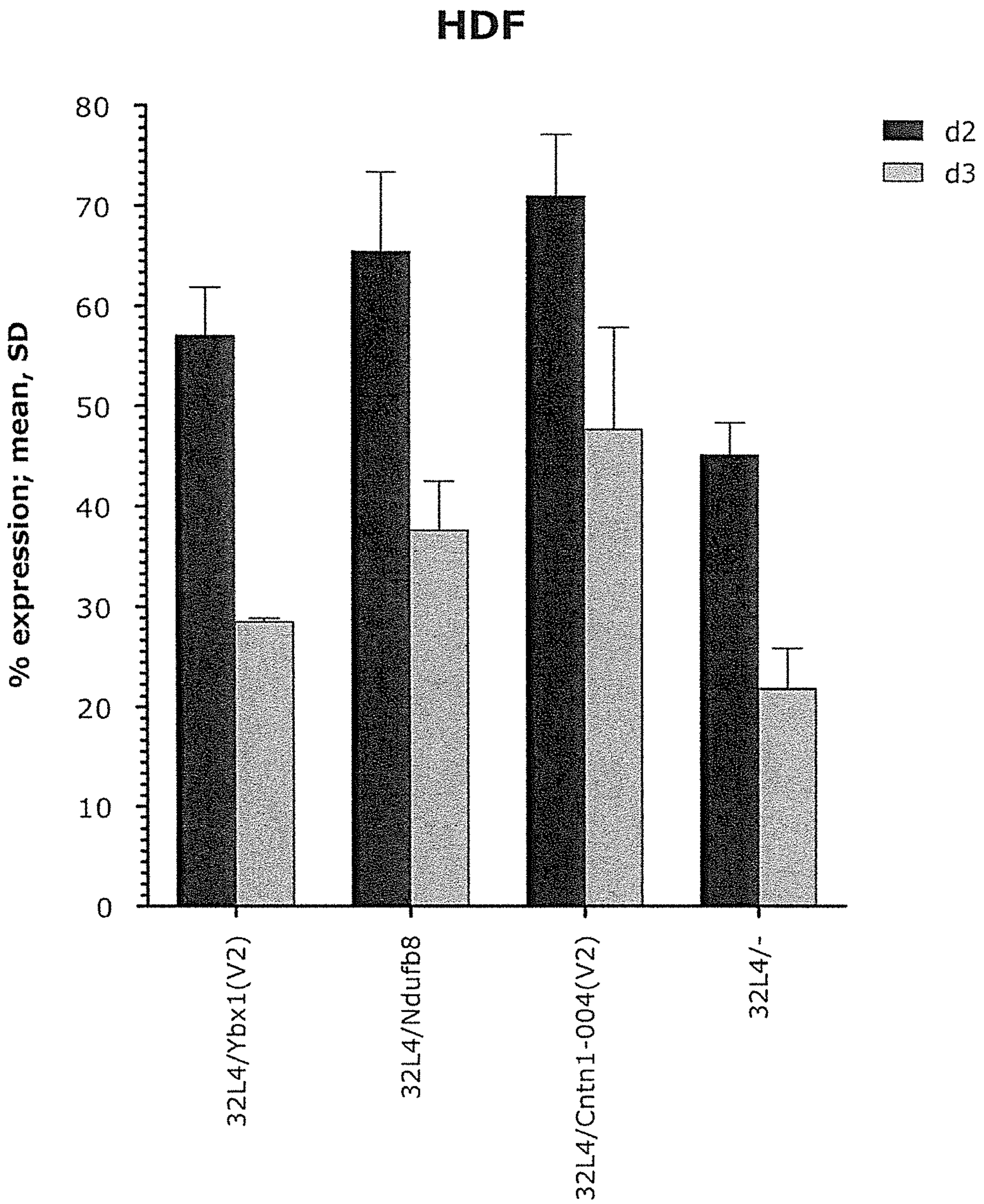
HDF
% expression; mean, SD
80
70
60
50
40
30
20
10
0
d2
d3
32L4/Ybx1(V2)
32L4/Ndufb8
32L4/Cntn1-004(V2)
32L4/-

Fig. 18

32L4 – PpLuc(GC) – Ybx1(V2)-A64-C30-hSL (R3623)

3´UTR with mutation T128bpG and deletion del236-237bp

(SEQ ID NO: 46):

GGGGCGCUGCCUACGGAGGUGGCAGCCAUCUCCUUCUCGGCAUCAAGCUUGAGGAUGGAG
GACGCCAAGAACAUCAAGAAGGGCCCGGCGCCCUUCUACCCGCUGGAGGACGGGACCGCC
GGCGAGCAGCUCCACAAGGCCAUGAAGCGGUACGCCCUGGUGCCGGGCACGAUCGCCUUC
ACCGACGCCCACAUCGAGGUCGACAUCACCUACGCGGAGUACUUCGAGAUGAGCGUGCGC
CUGGCCGAGGCCAUGAAGCGGUACGGCCUGAACACCAACCACCGGAUCGUGGUGUGCUCG
GAGAACAGCCUGCAGUUCUUCAUGCCGGUGCUGGGCGCCCUCUUCAUCGGCGUGGCCGUC
GCCCCGGCGAACGACAUCUACAACGAGCGGGAGCUGCUGAACAGCAUGGGGAUCAGCCAG
CCGACCGUGGUGUUCGUGAGCAAGAAGGGCCUGCAGAAGAUCCUGAACGUGCAGAAGAAG
CUGCCCAUCAUCCAGAAGAUCAUCAUCAUGGACAGCAAGACCGACUACCAGGGCUUCCAG
UCGAUGUACACGUUCGUGACCAGCCACCUCCCGCCGGGCUUCAACGAGUACGACUUCGUC
CCGGAGAGCUUCGACCGGGACAAGACCAUCGCCCUGAUCAUGAACAGCAGCGGCAGCACC
GGCCUGCCGAAGGGGGUGGCCCUGCCGCACCGGACCGCCUGCGUGCGCUUCUCGCACGCC
CGGGACCCCAUCUUCGGCAACCAGAUCAUCCCGGACACCGCCAUCCUGAGCGUGGUGCCG
UUCCACCACGGCUUCGGCAUGUUCACGACCCUGGGCUACCUCAUCUGCGGCUUCCGGGUG
GUCCUGAUGUACCGGUUCGAGGAGGAGCUGUUCCUGCGGAGCCUGCAGGACUACAAGAUC
CAGAGCGCGCUGCUCGUGCCGACCCUGUUCAGCUUCUUCGCCAAGAGCACCCUGAUCGAC
AAGUACGACCUGUCGAACCUGCACGAGAUCGCCAGCGGGGGCGCCCCGCUGAGCAAGGAG
GUGGGCGAGGCCGUGGCCAAGCGGUUCCACCUCCCGGGCAUCCGCCAGGGCUACGGCCUG
ACCGAGACCACGAGCGCGAUCCUGAUCACCCCCGAGGGGGACGACAAGCCGGGCGCCGUG
GGCAAGGUGGUCCCGUUCUUCGAGGCCAAGGUGGUGGACCUGGACACCGGCAAGACCCUG
GGCGUGAACCAGCGGGGCGAGCUGUGCGUGCGGGGGCCGAUGAUCAUGAGCGGCUACGUG
AACAACCCGGAGGCCACCAACGCCCUCAUCGACAAGGACGGCUGGCUGCACAGCGGCGAC
AUCGCCUACUGGGACGAGGACGAGCACUUCUUCAUCGUCGACCGGCUGAAGUCGCUGAUC
AAGUACAAGGGCUACCAGGUGGCGCCGGCCGAGCUGGAGAGCAUCCUGCUCCAGCACCCC
AACAUCUUCGACGCCGGCGUGGCCGGGCUGCCGGACGACGACGCCGGCGAGCUGCCGGCC
GCGGUGGUGGUGCUGGAGCACGGCAAGACCAUGACGGAGAAGGAGAUCGUCGACUACGUG
GCCAGCCAGGUGACCACCGCCAAGAAGCUGCGGGGCGGCGUGGUGUUCGUGGACGAGGUC
CCGAAGGGCCUGACCGGGAAGCUCGACGCCCGGAAGAUCCGCGAGAUCCUGAUCAAGGCC
AAGAAGGGCGGCAAGAUCGCCGUGUAAGACUAGUAUGCCGGCUUACCAUCUCUACCAUCA
UCCGGUUUGGUCAUCCAACAAGAAGAAAUGAAUAUGAAAUUCCAGCAAUAAGAAAUGAAC
AAAGAUUGGAGCUGAAGACCUUAAGUGCUUGCUUUUUGCCCGCUGACCAGAUAACAUUAG
AACUAUCUGCAUUAUCUAUGCAGCAUGGGGUUUUUAUUAUUUUUACCUAAAGAUGUCUCU
UUUUGGUAAUGACAAACGUGUUUUUUAAGAAAAAAAAAAAAAGGCCUGGUUUUUCUCAAUA
CACCUUUAACGGUUUUUAAAUUGUUUCAUAUCUGGUCAAGUUGAGAUUUUUAAGAACUUC
AUUUUUAAUUUGUAAUAAAGUUUACAACUUGAUUUUUUCAAAAAAGUCAACAAACUGCAA
GCACCUGUUAAUAAAGGUCUUAAAUAAUAAAGAUCUAAAAAAAAAAAAAAAAAAAAAAAA
AAAAAAAAAAAAAAAAAAAAAAAAAAAAAAAAAAAAAAAAAUGCAUCCCCCCCCCCCCCCC
CCCCCCCCCCCCCCCCAAAGGCUCUUUUCAGAGCCACCAGAAUU

Fig. 19

32L4 – PpLuc(GC) – Ndufb8-A64-C30-hSL (R3624)

(SEQ ID NO: 47):

GGGGCGCUGCCUACGGAGGUGGCAGCCAUCUCCUUCUCGGCAUCAAGCUUGAGG*AUGGAG*
*GACGCCAAGAACAUCAAGAAGGGCCCGGCGCCCUUCUACCCGCUGGAGGACGGGACCGCC*
*GGCGAGCAGCUCCACAAGGCCAUGAAGCGGUACGCCCUGGUGCCGGGCACGAUCGCCUUC*
*ACCGACGCCCACAUCGAGGUCGACAUCACCUACGCGGAGUACUUCGAGAUGAGCGUGCGC*
*CUGGCCGAGGCCAUGAAGCGGUACGGCCUGAACACCAACCACCGGAUCGUGGUGUGCUCG*
*GAGAACAGCCUGCAGUUCUUCAUGCCGGUGCUGGGCGCCCUCUUCAUCGGCGUGGCCGUC*
*GCCCCGGCGAACGACAUCUACAACGAGCGGGAGCUGCUGAACAGCAUGGGGAUCAGCCAG*
*CCGACCGUGGUGUUCGUGAGCAAGAAGGGCCUGCAGAAGAUCCUGAACGUGCAGAAGAAG*
*CUGCCCAUCAUCCAGAAGAUCAUCAUCAUGGACAGCAAGACCGACUACCAGGGCUUCCAG*
*UCGAUGUACACGUUCGUGACCAGCCACCUCCCGCCGGGCUUCAACGAGUACGACUUCGUC*
*CCGGAGAGCUUCGACCGGGACAAGACCAUCGCCCUGAUCAUGAACAGCAGCGGCAGCACC*
*GGCCUGCCGAAGGGGGUGGCCCUGCCGCACCGGACCGCCUGCGUGCGCUUCUCGCACGCC*
*CGGGACCCCAUCUUCGGCAACCAGAUCAUCCCGGACACCGCCAUCCUGAGCGUGGUGCCG*
*UUCCACCACGGCUUCGGCAUGUUCACGACCCUGGGCUACCUCAUCUGCGGCUUCCGGGUG*
*GUCCUGAUGUACCGGUUCGAGGAGGAGCUGUUCCUGCGGAGCCUGCAGGACUACAAGAUC*
*CAGAGCGCGCUGCUCGUGCCGACCCUGUUCAGCUUCUUCGCCAAGAGCACCCUGAUCGAC*
*AAGUACGACCUGUCGAACCUGCACGAGAUCGCCAGCGGGGGCGCCCCGCUGAGCAAGGAG*
*GUGGGCGAGGCCGUGGCCAAGCGGUUCCACCUCCCGGGCAUCCGCCAGGGCUACGGCCUG*
*ACCGAGACCACGAGCGCGAUCCUGAUCACCCCCGAGGGGGACGACAAGCCGGGCGCCGUG*
*GGCAAGGUGGUCCCGUUCUUCGAGGCCAAGGUGGUGGACCUGGACACCGGCAAGACCCUG*
*GGCGUGAACCAGCGGGGCGAGCUGUGCGUGCGGGGGCCGAUGAUCAUGAGCGGCUACGUG*
*AACAACCCGGAGGCCACCAACGCCCUCAUCGACAAGGACGGCUGGCUGCACAGCGGCGAC*
*AUCGCCUACUGGGACGAGGACGAGCACUUCUUCAUCGUCGACCGGCUGAAGUCGCUGAUC*
*AAGUACAAGGGCUACCAGGUGGCGCCGGCCGAGCUGGAGAGCAUCCUGCUCCAGCACCCC*
*AACAUCUUCGACGCCGGCGUGGCCGGGCUGCCGGACGACGACGCCGGCGAGCUGCCGGCC*
*GCGGUGGUGGUGCUGGAGCACGGCAAGACCAUGACGGAGAAGGAGAUCGUCGACUACGUG*
*GCCAGCCAGGUGACCACCGCCAAGAAGCUGCGGGGCGGCGUGGUGUUCGUGGACGAGGUC*
*CCGAAGGGCCUGACCGGGAAGCUCGACGCCCGGAAGAUCCGCGAGAUCCUGAUCAAGGCC*
*AAGAAGGGCGGCAAGAUCGCCGUGUAA*GACUAGUGGAGGCUUGAUGGGCUUUUGCCCUC
GUUCCUAGAGGCUUAACCAUAAUAAAAUCCCUAAUAAAGCAGAUCUAAAAAAAAAAAAAAA
AAAAAAAAAAAAAAAAAAAAAAAAAAAAAAAAAAAAAAAAAAAAAAAAAUGCAUCCCCCC
CCCCCCCCCCCCCCCCCCCCCCCCAAAGGCUCUUUUCAGAGCCACCAGAAUU

Fig. 20

32L4 – PpLuc(GC) – Cntn1-004(V2)-A64-C30-hSL (R3625)
+T at pos. 30bp, mutations G727bpT, A840bpG

(SEQ ID NO: 48):

GGGGCGCUGCCUACGGAGGUGGCAGCCAUCUCCUUCUCGGCAUCAAGCUUGAGG*AUGGAG*
*GACGCCAAGAACAUCAAGAAGGGCCCGGCGCCCUUCUACCCGCUGGAGGACGGGACCGCC*
*GGCGAGCAGCUCCACAAGGCCAUGAAGCGGUACGCCCUGGUGCCGGGCACGAUCGCCUUC*
*ACCGACGCCCACAUCGAGGUCGACAUCACCUACGCGGAGUACUUCGAGAUGAGCGUGCGC*
*CUGGCCGAGGCCAUGAAGCGGUACGGCCUGAACACCAACCACCGGAUCGUGGUGUGCUCG*
*GAGAACAGCCUGCAGUUCUUCAUGCCGGUGCUGGGCGCCCUCUUCAUCGGCGUGGCCGUC*
*GCCCCGGCGAACGACAUCUACAACGAGCGGGAGCUGCUGAACAGCAUGGGGAUCAGCCAG*
*CCGACCGUGGUGUUCGUGAGCAAGAAGGGCCUGCAGAAGAUCCUGAACGUGCAGAAGAAG*
*CUGCCCAUCAUCCAGAAGAUCAUCAUCAUGGACAGCAAGACCGACUACCAGGGCUUCCAG*
*UCGAUGUACACGUUCGUGACCAGCCACCUCCCGCCGGGCUUCAACGAGUACGACUUCGUC*
*CCGGAGAGCUUCGACCGGGACAAGACCAUCGCCCUGAUCAUGAACAGCAGCGGCAGCACC*
*GGCCUGCCGAAGGGGGUGGCCCUGCCGCACCGGACCGCCUGCGUGCGCUUCUCGCACGCC*
*CGGGACCCCAUCUUCGGCAACCAGAUCAUCCCGGACACCGCCAUCCUGAGCGUGGUGCCG*
*UUCCACCACGGCUUCGGCAUGUUCACGACCCUGGGCUACCUCAUCUGCGGCUUCCGGGUG*
*GUCCUGAUGUACCGGUUCGAGGAGGAGCUGUUCCUGCGGAGCCUGCAGGACUACAAGAUC*
*CAGAGCGCGCUGCUCGUGCCGACCCUGUUCAGCUUCUUCGCCAAGAGCACCCUGAUCGAC*
*AAGUACGACCUGUCGAACCUGCACGAGAUCGCCAGCGGGGGCGCCCCGCUGAGCAAGGAG*
*GUGGGCGAGGCCGUGGCCAAGCGGUUCCACCUCCCGGGCAUCCGCCAGGGCUACGGCCUG*
*ACCGAGACCACGAGCGCGAUCCUGAUCACCCCCGAGGGGGACGACAAGCCGGGCGCCGUG*
*GGCAAGGUGGUCCCGUUCUUCGAGGCCAAGGUGGUGGACCUGGACACCGGCAAGACCCUG*
*GGCGUGAACCAGCGGGGCGAGCUGUGCGUGCGGGGGCCGAUGAUCAUGAGCGGCUACGUG*
*AACAACCCGGAGGCCACCAACGCCCUCAUCGACAAGGACGGCUGGCUGCACAGCGGCGAC*
*AUCGCCUACUGGGACGAGGACGAGCACUUCUUCAUCGUCGACCGGCUGAAGUCGCUGAUC*
*AAGUACAAGGGCUACCAGGUGGCGCCGGCCGAGCUGGAGAGCAUCCUGCUCCAGCACCCC*
*AACAUCUUCGACGCCGGCGUGGCCGGGCUGCCGGACGACGACGCCGGCGAGCUGCCGGCC*
*GCGGUGGUGGUGCUGGAGCACGGCAAGACCAUGACGGAGAAGGAGAUCGUCGACUACGUG*
*GCCAGCCAGGUGACCACCGCCAAGAAGCUGCGGGGCGGCGUGGUGUUCGUGGACGAGGUC*
*CCGAAGGGCCUGACCGGGAAGCUCGACGCCCGGAAGAUCCGCGAGAUCCUGAUCAAGGCC*
*AAGAAGGGCGGCAAGAUCGCCGUGUAA*GACUAGUUCGUUGACACUCACCAUUUCUGUGAA
AGACUUUUUUUUUUUUUAACAUAUUAUACUAGAUUUGACUAACUCAAUCUUGUAGCUUCU
GCAGUUCUCCCCACCCCCAACCUAGUUCUUAGAGUAUGUUUCCCCUUUUGAAACAUGUAA
ACAUACUUUGGGCAUAAAUAUUUUUAAAAUAUAACUAUAAUGCUUCACUAAUACCUUAA
AAAUGCCUAGUGAACUAACUCAGUACAUUAUAUAAUGGCCAAGUGAAAGUUUUGUGUUUU
CAUGUCCUGUUUUUCUUUGAAAUUAUAUAGCCCAGAAAUUAGCUCAUUAUCUGAAAAACG
UAUGAAGAACUGAUGAAUUGUAUAAUACAGGAGUAUUGCCAUUGAAUGUACUGUUUGAUU
UAUUCAAGCAGGUAAUGAACAAUGUUGUCAAACUCUCUAAUGAGACAUCAUAAUUAGGAC
AUAAGCUAAAAGGGGCAUUACUCCGGCAGUCUUUUUUUCUUAAUCCUAGUACCAUACAUA
UUCUUUGGCAUGAAAGAAUGAAAAGCAUUAGUAAACAACUGAAGUCCUACCAUGGCUCUG
UAGGGUUUUUGGAACAAUUCCUGGAAUUGGAAAGUGAAAAUGGAUAGCAUGUGGGGAAA
CCCUCAUCUGAGUAGCAAGAUUUUAGUAAAGAUGACUAAGCCAUUAACAGCAUGCAUUCA
UAUUUAAUUUUAUUGACUCCUGCCAUCAGCUUUUGUAGAUCUUUUGGGUGGAAGGUUGUG
AUUUUUACUGGGAGGACUUGAGUAGAAGUGGAUGAUUAAAAUUGAGGAGUAUAUAAUUCU
UUCUGGGACUGCUUAAAUGUUAUUGUUUGAAAAUGCCUUCACUUUCCCCCUUUGGUCAAA
GAGAUGUGCUUAAAAUUCUUAUUCCUUCACAAUAAAUAAUUUUGAUUUUCUUAGACAAGA
UCUAAAAAAAAAAAAAAAAAAAAAAAAAAAAAAAAAAAAAAAAAAAAAAAAAAAAAAAAAA
AAAAAAAUGCAUCCCCCCCCCCCCCCCCCCCCCCCCCCCCCCCAAAGGCUCUUUUCAGAG
CCACCAGAAUU

Fig. 21

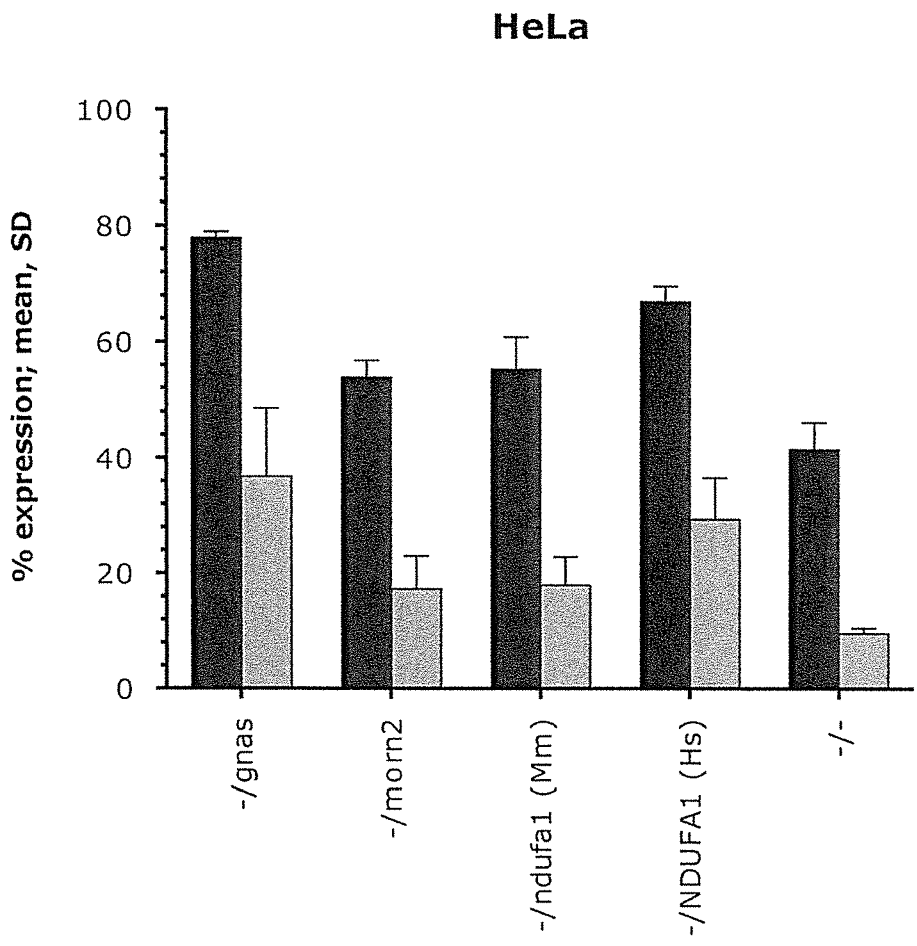
HeLa
% expression; mean, SD
100
80
60
40
20
0
-/gnas
-/morn2
-/ndufa1 (Mm)
-/NDUFA1 (Hs)
-/-

Fig. 22

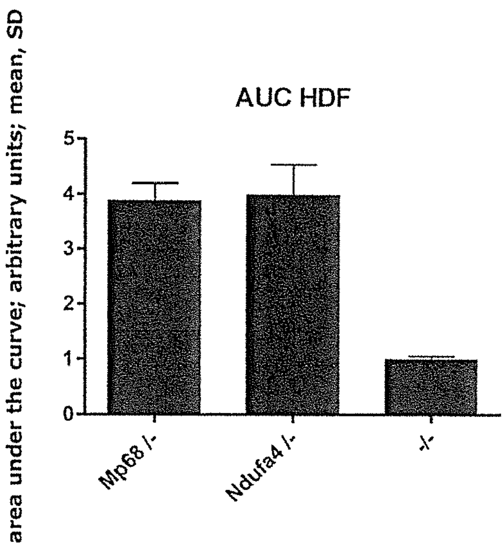
AUC HDF
area under the curve; arbitrary units; mean, SD
5
4
3
2
1
0
Mp68 /-
Ndufa4 /-
-/-

Fig. 23

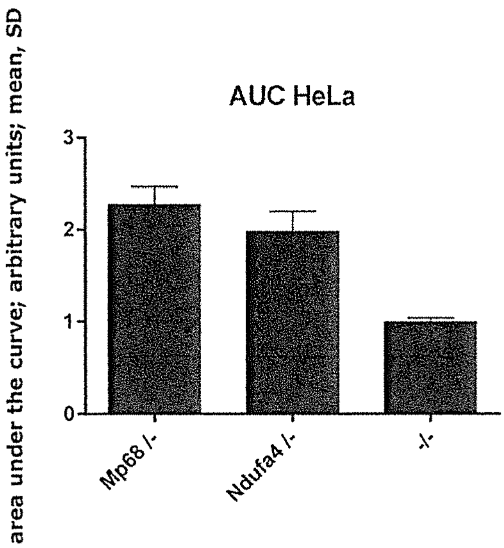
area under the curve; arbitrary units; mean, SD
AUC HeLa
3
2
1
0
Mp68 /-
Ndufa4 /-
-/-

Fig. 24

32L4 – PpLuc(GC) – A64-C30-hSL (R2462)
(SEQ ID NO: 383)

GGGAGAAAGCUUGAGG*AUGGAGGACGCCAAGAACAUCAAGAAGGGCCCGGCGCCCUUCUA*
*CCCGCUGGAGGACGGGACCGCCGGCGAGCAGCUCCACAAGGCCAUGAAGCGGUACGCCCU*
*GGUGCCGGGCACGAUCGCCUUCACCGACGCCCACAUCGAGGUCGACAUCACCUACGCGGA*
*GUACUUCGAGAUGAGCGUGCGCCUGGCCGAGGCCAUGAAGCGGUACGGCCUGAACACCAA*
*CCACCGGAUCGUGGUGUGCUCGGAGAACAGCCUGCAGUUCUUCAUGCCGGUGCUGGGCGC*
*CCUCUUCAUCGGCGUGGCCGUCGCCCCGGCGAACGACAUCUACAACGAGCGGGAGCUGCU*
*GAACAGCAUGGGGAUCAGCCAGCCGACCGUGGUGUUCGUGAGCAAGAAGGGCCUGCAGAA*
*GAUCCUGAACGUGCAGAAGAAGCUGCCCAUCAUCCAGAAGAUCAUCAUCAUGGACAGCAA*
*GACCGACUACCAGGGCUUCCAGUCGAUGUACACGUUCGUGACCAGCCACCUCCCGCCGGG*
*CUUCAACGAGUACGACUUCGUCCCGGAGAGCUUCGACCGGGACAAGACCAUCGCCCUGAU*
*CAUGAACAGCAGCGGCAGCACCGGCCUGCCGAAGGGGGUGGCCCUGCCGCACCGGACCGC*
*CUGCGUGCGCUUCUCGCACGCCCGGGACCCCAUCUUCGGCAACCAGAUCAUCCCGGACAC*
*CGCCAUCCUGAGCGUGGUGCCGUUCCACCACGGCUUCGGCAUGUUCACGACCCUGGGCUA*
*CCUCAUCUGCGGCUUCCGGGUGGUCCUGAUGUACCGGUUCGAGGAGGAGCUGUUCCUGCG*
*GAGCCUGCAGGACUACAAGAUCCAGAGCGCGCUGCUCGUGCCGACCCUGUUCAGCUUCUU*
*CGCCAAGAGCACCCUGAUCGACAAGUACGACCUGUCGAACCUGCACGAGAUCGCCAGCGG*
*GGGCGCCCCGCUGAGCAAGGAGGUGGGCGAGGCCGUGGCCAAGCGGUUCCACCUCCCGGG*
*CAUCCGCCAGGGCUACGGCCUGACCGAGACCACGAGCGCGAUCCUGAUCACCCCCGAGGG*
*GGACGACAAGCCGGGCGCCGUGGGCAAGGUGGUCCCGUUCUUCGAGGCCAAGGUGGUGGA*
*CCUGGACACCGGCAAGACCCUGGGCGUGAACCAGCGGGGCGAGCUGUGCGUGCGGGGCC*
*GAUGAUCAUGAGCGGCUACGUGAACAACCCGGAGGCCACCAACGCCCUCAUCGACAAGGA*
*CGGCUGGCUGCACAGCGGCGACAUCGCCUACUGGGACGAGGACGAGCACUUCUUCAUCGU*
*CGACCGGCUGAAGUCGCUGAUCAAGUACAAGGGCUACCAGGUGGCGCCGGCCGAGCUGGA*
*GAGCAUCCUGCUCCAGCACCCCAACAUCUUCGACGCCGGCGUGGCCGGGCUGCCGGACGA*
*CGACGCCGGCGAGCUGCCGGCCGCGGUGGUGGUGCUGGAGCACGGCAAGACCAUGACGGA*
*GAAGGAGAUCGUCGACUACGUGGCCAGCCAGGUGACCACCGCCAAGAAGCUGCGGGGCGG*
*CGUGGUGUUCGUGGACGAGGUCCCGAAGGGCCUGACCGGGAAGCUCGACGCCCGGAAGAU*
*CCGCGAGAUCCUGAUCAAGGCCAAGAAGGGCGGCAAGAUCGCCGUGUAA*GACUAGUAGAU
CUAAAAAAAAAAAAAAAAAAAAAAAAAAAAAAAAAAAAAAAAAAAAAAAAAAAAAAAAAA
AAAAAAUGCAUCCCCCCCCCCCCCCCCCCCCCCCCCCCCCCAAAGGCUCUUUUCAGAGC
CACCAGAAUU

Fig. 25

PpLuc(GC) – morn2– A64 - C30 - hSL (R3948)
(SEQ ID NO: 384)

GGGAGAAAGCUUGAGG*AUGGAGGACGCCAAGAACAUCAAGAAGGGCCCGGCGCCCUUCUA*
*CCCGCUGGAGGACGGGACCGCCGGCGAGCAGCUCCACAAGGCCAUGAAGCGGUACGCCCU*
*GGUGCCGGGCACGAUCGCCUUCACCGACGCCCACAUCGAGGUCGACAUCACCUACGCGGA*
*GUACUUCGAGAUGAGCGUGCGCCUGGCCGAGGCCAUGAAGCGGUACGGCCUGAACACCAA*
*CCACCGGAUCGUGGUGUGCUCGGAGAACAGCCUGCAGUUCUUCAUGCCGGUGCUGGGCGC*
*CCUCUUCAUCGGCGUGGCCGUCGCCCCGGCGAACGACAUCUACAACGAGCGGGAGCUGCU*
*GAACAGCAUGGGGAUCAGCCAGCCGACCGUGGUGUUCGUGAGCAAGAAGGGCCUGCAGAA*
*GAUCCUGAACGUGCAGAAGAAGCUGCCCAUCAUCCAGAAGAUCAUCAUCAUGGACAGCAA*
*GACCGACUACCAGGGCUUCCAGUCGAUGUACACGUUCGUGACCAGCCACCUCCCGCCGGG*
*CUUCAACGAGUACGACUUCGUCCCGGAGAGCUUCGACCGGGACAAGACCAUCGCCCUGAU*
*CAUGAACAGCAGCGGCAGCACCGGCCUGCCGAAGGGGGUGGCCCUGCCGCACCGGACCGC*
*CUGCGUGCGCUUCUCGCACGCCCGGGACCCCAUCUUCGGCAACCAGAUCAUCCCGGACAC*
*CGCCAUCCUGAGCGUGGUGCCGUUCCACCACGGCUUCGGCAUGUUCACGACCCUGGGCUA*
*CCUCAUCUGCGGCUUCCGGGUGGUCCUGAUGUACCGGUUCGAGGAGGAGCUGUUCCUGCG*
*GAGCCUGCAGGACUACAAGAUCCAGAGCGCGCUGCUCGUGCCGACCCUGUUCAGCUUCUU*
*CGCCAAGAGCACCCUGAUCGACAAGUACGACCUGUCGAACCUGCACGAGAUCGCCAGCGG*
*GGGCGCCCCGCUGAGCAAGGAGGUGGGCGAGGCCGUGGCCAAGCGGUUCCACCUCCCGGG*
*CAUCCGCCAGGGCUACGGCCUGACCGAGACCACGAGCGCGAUCCUGAUCACCCCCGAGGG*
*GGACGACAAGCCGGGCGCCGUGGGCAAGGUGGUCCCGUUCUUCGAGGCCAAGGUGGUGGA*
*CCUGGACACCGGCAAGACCCUGGGCGUGAACCAGCGGGGCGAGCUGUGCGUGCGGGGGCC*
*GAUGAUCAUGAGCGGCUACGUGAACAACCCGGAGGCCACCAACGCCCUCAUCGACAAGGA*
*CGGCUGGCUGCACAGCGGCGACAUCGCCUACUGGGACGAGGACGAGCACUUCUUCAUCGU*
*CGACCGGCUGAAGUCGCUGAUCAAGUACAAGGGCUACCAGGUGGCGCCGGCCGAGCUGGA*
*GAGCAUCCUGCUCCAGCACCCCAACAUCUUCGACGCCGGCGUGGCCGGGCUGCCGGACGA*
*CGACGCCGGCGAGCUGCCGGCCGCGGUGGUGGUGCUGGAGCACGGCAAGACCAUGACGGA*
*GAAGGAGAUCGUCGACUACGUGGCCAGCCAGGUGACCACCGCCAAGAAGCUGCGGGGCGG*
*CGUGGUGUUCGUGGACGAGGUCCCGAAGGGCCUGACCGGGAAGCUCGACGCCCGGAAGAU*
*CCGCGAGAUCCUGAUCAAGGCCAAGAAGGGCGGCAAGAUCGCCGUGUAA*GACUAGUACCU
GCUGCCUUAACGCUGAGAUGUGGCCUCUGCAACCCCCCUUAGGCAAAGCAACUGAACCUU
CUGCUAAAGUGACCUGCCCUCUUCCGUAAGUCCAAUAAAGUUGUCAUGCACCCAGAUCUA
AAAAAAAAAAAAAAAAAAAAAAAAAAAAAAAAAAAAAAAAAAAAAAAAAAAAAAAAAAAA
AAAUGCAUCCCCCCCCCCCCCCCCCCCCCCCCCCCCCCAAAGGCUCUUUUCAGAGCCAC
CAGAAUU

Fig. 26

PpLuc(GC) – ndufa1– A64 - C30 - hSL (R4043)
(SEQ ID NO: 385)

GGGAGAAAGCUUGAGGAUGGAGGACGCCAAGAACAUCAAGAAGGGCCCGGCGCCCUUCUA
CCCGCUGGAGGACGGGACCGCCGGCGAGCAGCUCCACAAGGCCAUGAAGCGGUACGCCCU
GGUGCCGGGCACGAUCGCCUUCACCGACGCCCACAUCGAGGUCGACAUCACCUACGCGGA
GUACUUCGAGAUGAGCGUGCGCCUGGCCGAGGCCAUGAAGCGGUACGGCCUGAACACCAA
CCACCGGAUCGUGGUGUGCUCGGAGAACAGCCUGCAGUUCUUCAUGCCGGUGCUGGGCGC
CCUCUUCAUCGGCGUGGCCGUCGCCCCGGCGAACGACAUCUACAACGAGCGGGAGCUGCU
GAACAGCAUGGGGAUCAGCCAGCCGACCGUGGUGUUCGUGAGCAAGAAGGGCCUGCAGAA
GAUCCUGAACGUGCAGAAGAAGCUGCCCAUCAUCCAGAAGAUCAUCAUCAUGGACAGCAA
GACCGACUACCAGGGCUUCCAGUCGAUGUACACGUUCGUGACCAGCCACCUCCCGCCGGG
CUUCAACGAGUACGACUUCGUCCCGGAGAGCUUCGACCGGGACAAGACCAUCGCCCUGAU
CAUGAACAGCAGCGGCAGCACCGGCCUGCCGAAGGGGGUGGCCCUGCCGCACCGGACCGC
CUGCGUGCGCUUCUCGCACGCCCGGGACCCCAUCUUCGGCAACCAGAUCAUCCCGGACAC
CGCCAUCCUGAGCGUGGUGCCGUUCCACCACGGCUUCGGCAUGUUCACGACCCUGGGCUA
CCUCAUCUGCGGCUUCCGGGUGGUCCUGAUGUACCGGUUCGAGGAGGAGCUGUUCCUGCG
GAGCCUGCAGGACUACAAGAUCCAGAGCGCGCUGCUCGUGCCGACCCUGUUCAGCUUCUU
CGCCAAGAGCACCCUGAUCGACAAGUACGACCUGUCGAACCUGCACGAGAUCGCCAGCGG
GGGCGCCCCGCUGAGCAAGGAGGUGGGCGAGGCCGUGGCCAAGCGGUUCCACCUCCCGGG
CAUCCGCCAGGGCUACGGCCUGACCGAGACCACGAGCGCGAUCCUGAUCACCCCCGAGGG
GGACGACAAGCCGGGCGCCGUGGGCAAGGUGGUCCCGUUCUUCGAGGCCAAGGUGGUGGA
CCUGGACACCGGCAAGACCCUGGGCGUGAACCAGCGGGGCGAGCUGUGCGUGCGGGGCC
GAUGAUCAUGAGCGGCUACGUGAACAACCCGGAGGCCACCAACGCCCUCAUCGACAAGGA
CGGCUGGCUGCACAGCGGCGACAUCGCCUACUGGGACGAGGACGAGCACUUCUUCAUCGU
CGACCGGCUGAAGUCGCUGAUCAAGUACAAGGGCUACCAGGUGGCGCCGGCCGAGCUGGA
GAGCAUCCUGCUCCAGCACCCCAACAUCUUCGACGCCGGCGUGGCCGGGCUGCCGGACGA
CGACGCCGGCGAGCUGCCGGCCGCGGUGGUGGUGCUGGAGCACGGCAAGACCAUGACGGA
GAAGGAGAUCGUCGACUACGUGGCCAGCCAGGUGACCACCGCCAAGAAGCUGCGGGGCGG
CGUGGUGUUCGUGGACGAGGUCCCGAAGGGCCUGACCGGGAAGCUCGACGCCCGGAAGAU
CCGCGAGAUCCUGAUCAAGGCCAAGAAGGGCGGCAAGAUCGCCGUGUAAGACUAGUGGAA
GCAUUUUCCUGGCUGAUUAAAAGAAAUUACUCAGCUAUGGUCAUCUGUUCCUGUUAGAAG
GCUAUGCAGCAUAUUAUAUACUAUGCGCAUGUUAUGAAAUGCAUAAUAAAAAAUUUAAA
AAAUCUAAAAGAUCUAAAAAAAAAAAAAAAAAAAAAAAAAAAAAAAAAAAAAAAAAAAAA
AAAAAAAAAAAAAAAAAAAUGCAUCCCCCCCCCCCCCCCCCCCCCCCCCCCCCCCAAAGG
CUCUUUUCAGAGCCACCAGAAUU

Fig. 27

PpLuc(GC) – NDUFA1– A64 - C30 - hSL (R3948)
(SEQ ID NO: 386)

GGGAGAAAGCUUGAGGAUGGAGGACGCCAAGAACAUCAAGAAGGGCCCGGCGCCCUUCUA
CCCGCUGGAGGACGGGACCGCCGGCGAGCAGCUCCACAAGGCCAUGAAGCGGUACGCCCU
GGUGCCGGGCACGAUCGCCUUCACCGACGCCCACAUCGAGGUCGACAUCACCUACGCGGA
GUACUUCGAGAUGAGCGUGCGCCUGGCCGAGGCCAUGAAGCGGUACGGCCUGAACACCAA
CCACCGGAUCGUGGUGUGCUCGGAGAACAGCCUGCAGUUCUUCAUGCCGGUGCUGGGCGC
CCUCUUCAUCGGCGUGGCCGUCGCCCCGGCGAACGACAUCUACAACGAGCGGGAGCUGCU
GAACAGCAUGGGGAUCAGCCAGCCGACCGUGGUGUUCGUGAGCAAGAAGGGCCUGCAGAA
GAUCCUGAACGUGCAGAAGAAGCUGCCCAUCAUCCAGAAGAUCAUCAUCAUGGACAGCAA
GACCGACUACCAGGGCUUCCAGUCGAUGUACACGUUCGUGACCAGCCACCUCCCGCCGGG
CUUCAACGAGUACGACUUCGUCCCGGAGAGCUUCGACCGGGACAAGACCAUCGCCCUGAU
CAUGAACAGCAGCGGCAGCACCGGCCUGCCGAAGGGGGUGGCCCUGCCGCACCGGACCGC
CUGCGUGCGCUUCUCGCACGCCCGGGACCCCAUCUUCGGCAACCAGAUCAUCCCGGACAC
CGCCAUCCUGAGCGUGGUGCCGUUCCACCACGGCUUCGGCAUGUUCACGACCCUGGGCUA
CCUCAUCUGCGGCUUCCGGGUGGUCCUGAUGUACCGGUUCGAGGAGGAGCUGUUCCUGCG
GAGCCUGCAGGACUACAAGAUCCAGAGCGCGCUGCUCGUGCCGACCCUGUUCAGCUUCUU
CGCCAAGAGCACCCUGAUCGACAAGUACGACCUGUCGAACCUGCACGAGAUCGCCAGCGG
GGGCGCCCCGCUGAGCAAGGAGGUGGGCGAGGCCGUGGCCAAGCGGUUCCACCUCCCGGG
CAUCCGCCAGGGCUACGGCCUGACCGAGACCACGAGCGCGAUCCUGAUCACCCCCGAGGG
GGACGACAAGCCGGGCGCCGUGGGCAAGGUGGUCCCGUUCUUCGAGGCCAAGGUGGUGGA
CCUGGACACCGGCAAGACCCUGGGCGUGAACCAGCGGGGCGAGCUGUGCGUGCGGGGCC
GAUGAUCAUGAGCGGCUACGUGAACAACCCGGAGGCCACCAACGCCCUCAUCGACAAGGA
CGGCUGGCUGCACAGCGGCGACAUCGCCUACUGGGACGAGGACGAGCACUUCUUCAUCGU
CGACCGGCUGAAGUCGCUGAUCAAGUACAAGGGCUACCAGGUGGCGCCGGCCGAGCUGGA
GAGCAUCCUGCUCCAGCACCCCAACAUCUUCGACGCCGGCGUGGCCGGGCUGCCGGACGA
CGACGCCGGCGAGCUGCCGGCCGCGGUGGUGGUGCUGGAGCACGGCAAGACCAUGACGGA
GAAGGAGAUCGUCGACUACGUGGCCAGCCAGGUGACCACCGCCAAGAAGCUGCGGGGCGG
CGUGGUGUUCGUGGACGAGGUCCCGAAGGGCCUGACCGGGAAGCUCGACGCCCGGAAGAU
CCGCGAGAUCCUGAUCAAGGCCAAGAAGGGCGGCAAGAUCGCCGUGUAAGACUAGUGGAA
GCAUUUUCCUGAUUGAUGAAAAAAAUAACUCAGUUAUGGCCAUCUACCCCUGCUAGAAGG
UUACAGUGUAUUAUGUAGCAUGCAAUGUGUUAUGUAGUGCUUAAUAAAAAUAAAAUGAAA
AAAAUGCAGAUCUAAAAAAAAAAAAAAAAAAAAAAAAAAAAAAAAAAAAAAAAAAAAAA
AAAAAAAAAAAAAAAAAUGCAUCCCCCCCCCCCCCCCCCCCCCCCCCCCCCCCAAAGGCU
CUUUUCAGAGCCACCAGAAUU

Fig. 28

Mp68 - PpLuc(GC) – A64 - C30 - hSL (R3954)
(SEQ ID NO: 387)

GGGCUUUCCCAUUCUGUAGCAGAAUUUGGUGUUGCCUGUGGUCUUGGUCCCGCGGAGAAG
CUUGAGGAUGGAGGACGCCAAGAACAUCAAGAAGGGCCCGGCGCCCUUCUACCCGCUGGA
GGACGGGACCGCCGGCGAGCAGCUCCACAAGGCCAUGAAGCGGUACGCCCUGGUGCCGGG
CACGAUCGCCUUCACCGACGCCCACAUCGAGGUCGACAUCACCUACGCGGAGUACUUCGA
GAUGAGCGUGCGCCUGGCCGAGGCCAUGAAGCGGUACGGCCUGAACACCAACCACCGGAU
CGUGGUGUGCUCGGAGAACAGCCUGCAGUUCUUCAUGCCGGUGCUGGGCGCCCUCUUCAU
CGGCGUGGCCGUCGCCCCGGCGAACGACAUCUACAACGAGCGGGAGCUGCUGAACAGCAU
GGGGAUCAGCCAGCCGACCGUGGUGUUCGUGAGCAAGAAGGGCCUGCAGAAGAUCCUGAA
CGUGCAGAAGAAGCUGCCCAUCAUCCAGAAGAUCAUCAUCAUGGACAGCAAGACCGACUA
CCAGGGCUUCCAGUCGAUGUACACGUUCGUGACCAGCCACCUCCCGCCGGGCUUCAACGA
GUACGACUUCGUCCCGGAGAGCUUCGACCGGGACAAGACCAUCGCCCUGAUCAUGAACAG
CAGCGGCAGCACCGGCCUGCCGAAGGGGGUGGCCCUGCCGCACCGGACCGCCUGCGUGCG
CUUCUCGCACGCCCGGGACCCCAUCUUCGGCAACCAGAUCAUCCCGGACACCGCCAUCCU
GAGCGUGGUGCCGUUCCACCACGGCUUCGGCAUGUUCACGACCCUGGGCUACCUCAUCUG
CGGCUUCCGGGUGGUCCUGAUGUACCGGUUCGAGGAGGAGCUGUUCCUGCGGAGCCUGCA
GGACUACAAGAUCCAGAGCGCGCUGCUCGUGCCGACCCUGUUCAGCUUCUUCGCCAAGAG
CACCCUGAUCGACAAGUACGACCUGUCGAACCUGCACGAGAUCGCCAGCGGGGGCGCCCC
GCUGAGCAAGGAGGUGGGCGAGGCCGUGGCCAAGCGGUUCCACCUCCCGGGCAUCCGCCA
GGGCUACGGCCUGACCGAGACCACGAGCGCGAUCCUGAUCACCCCCGAGGGGGACGACAA
GCCGGGCGCCGUGGGCAAGGUGGUCCCGUUCUUCGAGGCCAAGGUGGUGGACCUGGACAC
CGGCAAGACCCUGGGCGUGAACCAGCGGGGCGAGCUGUGCGUGCGGGGGCCGAUGAUCAU
GAGCGGCUACGUGAACAACCCGGAGGCCACCAACGCCCUCAUCGACAAGGACGGCUGGCU
GCACAGCGGCGACAUCGCCUACUGGGACGAGGACGAGCACUUCUUCAUCGUCGACCGGCU
GAAGUCGCUGAUCAAGUACAAGGGCUACCAGGUGGCGCCGGCCGAGCUGGAGAGCAUCCU
GCUCCAGCACCCCAACAUCUUCGACGCCGGCGUGGCCGGGCUGCCGGACGACGACGCCGG
CGAGCUGCCGGCCGCGGUGGUGGUGCUGGAGCACGGCAAGACCAUGACGGAGAAGGAGAU
CGUCGACUACGUGGCCAGCCAGGUGACCACCGCCAAGAAGCUGCGGGGCGGCGUGGUGUU
CGUGGACGAGGUCCCGAAGGGCCUGACCGGGAAGCUCGACGCCCGGAAGAUCCGCGAGAU
CCUGAUCAAGGCCAAGAAGGGCGGCAAGAUCGCCGUGUAAGACUAGUAGAUCUAAAAAAA
AAAAAAAAAAAAAAAAAAAAAAAAAAAAAAAAAAAAAAAAAAAAAAAAAAAAAAAAAUGC
AUCCCCCCCCCCCCCCCCCCCCCCCCCCCCCCCAAAGGCUCUUUUCAGAGCCACCAGAAU
U

Fig. 29

Ndufa4 - PpLuc(GC) – A64 - C30 - hSL (R3951)
(SEQ ID NO: 388)

GGGGUCCGCUCAGCCAGGUUGCAGAAGCGGCUUAGCGUGUGUCCUAAUCUUCUCUGCG
UGUAGGUAGGCCUGUGCCGCAAACAAGCUUGAGG*AUGGAGGACGCCAAGAACAUCAAGAA*
*GGGCCCGGCGCCCUUCUACCCGCUGGAGGACGGGACCGCCGGCGAGCAGCUCCACAAGGC*
*CAUGAAGCGGUACGCCCUGGUGCCGGGCACGAUCGCCUUCACCGACGCCCACAUCGAGGU*
*CGACAUCACCUACGCGGAGUACUUCGAGAUGAGCGUGCGCCUGGCCGAGGCCAUGAAGCG*
*GUACGGCCUGAACACCAACCACCGGAUCGUGGUGUGCUCGGAGAACAGCCUGCAGUUCUU*
*CAUGCCGGUGCUGGGCGCCCUCUUCAUCGGCGUGGCCGUCGCCCCGGCGAACGACAUCUA*
*CAACGAGCGGGAGCUGCUGAACAGCAUGGGGAUCAGCCAGCCGACCGUGGUGUUCGUGAG*
*CAAGAAGGGCCUGCAGAAGAUCCUGAACGUGCAGAAGAAGCUGCCCAUCAUCCAGAAGAU*
*CAUCAUCAUGGACAGCAAGACCGACUACCAGGGCUUCCAGUCGAUGUACACGUUCGUGAC*
*CAGCCACCUCCCGCCGGGCUUCAACGAGUACGACUUCGUCCCGGAGAGCUUCGACCGGGA*
*CAAGACCAUCGCCCUGAUCAUGAACAGCAGCGGCAGCACCGGCCUGCCGAAGGGGGUGGC*
*CCUGCCGCACCGGACCGCCUGCGUGCGCUUCUCGCACGCCCGGGACCCCAUCUUCGGCAA*
*CCAGAUCAUCCCGGACACCGCCAUCCUGAGCGUGGUGCCGUUCCACCACGGCUUCGGCAU*
*GUUCACGACCCUGGGCUACCUCAUCUGCGGCUUCCGGGUGGUCCUGAUGUACCGGUUCGA*
*GGAGGAGCUGUUCCUGCGGAGCCUGCAGGACUACAAGAUCCAGAGCGCGCUGCUCGUGCC*
*GACCCUGUUCAGCUUCUUCGCCAAGAGCACCCUGAUCGACAAGUACGACCUGUCGAACCU*
*GCACGAGAUCGCCAGCGGGGGCGCCCCGCUGAGCAAGGAGGUGGGCGAGGCCGUGGCCAA*
*GCGGUUCCACCUCCCGGGCAUCCGCCAGGGCUACGGCCUGACCGAGACCACGAGCGCGAU*
*CCUGAUCACCCCCGAGGGGGACGACAAGCCGGGCGCCGUGGGCAAGGUGGUCCCGUUCUU*
*CGAGGCCAAGGUGGUGGACCUGGACACCGGCAAGACCCUGGGCGUGAACCAGCGGGGCGA*
*GCUGUGCGUGCGGGGGCCGAUGAUCAUGAGCGGCUACGUGAACAACCCGGAGGCCACCAA*
*CGCCCUCAUCGACAAGGACGGCUGGCUGCACAGCGGCGACAUCGCCUACUGGGACGAGGA*
*CGAGCACUUCUUCAUCGUCGACCGGCUGAAGUCGCUGAUCAAGUACAAGGGCUACCAGGU*
*GGCGCCGGCCGAGCUGGAGAGCAUCCUGCUCCAGCACCCCAACAUCUUCGACGCCGGCGU*
*GGCCGGGCUGCCGGACGACGACGCCGGCGAGCUGCCGGCCGCGGUGGUGGUGCUGGAGCA*
*CGGCAAGACCAUGACGGAGAAGGAGAUCGUCGACUACGUGGCCAGCCAGGUGACCACCGC*
*CAAGAAGCUGCGGGGCGGCGUGGUGUUCGUGGACGAGGUCCCGAAGGGCCUGACCGGGAA*
*GCUCGACGCCCGGAAGAUCCGCGAGAUCCUGAUCAAGGCCAAGAAGGGCGGCAAGAUCGC*
*CGUGUAA*GACUAGUAGAUCUAAAAAAAAAAAAAAAAAAAAAAAAAAAAAAAAAAAAAAAA
AAAAAAAAAAAAAAAAAAAAAAAAUGCAUCCCCCCCCCCCCCCCCCCCCCCCCCCCCCCC
AAAGGCUCUUUUCAGAGCCACCAGAAUU

Fig. 30

Europäisches Patentamt
European Patent Office
Office européen des brevets

# EUROPEAN SEARCH REPORT

Application Number
EP 19 15 2916

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2013/143700 A2 (CUREVAC GMBH [DE]; THES ANDREAS [DE]) 3 October 2013 (2013-10-03)<br>* the whole document *<br>* in particular abstract; page 39, lines 28-33;page 52, lines 9-11; page 65 lines 5-20; page 80, lines 10-26; claims 1-89; *<br>----- | 1-22 | INV.<br>A61K38/00<br>C12N15/67<br>C12N15/85 |
| X | WO 2007/068265 A1 (KING FAISAL SPECIALIST HOSPITA [SA]; TERRAMARK MARKENCREATION GMBH [DE) 21 June 2007 (2007-06-21)<br>* the whole document *<br>* in particular abstract; examples 1, 2, 3; Table 1; claims 1-16 *<br>----- | 1-22 | |
| A | WO 2013/120629 A1 (CUREVAC GMBH [DE]; THEBETA ANDREAS [DE]; SCHLAKE THOMAS [DE]; PROBST J) 22 August 2013 (2013-08-22)<br>* page 113; Fig. 25; SEQ ID NO: 58 *<br>----- | 13 | |
| X,P | WO 2015/101414 A2 (CUREVAC GMBH [DE]) 9 July 2015 (2015-07-09)<br>* the whole document *<br>* in particular claims 1-77 *<br>----- | 1-22 | TECHNICAL FIELDS SEARCHED (IPC)<br>A61K<br>C12N |
| X,P | WO 2015/101415 A1 (CUREVAC GMBH [DE]) 9 July 2015 (2015-07-09)<br>* the whole document *<br>* in particular claims 1-64 *<br>----- | 1-22 | |

The present search report has been drawn up for all claims

1

| Place of search | Date of completion of the search | Examiner |
|---|---|---|
| Munich | 4 March 2019 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 19 15 2916

This annex lists the patent family members relating to the patent documents cited in the above-mentioned European search report. The members are as contained in the European Patent Office EDP file on
The European Patent Office is in no way liable for these particulars which are merely given for the purpose of information.

04-03-2019

| Patent document cited in search report | Publication date | Patent family member(s) | Publication date |
|---|---|---|---|
| WO 2013143700 A2 | 03-10-2013 | AU 2013242405 A1 | 25-09-2014 |
| | | BR 112014023898 A2 | 11-07-2017 |
| | | CA 2866945 A1 | 03-10-2013 |
| | | CN 104321432 A | 28-01-2015 |
| | | CN 108929880 A | 04-12-2018 |
| | | ES 2660129 T3 | 20-03-2018 |
| | | JP 6301906 B2 | 28-03-2018 |
| | | JP 2015517803 A | 25-06-2015 |
| | | KR 20140139101 A | 04-12-2014 |
| | | MX 358706 B | 31-08-2018 |
| | | RU 2014142881 A | 20-05-2016 |
| | | SG 10201607966U A | 29-11-2016 |
| | | SG 11201405545X A | 27-11-2014 |
| | | US 2015050302 A1 | 19-02-2015 |
| | | WO 2013143700 A2 | 03-10-2013 |
| WO 2007068265 A1 | 21-06-2007 | AT 497009 T | 15-02-2011 |
| | | AU 2005339232 A1 | 21-06-2007 |
| | | CA 2632900 A1 | 21-06-2007 |
| | | EP 1974042 A1 | 01-10-2008 |
| | | US 2009181427 A1 | 16-07-2009 |
| | | WO 2007068265 A1 | 21-06-2007 |
| WO 2013120629 A1 | 22-08-2013 | AU 2013220749 A1 | 24-07-2014 |
| | | BR 112014019627 A2 | 27-06-2017 |
| | | CA 2862476 A1 | 22-08-2013 |
| | | CN 104114705 A | 22-10-2014 |
| | | CN 108570468 A | 25-09-2018 |
| | | EP 2814963 A1 | 24-12-2014 |
| | | JP 6357111 B2 | 11-07-2018 |
| | | JP 2015508646 A | 23-03-2015 |
| | | JP 2018110588 A | 19-07-2018 |
| | | KR 20140125434 A | 28-10-2014 |
| | | MX 359953 B | 17-10-2018 |
| | | RU 2014137109 A | 10-04-2016 |
| | | SG 10201606784Q A | 29-12-2016 |
| | | SG 11201403944R A | 30-10-2014 |
| | | US 2015057340 A1 | 26-02-2015 |
| | | US 2017056529 A1 | 02-03-2017 |
| | | US 2018177894 A1 | 28-06-2018 |
| | | US 2018185517 A1 | 05-07-2018 |
| | | WO 2013120497 A1 | 22-08-2013 |
| | | WO 2013120629 A1 | 22-08-2013 |
| WO 2015101414 A2 | 09-07-2015 | BR 112016014462 A2 | 24-10-2017 |
| | | CA 2927862 A1 | 09-07-2015 |



For more details about this annex : see Official Journal of the European Patent Office, No. 12/82

## ANNEX TO THE EUROPEAN SEARCH REPORT ON EUROPEAN PATENT APPLICATION NO. EP 19 15 2916

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2019

| Patent document cited in search report | Publication date | Patent family member(s) | Publication date |
|---|---|---|---|
| | | CN 105874072 A | 17-08-2016 |
| | | DK 3090053 T3 | 25-02-2019 |
| | | JP 2017502670 A | 26-01-2017 |
| | | KR 20160104062 A | 02-09-2016 |
| | | RU 2016131391 A | 02-02-2018 |
| | | SG 11201603144Q A | 28-07-2016 |
| | | US 2017029847 A1 | 02-02-2017 |
| | | US 2019032077 A1 | 31-01-2019 |
| | | WO 2015101414 A2 | 09-07-2015 |
| WO 2015101415 A1 | 09-07-2015 | CA 2927254 A1 | 09-07-2015 |
| | | JP 2017502669 A | 26-01-2017 |
| | | US 2016304883 A1 | 20-10-2016 |
| | | WO 2015101415 A1 | 09-07-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2014003479 W
- WO 02098443 A
- WO 2013143700 A
- WO 2007068265 A1
- WO 2012019780 A
- US 4373071 A
- US 4401796 A
- US 4415732 A
- US 4458066 A
- US 4500707 A
- US 4668777 A
- US 4973679 A
- US 5047524 A
- US 5132418 A
- US 5153319 A
- US 5262530 A
- US 5700642 A
- WO 2010037539 A


### Non-patent literature cited in the description


- **FRIEDEL et al.** Conserved principles of mammalian transcriptional regulation revealed by RNA half-life. *Nucleic Acid Research,* 2009, vol. 37 (17), 1-12
- **JOHNSON et al.** Newly synthesized RNA: Simultaneous measurement in intact cells of transcription rates and RNA stability of insulin-like growth factor I, actin, and albumin in growth hormone-stimulated hepatocytes. *Proc. Natl. Acad. Sci.,* 1991, vol. 88, 5287-5291
- **RODGERS et al.** Regulated α-globin mRNA decay is a cytoplasmic event proceeding through 3'-to-5' exosome-dependent decapping. *RNA,* 2002, vol. 8, 1526-1537
- **WANG et al.** An mRNA stability complex functions with poly(A)-binding protein to stabilize mRNA in vitro. *Molecular and Cellular biology,* July 1999, vol. 19 (7), 4552-4560
- **LEDDA et al.** Effect of the 3'-UTR length on the translational regulation of 5'-terminal oligopyrimidine mRNAs. *Gene,* 2005, vol. 344, 213-220
- **JANOVICK-GURETZKY et al.** Housekeeping Gene Expression in Bovine Liver is Affected by Physiological State, Feed Intake, and Dietary Treatment. *J. Dairy Sci.,* 2007, vol. 90, 2246-2252
- **EISENBERG, E. ; E. Y. LEVANON.** Human housekeeping genes are compact. *Trends Genet.,* 2003, vol. 19 (7), 362-365
- **BAKHEET, T. ; FREVEL, M. ; WILLIAMS, BR ; K.S. KHABAR.** ARED: Human AU-rich element-containing mRNA database reveals unexpectedly diverse functional repertoire of encoded proteins. *Nucleic Acids Research,* 2001, vol. 29, 246-254
- **AKASHI.** *Curr. Opin. Genet. Dev.,* 2001, vol. 11 (6), 660-666
- **CAKMAKCI ; LERNER ; WAGNER ; ZHENG ; WILLIAM F MARZLUFF.** *Mol. Cell. Biol.,* 2008, vol. 28 (3), 1182-94